# EUROPEAN PATENT SPECIFICATION

(11) **EP 2 966 181 B1**
(45) Date of publication and mention of the grant of the patent: **14.02.2018**
(21) Application number: 15173927.3
(22) Date of filing: 25.06.2010
(51) Int. Cl.: C12Q 1/68

(54) **METHODS AND NUCLEIC ACIDS FOR ANALYSIS OF BLADDER CARCINOMA**
VERFAHREN UND NUKLEINSÄUREN ZUR ANALYSE DES BLASENKARZINOM
PROCEDES ET ACIDES NUCLEIQUES POUR L'ANALYSE DU CARCINOME DE LA VESSIE

(30) Priority: 26.06.2009 EP 09163934
(43) Date of publication of application: 13.01.2016
(62) Divisional of application: 10726117.4
(73) Proprietor: Epigenomics AG, 10829 Berlin (DE)
(72) Inventor: Wasserkort, Reinhold, 18055 Rostock (DE)
(74) Representative: Zwicker, Jörk

(56) References cited:
- EP-A1- 1 291 427
- WO-A2-02/18632
- WO-A2-2006/004597
- WO-A2-2009/069984

## Description

### FIELD OF THE INVENTION

The present invention relates to genomic DNA sequences that exhibit altered expression patterns in disease states relative to normal. Particular embodiments provide methods, nucleic acids, nucleic acid arrays and kits useful for detecting, or for diagnosing bladder carcinoma.

### BACKGROUND

*CpG island methylation.* Aberrant methylation of CpG islands has been shown to lead to the transcriptional silencing of certain genes that have been previously linked to the pathogenesis of various cell proliferative disorders, including bladder carcinoma. CpG islands are sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting. For example, WO 2009/069984 A1 discloses the diagnosis of bladder cancer by detecting the methylation of the promoter or exon region of the CYP1B1 gene in a clinical sample. WO 02/18632 A2 discloses a library of genomic sequences for methylation analysis of CpG dinucleotides, including regions to the CYP1B1 gene.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e.,* individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e.,* individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cell proliferative disorders, often involve a plurality of genes and proteins. Additionally, certain proteins may be elevated for reasons unrelated to a disease state. Specificity is important when the cost or risk associated with further diagnostic procedures or further medical intervention are very high.

### SUMMARY OF THE INVENTION

The present invention provides a method for detecting bladder carcinoma, in a subject comprising determining methylation status of CYP1B1 genomic DNA according to SEQ ID NO: 12 in a biological sample isolated from said subject wherein hypermethylation is indicative of the presence of said disorder. Various aspects of the present invention provide an efficient and unique genetic marker, whereby methylation analysis of said marker enables the detection of bladder carcinoma with a particularly high sensitivity, specificity and/or predictive value.

Said method comprises the following steps: i) contacting genomic DNA isolated from a biological sample (preferably selected from the group consisting of bladder tissue samples, histological slides, tissue embedded in paraffin, body fluids like blood plasma or serum, cell lines, urine specimen) obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence of CYP1B1 genomic DNA according to SEQ ID NO: 12 and ii) detecting bladder carcinoma , at least in part.

The present invention provides for ascertaining epigenetic parameters of genomic DNA associated with the development of bladder carcinoma. The method has utility for the improved detection and diagnosis of said disease.

Preferably, the source of the test sample is selected from the group consisting of bladder tissue samples, histological slides, tissue embedded in paraffin, body fluids like blood plasma or serum, cell lines, urine specimen and combinations thereof.

Specifically, the present invention provides a method for detecting bladder carcinoma suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within a target sequence of the subject nucleic acid, wherein the target sequence comprises, or hybridises under stringent conditions to, a sequence comprising at least 16 contiguous nucleotides of a genomic sequence according to SEQ ID NO: 12 said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 38, 39, 68, 69 and contiguous regions thereof corresponding to the target sequence. The nucleotide sequences which correspond to the individual sequence ID NOS are summarized by tables 1, 2 and 3.

It is envisaged that at least parts of the nucleotide sequences according to the invention are utilised for at least one of: detection of; detection and differentiation between or among subclasses of; diagnosis of; prognosis of; treatment of; monitoring of; treatment and monitoring of; monitoring the treatment of; predicting the outcome of treatment of; and stage differentiation of bladder carcinoma.

Additional embodiments provide a method for the detection of bladder carcinoma comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting of SEQ ID NO: 37, 38, 68, 69 and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one but more preferably a plurality of CpG dinucleotides of at least one genomic sequence according to SEQ ID NO: 12.
Preferably, determining comprises use of at least one method selected from the group consisting of: i) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 38, 39, 68, 69 and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 38, 39, 68, 69 and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 38, 39, 68, 69 and complements thereof, and extending at least one such hybridized nucleic acid molecule by at least one nucleotide base; and iv) sequencing of the amplificate.

Further embodiments provide a method for the analysis (i.e. detection or diagnosis) of bladder carcinoma, comprising: obtaining a biological sample having subject genomic DNA; extracting the genomic DNA; contacting the genomic DNA, or a fragment thereof, comprising a sequence according to SEQ ID NO: 12 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of a sequence according to SEQ ID NO: 12 or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within a sequence according to SEQ ID NO: 12 for use in the method of the invention.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5.

"Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."
In reference to composite array sequences, the phrase "contiguous nucleotides" refers to a contiguous sequence region of any individual contiguous sequence of the composite array, but does not include a region of the composite array sequence that includes a "node," as defined herein above.

Furthermore as a plurality of SNPs are known within CYPB1 the term shall be taken to include all sequence variants thereof.

### Overview:

The present invention provides a method for detecting bladder carcinoma in a subject comprising determining the methylation status of CYP1B1 genomic DNA according to SEQ ID NO: 12 in a biological sample isolated from said subject wherein hyper-methylation is indicative of the presence of said disorder. Said marker may be used for the diagnosis of bladder carcinoma.

The gene product of CYP1 B1 belongs to the cytochrome p450 super family of monooxygenases and serves as a source of retinoic acid during embryonic and early postnatal development.

In addition to the embodiments above wherein the methylation analysis of genomic DNA according to SEQ ID NO: 12 CYP1B1 is analysed, the invention presents panels of genes further comprising at least one gene or genomic sequence selected from the group consisting of AC051635.7, PRDM14, DMRT2 and SOX1 and regulatory sequences thereof with novel utility for the detection of bladder carcinoma.

*Bisulfite modification of DNA is an art-recognized tool used to assess CpG methylation status.* The most frequently used method for analyzing DNA for the presence of 5-methylcytosine is based upon the reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

An overview of art-recognized methods for detecting 5-methylcytosine is provided by Rein, T., et al., Nucleic Acids Res., 26:2255, 1998.

The bisulfite technique, barring few exceptions (*e.g*., Zeschnigk M, et al., Eur J Hum Genet. 5:94-98, 1997), is currently only used in research. In general, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bio-essays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

Disclosed is the use of the bisulfite technique, in combination with one or more methylation assays, for determination of the methylation status of CpG dinucleotide sequences within at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood or ejaculate. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present diclosure the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) at least one gene or genomic sequence selected from the group consisting of AC051635.7, PRDM14, DMRT2, CYP1 B1 and SOX1 and regulatory sequences thereof.

According to the present disclosure, determination of the methylation status of CpG dinucleotide sequences within at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15 has utility in the diagnosis and detection of bladder carcinoma.

*Methylation Assay Procedures.* Various methylation assay procedures are known in the art, and can be used in conjunction with the present invention. These assays allow for determination of the methylation state of one or a plurality of CpG dinucleotides (*e.g*., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g*., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA.* COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (*e.g*., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g*., as might be found in a typical MethyLight™-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1 % methylated alleles of a given CpG island locus, and can be performed on DNA extracted from paraffin-embedded samples. Typical reagents (*e.g*., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MethyLight*™. The MethyLight™ assay is a high-throughput quantitative methylation assay that utilizes fluorescence-based real-time PCR (TaqMan®) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight™ process can by used with any suitable probes e.g. "TaqMan®" , Lightcycler® etc.... For example, double-stranded genomic DNA is treated with sodium bisulfite and subjected to one of two sets of PCR reactions using TaqMan® probes; *e.g*., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical MethyLight™-based kit) for MethyLight™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM™ (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM™ process can by used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical QM™ -based kit) for QM™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g*., microdissected pathology sections), and it avoids utilization of restriction enzymes for determining the methylation status at CpG sites.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labeled nucleotides. Additionally, bisulfite conversion reagents may include: DNA denaturation buffer; sulfonation buffer; DNA recovery regents or kit (*e.g*., precipitation, ultrafiltration, affinity column); desulfonation buffer; and DNA recovery components.

The genomic sequence(s) according to at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15 , and non-naturally occurring treated variants thereof according to SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75, were determined to have novel utility for the detection of bladder carcinoma.

Aberrant expression of mRNA transcribed from at least one gene or genomic sequence selected from the group consisting of AC051635.7, PRDM14, DMRT2, CYP1B1 and SOX1 and regulatory sequences thereof is associated with the presence of bladder carcinoma in a subject. According to the present disclosure, hyper-methylation and /or under-expression is associated with the presence of bladder carcinoma.

Aberrant levels of polypeptide expression of the polypeptides encoded at least one gene or genomic sequence selected from the group consisting of AC051635.7, PRDM14, DMRT2, CYP1 B1 and SOX1 and regulatory sequences thereof are associated with the presence of bladder carcinoma.

According to the present disclosure under-expression of said polypeptides is associated with the presence of bladder carcinoma.

Particular embodiments of the present invention provide a novel application of the analysis of methylation levels and/or patterns within CYP1B1 genomic DNA according to SEQ ID NO:12 that enables a precise detection, characterisation and/or treatment of bladder carcinoma. Early detection of bladder carcinoma is directly linked with disease prognosis, and the disclosed method thereby enables the physician and patient to make better and more informed treatment decisions.

In the most preferred embodiment of the method, the presence or absence of bladder carcinoma is determined by analysis of the methylation status of one or more CpG dinucleotides of CYP1B1 genomic DNA according to SEQ ID NO:12.

In one embodiment the invention of said method comprises the following steps: i) contacting genomic DNA (preferably isolated from urine specimen) obtained from the subject with at least one reagent, or series of reagents that distinguishes between methylated and non-methylated CpG dinucleotides within CYP1B1 genomic DNA according to SEQ ID NO: 12 and ii) detecting bladder carcinoma.

The present invention further provides a method for ascertaining genetic and/or epigenetic parameters of CYP1B1 genomic DNA according to SEQ ID NO: 12 within a subject by analyzing cytosine methylation. Said method comprising contacting a nucleic acid comprising at least one genomic sequence according to SEQ ID NO: 12 in a biological sample obtained from said subject with at least one reagent or a series of reagents, wherein said reagent or series of reagents, distinguishes between methylated and non-methylated CpG dinucleotides within the target nucleic acid.
In a preferred embodiment, said method comprises the following steps: In the *first step,* a sample of the tissue to be analysed is obtained. The source may be any suitable source, such as bladder tissue samples, histological slides, tissue embedded in paraffin, body fluids like blood plasma or serum, cell lines and all possible combinations thereof. It is preferred that said sources of DNA are urine specimen.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, -tetramethylchromane 2-carboxylic acid or trihydroxybenzoe acid and derivates thereof, e.g. Gallic acid (see: PCT/EP2004/011715). The bisulfite conversion is preferably carried out at a reaction temperature between 30°C and 70°C, whereby the temperature is increased to over 85°C for short periods of times during the reaction (see: PCT/EP2004/011715). The bisulfite treated DNA is preferably purified prior to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715).

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NOS: 38, 39, 68, 69 and sequences complementary thereto.

In an alternate embodiment of the method, the methylation status of pre-selected CpG positions within CYP1B1 genomic DNA according to SEQ ID NO: 12 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridises to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridises to a treated nucleic acid sequence according to one of SEQ ID NOS: 38, 39, 68, 69 and sequences complementary thereto, wherein the base sequence of said oligomers comprises at least one CpG dinucleotide .A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or `TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridises to a treated nucleic acid sequence according to one of SEQ ID NOS: 38, 39, 68, 69 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence according to SEQ ID NO: 12, and the equivalent positions within SEQ ID NOS: 38, 39, 68, 69. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

### BEST MODE

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.
Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NOS: 38, 39, 68, 69 and sequences complementary thereto, wherein the base sequence of said oligomers comprise at least one CpG dinucleotide.

Step e) of the method, namely the detection of the specific amplificates indicative of the methylation status of one or more CpG positions according to at least one genomic sequence according to SEQ ID NO: 12 is carried out by means of *real-time* detection methods as described above.

Additional embodiments of the invention provide a method for the analysis of the methylation status of CYP1B1 genomic DNA according to SEQ ID NO: 12 without the need for bisulfite conversion. Methods are known in the art wherein a methylation sensitive restriction enzyme reagent, or a series of restriction enzyme reagents comprising methylation sensitive restriction enzyme reagents that distinguish between methylated and non-methylated CpG dinucleotides within a target region are utilized in determining methylation, for example but not limited to differential methylation hybridization (DMH).

In the *first step* of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are bladder tissue samples, histological slides, tissue embedded in paraffin, body fluids like blood plasma or serum, cell lines and combinations thereof. Body fluids are the preferred source of the DNA; particularly preferred are urine specimen.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, BfaI, Csp6I, Tru1I, Tvu1I, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of at least one gene or genomic sequence selected from the group consisting of AC051635.7, PRDM14, DMRT2, CYP1 B1 and SOX1 and regulatory sequences thereof.

Preferably, the methylation-specific restriction enzyme is selected from the group consisting of *Bsi E1, Hga I HinPI, Hpy99I, Ava I, Bce AI, Bsa HI, BisI, BstUI, BshI236I, Accll, BstFNI, McrBC, GlaI, MvnI, HpaII (HapII), HhaI, AciI, SmaI, HinP1I, HpyCH4IV, EagI* and mixtures of two or more of the above enzymes. Preferred is a mixture containing the restriction enzymes BstUI, HpaII, HpyCH4IV and HinP1I.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from a group comprising SEQ ID NO: 12, and the complements thereof. Preferably said contiguous sequence is at least 16, 20 or 25 nucleotides in length. In an alternative embodiment said primers may be complementary to any adaptors linked to the fragments.

In the *fifth step* the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence selected from a group comprising SEQ ID NO: 12, and the complements thereof. Preferably said contiguous sequence is at least 16, 20 or 25 nucleotides in length.

Subsequent to the determination of the methylation state or level of the genomic nucleic acids the presence, absence of bladder carcinoma , is deduced based upon the methylation state or level of at least one CpG dinucleotide sequence of a genomic sequence according to SEQ ID NO: 12 or an average, or a value reflecting an average methylation state of a plurality of CpG dinucleotide sequences of a genomic sequence according to SEQ ID NO: 12 wherein methylation is associated with the presence of bladder carcinoma. Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

Upon determination of the methylation CYP1B1 genomic DNA according to SEQ ID NO: 12, the presence or absence of bladder carcinoma is determined, wherein hyper-methylation indicates the presence of bladder carcinoma and hypo-methylation indicates the absence of bladder carcinoma within the subject.

### FURTHER IMPROVEMENTS

The disclosed invention provides treated nucleic acids, derived from at least one genomic sequence according to SEQ ID NO: 12 wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the invention, the invention provides a non-naturally occurring modified nucleic acid comprising a sequence of at least 16 contiguous nucleotide bases in length of a sequence selected from the group consisting of SEQ ID NOS: 38, 39, 68, 69. In further preferred embodiments of the invention said nucleic acid is at least 50, 100, 150, 200, 250 or 500 base pairs in length of a segment of the nucleic acid sequence disclosed in SEQ ID NOS: 38, 39, 68, 69. Particularly preferred is a nucleic acid molecule that is not identical or complementary to all or a portion of the sequences SEQ ID NO: 38, 39, 40, 41, 42, 43, 44, 45, 46, 47, 48, 49, 50, 51, 52, 53, 54, 55, 56, 57, 64 65. 66, 67, 68, 69, but not a genomic sequence according to SEQ ID NO: 12 or other naturally occurring DNA.

It is preferred that said sequence comprises at least one CpG, TpA or CpA dinucleotide and sequences complementary thereto. The sequences of SEQ ID NOS: 38, 39, 68, 69, provide non-naturally occurring modified versions of the nucleic acid according to SEQ ID NO: 12, wherein the modification of each genomic sequence results in the synthesis of a nucleic acid having a sequence that is unique and distinct from said genomic sequence as follows. For each sense strand genomic DNA, *e.g.,* derived from the genomic sequence according to SEQ ID NO: 12, four converted versions are disclosed. A first version wherein "C" is converted to "T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (i.e. *anti*sense strand), wherein "C" is converted to "T," but "CpG" remains "CpG" (*i.e.,* corresponds to case where, for all "C" residues of CpG dinucleotide sequences are methylated and are thus not converted). The 'upmethylated' converted sequences of the genomic sequence according to SEQ ID NO: 12 correspond to SEQ ID NOS: 38, 39. A third chemically converted version of each genomic sequences is provided, wherein "C" is converted to "T" for all "C" residues, including those of "CpG" dinucleotide sequences (*i.e.,* corresponds to case where, for the genomic sequences, all "C" residues of CpG dinucleotide sequences are *un*methylated); a final chemically converted version of each sequence, discloses the complement of the disclosed genomic DNA sequence (i.e. antisense strand), wherein "C" is converted to "T" for all "C" residues, including those of "CpG" dinucleotide sequences (*i.e.,* corresponds to case where, for the complement (*anti*sense strand) of each genomic sequence, all "C" residues of CpG dinucleotide sequences are *un*methylated). The 'downmethylated' converted sequences of the genomic sequence according to SEQ ID NO: 12 corresponds to SEQ ID NOS: 68, 69.
Significantly, heretofore, the nucleic acid sequences and molecules according SEQ ID NOS: 38, 39, 68, 69 were not implicated in or connected with the detection or diagnosis of bladder carcinoma.

In an alternative preferred embodiment, the invention further provides oligonucleotides or oligomers suitable for use in the methods of the invention for detecting the cytosine methylation state within genomic or treated (chemically modified) DNA, according to SEQ ID NOS: 12, 38, 39, 68, 69 or to the complements thereof. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NOS: 38, 39, 68, 69 and/or sequences complementary thereto, or to a genomic sequence according to the genomic sequence according to SEQ ID NO: 12 and/or a sequence complementary thereto.

Thus, the present invention includes nucleic acid molecules (*e.g*., oligonucleotides and peptide nucleic acid (PNA) molecules (PNA-oligomers)) that hybridize under moderately stringent and/or stringent hybridization conditions to all or a portion of the sequences SEQ ID NOS: 12, 38, 68, 69, or to the complements thereof. Particularly preferred is a nucleic acid molecule that hybridizes under moderately stringent and/or stringent hybridization conditions to all or a portion of the sequences SEQ ID NOS: 38, 68, 69, but not to the genomic sequence according to SEQ ID NO: 12 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NOS: 12, 38, 39, 68, 69, or to the complements thereof.

Hybridizing nucleic acids of the type described herein can be used, for example, as a primer (e.g., a PCR primer), or a diagnostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the genomic sequence according to SEQ ID NO: 12 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, e.g., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions: n to (n + (X-1)); where n = 1, 2, 3,...(Y-(X-1)); where Y equals the length (nucleotides or base pairs) of SEQ ID NO: 1 (3300); where X equals the common length (in nucleotides) of each oligonucleotide in the set (e.g., X = 20 for a set of consecutively overlapping 20-mers); and where the number (Z) of consecutively overlapping oligomers of length X for a given SEQ ID NO: 1 of length Y is equal to Y - (X-1). For example Z = 3300 - 19 = 3281 for either sense or antisense sets of SEQ ID NO: 1, where X = 20.

Examples of 20-mer oligonucleotides include the following set of 3281 oligomers (and the antisense set complementary thereto), indicated by polynucleotide positions with reference to SEQ ID NO 1: 1 - 20, 2 - 21, 3 - 22, 4 - 23, 5 - 24, .............. and 3281 - 3300.

Likewise, examples of 25-mer oligonucleotides include the following set of 3276 oligomers (and the antisense set complementary thereto), indicated by polynucleotide positions with reference to SEQ ID NO 1: 1-25, 2-26, 3-27, 4-28, 5-29, ...... and 3276-3300.

Preferably, the set is limited to those oligomers that comprise at least one CpG, TpG or CpA dinucleotide.

The present invention encompasses, for each of SEQ ID NOS: 12, 38, 39 (sense and antisense), multiple consecutively overlapping sets of oligonucleotides or modified oligonucleotides of length X, where, *e.g*., X = 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to the genomic sequence according to SEQ ID NO: 12. Preferred sets of such oligonucleotides or modified oligonucleotides of length X are those consecutively overlapping sets of oligomers corresponding to SEQ ID NOS: 12, 38, 39, (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758; 5,565,552; 5,567,810; 5,574,142; 5,585,481; 5,587,371; 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of the genomic sequence according to SEQ ID NO: 12 and sequences complementary thereto, or to the corresponding CpG, TpG or CpA dinucleotide within a sequence of the treated nucleic acids according to SEQ ID NOS: 38, 39, 68, 69 and sequences complementary thereto. However, it is anticipated that for economic or other factors it may be preferable to analyse a limited selection of the CpG dinucleotides within said sequences, and the content of the set of oligonucleotides is altered accordingly.
in particular embodiments, the set further comprises at least one (oligonucleotides and/or PNA-oligomers) useful for detecting the cytosine methylation state in treated genomic DNA (SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 40, 41, 42, 43, 44, 45, 46, 47, 48, 49, 50, 51, 52, 53, 54, 55, 56, 57, 64, 65, 66, 67, 70, 71, 72, 73, 74, 75), or in genomic DNA (at least one genomic sequence, selected from a group comprising SEQ ID NO: 7, 1,4, 10 and 13; preferably from SEQ ID NO: 8, 2, 5, 11, 14 and most preferably from SEQ ID NO: 9, 3, 6, 15 and sequences complementary thereto). These probes enable diagnosis and detection of bladder carcinoma. The set of oligomers may also be used for detecting single nucleotide polymorphisms (SNPs) in treated genomic DNA (SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75), or in genomic DNA (at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NOS: 12 38, 39, and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.,* each analyte) can be uniquely bound by a unique label and thus detected (each label is directly counted, resulting in a digital read-out of each molecular species in the mixture). It is particularly preferred that the oligomers according to the invention are utilised for detecting, or for diagnosing bladder carcinoma.

### Kits

Disclosed is a kit comprising: a means for determining methylation of at least one gene or genomic sequence selected from the group consisting of AC051635.7, PRDM14, DMRT2, CYP1 B1 and SOX1 and regulatory sequences thereof. The means for determining methylation of at least one gene or genomic sequence selected from the group consisting of AC051635.7, PRDM14, DMRT2, CYP1B1 and SOX1 and regulatory sequences thereof comprise preferably a bisulfite-containing reagent; one or a plurality of oligonucleotides consisting whose sequences in each case are identical, are complementary, or hybridise under stringent or highly stringent conditions to a 9 or more preferably 18 base long segment of a sequence selected from SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75; and optionally instructions for carrying out and evaluating the described method of methylation analysis. In one embodiment the base sequence of said oligonucleotides comprises at least one CpG, CpA or TpG dinucleotide.
Said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit can also contain only part of the aforementioned components.
The kit may comprise additional bisulfite conversion reagents selected from the group consisting: DNA denaturation buffer; sulfonation buffer; DNA recovery reagents or kits (*e.g*., precipitation, ultrafiltration, affinity column); desulfonation buffer; and DNA recovery components.
The kit may contain, packaged in separate containers, a polymerase and a reaction buffer optimised for primer extension mediated by the polymerase, such as PCR. The kit may further comprise means for obtaining a biological sample of the patient. Preferred is a kit, which further comprises a container suitable for containing the means for determining methylation of at least one gene or genomic sequence selected from the group consisting of AC051635.7, PRDM14, DMRT2, CYP1 B1 and SOX1 and regulatory sequences thereof in the biological sample of the patient, and most preferably further comprises instructions for use and interpretation of the kit results. Preferably the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75; and optionally (d) instructions for use and interpretation of the kit results. Alternatively the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75 and sequences complementary thereto; and optionally (d) instructions for use and interpretation of the kit results.

Alternatively the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75; (d) at least one oligonucleotides and/or PNA-oligomer having a length of at least 9 or 16 nucleotides which is identical to or hybridises to a pre-treated nucleic acid sequence according to one of SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75 and sequences complementary thereto; and optionally (e) instructions for use and interpretation of the kit results.

The kit may also contain other components such as buffers or solutions suitable for blocking, washing or coating, packaged in a separate container.

Typical reagents (*e.g*., as might be found in a typical COBRA™-based kit) for COBRA™ analysis may include, but are not limited to: PCR primers for at least one gene or genomic sequence selected from the group consisting of AC051635.7, PRDM14, DMRT2, CYP1 B1 and SOX1 and regulatory sequences thereof restriction enzyme and appropriate buffer; gene-hybridization oligo; control hybridization oligo; kinase labeling kit for oligo probe; and labeled nucleotides. Typical reagents (*e.g*., as might be found in a typical MethyLight™ -based kit) for MethyLight™ analysis may include, but are not limited to: PCR primers for the bisulfite converted sequence of at least one gene or genomic sequence selected from the group consisting of AC051635.7, PRDM14, DMRT2, CYP1 B1 and SOX1 and regulatory sequences thereof bisulfite specific probes (e.g. TaqMan™ or Lightcycler™); optimized PCR buffers and deoxynucleotides; and Taq polymerase.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of at least one gene or genomic sequence selected from the group consisting of AC051635.7, PRDM14, DMRT2, CYP1 B1 and SOX1 and regulatory sequences thereof reaction buffer (for the Ms-SNuPE reaction); and labelled nucleotides.

Typical reagents (*e.g.,* as might be found in a typical MSP-based kit) for MSP analysis may include, but are not limited to: methylated and unmethylated PCR primers for the bisulfite converted sequence of at least one gene or genomic sequence selected from the group consisting of AC051635.7, PRDM14, DMRT2, CYP1 B1 and SOX1 and regulatory sequences thereof, optimized PCR buffers and deoxynucleotides, and specific probes.

Moreover, an additional aspect of the disclosure is an alternative kit comprising a means for determining at least one gene or genomic sequence selected from the group consisting of AC051635.7, PRDM14, DMRT2, CYP1 B1 and SOX1 and regulatory sequences thereof methylation, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15; and optionally instructions for carrying out and evaluating the described method of methylation analysis. the base sequence of said oligonucleotides may be identical, complementary, or hybridise under stringent or highly stringent conditions to an at least 18 base long segment of at least one sequence selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15.
Said kit may comprise one or a plurality of oligonucleotide probes for the analysis of the digest fragments, preferably said oligonucleotides are identical, are complementary, or hybridise under stringent or highly stringent conditions to an at least 16 base long segment of at least one sequence selected from a group comprising SEQ ID NO: 7, 1,4, 10 and 13; preferably from SEQ ID NO: 8, 2, 5, 11, 14 and most preferably from SEQ ID NO: 9, 3, 6, 12, 15.
The kit may comprise additional reagents selected from the group consisting: buffer (e.g. restriction enzyme, PCR, storage or washing buffers); DNA recovery reagents or kits (*e.g.,* precipitation, ultrafiltration, affinity column) and DNA recovery components.

Alternatively the kit may contain, packaged in separate containers, a polymerase and a reaction buffer optimised for primer extension mediated by the polymerase, such as PCR. The kit may further comprise for obtaining a biological sample of the patient. The kit preferably comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of at least one sequence selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15 ; and optionally (d) instructions for use and interpretation of the kit results.
Alternatively the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15 ; and optionally (d) instructions for use and interpretation of the kit results.

Alternatively the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15; (d) at least one set of oligonucleotides one or a plurality of nucleic acids or peptide nucleic acids which are identical , are complementary, or hybridise under stringent or highly stringent conditions to an at least 9 base long segment of a sequence selected from at least one genomic sequence, selected from a group comprising SEQ ID NO: 7, 1,4, 10 and 13; preferably from SEQ ID NO: 8, 2, 5, 11, 14 and most preferably from SEQ ID NO: 9, 3, 6, 12, 15 and optionally (e) instructions for use and interpretation of the kit results.

The kit may also contain other components such as buffers or solutions suitable for blocking, washing or coating, packaged in a separate container.

Disclosed is further a kit for use in providing a diagnosis of the presence or absence of bladder carcinoma , in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for at least one gene or genomic sequence selected from the group consisting of AC051635.7, PRDM14, DMRT2, CYP1 B1 and SOX1 and regulatory sequences thereof and comprises a sequence of at least 15 base pairs in length but no more than 200 bp of a sequence according to at least one genomic sequence, selected from a group comprising SEQ ID NO: 7, 1, 4, 10 and 13; preferably from SEQ ID NO: 8, 2, 5, 11, 14 and most preferably from SEQ ID NO: 9, 3, 6, 12, 15. Preferably said sequence is at least 15 base pairs in length but no more than 80 bp of at least one sequence according to one of SEQ ID NO: 7, 1, 4, 10 and 13; preferably from SEQ ID NO: 8, 2, 5, 11, 14 and most preferably from SEQ ID NO: 9, 3, 6, 12, 15. It is further preferred that said sequence is at least 20 base pairs in length but no more than 30 bp of a sequence according to least one sequence according to one of SEQ ID NO: 7, 1,4, 10 and 13; preferably from SEQ ID NO: 8, 2, 5, 11, 14 and most preferably from SEQ ID NO: 9, 3, 6, 12, 15.

Said test kit preferably further comprises a restriction enzyme component comprising one or a plurality of methylation-sensitive restriction enzymes.
Said test kit is optionally further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The disclosure further provides a composition of matter useful for detecting, or for diagnosing bladder carcinoma. Said composition comprising at least one nucleic acid 18 base pairs in length of a segment of the nucleic acid sequence disclosed in SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75, and one or more substances taken from the group comprising: 1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.
Said at least one nucleic acid is preferably at least 50, 100, 150, 200, 250 or 500 base pairs in length of a segment of the nucleic acid sequence disclosed in SEQ ID NOS: 28, 29, 30, 31, 32, 33, 58, 59, 60, 61, 62, 63, 16, 17, 18, 19, 20, 21, 22, 23, 24, 25, 26, 27, 34, 35, 36, 37, 38, 39, 40, 41, 42, 43, 44, 45, 46, 47, 48, 49, 50, 51, 52, 53, 54, 55, 56, 57, 64, 65, 66, 67, 68, 69, 70, 71, 72, 73, 74, 75.

### DESCRIPTION OF FIGURES

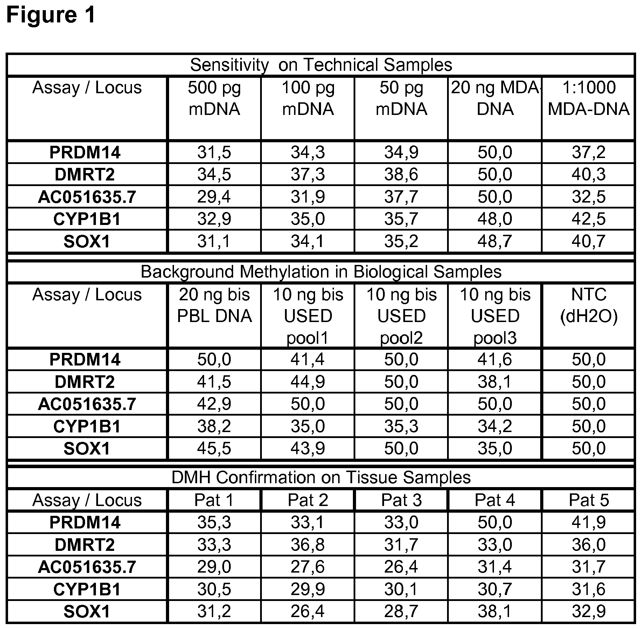

**Figure 1** shows the results from the technical evaluation of MSP assays. mDNA = fully methylated DNA, MDA = Multiple Displacement Amplification; this results in fully unmethylated DNA; a 1:1000 mixture of methylated in unmethylated DNA is used for assessing the relative sensitivity. USED= urine sediment pool. 3 pools with DNA from 3 to 5 different healthy people were analysed, therefore each pool represents a unique sample; NTCs = No template controls, samples which must be negative; Pat1-5: bladder tumour tissue samples which were used in DMH, therefore a positive assay performance on these samples provides confirmation of the DMH results. The values are qPCR data shown as Crossing Points (CPs). A CP of 50 means no methylated DNA detected; smaller correspond to increasing amounts of methylated DNA detected by the respective assay.

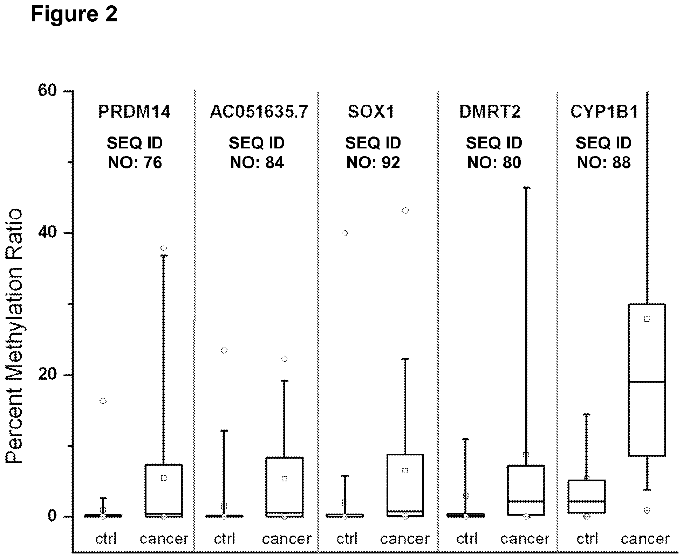

**Figure 2** shows the ratios of methylated versus total DNA within whole urine specimen obtained from healthy (crtl) individuals and from patients diagnosed with bladder carcinoma (cancer). Methylation was detected within PCR products (SEQ ID NOS: 84, 76, 80, 88, 92), each of which represents a sequence fragment of the genes indicated.

### EXAMPLES

### 1. Introduction

A reliable and non-invasive diagnosis of recurrent bladder carcinoma remains a challenge for the clinical practice. We employed differential methylation hybridisation (DMH) as methodology to discover DNA methylation-based biomarkers which are suitable for the early diagnosis of non-muscle invasive bladder carcinoma. Tumour tissue and DNA from urine sediments of healthy people was chosen as sample material for the discovery. Genomic loci identified as differentially methylated were filtered to select those candidates which are hypermethylated in tumour and predominantly unmethylated in the DNA derived from urine sediment of healthy people as well as in normal peripheral blood lymphocytes.

For a subgroup of these marker candidates we developed methylation specific PCR (MSP) assays to validate the findings from this discovery study. The best performing assays were finally tested on a collection of urine samples from patients with non-muscle invasive bladder carcinoma and patients free of bladder carcinoma.
Detailed results are shown for five marker candidates which are suitable for further assay development in order to obtain clinically useful tools for non-invasive and early diagnosis of bladder carcinoma.

### 2. Methods

### 2.1. Differential Methylation Hybridisation

Samples: Bladder carcinoma tumour tissue from six patients with a tumour cell content of 70 percent or more was chosen as one test group; DNA extracted from urine sediment samples from seven healthy people as the control group.

DMH: A whole genome library was prepared using methylation unspecific digestion, followed by adapter ligation and a methylation sensitive restriction digest. Amplification with primers matching the generic adapters yielded an amplificate which was subsequently hybridized to a microarray covering more than 50,000 genomic loci. Each sample was processed twice independently and hybridized to two arrays.

Analysis: Data were quality controlled using on-chip control oligos and additional quality criteria; chips of each sample that passed the quality controls were averaged and normalised based on technical samples with known methylation status. Potential biomarker candidates were selected based on effect size and intra group variation between individual loci of the two groups. A detailed description of the DMH technology is provided by FassbenderA. et al., Humana Press. Methods in Molecular Medicine. 2009, Christian Pilarsky and Robert Grützmann (Editor) ISBN: 978-1-934115-76-3 and by Lewin J. et al., Int J Biochem Cell Biol. 2007;39(7-8):1539-50. Candidate markers hypermethylated in tumour and predominantly unmethylated in healthy urine sediment as well as in normal peripheral blood lymphocytes were tagged with higher scores versus loci displaying other patterns of differential methylation.

### 2.2. Methylation Specific PCR Assays

Technical evaluation: 62 real time MSP assays were developed for the top scoring DMH loci. Most assays were designed with two primer pairs which allowed to select the better performing primer pairs.

All assays were then subjected to a testing scheme which was designed to allow:
- Assessment of assay performance using technical samples
   Minimum requirement: Detection of at least 50pg methylated DNA absolute and relative sensitivities, i.e. 50pg methylated DNA in a background of 50 ng of unmethylated DNA.
- Estimation of background methylation in biological controls
   Minimum requirement: Methylation levels in pooled samples of healthy urine sediment and peripheral blood lymphocyte DNA was expected to differ by 2 crossing points compared to technical samples with methylated DNA
- Verification of DMH results for the respective locus
   Minimum requirement: Positive assay response in at least 70% of tumour tissue samples Evaluation on Clinical Samples: Only assays with sufficient technical performance and acceptable background methylation levels on biological controls were selected for analysis on clinical urine samples. Of the 62 assays 8 assays were selected for further analysis. These assays were used to analyse whole urine samples from 20 bladder carcinoma patients, newly diagnosed or relapses, and 22 control samples, including cases with a history of bladder carcinoma.

All extracted DNA were subjected to bisulfite treatment, using protocols developed at Epigenomics. Real time PCRs were analysed on the ABI 7300 TaqMan platform. Total amounts of amplifiable DNA were quantified using a methylation unspecific assay.

### 3. Results

An overview of the technical evaluation of the MSP assays and the results from five assays is shown in Figure 1.

With those assays which could verify the methylation difference initially found by DMH on tumour tissue, elevated DNA methylation levels were also found in urine specimen. The sensitivities achieved with these research assays ranged from 40% to 65%. However, all targeted loci would allow refined assay designs which may allow to significantly improve assay sensitivities and specificities. In addition, the combination of assays in panels seems to be promising with regards to the results obtained so far.

### 4. Conclusions

• New biomarkers with the potential for clinical applicability in bladder carcinoma diagnosis were discovered using DMH technology.
• DNA methylation levels were found to be significantly higher in most urine samples from cancer patients as compared to samples from cancer free individuals.

**Table 1: Genomic Sequences (SEQ ID NOS)**

| Gene or Genomic Sequence | Genomic Sequence | Preferred genomic Region (ROI ± 300bp) | More preferred genomic region (ROI) | Most preferred genomic region (DMH Fragment) |
|---|---|---|---|---|
| AC051635.7 | 96 | 7 | 8 | 9 |
| PRDM14 | 97 | 1 | 2 | 3 |
| DMRT2 | 98 | 4 | 5 | 6 |
| CYP1B1 | 99 | 10 | 11 | 12 |
| SOX1 | 100 | 13 | 14 | 15 |

**Table 2: Methylated Bisulphite-converted (SEQ ID NOS)**

| Gene or Genomic Sequence | Genomic Sequence Methylated (sense) | Genomic Sequence Methylated (antisense) | Preferred genomic Region (ROI ± 300bp) Methylated (sense) | Preferred genomic Region (ROI ± 300bp) Methylated (antisense) | More preferred genomic region (ROI) Methylated (sense) | More preferred genomic region (ROI) Methylated (antisense) | Most preferred genomic region (DMH Fragment) Methylated (sense) | Most preferred genomic region (DMH Fragment) Methylated (antisense) |
|---|---|---|---|---|---|---|---|---|
| AC051635.7 | 101 | 102 | 28 | 29 | 30 | 31 | 32 | 33 |
| PRDM14 | 103 | 104 | 16 | 17 | 18 | 19 | 20 | 21 |
| DMRT2 | 105 | 106 | 22 | 23 | 24 | 25 | 26 | 27 |
| CYP1B1 | 107 | 108 | 34 | 35 | 36 | 37 | 38 | 39 |
| SOX1 | 109 | 110 | 40 | 41 | 42 | 43 | 44 | 45 |

**Table 3: Unmethylated Bisulphite-converted Sequences (SEQ ID NOS)**

| Gene or Genomic Sequence | Genomic Sequence Unmethylated (sense) | Genomic Sequence Unmethylated (antisense) | Preferred genomic Region (ROI ± 300bp) Unmethylated (sense) | Preferred genomic Region (ROI ± 300bp) Unmethylated (antisense) | More preferred genomic region (ROI) Unmethylated (sense) | More preferred genomic region (ROI) Unmethylated (antisense) | Most preferred genomic region (DMH Fragment) Unmethylated (sense) | Most preferred genomic region (DMH Fragment) Unmethylated (antisense) |
|---|---|---|---|---|---|---|---|---|
| AC051635.7 | 111 | 112 | 58 | 59 | 60 | 61 | 62 | 63 |
| PRDM14 | 113 | 114 | 46 | 47 | 48 | 49 | 50 | 51 |
| DMRT2 | 115 | 116 | 52 | 53 | 54 | 55 | 56 | 57 |
| CYP1B1 | 117 | 118 | 64 | 65 | 66 | 67 | 68 | 69 |
| SOX1 | 119 | 120 | 70 | 71 | 72 | 73 | 74 | 75 |

### Sequence listing

<110> Epigenomics AG
<120> Methods and nucleic acids for analysis of bladder cell proliferative disorders.
<130> 536-46 EPT1
<150> EP09163934.4
   <151> 2009-06-26
<160> 120
<210> 1
   <211> 3300
   <212> DNA
   <213> Homo Sapiens
<400> 1
   gacaccgcgc gggagcttcc cggggtccga cccgcgcctc cccaccagcc tcctccaccc     60
   cagccagccc cagatccaag ggaacaggat aaacgccccc agtccgatcc cgccagcccg    120
   ctcccacaat taagacaaaa cgaaaaaaca gcaatagaag caaacacaaa aaagccccaa    180
   acggaaatct gccgggatcc tgccctactc taaattccgg gttagtgttg ccttcccctt    240
   ccctctccaa gcaccccttc cctaagcccg gccgccttcc cacacccgct ctccgctttc    300
   ggcccccaac ccggttcctg caaaacccat tgaagttccc tttccttcct tcttctcagt    360
   cctacaaaac tcgttggtag ggtctgtggg agccgcgggg ccctgccaac ctcggggctg    420
   ggccgggagc cgagcgcagg cggtcggagg gcaatggagc aagcgacagc tccaggcgct    480
   cctgggccct cggctgggag ggaagagccg aggaccctgg gtcgcaccgc gcagatggag    540
   acgcgctccc agagcccccg ggcaggtcca gggaccccgc gacctgctct ggcccagcgg    600
   tgtgaccccg cgggtcctgg cggtcctgac tgcccgcagg ggaggggcgc gccacttttg    660
   gctgccctag gatgcgccgc ctgaacctct tttccctcgc gggcagcgtc cgccacattc    720
   cccgggttcc tcggaaactc caatcattct accaggacta ttggggcctg gggtagcccc    780
   tcgggagccg cgtggacgag ccctggccag gtgggagcga agagcctcgg cgactgccag    840
   tcctcccgcc cccgcaccgc cgggaaagga tggcgtttta atagacaggc agcaagttca    900
   ccgaggctga agaatgaagc cccttcaggg ccggcgggtc ttgagatcaa tgagcccaat    960
   aagaaaacca agcagggtca tgactcagaa attaaagaac acggggtcag gagaagaggt   1020
   cagccacgac acaggcagcg gcccgtctcc tcccgatcct ccaaaggcgc ccttctccta   1080
   gcctcctccg acctcaggga tctgcaactt gggattcaaa gcccccgaga gggcagaacg   1140
   accatccaaa acgaactgcg ccagcgagtg ctgccgccct ccccaacggc cctggaagac   1200
   caccgccccg ggccccaggc gcgtcctgtc ccgtgcctac ctccgacacc acctccaaga   1260
   gcgccaccgc ggagcgggcg cctcctggac gctgcggggc cactcgggct cggtaggcca   1320
   ctcgggctcg gtagacttcg ggcgggccct gagggctgag ccaggcttaa gagggcgggc   1380
   ggggccgcgg ggcatcgaca ggtcgagggt agggaattga cccggcccac gcagatcccc   1440
   gcgtcgtctt ctccccgccc accgcagcca ttggccgact ctcgactcgc cctctgtccc   1500
   ctggggtcgc tcacttaact catcagtttt ccaaaccctc cctgggaccc tagtggcgag   1560
   cactcgccgc tggccttcta gggcaggcag tattgccgct ccaggcgtgc ggagtcgggg   1620
   agaaaaaaac cgaacacgtg tgctacccag ggcccccaga taagcccctc cgcgcgcaca    1680
   cacaccctga accggagtcc agaaggccgg actcgcgtcc cggggtcgag ccccttactc    1740
   tccgctccca gttcggaaga tcccagcctg cccgaaggcg gcgtctgacc cgctggtgga   1800
   cctggtgggt gtctgttcct gatacctggg gacgaggcgg gagcgccttg agcgctggga   1860
   agcttcgcct cgcgagtccc cggcagtgac acgtgggagc gttgggcccg gcacccccga   1920
   cgcttccgag gtttcgggtg gtcgttttgc tctctcgggg gcttttattc cttaagtggc   1980
   tttaaacgac cgaggatggg cctcccgcgc cgccgcagct gtcacttcct tcgggaaagg   2040
   ctagcgacct tggacgcgca ggctctgaaa accaaggtcc agacccatac cgagcgccca   2100
   aagaccgcat ttgaattctc ctcgccggcc attgagggag agaaaggaac gcttagttcc   2160
   attcacattc acagaaagaa gcgccgaggg tgggggaaac gcagtcttgc cgggtgagcc   2220
   gggacaggtt cctcgcctgc cccccggccg ctgcttcctc ttagctgaat ggggagcgac   2280
   ccgccccggg cgcggccttc ggggctgaag actgaggtgc agcctcaccc ccggcctggc   2340
   agcggcttgg aagagagagg gaaaggagga acatctaccc ggctaagaga cgccgccaga   2400
   gtccctaaag ctggcggggg acggaggccg gggtcccgga tcttcagagc gcccgcagcc   2460
   gggcttctgg gctcctcggt gcggggaacc tgaccctgta gccttcgcca gggcgcggcg   2520
   gggctgcaag aagggaggaa ggagtgggac tagagccttc gagggcgcgg gcggcgtgca   2580
   ctcagccttg tctccccgcg cgccctgccg cctgcttgcc ccctttgctg caccgggtgg   2640
   ccctggtccc cgcgcccgcc cgcccccgac gccttcgcgt ggttccgcgc tgcccgcggc   2700
   cagccccgtt tcgcttcgca aggagcgtgt tgttttctca atcgaaatgg gatcaagatc   2760
   ccagggtttc ctcgtctcct tgttttgagg tgataggatc ttagccaata gatcgaaaac   2820
   gtgaaaagta cccgtcgcct cttgtcccaa gtgcctctgt ggaaggatca tttacttgga   2880
   aaagcagaat aaattctgtt gccgttttcc ttccggaggc gtgctcaccc cagagctcct   2940
   agacccactg gcattcgcta tacttcctcc tgggctttac aggtaggatt tcaagaatga   3000
   tatgggggca cctgggtgat ctgtgggttg caggtcctca agaggccgtt ttttcgtgca   3060
   cggaagtgtc gtagctttat tttagcggaa gcaatatcaa accttatctt agaggtttgt   3120
   tttgcaaaga atccagagtt tactatattt gacactattt taaaataaca cttgcttgtt   3180
   ttaagaagtt gggagaaatc gataaaagaa aagttgaaat ccatgcttcc catttctact   3240
   ctaggagata atcctgatca acagtttggt ttgctcccta cccccattcc ttaaaaacaa   3300
<210> 2
   <211> 2700
   <212> DNA
   <213> Homo Sapiens
<400> 2
   ggcccccaac ccggttcctg caaaacccat tgaagttccc tttccttcct tcttctcagt 60
   cctacaaaac tcgttggtag ggtctgtggg agccgcgggg ccctgccaac ctcggggctg 120
   ggccgggagc cgagcgcagg cggtcggagg gcaatggagc aagcgacagc tccaggcgct 180
   cctgggccct cggctgggag ggaagagccg aggaccctgg gtcgcaccgc gcagatggag 240
   acgcgctccc agagcccccg ggcaggtcca gggaccccgc gacctgctct ggcccagcgg 300
   tgtgaccccg cgggtcctgg cggtcctgac tgcccgcagg ggaggggcgc gccacttttg 360
   gctgccctag gatgcgccgc ctgaacctct tttccctcgc gggcagcgtc cgccacattc 420
   cccgggttcc tcggaaactc caatcattct accaggacta ttggggcctg gggtagcccc 480
   tcgggagccg cgtggacgag ccctggccag gtgggagcga agagcctcgg cgactgccag 540
   tcctcccgcc cccgcaccgc cgggaaagga tggcgtttta atagacaggc agcaagttca 600
   ccgaggctga agaatgaagc cccttcaggg ccggcgggtc ttgagatcaa tgagcccaat 660
   aagaaaacca agcagggtca tgactcagaa attaaagaac acggggtcag gagaagaggt 720
   cagccacgac acaggcagcg gcccgtctcc tcccgatcct ccaaaggcgc ccttctccta 780
   gcctcctccg acctcaggga tctgcaactt gggattcaaa gcccccgaga gggcagaacg 840
   accatccaaa acgaactgcg ccagcgagtg ctgccgccct ccccaacggc cctggaagac 900
   caccgccccg ggccccaggc gcgtcctgtc ccgtgcctac ctccgacacc acctccaaga 960
   gcgccaccgc ggagcgggcg cctcctggac gctgcggggc cactcgggct cggtaggcca 1020
   ctcgggctcg gtagacttcg ggcgggccct gagggctgag ccaggcttaa gagggcgggc 1080
   ggggccgcgg ggcatcgaca ggtcgagggt agggaattga cccggcccac gcagatcccc 1140
   gcgtcgtctt ctccccgccc accgcagcca ttggccgact ctcgactcgc cctctgtccc 1200
   ctggggtcgc tcacttaact catcagtttt ccaaaccctc cctgggaccc tagtggcgag 1260
   cactcgccgc tggccttcta gggcaggcag tattgccgct ccaggcgtgc ggagtcgggg 1320
   agaaaaaaac cgaacacgtg tgctacccag ggcccccaga taagcccctc cgcgcgcaca 1380
   cacaccctga accggagtcc agaaggccgg actcgcgtcc cggggtcgag ccccttactc 1440
   tccgctccca gttcggaaga tcccagcctg cccgaaggcg gcgtctgacc cgctggtgga 1500
   cctggtgggt gtctgttcct gatacctggg gacgaggcgg gagcgccttg agcgctggga 1560
   agcttcgcct cgcgagtccc cggcagtgac acgtgggagc gttgggcccg gcacccccga 1620
   cgcttccgag gtttcgggtg gtcgttttgc tctctcgggg gcttttattc cttaagtggc 1680
   tttaaacgac cgaggatggg cctcccgcgc cgccgcagct gtcacttcct tcgggaaagg 1740
   ctagcgacct tggacgcgca ggctctgaaa accaaggtcc agacccatac cgagcgccca 1800
   aagaccgcat ttgaattctc ctcgccggcc attgagggag agaaaggaac gcttagttcc 1860
   attcacattc acagaaagaa gcgccgaggg tgggggaaac gcagtcttgc cgggtgagcc 1920
   gggacaggtt cctcgcctgc cccccggccg ctgcttcctc ttagctgaat ggggagcgac 1980
   ccgccccggg cgcggccttc ggggctgaag actgaggtgc agcctcaccc ccggcctggc 2040
   agcggcttgg aagagagagg gaaaggagga acatctaccc ggctaagaga cgccgccaga 2100
   gtccctaaag ctggcggggg acggaggccg gggtcccgga tcttcagagc gcccgcagcc 2160
   gggcttctgg gctcctcggt gcggggaacc tgaccctgta gccttcgcca gggcgcggcg 2220
   gggctgcaag aagggaggaa ggagtgggac tagagccttc gagggcgcgg gcggcgtgca 2280
   ctcagccttg tctccccgcg cgccctgccg cctgcttgcc ccctttgctg caccgggtgg 2340
   ccctggtccc cgcgcccgcc cgcccccgac gccttcgcgt ggttccgcgc tgcccgcggc 2400
   cagccccgtt tcgcttcgca aggagcgtgt tgttttctca atcgaaatgg gatcaagatc 2460
   ccagggtttc ctcgtctcct tgttttgagg tgataggatc ttagccaata gatcgaaaac 2520
   gtgaaaagta cccgtcgcct cttgtcccaa gtgcctctgt ggaaggatca tttacttgga 2580
   aaagcagaat aaattctgtt gccgttttcc ttccggaggc gtgctcaccc cagagctcct 2640
   agacccactg gcattcgcta tacttcctcc tgggctttac aggtaggatt tcaagaatga 2700
<210> 3
   <211> 280
   <212> DNA
   <213> Homo Sapiens
<400> 3
   tagagccttc gagggcgcgg gcggcgtgca ctcagccttg tctccccgcg cgccctgccg 60
   cctgcttgcc ccctttgctg caccgggtgg ccctggtccc cgcgcccgcc cgcccccgac 120
   gccttcgcgt ggttccgcgc tgcccgcggc cagccccgtt tcgcttcgca aggagcgtgt 180
   tgttttctca atcgaaatgg gatcaagatc ccagggtttc ctcgtctcct tgttttgagg 240
   tgataggatc ttagccaata gatcgaaaac gtgaaaagta 280
<210> 4
   <211> 1968
   <212> DNA
   <213> Homo Sapiens
<400> 4
   attttccaaa acttcacaag tgctccagga atgatttggt gtcttttatg ttttcagagc 60
   ccaggcttcc tgtgttctat gcagtgtata gatttgtctt tgggttattt cctataccaa 120
   actttagtct ttctcactgg gtacaaaggg aaattctaaa attctgttcc ccatggaaaa 180
   atgtcctttg ctcctcgggg cagattctgc agggagagac ttgcaaatat gaaggaggag 240
   tggaaggtgt gatcggagaa aataccaagg actgaagtgt gaaggggtat gggatggatt 300
   ggaggcagtt ctgagatgag agcctgctga attcgtgttc cgggagatca agttggcttc 360
   ttctggaaaa aacctgtaag cttgggaaag ctgggattgg cttccatcac aagcagctgg 420
   gatctaagtt catttgcatc agggccactc cagctcagtt ctgctgggaa ccgtaccggg 480
   atgggtgtgg gacggcctcg aattcagatc cgggtgctgt tttttctagg cagtctctcg 540
   gagcctgctt cctggactac aaaatgaaga gttgtgaaga gtcaataatg tcaagttgga 600
   agggtctgac acggaatctg accaaggctg gaccctcaat aattgtgatt tcttttcccc 660
   cttttccttc ttggtaaaat catcccacga atctacgcaa gtagggccct tcgtcattct 720
   tcggagtagc cgcttgaggg ctggaaggag cagtgataga aaccccagag acgcagagac 780
   cctccgaact tcgaactcga tcactgtcct cccccgaccg ccgaacccgc tggagaagcg 840
   ggcgcgacag ggcgatgagt taacgcggag ggagcgcgga ggccgcggaa gccgggggcg 900
   ctgggtctca ggcccggatg ctgagcgcgg accggcgtgt cctccccaca gcgcccccgc 960
   gcggcctcct cccgctgcgc cccgcacggc gacccgccgc gggtagccct ggcgtttggc 1020
   cacgccgtcg gctgaggacc gctagagctg gggggagatc aaagcattcc tatggggccc 1080
   aaagagcctg ggattgcagt gttgttagcc tggcctcgcc gcgtcaataa attttcggcg 1140
   aaagttggga gcaggagtgg aggcttgggg cgggggttgg tggggcgcgg gggttggccg 1200
   gagggagggt ccgggggttt cttgcacctg cgctcctgcc gcgttaagtc agtgggaagc 1260
   aagtgacatt tccctttcac ccgccccctt ggaacttgct catctccctc ctttagaaca 1320
   ccgcctgctc tccggggtca gcattatcaa agagaagatt tcctccaggc ccttaaaatt 1380
   tacgtggcgc cttgttacaa ggagcgcgct ggaagctggc tttggtatct ccttgtcccc 1440
   tctttctcct gccccgcccc cacatccccg tggagaaacc cccgagcagg ccttttgcat 1500
   tttctcaccc cacttagaac tacaaaaaat atatgggaag gtgatttggt ttcacttcaa 1560
   cctcttcatt ttgcagaagc ctaaagtggt gatggtgaga aaagtggccc aaggtcaagg 1620
   gttacttcag ttggcaaaac caggagtaga acaggagaat ccctagagtc ttactatata 1680
   ttttttgcta cttatttcat ggacaaaaat aatcattctc ttaggcaggt caagtactct 1740
   ttaaaggtgt ctgtgctttt gccagttggc tgcaaaactt agcatcacac ttgtcacctg 1800
   gacatcatgg ggagtcgctg gctatccaac gacagagccg agtacactct gatccctcct 1860
   ctgagacact cctttctcac tcccactcca tcctgaaaaa cactatggag catgtgttct 1920
   gggcaagtct tctcccaggc gtgggtaaaa tttgagcaca gggattct 1968
<210> 5
   <211> 1368
   <212> DNA
   <213> Homo Sapiens
<400> 5
   ggaggcagtt ctgagatgag agcctgctga attcgtgttc cgggagatca agttggcttc 60
   ttctggaaaa aacctgtaag cttgggaaag ctgggattgg cttccatcac aagcagctgg 120
   gatctaagtt catttgcatc agggccactc cagctcagtt ctgctgggaa ccgtaccggg 180
   atgggtgtgg gacggcctcg aattcagatc cgggtgctgt tttttctagg cagtctctcg 240
   gagcctgctt cctggactac aaaatgaaga gttgtgaaga gtcaataatg tcaagttgga      300
   agggtctgac acggaatctg accaaggctg gaccctcaat aattgtgatt tcttttcccc      360
   cttttccttc ttggtaaaat catcccacga atctacgcaa gtagggccct tcgtcattct      420
   tcggagtagc cgcttgaggg ctggaaggag cagtgataga aaccccagag acgcagagac      480
   cctccgaact tcgaactcga tcactgtcct cccccgaccg ccgaacccgc tggagaagcg      540
   ggcgcgacag ggcgatgagt taacgcggag ggagcgcgga ggccgcggaa gccgggggcg      600
   ctgggtctca ggcccggatg ctgagcgcgg accggcgtgt cctccccaca gcgcccccgc      660
   gcggcctcct cccgctgcgc cccgcacggc gacccgccgc gggtagccct ggcgtttggc      720
   cacgccgtcg gctgaggacc gctagagctg gggggagatc aaagcattcc tatggggccc      780
   aaagagcctg ggattgcagt gttgttagcc tggcctcgcc gcgtcaataa attttcggcg      840
   aaagttggga gcaggagtgg aggcttgggg cgggggttgg tggggcgcgg gggttggccg      900
   gagggagggt ccgggggttt cttgcacctg cgctcctgcc gcgttaagtc agtgggaagc      960
   aagtgacatt tccctttcac ccgccccctt ggaacttgct catctccctc ctttagaaca     1020
   ccgcctgctc tccggggtca gcattatcaa agagaagatt tcctccaggc ccttaaaatt     1080
   tacgtggcgc cttgttacaa ggagcgcgct ggaagctggc tttggtatct ccttgtcccc     1140
   tctttctcct gccccgcccc cacatccccg tggagaaacc cccgagcagg ccttttgcat     1200
   tttctcaccc cacttagaac tacaaaaaat atatgggaag gtgatttggt ttcacttcaa     1260
   cctcttcatt ttgcagaagc ctaaagtggt gatggtgaga aaagtggccc aaggtcaagg     1320
   gttacttcag ttggcaaaac caggagtaga acaggagaat ccctagag                  1368
<210> 6
   <211> 336
   <212> DNA
   <213> Homo Sapiens
<400> 6
   taggcagtct ctcggagcct gcttcctgga ctacaaaatg aagagttgtg aagagtcaat       60
   aatgtcaagt tggaagggtc tgacacggaa tctgaccaag gctggaccct caataattgt      120
   gatttctttt cccccttttc cttcttggta aaatcatccc acgaatctac gcaagtaggg      180
   cccttcgtca ttcttcggag tagccgcttg agggctggaa ggagcagtga tagaaacccc      240
   agagacgcag agaccctccg aacttcgaac tcgatcactg tcctcccccg accgccgaac      300
   ccgctggaga agcgggcgcg acagggcgat gagtta                                336
<210> 7
   <211> 1900
   <212> DNA
   <213> Homo Sapiens
<400> 7
   aaaaacctac ttgctgaact gagtggttct ggtctcagat ggttaggtag cttaaaaaca      60
   tgaagttgaa gatggtagtc atttaaaaac tgttttactg tcatttccat gacgataaga     120
   aagtaagtag aggcaagggt gttaaaccca gaaacagtac acaggaatga acaattcttg     180
   gggatcttca ttggcagact tcaaaaggca aattttttag cttgatgtga accaccttct     240
   gtgactcttt tcccctaatt tattttgata tctctatatc tttatgggga aaagctgccc     300
   caccatacag ccaatcggtg gactaggtaa tctatgggag tgttgaaagt cgaaatccca     360
   ggttgggtct gtggtaaaat ttctaacttc acctccagtc aacagaatgc cacaatgagg     420
   gaaaatattc agtaagttga aaatacagaa caaggaagcg gaaagaaatt ttagtgacaa     480
   tgccactaag tggaaagaag gaaagtggct cactccctag gaaaacaaac actgatcttt     540
   ccacagctac atctcaagcg cattggccaa ggcatgcttt gccctgaaaa atcaccagag     600
   gccatagcgt ggcccctttc ggccacgtac agcgagttcg ctattacagt gctttcagag     660
   ggtacacgat cttactccct cacccaattt cttatccgtg tcctctctct gggcccctgt     720
   ggcatctata cagcagcctc tgtcctgctg ccccctggat cctcagttct cattacattt     780
   ggcggcctcc aggaagcttg aacaaacctc gacgcgatca aaggagagga aagcctgctt     840
   gtcactttgg gtggatcaga gacaatattt ccaggggaca gggcgttaca tggcagttca     900
   aggcgcagtg acacccgagt ctgatacaac caacactagc ttcgtcaggt ggcccttcga     960
   tgctcatttc cctaccaaac aagattcata tccgcttaat aagaccctgg gataattcca    1020
   actctcgctc cccagcaagg cgcgcggctg gccagccggg gcgggaagct ggctctgcgt    1080
   taaggactcg cagccttagc agcagtagac caggcagaag tgtctcctca cttttacccc    1140
   aagccctccc agaaactgcc gtcagagcag acacaaggtt ttggttgggg cattattttt    1200
   tgggtgggtg gggaggggag aagagacaat taaaagttag ttgaactgag aaaggaacct    1260
   ggtgaacttc cctatcctgc ccaacagcca aagacccccc cacccccaac ttagttctcc    1320
   cgactcccaa accagtcacc acggaagccc accgctgccc ctcaacctaa cgcggaaaat    1380
   cctaccgctg gggatctcaa ataggtgccc agcaagctgt gtgccggatt ggggcggccg    1440
   gttccggcaa tacctggtaa cctctgggtg tctttaggcg tgtggctgcc ggcccatctt    1500
   ccgagggcgc tcggaggcga agcatccaag cgggtcctcg tatccagcgg gcgggactct    1560
   ggcgggctcc gcgcgaagat ccgggctctg gcccgagcga atggcgctca aaccccggcc    1620
   tggggagaat gacggcctga attgcggcgg ggcgtcccct ccaccgcaga aagtgtgggt    1680
   acgcagaaac ccgcggcagg agagccccaa gtggagcgca gtctgacagc gtctgtctct    1740
   gaactcgtgt cggaccgagg ggacgccacc cagcgggcag ggtgacgtca ccgtctccct    1800
   gacactccac attaaagggc tggcatgtta cagggaggga gggggagagc gaaagccagc    1860
   gcgagcaaca gagagatcct ttctcagcag gcacgaaagc                          1900
<210> 8
   <211> 1002
   <212> DNA
   <213> Homo Sapiens
<400> 8
   ccaccataca gccaatcggt ggactaggta atctatggga gtgttgaaag tcgaaatccc      60
   aggttgggtc tgtggtaaaa tttctaactt cacctccagt caacagaatg ccacaatgag     120
   ggaaaatatt cagtaagttg aaaatacaga acaaggaagc ggaaagaaat tttagtgaca     180
   atgccactaa gtggaaagaa ggaaagtggc tcactcccta ggaaaacaaa cactgatctt     240
   tccacagcta catctcaagc gcattggcca aggcatgctt tgccctgaaa aatcaccaga     300
   ggccatagcg tggccccttt cggccacgta cagcgagttc gctattacag tgctttcaga     360
   gggtacacga tcttactccc tcacccaatt tcttatccgt gtcctctctc tgggcccctg     420
   tggcatctat acagcagcct ctgtcctgct gccccctgga tcctcagttc tcattacatt     480
   tggcggcctc caggaagctt gaacaaacct cgacgcgatc aaaggagagg aaagcctgct     540
   tgtcactttg ggtggatcag agacaatatt tccaggggac agggcgttac atggcagttc     600
   aaggcgcagt gacacccgag tctgatacaa ccaacactag cttcgtcagg tggcccttcg     660
   atgctcattt ccctaccaaa caagattcat atccgcttaa taagaccctg ggataattcc     720
   aactctcgct ccccagcaag gcgcgcggct ggccagccgg ggcgggaagc tggctctgcg     780
   ttaaggactc gcagccttag cagcagtaga ccaggcagaa gtgtctcctc acttttaccc     840
   caagccctcc cagaaactgc cgtcagagca gacacaaggt tttggttggg gcattatttt     900
   ttgggtgggt ggggagggga gaagagacaa ttaaaagtta gttgaactga gaaaggaacc     960
   tggtgaactt ccctatcctg cccaacagcc aaagaccccc cc                       1002
<210> 9
   <211> 276
   <212> DNA
   <213> Homo Sapiens
<400> 9
   tacacgatct tactccctca cccaatttct tatccgtgtc ctctctctgg gcccctgtgg      60
   catctataca gcagcctctg tcctgctgcc ccctggatcc tcagttctca ttacatttgg     120
   cggcctccag gaagcttgaa caaacctcga cgcgatcaaa ggagaggaaa gcctgcttgt     180
   cactttgggt ggatcagaga caatatttcc aggggacagg gcgttacatg gcagttcaag     240
   gcgcagtgac acccgagtct gatacaacca acacta                               276
<210> 10
   <211> 3100
   <212> DNA
   <213> Homo Sapiens
<400> 10
   cgcctttttc agaggagaaa agacctggcc cacgcctccc agaggcttta cctggtgaag      60
   aggaggagca gccactgcag cgcggtggac agggtgtcct ggctggcgcc gaagatgtca     120
   gtgatagtgg ccggtacgtt ctccaaatcc agccgcgcgc caccaccgtg cgagtccccg     180
   gccgccttct tttccgcaga gaggataaag gcgtccatca tgtcgcgggg ggcggccccg     240
   ggccgaaggc tttcgcagtg cctcaagaac ttgtccagga tgaagttgct gaagttgcgg     300
   ttgagctgct cgaattcgcg gaaaacggtg cgcaccgggt tggggaagta ctgcagccag     360
   ggcatcacgt ccaccaggct gcccgcgccc accgtgcgcc cgaactcttc gttgtggctg     420
   agcagctcac ggaactcggg gtcgtcgtgg ctgtagcggc agccgaaaca cacggcactc     480
   atgacgttgg ccacggccac gacggtcagc ggcctcgggt cgaggaaggc gccgtccgcg     540
   ctgccgcgca ccagcagcgc caccagctcg cgcgcctcgc tcagcacgtg gccctcgagg     600
   acttggcggc tgcgcggctg gcgcgtgaag aagttgcgca tcatgctgtg ggctgcgcgc     660
   cgctgcacct tccagtgctc cgagtagtgg ccgaaagcca tgctgcggcc gccggacacc    720
   acacggaagg aggcgaaggc cggccggtcg gcgaaggccg agccctgctg caccagggcc    780
   tggtggatgg cgcgctcgcc attcagcacc actatggggc agctgcccag gcggatctgg    840
   aaaacgtcgc cgtagcgccg cgccaggcga gcgaacgaga ggtgagccgc ctggcccacc    900
   gccgccgcgt ttccgatcag tggccacgca aacgggcccg ggggcgcgga ccggagctgc    960
   cgcctccgtt gcctcagcag ccgctggccc acatgcacag tggccagcac cgacaggagt   1020
   agcaggagcg tggtctgctg gatggacagc gggtttagcg gccaagggtc gttcgggctg   1080
   aggctggtgc ccatgctggg gacagagagg agaaggcgtg acactcaggg gtgcagagac   1140
   aggagcgggc gccccacgcc cctaccccag cctctgggga ctgagtgccg ttgggtggag   1200
   aggtcggagc tgactctctg gagaaatggc cgaagacgcc ccttcccatc cccaacccac   1260
   tctcccttgg agaagagaag gggcgtgttt ccacctcgct gtaacccagc gccaaaccct   1320
   tcccctcccc gcaaggcgcg taacggttcc tgcaatctgg ggacaacgct gcgggcatcg   1380
   aggcggtggc gcttgatttc ctttaaagta cctaccacgc cacccgctac ctgtaataat   1440
   ccatctgaag aggtcgccgg gcagcgcctc ggcagacaga ctgacctgcg gggaggtgcg   1500
   gtttccagtg gcgcgggaca gccggctccg agaaggaact gggacctttg cctagggcaa   1560
   gacgtcaaca ggaacccgca gcccggcctg gacacctgct gccctcactg gaagctttaa   1620
   ctcccactcg agtctcttgg cgtcgtcagt gccaggagcg cttggattgg gatggggacg   1680
   gagaagggtg cctcgctcgc cgagccccgc tgcccttggg gacctggcaa agtcgaggtt   1740
   tcctcacagc gttgagattg agactggggg tcggtgagtg gcgtcaattc ccatgccctt   1800
   gcggctctca caactggagt cgcagaagcg ctcgctccca ctcccactcc agagtcaaag   1860
   cgcgccatcc cgctcctccg ggttttaagg actgggtaga aggagggcag cgcgacctgg   1920
   cagggcgggg cctgcggggg gcggggcgcg ccgcacacca ggccgctttg acccggactc   1980
   ccgtccaggc gcaccggccg gcctcggagc tctaccagca ggctttcatg ggagcggagg   2040
   tgcccacgtt tccattgtgc ggtaaccgcg cttcatcaca gccacctcca atcgaggccg   2100
   cacggtgtcc ccagaaccac gctcggtaca acctgccccg gccgctgcgc tcgacttggg   2160
   agcgcgcccc gccccgagcg cacgacctcc cttccctctc cgcgacttga gcttgcttcc   2220
   cggaaagcca gtgtagtggc agcggtttgc ctcacagcgc ccctggttgg ttgtttaaat   2280
   tttatcttag gttctcgcaa acaaattgcc acctcagtgg aggctctttg gcatgctaaa   2340
   ttttttccag ttcttgcggc gagatcagga atccctaaaa gtagccgctc ccccgccccg   2400
   gggccccgac agtgcttggg cacacaaagg ctcctggggc gcagctggcc ctgagatttc   2460
   ccgcgtagag gccactccag gtggcgccgc ccgctggtgg gcgcggggcc gcccaggctg   2520
   cgatggaagc cgttggcgca gggcggggag ccaagctggg gcgactgggg gggcctgggg   2580
   gagctagcgg ggcgggaccc gagcggggcg gagagtggca ggaggaggcg aatctccgcg   2640
   ctccggcgaa ctttatcggg ttgaagtttc tgctgtcgcc tcccctttgc gtgcggagct   2700
   gggctttgcg tgcgccgctt ctggaaagtc ggctccagtc atatccctgg gcgctgcctg   2760
   cggccgctcc tcccgcgctt ctcacggcac ctgacacgcg gaggcggcgg ccgagggtgg   2820
   ggtgccggcc accaccaccc ttggcgtggg gtgccggccg ccaccaccct cggctgcgca   2880
   cgcacagtcg cgcctgccag gccagcgagg cggacgcttg ggccaccacc cgcaggccga   2940
   gcccgggcgc gtatcgccac ctcgctgcgc accggcggct ctgtggtctt cctgggcgcg   3000
   cttgcggtcg cgaagggggc cctcccttta taagggaagc cggaggcgct tgggcggggg   3060
   tggcttcccc cgcctggcgc gccctttcct acatgctgat                         3100
<210> 11
   <211> 2500
   <212> DNA
   <213> Homo Sapiens
<400> 11
   ttgagctgct cgaattcgcg gaaaacggtg cgcaccgggt tggggaagta ctgcagccag 60
   ggcatcacgt ccaccaggct gcccgcgccc accgtgcgcc cgaactcttc gttgtggctg 120
   agcagctcac ggaactcggg gtcgtcgtgg ctgtagcggc agccgaaaca cacggcactc 180
   atgacgttgg ccacggccac gacggtcagc ggcctcgggt cgaggaaggc gccgtccgcg 240
   ctgccgcgca ccagcagcgc caccagctcg cgcgcctcgc tcagcacgtg gccctcgagg 300
   acttggcggc tgcgcggctg gcgcgtgaag aagttgcgca tcatgctgtg ggctgcgcgc 360
   cgctgcacct tccagtgctc cgagtagtgg ccgaaagcca tgctgcggcc gccggacacc 420
   acacggaagg aggcgaaggc cggccggtcg gcgaaggccg agccctgctg caccagggcc 480
   tggtggatgg cgcgctcgcc attcagcacc actatggggc agctgcccag gcggatctgg 540
   aaaacgtcgc cgtagcgccg cgccaggcga gcgaacgaga ggtgagccgc ctggcccacc 600
   gccgccgcgt ttccgatcag tggccacgca aacgggcccg ggggcgcgga ccggagctgc 660
   cgcctccgtt gcctcagcag ccgctggccc acatgcacag tggccagcac cgacaggagt 720
   agcaggagcg tggtctgctg gatggacagc gggtttagcg gccaagggtc gttcgggctg 780
   aggctggtgc ccatgctggg gacagagagg agaaggcgtg acactcaggg gtgcagagac 840
   aggagcgggc gccccacgcc cctaccccag cctctgggga ctgagtgccg ttgggtggag 900
   aggtcggagc tgactctctg gagaaatggc cgaagacgcc ccttcccatc cccaacccac 960
   tctcccttgg agaagagaag ggcgtgtttt ccacctcgct gtaacccagc gccaaaccct 1020
   tcccctcccc gcaaggcgcg taacggttcc tgcaatctgg ggacaacgct gcgggcatcg 1080
   aggcggtggc gcttgatttc ctttaaagta cctaccacgc cacccgctac ctgtaataat 1140
   ccatctgaag aggtcgccgg gcagcgcctc ggcagacaga ctgacctgcg gggaggtgcg 1200
   gtttccagtg gcgcgggaca gccggctccg agaaggaact gggacctttg cctagggcaa 1260
   gacgtcaaca ggaacccgca gcccggcctg gacacctgct gccctcactg gaagctttaa 1320
   ctcccactcg agtctcttgg cgtcgtcagt gccaggagcg cttggattgg gatggggacg 1380
   gagaagggtg cctcgctcgc cgagccccgc tgcccttggg gacctggcaa agtcgaggtt 1440
   tcctcacagc gttgagattg agactggggg tcggtgagtg gcgtcaattc ccatgccctt 1500
   gcggctctca caactggagt cgcagaagcg ctcgctccca ctcccactcc agagtcaaag 1560
   cgcgccatcc cgctcctccg ggttttaagg actgggtaga aggagggcag cgcgacctgg 1620
   cagggcgggg cctgcggggg gcggggcgcg ccgcacacca ggccgctttg acccggactc 1680
   ccgtccaggc gcaccggccg gcctcggagc tctaccagca ggctttcatg ggagcggagg 1740
   tgcccacgtt tccattgtgc ggtaaccgcg cttcatcaca gccacctcca atcgaggccg 1800
   cacggtgtcc ccagaaccac gctcggtaca acctgccccg gccgctgcgc tcgacttggg 1860
   agcgcgcccc gccccgagcg cacgacctcc cttccctctc cgcgacttga gcttgcttcc 1920
   cggaaagcca gtgtagtggc agcggtttgc ctcacagcgc ccctggttgg ttgtttaaat 1980
   tttatcttag gttctcgcaa acaaattgcc acctcagtgg aggctctttg gcatgctaaa 2040
   ttttttccag ttcttgcggc gagatcagga atccctaaaa gtagccgctc ccccgccccg 2100
   gggccccgac agtgcttggg cacacaaagg ctcctggggc gcagctggcc ctgagatttc 2160
   ccgcgtagag gccactccag gtggcgccgc ccgctggtgg gcgcggggcc gcccaggctg 2220
   cgatggaagc cgttggcgca gggcggggag ccaagctggg gcgactgggg gggcctgggg 2280
   gagctagcgg ggcgggaccc gagcggggcg gagagtggca ggaggaggcg aatctccgcg 2340
   ctccggcgaa ctttatcggg ttgaagtttc tgctgtcgcc tcccctttgc gtgcggagct 2400
   gggctttgcg tgcgccgctt ctggaaagtc ggctccagtc atatccctgg gcgctgcctg 2460
   cggccgctcc tcccgcgctt ctcacggcac ctgacacgcg 2500
<210> 12
   <211> 216
   <212> DNA
   <213> Homo Sapiens
<400> 12
   tacgttctcc aaatccagcc gcgcgccacc accgtgcgag tccccggccg ccttcttttc 60
   cgcagagagg ataaaggcgt ccatcatgtc gcggggggcg gccccgggcc gaaggctttc 120
   gcagtgcctc aagaacttgt ccaggatgaa gttgctgaag ttgcggttga gctgctcgaa 180
   ttcgcggaaa acggtgcgca ccgggttggg gaagta 216
<210> 13
   <211> 9100
   <212> DNA
   <213> Homo Sapiens
<400> 13
   attcacaacc tcagaagttt ttttaagagg aaaaaaaagg tgtcacgtgt tcgctgccca      60
   acagactcaa acatcctatt actcctatta ccgttatcat catctttact gaatattcct     120
   actacaagac aggcagacat gtgacatgcg gtgaaggttc tactttaccc accaccaatc     180
   tccacaagca ctctcatctc agggtgcctc ggggagggca gaggaggaat acttgggctc     240
   aaaagtcgtc tttggaccac tttcagatca gtctggtgga aacggtagtg tgagcgctat     300
   gctacttgag actgcgtttg aaaatctctt tcacgttccc aaatcaaagc cactttgagg     360
   tttaagaatg ataaccacag gtgaatgcct tactctttcc acgagccagg cctttccttt     420
   gcatgacgca gaccggcggc tgagacccgt cctgaggccc cgcctttcat tcggtttatg     480
   tggccccgcg cagttcacgc agttttctcc gttcttaggc aaccagctcg tgggaaattc     540
   actccagaaa agcgtgcgcc atatcattat tttgcgattc aacaaacttt ttcaactgtt     600
   gttcaggaac tagattccag atagatcttg ttgtgttcgg ccttcctaga aattcctttt     660
   ccagaggaag aagatccggg ttgggaagag tgcgtgacta tggccccggg ctcattgaaa     720
   gactcctccc cacaaaagtt tagggctgat actaaacgaa gctatggagc catgcccaca     780
   caaatactcc ccctttaccc gaattgtggg gcctggactg tgaaggcctt cgctgcaaga     840
   gcgggcactg gcgaacttca gtgcacgccg cggccgagaa cggatgcagg gcgggggatg     900
   gctgagccgc ctgatccttg cagagaacct gcaggggccc tcggagggta tcccctgcgt     960
   ccaagggagc gcccctcctt ttcagcactc gggggaactg agggcgacgt gccagccccg    1020
   cactcaactt tccccttccc tgcaggcaca gagttggccg gcgggggcag aggaggagct    1080
   gggtctccac tgcgcccgtt taaacctggc caggggctgc gtttcctccc cccaccccac    1140
   gacgatcctt tcttagtctt cgcttttcaa cccaatcgtt aatcattcgg aacgcgcggg    1200
   cggggagcgg cgaggagggc gagctcgggg ttcgccgccg ccgccgccgc cgcgcgcgcg    1260
   cgctcaggaa gcggtgtggc tgtcaccccc tcccgggcct cctcccccct ccttcctgct   1320
   ttgctccccc tccttcctcc cctcctcccc gctccgccgc ccgcgcccag tgtatctact    1380
   ccctccccac gtcactcgcc agcgcgccat gcaaatcacc gccgccgccg gctcccattg    1440
   gccgcggcgc gctcatttaa tggcagcccg ggcccggcgt atggctgctg ggccccgcgc    1500
   gccgccggcc ccgcgtgcgc ctccgctccg agcgcacggc cccgggcagg cagcgggcag    1560
   cccatcccgg gctcggcggc cccggctctc cggccctctc cgcgagcccg cgctcctccc    1620
   gctgtccccg ggcccctccc tggctgcacc gtaatcgccc cctgcaggcc cccctgcgcc    1680
   tccccccccc cgccactggc gcctggcttc ccccgggcac ctgggaccag cacatgccca    1740
   gcgcacgcgg cgcgccgccc tgctagaagt tgcagcctcc gagttggagg ccgctgagga    1800
   ccgagcgcag gaggaaggag acagcgcgca gcggcggccg gcgaggagac agcacacccc    1860
   gggccgggcc cagcgcaccg ctcccggccc caaaagcgga gctgcaactt ggccacgact    1920
   gcacctgttt gcaccgctcc gccgagggcg cctgggctgc ggtggcggcg aagacggcga    1980
   ccccgaccgt cggcctcttt ggcaagtggt ttgtgcatca ggagaaactt tccacctgcg    2040
   agccgaaccg gcgccgagtg cgtgtgtttc tgcctttttt tgttgtcgtt gcctccaccc    2100
   ctccccattc ttctctccgc taggaccccc ccgcccccgt ctcactccgt ctgaattcct   2160
   ctccgtctcc ctcccacccc ggccgtctat gctccaggcc ctctcctcgc ggtgccggtg    2220
   aacccgccag ccgccccgat gtacagcatg atgatggaga ccgacctgca ctcgcccggc    2280
   ggcgcccagg cccccacgaa cctctcgggc cccgccgggg cgggcggcgg cgggggcgga    2340
   ggcgggggcg gcggcggcgg cgggggcgcc aaggccaacc aggaccgggt caaacggccc    2400
   atgaacgcct tcatggtgtg gtcccgcggg cagcggcgca agatggccca ggagaacccc    2460
   aagatgcaca actcggagat cagcaagcgc ctgggggccg agtggaaggt catgtccgag    2520
   gccgagaagc ggccgttcat cgacgaggcc aagcggctgc gcgcgctgca catgaaggag    2580
   cacccggatt acaagtaccg gccgcgccgc aagaccaaga cgctgctcaa gaaggacaag    2640
   tactcgctgg ccggcgggct cctggcggcc ggcgcgggtg gcggcggcgc ggctgtggcc    2700
   atgggcgtgg gcgtgggcgt gggcgcggcg gccgtgggcc agcgcctgga gagcccaggc    2760
   ggcgcggcgg gcggcggcta cgcgcacgtc aacggctggg ccaacggcgc ctaccccggc    2820
   tcggtggcgg cggcggcggc ggccgcggcc atgatgcagg aggcgcagct ggcctacggg    2880
   cagcacccgg gcgcgggcgg cgcgcacccg cacgcgcacc ccgcgcaccc gcacccgcac    2940
   cacccgcacg cgcacccgca caacccgcag cccatgcacc gctacgacat gggcgcgctg    3000
   cagtacagcc ccatctccaa ctcgcagggc tacatgagcg cgtcgccctc gggctacggc    3060
   ggcctcccct acggcgccgc ggccgccgcc gccgccgctg cgggcggcgc gcaccagaac    3120
   tcggccgtgg cggcggcggc ggcggcggcg gccgcgtcgt cgggcgccct gggcgcgctg    3180
   ggctctctgg tgaagtcgga gcccagcggc agcccgcccg ccccagcgca ctcgcgggcg    3240
   ccgtgccccg gggacctgcg cgagatgatc agcatgtact tgcccgccgg cgaggggggc     3300
   gacccggcgg cggcagcagc ggccgcggcg cagagccggc tgcactcgct gccgcagcac    3360
   taccagggcg cgggcgcggg cgtgaacggc acggtgcccc tgacgcacat ctagcgcctt    3420
   cgggacgccg gggactctgc ggcggcgacc cacgagctcg cggcccgcgc ccggctcccg    3480
   ccccgccccg gcgcggcgtg gcttttgtac agacgttccc acattcttgt caaaaggaaa    3540
   atactggaga cgaacgccgg gtgacgcgtg tcccccactc accttccccg gagaccctgg   3600
   cgaccgccgg gcgctgacac cagacttggg ttttagactg aacttcggtg ttttcttgag    3660
   actttttgta cagtatttat cacctacgga ggaagcggaa agcgttttct ttgctcgagg    3720
   ggacaaaaaa gtcaaaacga ggcgagaggc gaagcccact tttgtatacc ggccggcgcg    3780
   ctcactttcc tccgcgttgc ttccggacgg cgccgaccgc cggagcccaa gtgacgcgga    3840
   gctcgtcgca tttgttataa atgtagtaag gcaggtccaa gcacttacaa gttttttgta   3900
   gttgttaccg ctcttttggg ttggtttgtt aatttataca aagagattac caccaccacc    3960
   ccctccttca gacggcggag ttatattctg ggttttgtaa aactttatgt atctgagcat    4020
   ttccattttt ttttttgggt tttgtattat ttcttgtaaa tgcattgtga aaaattttat   4080
   tttcggcgtt gcaatgcggg gaggagaagt cagattatgt acatagtttt ctaaaaagcc    4140
   tttcttctaa aaacgaaaaa agacccccca cccaaaatgt ttcgagtcaa caaatttaag   4200
   agacagagcc cattttctcc ataaatttgt aacatgctat ttttatgtgc atgttttatg    4260
   agttcaaaat gcaatgagga aatctgacag ggaaattatc tgtatgaact aaaagtaagg    4320
   gaaccccggg gaatgggagg acaggatttt tcaaggaacc tttttcaatg aaagagaagg    4380
   aagttaaaac ctataggtta ttttgtagag ctgagtgtta atacgggccg agaaataaaa    4440
   gtatcttctg ctccggctgt ttcactgcgg acggctgggg ctgctgcgcg ttaccttgct    4500
   gcaagcgggg cgccttccac ctggctgggg gtctgcgcca cagtttggtc cagaggaggg    4560
   aggaggaagg gaagacccca gtggtgggac cctggaccag gccatggatg aaggacaaag    4620
   accagggcag gtcacgggtt tcccaattcc ccagcaatta agatttcgag cagaatttat    4680
   ctaaatgtgt ttcaaggaaa cacaatcgct gaaccaaaac gtactgcagc cgagccccct   4740
   ccgtccatcc tctgcccctc cccctggctt ctttctcttg ggaaaacggg caaaataatt    4800
   gtgctggatt ctcacacaca cagaaatatc gaccatcacc ctcccccgcg tgaactggga    4860
   tgcaagttgc taaccgatgt gaacgcaaaa tgccttgttc attattcctg acgagatctt    4920
   gaggttgttt gatgctttaa attttttaat tatattattt tctaggtgtt tattggtaca     4980
   ttgcagtttt ttttttgaaa tttaaaaatt tctgtaaaac tttgtcttca agtaatctga     5040
   cagcattaaa tattgcattt aaaaattata ctgtagcaaa tacatttaaa aattaatcac 5100
   aacgttaaga tgaaattata tttttggaaa aaaaaaacac ttgaagccca gatggaaata 5160
   cgtttatttc agcagcctta ggtttcccct cgctttctca acacccttcc ttgtcctgga 5220
   gtatggactg tccgtccaaa agtgagccta tgctataagt ttaatgagaa ccgaattcag 5280
   cctgcattcg agaatagctt taagtataat gctgatctga caattgacgt gtaatttggg 5340
   aagtcatttt gataattttg cttaaaccac tcattcgtta aagtgattac aaaaaagttc 5400
   aagaatgatg tccactgctt tctaacaaga taataaaccc cccccctctt ttctttttct 5460
   ttatttttat ttcttttagc tatttgatcc tttctgaagc agttgtttct ggaagagtct 5520
   gtgcgcccat ggatggctga gcaccactac gacttagtcc gggataaggg cctccccagt 5580
   cctctccggg agatgatttg ggaaatttta taatgcttgt tctgttaact caccgggacc 5640
   ttgagggtcc aatgggacct tgagggtttt ctctgaaata tacaaactta aaggactctc 5700
   tctgaggttc tttgactgac gtccactctc agtctggccc ctgtgctccc ctgtgtgtac 5760
   cctggagttt ctgtgtccaa ttgttggcat ctaggtcttg gctcaagatt aggatgtggg 5820
   ccccacttta gaggcacaga ctatgaaaag ctgagttagt gcgcccggga cgccaggcaa 5880
   gcagctttta cagtttggca tcttattgca ggtgcttcgt gcacagtcag ctgaaatagc 5940
   caatgccagg tgctccaacc accttatttc cttgttttgt tgattagaac aacacagaaa 6000
   aaagcaaata taaattttta atgactccat ttaaaaatat cacagggtgg gggcaaggaa 6060
   attagctgag attcatctca ggattgagat tctatccccc cttccccgcc cccagcagtg 6120
   tcgctccaat tcaaattagt ggagaaaaga ttacagtagg ccctgagccg actgtgaatt 6180
   cggtgcttgg ccaaggtaac actcatcgta ttcacggagt gaaatactat atgatgatag 6240
   ttattatatt atatgacgac ttcattcact tcccaaatca cagggtggtg cttctgtcgc 6300
   ttagtccaat tatgtttgca acccgtgtag ggaaggcgga taggagtctt tgaggcctag 6360
   tgcaaagttt gcatcagttt ctgcgggggc gacagtgtga cttcgtaaat ctgccattgc 6420
   gctcgggccc tgccctccaa actgagggta ggagggttta cttgagggtt tttttgcttt 6480
   gttttgttct gtttttgcct tgttttgttt ttcaactcac ccgccccttt tgttgttgag 6540
   attttgacgt ccccttcccc ggagaaaccc acccgtaacg tactagatgg attagtttta 6600
   aagaagactc gggcacagcg tgatctccag atgcgcgtcg ggagcgtgac tgataggtct 6660
   attaaaatca cgcgtgtggg agaacggcac tcatgctccc gcgtttcatt cccccggcaa 6720
   agactgggcg ccgcagattt ctgtctccgg aaaattccag ttaattcaag aaacccatcc 6780
   ccggacgtgt gcccgccccc ccccgaaggc tcgcttcagg ccagcccgga ctccagcgcc 6840
   ccctcgaccc cgtcgggctc cggccgactc ccgccccctc cccacccacg ctcggcctgg 6900
   gcccgggggc agcggctcca gaggcgtcgg cggcagaatt taacccccgg tctgagtcct 6960
   tggaaacttc aagtgaaaag cagacttggc cttggcgcgg ctctctttcc agcgtgacta 7020
   tctggcgaga ctgaaaggcc aggccagatg gggaaaagtg agcggggtgc gggcccccag 7080
   tatccggcgg ggtggggcgg cgtcaggacg gagcgagggc cggcgggggg gctcgcgtgg 7140
   aggcccaggc cgggaggtgg ggacgcaggg acccctcacc cagtcctctg ctcccgcccg 7200
   agccaggcct cgggatcacc tccccttgcc tgagggcgag ctctcctcct ccctcttcct 7260
   ccccgcctcc cccgttcttc cctccctctc tccctcctcc ctccccggcc cttcctctcc 7320
   agctgcgcgc ccgccgggac cacgctcctc ccgcccccgg gagccgagcg gcgtctgcgg 7380
   ctggtccgga cgtgtcccct ccgggcgggg gtggccgggc ctcctcgggg cctcgggcct 7440
   cccgggcgca cccccctgcc tgcgcctctc caagtcgccc tttctccggc tctgcccgcg 7500
   gctgagcccc gcacaccgcg ccgctgtcct tggggtcccg gcacacggcg ttccctcccc 7560
   gcccccgcca tccactccca ccgcctctcc agtccctgtg ccgtctccac cgccctctga 7620
   ctcccgcacc gagctgcgtc tccgccgccg gcctctctca gcgcggcccg gcctcctctt 7680
   tgtctttgaa gctcccccgc cgaggcccgc gcgcggctcc cctctccgtc cctctccggc 7740
   ccttgggcgc ccagtcggag gtcggggcgg gatggggcac gagtctcggg gcggcccgcg 7800
   ctccccctac tagtcccctc attgcctcgg ccccgtcccc gcagcctcgg ggcgggaggg 7860
   cgcaggggtc cccgggcgcc ctgggagccc atctccctca ggtccgcgcc ggtgtcagcc 7920
   gccggcgggg ggcgggtgga gattaagatg agtgctgcga gggacggggc cgagggtttt 7980
   gtgtagaagg agagcaggat tagtggagag aaagtgctga cccggggtca gccgcaagtc 8040
   aaaccctttt acactcagcc ggcggaacga gcacggctcg ggccggacgg caacaagtgg 8100
   gcgcgaaggg aggggagggg gaggccgggt accggccggg tccggcgtga gagaggggag 8160
   ggggagggga cgcggccaca gcgccccagg gcctcagcgt catcggggac gggcgccgag 8220
   gggagcgcgc accagcgacc cgggcgcgga ctggggcctg cagggcgtga ctggctgccc 8280
   cccaggactc catctccttc ataaacacct gccttttcca ttccccgggg ccgcttcggc 8340
   tgctcctctg gagggaagac tggggagaag gttgcctgcg ctctctgccc cagaggctct 8400
   gttttagaaa ctgtttctta atttccataa gaaacaaaaa aatcacagga aggcctggtt 8460
   cctctcccgg acgactgcgt ccccaggctc tccaggaatc ttcgccttat ccgagcttct 8520
   tcgcagccct ggatctattt ctggggggcg tgtgtgacgc agaatcgggg tataaaatat 8580
   caatgctgtt tgtcacgaca ccgctttctc cacccgggca gagctgcggc tcccgggcgt 8640
   tgcgcgtggc gtttttcatc acggagggaa agccgcggct ccgcgggaaa ggagtgaaaa 8700
   ccgagtgtgg gtgaagagga gagggaggtc agggggcaag ggaagaaagc agataaatgc 8760
   atcttcccta gcagccaagc tggaaatttg tgctgaaagt acaacagcaa gagttttcac 8820
   atgaaaaaca cgtgaagaac ccgagcaagt gatagatcca aagacggaaa atgatcgtct 8880
   attttgatag taaaaataaa aagaacagaa ttgacatatt ttcgtctaaa ttacttttca 8940
   tctgccgata tgaaacacca acactcaaac aaaaacaaag gacaaagcag tttttaaaaa     9000
   caacacaaaa caagcaactc tgagtgaatg acctgtccac agcttaccct gggtgacatg     9060
   ggggcgctgt gatggaaaat gggttctggg ttcaaaatca                           9100
<210> 14
   <211> 8500
   <212> DNA
   <213> Homo Sapiens
<400> 14
   gctacttgag actgcgtttg aaaatctctt tcacgttccc aaatcaaagc cactttgagg 60
   tttaagaatg ataaccacag gtgaatgcct tactctttcc acgagccagg cctttccttt 120
   gcatgacgca gaccggcggc tgagacccgt cctgaggccc cgcctttcat tcggtttatg 180
   tggccccgcg cagttcacgc agttttctcc gttcttaggc aaccagctcg tgggaaattc 240
   actccagaaa agcgtgcgcc atatcattat tttgcgattc aacaaacttt ttcaactgtt 300
   gttcaggaac tagattccag atagatcttg ttgtgttcgg ccttcctaga aattcctttt 360
   ccagaggaag aagatccggg ttgggaagag tgcgtgacta tggccccggg ctcattgaaa 420
   gactcctccc cacaaaagtt tagggctgat actaaacgaa gctatggagc catgcccaca 480
   caaatactcc ccctttaccc gaattgtggg gcctggactg tgaaggcctt cgctgcaaga 540
   gcgggcactg gcgaacttca gtgcacgccg cggccgagaa cggatgcagg gcgggggatg 600
   gctgagccgc ctgatccttg cagagaacct gcaggggccc tcggagggta tcccctgcgt 660
   ccaagggagc gcccctcctt ttcagcactc gggggaactg agggcgacgt gccagccccg 720
   cactcaactt tccccttccc tgcaggcaca gagttggccg gcgggggcag aggaggagct 780
   gggtctccac tgcgcccgtt taaacctggc caggggctgc gtttcctccc cccaccccac 840
   gacgatcctt tcttagtctt cgcttttcaa cccaatcgtt aatcattcgg aacgcgcggg 900
   cggggagcgg cgaggagggc gagctcgggg ttcgccgccg ccgccgccgc cgcgcgcgcg 960
   cgctcaggaa gcggtgtggc tgtcaccccc tcccgggcct cctcccccct ccttcctgct 1020
   ttgctccccc tccttcctcc cctcctcccc gctccgccgc ccgcgcccag tgtatctact 1080
   ccctccccac gtcactcgcc agcgcgccat gcaaatcacc gccgccgccg gctcccattg 1140
   gccgcggcgc gctcatttaa tggcagcccg ggcccggcgt atggctgctg ggccccgcgc 1200
   gccgccggcc ccgcgtgcgc ctccgctccg agcgcacggc cccgggcagg cagcgggcag 1260
   cccatcccgg gctcggcggc cccggctctc cggccctctc cgcgagcccg cgctcctccc 1320
   gctgtccccg ggcccctccc tggctgcacc gtaatcgccc cctgcaggcc cccctgcgcc 1380
   tccccccccc cgccactggc gcctggcttc ccccgggcac ctgggaccag cacatgccca 1440
   gcgcacgcgg cgcgccgccc tgctagaagt tgcagcctcc gagttggagg ccgctgagga 1500
   ccgagcgcag gaggaaggag acagcgcgca gcggcggccg gcgaggagac agcacacccc 1560
   gggccgggcc cagcgcaccg ctcccggccc caaaagcgga gctgcaactt ggccacgact 1620
   gcacctgttt gcaccgctcc gccgagggcg cctgggctgc ggtggcggcg aagacggcga 1680
   ccccgaccgt cggcctcttt ggcaagtggt ttgtgcatca ggagaaactt tccacctgcg 1740
   agccgaaccg gcgccgagtg cgtgtgtttc tgcctttttt tgttgtcgtt gcctccaccc   1800
   ctccccattc ttctctccgc taggaccccc ccgcccccgt ctcactccgt ctgaattcct   1860
   ctccgtctcc ctcccacccc ggccgtctat gctccaggcc ctctcctcgc ggtgccggtg 1920
   aacccgccag ccgccccgat gtacagcatg atgatggaga ccgacctgca ctcgcccggc 1980
   ggcgcccagg cccccacgaa cctctcgggc cccgccgggg cgggcggcgg cgggggcgga 2040
   ggcgggggcg gcggcggcgg cgggggcgcc aaggccaacc aggaccgggt caaacggccc 2100
   atgaacgcct tcatggtgtg gtcccgcggg cagcggcgca agatggccca ggagaacccc 2160
   aagatgcaca actcggagat cagcaagcgc ctgggggccg agtggaaggt catgtccgag 2220
   gccgagaagc ggccgttcat cgacgaggcc aagcggctgc gcgcgctgca catgaaggag 2280
   cacccggatt acaagtaccg gccgcgccgc aagaccaaga cgctgctcaa gaaggacaag 2340
   tactcgctgg ccggcgggct cctggcggcc ggcgcgggtg gcggcggcgc ggctgtggcc 2400
   atgggcgtgg gcgtgggcgt gggcgcggcg gccgtgggcc agcgcctgga gagcccaggc 2460
   ggcgcggcgg gcggcggcta cgcgcacgtc aacggctggg ccaacggcgc ctaccccggc 2520
   tcggtggcgg cggcggcggc ggccgcggcc atgatgcagg aggcgcagct ggcctacggg 2580
   cagcacccgg gcgcgggcgg cgcgcacccg cacgcgcacc ccgcgcaccc gcacccgcac 2640
   cacccgcacg cgcacccgca caacccgcag cccatgcacc gctacgacat gggcgcgctg 2700
   cagtacagcc ccatctccaa ctcgcagggc tacatgagcg cgtcgccctc gggctacggc 2760
   ggcctcccct acggcgccgc ggccgccgcc gccgccgctg cgggcggcgc gcaccagaac 2820
   tcggccgtgg cggcggcggc ggcggcggcg gccgcgtcgt cgggcgccct gggcgcgctg 2880
   ggctctctgg tgaagtcgga gcccagcggc agcccgcccg ccccagcgca ctcgcgggcg 2940
   ccgtgccccg gggacctgcg cgagatgatc agcatgtact tgcccgccgg cgaggggggc 3000
   gacccggcgg cggcagcagc ggccgcggcg cagagccggc tgcactcgct gccgcagcac 3060
   taccagggcg cgggcgcggg cgtgaacggc acggtgcccc tgacgcacat ctagcgcctt 3120
   cgggacgccg gggactctgc ggcggcgacc cacgagctcg cggcccgcgc ccggctcccg 3180
   ccccgccccg gcgcggcgtg gcttttgtac agacgttccc acattcttgt caaaaggaaa 3240
   atactggaga cgaacgccgg gtgacgcgtg tcccccactc accttccccg gagaccctgg 3300
   cgaccgccgg gcgctgacac cagacttggg ttttagactg aacttcggtg ttttcttgag 3360
   actttttgta cagtatttat cacctacgga ggaagcggaa agcgttttct ttgctcgagg 3420
   ggacaaaaaa gtcaaaacga ggcgagaggc gaagcccact tttgtatacc ggccggcgcg 3480
   ctcactttcc tccgcgttgc ttccggacgg cgccgaccgc cggagcccaa gtgacgcgga 3540
   gctcgtcgca tttgttataa atgtagtaag gcaggtccaa gcacttacaa gttttttgta 3600
   gttgttaccg ctcttttggg ttggtttgtt aatttataca aagagattac caccaccacc 3660
   ccctccttca gacggcggag ttatattctg ggttttgtaa aactttatgt atctgagcat 3720
   ttccattttt ttttttgggt tttgtattat ttcttgtaaa tgcattgtga aaaattttat 3780
   tttcggcgtt gcaatgcggg gaggagaagt cagattatgt acatagtttt ctaaaaagcc 3840
   tttcttctaa aaacgaaaaa agacccccca cccaaaatgt ttcgagtcaa caaatttaag 3900
   agacagagcc cattttctcc ataaatttgt aacatgctat ttttatgtgc atgttttatg 3960
   agttcaaaat gcaatgagga aatctgacag ggaaattatc tgtatgaact aaaagtaagg 4020
   gaaccccggg gaatgggagg acaggatttt tcaaggaacc tttttcaatg aaagagaagg 4080
   aagttaaaac ctataggtta ttttgtagag ctgagtgtta atacgggccg agaaataaaa 4140
   gtatcttctg ctccggctgt ttcactgcgg acggctgggg ctgctgcgcg ttaccttgct 4200
   gcaagcgggg cgccttccac ctggctgggg gtctgcgcca cagtttggtc cagaggaggg 4260
   aggaggaagg gaagacccca gtggtgggac cctggaccag gccatggatg aaggacaaag 4320
   accagggcag gtcacgggtt tcccaattcc ccagcaatta agatttcgag cagaatttat 4380
   ctaaatgtgt ttcaaggaaa cacaatcgct gaaccaaaac gtactgcagc cgagccccct 4440
   ccgtccatcc tctgcccctc cccctggctt ctttctcttg ggaaaacggg caaaataatt 4500
   gtgctggatt ctcacacaca cagaaatatc gaccatcacc ctcccccgcg tgaactggga 4560
   tgcaagttgc taaccgatgt gaacgcaaaa tgccttgttc attattcctg acgagatctt 4620
   gaggttgttt gatgctttaa attttttaat tatattattt tctaggtgtt tattggtaca 4680
   ttgcagtttt ttttttgaaa tttaaaaatt tctgtaaaac tttgtcttca agtaatctga 4740
   cagcattaaa tattgcattt aaaaattata ctgtagcaaa tacatttaaa aattaatcac 4800
   aacgttaaga tgaaattata tttttggaaa aaaaaaacac ttgaagccca gatggaaata 4860
   cgtttatttc agcagcctta ggtttcccct cgctttctca acacccttcc ttgtcctgga 4920
   gtatggactg tccgtccaaa agtgagccta tgctataagt ttaatgagaa ccgaattcag 4980
   cctgcattcg agaatagctt taagtataat gctgatctga caattgacgt gtaatttggg 5040
   aagtcatttt gataattttg cttaaaccac tcattcgtta aagtgattac aaaaaagttc 5100
   aagaatgatg tccactgctt tctaacaaga taataaaccc cccccctctt ttctttttct 5160
   ttatttttat ttcttttagc tatttgatcc tttctgaagc agttgtttct ggaagagtct 5220
   gtgcgcccat ggatggctga gcaccactac gacttagtcc gggataaggg cctccccagt 5280
   cctctccggg agatgatttg ggaaatttta taatgcttgt tctgttaact caccgggacc 5340
   ttgagggtcc aatgggacct tgagggtttt ctctgaaata tacaaactta aaggactctc 5400
   tctgaggttc tttgactgac gtccactctc agtctggccc ctgtgctccc ctgtgtgtac 5460
   cctggagttt ctgtgtccaa ttgttggcat ctaggtcttg gctcaagatt aggatgtggg 5520
   ccccacttta gaggcacaga ctatgaaaag ctgagttagt gcgcccggga cgccaggcaa 5580
   gcagctttta cagtttggca tcttattgca ggtgcttcgt gcacagtcag ctgaaatagc 5640
   caatgccagg tgctccaacc accttatttc cttgttttgt tgattagaac aacacagaaa 5700
   aaagcaaata taaattttta atgactccat ttaaaaatat cacagggtgg gggcaaggaa 5760
   attagctgag attcatctca ggattgagat tctatccccc cttccccgcc cccagcagtg 5820
   tcgctccaat tcaaattagt ggagaaaaga ttacagtagg ccctgagccg actgtgaatt 5880
   cggtgcttgg ccaaggtaac actcatcgta ttcacggagt gaaatactat atgatgatag 5940
   ttattatatt atatgacgac ttcattcact tcccaaatca cagggtggtg cttctgtcgc 6000
   ttagtccaat tatgtttgca acccgtgtag ggaaggcgga taggagtctt tgaggcctag 6060
   tgcaaagttt gcatcagttt ctgcgggggc gacagtgtga cttcgtaaat ctgccattgc 6120
   gctcgggccc tgccctccaa actgagggta ggagggttta cttgagggtt tttttgcttt 6180
   gttttgttct gtttttgcct tgttttgttt ttcaactcac ccgccccttt tgttgttgag 6240
   attttgacgt ccccttcccc ggagaaaccc acccgtaacg tactagatgg attagtttta 6300
   aagaagactc gggcacagcg tgatctccag atgcgcgtcg ggagcgtgac tgataggtct 6360
   attaaaatca cgcgtgtggg agaacggcac tcatgctccc gcgtttcatt cccccggcaa 6420
   agactgggcg ccgcagattt ctgtctccgg aaaattccag ttaattcaag aaacccatcc 6480
   ccggacgtgt gcccgccccc ccccgaaggc tcgcttcagg ccagcccgga ctccagcgcc 6540
   ccctcgaccc cgtcgggctc cggccgactc ccgccccctc cccacccacg ctcggcctgg 6600
   gcccgggggc agcggctcca gaggcgtcgg cggcagaatt taacccccgg tctgagtcct 6660
   tggaaacttc aagtgaaaag cagacttggc cttggcgcgg ctctctttcc agcgtgacta 6720
   tctggcgaga ctgaaaggcc aggccagatg gggaaaagtg agcggggtgc gggcccccag 6780
   tatccggcgg ggtggggcgg cgtcaggacg gagcgagggc cggcgggggg gctcgcgtgg 6840
   aggcccaggc cgggaggtgg ggacgcaggg acccctcacc cagtcctctg ctcccgcccg 6900
   agccaggcct cgggatcacc tccccttgcc tgagggcgag ctctcctcct ccctcttcct 6960
   ccccgcctcc cccgttcttc cctccctctc tccctcctcc ctccccggcc cttcctctcc 7020
   agctgcgcgc ccgccgggac cacgctcctc ccgcccccgg gagccgagcg gcgtctgcgg 7080
   ctggtccgga cgtgtcccct ccgggcgggg gtggccgggc ctcctcgggg cctcgggcct 7140
   cccgggcgca cccccctgcc tgcgcctctc caagtcgccc tttctccggc tctgcccgcg     7200
   gctgagcccc gcacaccgcg ccgctgtcct tggggtcccg gcacacggcg ttccctcccc     7260
   gcccccgcca tccactccca ccgcctctcc agtccctgtg ccgtctccac cgccctctga     7320
   ctcccgcacc gagctgcgtc tccgccgccg gcctctctca gcgcggcccg gcctcctctt     7380
   tgtctttgaa gctcccccgc cgaggcccgc gcgcggctcc cctctccgtc cctctccggc     7440
   ccttgggcgc ccagtcggag gtcggggcgg gatggggcac gagtctcggg gcggcccgcg     7500
   ctccccctac tagtcccctc attgcctcgg ccccgtcccc gcagcctcgg ggcgggaggg     7560
   cgcaggggtc cccgggcgcc ctgggagccc atctccctca ggtccgcgcc ggtgtcagcc     7620
   gccggcgggg ggcgggtgga gattaagatg agtgctgcga gggacggggc cgagggtttt      7680
   gtgtagaagg agagcaggat tagtggagag aaagtgctga cccggggtca gccgcaagtc     7740
   aaaccctttt acactcagcc ggcggaacga gcacggctcg ggccggacgg caacaagtgg     7800
   gcgcgaaggg agggggaggg gaggccgggt accggccggg tccggcgtga gagaggggag     7860
   ggggagggga cgcggccaca gcgccccagg gcctcagcgt catcggggac gggcgccgag     7920
   gggagcgcgc accagcgacc cgggcgcgga ctggggcctg cagggcgtga ctggctgccc     7980
   cccaggactc catctccttc ataaacacct gccttttcca ttccccgggg ccgcttcggc     8040
   tgctcctctg gagggaagac tggggagaag gttgcctgcg ctctctgccc cagaggctct     8100
   gttttagaaa ctgtttctta atttccataa gaaacaaaaa aatcacagga aggcctggtt     8160
   cctctcccgg acgactgcgt ccccaggctc tccaggaatc ttcgccttat ccgagcttct     8220
   tcgcagccct ggatctattt ctggggggcg tgtgtgacgc agaatcgggg tataaaatat      8280
   caatgctgtt tgtcacgaca ccgctttctc cacccgggca gagctgcggc tcccgggcgt     8340
   tgcgcgtggc gtttttcatc acggagggaa agccgcggct ccgcgggaaa ggagtgaaaa     8400
   ccgagtgtgg gtgaagagga gagggaggtc agggggcaag ggaagaaagc agataaatgc      8460
   atcttcccta gcagccaagc tggaaatttg tgctgaaagt                           8500
<210> 15
   <211> 263
   <212> DNA
   <213> Homo Sapiens
<400> 15
   tacagtattt atcacctacg gaggaagcgg aaagcgtttt ctttgctcga ggggacaaaa       60
   aagtcaaaac gaggcgagag gcgaagccca cttttgtata ccggccggcg cgctcacttt      120
   cctccgcgtt gcttccggac ggcgccgacc gccggagccc aagtgacgcg gagctcgtcg      180
   catttgttat aaatgtagta aggcaggtcc aagcacttac aagttttttg tagttgttac       240
   cgctcttttg ggttggtttg tta                                              263
<210> 16
   <211> 3300
   <212> DNA
   <213> Artificial Sequence
<220>
   <223> chemically treated genomic DNA (Homo sapiens)
<400> 16
   gatatcgcgc gggagttttt cggggttcga ttcgcgtttt tttattagtt ttttttattt      60
   tagttagttt tagatttaag ggaataggat aaacgttttt agttcgattt cgttagttcg     120
   tttttataat taagataaaa cgaaaaaata gtaatagaag taaatataaa aaagttttaa     180
   acggaaattt gtcgggattt tgttttattt taaatttcgg gttagtgttg tttttttttt     240
   ttttttttaa gtattttttt tttaagttcg gtcgtttttt tatattcgtt tttcgttttc     300
   ggtttttaat tcggtttttg taaaatttat tgaagttttt tttttttttt tttttttagt     360
   tttataaaat tcgttggtag ggtttgtggg agtcgcgggg ttttgttaat ttcggggttg     420
   ggtcgggagt cgagcgtagg cggtcggagg gtaatggagt aagcgatagt tttaggcgtt     480
   tttgggtttt cggttgggag ggaagagtcg aggattttgg gtcgtatcgc gtagatggag     540
   acgcgttttt agagttttcg ggtaggttta gggatttcgc gatttgtttt ggtttagcgg     600
   tgtgatttcg cgggttttgg cggttttgat tgttcgtagg ggaggggcgc gttatttttg     660
   gttgttttag gatgcgtcgt ttgaattttt tttttttcgc gggtagcgtt cgttatattt     720
   ttcgggtttt tcggaaattt taattatttt attaggatta ttggggtttg gggtagtttt     780
   tcgggagtcg cgtggacgag ttttggttag gtgggagcga agagtttcgg cgattgttag     840
   ttttttcgtt ttcgtatcgt cgggaaagga tggcgtttta atagataggt agtaagttta     900
   tcgaggttga agaatgaagt ttttttaggg tcggcgggtt ttgagattaa tgagtttaat     960
   aagaaaatta agtagggtta tgatttagaa attaaagaat acggggttag gagaagaggt    1020
   tagttacgat ataggtagcg gttcgttttt tttcgatttt ttaaaggcgt ttttttttta    1080
   gtttttttcg attttaggga tttgtaattt gggatttaaa gttttcgaga gggtagaacg    1140
   attatttaaa acgaattgcg ttagcgagtg ttgtcgtttt ttttaacggt tttggaagat    1200
   tatcgtttcg ggttttaggc gcgttttgtt tcgtgtttat tttcgatatt atttttaaga    1260
   gcgttatcgc ggagcgggcg ttttttggac gttgcggggt tattcgggtt cggtaggtta    1320
   ttcgggttcg gtagatttcg ggcgggtttt gagggttgag ttaggtttaa gagggcgggc    1380
   ggggtcgcgg ggtatcgata ggtcgagggt agggaattga ttcggtttac gtagattttc    1440
   gcgtcgtttt tttttcgttt atcgtagtta ttggtcgatt ttcgattcgt tttttgtttt    1500
   ttggggtcgt ttatttaatt tattagtttt ttaaattttt tttgggattt tagtggcgag    1560
   tattcgtcgt tggtttttta gggtaggtag tattgtcgtt ttaggcgtgc ggagtcgggg    1620
   agaaaaaaat cgaatacgtg tgttatttag ggtttttaga taagtttttt cgcgcgtata    1680
   tatattttga atcggagttt agaaggtcgg attcgcgttt cggggtcgag ttttttattt    1740
   ttcgttttta gttcggaaga ttttagtttg ttcgaaggcg gcgtttgatt cgttggtgga    1800
   tttggtgggt gtttgttttt gatatttggg gacgaggcgg gagcgttttg agcgttggga    1860
   agtttcgttt cgcgagtttt cggtagtgat acgtgggagc gttgggttcg gtattttcga    1920
   cgttttcgag gtttcgggtg gtcgttttgt tttttcgggg gtttttattt tttaagtggt    1980
   tttaaacgat cgaggatggg tttttcgcgt cgtcgtagtt gttatttttt tcgggaaagg    2040
   ttagcgattt tggacgcgta ggttttgaaa attaaggttt agatttatat cgagcgttta    2100
   aagatcgtat ttgaattttt ttcgtcggtt attgagggag agaaaggaac gtttagtttt    2160
   atttatattt atagaaagaa gcgtcgaggg tgggggaaac gtagttttgt cgggtgagtc    2220
   gggataggtt tttcgtttgt ttttcggtcg ttgttttttt ttagttgaat ggggagcgat    2280
   tcgtttcggg cgcggttttc ggggttgaag attgaggtgt agttttattt tcggtttggt    2340
   agcggtttgg aagagagagg gaaaggagga atatttattc ggttaagaga cgtcgttaga    2400
   gtttttaaag ttggcggggg acggaggtcg gggtttcgga tttttagagc gttcgtagtc    2460
   gggtttttgg gtttttcggt gcggggaatt tgattttgta gttttcgtta gggcgcggcg    2520
   gggttgtaag aagggaggaa ggagtgggat tagagttttc gagggcgcgg gcggcgtgta    2580
   tttagttttg ttttttcgcg cgttttgtcg tttgtttgtt ttttttgttg tatcgggtgg    2640
   ttttggtttt cgcgttcgtt cgttttcgac gttttcgcgt ggtttcgcgt tgttcgcggt    2700
   tagtttcgtt tcgtttcgta aggagcgtgt tgttttttta atcgaaatgg gattaagatt    2760
   ttagggtttt ttcgtttttt tgttttgagg tgataggatt ttagttaata gatcgaaaac    2820
   gtgaaaagta ttcgtcgttt tttgttttaa gtgtttttgt ggaaggatta tttatttgga    2880
   aaagtagaat aaattttgtt gtcgtttttt tttcggaggc gtgtttattt tagagttttt    2940
   agatttattg gtattcgtta tatttttttt tgggttttat aggtaggatt ttaagaatga    3000
   tatgggggta tttgggtgat ttgtgggttg taggttttta agaggtcgtt ttttcgtgta    3060
   cggaagtgtc gtagttttat tttagcggaa gtaatattaa attttatttt agaggtttgt    3120
   tttgtaaaga atttagagtt tattatattt gatattattt taaaataata tttgtttgtt    3180
   ttaagaagtt gggagaaatc gataaaagaa aagttgaaat ttatgttttt tatttttatt    3240
   ttaggagata attttgatta atagtttggt ttgtttttta tttttatttt ttaaaaataa    3300
<210> 17
   <211> 3300
   <212> DNA
   <213> Artificial Sequence
<220>
   <223> chemically treated genomic DNA (Homo sapiens)
<400> 17
   ttgtttttaa ggaatggggg tagggagtaa attaaattgt tgattaggat tattttttag      60
   agtagaaatg ggaagtatgg attttaattt tttttttatc gatttttttt aattttttaa    120
   aataagtaag tgttatttta aaatagtgtt aaatatagta aattttggat tttttgtaaa     180
   ataaattttt aagataaggt ttgatattgt tttcgttaaa ataaagttac gatattttcg     240
   tgtacgaaaa aacggttttt tgaggatttg taatttatag attatttagg tgtttttata     300
   ttatttttga aattttattt gtaaagttta ggaggaagta tagcgaatgt tagtgggttt     360
   aggagttttg gggtgagtac gttttcggaa ggaaaacggt aatagaattt attttgtttt      420
   tttaagtaaa tgattttttt atagaggtat ttgggataag aggcgacggg tattttttac    480
   gttttcgatt tattggttaa gattttatta ttttaaaata aggagacgag gaaattttgg     540
   gattttgatt ttatttcgat tgagaaaata atacgttttt tgcgaagcga aacggggttg     600
   gtcgcgggta gcgcggaatt acgcgaaggc gtcgggggcg ggcgggcgcg gggattaggg     660
   ttattcggtg tagtaaaggg ggtaagtagg cggtagggcg cgcggggaga taaggttgag     720
   tgtacgtcgt tcgcgttttc gaaggtttta gttttatttt tttttttttt tttgtagttt      780
   cgtcgcgttt tggcgaaggt tatagggtta ggtttttcgt atcgaggagt ttagaagttc     840
   ggttgcgggc gttttgaaga ttcgggattt cggttttcgt ttttcgttag ttttagggat     900
   tttggcggcg ttttttagtc gggtagatgt tttttttttt tttttttttt ttaagtcgtt      960
   gttaggtcgg gggtgaggtt gtattttagt ttttagtttc gaaggtcgcg ttcggggcgg    1020
   gtcgtttttt atttagttaa gaggaagtag cggtcggggg gtaggcgagg aatttgtttc    1080
   ggtttattcg gtaagattgc gtttttttta ttttcggcgt ttttttttgt gaatgtgaat    1140
   ggaattaagc gttttttttt tttttttaat ggtcggcgag gagaatttaa atgcggtttt    1200
   tgggcgttcg gtatgggttt ggattttggt ttttagagtt tgcgcgttta aggtcgttag    1260
   tttttttcga aggaagtgat agttgcggcg gcgcgggagg tttattttcg gtcgtttaaa    1320
   gttatttaag gaataaaagt tttcgagaga gtaaaacgat tattcgaaat ttcggaagcg    1380
   tcgggggtgt cgggtttaac gtttttacgt gttattgtcg gggattcgcg aggcgaagtt   1440
   ttttagcgtt taaggcgttt tcgtttcgtt tttaggtatt aggaatagat atttattagg    1500
   tttattagcg ggttagacgt cgttttcggg taggttggga tttttcgaat tgggagcgga    1560
   gagtaagggg ttcgatttcg ggacgcgagt tcggtttttt ggatttcggt ttagggtgtg    1620
   tgtgcgcgcg gaggggttta tttgggggtt ttgggtagta tacgtgttcg gttttttttt    1680
   tttcgatttc gtacgtttgg agcggtaata ttgtttgttt tagaaggtta gcggcgagtg    1740
   ttcgttatta gggttttagg gagggtttgg aaaattgatg agttaagtga gcgattttag    1800
   gggatagagg gcgagtcgag agtcggttaa tggttgcggt gggcggggag aagacgacgc    1860
   ggggatttgc gtgggtcggg ttaatttttt attttcgatt tgtcgatgtt tcgcggtttc    1920
   gttcgttttt ttaagtttgg tttagttttt agggttcgtt cgaagtttat cgagttcgag    1980
   tggtttatcg agttcgagtg gtttcgtagc gtttaggagg cgttcgtttc gcggtggcgt    2040
   ttttggaggt ggtgtcggag gtaggtacgg gataggacgc gtttggggtt cggggcggtg    2100
   gttttttagg gtcgttgggg agggcggtag tattcgttgg cgtagttcgt tttggatggt   2160
   cgttttgttt tttcgggggt tttgaatttt aagttgtaga tttttgaggt cggaggaggt    2220
   taggagaagg gcgtttttgg aggatcggga ggagacgggt cgttgtttgt gtcgtggttg    2280
   attttttttt ttgatttcgt gttttttaat ttttgagtta tgattttgtt tggttttttt  2340
   attgggttta ttgattttaa gattcgtcgg ttttgaaggg gttttatttt ttagtttcgg    2400
   tgaatttgtt gtttgtttat taaaacgtta ttttttttcg gcggtgcggg ggcgggagga   2460
   ttggtagtcg tcgaggtttt tcgtttttat ttggttaggg ttcgtttacg cggttttcga   2520
   ggggttattt taggttttaa tagttttggt agaatgattg gagttttcga ggaattcggg    2580
   gaatgtggcg gacgttgttc gcgagggaaa agaggtttag gcggcgtatt ttagggtagt    2640
   taaaagtggc gcgttttttt tttgcgggta gttaggatcg ttaggattcg cggggttata    2700
   tcgttgggtt agagtaggtc gcggggtttt tggatttgtt cgggggtttt gggagcgcgt    2760
   ttttatttgc gcggtgcgat ttagggtttt cggttttttt tttttagtcg agggtttagg    2820
   agcgtttgga gttgtcgttt gttttattgt ttttcgatcg tttgcgttcg gttttcggtt    2880
   tagtttcgag gttggtaggg tttcgcggtt tttatagatt ttattaacga gttttgtagg    2940
   attgagaaga aggaaggaaa gggaatttta atgggttttg taggaatcgg gttgggggtc    3000
   gaaagcggag agcgggtgtg ggaaggcggt cgggtttagg gaaggggtgt ttggagaggg    3060
   aaggggaagg taatattaat tcggaattta gagtagggta ggatttcggt agattttcgt    3120
   ttggggtttt tttgtgtttg tttttattgt tgttttttcg ttttgtttta attgtgggag    3180
   cgggttggcg ggatcggatt gggggcgttt attttgtttt tttggatttg gggttggttg    3240
   gggtggagga ggttggtggg gaggcgcggg tcggatttcg ggaagttttc gcgcggtgtt    3300
<210> 18
   <211> 2700
   <212> DNA
   <213> Artificial Sequence
<220>
   <223> chemically treated genomic DNA (Homo sapiens)
<400> 18
   ggtttttaat tcggtttttg taaaatttat tgaagttttt tttttttttt tttttttagt 60
   tttataaaat tcgttggtag ggtttgtggg agtcgcgggg ttttgttaat ttcggggttg 120
   ggtcgggagt cgagcgtagg cggtcggagg gtaatggagt aagcgatagt tttaggcgtt 180
   tttgggtttt cggttgggag ggaagagtcg aggattttgg gtcgtatcgc gtagatggag 240
   acgcgttttt agagttttcg ggtaggttta gggatttcgc gatttgtttt ggtttagcgg 300
   tgtgatttcg cgggttttgg cggttttgat tgttcgtagg ggaggggcgc gttatttttg 360
   gttgttttag gatgcgtcgt ttgaattttt tttttttcgc gggtagcgtt cgttatattt 420
   ttcgggtttt tcggaaattt taattatttt attaggatta ttggggtttg gggtagtttt 480
   tcgggagtcg cgtggacgag ttttggttag gtgggagcga agagtttcgg cgattgttag 540
   ttttttcgtt ttcgtatcgt cgggaaagga tggcgtttta atagataggt agtaagttta 600
   tcgaggttga agaatgaagt ttttttaggg tcggcgggtt ttgagattaa tgagtttaat 660
   aagaaaatta agtagggtta tgatttagaa attaaagaat acggggttag gagaagaggt 720
   tagttacgat ataggtagcg gttcgttttt tttcgatttt ttaaaggcgt ttttttttta 780
   gtttttttcg attttaggga tttgtaattt gggatttaaa gttttcgaga gggtagaacg 840
   attatttaaa acgaattgcg ttagcgagtg ttgtcgtttt ttttaacggt tttggaagat 900
   tatcgtttcg ggttttaggc gcgttttgtt tcgtgtttat tttcgatatt atttttaaga 960
   gcgttatcgc ggagcgggcg ttttttggac gttgcggggt tattcgggtt cggtaggtta 1020
   ttcgggttcg gtagatttcg ggcgggtttt gagggttgag ttaggtttaa gagggcgggc 1080
   ggggtcgcgg ggtatcgata ggtcgagggt agggaattga ttcggtttac gtagattttc 1140
   gcgtcgtttt tttttcgttt atcgtagtta ttggtcgatt ttcgattcgt tttttgtttt 1200
   ttggggtcgt ttatttaatt tattagtttt ttaaattttt tttgggattt tagtggcgag 1260
   tattcgtcgt tggtttttta gggtaggtag tattgtcgtt ttaggcgtgc ggagtcgggg 1320
   agaaaaaaat cgaatacgtg tgttatttag ggtttttaga taagtttttt cgcgcgtata 1380
   tatattttga atcggagttt agaaggtcgg attcgcgttt cggggtcgag ttttttattt 1440
   ttcgttttta gttcggaaga ttttagtttg ttcgaaggcg gcgtttgatt cgttggtgga 1500
   tttggtgggt gtttgttttt gatatttggg gacgaggcgg gagcgttttg agcgttggga 1560
   agtttcgttt cgcgagtttt cggtagtgat acgtgggagc gttgggttcg gtattttcga 1620
   cgttttcgag gtttcgggtg gtcgttttgt tttttcgggg gtttttattt tttaagtggt 1680
   tttaaacgat cgaggatggg tttttcgcgt cgtcgtagtt gttatttttt tcgggaaagg 1740
   ttagcgattt tggacgcgta ggttttgaaa attaaggttt agatttatat cgagcgttta 1800
   aagatcgtat ttgaattttt ttcgtcggtt attgagggag agaaaggaac gtttagtttt 1860
   atttatattt atagaaagaa gcgtcgaggg tgggggaaac gtagttttgt cgggtgagtc 1920
   gggataggtt tttcgtttgt ttttcggtcg ttgttttttt ttagttgaat ggggagcgat 1980
   tcgtttcggg cgcggttttc ggggttgaag attgaggtgt agttttattt tcggtttggt 2040
   agcggtttgg aagagagagg gaaaggagga atatttattc ggttaagaga cgtcgttaga 2100
   gtttttaaag ttggcggggg acggaggtcg gggtttcgga tttttagagc gttcgtagtc 2160
   gggtttttgg gtttttcggt gcggggaatt tgattttgta gttttcgtta gggcgcggcg 2220
   gggttgtaag aagggaggaa ggagtgggat tagagttttc gagggcgcgg gcggcgtgta 2280
   tttagttttg ttttttcgcg cgttttgtcg tttgtttgtt ttttttgttg tatcgggtgg 2340
   ttttggtttt cgcgttcgtt cgttttcgac gttttcgcgt ggtttcgcgt tgttcgcggt 2400
   tagtttcgtt tcgtttcgta aggagcgtgt tgttttttta atcgaaatgg gattaagatt 2460
   ttagggtttt ttcgtttttt tgttttgagg tgataggatt ttagttaata gatcgaaaac 2520
   gtgaaaagta ttcgtcgttt tttgttttaa gtgtttttgt ggaaggatta tttatttgga 2580
   aaagtagaat aaattttgtt gtcgtttttt tttcggaggc gtgtttattt tagagttttt 2640
   agatttattg gtattcgtta tatttttttt tgggttttat aggtaggatt ttaagaatga 2700
<210> 19
   <211> 2700
   <212> DNA
   <213> Artificial Sequence
<220>
   <223> chemically treated genomic DNA (Homo sapiens)
<400> 19
   ttatttttga aattttattt gtaaagttta ggaggaagta tagcgaatgt tagtgggttt      60
   aggagttttg gggtgagtac gttttcggaa ggaaaacggt aatagaattt attttgtttt     120
   tttaagtaaa tgattttttt atagaggtat ttgggataag aggcgacggg tattttttac     180
   gttttcgatt tattggttaa gattttatta ttttaaaata aggagacgag gaaattttgg     240
   gattttgatt ttatttcgat tgagaaaata atacgttttt tgcgaagcga aacggggttg     300
   gtcgcgggta gcgcggaatt acgcgaaggc gtcgggggcg ggcgggcgcg gggattaggg     360
   ttattcggtg tagtaaaggg ggtaagtagg cggtagggcg cgcggggaga taaggttgag     420
   tgtacgtcgt tcgcgttttc gaaggtttta gttttatttt tttttttttt tttgtagttt     480
   cgtcgcgttt tggcgaaggt tatagggtta ggtttttcgt atcgaggagt ttagaagttc     540
   ggttgcgggc gttttgaaga ttcgggattt cggttttcgt ttttcgttag ttttagggat     600
   tttggcggcg ttttttagtc gggtagatgt tttttttttt tttttttttt ttaagtcgtt     660
   gttaggtcgg gggtgaggtt gtattttagt ttttagtttc gaaggtcgcg ttcggggcgg     720
   gtcgtttttt atttagttaa gaggaagtag cggtcggggg gtaggcgagg aatttgtttc     780
   ggtttattcg gtaagattgc gtttttttta ttttcggcgt ttttttttgt gaatgtgaat     840
   ggaattaagc gttttttttt tttttttaat ggtcggcgag gagaatttaa atgcggtttt     900
   tgggcgttcg gtatgggttt ggattttggt ttttagagtt tgcgcgttta aggtcgttag     960
   tttttttcga aggaagtgat agttgcggcg gcgcgggagg tttattttcg gtcgtttaaa    1020
   gttatttaag gaataaaagt tttcgagaga gtaaaacgat tattcgaaat ttcggaagcg    1080
   tcgggggtgt cgggtttaac gtttttacgt gttattgtcg gggattcgcg aggcgaagtt    1140
   ttttagcgtt taaggcgttt tcgtttcgtt tttaggtatt aggaatagat atttattagg    1200
   tttattagcg ggttagacgt cgttttcggg taggttggga tttttcgaat tgggagcgga    1260
   gagtaagggg ttcgatttcg ggacgcgagt tcggtttttt ggatttcggt ttagggtgtg    1320
   tgtgcgcgcg gaggggttta tttgggggtt ttgggtagta tacgtgttcg gttttttttt    1380
   tttcgatttc gtacgtttgg agcggtaata ttgtttgttt tagaaggtta gcggcgagtg    1440
   ttcgttatta gggttttagg gagggtttgg aaaattgatg agttaagtga gcgattttag    1500
   gggatagagg gcgagtcgag agtcggttaa tggttgcggt gggcggggag aagacgacgc    1560
   ggggatttgc gtgggtcggg ttaatttttt attttcgatt tgtcgatgtt tcgcggtttc    1620
   gttcgttttt ttaagtttgg tttagttttt agggttcgtt cgaagtttat cgagttcgag    1680
   tggtttatcg agttcgagtg gtttcgtagc gtttaggagg cgttcgtttc gcggtggcgt    1740
   ttttggaggt ggtgtcggag gtaggtacgg gataggacgc gtttggggtt cggggcggtg    1800
   gttttttagg gtcgttgggg agggcggtag tattcgttgg cgtagttcgt tttggatggt    1860
   cgttttgttt tttcgggggt tttgaatttt aagttgtaga tttttgaggt cggaggaggt    1920
   taggagaagg gcgtttttgg aggatcggga ggagacgggt cgttgtttgt gtcgtggttg    1980
   attttttttt ttgatttcgt gttttttaat ttttgagtta tgattttgtt tggttttttt    2040
   attgggttta ttgattttaa gattcgtcgg ttttgaaggg gttttatttt ttagtttcgg    2100
   tgaatttgtt gtttgtttat taaaacgtta ttttttttcg gcggtgcggg ggcgggagga    2160
   ttggtagtcg tcgaggtttt tcgtttttat ttggttaggg ttcgtttacg cggttttcga    2220
   ggggttattt taggttttaa tagttttggt agaatgattg gagttttcga ggaattcggg    2280
   gaatgtggcg gacgttgttc gcgagggaaa agaggtttag gcggcgtatt ttagggtagt    2340
   taaaagtggc gcgttttttt tttgcgggta gttaggatcg ttaggattcg cggggttata    2400
   tcgttgggtt agagtaggtc gcggggtttt tggatttgtt cgggggtttt gggagcgcgt    2460
   ttttatttgc gcggtgcgat ttagggtttt cggttttttt tttttagtcg agggtttagg    2520
   agcgtttgga gttgtcgttt gttttattgt ttttcgatcg tttgcgttcg gttttcggtt    2580
   tagtttcgag gttggtaggg tttcgcggtt tttatagatt ttattaacga gttttgtagg    2640
   attgagaaga aggaaggaaa gggaatttta atgggttttg taggaatcgg gttgggggtt    2700
<210> 20
   <211> 280
   <212> DNA
   <213> Artificial Sequence
<220>
   <223> chemically treated genomic DNA (Homo sapiens)
<400> 20
   tagagttttc gagggcgcgg gcggcgtgta tttagttttg ttttttcgcg cgttttgtcg      60
   tttgtttgtt ttttttgttg tatcgggtgg ttttggtttt cgcgttcgtt cgttttcgac     120
   gttttcgcgt ggtttcgcgt tgttcgcggt tagtttcgtt tcgtttcgta aggagcgtgt     180
   tgttttttta atcgaaatgg gattaagatt ttagggtttt ttcgtttttt tgttttgagg     240
   tgataggatt ttagttaata gatcgaaaac gtgaaaagta                           280
<210> 21
   <211> 280
   <212> DNA
   <213> Artificial Sequence
<220>
   <223> chemically treated genomic DNA (Homo sapiens)
<400> 21
   tattttttac gttttcgatt tattggttaa gattttatta ttttaaaata aggagacgag       60
   gaaattttgg gattttgatt ttatttcgat tgagaaaata atacgttttt tgcgaagcga     120
   aacggggttg gtcgcgggta gcgcggaatt acgcgaaggc gtcgggggcg ggcgggcgcg     180
   gggattaggg ttattcggtg tagtaaaggg ggtaagtagg cggtagggcg cgcggggaga     240
   taaggttgag tgtacgtcgt tcgcgttttc gaaggtttta                           280
<210> 22
   <211> 1968
   <212> DNA
   <213> Artificial Sequence
<220>
   <223> chemically treated genomic DNA (Homo sapiens)
<400> 22
   attttttaaa attttataag tgttttagga atgatttggt gttttttatg tttttagagt       60
   ttaggttttt tgtgttttat gtagtgtata gatttgtttt tgggttattt tttatattaa     120
   attttagttt tttttattgg gtataaaggg aaattttaaa attttgtttt ttatggaaaa 180
   atgttttttg tttttcgggg tagattttgt agggagagat ttgtaaatat gaaggaggag 240
   tggaaggtgt gatcggagaa aatattaagg attgaagtgt gaaggggtat gggatggatt 300
   ggaggtagtt ttgagatgag agtttgttga attcgtgttt cgggagatta agttggtttt 360
   ttttggaaaa aatttgtaag tttgggaaag ttgggattgg tttttattat aagtagttgg 420
   gatttaagtt tatttgtatt agggttattt tagtttagtt ttgttgggaa tcgtatcggg 480
   atgggtgtgg gacggtttcg aatttagatt cgggtgttgt tttttttagg tagtttttcg 540
   gagtttgttt tttggattat aaaatgaaga gttgtgaaga gttaataatg ttaagttgga 600
   agggtttgat acggaatttg attaaggttg gatttttaat aattgtgatt tttttttttt 660
   tttttttttt ttggtaaaat tattttacga atttacgtaa gtagggtttt tcgttatttt 720
   tcggagtagt cgtttgaggg ttggaaggag tagtgataga aattttagag acgtagagat 780
   ttttcgaatt tcgaattcga ttattgtttt ttttcgatcg tcgaattcgt tggagaagcg 840
   ggcgcgatag ggcgatgagt taacgcggag ggagcgcgga ggtcgcggaa gtcgggggcg 900
   ttgggtttta ggttcggatg ttgagcgcgg atcggcgtgt tttttttata gcgttttcgc 960
   gcggtttttt ttcgttgcgt ttcgtacggc gattcgtcgc gggtagtttt ggcgtttggt 1020
   tacgtcgtcg gttgaggatc gttagagttg gggggagatt aaagtatttt tatggggttt 1080
   aaagagtttg ggattgtagt gttgttagtt tggtttcgtc gcgttaataa attttcggcg 1140
   aaagttggga gtaggagtgg aggtttgggg cgggggttgg tggggcgcgg gggttggtcg 1200
   gagggagggt tcgggggttt tttgtatttg cgtttttgtc gcgttaagtt agtgggaagt 1260
   aagtgatatt ttttttttat tcgttttttt ggaatttgtt tatttttttt ttttagaata 1320
   tcgtttgttt ttcggggtta gtattattaa agagaagatt ttttttaggt ttttaaaatt 1380
   tacgtggcgt tttgttataa ggagcgcgtt ggaagttggt tttggtattt ttttgttttt 1440
   tttttttttt gtttcgtttt tatattttcg tggagaaatt ttcgagtagg ttttttgtat 1500
   ttttttattt tatttagaat tataaaaaat atatgggaag gtgatttggt tttattttaa 1560
   tttttttatt ttgtagaagt ttaaagtggt gatggtgaga aaagtggttt aaggttaagg 1620
   gttattttag ttggtaaaat taggagtaga ataggagaat ttttagagtt ttattatata 1680
   ttttttgtta tttattttat ggataaaaat aattattttt ttaggtaggt taagtatttt 1740
   ttaaaggtgt ttgtgttttt gttagttggt tgtaaaattt agtattatat ttgttatttg 1800
   gatattatgg ggagtcgttg gttatttaac gatagagtcg agtatatttt gatttttttt 1860
   ttgagatatt ttttttttat ttttatttta ttttgaaaaa tattatggag tatgtgtttt 1920
   gggtaagttt ttttttaggc gtgggtaaaa tttgagtata gggatttt 1968
<210> 23
   <211> 1968
   <212> DNA
   <213> Artificial Sequence
<220>
   <223> chemically treated genomic DNA (Homo sapiens)
<400> 23
   agaatttttg tgtttaaatt ttatttacgt ttgggagaag atttgtttag aatatatgtt 60
   ttatagtgtt ttttaggatg gagtgggagt gagaaaggag tgttttagag gagggattag 120
   agtgtattcg gttttgtcgt tggatagtta gcgatttttt atgatgttta ggtgataagt 180
   gtgatgttaa gttttgtagt taattggtaa aagtatagat atttttaaag agtatttgat 240
   ttgtttaaga gaatgattat ttttgtttat gaaataagta gtaaaaaata tatagtaaga 300
   ttttagggat ttttttgttt tatttttggt tttgttaatt gaagtaattt ttgattttgg 360
   gttatttttt ttattattat tattttaggt ttttgtaaaa tgaagaggtt gaagtgaaat 420
   taaattattt ttttatatat tttttgtagt tttaagtggg gtgagaaaat gtaaaaggtt 480
   tgttcggggg tttttttacg gggatgtggg ggcggggtag gagaaagagg ggataaggag 540
   atattaaagt tagtttttag cgcgtttttt gtaataaggc gttacgtaaa ttttaagggt 600
   ttggaggaaa tttttttttt gataatgttg atttcggaga gtaggcggtg ttttaaagga 660
   gggagatgag taagttttaa gggggcgggt gaaagggaaa tgttatttgt tttttattga 720
   tttaacgcgg taggagcgta ggtgtaagaa attttcggat ttttttttcg gttaattttc 780
   gcgttttatt aattttcgtt ttaagttttt atttttgttt ttaattttcg tcgaaaattt 840
   attgacgcgg cgaggttagg ttaataatat tgtaatttta ggttttttgg gttttatagg 900
   aatgttttga ttttttttta gttttagcgg tttttagtcg acggcgtggt taaacgttag 960
   ggttattcgc ggcgggtcgt cgtgcggggc gtagcgggag gaggtcgcgc gggggcgttg 1020
   tggggaggat acgtcggttc gcgtttagta ttcgggtttg agatttagcg ttttcggttt 1080
   tcgcggtttt cgcgtttttt tcgcgttaat ttatcgtttt gtcgcgttcg tttttttagc 1140
   gggttcggcg gtcgggggag gatagtgatc gagttcgaag ttcggagggt ttttgcgttt 1200
   ttggggtttt tattattgtt ttttttagtt tttaagcggt tatttcgaag aatgacgaag 1260
   ggttttattt gcgtagattc gtgggatgat tttattaaga aggaaaaggg ggaaaagaaa 1320
   ttataattat tgagggttta gttttggtta gatttcgtgt tagatttttt taatttgata 1380
   ttattgattt tttataattt tttattttgt agtttaggaa gtaggtttcg agagattgtt 1440
   tagaaaaaat agtattcgga tttgaattcg aggtcgtttt atatttattt cggtacggtt 1500
   tttagtagaa ttgagttgga gtggttttga tgtaaatgaa tttagatttt agttgtttgt 1560
   gatggaagtt aattttagtt tttttaagtt tataggtttt ttttagaaga agttaatttg 1620
   atttttcgga atacgaattt agtaggtttt tattttagaa ttgtttttaa tttattttat 1680
   atttttttat attttagttt ttggtatttt tttcgattat attttttatt ttttttttat 1740
   atttgtaagt ttttttttgt agaatttgtt tcgaggagta aaggatattt ttttatgggg 1800
   aatagaattt tagaattttt ttttgtattt agtgagaaag attaaagttt ggtataggaa 1860
   ataatttaaa gataaattta tatattgtat agaatatagg aagtttgggt tttgaaaata 1920
   taaaagatat taaattattt ttggagtatt tgtgaagttt tggaaaat 1968
<210> 24
   <211> 1368
   <212> DNA
   <213> Artificial Sequence
<220>
   <223> chemically treated genomic DNA (Homo sapiens)
<400> 24
   ggaggtagtt ttgagatgag agtttgttga attcgtgttt cgggagatta agttggtttt      60
   ttttggaaaa aatttgtaag tttgggaaag ttgggattgg tttttattat aagtagttgg     120
   gatttaagtt tatttgtatt agggttattt tagtttagtt ttgttgggaa tcgtatcggg     180
   atgggtgtgg gacggtttcg aatttagatt cgggtgttgt tttttttagg tagtttttcg      240
   gagtttgttt tttggattat aaaatgaaga gttgtgaaga gttaataatg ttaagttgga     300
   agggtttgat acggaatttg attaaggttg gatttttaat aattgtgatt tttttttttt      360
   tttttttttt ttggtaaaat tattttacga atttacgtaa gtagggtttt tcgttatttt      420
   tcggagtagt cgtttgaggg ttggaaggag tagtgataga aattttagag acgtagagat     480
   ttttcgaatt tcgaattcga ttattgtttt ttttcgatcg tcgaattcgt tggagaagcg     540
   ggcgcgatag ggcgatgagt taacgcggag ggagcgcgga ggtcgcggaa gtcgggggcg     600
   ttgggtttta ggttcggatg ttgagcgcgg atcggcgtgt tttttttata gcgttttcgc      660
   gcggtttttt ttcgttgcgt ttcgtacggc gattcgtcgc gggtagtttt ggcgtttggt     720
   tacgtcgtcg gttgaggatc gttagagttg gggggagatt aaagtatttt tatggggttt     780
   aaagagtttg ggattgtagt gttgttagtt tggtttcgtc gcgttaataa attttcggcg     840
   aaagttggga gtaggagtgg aggtttgggg cgggggttgg tggggcgcgg gggttggtcg     900
   gagggagggt tcgggggttt tttgtatttg cgtttttgtc gcgttaagtt agtgggaagt     960
   aagtgatatt ttttttttat tcgttttttt ggaatttgtt tatttttttt ttttagaata   1020
   tcgtttgttt ttcggggtta gtattattaa agagaagatt ttttttaggt ttttaaaatt     1080
   tacgtggcgt tttgttataa ggagcgcgtt ggaagttggt tttggtattt ttttgttttt    1140
   tttttttttt gtttcgtttt tatattttcg tggagaaatt ttcgagtagg ttttttgtat       1200
   tttttatttt tatttagaat tataaaaaat atatgggaag gtgatttggt tttattttaa    1260
   tttttttatt ttgtagaagt ttaaagtggt gatggtgaga aaagtggttt aaggttaagg    1320
   gttattttag ttggtaaaat taggagtaga ataggagaat ttttagag                1368
<210> 25
   <211> 1368
   <212> DNA
   <213> Artificial Sequence
<220>
   <223> chemically treated genomic DNA (Homo sapiens)
<400> 25
   ttttagggat ttttttgttt tatttttggt tttgttaatt gaagtaattt ttgattttgg      60
   gttatttttt ttattattat tattttaggt ttttgtaaaa tgaagaggtt gaagtgaaat   120
   taaattattt ttttatatat tttttgtagt tttaagtggg gtgagaaaat gtaaaaggtt    180
   tgttcggggg tttttttacg gggatgtggg ggcggggtag gagaaagagg ggataaggag   240
   atattaaagt tagtttttag cgcgtttttt gtaataaggc gttacgtaaa ttttaagggt    300
   ttggaggaaa tttttttttt gataatgttg atttcggaga gtaggcggtg ttttaaagga     360
   gggagatgag taagttttaa gggggcgggt gaaagggaaa tgttatttgt tttttattga    420
   tttaacgcgg taggagcgta ggtgtaagaa attttcggat ttttttttcg gttaattttc   480
   gcgttttatt aattttcgtt ttaagttttt atttttgttt ttaattttcg tcgaaaattt    540
   attgacgcgg cgaggttagg ttaataatat tgtaatttta ggttttttgg gttttatagg    600
   aatgttttga ttttttttta gttttagcgg tttttagtcg acggcgtggt taaacgttag    660
   ggttattcgc ggcgggtcgt cgtgcggggc gtagcgggag gaggtcgcgc gggggcgttg    720
   tggggaggat acgtcggttc gcgtttagta ttcgggtttg agatttagcg ttttcggttt    780
   tcgcggtttt cgcgtttttt tcgcgttaat ttatcgtttt gtcgcgttcg tttttttagc    840
   gggttcggcg gtcgggggag gatagtgatc gagttcgaag ttcggagggt ttttgcgttt    900
   ttggggtttt tattattgtt ttttttagtt tttaagcggt tatttcgaag aatgacgaag    960
   ggttttattt gcgtagattc gtgggatgat tttattaaga aggaaaaggg ggaaaagaaa   1020
   ttataattat tgagggttta gttttggtta gatttcgtgt tagatttttt taatttgata   1080
   ttattgattt tttataattt tttattttgt agtttaggaa gtaggtttcg agagattgtt   1140
   tagaaaaaat agtattcgga tttgaattcg aggtcgtttt atatttattt cggtacggtt   1200
   tttagtagaa ttgagttgga gtggttttga tgtaaatgaa tttagatttt agttgtttgt   1260
   gatggaagtt aattttagtt tttttaagtt tataggtttt ttttagaaga agttaatttg    1320
   atttttcgga atacgaattt agtaggtttt tattttagaa ttgttttt                1368
<210> 26
   <211> 336
   <212> DNA
   <213> Artificial Sequence
<220>
   <223> chemically treated genomic DNA (Homo sapiens)
<400> 26
   taggtagttt ttcggagttt gttttttgga ttataaaatg aagagttgtg aagagttaat      60
   aatgttaagt tggaagggtt tgatacggaa tttgattaag gttggatttt taataattgt     120
   gatttttttt tttttttttt ttttttggta aaattatttt acgaatttac gtaagtaggg     180
   tttttcgtta tttttcggag tagtcgtttg agggttggaa ggagtagtga tagaaatttt     240
   agagacgtag agatttttcg aatttcgaat tcgattattg ttttttttcg atcgtcgaat      300
   tcgttggaga agcgggcgcg atagggcgat gagtta                               336
<210> 27
   <211> 336
   <212> DNA
   <213> Artificial Sequence
<220>
   <223> chemically treated genomic DNA (Homo sapiens)
<400> 27
   taatttatcg ttttgtcgcg ttcgtttttt tagcgggttc ggcggtcggg ggaggatagt      60
   gatcgagttc gaagttcgga gggtttttgc gtttttgggg tttttattat tgtttttttt     120
   agtttttaag cggttatttc gaagaatgac gaagggtttt atttgcgtag attcgtggga     180
   tgattttatt aagaaggaaa aggggaaaaa gaaattataa ttattgaggg tttagttttg     240
   gttagatttc gtgttagatt tttttaattt gatattattg attttttata attttttatt     300
   ttgtagttta ggaagtaggt ttcgagagat tgttta                               336
<210> 28
   <211> 1900
   <212> DNA
   <213> Artificial Sequence
<220>
   <223> chemically treated genomic DNA (Homo sapiens)
<400> 28
   aaaaatttat ttgttgaatt gagtggtttt ggttttagat ggttaggtag tttaaaaata      60
   tgaagttgaa gatggtagtt atttaaaaat tgttttattg ttatttttat gacgataaga     120
   aagtaagtag aggtaagggt gttaaattta gaaatagtat ataggaatga ataatttttg    180
   gggattttta ttggtagatt ttaaaaggta aattttttag tttgatgtga attatttttt     240
   gtgatttttt ttttttaatt tattttgata tttttatatt tttatgggga aaagttgttt     300
   tattatatag ttaatcggtg gattaggtaa tttatgggag tgttgaaagt cgaaatttta     360
   ggttgggttt gtggtaaaat ttttaatttt atttttagtt aatagaatgt tataatgagg     420
   gaaaatattt agtaagttga aaatatagaa taaggaagcg gaaagaaatt ttagtgataa      480
   tgttattaag tggaaagaag gaaagtggtt tattttttag gaaaataaat attgattttt      540
   ttatagttat attttaagcg tattggttaa ggtatgtttt gttttgaaaa attattagag      600
   gttatagcgt ggtttttttc ggttacgtat agcgagttcg ttattatagt gtttttagag       660
   ggtatacgat tttatttttt tatttaattt tttattcgtg tttttttttt gggtttttgt       720
   ggtatttata tagtagtttt tgttttgttg ttttttggat ttttagtttt tattatattt      780
   ggcggttttt aggaagtttg aataaatttc gacgcgatta aaggagagga aagtttgttt      840
   gttattttgg gtggattaga gataatattt ttaggggata gggcgttata tggtagttta      900
   aggcgtagtg atattcgagt ttgatataat taatattagt ttcgttaggt ggtttttcga      960
   tgtttatttt tttattaaat aagatttata ttcgtttaat aagattttgg gataatttta     1020
   attttcgttt tttagtaagg cgcgcggttg gttagtcggg gcgggaagtt ggttttgcgt     1080
   taaggattcg tagttttagt agtagtagat taggtagaag tgttttttta tttttatttt      1140
   aagttttttt agaaattgtc gttagagtag atataaggtt ttggttgggg tattattttt      1200
   tgggtgggtg gggaggggag aagagataat taaaagttag ttgaattgag aaaggaattt     1260
   ggtgaatttt tttattttgt ttaatagtta aagatttttt tatttttaat ttagtttttt     1320
   cgatttttaa attagttatt acggaagttt atcgttgttt tttaatttaa cgcggaaaat     1380
   tttatcgttg gggattttaa ataggtgttt agtaagttgt gtgtcggatt ggggcggtcg     1440
   gtttcggtaa tatttggtaa tttttgggtg tttttaggcg tgtggttgtc ggtttatttt     1500
   tcgagggcgt tcggaggcga agtatttaag cgggttttcg tatttagcgg gcgggatttt     1560
   ggcgggtttc gcgcgaagat tcgggttttg gttcgagcga atggcgttta aatttcggtt     1620
   tggggagaat gacggtttga attgcggcgg ggcgtttttt ttatcgtaga aagtgtgggt     1680
   acgtagaaat tcgcggtagg agagttttaa gtggagcgta gtttgatagc gtttgttttt     1740
   gaattcgtgt cggatcgagg ggacgttatt tagcgggtag ggtgacgtta tcgttttttt     1800
   gatattttat attaaagggt tggtatgtta tagggaggga gggggagagc gaaagttagc     1860
   gcgagtaata gagagatttt tttttagtag gtacgaaagt                           1900
<210> 29
   <211> 1900
   <212> DNA
   <213> Artificial Sequence
<220>
   <223> chemically treated genomic DNA (Homo sapiens)
<400> 29
   gttttcgtgt ttgttgagaa aggatttttt tgttgttcgc gttggttttc gttttttttt 60
   tttttttttg taatatgtta gttttttaat gtggagtgtt agggagacgg tgacgttatt 120
   ttgttcgttg ggtggcgttt tttcggttcg atacgagttt agagatagac gttgttagat 180
   tgcgttttat ttggggtttt tttgtcgcgg gtttttgcgt atttatattt tttgcggtgg 240
   aggggacgtt tcgtcgtaat ttaggtcgtt atttttttta ggtcggggtt tgagcgttat 300
   tcgttcgggt tagagttcgg attttcgcgc ggagttcgtt agagtttcgt tcgttggata 360
   cgaggattcg tttggatgtt tcgttttcga gcgttttcgg aagatgggtc ggtagttata 420
   cgtttaaaga tatttagagg ttattaggta ttgtcggaat cggtcgtttt aattcggtat 480
   atagtttgtt gggtatttat ttgagatttt tagcggtagg atttttcgcg ttaggttgag 540
   gggtagcggt gggttttcgt ggtgattggt ttgggagtcg ggagaattaa gttgggggtg 600
   ggggggtttt tggttgttgg gtaggatagg gaagtttatt aggttttttt tttagtttaa 660
   ttaattttta attgtttttt tttttttttt tatttattta aaaaataatg ttttaattaa 720
   aattttgtgt ttgttttgac ggtagttttt gggagggttt ggggtaaaag tgaggagata 780
   tttttgtttg gtttattgtt gttaaggttg cgagttttta acgtagagtt agtttttcgt 840
   ttcggttggt tagtcgcgcg ttttgttggg gagcgagagt tggaattatt ttagggtttt 900
   attaagcgga tatgaatttt gtttggtagg gaaatgagta tcgaagggtt atttgacgaa 960
   gttagtgttg gttgtattag attcgggtgt tattgcgttt tgaattgtta tgtaacgttt 1020
   tgttttttgg aaatattgtt tttgatttat ttaaagtgat aagtaggttt tttttttttt 1080
   tgatcgcgtc gaggtttgtt taagtttttt ggaggtcgtt aaatgtaatg agaattgagg 1140
   atttaggggg tagtaggata gaggttgttg tatagatgtt ataggggttt agagagagga 1200
   tacggataag aaattgggtg agggagtaag atcgtgtatt ttttgaaagt attgtaatag 1260
   cgaattcgtt gtacgtggtc gaaaggggtt acgttatggt ttttggtgat tttttagggt 1320
   aaagtatgtt ttggttaatg cgtttgagat gtagttgtgg aaagattagt gtttgttttt 1380
   ttagggagtg agttattttt ttttttttta tttagtggta ttgttattaa aatttttttt 1440
   cgtttttttg ttttgtattt ttaatttatt gaatattttt ttttattgtg gtattttgtt 1500
   gattggaggt gaagttagaa attttattat agatttaatt tgggatttcg atttttaata 1560
   tttttataga ttatttagtt tatcgattgg ttgtatggtg gggtagtttt tttttataaa 1620
   gatatagaga tattaaaata aattagggga aaagagttat agaaggtggt ttatattaag 1680
   ttaaaaaatt tgttttttga agtttgttaa tgaagatttt taagaattgt ttatttttgt 1740
   gtattgtttt tgggtttaat atttttgttt ttatttattt ttttatcgtt atggaaatga 1800
   tagtaaaata gtttttaaat gattattatt tttaatttta tgtttttaag ttatttaatt 1860
   atttgagatt agaattattt agtttagtaa gtaggttttt 1900
<210> 30
   <211> 1002
   <212> DNA
   <213> Artificial Sequence
<220>
   <223> chemically treated genomic DNA (Homo sapiens)
<400> 30
   ttattatata gttaatcggt ggattaggta atttatggga gtgttgaaag tcgaaatttt 60
   aggttgggtt tgtggtaaaa tttttaattt tatttttagt taatagaatg ttataatgag 120
   ggaaaatatt tagtaagttg aaaatataga ataaggaagc ggaaagaaat tttagtgata 180
   atgttattaa gtggaaagaa ggaaagtggt ttatttttta ggaaaataaa tattgatttt 240
   tttatagtta tattttaagc gtattggtta aggtatgttt tgttttgaaa aattattaga 300
   ggttatagcg tggttttttt cggttacgta tagcgagttc gttattatag tgtttttaga 360
   gggtatacga ttttattttt ttatttaatt ttttattcgt gttttttttt tgggtttttg 420
   tggtatttat atagtagttt ttgttttgtt gttttttgga tttttagttt ttattatatt 480
   tggcggtttt taggaagttt gaataaattt cgacgcgatt aaaggagagg aaagtttgtt 540
   tgttattttg ggtggattag agataatatt tttaggggat agggcgttat atggtagttt 600
   aaggcgtagt gatattcgag tttgatataa ttaatattag tttcgttagg tggtttttcg 660
   atgtttattt ttttattaaa taagatttat attcgtttaa taagattttg ggataatttt 720
   aattttcgtt ttttagtaag gcgcgcggtt ggttagtcgg ggcgggaagt tggttttgcg 780
   ttaaggattc gtagttttag tagtagtaga ttaggtagaa gtgttttttt atttttattt 840
   taagtttttt tagaaattgt cgttagagta gatataaggt tttggttggg gtattatttt 900
   ttgggtgggt ggggagggga gaagagataa ttaaaagtta gttgaattga gaaaggaatt 960
   tggtgaattt ttttattttg tttaatagtt aaagattttt tt 1002
<210> 31
   <211> 1002
   <212> DNA
   <213> Artificial Sequence
<220>
   <223> chemically treated genomic DNA (Homo sapiens)
<400> 31
   gggggggttt ttggttgttg ggtaggatag ggaagtttat taggtttttt ttttagttta      60
   attaattttt aattgttttt tttttttttt ttatttattt aaaaaataat gttttaatta     120
   aaattttgtg tttgttttga cggtagtttt tgggagggtt tggggtaaaa gtgaggagat     180
   atttttgttt ggtttattgt tgttaaggtt gcgagttttt aacgtagagt tagtttttcg     240
   tttcggttgg ttagtcgcgc gttttgttgg ggagcgagag ttggaattat tttagggttt     300
   tattaagcgg atatgaattt tgtttggtag ggaaatgagt atcgaagggt tatttgacga     360
   agttagtgtt ggttgtatta gattcgggtg ttattgcgtt ttgaattgtt atgtaacgtt     420
   ttgttttttg gaaatattgt ttttgattta tttaaagtga taagtaggtt tttttttttt     480
   ttgatcgcgt cgaggtttgt ttaagttttt tggaggtcgt taaatgtaat gagaattgag     540
   gatttagggg gtagtaggat agaggttgtt gtatagatgt tataggggtt tagagagagg     600
   atacggataa gaaattgggt gagggagtaa gatcgtgtat tttttgaaag tattgtaata     660
   gcgaattcgt tgtacgtggt cgaaaggggt tacgttatgg tttttggtga ttttttaggg     720
   taaagtatgt tttggttaat gcgtttgaga tgtagttgtg gaaagattag tgtttgtttt     780
   tttagggagt gagttatttt tttttttttt atttagtggt attgttatta aaattttttt     840
   tcgttttttt gttttgtatt tttaatttat tgaatatttt tttttattgt ggtattttgt     900
   tgattggagg tgaagttaga aattttatta tagatttaat ttgggatttc gatttttaat     960
   atttttatag attatttagt ttatcgattg gttgtatggt gg                       1002
<210> 32
   <211> 276
   <212> DNA
   <213> Artificial Sequence
<220>
   <223> chemically treated genomic DNA (Homo sapiens)
<400> 32
   tatacgattt tattttttta tttaattttt tattcgtgtt ttttttttgg gtttttgtgg      60
   tatttatata gtagtttttg ttttgttgtt ttttggattt ttagttttta ttatatttgg     120
   cggtttttag gaagtttgaa taaatttcga cgcgattaaa ggagaggaaa gtttgtttgt     180
   tattttgggt ggattagaga taatattttt aggggatagg gcgttatatg gtagtttaag     240
   gcgtagtgat attcgagttt gatataatta atatta                               276
<210> 33
   <211> 276
   <212> DNA
   <213> Artificial Sequence
<220>
   <223> chemically treated genomic DNA (Homo sapiens)
<400> 33
   tagtgttggt tgtattagat tcgggtgtta ttgcgttttg aattgttatg taacgttttg      60
   ttttttggaa atattgtttt tgatttattt aaagtgataa gtaggttttt tttttttttg     120
   atcgcgtcga ggtttgttta agttttttgg aggtcgttaa atgtaatgag aattgaggat     180
   ttagggggta gtaggataga ggttgttgta tagatgttat aggggtttag agagaggata     240
   cggataagaa attgggtgag ggagtaagat cgtgta                               276
<210> 34
   <211> 3100
   <212> DNA
   <213> Artificial Sequence
<220>
   <223> chemically treated genomic DNA (Homo sapiens)
<400> 34
   cgtttttttt agaggagaaa agatttggtt tacgtttttt agaggtttta tttggtgaag      60
   aggaggagta gttattgtag cgcggtggat agggtgtttt ggttggcgtc gaagatgtta     120
   gtgatagtgg tcggtacgtt ttttaaattt agtcgcgcgt tattatcgtg cgagttttcg     180
   gtcgtttttt ttttcgtaga gaggataaag gcgtttatta tgtcgcgggg ggcggtttcg     240
   ggtcgaaggt tttcgtagtg ttttaagaat ttgtttagga tgaagttgtt gaagttgcgg     300
   ttgagttgtt cgaattcgcg gaaaacggtg cgtatcgggt tggggaagta ttgtagttag     360
   ggtattacgt ttattaggtt gttcgcgttt atcgtgcgtt cgaatttttc gttgtggttg     420
   agtagtttac ggaattcggg gtcgtcgtgg ttgtagcggt agtcgaaata tacggtattt     480
   atgacgttgg ttacggttac gacggttagc ggtttcgggt cgaggaaggc gtcgttcgcg     540
   ttgtcgcgta ttagtagcgt tattagttcg cgcgtttcgt ttagtacgtg gttttcgagg     600
   atttggcggt tgcgcggttg gcgcgtgaag aagttgcgta ttatgttgtg ggttgcgcgt     660
   cgttgtattt tttagtgttt cgagtagtgg tcgaaagtta tgttgcggtc gtcggatatt     720
   atacggaagg aggcgaaggt cggtcggtcg gcgaaggtcg agttttgttg tattagggtt     780
   tggtggatgg cgcgttcgtt atttagtatt attatggggt agttgtttag gcggatttgg     840
   aaaacgtcgt cgtagcgtcg cgttaggcga gcgaacgaga ggtgagtcgt ttggtttatc     900
   gtcgtcgcgt tttcgattag tggttacgta aacgggttcg gggcgcggga tcggagttgt      960
   cgttttcgtt gttttagtag tcgttggttt atatgtatag tggttagtat cgataggagt    1020
   agtaggagcg tggtttgttg gatggatagc gggtttagcg gttaagggtc gttcgggttg    1080
   aggttggtgt ttatgttggg gatagagagg agaaggcgtg atatttaggg gtgtagagat    1140
   aggagcgggc gttttacgtt tttattttag tttttgggga ttgagtgtcg ttgggtggag    1200
   aggtcggagt tgattttttg gagaaatggt cgaagacgtt tttttttatt tttaatttat    1260
   ttttttttgg agaagagaag ggcgtgtttt ttatttcgtt gtaatttagc gttaaatttt    1320
   tttttttttc gtaaggcgcg taacggtttt tgtaatttgg ggataacgtt gcgggtatcg    1380
   aggcggtggc gtttgatttt ttttaaagta tttattacgt tattcgttat ttgtaataat    1440
   ttatttgaag aggtcgtcgg gtagcgtttc ggtagataga ttgatttgcg gggaggtgcg    1500
   gtttttagtg gcgcgggata gtcggtttcg agaaggaatt gggatttttg tttagggtaa    1560
   gacgttaata ggaattcgta gttcggtttg gatatttgtt gtttttattg gaagttttaa    1620
   tttttattcg agttttttgg cgtcgttagt gttaggagcg tttggattgg gatggggacg    1680
   gagaagggtg tttcgttcgt cgagtttcgt tgtttttggg gatttggtaa agtcgaggtt 1740
   tttttatagc gttgagattg agattggggg tcggtgagtg gcgttaattt ttatgttttt 1800
   gcggttttta taattggagt cgtagaagcg ttcgttttta tttttatttt agagttaaag 1860
   cgcgttattt cgttttttcg ggttttaagg attgggtaga aggagggtag cgcgatttgg 1920
   tagggcgggg tttgcggggg gcggggcgcg tcgtatatta ggtcgttttg attcggattt 1980
   tcgtttaggc gtatcggtcg gtttcggagt tttattagta ggtttttatg ggagcggagg 2040
   tgtttacgtt tttattgtgc ggtaatcgcg ttttattata gttattttta atcgaggtcg 2100
   tacggtgttt ttagaattac gttcggtata atttgtttcg gtcgttgcgt tcgatttggg 2160
   agcgcgtttc gtttcgagcg tacgattttt tttttttttt cgcgatttga gtttgttttt 2220
   cggaaagtta gtgtagtggt agcggtttgt tttatagcgt ttttggttgg ttgtttaaat 2280
   tttattttag gttttcgtaa ataaattgtt attttagtgg aggttttttg gtatgttaaa 2340
   ttttttttag tttttgcggc gagattagga atttttaaaa gtagtcgttt tttcgtttcg 2400
   gggtttcgat agtgtttggg tatataaagg tttttggggc gtagttggtt ttgagatttt 2460
   tcgcgtagag gttattttag gtggcgtcgt tcgttggtgg gcgcggggtc gtttaggttg 2520
   cgatggaagt cgttggcgta gggcggggag ttaagttggg gcgattgggg gggtttgggg 2580
   gagttagcgg ggcgggattc gagcggggcg gagagtggta ggaggaggcg aattttcgcg 2640
   tttcggcgaa ttttatcggg ttgaagtttt tgttgtcgtt ttttttttgc gtgcggagtt 2700
   gggttttgcg tgcgtcgttt ttggaaagtc ggttttagtt atatttttgg gcgttgtttg 2760
   cggtcgtttt tttcgcgttt tttacggtat ttgatacgcg gaggcggcgg tcgagggtgg 2820
   ggtgtcggtt attattattt ttggcgtggg gtgtcggtcg ttattatttt cggttgcgta 2880
   cgtatagtcg cgtttgttag gttagcgagg cggacgtttg ggttattatt cgtaggtcga 2940
   gttcgggcgc gtatcgttat ttcgttgcgt atcggcggtt ttgtggtttt tttgggcgcg 3000
   tttgcggtcg cgaagggggt ttttttttta taagggaagt cggaggcgtt tgggcggggg 3060
   tggttttttt cgtttggcgc gttttttttt atatgttgat 3100
<210> 35
   <211> 3100
   <212> DNA
   <213> Artificial Sequence
<220>
   <223> chemically treated genomic DNA (Homo sapiens)
<400> 35
   attagtatgt aggaaagggc gcgttaggcg ggggaagtta ttttcgttta agcgttttcg 60
   gtttttttta taaagggagg gtttttttcg cgatcgtaag cgcgtttagg aagattatag 120
   agtcgtcggt gcgtagcgag gtggcgatac gcgttcgggt tcggtttgcg ggtggtggtt 180
   taagcgttcg tttcgttggt ttggtaggcg cgattgtgcg tgcgtagtcg agggtggtgg 240
   cggtcggtat tttacgttaa gggtggtggt ggtcggtatt ttattttcgg tcgtcgtttt 300
   cgcgtgttag gtgtcgtgag aagcgcggga ggagcggtcg taggtagcgt ttagggatat 360
   gattggagtc gattttttag aagcggcgta cgtaaagttt agtttcgtac gtaaagggga 420
   ggcgatagta gaaattttaa ttcgataaag ttcgtcggag cgcggagatt cgtttttttt 480
   tgttattttt cgtttcgttc gggtttcgtt tcgttagttt ttttaggttt ttttagtcgt 540
   tttagtttgg tttttcgttt tgcgttaacg gtttttatcg tagtttgggc ggtttcgcgt 600
   ttattagcgg gcggcgttat ttggagtggt ttttacgcgg gaaattttag ggttagttgc 660
   gttttaggag tttttgtgtg tttaagtatt gtcggggttt cggggcgggg gagcggttat 720
   ttttagggat ttttgatttc gtcgtaagaa ttggaaaaaa tttagtatgt taaagagttt 780
   ttattgaggt ggtaatttgt ttgcgagaat ttaagataaa atttaaataa ttaattaggg 840
   gcgttgtgag gtaaatcgtt gttattatat tggtttttcg ggaagtaagt ttaagtcgcg 900
   gagagggaag ggaggtcgtg cgttcggggc ggggcgcgtt tttaagtcga gcgtagcggt 960
   cggggtaggt tgtatcgagc gtggttttgg ggatatcgtg cggtttcgat tggaggtggt 1020
   tgtgatgaag cgcggttatc gtataatgga aacgtgggta ttttcgtttt tatgaaagtt 1080
   tgttggtaga gtttcgaggt cggtcggtgc gtttggacgg gagttcgggt taaagcggtt 1140
   tggtgtgcgg cgcgtttcgt ttttcgtagg tttcgttttg ttaggtcgcg ttgttttttt 1200
   tttatttagt ttttaaaatt cggaggagcg ggatggcgcg ttttgatttt ggagtgggag 1260
   tgggagcgag cgtttttgcg attttagttg tgagagtcgt aagggtatgg gaattgacgt 1320
   tatttatcga tttttagttt taattttaac gttgtgagga aatttcgatt ttgttaggtt 1380
   tttaagggta gcggggttcg gcgagcgagg tatttttttt cgtttttatt ttaatttaag 1440
   cgtttttggt attgacgacg ttaagagatt cgagtgggag ttaaagtttt tagtgagggt 1500
   agtaggtgtt taggtcgggt tgcgggtttt tgttgacgtt ttgttttagg taaaggtttt 1560
   agtttttttt cggagtcggt tgtttcgcgt tattggaaat cgtatttttt cgtaggttag 1620
   tttgtttgtc gaggcgttgt tcggcgattt ttttagatgg attattatag gtagcgggtg 1680
   gcgtggtagg tattttaaag gaaattaagc gttatcgttt cgatgttcgt agcgttgttt 1740
   ttagattgta ggaatcgtta cgcgttttgc ggggagggga agggtttggc gttgggttat 1800
   agcgaggtgg aaatacgttt tttttttttt ttaagggaga gtgggttggg gatgggaagg 1860
   ggcgttttcg gttatttttt tagagagtta gtttcgattt ttttatttaa cggtatttag 1920
   tttttagagg ttggggtagg ggcgtggggc gttcgttttt gtttttgtat ttttgagtgt 1980
   tacgtttttt tttttttgtt tttagtatgg gtattagttt tagttcgaac gatttttggt 2040
   cgttaaattc gttgtttatt tagtagatta cgtttttgtt atttttgtcg gtgttggtta 2100
   ttgtgtatgt gggttagcgg ttgttgaggt aacggaggcg gtagtttcgg ttcgcgtttt 2160
   cgggttcgtt tgcgtggtta ttgatcggaa acgcggcggc ggtgggttag gcggtttatt 2220
   tttcgttcgt tcgtttggcg cggcgttacg gcgacgtttt ttagattcgt ttgggtagtt 2280
   gttttatagt ggtgttgaat ggcgagcgcg ttatttatta ggttttggtg tagtagggtt 2340
   cggttttcgt cgatcggtcg gttttcgttt tttttcgtgt ggtgttcggc ggtcgtagta 2400
   tggttttcgg ttattattcg gagtattgga aggtgtagcg gcgcgtagtt tatagtatga 2460
   tgcgtaattt ttttacgcgt tagtcgcgta gtcgttaagt tttcgagggt tacgtgttga 2520
   gcgaggcgcg cgagttggtg gcgttgttgg tgcgcggtag cgcggacggc gtttttttcg 2580
   attcgaggtc gttgatcgtc gtggtcgtgg ttaacgttat gagtgtcgtg tgtttcggtt 2640
   gtcgttatag ttacgacgat ttcgagtttc gtgagttgtt tagttataac gaagagttcg 2700
   ggcgtacggt gggcgcgggt agtttggtgg acgtgatgtt ttggttgtag tattttttta 2760
   attcggtgcg tatcgttttt cgcgaattcg agtagtttaa tcgtaatttt agtaatttta 2820
   ttttggataa gtttttgagg tattgcgaaa gttttcggtt cggggtcgtt tttcgcgata 2880
   tgatggacgt ttttattttt tttgcggaaa agaaggcggt cggggattcg tacggtggtg 2940
   gcgcgcggtt ggatttggag aacgtatcgg ttattattat tgatattttc ggcgttagtt 3000
   aggatatttt gtttatcgcg ttgtagtggt tgtttttttt ttttattagg taaagttttt 3060
   gggaggcgtg ggttaggttt tttttttttt gaaaaaggcg 3100
<210> 36
   <211> 2500
   <212> DNA
   <213> Artificial Sequence
<220>
   <223> chemically treated genomic DNA (Homo sapiens)
<400> 36
   ttgagttgtt cgaattcgcg gaaaacggtg cgtatcgggt tggggaagta ttgtagttag     60
   ggtattacgt ttattaggtt gttcgcgttt atcgtgcgtt cgaatttttc gttgtggttg    120
   agtagtttac ggaattcggg gtcgtcgtgg ttgtagcggt agtcgaaata tacggtattt    180
   atgacgttgg ttacggttac gacggttagc ggtttcgggt cgaggaaggc gtcgttcgcg    240
   ttgtcgcgta ttagtagcgt tattagttcg cgcgtttcgt ttagtacgtg gttttcgagg    300
   atttggcggt tgcgcggttg gcgcgtgaag aagttgcgta ttatgttgtg ggttgcgcgt    360
   cgttgtattt tttagtgttt cgagtagtgg tcgaaagtta tgttgcggtc gtcggatatt    420
   atacggaagg aggcgaaggt cggtcggtcg gcgaaggtcg agttttgttg tattagggtt    480
   tggtggatgg cgcgttcgtt atttagtatt attatggggt agttgtttag gcggatttgg    540
   aaaacgtcgt cgtagcgtcg cgttaggcga gcgaacgaga ggtgagtcgt ttggtttatc    600
   gtcgtcgcgt tttcgattag tggttacgta aacgggttcg ggggcgcgga tcggagttgt    660
   cgttttcgtt gttttagtag tcgttggttt atatgtatag tggttagtat cgataggagt    720
   agtaggagcg tggtttgttg gatggatagc gggtttagcg gttaagggtc gttcgggttg    780
   aggttggtgt ttatgttggg gatagagagg agaaggcgtg atatttaggg gtgtagagat    840
   aggagcgggc gttttacgtt tttattttag tttttgggga ttgagtgtcg ttgggtggag    900
   aggtcggagt tgattttttg gagaaatggt cgaagacgtt tttttttatt tttaatttat    960
   ttttttttgg agaagagaag gggcgtgttt ttatttcgtt gtaatttagc gttaaatttt   1020
   tttttttttc gtaaggcgcg taacggtttt tgtaatttgg ggataacgtt gcgggtatcg   1080
   aggcggtggc gtttgatttt ttttaaagta tttattacgt tattcgttat ttgtaataat   1140
   ttatttgaag aggtcgtcgg gtagcgtttc ggtagataga ttgatttgcg gggaggtgcg   1200
   gtttttagtg gcgcgggata gtcggtttcg agaaggaatt gggatttttg tttagggtaa   1260
   gacgttaata ggaattcgta gttcggtttg gatatttgtt gtttttattg gaagttttaa   1320
   tttttattcg agttttttgg cgtcgttagt gttaggagcg tttggattgg gatggggacg   1380
   gagaagggtg tttcgttcgt cgagtttcgt tgtttttggg gatttggtaa agtcgaggtt   1440
   tttttatagc gttgagattg agattggggg tcggtgagtg gcgttaattt ttatgttttt   1500
   gcggttttta taattggagt cgtagaagcg ttcgttttta tttttatttt agagttaaag   1560
   cgcgttattt cgttttttcg ggttttaagg attgggtaga aggagggtag cgcgatttgg   1620
   tagggcgggg tttgcggggg gcggggcgcg tcgtatatta ggtcgttttg attcggattt   1680
   tcgtttaggc gtatcggtcg gtttcggagt tttattagta ggtttttatg ggagcggagg   1740
   tgtttacgtt tttattgtgc ggtaatcgcg ttttattata gttattttta atcgaggtcg   1800
   tacggtgttt ttagaattac gttcggtata atttgtttcg gtcgttgcgt tcgatttggg   1860
   agcgcgtttc gtttcgagcg tacgattttt tttttttttt cgcgatttga gtttgttttt   1920
   cggaaagtta gtgtagtggt agcggtttgt tttatagcgt ttttggttgg ttgtttaaat   1980
   tttattttag gttttcgtaa ataaattgtt attttagtgg aggttttttg gtatgttaaa   2040
   ttttttttag tttttgcggc gagattagga atttttaaaa gtagtcgttt tttcgtttcg   2100
   gggtttcgat agtgtttggg tatataaagg tttttggggc gtagttggtt ttgagatttt   2160
   tcgcgtagag gttattttag gtggcgtcgt tcgttggtgg gcgcggggtc gtttaggttg   2220
   cgatggaagt cgttggcgta gggcggggag ttaagttggg gcgattgggg gggtttgggg   2280
   gagttagcgg ggcgggattc gagcggggcg gagagtggta ggaggaggcg aattttcgcg   2340
   tttcggcgaa ttttatcggg ttgaagtttt tgttgtcgtt ttttttttgc gtgcggagtt   2400
   gggttttgcg tgcgtcgttt ttggaaagtc ggttttagtt atatttttgg gcgttgtttg   2460
   cggtcgtttt tttcgcgttt tttacggtat ttgatacgcg                          2500
<210> 37
   <211> 2500
   <212> DNA
   <213> Artificial Sequence
<220>
   <223> chemically treated genomic DNA (Homo sapiens)
<400> 37
   cgcgtgttag gtgtcgtgag aagcgcggga ggagcggtcg taggtagcgt ttagggatat      60
   gattggagtc gattttttag aagcggcgta cgtaaagttt agtttcgtac gtaaagggga      120
   ggcgatagta gaaattttaa ttcgataaag ttcgtcggag cgcggagatt cgtttttttt     180
   tgttattttt cgtttcgttc gggtttcgtt tcgttagttt ttttaggttt ttttagtcgt     240
   tttagtttgg tttttcgttt tgcgttaacg gtttttatcg tagtttgggc ggtttcgcgt     300
   ttattagcgg gcggcgttat ttggagtggt ttttacgcgg gaaattttag ggttagttgc     360
   gttttaggag tttttgtgtg tttaagtatt gtcggggttt cggggcgggg gagcggttat     420
   ttttagggat ttttgatttc gtcgtaagaa ttggaaaaaa tttagtatgt taaagagttt     480
   ttattgaggt ggtaatttgt ttgcgagaat ttaagataaa atttaaataa ttaattaggg     540
   gcgttgtgag gtaaatcgtt gttattatat tggtttttcg ggaagtaagt ttaagtcgcg     600
   gagagggaag ggaggtcgtg cgttcggggc ggggcgcgtt tttaagtcga gcgtagcggt     660
   cggggtaggt tgtatcgagc gtggttttgg ggatatcgtg cggtttcgat tggaggtggt     720
   tgtgatgaag cgcggttatc gtataatgga aacgtgggta ttttcgtttt tatgaaagtt     780
   tgttggtaga gtttcgaggt cggtcggtgc gtttggacgg gagttcgggt taaagcggtt     840
   tggtgtgcgg cgcgtttcgt ttttcgtagg tttcgttttg ttaggtcgcg ttgttttttt     900
   tttatttagt ttttaaaatt cggaggagcg ggatggcgcg ttttgatttt ggagtgggag     960
   tgggagcgag cgtttttgcg attttagttg tgagagtcgt aagggtatgg gaattgacgt    1020
   tatttatcga tttttagttt taattttaac gttgtgagga aatttcgatt ttgttaggtt    1080
   tttaagggta gcggggttcg gcgagcgagg tatttttttt cgtttttatt ttaatttaag    1140
   cgtttttggt attgacgacg ttaagagatt cgagtgggag ttaaagtttt tagtgagggt    1200
   agtaggtgtt taggtcgggt tgcgggtttt tgttgacgtt ttgttttagg taaaggtttt    1260
   agtttttttt cggagtcggt tgtttcgcgt tattggaaat cgtatttttt cgtaggttag    1320
   tttgtttgtc gaggcgttgt tcggcgattt ttttagatgg attattatag gtagcgggtg    1380
   gcgtggtagg tattttaaag gaaattaagc gttatcgttt cgatgttcgt agcgttgttt    1440
   ttagattgta ggaatcgtta cgcgttttgc ggggagggga agggtttggc gttgggttat      1500
   agcgaggtgg aaatacgttt tttttttttt ttaagggaga gtgggttggg gatgggaagg    1560
   ggcgttttcg gttatttttt tagagagtta gtttcgattt ttttatttaa cggtatttag    1620
   tttttagagg ttggggtagg ggcgtggggc gttcgttttt gtttttgtat ttttgagtgt    1680
   tacgtttttt tttttttgtt tttagtatgg gtattagttt tagttcgaac gatttttggt    1740
   cgttaaattc gttgtttatt tagtagatta cgtttttgtt atttttgtcg gtgttggtta    1800
   ttgtgtatgt gggttagcgg ttgttgaggt aacggaggcg gtagtttcgg ttcgcgtttt    1860
   cgggttcgtt tgcgtggtta ttgatcggaa acgcggcggc ggtgggttag gcggtttatt    1920
   tttcgttcgt tcgtttggcg cggcgttacg gcgacgtttt ttagattcgt ttgggtagtt    1980
   gttttatagt ggtgttgaat ggcgagcgcg ttatttatta ggttttggtg tagtagggtt    2040
   cggttttcgt cgatcggtcg gttttcgttt tttttcgtgt ggtgttcggc ggtcgtagta    2100
   tggttttcgg ttattattcg gagtattgga aggtgtagcg gcgcgtagtt tatagtatga    2160
   tgcgtaattt ttttacgcgt tagtcgcgta gtcgttaagt tttcgagggt tacgtgttga    2220
   gcgaggcgcg cgagttggtg gcgttgttgg tgcgcggtag cgcggacggc gtttttttcg   2280
   attcgaggtc gttgatcgtc gtggtcgtgg ttaacgttat gagtgtcgtg tgtttcggtt    2340
   gtcgttatag ttacgacgat ttcgagtttc gtgagttgtt tagttataac gaagagttcg    2400
   ggcgtacggt gggcgcgggt agtttggtgg acgtgatgtt ttggttgtag tattttttta    2460
   attcggtgcg tatcgttttt cgcgaattcg agtagtttaa                          2500
<210> 38
   <211> 216
   <212> DNA
   <213> Artificial Sequence
<220>
   <223> chemically treated genomic DNA (Homo sapiens)
<400> 38
   tacgtttttt aaatttagtc gcgcgttatt atcgtgcgag ttttcggtcg tttttttttt      60
   cgtagagagg ataaaggcgt ttattatgtc gcggggggcg gtttcgggtc gaaggttttc     120
   gtagtgtttt aagaatttgt ttaggatgaa gttgttgaag ttgcggttga gttgttcgaa     180
   ttcgcggaaa acggtgcgta tcgggttggg gaagta                               216
<210> 39
   <211> 216
   <212> DNA
   <213> Artificial Sequence
<220>
   <223> chemically treated genomic DNA (Homo sapiens)
<400> 39
   tattttttta attcggtgcg tatcgttttt cgcgaattcg agtagtttaa tcgtaatttt      60
   agtaatttta ttttggataa gtttttgagg tattgcgaaa gttttcggtt cggggtcgtt     120
   tttcgcgata tgatggacgt ttttattttt tttgcggaaa agaaggcggt cggggattcg     180
   tacggtggtg gcgcgcggtt ggatttggag aacgta                               216
<210> 40
   <211> 9100
   <212> DNA
   <213> Artificial Sequence
<220>
   <223> chemically treated genomic DNA (Homo sapiens)
<400> 40
   atttataatt ttagaagttt ttttaagagg aaaaaaaagg tgttacgtgt tcgttgttta      60
   atagatttaa atattttatt atttttatta tcgttattat tatttttatt gaatattttt     120
   attataagat aggtagatat gtgatatgcg gtgaaggttt tattttattt attattaatt     180
   tttataagta tttttatttt agggtgtttc ggggagggta gaggaggaat atttgggttt     240
   aaaagtcgtt tttggattat ttttagatta gtttggtgga aacggtagtg tgagcgttat     300
   gttatttgag attgcgtttg aaaatttttt ttacgttttt aaattaaagt tattttgagg     360
   tttaagaatg ataattatag gtgaatgttt tatttttttt acgagttagg tttttttttt     420
   gtatgacgta gatcggcggt tgagattcgt tttgaggttt cgttttttat tcggtttatg     480
   tggtttcgcg tagtttacgt agtttttttc gtttttaggt aattagttcg tgggaaattt     540
   attttagaaa agcgtgcgtt atattattat tttgcgattt aataaatttt tttaattgtt     600
   gtttaggaat tagattttag atagattttg ttgtgttcgg tttttttaga aatttttttt     660
   ttagaggaag aagattcggg ttgggaagag tgcgtgatta tggtttcggg tttattgaaa     720
   gatttttttt tataaaagtt tagggttgat attaaacgaa gttatggagt tatgtttata     780
   taaatatttt ttttttattc gaattgtggg gtttggattg tgaaggtttt cgttgtaaga     840
   gcgggtattg gcgaatttta gtgtacgtcg cggtcgagaa cggatgtagg gcgggggatg     900
   gttgagtcgt ttgatttttg tagagaattt gtaggggttt tcggagggta ttttttgcgt     960
   ttaagggagc gttttttttt tttagtattc gggggaattg agggcgacgt gttagtttcg    1020
   tatttaattt tttttttttt tgtaggtata gagttggtcg gcgggggtag aggaggagtt    1080
   gggtttttat tgcgttcgtt taaatttggt taggggttgc gttttttttt tttattttac    1140
   gacgattttt ttttagtttt cgttttttaa tttaatcgtt aattattcgg aacgcgcggg    1200
   cggggagcgg cgaggagggc gagttcgggg ttcgtcgtcg tcgtcgtcgt cgcgcgcgcg    1260
   cgtttaggaa gcggtgtggt tgttattttt tttcgggttt tttttttttt tttttttgtt    1320
   ttgttttttt tttttttttt tttttttttc gtttcgtcgt tcgcgtttag tgtatttatt    1380
   ttttttttac gttattcgtt agcgcgttat gtaaattatc gtcgtcgtcg gtttttattg    1440
   gtcgcggcgc gtttatttaa tggtagttcg ggttcggcgt atggttgttg ggtttcgcgc    1500
   gtcgtcggtt tcgcgtgcgt tttcgtttcg agcgtacggt ttcgggtagg tagcgggtag    1560
   tttatttcgg gttcggcggt ttcggttttt cggttttttt cgcgagttcg cgtttttttc   1620
   gttgttttcg ggtttttttt tggttgtatc gtaatcgttt tttgtaggtt tttttgcgtt   1680
   tttttttttt cgttattggc gtttggtttt tttcgggtat ttgggattag tatatgttta   1740
   gcgtacgcgg cgcgtcgttt tgttagaagt tgtagttttc gagttggagg tcgttgagga   1800
   tcgagcgtag gaggaaggag atagcgcgta gcggcggtcg gcgaggagat agtatatttc   1860
   gggtcgggtt tagcgtatcg ttttcggttt taaaagcgga gttgtaattt ggttacgatt   1920
   gtatttgttt gtatcgtttc gtcgagggcg tttgggttgc ggtggcggcg aagacggcga   1980
   tttcgatcgt cggttttttt ggtaagtggt ttgtgtatta ggagaaattt tttatttgcg   2040
   agtcgaatcg gcgtcgagtg cgtgtgtttt tgtttttttt tgttgtcgtt gtttttattt   2100
   ttttttattt tttttttcgt taggattttt tcgttttcgt tttatttcgt ttgaattttt   2160
   tttcgttttt tttttatttc ggtcgtttat gttttaggtt tttttttcgc ggtgtcggtg   2220
   aattcgttag tcgtttcgat gtatagtatg atgatggaga tcgatttgta ttcgttcggc   2280
   ggcgtttagg tttttacgaa tttttcgggt ttcgtcgggg cgggcggcgg cgggggcgga   2340
   ggcgggggcg gcggcggcgg cgggggcgtt aaggttaatt aggatcgggt taaacggttt   2400
   atgaacgttt ttatggtgtg gtttcgcggg tagcggcgta agatggttta ggagaatttt   2460
   aagatgtata attcggagat tagtaagcgt ttgggggtcg agtggaaggt tatgttcgag   2520
   gtcgagaagc ggtcgtttat cgacgaggtt aagcggttgc gcgcgttgta tatgaaggag   2580
   tattcggatt ataagtatcg gtcgcgtcgt aagattaaga cgttgtttaa gaaggataag   2640
   tattcgttgg tcggcgggtt tttggcggtc ggcgcgggtg gcggcggcgc ggttgtggtt   2700
   atgggcgtgg gcgtgggcgt gggcgcggcg gtcgtgggtt agcgtttgga gagtttaggc   2760
   ggcgcggcgg gcggcggtta cgcgtacgtt aacggttggg ttaacggcgt ttatttcggt   2820
   tcggtggcgg cggcggcggc ggtcgcggtt atgatgtagg aggcgtagtt ggtttacggg   2880
   tagtattcgg gcgcgggcgg cgcgtattcg tacgcgtatt tcgcgtattc gtattcgtat   2940
   tattcgtacg cgtattcgta taattcgtag tttatgtatc gttacgatat gggcgcgttg   3000
   tagtatagtt ttatttttaa ttcgtagggt tatatgagcg cgtcgttttc gggttacggc   3060
   ggtttttttt acggcgtcgc ggtcgtcgtc gtcgtcgttg cgggcggcgc gtattagaat   3120
   tcggtcgtgg cggcggcggc ggcggcggcg gtcgcgtcgt cgggcgtttt gggcgcgttg   3180
   ggttttttgg tgaagtcgga gtttagcggt agttcgttcg ttttagcgta ttcgcgggcg   3240
   tcgtgtttcg gggatttgcg cgagatgatt agtatgtatt tgttcgtcgg cgaggggggc   3300
   gattcggcgg cggtagtagc ggtcgcggcg tagagtcggt tgtattcgtt gtcgtagtat   3360
   tattagggcg cgggcgcggg cgtgaacggt acggtgtttt tgacgtatat ttagcgtttt   3420
   cgggacgtcg gggattttgc ggcggcgatt tacgagttcg cggttcgcgt tcggttttcg   3480
   tttcgtttcg gcgcggcgtg gtttttgtat agacgttttt atatttttgt taaaaggaaa   3540
   atattggaga cgaacgtcgg gtgacgcgtg ttttttattt atttttttcg gagattttgg   3600
   cgatcgtcgg gcgttgatat tagatttggg ttttagattg aatttcggtg tttttttgag   3660
   attttttgta tagtatttat tatttacgga ggaagcggaa agcgtttttt ttgttcgagg   3720
   ggataaaaaa gttaaaacga ggcgagaggc gaagtttatt tttgtatatc ggtcggcgcg   3780
   tttatttttt ttcgcgttgt tttcggacgg cgtcgatcgt cggagtttaa gtgacgcgga   3840
   gttcgtcgta tttgttataa atgtagtaag gtaggtttaa gtatttataa gttttttgta   3900
   gttgttatcg tttttttggg ttggtttgtt aatttatata aagagattat tattattatt   3960
   ttttttttta gacggcggag ttatattttg ggttttgtaa aattttatgt atttgagtat   4020
   ttttattttt ttttttgggt tttgtattat tttttgtaaa tgtattgtga aaaattttat   4080
   tttcggcgtt gtaatgcggg gaggagaagt tagattatgt atatagtttt ttaaaaagtt   4140
   ttttttttaa aaacgaaaaa agatttttta tttaaaatgt ttcgagttaa taaatttaag   4200
   agatagagtt tatttttttt ataaatttgt aatatgttat ttttatgtgt atgttttatg   4260
   agtttaaaat gtaatgagga aatttgatag ggaaattatt tgtatgaatt aaaagtaagg   4320
   gaatttcggg gaatgggagg ataggatttt ttaaggaatt ttttttaatg aaagagaagg   4380
   aagttaaaat ttataggtta ttttgtagag ttgagtgtta atacgggtcg agaaataaaa   4440
   gtattttttg tttcggttgt tttattgcgg acggttgggg ttgttgcgcg ttattttgtt   4500
   gtaagcgggg cgttttttat ttggttgggg gtttgcgtta tagtttggtt tagaggaggg   4560
   aggaggaagg gaagatttta gtggtgggat tttggattag gttatggatg aaggataaag   4620
   attagggtag gttacgggtt ttttaatttt ttagtaatta agatttcgag tagaatttat   4680
   ttaaatgtgt tttaaggaaa tataatcgtt gaattaaaac gtattgtagt cgagtttttt   4740
   tcgtttattt tttgtttttt tttttggttt tttttttttg ggaaaacggg taaaataatt   4800
   gtgttggatt tttatatata tagaaatatc gattattatt ttttttcgcg tgaattggga   4860
   tgtaagttgt taatcgatgt gaacgtaaaa tgttttgttt attatttttg acgagatttt   4920
   gaggttgttt gatgttttaa attttttaat tatattattt tttaggtgtt tattggtata   4980
   ttgtagtttt ttttttgaaa tttaaaaatt tttgtaaaat tttgttttta agtaatttga   5040
   tagtattaaa tattgtattt aaaaattata ttgtagtaaa tatatttaaa aattaattat   5100
   aacgttaaga tgaaattata tttttggaaa aaaaaaatat ttgaagttta gatggaaata   5160
   cgtttatttt agtagtttta ggtttttttt cgttttttta atattttttt ttgttttgga   5220
   gtatggattg ttcgtttaaa agtgagttta tgttataagt ttaatgagaa tcgaatttag   5280
   tttgtattcg agaatagttt taagtataat gttgatttga taattgacgt gtaatttggg   5340
   aagttatttt gataattttg tttaaattat ttattcgtta aagtgattat aaaaaagttt   5400
   aagaatgatg tttattgttt tttaataaga taataaattt tttttttttt tttttttttt   5460
   ttatttttat tttttttagt tatttgattt tttttgaagt agttgttttt ggaagagttt 5520
   gtgcgtttat ggatggttga gtattattac gatttagttc gggataaggg tttttttagt 5580
   ttttttcggg agatgatttg ggaaatttta taatgtttgt tttgttaatt tatcgggatt 5640
   ttgagggttt aatgggattt tgagggtttt ttttgaaata tataaattta aaggattttt 5700
   tttgaggttt tttgattgac gtttattttt agtttggttt ttgtgttttt ttgtgtgtat 5760
   tttggagttt ttgtgtttaa ttgttggtat ttaggttttg gtttaagatt aggatgtggg 5820
   ttttatttta gaggtataga ttatgaaaag ttgagttagt gcgttcggga cgttaggtaa 5880
   gtagttttta tagtttggta ttttattgta ggtgtttcgt gtatagttag ttgaaatagt 5940
   taatgttagg tgttttaatt attttatttt tttgttttgt tgattagaat aatatagaaa 6000
   aaagtaaata taaattttta atgattttat ttaaaaatat tatagggtgg gggtaaggaa 6060
   attagttgag atttatttta ggattgagat tttatttttt ttttttcgtt tttagtagtg 6120
   tcgttttaat ttaaattagt ggagaaaaga ttatagtagg ttttgagtcg attgtgaatt 6180
   cggtgtttgg ttaaggtaat atttatcgta tttacggagt gaaatattat atgatgatag 6240
   ttattatatt atatgacgat tttatttatt ttttaaatta tagggtggtg tttttgtcgt 6300
   ttagtttaat tatgtttgta attcgtgtag ggaaggcgga taggagtttt tgaggtttag 6360
   tgtaaagttt gtattagttt ttgcgggggc gatagtgtga tttcgtaaat ttgttattgc 6420
   gttcgggttt tgttttttaa attgagggta ggagggttta tttgagggtt tttttgtttt 6480
   gttttgtttt gtttttgttt tgttttgttt tttaatttat tcgttttttt tgttgttgag 6540
   attttgacgt tttttttttc ggagaaattt attcgtaacg tattagatgg attagtttta 6600
   aagaagattc gggtatagcg tgatttttag atgcgcgtcg ggagcgtgat tgataggttt 6660
   attaaaatta cgcgtgtggg agaacggtat ttatgttttc gcgttttatt ttttcggtaa 6720
   agattgggcg tcgtagattt ttgttttcgg aaaattttag ttaatttaag aaatttattt 6780
   tcggacgtgt gttcgttttt tttcgaaggt tcgttttagg ttagttcgga ttttagcgtt 6840
   ttttcgattt cgtcgggttt cggtcgattt tcgttttttt tttatttacg ttcggtttgg 6900
   gttcgggggt agcggtttta gaggcgtcgg cggtagaatt taattttcgg tttgagtttt 6960
   tggaaatttt aagtgaaaag tagatttggt tttggcgcgg tttttttttt agcgtgatta 7020
   tttggcgaga ttgaaaggtt aggttagatg gggaaaagtg agcggggtgc gggtttttag 7080
   tattcggcgg ggtggggcgg cgttaggacg gagcgagggt cggcgggggg gttcgcgtgg 7140
   aggtttaggt cgggaggtgg ggacgtaggg attttttatt tagttttttg ttttcgttcg 7200
   agttaggttt cgggattatt tttttttgtt tgagggcgag tttttttttt tttttttttt 7260
   tttcgttttt ttcgtttttt tttttttttt tttttttttt tttttcggtt tttttttttt 7320
   agttgcgcgt tcgtcgggat tacgtttttt tcgttttcgg gagtcgagcg gcgtttgcgg 7380
   ttggttcgga cgtgtttttt tcgggcgggg gtggtcgggt tttttcgggg tttcgggttt 7440
   ttcgggcgta tttttttgtt tgcgtttttt taagtcgttt ttttttcggt tttgttcgcg 7500
   gttgagtttc gtatatcgcg tcgttgtttt tggggtttcg gtatacggcg tttttttttc 7560
   gttttcgtta tttattttta tcgttttttt agtttttgtg tcgtttttat cgttttttga 7620
   ttttcgtatc gagttgcgtt ttcgtcgtcg gtttttttta gcgcggttcg gttttttttt 7680
   tgtttttgaa gttttttcgt cgaggttcgc gcgcggtttt ttttttcgtt ttttttcggt 7740
   ttttgggcgt ttagtcggag gtcggggcgg gatggggtac gagtttcggg gcggttcgcg 7800
   ttttttttat tagttttttt attgtttcgg tttcgttttc gtagtttcgg ggcgggaggg 7860
   cgtaggggtt ttcgggcgtt ttgggagttt atttttttta ggttcgcgtc ggtgttagtc 7920
   gtcggcgggg ggcgggtgga gattaagatg agtgttgcga gggacggggt cgagggtttt 7980
   gtgtagaagg agagtaggat tagtggagag aaagtgttga ttcggggtta gtcgtaagtt 8040
   aaattttttt atatttagtc ggcggaacga gtacggttcg ggtcggacgg taataagtgg 8100
   gcgcgaaggg aggggagggg gaggtcgggt atcggtcggg ttcggcgtga gagaggggag 8160
   ggggagggga cgcggttata gcgttttagg gttttagcgt tatcggggac gggcgtcgag 8220
   gggagcgcgt attagcgatt cgggcgcgga ttggggtttg tagggcgtga ttggttgttt 8280
   tttaggattt tatttttttt ataaatattt gtttttttta tttttcgggg tcgtttcggt 8340
   tgtttttttg gagggaagat tggggagaag gttgtttgcg ttttttgttt tagaggtttt 8400
   gttttagaaa ttgtttttta atttttataa gaaataaaaa aattatagga aggtttggtt 8460
   tttttttcgg acgattgcgt ttttaggttt tttaggaatt ttcgttttat tcgagttttt 8520
   tcgtagtttt ggatttattt ttggggggcg tgtgtgacgt agaatcgggg tataaaatat 8580
   taatgttgtt tgttacgata tcgttttttt tattcgggta gagttgcggt tttcgggcgt 8640
   tgcgcgtggc gttttttatt acggagggaa agtcgcggtt tcgcgggaaa ggagtgaaaa 8700
   tcgagtgtgg gtgaagagga gagggaggtt agggggtaag ggaagaaagt agataaatgt 8760
   atttttttta gtagttaagt tggaaatttg tgttgaaagt ataatagtaa gagtttttat 8820
   atgaaaaata cgtgaagaat tcgagtaagt gatagattta aagacggaaa atgatcgttt 8880
   attttgatag taaaaataaa aagaatagaa ttgatatatt ttcgtttaaa ttatttttta 8940
   tttgtcgata tgaaatatta atatttaaat aaaaataaag gataaagtag tttttaaaaa 9000
   taatataaaa taagtaattt tgagtgaatg atttgtttat agtttatttt gggtgatatg 9060
   ggggcgttgt gatggaaaat gggttttggg tttaaaatta 9100
<210> 41
   <211> 9100
   <212> DNA
   <213> Artificial Sequence
<220>
   <223> chemically treated genomic DNA (Homo sapiens)
<400> 41
   tgattttgaa tttagaattt attttttatt atagcgtttt tatgttattt agggtaagtt     60
   gtggataggt tatttattta gagttgtttg ttttgtgttg tttttaaaaa ttgttttgtt    120
   ttttgttttt gtttgagtgt tggtgtttta tatcggtaga tgaaaagtaa tttagacgaa    180
   aatatgttaa ttttgttttt tttattttta ttattaaaat agacgattat ttttcgtttt    240
   tggatttatt atttgttcgg gttttttacg tgttttttat gtgaaaattt ttgttgttgt    300
   atttttagta taaattttta gtttggttgt tagggaagat gtatttattt gttttttttt    360
   tttgtttttt gatttttttt ttttttttat ttatattcgg tttttatttt tttttcgcgg    420
   agtcgcggtt tttttttcgt gatgaaaaac gttacgcgta acgttcggga gtcgtagttt    480
   tgttcgggtg gagaaagcgg tgtcgtgata aatagtattg atattttata tttcgatttt    540
   gcgttatata cgttttttag aaatagattt agggttgcga agaagttcgg ataaggcgaa    600
   gatttttgga gagtttgggg acgtagtcgt tcgggagagg aattaggttt ttttgtgatt    660
   tttttgtttt ttatggaaat taagaaatag tttttaaaat agagtttttg gggtagagag    720
   cgtaggtaat ttttttttta gttttttttt tagaggagta gtcgaagcgg tttcggggaa    780
   tggaaaaggt aggtgtttat gaaggagatg gagttttggg gggtagttag ttacgttttg    840
   taggttttag ttcgcgttcg ggtcgttggt gcgcgttttt ttcggcgttc gttttcgatg    900
   acgttgaggt tttggggcgt tgtggtcgcg tttttttttt tttttttttt ttacgtcgga    960
   ttcggtcggt attcggtttt tttttttttt tttttcgcgt ttatttgttg tcgttcggtt   1020
   cgagtcgtgt tcgtttcgtc ggttgagtgt aaaagggttt gatttgcggt tgatttcggg   1080
   ttagtatttt ttttttatta attttgtttt ttttttatat aaaattttcg gtttcgtttt   1140
   tcgtagtatt tattttaatt tttattcgtt tttcgtcggc ggttgatatc ggcgcggatt   1200
   tgagggagat gggtttttag ggcgttcggg gatttttgcg ttttttcgtt tcgaggttgc   1260
   ggggacgggg tcgaggtaat gaggggatta gtagggggag cgcgggtcgt ttcgagattc   1320
   gtgttttatt tcgtttcgat tttcgattgg gcgtttaagg gtcggagagg gacggagagg   1380
   ggagtcgcgc gcgggtttcg gcgggggagt tttaaagata aagaggaggt cgggtcgcgt   1440
   tgagagaggt cggcggcgga gacgtagttc ggtgcgggag ttagagggcg gtggagacgg   1500
   tatagggatt ggagaggcgg tgggagtgga tggcgggggc ggggagggaa cgtcgtgtgt   1560
   cgggatttta aggatagcgg cgcggtgtgc ggggtttagt cgcgggtaga gtcggagaaa   1620
   gggcgatttg gagaggcgta ggtagggggg tgcgttcggg aggttcgagg tttcgaggag   1680
   gttcggttat tttcgttcgg aggggatacg ttcggattag tcgtagacgt cgttcggttt   1740
   tcgggggcgg gaggagcgtg gtttcggcgg gcgcgtagtt ggagaggaag ggtcggggag   1800
   ggaggaggga gagagggagg gaagaacggg ggaggcgggg aggaagaggg aggaggagag   1860
   ttcgttttta ggtaagggga ggtgatttcg aggtttggtt cgggcgggag tagaggattg   1920
   ggtgaggggt ttttgcgttt ttatttttcg gtttgggttt ttacgcgagt tttttcgtcg   1980
   gttttcgttt cgttttgacg tcgttttatt tcgtcggata ttgggggttc gtatttcgtt   2040
   tatttttttt tatttggttt ggttttttag tttcgttaga tagttacgtt ggaaagagag   2100
   tcgcgttaag gttaagtttg ttttttattt gaagttttta aggatttaga tcgggggtta   2160
   aattttgtcg tcgacgtttt tggagtcgtt gttttcgggt ttaggtcgag cgtgggtggg   2220
   gagggggcgg gagtcggtcg gagttcgacg gggtcgaggg ggcgttggag ttcgggttgg   2280
   tttgaagcga gttttcgggg gggggcgggt atacgttcgg ggatgggttt tttgaattaa   2340
   ttggaatttt tcggagatag aaatttgcgg cgtttagttt ttgtcggggg aatgaaacgc   2400
   gggagtatga gtgtcgtttt tttatacgcg tgattttaat agatttatta gttacgtttt   2460
   cgacgcgtat ttggagatta cgttgtgttc gagttttttt taaaattaat ttatttagta   2520
   cgttacgggt gggttttttc ggggaagggg acgttaaaat tttaataata aaaggggcgg    2580
   gtgagttgaa aaataaaata aggtaaaaat agaataaaat aaagtaaaaa aatttttaag   2640
   taaatttttt tatttttagt ttggagggta gggttcgagc gtaatggtag atttacgaag   2700
   ttatattgtc gttttcgtag aaattgatgt aaattttgta ttaggtttta aagattttta   2760
   ttcgtttttt ttatacgggt tgtaaatata attggattaa gcgatagaag tattattttg   2820
   tgatttggga agtgaatgaa gtcgttatat aatataataa ttattattat atagtatttt   2880
   atttcgtgaa tacgatgagt gttattttgg ttaagtatcg aatttatagt cggtttaggg   2940
   tttattgtaa tttttttttt attaatttga attggagcga tattgttggg ggcggggaag   3000
   gggggataga attttaattt tgagatgaat tttagttaat ttttttgttt ttattttgtg   3060
   atatttttaa atggagttat taaaaattta tatttgtttt tttttgtgtt gttttaatta   3120
   ataaaataag gaaataaggt ggttggagta tttggtattg gttattttag ttgattgtgt   3180
   acgaagtatt tgtaataaga tgttaaattg taaaagttgt ttgtttggcg tttcgggcgt   3240
   attaatttag ttttttatag tttgtgtttt taaagtgggg tttatatttt aattttgagt   3300
   taagatttag atgttaataa ttggatatag aaattttagg gtatatatag gggagtatag   3360
   gggttagatt gagagtggac gttagttaaa gaattttaga gagagttttt taagtttgta   3420
   tattttagag aaaattttta aggttttatt ggatttttaa ggtttcggtg agttaataga   3480
   ataagtatta taaaattttt taaattattt ttcggagagg attggggagg tttttatttc     3540
   ggattaagtc gtagtggtgt ttagttattt atgggcgtat agattttttt agaaataatt     3600
   gttttagaaa ggattaaata gttaaaagaa ataaaaataa agaaaaagaa aagagggggg     3660
   gggtttatta ttttgttaga aagtagtgga tattattttt gaattttttt gtaattattt     3720
   taacgaatga gtggtttaag taaaattatt aaaatgattt tttaaattat acgttaattg     3780
   ttagattagt attatattta aagttatttt cgaatgtagg ttgaattcgg tttttattaa     3840
   atttatagta taggtttatt tttggacgga tagtttatat tttaggataa ggaagggtgt     3900
   tgagaaagcg aggggaaatt taaggttgtt gaaataaacg tatttttatt tgggttttaa     3960
   gtgttttttt tttttaaaaa tataatttta ttttaacgtt gtgattaatt tttaaatgta     4020
   tttgttatag tataattttt aaatgtaata tttaatgttg ttagattatt tgaagataaa     4080
   gttttataga aatttttaaa ttttaaaaaa aaaattgtaa tgtattaata aatatttaga     4140
   aaataatata attaaaaaat ttaaagtatt aaataatttt aagatttcgt taggaataat     4200
   gaataaggta ttttgcgttt atatcggtta gtaatttgta ttttagttta cgcgggggag     4260
   ggtgatggtc gatatttttg tgtgtgtgag aatttagtat aattattttg ttcgtttttt     4320
   taagagaaag aagttagggg gaggggtaga ggatggacgg agggggttcg gttgtagtac     4380
   gttttggttt agcgattgtg tttttttgaa atatatttag ataaattttg ttcgaaattt     4440
   taattgttgg ggaattggga aattcgtgat ttgttttggt ttttgttttt tatttatggt     4500
   ttggtttagg gttttattat tggggttttt tttttttttt tttttttttg gattaaattg     4560
   tggcgtagat ttttagttag gtggaaggcg tttcgtttgt agtaaggtaa cgcgtagtag     4620
   ttttagtcgt tcgtagtgaa atagtcggag tagaagatat ttttattttt cggttcgtat     4680
   taatatttag ttttataaaa taatttatag gttttaattt tttttttttt tattgaaaaa     4740
   ggttttttga aaaattttgt ttttttattt ttcggggttt ttttattttt agtttatata     4800
   gataattttt ttgttagatt tttttattgt attttgaatt tataaaatat gtatataaaa     4860
   atagtatgtt ataaatttat ggagaaaatg ggttttgttt tttaaatttg ttgattcgaa     4920
   atattttggg tgggggtttt tttttcgttt ttagaagaaa ggttttttag aaaattatgt     4980
   atataatttg attttttttt ttcgtattgt aacgtcgaaa ataaaatttt ttataatgta     5040
   tttataagaa ataatataaa atttaaaaaa aaaaatggaa atgtttagat atataaagtt     5100
   ttataaaatt tagaatataa tttcgtcgtt tgaaggaggg ggtggtggtg gtaatttttt     5160
   tgtataaatt aataaattaa tttaaaagag cggtaataat tataaaaaat ttgtaagtgt     5220
   ttggatttgt tttattatat ttataataaa tgcgacgagt ttcgcgttat ttgggtttcg     5280
   gcggtcggcg tcgttcggaa gtaacgcgga ggaaagtgag cgcgtcggtc ggtatataaa     5340
   agtgggtttc gtttttcgtt tcgttttgat ttttttgttt tttcgagtaa agaaaacgtt     5400
   tttcgttttt ttcgtaggtg ataaatattg tataaaaagt tttaagaaaa tatcgaagtt     5460
   tagtttaaaa tttaagtttg gtgttagcgt tcggcggtcg ttagggtttt cggggaaggt     5520
   gagtggggga tacgcgttat tcggcgttcg tttttagtat tttttttttg ataagaatgt     5580
   gggaacgttt gtataaaagt tacgtcgcgt cggggcgggg cgggagtcgg gcgcgggtcg     5640
   cgagttcgtg ggtcgtcgtc gtagagtttt cggcgtttcg aaggcgttag atgtgcgtta     5700
   ggggtatcgt gtcgtttacg ttcgcgttcg cgttttggta gtgttgcggt agcgagtgta     5760
   gtcggttttg cgtcgcggtc gttgttgtcg tcgtcgggtc gtttttttcg tcggcgggta     5820
   agtatatgtt gattatttcg cgtaggtttt cggggtacgg cgttcgcgag tgcgttgggg     5880
   cgggcgggtt gtcgttgggt ttcgatttta ttagagagtt tagcgcgttt agggcgttcg     5940
   acgacgcggt cgtcgtcgtc gtcgtcgtcg ttacggtcga gttttggtgc gcgtcgttcg     6000
   tagcggcggc ggcggcggtc gcggcgtcgt aggggaggtc gtcgtagttc gagggcgacg     6060
   cgtttatgta gttttgcgag ttggagatgg ggttgtattg tagcgcgttt atgtcgtagc     6120
   ggtgtatggg ttgcgggttg tgcgggtgcg cgtgcgggtg gtgcgggtgc gggtgcgcgg     6180
   ggtgcgcgtg cgggtgcgcg tcgttcgcgt tcgggtgttg ttcgtaggtt agttgcgttt     6240
   tttgtattat ggtcgcggtc gtcgtcgtcg tcgttatcga gtcggggtag gcgtcgttgg     6300
   tttagtcgtt gacgtgcgcg tagtcgtcgt tcgtcgcgtc gtttgggttt tttaggcgtt     6360
   ggtttacggt cgtcgcgttt acgtttacgt ttacgtttat ggttatagtc gcgtcgtcgt     6420
   tattcgcgtc ggtcgttagg agttcgtcgg ttagcgagta tttgtttttt ttgagtagcg     6480
   ttttggtttt gcggcgcggt cggtatttgt aattcgggtg tttttttatg tgtagcgcgc     6540
   gtagtcgttt ggtttcgtcg atgaacggtc gtttttcggt ttcggatatg attttttatt     6600
   cggtttttag gcgtttgttg attttcgagt tgtgtatttt ggggtttttt tgggttattt     6660
   tgcgtcgttg ttcgcgggat tatattatga aggcgtttat gggtcgtttg attcggtttt     6720
   ggttggtttt ggcgttttcg tcgtcgtcgt cgttttcgtt ttcgttttcg tcgtcgttcg     6780
   tttcggcggg gttcgagagg ttcgtggggg tttgggcgtc gtcgggcgag tgtaggtcgg     6840
   tttttattat tatgttgtat atcggggcgg ttggcgggtt tatcggtatc gcgaggagag     6900
   ggtttggagt atagacggtc ggggtgggag ggagacggag aggaatttag acggagtgag     6960
   acgggggcgg gggggtttta gcggagagaa gaatggggag gggtggaggt aacgataata     7020
   aaaaaaggta gaaatatacg tattcggcgt cggttcggtt cgtaggtgga aagttttttt     7080
   tgatgtataa attatttgtt aaagaggtcg acggtcgggg tcgtcgtttt cgtcgttatc     7140
   gtagtttagg cgttttcggc ggagcggtgt aaataggtgt agtcgtggtt aagttgtagt     7200
   ttcgtttttg gggtcgggag cggtgcgttg ggttcggttc ggggtgtgtt gttttttcgt     7260
   cggtcgtcgt tgcgcgttgt tttttttttt ttgcgttcgg tttttagcgg tttttaattc     7320
   ggaggttgta atttttagta gggcggcgcg tcgcgtgcgt tgggtatgtg ttggttttag     7380
   gtgttcgggg gaagttaggc gttagtggcg ggggggggga ggcgtagggg ggtttgtagg    7440
   gggcgattac ggtgtagtta gggaggggtt cggggatagc gggaggagcg cgggttcgcg   7500
   gagagggtcg gagagtcggg gtcgtcgagt tcgggatggg ttgttcgttg tttgttcggg   7560
   gtcgtgcgtt cggagcggag gcgtacgcgg ggtcggcggc gcgcggggtt tagtagttat   7620
   acgtcgggtt cgggttgtta ttaaatgagc gcgtcgcggt taatgggagt cggcggcggc   7680
   ggtgatttgt atggcgcgtt ggcgagtgac gtggggaggg agtagatata ttgggcgcgg   7740
   gcggcggagc ggggaggagg ggaggaagga gggggagtaa agtaggaagg aggggggagg   7800
   aggttcggga gggggtgata gttatatcgt tttttgagcg cgcgcgcgcg gcggcggcgg   7860
   cggcggcgaa tttcgagttc gtttttttcg tcgtttttcg ttcgcgcgtt tcgaatgatt   7920
   aacgattggg ttgaaaagcg aagattaaga aaggatcgtc gtggggtggg gggaggaaac   7980
   gtagtttttg gttaggttta aacgggcgta gtggagattt agtttttttt ttgttttcgt   8040
   cggttaattt tgtgtttgta gggaagggga aagttgagtg cggggttggt acgtcgtttt   8100
   tagttttttc gagtgttgaa aaggaggggc gtttttttgg acgtagggga tatttttcga  8160
   gggtttttgt aggttttttg taaggattag gcggtttagt tatttttcgt tttgtattcg  8220
   ttttcggtcg cggcgtgtat tgaagttcgt tagtgttcgt ttttgtagcg aaggttttta   8280
   tagtttaggt tttataattc gggtaaaggg ggagtatttg tgtgggtatg gttttatagt   8340
   ttcgtttagt attagtttta aatttttgtg gggaggagtt ttttaatgag ttcggggtta   8400
   tagttacgta ttttttttaa ttcggatttt ttttttttgg aaaaggaatt tttaggaagg   8460
   tcgaatataa taagatttat ttggaattta gtttttgaat aatagttgaa aaagtttgtt   8520
   gaatcgtaaa ataatgatat ggcgtacgtt tttttggagt gaatttttta cgagttggtt    8580
   gtttaagaac ggagaaaatt gcgtgaattg cgcggggtta tataaatcga atgaaaggcg   8640
   gggttttagg acgggtttta gtcgtcggtt tgcgttatgt aaaggaaagg tttggttcgt   8700
   ggaaagagta aggtatttat ttgtggttat tatttttaaa ttttaaagtg gttttgattt   8760
   gggaacgtga aagagatttt taaacgtagt tttaagtagt atagcgttta tattatcgtt   8820
   tttattagat tgatttgaaa gtggtttaaa gacgattttt gagtttaagt attttttttt   8880
   tgtttttttc gaggtatttt gagatgagag tgtttgtgga gattggtggt gggtaaagta   8940
   gaatttttat cgtatgttat atgtttgttt gttttgtagt aggaatattt agtaaagatg   9000
   atgataacgg taataggagt aataggatgt ttgagtttgt tgggtagcga atacgtgata   9060
   tttttttttt ttttttaaaa aaatttttga ggttgtgaat                          9100
<210> 42
   <211> 8500
   <212> DNA
   <213> Artificial Sequence
<220>
   <223> chemically treated genomic DNA (Homo sapiens)
<400> 42
   gttatttgag attgcgtttg aaaatttttt ttacgttttt aaattaaagt tattttgagg      60
   tttaagaatg ataattatag gtgaatgttt tatttttttt acgagttagg tttttttttt     120
   gtatgacgta gatcggcggt tgagattcgt tttgaggttt cgttttttat tcggtttatg     180
   tggtttcgcg tagtttacgt agtttttttc gtttttaggt aattagttcg tgggaaattt     240
   attttagaaa agcgtgcgtt atattattat tttgcgattt aataaatttt tttaattgtt     300
   gtttaggaat tagattttag atagattttg ttgtgttcgg tttttttaga aatttttttt   360
   ttagaggaag aagattcggg ttgggaagag tgcgtgatta tggtttcggg tttattgaaa     420
   gatttttttt tataaaagtt tagggttgat attaaacgaa gttatggagt tatgtttata     480
   taaatatttt ttttttattc gaattgtggg gtttggattg tgaaggtttt cgttgtaaga      540
   gcgggtattg gcgaatttta gtgtacgtcg cggtcgagaa cggatgtagg gcgggggatg     600
   gttgagtcgt ttgatttttg tagagaattt gtaggggttt tcggagggta ttttttgcgt       660
   ttaagggagc gttttttttt tttagtattc gggggaattg agggcgacgt gttagtttcg     720
   tatttaattt tttttttttt tgtaggtata gagttggtcg gcgggggtag aggaggagtt     780
   gggtttttat tgcgttcgtt taaatttggt taggggttgc gttttttttt tttattttac     840
   gacgattttt ttttagtttt cgttttttaa tttaatcgtt aattattcgg aacgcgcggg     900
   cggggagcgg cgaggagggc gagttcgggg ttcgtcgtcg tcgtcgtcgt cgcgcgcgcg     960
   cgtttaggaa gcggtgtggt tgttattttt tttcgggttt tttttttttt tttttttgtt     1020
   ttgttttttt tttttttttt tttttttttc gtttcgtcgt tcgcgtttag tgtatttatt    1080
   ttttttttac gttattcgtt agcgcgttat gtaaattatc gtcgtcgtcg gtttttattg     1140
   gtcgcggcgc gtttatttaa tggtagttcg ggttcggcgt atggttgttg ggtttcgcgc    1200
   gtcgtcggtt tcgcgtgcgt tttcgtttcg agcgtacggt ttcgggtagg tagcgggtag    1260
   tttatttcgg gttcggcggt ttcggttttt cggttttttt cgcgagttcg cgtttttttc     1320
   gttgttttcg ggtttttttt tggttgtatc gtaatcgttt tttgtaggtt tttttgcgtt    1380
   tttttttttt cgttattggc gtttggtttt tttcgggtat ttgggattag tatatgttta    1440
   gcgtacgcgg cgcgtcgttt tgttagaagt tgtagttttc gagttggagg tcgttgagga     1500
   tcgagcgtag gaggaaggag atagcgcgta gcggcggtcg gcgaggagat agtatatttc 1560
   gggtcgggtt tagcgtatcg ttttcggttt taaaagcgga gttgtaattt ggttacgatt 1620
   gtatttgttt gtatcgtttc gtcgagggcg tttgggttgc ggtggcggcg aagacggcga 1680
   tttcgatcgt cggttttttt ggtaagtggt ttgtgtatta ggagaaattt tttatttgcg 1740
   agtcgaatcg gcgtcgagtg cgtgtgtttt tgtttttttt tgttgtcgtt gtttttattt 1800
   ttttttattt tttttttcgt taggattttt tcgttttcgt tttatttcgt ttgaattttt 1860
   tttcgttttt tttttatttc ggtcgtttat gttttaggtt tttttttcgc ggtgtcggtg 1920
   aattcgttag tcgtttcgat gtatagtatg atgatggaga tcgatttgta ttcgttcggc 1980
   ggcgtttagg tttttacgaa tttttcgggt ttcgtcgggg cgggcggcgg cgggggcgga 2040
   ggcgggggcg gcggcggcgg cgggggcgtt aaggttaatt aggatcgggt taaacggttt 2100
   atgaacgttt ttatggtgtg gtttcgcggg tagcggcgta agatggttta ggagaatttt 2160
   aagatgtata attcggagat tagtaagcgt ttgggggtcg agtggaaggt tatgttcgag 2220
   gtcgagaagc ggtcgtttat cgacgaggtt aagcggttgc gcgcgttgta tatgaaggag 2280
   tattcggatt ataagtatcg gtcgcgtcgt aagattaaga cgttgtttaa gaaggataag 2340
   tattcgttgg tcggcgggtt tttggcggtc ggcgcgggtg gcggcggcgc ggttgtggtt 2400
   atgggcgtgg gcgtgggcgt gggcgcggcg gtcgtgggtt agcgtttgga gagtttaggc 2460
   ggcgcggcgg gcggcggtta cgcgtacgtt aacggttggg ttaacggcgt ttatttcggt 2520
   tcggtggcgg cggcggcggc ggtcgcggtt atgatgtagg aggcgtagtt ggtttacggg 2580
   tagtattcgg gcgcgggcgg cgcgtattcg tacgcgtatt tcgcgtattc gtattcgtat 2640
   tattcgtacg cgtattcgta taattcgtag tttatgtatc gttacgatat gggcgcgttg 2700
   tagtatagtt ttatttttaa ttcgtagggt tatatgagcg cgtcgttttc gggttacggc 2760
   ggtttttttt acggcgtcgc ggtcgtcgtc gtcgtcgttg cgggcggcgc gtattagaat 2820
   tcggtcgtgg cggcggcggc ggcggcggcg gtcgcgtcgt cgggcgtttt gggcgcgttg 2880
   ggttttttgg tgaagtcgga gtttagcggt agttcgttcg ttttagcgta ttcgcgggcg 2940
   tcgtgtttcg gggatttgcg cgagatgatt agtatgtatt tgttcgtcgg cgaggggggc 3000
   gattcggcgg cggtagtagc ggtcgcggcg tagagtcggt tgtattcgtt gtcgtagtat 3060
   tattagggcg cgggcgcggg cgtgaacggt acggtgtttt tgacgtatat ttagcgtttt 3120
   cgggacgtcg gggattttgc ggcggcgatt tacgagttcg cggttcgcgt tcggttttcg 3180
   tttcgtttcg gcgcggcgtg gtttttgtat agacgttttt atatttttgt taaaaggaaa 3240
   atattggaga cgaacgtcgg gtgacgcgtg ttttttattt atttttttcg gagattttgg 3300
   cgatcgtcgg gcgttgatat tagatttggg ttttagattg aatttcggtg tttttttgag 3360
   attttttgta tagtatttat tatttacgga ggaagcggaa agcgtttttt ttgttcgagg 3420
   ggataaaaaa gttaaaacga ggcgagaggc gaagtttatt tttgtatatc ggtcggcgcg 3480
   tttatttttt ttcgcgttgt tttcggacgg cgtcgatcgt cggagtttaa gtgacgcgga 3540
   gttcgtcgta tttgttataa atgtagtaag gtaggtttaa gtatttataa gttttttgta 3600
   gttgttatcg tttttttggg ttggtttgtt aatttatata aagagattat tattattatt 3660
   ttttttttta gacggcggag ttatattttg ggttttgtaa aattttatgt atttgagtat 3720
   ttttattttt ttttttgggt tttgtattat tttttgtaaa tgtattgtga aaaattttat 3780
   tttcggcgtt gtaatgcggg gaggagaagt tagattatgt atatagtttt ttaaaaagtt 3840
   ttttttttaa aaacgaaaaa agatttttta tttaaaatgt ttcgagttaa taaatttaag 3900
   agatagagtt tatttttttt ataaatttgt aatatgttat ttttatgtgt atgttttatg 3960
   agtttaaaat gtaatgagga aatttgatag ggaaattatt tgtatgaatt aaaagtaagg 4020
   gaatttcggg gaatgggagg ataggatttt ttaaggaatt ttttttaatg aaagagaagg 4080
   aagttaaaat ttataggtta ttttgtagag ttgagtgtta atacgggtcg agaaataaaa 4140
   gtattttttg tttcggttgt tttattgcgg acggttgggg ttgttgcgcg ttattttgtt 4200
   gtaagcgggg cgttttttat ttggttgggg gtttgcgtta tagtttggtt tagaggaggg 4260
   aggaggaagg gaagatttta gtggtgggat tttggattag gttatggatg aaggataaag 4320
   attagggtag gttacgggtt ttttaatttt ttagtaatta agatttcgag tagaatttat 4380
   ttaaatgtgt tttaaggaaa tataatcgtt gaattaaaac gtattgtagt cgagtttttt 4440
   tcgtttattt tttgtttttt tttttggttt tttttttttg ggaaaacggg taaaataatt 4500
   gtgttggatt tttatatata tagaaatatc gattattatt ttttttcgcg tgaattggga 4560
   tgtaagttgt taatcgatgt gaacgtaaaa tgttttgttt attatttttg acgagatttt 4620
   gaggttgttt gatgttttaa attttttaat tatattattt tttaggtgtt tattggtata 4680
   ttgtagtttt ttttttgaaa tttaaaaatt tttgtaaaat tttgttttta agtaatttga 4740
   tagtattaaa tattgtattt aaaaattata ttgtagtaaa tatatttaaa aattaattat 4800
   aacgttaaga tgaaattata tttttggaaa aaaaaaatat ttgaagttta gatggaaata 4860
   cgtttatttt agtagtttta ggtttttttt cgttttttta atattttttt ttgttttgga 4920
   gtatggattg ttcgtttaaa agtgagttta tgttataagt ttaatgagaa tcgaatttag 4980
   tttgtattcg agaatagttt taagtataat gttgatttga taattgacgt gtaatttggg 5040
   aagttatttt gataattttg tttaaattat ttattcgtta aagtgattat aaaaaagttt 5100
   aagaatgatg tttattgttt tttaataaga taataaattt tttttttttt tttttttttt 5160
   ttatttttat tttttttagt tatttgattt tttttgaagt agttgttttt ggaagagttt 5220
   gtgcgtttat ggatggttga gtattattac gatttagttc gggataaggg tttttttagt 5280
   ttttttcggg agatgatttg ggaaatttta taatgtttgt tttgttaatt tatcgggatt 5340
   ttgagggttt aatgggattt tgagggtttt ttttgaaata tataaattta aaggattttt 5400
   tttgaggttt tttgattgac gtttattttt agtttggttt ttgtgttttt ttgtgtgtat 5460
   tttggagttt ttgtgtttaa ttgttggtat ttaggttttg gtttaagatt aggatgtggg 5520
   ttttatttta gaggtataga ttatgaaaag ttgagttagt gcgttcggga cgttaggtaa 5580
   gtagttttta tagtttggta ttttattgta ggtgtttcgt gtatagttag ttgaaatagt 5640
   taatgttagg tgttttaatt attttatttt tttgttttgt tgattagaat aatatagaaa 5700
   aaagtaaata taaattttta atgattttat ttaaaaatat tatagggtgg gggtaaggaa 5760
   attagttgag atttatttta ggattgagat tttatttttt ttttttcgtt tttagtagtg 5820
   tcgttttaat ttaaattagt ggagaaaaga ttatagtagg ttttgagtcg attgtgaatt 5880
   cggtgtttgg ttaaggtaat atttatcgta tttacggagt gaaatattat atgatgatag 5940
   ttattatatt atatgacgat tttatttatt ttttaaatta tagggtggtg tttttgtcgt 6000
   ttagtttaat tatgtttgta attcgtgtag ggaaggcgga taggagtttt tgaggtttag 6060
   tgtaaagttt gtattagttt ttgcgggggc gatagtgtga tttcgtaaat ttgttattgc 6120
   gttcgggttt tgttttttaa attgagggta ggagggttta tttgagggtt tttttgtttt 6180
   gttttgtttt gtttttgttt tgttttgttt tttaatttat tcgttttttt tgttgttgag 6240
   attttgacgt tttttttttc ggagaaattt attcgtaacg tattagatgg attagtttta 6300
   aagaagattc gggtatagcg tgatttttag atgcgcgtcg ggagcgtgat tgataggttt 6360
   attaaaatta cgcgtgtggg agaacggtat ttatgttttc gcgttttatt ttttcggtaa 6420
   agattgggcg tcgtagattt ttgttttcgg aaaattttag ttaatttaag aaatttattt 6480
   tcggacgtgt gttcgttttt tttcgaaggt tcgttttagg ttagttcgga ttttagcgtt 6540
   ttttcgattt cgtcgggttt cggtcgattt tcgttttttt tttatttacg ttcggtttgg 6600
   gttcgggggt agcggtttta gaggcgtcgg cggtagaatt taattttcgg tttgagtttt 6660
   tggaaatttt aagtgaaaag tagatttggt tttggcgcgg tttttttttt agcgtgatta 6720
   tttggcgaga ttgaaaggtt aggttagatg gggaaaagtg agcggggtgc gggtttttag 6780
   tattcggcgg ggtggggcgg cgttaggacg gagcgagggt cggcgggggg gttcgcgtgg 6840
   aggtttaggt cgggaggtgg ggacgtaggg attttttatt tagttttttg ttttcgttcg 6900
   agttaggttt cgggattatt tttttttgtt tgagggcgag tttttttttt tttttttttt 6960
   tttcgttttt ttcgtttttt tttttttttt tttttttttt tttttcggtt tttttttttt 7020
   agttgcgcgt tcgtcgggat tacgtttttt tcgttttcgg gagtcgagcg gcgtttgcgg 7080
   ttggttcgga cgtgtttttt tcgggcgggg gtggtcgggt tttttcgggg tttcgggttt 7140
   ttcgggcgta tttttttgtt tgcgtttttt taagtcgttt ttttttcggt tttgttcgcg 7200
   gttgagtttc gtatatcgcg tcgttgtttt tggggtttcg gtatacggcg tttttttttc 7260
   gttttcgtta tttattttta tcgttttttt agtttttgtg tcgtttttat cgttttttga 7320
   ttttcgtatc gagttgcgtt ttcgtcgtcg gtttttttta gcgcggttcg gttttttttt 7380
   tgtttttgaa gttttttcgt cgaggttcgc gcgcggtttt ttttttcgtt ttttttcggt 7440
   ttttgggcgt ttagtcggag gtcggggcgg gatggggtac gagtttcggg gcggttcgcg 7500
   ttttttttat tagttttttt attgtttcgg tttcgttttc gtagtttcgg ggcgggaggg 7560
   cgtaggggtt ttcgggcgtt ttgggagttt atttttttta ggttcgcgtc ggtgttagtc 7620
   gtcggcgggg ggcgggtgga gattaagatg agtgttgcga gggacggggt cgagggtttt 7680
   gtgtagaagg agagtaggat tagtggagag aaagtgttga ttcggggtta gtcgtaagtt 7740
   aaattttttt atatttagtc ggcggaacga gtacggttcg ggtcggacgg taataagtgg 7800
   gcgcgaaggg agggggaggg gaggtcgggt atcggtcggg ttcggcgtga gagaggggag 7860
   ggggagggga cgcggttata gcgttttagg gttttagcgt tatcggggac gggcgtcgag 7920
   gggagcgcgt attagcgatt cgggcgcgga ttggggtttg tagggcgtga ttggttgttt 7980
   tttaggattt tatttttttt ataaatattt gtttttttta tttttcgggg tcgtttcggt 8040
   tgtttttttg gagggaagat tggggagaag gttgtttgcg ttttttgttt tagaggtttt 8100
   gttttagaaa ttgtttttta atttttataa gaaataaaaa aattatagga aggtttggtt 8160
   tttttttcgg acgattgcgt ttttaggttt tttaggaatt ttcgttttat tcgagttttt 8220
   tcgtagtttt ggatttattt ttggggggcg tgtgtgacgt agaatcgggg tataaaatat 8280
   taatgttgtt tgttacgata tcgttttttt tattcgggta gagttgcggt tttcgggcgt 8340
   tgcgcgtggc gttttttatt acggagggaa agtcgcggtt tcgcgggaaa ggagtgaaaa 8400
   tcgagtgtgg gtgaagagga gagggaggtt agggggtaag ggaagaaagt agataaatgt 8460
   atttttttta gtagttaagt tggaaatttg tgttgaaagt 8500
<210> 43
   <211> 8500
   <212> DNA
   <213> Artificial Sequence
<220>
   <223> chemically treated genomic DNA (Homo sapiens)
<400> 43
   atttttagta taaattttta gtttggttgt tagggaagat gtatttattt gttttttttt        60
   tttgtttttt gatttttttt ttttttttat ttatattcgg tttttatttt tttttcgcgg       120
   agtcgcggtt tttttttcgt gatgaaaaac gttacgcgta acgttcggga gtcgtagttt 180
   tgttcgggtg gagaaagcgg tgtcgtgata aatagtattg atattttata tttcgatttt 240
   gcgttatata cgttttttag aaatagattt agggttgcga agaagttcgg ataaggcgaa 300
   gatttttgga gagtttgggg acgtagtcgt tcgggagagg aattaggttt ttttgtgatt 360
   tttttgtttt ttatggaaat taagaaatag tttttaaaat agagtttttg gggtagagag 420
   cgtaggtaat ttttttttta gttttttttt tagaggagta gtcgaagcgg tttcggggaa 480
   tggaaaaggt aggtgtttat gaaggagatg gagttttggg gggtagttag ttacgttttg 540
   taggttttag ttcgcgttcg ggtcgttggt gcgcgttttt ttcggcgttc gttttcgatg 600
   acgttgaggt tttggggcgt tgtggtcgcg tttttttttt tttttttttt ttacgtcgga 660
   ttcggtcggt attcggtttt tttttttttt tttttcgcgt ttatttgttg tcgttcggtt 720
   cgagtcgtgt tcgtttcgtc ggttgagtgt aaaagggttt gatttgcggt tgatttcggg 780
   ttagtatttt ttttttatta attttgtttt ttttttatat aaaattttcg gtttcgtttt 840
   tcgtagtatt tattttaatt tttattcgtt tttcgtcggc ggttgatatc ggcgcggatt 900
   tgagggagat gggtttttag ggcgttcggg gatttttgcg ttttttcgtt tcgaggttgc 960
   ggggacgggg tcgaggtaat gaggggatta gtagggggag cgcgggtcgt ttcgagattc 1020
   gtgttttatt tcgtttcgat tttcgattgg gcgtttaagg gtcggagagg gacggagagg 1080
   ggagtcgcgc gcgggtttcg gcgggggagt tttaaagata aagaggaggt cgggtcgcgt 1140
   tgagagaggt cggcggcgga gacgtagttc ggtgcgggag ttagagggcg gtggagacgg 1200
   tatagggatt ggagaggcgg tgggagtgga tggcgggggc ggggagggaa cgtcgtgtgt 1260
   cgggatttta aggatagcgg cgcggtgtgc ggggtttagt cgcgggtaga gtcggagaaa 1320
   gggcgatttg gagaggcgta ggtagggggg tgcgttcggg aggttcgagg tttcgaggag 1380
   gttcggttat tttcgttcgg aggggatacg ttcggattag tcgtagacgt cgttcggttt 1440
   tcgggggcgg gaggagcgtg gtttcggcgg gcgcgtagtt ggagaggaag ggtcggggag 1500
   ggaggaggga gagagggagg gaagaacggg ggaggcgggg aggaagaggg aggaggagag 1560
   ttcgttttta ggtaagggga ggtgatttcg aggtttggtt cgggcgggag tagaggattg 1620
   ggtgaggggt ttttgcgttt ttatttttcg gtttgggttt ttacgcgagt tttttcgtcg 1680
   gttttcgttt cgttttgacg tcgttttatt tcgtcggata ttgggggttc gtatttcgtt 1740
   tatttttttt tatttggttt ggttttttag tttcgttaga tagttacgtt ggaaagagag 1800
   tcgcgttaag gttaagtttg ttttttattt gaagttttta aggatttaga tcgggggtta 1860
   aattttgtcg tcgacgtttt tggagtcgtt gttttcgggt ttaggtcgag cgtgggtggg 1920
   gagggggcgg gagtcggtcg gagttcgacg gggtcgaggg ggcgttggag ttcgggttgg 1980
   tttgaagcga gttttcgggg gggggcgggt atacgttcgg ggatgggttt tttgaattaa 2040
   ttggaatttt tcggagatag aaatttgcgg cgtttagttt ttgtcggggg aatgaaacgc 2100
   gggagtatga gtgtcgtttt tttatacgcg tgattttaat agatttatta gttacgtttt 2160
   cgacgcgtat ttggagatta cgttgtgttc gagttttttt taaaattaat ttatttagta 2220
   cgttacgggt gggttttttc ggggaagggg acgttaaaat tttaataata aaaggggcgg 2280
   gtgagttgaa aaataaaata aggtaaaaat agaataaaat aaagtaaaaa aatttttaag 2340
   taaatttttt tatttttagt ttggagggta gggttcgagc gtaatggtag atttacgaag 2400
   ttatattgtc gttttcgtag aaattgatgt aaattttgta ttaggtttta aagattttta 2460
   ttcgtttttt ttatacgggt tgtaaatata attggattaa gcgatagaag tattattttg 2520
   tgatttggga agtgaatgaa gtcgttatat aatataataa ttattattat atagtatttt 2580
   atttcgtgaa tacgatgagt gttattttgg ttaagtatcg aatttatagt cggtttaggg 2640
   tttattgtaa tttttttttt attaatttga attggagcga tattgttggg ggcggggaag 2700
   gggggataga attttaattt tgagatgaat tttagttaat ttttttgttt ttattttgtg 2760
   atatttttaa atggagttat taaaaattta tatttgtttt tttttgtgtt gttttaatta 2820
   ataaaataag gaaataaggt ggttggagta tttggtattg gttattttag ttgattgtgt 2880
   acgaagtatt tgtaataaga tgttaaattg taaaagttgt ttgtttggcg tttcgggcgt 2940
   attaatttag ttttttatag tttgtgtttt taaagtgggg tttatatttt aattttgagt 3000
   taagatttag atgttaataa ttggatatag aaattttagg gtatatatag gggagtatag 3060
   gggttagatt gagagtggac gttagttaaa gaattttaga gagagttttt taagtttgta 3120
   tattttagag aaaattttta aggttttatt ggatttttaa ggtttcggtg agttaataga 3180
   ataagtatta taaaattttt taaattattt ttcggagagg attggggagg tttttatttc 3240
   ggattaagtc gtagtggtgt ttagttattt atgggcgtat agattttttt agaaataatt 3300
   gttttagaaa ggattaaata gttaaaagaa ataaaaataa agaaaaagaa aagagggggg 3360
   gggtttatta ttttgttaga aagtagtgga tattattttt gaattttttt gtaattattt 3420
   taacgaatga gtggtttaag taaaattatt aaaatgattt tttaaattat acgttaattg 3480
   ttagattagt attatattta aagttatttt cgaatgtagg ttgaattcgg tttttattaa 3540
   atttatagta taggtttatt tttggacgga tagtttatat tttaggataa ggaagggtgt 3600
   tgagaaagcg aggggaaatt taaggttgtt gaaataaacg tatttttatt tgggttttaa 3660
   gtgttttttt tttttaaaaa tataatttta ttttaacgtt gtgattaatt tttaaatgta 3720
   tttgttatag tataattttt aaatgtaata tttaatgttg ttagattatt tgaagataaa 3780
   gttttataga aatttttaaa ttttaaaaaa aaaattgtaa tgtattaata aatatttaga 3840
   aaataatata attaaaaaat ttaaagtatt aaataatttt aagatttcgt taggaataat 3900
   gaataaggta ttttgcgttt atatcggtta gtaatttgta ttttagttta cgcgggggag 3960
   ggtgatggtc gatatttttg tgtgtgtgag aatttagtat aattattttg ttcgtttttt 4020
   taagagaaag aagttagggg gaggggtaga ggatggacgg agggggttcg gttgtagtac 4080
   gttttggttt agcgattgtg tttttttgaa atatatttag ataaattttg ttcgaaattt 4140
   taattgttgg ggaattggga aattcgtgat ttgttttggt ttttgttttt tatttatggt 4200
   ttggtttagg gttttattat tggggttttt tttttttttt tttttttttg gattaaattg 4260
   tggcgtagat ttttagttag gtggaaggcg tttcgtttgt agtaaggtaa cgcgtagtag 4320
   ttttagtcgt tcgtagtgaa atagtcggag tagaagatat ttttattttt cggttcgtat 4380
   taatatttag ttttataaaa taatttatag gttttaattt tttttttttt tattgaaaaa 4440
   ggttttttga aaaattttgt ttttttattt ttcggggttt ttttattttt agtttatata 4500
   gataattttt ttgttagatt tttttattgt attttgaatt tataaaatat gtatataaaa 4560
   atagtatgtt ataaatttat ggagaaaatg ggttttgttt tttaaatttg ttgattcgaa 4620
   atattttggg tggggggttt tttttcgttt ttagaagaaa ggttttttag aaaattatgt 4680
   atataatttg attttttttt ttcgtattgt aacgtcgaaa ataaaatttt ttataatgta 4740
   tttataagaa ataatataaa atttaaaaaa aaaaatggaa atgtttagat atataaagtt 4800
   ttataaaatt tagaatataa tttcgtcgtt tgaaggaggg ggtggtggtg gtaatttttt 4860
   tgtataaatt aataaattaa tttaaaagag cggtaataat tataaaaaat ttgtaagtgt 4920
   ttggatttgt tttattatat ttataataaa tgcgacgagt ttcgcgttat ttgggtttcg 4980
   gcggtcggcg tcgttcggaa gtaacgcgga ggaaagtgag cgcgtcggtc ggtatataaa 5040
   agtgggtttc gtttttcgtt tcgttttgat ttttttgttt tttcgagtaa agaaaacgtt 5100
   tttcgttttt ttcgtaggtg ataaatattg tataaaaagt tttaagaaaa tatcgaagtt 5160
   tagtttaaaa tttaagtttg gtgttagcgt tcggcggtcg ttagggtttt cggggaaggt 5220
   gagtggggga tacgcgttat tcggcgttcg tttttagtat tttttttttg ataagaatgt 5280
   gggaacgttt gtataaaagt tacgtcgcgt cggggcgggg cgggagtcgg gcgcgggtcg 5340
   cgagttcgtg ggtcgtcgtc gtagagtttt cggcgtttcg aaggcgttag atgtgcgtta 5400
   ggggtatcgt gtcgtttacg ttcgcgttcg cgttttggta gtgttgcggt agcgagtgta 5460
   gtcggttttg cgtcgcggtc gttgttgtcg tcgtcgggtc gtttttttcg tcggcgggta 5520
   agtatatgtt gattatttcg cgtaggtttt cggggtacgg cgttcgcgag tgcgttgggg 5580
   cgggcgggtt gtcgttgggt ttcgatttta ttagagagtt tagcgcgttt agggcgttcg 5640
   acgacgcggt cgtcgtcgtc gtcgtcgtcg ttacggtcga gttttggtgc gcgtcgttcg 5700
   tagcggcggc ggcggcggtc gcggcgtcgt aggggaggtc gtcgtagttc gagggcgacg 5760
   cgtttatgta gttttgcgag ttggagatgg ggttgtattg tagcgcgttt atgtcgtagc 5820
   ggtgtatggg ttgcgggttg tgcgggtgcg cgtgcgggtg gtgcgggtgc gggtgcgcgg 5880
   ggtgcgcgtg cgggtgcgcg tcgttcgcgt tcgggtgttg ttcgtaggtt agttgcgttt 5940
   tttgtattat ggtcgcggtc gtcgtcgtcg tcgttatcga gtcggggtag gcgtcgttgg 6000
   tttagtcgtt gacgtgcgcg tagtcgtcgt tcgtcgcgtc gtttgggttt tttaggcgtt 6060
   ggtttacggt cgtcgcgttt acgtttacgt ttacgtttat ggttatagtc gcgtcgtcgt 6120
   tattcgcgtc ggtcgttagg agttcgtcgg ttagcgagta tttgtttttt ttgagtagcg 6180
   ttttggtttt gcggcgcggt cggtatttgt aattcgggtg tttttttatg tgtagcgcgc 6240
   gtagtcgttt ggtttcgtcg atgaacggtc gtttttcggt ttcggatatg attttttatt 6300
   cggtttttag gcgtttgttg attttcgagt tgtgtatttt ggggtttttt tgggttattt 6360
   tgcgtcgttg ttcgcgggat tatattatga aggcgtttat gggtcgtttg attcggtttt 6420
   ggttggtttt ggcgttttcg tcgtcgtcgt cgttttcgtt ttcgttttcg tcgtcgttcg 6480
   tttcggcggg gttcgagagg ttcgtggggg tttgggcgtc gtcgggcgag tgtaggtcgg 6540
   tttttattat tatgttgtat atcggggcgg ttggcgggtt tatcggtatc gcgaggagag 6600
   ggtttggagt atagacggtc ggggtgggag ggagacggag aggaatttag acggagtgag 6660
   acgggggcgg gggggtttta gcggagagaa gaatggggag gggtggaggt aacgataata 6720
   aaaaaaggta gaaatatacg tattcggcgt cggttcggtt cgtaggtgga aagttttttt 6780
   tgatgtataa attatttgtt aaagaggtcg acggtcgggg tcgtcgtttt cgtcgttatc 6840
   gtagtttagg cgttttcggc ggagcggtgt aaataggtgt agtcgtggtt aagttgtagt 6900
   ttcgtttttg gggtcgggag cggtgcgttg ggttcggttc ggggtgtgtt gttttttcgt 6960
   cggtcgtcgt tgcgcgttgt tttttttttt ttgcgttcgg tttttagcgg tttttaattc 7020
   ggaggttgta atttttagta gggcggcgcg tcgcgtgcgt tgggtatgtg ttggttttag 7080
   gtgttcgggg gaagttaggc gttagtggcg ggggggggga ggcgtagggg ggtttgtagg 7140
   gggcgattac ggtgtagtta gggaggggtt cggggatagc gggaggagcg cgggttcgcg 7200
   gagagggtcg gagagtcggg gtcgtcgagt tcgggatggg ttgttcgttg tttgttcggg 7260
   gtcgtgcgtt cggagcggag gcgtacgcgg ggtcggcggc gcgcggggtt tagtagttat 7320
   acgtcgggtt cgggttgtta ttaaatgagc gcgtcgcggt taatgggagt cggcggcggc 7380
   ggtgatttgt atggcgcgtt ggcgagtgac gtggggaggg agtagatata ttgggcgcgg 7440
   gcggcggagc ggggaggagg ggaggaagga gggggagtaa agtaggaagg aggggggagg 7500
   aggttcggga gggggtgata gttatatcgt tttttgagcg cgcgcgcgcg gcggcggcgg 7560
   cggcggcgaa tttcgagttc gtttttttcg tcgtttttcg ttcgcgcgtt tcgaatgatt 7620
   aacgattggg ttgaaaagcg aagattaaga aaggatcgtc gtggggtggg gggaggaaac 7680
   gtagtttttg gttaggttta aacgggcgta gtggagattt agtttttttt ttgttttcgt 7740
   cggttaattt tgtgtttgta gggaagggga aagttgagtg cggggttggt acgtcgtttt 7800
   tagttttttc gagtgttgaa aaggaggggc gtttttttgg acgtagggga tatttttcga 7860
   gggtttttgt aggttttttg taaggattag gcggtttagt tatttttcgt tttgtattcg 7920
   ttttcggtcg cggcgtgtat tgaagttcgt tagtgttcgt ttttgtagcg aaggttttta    7980
   tagtttaggt tttataattc gggtaaaggg ggagtatttg tgtgggtatg gttttatagt    8040
   ttcgtttagt attagtttta aatttttgtg gggaggagtt ttttaatgag ttcggggtta    8100
   tagttacgta ttttttttaa ttcggatttt ttttttttgg aaaaggaatt tttaggaagg    8160
   tcgaatataa taagatttat ttggaattta gtttttgaat aatagttgaa aaagtttgtt    8220
   gaatcgtaaa ataatgatat ggcgtacgtt tttttggagt gaatttttta cgagttggtt    8280
   gtttaagaac ggagaaaatt gcgtgaattg cgcggggtta tataaatcga atgaaaggcg    8340
   gggttttagg acgggtttta gtcgtcggtt tgcgttatgt aaaggaaagg tttggttcgt    8400
   ggaaagagta aggtatttat ttgtggttat tatttttaaa ttttaaagtg gttttgattt    8460
   gggaacgtga aagagatttt taaacgtagt tttaagtagt                           8500
<210> 44
   <211> 263
   <212> DNA
   <213> Artificial Sequence
<220>
   <223> chemically treated genomic DNA (Homo sapiens)
<400> 44
   tatagtattt attatttacg gaggaagcgg aaagcgtttt ttttgttcga ggggataaaa      60
   aagttaaaac gaggcgagag gcgaagttta tttttgtata tcggtcggcg cgtttatttt     120
   ttttcgcgtt gttttcggac ggcgtcgatc gtcggagttt aagtgacgcg gagttcgtcg     180
   tatttgttat aaatgtagta aggtaggttt aagtatttat aagttttttg tagttgttat     240
   cgtttttttg ggttggtttg tta                                             263
<210> 45
   <211> 263
   <212> DNA
   <213> Artificial Sequence
<220>
   <223> chemically treated genomic DNA (Homo sapiens)
<400> 45
   taataaatta atttaaaaga gcggtaataa ttataaaaaa tttgtaagtg tttggatttg      60
   ttttattata tttataataa atgcgacgag tttcgcgtta tttgggtttc ggcggtcggc     120
   gtcgttcgga agtaacgcgg aggaaagtga gcgcgtcggt cggtatataa aagtgggttt     180
   cgtttttcgt ttcgttttga tttttttgtt ttttcgagta aagaaaacgt ttttcgtttt     240
   tttcgtaggt gataaatatt gta                                             263
<210> 46
   <211> 3300
   <212> DNA
   <213> Artificial Sequence
<220>
   <223> chemically treated genomic DNA (Homo sapiens)
<400> 46
   gatattgtgt gggagttttt tggggtttga tttgtgtttt tttattagtt ttttttattt      60
   tagttagttt tagatttaag ggaataggat aaatgttttt agtttgattt tgttagtttg     120
   tttttataat taagataaaa tgaaaaaata gtaatagaag taaatataaa aaagttttaa     180
   atggaaattt gttgggattt tgttttattt taaattttgg gttagtgttg tttttttttt     240
   ttttttttaa gtattttttt tttaagtttg gttgtttttt tatatttgtt ttttgttttt     300
   ggtttttaat ttggtttttg taaaatttat tgaagttttt tttttttttt tttttttagt      360
   tttataaaat ttgttggtag ggtttgtggg agttgtgggg ttttgttaat tttggggttg     420
   ggttgggagt tgagtgtagg tggttggagg gtaatggagt aagtgatagt tttaggtgtt     480
   tttgggtttt tggttgggag ggaagagttg aggattttgg gttgtattgt gtagatggag     540
   atgtgttttt agagtttttg ggtaggttta gggattttgt gatttgtttt ggtttagtgg      600
   tgtgattttg tgggttttgg tggttttgat tgtttgtagg ggaggggtgt gttatttttg       660
   gttgttttag gatgtgttgt ttgaattttt ttttttttgt gggtagtgtt tgttatattt     720
   tttgggtttt ttggaaattt taattatttt attaggatta ttggggtttg gggtagtttt     780
   ttgggagttg tgtggatgag ttttggttag gtgggagtga agagttttgg tgattgttag     840
   tttttttgtt tttgtattgt tgggaaagga tggtgtttta atagataggt agtaagttta     900
   ttgaggttga agaatgaagt ttttttaggg ttggtgggtt ttgagattaa tgagtttaat     960
   aagaaaatta agtagggtta tgatttagaa attaaagaat atggggttag gagaagaggt    1020
   tagttatgat ataggtagtg gtttgttttt ttttgatttt ttaaaggtgt ttttttttta   1080
   gttttttttg attttaggga tttgtaattt gggatttaaa gtttttgaga gggtagaatg   1140
   attatttaaa atgaattgtg ttagtgagtg ttgttgtttt ttttaatggt tttggaagat    1200
   tattgttttg ggttttaggt gtgttttgtt ttgtgtttat ttttgatatt atttttaaga    1260
   gtgttattgt ggagtgggtg ttttttggat gttgtggggt tatttgggtt tggtaggtta   1320
   tttgggtttg gtagattttg ggtgggtttt gagggttgag ttaggtttaa gagggtgggt    1380
   ggggttgtgg ggtattgata ggttgagggt agggaattga tttggtttat gtagattttt    1440
   gtgttgtttt ttttttgttt attgtagtta ttggttgatt tttgatttgt tttttgtttt   1500
   ttggggttgt ttatttaatt tattagtttt ttaaattttt tttgggattt tagtggtgag    1560
   tatttgttgt tggtttttta gggtaggtag tattgttgtt ttaggtgtgt ggagttgggg    1620
   agaaaaaaat tgaatatgtg tgttatttag ggtttttaga taagtttttt tgtgtgtata    1680
   tatattttga attggagttt agaaggttgg atttgtgttt tggggttgag ttttttattt   1740
   tttgttttta gtttggaaga ttttagtttg tttgaaggtg gtgtttgatt tgttggtgga    1800
   tttggtgggt gtttgttttt gatatttggg gatgaggtgg gagtgttttg agtgttggga    1860
   agttttgttt tgtgagtttt tggtagtgat atgtgggagt gttgggtttg gtatttttga    1920
   tgtttttgag gttttgggtg gttgttttgt ttttttgggg gtttttattt tttaagtggt    1980
   tttaaatgat tgaggatggg ttttttgtgt tgttgtagtt gttatttttt ttgggaaagg   2040
   ttagtgattt tggatgtgta ggttttgaaa attaaggttt agatttatat tgagtgttta    2100
   aagattgtat ttgaattttt tttgttggtt attgagggag agaaaggaat gtttagtttt    2160
   atttatattt atagaaagaa gtgttgaggg tgggggaaat gtagttttgt tgggtgagtt    2220
   gggataggtt ttttgtttgt tttttggttg ttgttttttt ttagttgaat ggggagtgat    2280
   ttgttttggg tgtggttttt ggggttgaag attgaggtgt agttttattt ttggtttggt    2340
   agtggtttgg aagagagagg gaaaggagga atatttattt ggttaagaga tgttgttaga    2400
   gtttttaaag ttggtggggg atggaggttg gggttttgga tttttagagt gtttgtagtt    2460
   gggtttttgg gttttttggt gtggggaatt tgattttgta gtttttgtta gggtgtggtg    2520
   gggttgtaag aagggaggaa ggagtgggat tagagttttt gagggtgtgg gtggtgtgta    2580
   tttagttttg tttttttgtg tgttttgttg tttgtttgtt ttttttgttg tattgggtgg   2640
   ttttggtttt tgtgtttgtt tgtttttgat gtttttgtgt ggttttgtgt tgtttgtggt    2700
   tagttttgtt ttgttttgta aggagtgtgt tgttttttta attgaaatgg gattaagatt    2760
   ttagggtttt tttgtttttt tgttttgagg tgataggatt ttagttaata gattgaaaat    2820
   gtgaaaagta tttgttgttt tttgttttaa gtgtttttgt ggaaggatta tttatttgga    2880
   aaagtagaat aaattttgtt gttgtttttt ttttggaggt gtgtttattt tagagttttt   2940
   agatttattg gtatttgtta tatttttttt tgggttttat aggtaggatt ttaagaatga   3000
   tatgggggta tttgggtgat ttgtgggttg taggttttta agaggttgtt tttttgtgta    3060
   tggaagtgtt gtagttttat tttagtggaa gtaatattaa attttatttt agaggtttgt    3120
   tttgtaaaga atttagagtt tattatattt gatattattt taaaataata tttgtttgtt    3180
   ttaagaagtt gggagaaatt gataaaagaa aagttgaaat ttatgttttt tatttttatt    3240
   ttaggagata attttgatta atagtttggt ttgtttttta tttttatttt ttaaaaataa    3300
<210> 47
   <211> 3300
   <212> DNA
   <213> Artificial Sequence
<220>
   <223> chemically treated genomic DNA (Homo sapiens)
<400> 47
   ttgtttttaa ggaatggggg tagggagtaa attaaattgt tgattaggat tattttttag      60
   agtagaaatg ggaagtatgg attttaattt tttttttatt gatttttttt aattttttaa     120
   aataagtaag tgttatttta aaatagtgtt aaatatagta aattttggat tttttgtaaa     180
   ataaattttt aagataaggt ttgatattgt ttttgttaaa ataaagttat gatatttttg     240
   tgtatgaaaa aatggttttt tgaggatttg taatttatag attatttagg tgtttttata     300
   ttatttttga aattttattt gtaaagttta ggaggaagta tagtgaatgt tagtgggttt     360
   aggagttttg gggtgagtat gtttttggaa ggaaaatggt aatagaattt attttgtttt     420
   tttaagtaaa tgattttttt atagaggtat ttgggataag aggtgatggg tattttttat     480
   gtttttgatt tattggttaa gattttatta ttttaaaata aggagatgag gaaattttgg     540
   gattttgatt ttattttgat tgagaaaata atatgttttt tgtgaagtga aatggggttg     600
   gttgtgggta gtgtggaatt atgtgaaggt gttgggggtg ggtgggtgtg gggattaggg     660
   ttatttggtg tagtaaaggg ggtaagtagg tggtagggtg tgtggggaga taaggttgag     720
   tgtatgttgt ttgtgttttt gaaggtttta gttttatttt tttttttttt tttgtagttt     780
   tgttgtgttt tggtgaaggt tatagggtta ggttttttgt attgaggagt ttagaagttt     840
   ggttgtgggt gttttgaaga tttgggattt tggtttttgt tttttgttag ttttagggat     900
   tttggtggtg ttttttagtt gggtagatgt tttttttttt tttttttttt ttaagttgtt     960
   gttaggttgg gggtgaggtt gtattttagt ttttagtttt gaaggttgtg tttggggtgg    1020
   gttgtttttt atttagttaa gaggaagtag tggttggggg gtaggtgagg aatttgtttt    1080
   ggtttatttg gtaagattgt gtttttttta tttttggtgt ttttttttgt gaatgtgaat    1140
   ggaattaagt gttttttttt tttttttaat ggttggtgag gagaatttaa atgtggtttt    1200
   tgggtgtttg gtatgggttt ggattttggt ttttagagtt tgtgtgttta aggttgttag    1260
   ttttttttga aggaagtgat agttgtggtg gtgtgggagg tttatttttg gttgtttaaa    1320
   gttatttaag gaataaaagt ttttgagaga gtaaaatgat tatttgaaat tttggaagtg    1380
   ttgggggtgt tgggtttaat gtttttatgt gttattgttg gggatttgtg aggtgaagtt    1440
   ttttagtgtt taaggtgttt ttgttttgtt tttaggtatt aggaatagat atttattagg    1500
   tttattagtg ggttagatgt tgtttttggg taggttggga ttttttgaat tgggagtgga    1560
   gagtaagggg tttgattttg ggatgtgagt ttggtttttt ggattttggt ttagggtgtg    1620
   tgtgtgtgtg gaggggttta tttgggggtt ttgggtagta tatgtgtttg gttttttttt    1680
   ttttgatttt gtatgtttgg agtggtaata ttgtttgttt tagaaggtta gtggtgagtg    1740
   tttgttatta gggttttagg gagggtttgg aaaattgatg agttaagtga gtgattttag    1800
   gggatagagg gtgagttgag agttggttaa tggttgtggt gggtggggag aagatgatgt    1860
   ggggatttgt gtgggttggg ttaatttttt atttttgatt tgttgatgtt ttgtggtttt    1920
   gtttgttttt ttaagtttgg tttagttttt agggtttgtt tgaagtttat tgagtttgag    1980
   tggtttattg agtttgagtg gttttgtagt gtttaggagg tgtttgtttt gtggtggtgt    2040
   ttttggaggt ggtgttggag gtaggtatgg gataggatgt gtttggggtt tggggtggtg    2100
   gttttttagg gttgttgggg agggtggtag tatttgttgg tgtagtttgt tttggatggt    2160
   tgttttgttt ttttgggggt tttgaatttt aagttgtaga tttttgaggt tggaggaggt    2220
   taggagaagg gtgtttttgg aggattggga ggagatgggt tgttgtttgt gttgtggttg    2280
   attttttttt ttgattttgt gttttttaat ttttgagtta tgattttgtt tggttttttt    2340
   attgggttta ttgattttaa gatttgttgg ttttgaaggg gttttatttt ttagttttgg    2400
   tgaatttgtt gtttgtttat taaaatgtta tttttttttg gtggtgtggg ggtgggagga    2460
   ttggtagttg ttgaggtttt ttgtttttat ttggttaggg tttgtttatg tggtttttga    2520
   ggggttattt taggttttaa tagttttggt agaatgattg gagtttttga ggaatttggg    2580
   gaatgtggtg gatgttgttt gtgagggaaa agaggtttag gtggtgtatt ttagggtagt    2640
   taaaagtggt gtgttttttt tttgtgggta gttaggattg ttaggatttg tggggttata    2700
   ttgttgggtt agagtaggtt gtggggtttt tggatttgtt tgggggtttt gggagtgtgt    2760
   ttttatttgt gtggtgtgat ttagggtttt tggttttttt tttttagttg agggtttagg    2820
   agtgtttgga gttgttgttt gttttattgt tttttgattg tttgtgtttg gtttttggtt    2880
   tagttttgag gttggtaggg ttttgtggtt tttatagatt ttattaatga gttttgtagg    2940
   attgagaaga aggaaggaaa gggaatttta atgggttttg taggaattgg gttgggggtt    3000
   gaaagtggag agtgggtgtg ggaaggtggt tgggtttagg gaaggggtgt ttggagaggg    3060
   aaggggaagg taatattaat ttggaattta gagtagggta ggattttggt agatttttgt    3120
   ttggggtttt tttgtgtttg tttttattgt tgtttttttg ttttgtttta attgtgggag    3180
   tgggttggtg ggattggatt gggggtgttt attttgtttt tttggatttg gggttggttg    3240
   gggtggagga ggttggtggg gaggtgtggg ttggattttg ggaagttttt gtgtggtgtt    3300
<210> 48
   <211> 2700
   <212> DNA
   <213> Artificial Sequence
<220>
   <223> chemically treated genomic DNA (Homo sapiens)
<400> 48
   ggtttttaat ttggtttttg taaaatttat tgaagttttt tttttttttt tttttttagt      60
   tttataaaat ttgttggtag ggtttgtggg agttgtgggg ttttgttaat tttggggttg    120
   ggttgggagt tgagtgtagg tggttggagg gtaatggagt aagtgatagt tttaggtgtt    180
   tttgggtttt tggttgggag ggaagagttg aggattttgg gttgtattgt gtagatggag    240
   atgtgttttt agagtttttg ggtaggttta gggattttgt gatttgtttt ggtttagtgg     300
   tgtgattttg tgggttttgg tggttttgat tgtttgtagg ggaggggtgt gttatttttg     360
   gttgttttag gatgtgttgt ttgaattttt ttttttttgt gggtagtgtt tgttatattt    420
   tttgggtttt ttggaaattt taattatttt attaggatta ttggggtttg gggtagtttt    480
   ttgggagttg tgtggatgag ttttggttag gtgggagtga agagttttgg tgattgttag    540
   tttttttgtt tttgtattgt tgggaaagga tggtgtttta atagataggt agtaagttta    600
   ttgaggttga agaatgaagt ttttttaggg ttggtgggtt ttgagattaa tgagtttaat   660
   aagaaaatta agtagggtta tgatttagaa attaaagaat atggggttag gagaagaggt    720
   tagttatgat ataggtagtg gtttgttttt ttttgatttt ttaaaggtgt ttttttttta    780
   gttttttttg attttaggga tttgtaattt gggatttaaa gtttttgaga gggtagaatg   840
   attatttaaa atgaattgtg ttagtgagtg ttgttgtttt ttttaatggt tttggaagat    900
   tattgttttg ggttttaggt gtgttttgtt ttgtgtttat ttttgatatt atttttaaga    960
   gtgttattgt ggagtgggtg ttttttggat gttgtggggt tatttgggtt tggtaggtta  1020
   tttgggtttg gtagattttg ggtgggtttt gagggttgag ttaggtttaa gagggtgggt   1080
   ggggttgtgg ggtattgata ggttgagggt agggaattga tttggtttat gtagattttt   1140
   gtgttgtttt ttttttgttt attgtagtta ttggttgatt tttgatttgt tttttgtttt   1200
   ttggggttgt ttatttaatt tattagtttt ttaaattttt tttgggattt tagtggtgag  1260
   tatttgttgt tggtttttta gggtaggtag tattgttgtt ttaggtgtgt ggagttgggg   1320
   agaaaaaaat tgaatatgtg tgttatttag ggtttttaga taagtttttt tgtgtgtata   1380
   tatattttga attggagttt agaaggttgg atttgtgttt tggggttgag ttttttattt  1440
   tttgttttta gtttggaaga ttttagtttg tttgaaggtg gtgtttgatt tgttggtgga  1500
   tttggtgggt gtttgttttt gatatttggg gatgaggtgg gagtgttttg agtgttggga   1560
   agttttgttt tgtgagtttt tggtagtgat atgtgggagt gttgggtttg gtatttttga   1620
   tgtttttgag gttttgggtg gttgttttgt ttttttgggg gtttttattt tttaagtggt   1680
   tttaaatgat tgaggatggg ttttttgtgt tgttgtagtt gttatttttt ttgggaaagg   1740
   ttagtgattt tggatgtgta ggttttgaaa attaaggttt agatttatat tgagtgttta   1800
   aagattgtat ttgaattttt tttgttggtt attgagggag agaaaggaat gtttagtttt   1860
   atttatattt atagaaagaa gtgttgaggg tgggggaaat gtagttttgt tgggtgagtt   1920
   gggataggtt ttttgtttgt tttttggttg ttgttttttt ttagttgaat ggggagtgat   1980
   ttgttttggg tgtggttttt ggggttgaag attgaggtgt agttttattt ttggtttggt   2040
   agtggtttgg aagagagagg gaaaggagga atatttattt ggttaagaga tgttgttaga   2100
   gtttttaaag ttggtggggg atggaggttg gggttttgga tttttagagt gtttgtagtt   2160
   gggtttttgg gttttttggt gtggggaatt tgattttgta gtttttgtta gggtgtggtg   2220
   gggttgtaag aagggaggaa ggagtgggat tagagttttt gagggtgtgg gtggtgtgta   2280
   tttagttttg tttttttgtg tgttttgttg tttgtttgtt ttttttgttg tattgggtgg   2340
   ttttggtttt tgtgtttgtt tgtttttgat gtttttgtgt ggttttgtgt tgtttgtggt   2400
   tagttttgtt ttgttttgta aggagtgtgt tgttttttta attgaaatgg gattaagatt   2460
   ttagggtttt tttgtttttt tgttttgagg tgataggatt ttagttaata gattgaaaat   2520
   gtgaaaagta tttgttgttt tttgttttaa gtgtttttgt ggaaggatta tttatttgga   2580
   aaagtagaat aaattttgtt gttgtttttt ttttggaggt gtgtttattt tagagttttt   2640
   agatttattg gtatttgtta tatttttttt tgggttttat aggtaggatt ttaagaatga   2700
<210> 49
   <211> 2700
   <212> DNA
   <213> Artificial Sequence
<220>
   <223> chemically treated genomic DNA (Homo sapiens)
<400> 49
   ttatttttga aattttattt gtaaagttta ggaggaagta tagtgaatgt tagtgggttt 60
   aggagttttg gggtgagtat gtttttggaa ggaaaatggt aatagaattt attttgtttt 120
   tttaagtaaa tgattttttt atagaggtat ttgggataag aggtgatggg tattttttat 180
   gtttttgatt tattggttaa gattttatta ttttaaaata aggagatgag gaaattttgg 240
   gattttgatt ttattttgat tgagaaaata atatgttttt tgtgaagtga aatggggttg 300
   gttgtgggta gtgtggaatt atgtgaaggt gttgggggtg ggtgggtgtg gggattaggg 360
   ttatttggtg tagtaaaggg ggtaagtagg tggtagggtg tgtggggaga taaggttgag 420
   tgtatgttgt ttgtgttttt gaaggtttta gttttatttt tttttttttt tttgtagttt 480
   tgttgtgttt tggtgaaggt tatagggtta ggttttttgt attgaggagt ttagaagttt 540
   ggttgtgggt gttttgaaga tttgggattt tggtttttgt tttttgttag ttttagggat 600
   tttggtggtg ttttttagtt gggtagatgt tttttttttt tttttttttt ttaagttgtt 660
   gttaggttgg gggtgaggtt gtattttagt ttttagtttt gaaggttgtg tttggggtgg 720
   gttgtttttt atttagttaa gaggaagtag tggttggggg gtaggtgagg aatttgtttt 780
   ggtttatttg gtaagattgt gtttttttta tttttggtgt ttttttttgt gaatgtgaat 840
   ggaattaagt gttttttttt tttttttaat ggttggtgag gagaatttaa atgtggtttt 900
   tgggtgtttg gtatgggttt ggattttggt ttttagagtt tgtgtgttta aggttgttag 960
   ttttttttga aggaagtgat agttgtggtg gtgtgggagg tttatttttg gttgtttaaa 1020
   gttatttaag gaataaaagt ttttgagaga gtaaaatgat tatttgaaat tttggaagtg 1080
   ttgggggtgt tgggtttaat gtttttatgt gttattgttg gggatttgtg aggtgaagtt 1140
   ttttagtgtt taaggtgttt ttgttttgtt tttaggtatt aggaatagat atttattagg 1200
   tttattagtg ggttagatgt tgtttttggg taggttggga ttttttgaat tgggagtgga 1260
   gagtaagggg tttgattttg ggatgtgagt ttggtttttt ggattttggt ttagggtgtg 1320
   tgtgtgtgtg gaggggttta tttgggggtt ttgggtagta tatgtgtttg gttttttttt 1380
   ttttgatttt gtatgtttgg agtggtaata ttgtttgttt tagaaggtta gtggtgagtg 1440
   tttgttatta gggttttagg gagggtttgg aaaattgatg agttaagtga gtgattttag 1500
   gggatagagg gtgagttgag agttggttaa tggttgtggt gggtggggag aagatgatgt 1560
   ggggatttgt gtgggttggg ttaatttttt atttttgatt tgttgatgtt ttgtggtttt 1620
   gtttgttttt ttaagtttgg tttagttttt agggtttgtt tgaagtttat tgagtttgag 1680
   tggtttattg agtttgagtg gttttgtagt gtttaggagg tgtttgtttt gtggtggtgt 1740
   ttttggaggt ggtgttggag gtaggtatgg gataggatgt gtttggggtt tggggtggtg 1800
   gttttttagg gttgttgggg agggtggtag tatttgttgg tgtagtttgt tttggatggt 1860
   tgttttgttt ttttgggggt tttgaatttt aagttgtaga tttttgaggt tggaggaggt 1920
   taggagaagg gtgtttttgg aggattggga ggagatgggt tgttgtttgt gttgtggttg 1980
   attttttttt ttgattttgt gttttttaat ttttgagtta tgattttgtt tggttttttt 2040
   attgggttta ttgattttaa gatttgttgg ttttgaaggg gttttatttt ttagttttgg 2100
   tgaatttgtt gtttgtttat taaaatgtta tttttttttg gtggtgtggg ggtgggagga 2160
   ttggtagttg ttgaggtttt ttgtttttat ttggttaggg tttgtttatg tggtttttga 2220
   ggggttattt taggttttaa tagttttggt agaatgattg gagtttttga ggaatttggg 2280
   gaatgtggtg gatgttgttt gtgagggaaa agaggtttag gtggtgtatt ttagggtagt 2340
   taaaagtggt gtgttttttt tttgtgggta gttaggattg ttaggatttg tggggttata 2400
   ttgttgggtt agagtaggtt gtggggtttt tggatttgtt tgggggtttt gggagtgtgt 2460
   ttttatttgt gtggtgtgat ttagggtttt tggttttttt tttttagttg agggtttagg 2520
   agtgtttgga gttgttgttt gttttattgt tttttgattg tttgtgtttg gtttttggtt 2580
   tagttttgag gttggtaggg ttttgtggtt tttatagatt ttattaatga gttttgtagg 2640
   attgagaaga aggaaggaaa gggaatttta atgggttttg taggaattgg gttgggggtt 2700
<210> 50
   <211> 280
   <212> DNA
   <213> Artificial Sequence
<220>
   <223> chemically treated genomic DNA (Homo sapiens)
<400> 50
   tagagttttt gagggtgtgg gtggtgtgta tttagttttg tttttttgtg tgttttgttg 60
   tttgtttgtt ttttttgttg tattgggtgg ttttggtttt tgtgtttgtt tgtttttgat 120
   gtttttgtgt ggttttgtgt tgtttgtggt tagttttgtt ttgttttgta aggagtgtgt 180
   tgttttttta attgaaatgg gattaagatt ttagggtttt tttgtttttt tgttttgagg 240
   tgataggatt ttagttaata gattgaaaat gtgaaaagta 280
<210> 51
   <211> 280
   <212> DNA
   <213> Artificial Sequence
<220>
   <223> chemically treated genomic DNA (Homo sapiens)
<400> 51 <223> chemically treated genomic DNA (Homo sapiens)
<400> 52
<210> 53
   <211> 1968
   <212> DNA
   <213> Artificial Sequence
<220>
   <223> chemically treated genomic DNA (Homo sapiens)
<400> 53
   agaatttttg tgtttaaatt ttatttatgt ttgggagaag atttgtttag aatatatgtt       60
   ttatagtgtt ttttaggatg gagtgggagt gagaaaggag tgttttagag gagggattag    120
   agtgtatttg gttttgttgt tggatagtta gtgatttttt atgatgttta ggtgataagt    180
   gtgatgttaa gttttgtagt taattggtaa aagtatagat atttttaaag agtatttgat    240
   ttgtttaaga gaatgattat ttttgtttat gaaataagta gtaaaaaata tatagtaaga    300
   ttttagggat ttttttgttt tatttttggt tttgttaatt gaagtaattt ttgattttgg   360
   gttatttttt ttattattat tattttaggt ttttgtaaaa tgaagaggtt gaagtgaaat    420
   taaattattt ttttatatat tttttgtagt tttaagtggg gtgagaaaat gtaaaaggtt    480
   tgtttggggg tttttttatg gggatgtggg ggtggggtag gagaaagagg ggataaggag   540
   atattaaagt tagtttttag tgtgtttttt gtaataaggt gttatgtaaa ttttaagggt    600
   ttggaggaaa tttttttttt gataatgttg attttggaga gtaggtggtg ttttaaagga    660
   gggagatgag taagttttaa gggggtgggt gaaagggaaa tgttatttgt tttttattga    720
   tttaatgtgg taggagtgta ggtgtaagaa atttttggat tttttttttg gttaattttt    780
   gtgttttatt aatttttgtt ttaagttttt atttttgttt ttaatttttg ttgaaaattt    840
   attgatgtgg tgaggttagg ttaataatat tgtaatttta ggttttttgg gttttatagg   900
   aatgttttga ttttttttta gttttagtgg tttttagttg atggtgtggt taaatgttag    960
   ggttatttgt ggtgggttgt tgtgtggggt gtagtgggag gaggttgtgt gggggtgttg   1020
   tggggaggat atgttggttt gtgtttagta tttgggtttg agatttagtg tttttggttt    1080
   ttgtggtttt tgtgtttttt ttgtgttaat ttattgtttt gttgtgtttg tttttttagt    1140
   gggtttggtg gttgggggag gatagtgatt gagtttgaag tttggagggt ttttgtgttt    1200
   ttggggtttt tattattgtt ttttttagtt tttaagtggt tattttgaag aatgatgaag    1260
   ggttttattt gtgtagattt gtgggatgat tttattaaga aggaaaaggg ggaaaagaaa    1320
   ttataattat tgagggttta gttttggtta gattttgtgt tagatttttt taatttgata    1380
   ttattgattt tttataattt tttattttgt agtttaggaa gtaggttttg agagattgtt    1440
   tagaaaaaat agtatttgga tttgaatttg aggttgtttt atatttattt tggtatggtt    1500
   tttagtagaa ttgagttgga gtggttttga tgtaaatgaa tttagatttt agttgtttgt    1560
   gatggaagtt aattttagtt tttttaagtt tataggtttt ttttagaaga agttaatttg    1620
   attttttgga atatgaattt agtaggtttt tattttagaa ttgtttttaa tttattttat    1680
   atttttttat attttagttt ttggtatttt ttttgattat attttttatt ttttttttat    1740
   atttgtaagt ttttttttgt agaatttgtt ttgaggagta aaggatattt ttttatgggg    1800
   aatagaattt tagaattttt ttttgtattt agtgagaaag attaaagttt ggtataggaa    1860
   ataatttaaa gataaattta tatattgtat agaatatagg aagtttgggt tttgaaaata    1920
   taaaagatat taaattattt ttggagtatt tgtgaagttt tggaaaat                 1968
<210> 54
   <211> 1368
   <212> DNA
   <213> Artificial Sequence
<220>
   <223> chemically treated genomic DNA (Homo sapiens)
<400> 54
   ggaggtagtt ttgagatgag agtttgttga atttgtgttt tgggagatta agttggtttt      60
   ttttggaaaa aatttgtaag tttgggaaag ttgggattgg tttttattat aagtagttgg     120
   gatttaagtt tatttgtatt agggttattt tagtttagtt ttgttgggaa ttgtattggg     180
   atgggtgtgg gatggttttg aatttagatt tgggtgttgt tttttttagg tagttttttg     240
   gagtttgttt tttggattat aaaatgaaga gttgtgaaga gttaataatg ttaagttgga     300
   agggtttgat atggaatttg attaaggttg gatttttaat aattgtgatt tttttttttt     360
   tttttttttt ttggtaaaat tattttatga atttatgtaa gtagggtttt ttgttatttt     420
   ttggagtagt tgtttgaggg ttggaaggag tagtgataga aattttagag atgtagagat     480
   tttttgaatt ttgaatttga ttattgtttt tttttgattg ttgaatttgt tggagaagtg     540
   ggtgtgatag ggtgatgagt taatgtggag ggagtgtgga ggttgtggaa gttgggggtg     600
   ttgggtttta ggtttggatg ttgagtgtgg attggtgtgt tttttttata gtgtttttgt     660
   gtggtttttt tttgttgtgt tttgtatggt gatttgttgt gggtagtttt ggtgtttggt     720
   tatgttgttg gttgaggatt gttagagttg gggggagatt aaagtatttt tatggggttt     780
   aaagagtttg ggattgtagt gttgttagtt tggttttgtt gtgttaataa atttttggtg     840
   aaagttggga gtaggagtgg aggtttgggg tgggggttgg tggggtgtgg gggttggttg     900
   gagggagggt ttgggggttt tttgtatttg tgtttttgtt gtgttaagtt agtgggaagt     960
   aagtgatatt ttttttttat ttgttttttt ggaatttgtt tatttttttt ttttagaata    1020
   ttgtttgttt tttggggtta gtattattaa agagaagatt ttttttaggt ttttaaaatt    1080
   tatgtggtgt tttgttataa ggagtgtgtt ggaagttggt tttggtattt ttttgttttt    1140
   tttttttttt gttttgtttt tatatttttg tggagaaatt tttgagtagg ttttttgtat    1200
   ttttttattt tatttagaat tataaaaaat atatgggaag gtgatttggt tttattttaa    1260
   tttttttatt ttgtagaagt ttaaagtggt gatggtgaga aaagtggttt aaggttaagg    1320
   gttattttag ttggtaaaat taggagtaga ataggagaat ttttagag                 1368
<210> 55
   <211> 1368
   <212> DNA
   <213> Artificial Sequence
<220>
   <223> chemically treated genomic DNA (Homo sapiens)
<400> 55
   ttttagggat ttttttgttt tatttttggt tttgttaatt gaagtaattt ttgattttgg      60
   gttatttttt ttattattat tattttaggt ttttgtaaaa tgaagaggtt gaagtgaaat    120
   taaattattt ttttatatat tttttgtagt tttaagtggg gtgagaaaat gtaaaaggtt   180
   tgtttggggg tttttttatg gggatgtggg ggtggggtag gagaaagagg ggataaggag    240
   atattaaagt tagtttttag tgtgtttttt gtaataaggt gttatgtaaa ttttaagggt   300
   ttggaggaaa tttttttttt gataatgttg attttggaga gtaggtggtg ttttaaagga   360
   gggagatgag taagttttaa gggggtgggt gaaagggaaa tgttatttgt tttttattga   420
   tttaatgtgg taggagtgta ggtgtaagaa atttttggat tttttttttg gttaattttt   480
   gtgttttatt aatttttgtt ttaagttttt atttttgttt ttaatttttg ttgaaaattt  540
   attgatgtgg tgaggttagg ttaataatat tgtaatttta ggttttttgg gttttatagg   600
   aatgttttga ttttttttta gttttagtgg tttttagttg atggtgtggt taaatgttag   660
   ggttatttgt ggtgggttgt tgtgtggggt gtagtgggag gaggttgtgt gggggtgttg   720
   tggggaggat atgttggttt gtgtttagta tttgggtttg agatttagtg tttttggttt   780
   ttgtggtttt tgtgtttttt ttgtgttaat ttattgtttt gttgtgtttg tttttttagt   840
   gggtttggtg gttgggggag gatagtgatt gagtttgaag tttggagggt ttttgtgttt   900
   ttggggtttt tattattgtt ttttttagtt tttaagtggt tattttgaag aatgatgaag   960
   ggttttattt gtgtagattt gtgggatgat tttattaaga aggaaaaggg ggaaaagaaa  1020
   ttataattat tgagggttta gttttggtta gattttgtgt tagatttttt taatttgata  1080
   ttattgattt tttataattt tttattttgt agtttaggaa gtaggttttg agagattgtt  1140
   tagaaaaaat agtatttgga tttgaatttg aggttgtttt atatttattt tggtatggtt  1200
   tttagtagaa ttgagttgga gtggttttga tgtaaatgaa tttagatttt agttgtttgt  1260
   gatggaagtt aattttagtt tttttaagtt tataggtttt ttttagaaga agttaatttg  1320
   attttttgga atatgaattt agtaggtttt tattttagaa ttgttttt               1368
<210> 56
   <211> 336
   <212> DNA
   <213> Artificial Sequence
<220>
   <223> chemically treated genomic DNA (Homo sapiens)
<400> 56
   taggtagttt tttggagttt gttttttgga ttataaaatg aagagttgtg aagagttaat    60
   aatgttaagt tggaagggtt tgatatggaa tttgattaag gttggatttt taataattgt   120
   gatttttttt tttttttttt ttttttggta aaattatttt atgaatttat gtaagtaggg    180
   ttttttgtta ttttttggag tagttgtttg agggttggaa ggagtagtga tagaaatttt     240
   agagatgtag agattttttg aattttgaat ttgattattg tttttttttg attgttgaat   300
   ttgttggaga agtgggtgtg atagggtgat gagtta                             336
<210> 57
   <211> 336
   <212> DNA
   <213> Artificial Sequence
<220>
   <223> chemically treated genomic DNA (Homo sapiens)
<400> 57
   taatttattg ttttgttgtg tttgtttttt tagtgggttt ggtggttggg ggaggatagt    60
   gattgagttt gaagtttgga gggtttttgt gtttttgggg tttttattat tgtttttttt   120
   agtttttaag tggttatttt gaagaatgat gaagggtttt atttgtgtag atttgtggga   180
   tgattttatt aagaaggaaa agggggaaaa gaaattataa ttattgaggg tttagttttg   240
   gttagatttt gtgttagatt tttttaattt gatattattg attttttata attttttatt   300
   ttgtagttta ggaagtaggt tttgagagat tgttta                             336
<210> 58
   <211> 1900
   <212> DNA
   <213> Artificial Sequence
<220>
   <223> chemically treated genomic DNA (Homo sapiens)
<400> 58
   aaaaatttat ttgttgaatt gagtggtttt ggttttagat ggttaggtag tttaaaaata 60
   tgaagttgaa gatggtagtt atttaaaaat tgttttattg ttatttttat gatgataaga 120
   aagtaagtag aggtaagggt gttaaattta gaaatagtat ataggaatga ataatttttg 180
   gggattttta ttggtagatt ttaaaaggta aattttttag tttgatgtga attatttttt 240
   gtgatttttt ttttttaatt tattttgata tttttatatt tttatgggga aaagttgttt 300
   tattatatag ttaattggtg gattaggtaa tttatgggag tgttgaaagt tgaaatttta 360
   ggttgggttt gtggtaaaat ttttaatttt atttttagtt aatagaatgt tataatgagg 420
   gaaaatattt agtaagttga aaatatagaa taaggaagtg gaaagaaatt ttagtgataa 480
   tgttattaag tggaaagaag gaaagtggtt tattttttag gaaaataaat attgattttt 540
   ttatagttat attttaagtg tattggttaa ggtatgtttt gttttgaaaa attattagag 600
   gttatagtgt ggtttttttt ggttatgtat agtgagtttg ttattatagt gtttttagag 660
   ggtatatgat tttatttttt tatttaattt tttatttgtg tttttttttt gggtttttgt 720
   ggtatttata tagtagtttt tgttttgttg ttttttggat ttttagtttt tattatattt 780
   ggtggttttt aggaagtttg aataaatttt gatgtgatta aaggagagga aagtttgttt 840
   gttattttgg gtggattaga gataatattt ttaggggata gggtgttata tggtagttta 900
   aggtgtagtg atatttgagt ttgatataat taatattagt tttgttaggt ggttttttga 960
   tgtttatttt tttattaaat aagatttata tttgtttaat aagattttgg gataatttta 1020
   atttttgttt tttagtaagg tgtgtggttg gttagttggg gtgggaagtt ggttttgtgt 1080
   taaggatttg tagttttagt agtagtagat taggtagaag tgttttttta tttttatttt 1140
   aagttttttt agaaattgtt gttagagtag atataaggtt ttggttgggg tattattttt 1200
   tgggtgggtg gggaggggag aagagataat taaaagttag ttgaattgag aaaggaattt 1260
   ggtgaatttt tttattttgt ttaatagtta aagatttttt tatttttaat ttagtttttt 1320
   tgatttttaa attagttatt atggaagttt attgttgttt tttaatttaa tgtggaaaat 1380
   tttattgttg gggattttaa ataggtgttt agtaagttgt gtgttggatt ggggtggttg 1440
   gttttggtaa tatttggtaa tttttgggtg tttttaggtg tgtggttgtt ggtttatttt 1500
   ttgagggtgt ttggaggtga agtatttaag tgggtttttg tatttagtgg gtgggatttt 1560
   ggtgggtttt gtgtgaagat ttgggttttg gtttgagtga atggtgttta aattttggtt 1620
   tggggagaat gatggtttga attgtggtgg ggtgtttttt ttattgtaga aagtgtgggt 1680
   atgtagaaat ttgtggtagg agagttttaa gtggagtgta gtttgatagt gtttgttttt 1740
   gaatttgtgt tggattgagg ggatgttatt tagtgggtag ggtgatgtta ttgttttttt 1800
   gatattttat attaaagggt tggtatgtta tagggaggga gggggagagt gaaagttagt 1860
   gtgagtaata gagagatttt tttttagtag gtatgaaagt 1900
<210> 59
   <211> 1900
   <212> DNA
   <213> Artificial Sequence
<220>
   <223> chemically treated genomic DNA (Homo sapiens)
<400> 59
   gtttttgtgt ttgttgagaa aggatttttt tgttgtttgt gttggttttt gttttttttt      60
   tttttttttg taatatgtta gttttttaat gtggagtgtt agggagatgg tgatgttatt    120
   ttgtttgttg ggtggtgttt ttttggtttg atatgagttt agagatagat gttgttagat    180
   tgtgttttat ttggggtttt tttgttgtgg gtttttgtgt atttatattt tttgtggtgg    240
   aggggatgtt ttgttgtaat ttaggttgtt atttttttta ggttggggtt tgagtgttat    300
   ttgtttgggt tagagtttgg atttttgtgt ggagtttgtt agagttttgt ttgttggata    360
   tgaggatttg tttggatgtt ttgtttttga gtgtttttgg aagatgggtt ggtagttata    420
   tgtttaaaga tatttagagg ttattaggta ttgttggaat tggttgtttt aatttggtat    480
   atagtttgtt gggtatttat ttgagatttt tagtggtagg attttttgtg ttaggttgag    540
   gggtagtggt gggtttttgt ggtgattggt ttgggagttg ggagaattaa gttgggggtg    600
   ggggggtttt tggttgttgg gtaggatagg gaagtttatt aggttttttt tttagtttaa    660
   ttaattttta attgtttttt tttttttttt tatttattta aaaaataatg ttttaattaa    720
   aattttgtgt ttgttttgat ggtagttttt gggagggttt ggggtaaaag tgaggagata    780
   tttttgtttg gtttattgtt gttaaggttg tgagttttta atgtagagtt agttttttgt    840
   tttggttggt tagttgtgtg ttttgttggg gagtgagagt tggaattatt ttagggtttt    900
   attaagtgga tatgaatttt gtttggtagg gaaatgagta ttgaagggtt atttgatgaa    960
   gttagtgttg gttgtattag atttgggtgt tattgtgttt tgaattgtta tgtaatgttt   1020
   tgttttttgg aaatattgtt tttgatttat ttaaagtgat aagtaggttt tttttttttt   1080
   tgattgtgtt gaggtttgtt taagtttttt ggaggttgtt aaatgtaatg agaattgagg   1140
   atttaggggg tagtaggata gaggttgttg tatagatgtt ataggggttt agagagagga   1200
   tatggataag aaattgggtg agggagtaag attgtgtatt ttttgaaagt attgtaatag   1260
   tgaatttgtt gtatgtggtt gaaaggggtt atgttatggt ttttggtgat tttttagggt   1320
   aaagtatgtt ttggttaatg tgtttgagat gtagttgtgg aaagattagt gtttgttttt   1380
   ttagggagtg agttattttt ttttttttta tttagtggta ttgttattaa aatttttttt   1440
   tgtttttttg ttttgtattt ttaatttatt gaatattttt ttttattgtg gtattttgtt   1500
   gattggaggt gaagttagaa attttattat agatttaatt tgggattttg atttttaata   1560
   tttttataga ttatttagtt tattgattgg ttgtatggtg gggtagtttt tttttataaa   1620
   gatatagaga tattaaaata aattagggga aaagagttat agaaggtggt ttatattaag   1680
   ttaaaaaatt tgttttttga agtttgttaa tgaagatttt taagaattgt ttatttttgt   1740
   gtattgtttt tgggtttaat atttttgttt ttatttattt ttttattgtt atggaaatga   1800
   tagtaaaata gtttttaaat gattattatt tttaatttta tgtttttaag ttatttaatt   1860
   atttgagatt agaattattt agtttagtaa gtaggttttt                          1900
<210> 60
   <211> 1002
   <212> DNA
   <213> Artificial Sequence
<220>
   <223> chemically treated genomic DNA (Homo sapiens)
<400> 60
   ttattatata gttaattggt ggattaggta atttatggga gtgttgaaag ttgaaatttt      60
   aggttgggtt tgtggtaaaa tttttaattt tatttttagt taatagaatg ttataatgag    120
   ggaaaatatt tagtaagttg aaaatataga ataaggaagt ggaaagaaat tttagtgata    180
   atgttattaa gtggaaagaa ggaaagtggt ttatttttta ggaaaataaa tattgatttt    240
   tttatagtta tattttaagt gtattggtta aggtatgttt tgttttgaaa aattattaga    300
   ggttatagtg tggttttttt tggttatgta tagtgagttt gttattatag tgtttttaga    360
   gggtatatga ttttattttt ttatttaatt ttttatttgt gttttttttt tgggtttttg    420
   tggtatttat atagtagttt ttgttttgtt gttttttgga tttttagttt ttattatatt    480
   tggtggtttt taggaagttt gaataaattt tgatgtgatt aaaggagagg aaagtttgtt    540
   tgttattttg ggtggattag agataatatt tttaggggat agggtgttat atggtagttt    600
   aaggtgtagt gatatttgag tttgatataa ttaatattag ttttgttagg tggttttttg    660
   atgtttattt ttttattaaa taagatttat atttgtttaa taagattttg ggataatttt    720
   aatttttgtt ttttagtaag gtgtgtggtt ggttagttgg ggtgggaagt tggttttgtg    780
   ttaaggattt gtagttttag tagtagtaga ttaggtagaa gtgttttttt atttttattt    840
   taagtttttt tagaaattgt tgttagagta gatataaggt tttggttggg gtattatttt    900
   ttgggtgggt ggggagggga gaagagataa ttaaaagtta gttgaattga gaaaggaatt    960
   tggtgaattt ttttattttg tttaatagtt aaagattttt tt                       1002
<210> 61
   <211> 1002
   <212> DNA
   <213> Artificial Sequence
<220>
   <223> chemically treated genomic DNA (Homo sapiens)
<400> 61
   gggggggttt ttggttgttg ggtaggatag ggaagtttat taggtttttt ttttagttta      60
   attaattttt aattgttttt tttttttttt ttatttattt aaaaaataat gttttaatta     120
   aaattttgtg tttgttttga tggtagtttt tgggagggtt tggggtaaaa gtgaggagat     180
   atttttgttt ggtttattgt tgttaaggtt gtgagttttt aatgtagagt tagttttttg   240
   ttttggttgg ttagttgtgt gttttgttgg ggagtgagag ttggaattat tttagggttt     300
   tattaagtgg atatgaattt tgtttggtag ggaaatgagt attgaagggt tatttgatga     360
   agttagtgtt ggttgtatta gatttgggtg ttattgtgtt ttgaattgtt atgtaatgtt     420
   ttgttttttg gaaatattgt ttttgattta tttaaagtga taagtaggtt tttttttttt   480
   ttgattgtgt tgaggtttgt ttaagttttt tggaggttgt taaatgtaat gagaattgag     540
   gatttagggg gtagtaggat agaggttgtt gtatagatgt tataggggtt tagagagagg     600
   atatggataa gaaattgggt gagggagtaa gattgtgtat tttttgaaag tattgtaata     660
   gtgaatttgt tgtatgtggt tgaaaggggt tatgttatgg tttttggtga ttttttaggg   720
   taaagtatgt tttggttaat gtgtttgaga tgtagttgtg gaaagattag tgtttgtttt     780
   tttagggagt gagttatttt tttttttttt atttagtggt attgttatta aaattttttt     840
   ttgttttttt gttttgtatt tttaatttat tgaatatttt tttttattgt ggtattttgt     900
   tgattggagg tgaagttaga aattttatta tagatttaat ttgggatttt gatttttaat     960
   atttttatag attatttagt ttattgattg gttgtatggt gg                      1002
<210> 62
   <211> 276
   <212> DNA
   <213> Artificial Sequence
<220>
   <223> chemically treated genomic DNA (Homo sapiens)
<400> 62
   tatatgattt tattttttta tttaattttt tatttgtgtt ttttttttgg gtttttgtgg    60
   tatttatata gtagtttttg ttttgttgtt ttttggattt ttagttttta ttatatttgg  120
   tggtttttag gaagtttgaa taaattttga tgtgattaaa ggagaggaaa gtttgtttgt    180
   tattttgggt ggattagaga taatattttt aggggatagg gtgttatatg gtagtttaag    240
   gtgtagtgat atttgagttt gatataatta atatta                              276
<210> 63
   <211> 276
   <212> DNA
   <213> Artificial Sequence
<220>
   <223> chemically treated genomic DNA (Homo sapiens)
<400> 63
   tagtgttggt tgtattagat ttgggtgtta ttgtgttttg aattgttatg taatgttttg     60
   ttttttggaa atattgtttt tgatttattt aaagtgataa gtaggttttt tttttttttg  120
   attgtgttga ggtttgttta agttttttgg aggttgttaa atgtaatgag aattgaggat   180
   ttagggggta gtaggataga ggttgttgta tagatgttat aggggtttag agagaggata   240
   tggataagaa attgggtgag ggagtaagat tgtgta                              276
<210> 64
   <211> 3100
   <212> DNA
   <213> Artificial Sequence
<220>
   <223> chemically treated genomic DNA (Homo sapiens)
<400> 64
   tgtttttttt agaggagaaa agatttggtt tatgtttttt agaggtttta tttggtgaag      60
   aggaggagta gttattgtag tgtggtggat agggtgtttt ggttggtgtt gaagatgtta     120
   gtgatagtgg ttggtatgtt ttttaaattt agttgtgtgt tattattgtg tgagtttttg     180
   gttgtttttt tttttgtaga gaggataaag gtgtttatta tgttgtgggg ggtggttttg     240
   ggttgaaggt ttttgtagtg ttttaagaat ttgtttagga tgaagttgtt gaagttgtgg     300
   ttgagttgtt tgaatttgtg gaaaatggtg tgtattgggt tggggaagta ttgtagttag     360
   ggtattatgt ttattaggtt gtttgtgttt attgtgtgtt tgaatttttt gttgtggttg     420
   agtagtttat ggaatttggg gttgttgtgg ttgtagtggt agttgaaata tatggtattt     480
   atgatgttgg ttatggttat gatggttagt ggttttgggt tgaggaaggt gttgtttgtg     540
   ttgttgtgta ttagtagtgt tattagtttg tgtgttttgt ttagtatgtg gtttttgagg     600
   atttggtggt tgtgtggttg gtgtgtgaag aagttgtgta ttatgttgtg ggttgtgtgt     660
   tgttgtattt tttagtgttt tgagtagtgg ttgaaagtta tgttgtggtt gttggatatt     720
   atatggaagg aggtgaaggt tggttggttg gtgaaggttg agttttgttg tattagggtt     780
   tggtggatgg tgtgtttgtt atttagtatt attatggggt agttgtttag gtggatttgg     840
   aaaatgttgt tgtagtgttg tgttaggtga gtgaatgaga ggtgagttgt ttggtttatt     900
   gttgttgtgt ttttgattag tggttatgta aatgggtttg ggggtgtgga ttggagttgt     960
   tgtttttgtt gttttagtag ttgttggttt atatgtatag tggttagtat tgataggagt    1020
   agtaggagtg tggtttgttg gatggatagt gggtttagtg gttaagggtt gtttgggttg    1080
   aggttggtgt ttatgttggg gatagagagg agaaggtgtg atatttaggg gtgtagagat    1140
   aggagtgggt gttttatgtt tttattttag tttttgggga ttgagtgttg ttgggtggag    1200
   aggttggagt tgattttttg gagaaatggt tgaagatgtt tttttttatt tttaatttat    1260
   ttttttttgg agaagagaag gggtgtgttt ttattttgtt gtaatttagt gttaaatttt    1320
   tttttttttt gtaaggtgtg taatggtttt tgtaatttgg ggataatgtt gtgggtattg    1380
   aggtggtggt gtttgatttt ttttaaagta tttattatgt tatttgttat ttgtaataat    1440
   ttatttgaag aggttgttgg gtagtgtttt ggtagataga ttgatttgtg gggaggtgtg    1500
   gtttttagtg gtgtgggata gttggttttg agaaggaatt gggatttttg tttagggtaa   1560
   gatgttaata ggaatttgta gtttggtttg gatatttgtt gtttttattg gaagttttaa    1620
   tttttatttg agttttttgg tgttgttagt gttaggagtg tttggattgg gatggggatg   1680
   gagaagggtg ttttgtttgt tgagttttgt tgtttttggg gatttggtaa agttgaggtt    1740
   tttttatagt gttgagattg agattggggg ttggtgagtg gtgttaattt ttatgttttt    1800
   gtggttttta taattggagt tgtagaagtg tttgttttta tttttatttt agagttaaag    1860
   tgtgttattt tgtttttttg ggttttaagg attgggtaga aggagggtag tgtgatttgg   1920
   tagggtgggg tttgtggggg gtggggtgtg ttgtatatta ggttgttttg atttggattt    1980
   ttgtttaggt gtattggttg gttttggagt tttattagta ggtttttatg ggagtggagg    2040
   tgtttatgtt tttattgtgt ggtaattgtg ttttattata gttattttta attgaggttg    2100
   tatggtgttt ttagaattat gtttggtata atttgttttg gttgttgtgt ttgatttggg    2160
   agtgtgtttt gttttgagtg tatgattttt tttttttttt tgtgatttga gtttgttttt    2220
   tggaaagtta gtgtagtggt agtggtttgt tttatagtgt ttttggttgg ttgtttaaat    2280
   tttattttag gtttttgtaa ataaattgtt attttagtgg aggttttttg gtatgttaaa   2340
   ttttttttag tttttgtggt gagattagga atttttaaaa gtagttgttt ttttgttttg   2400
   gggttttgat agtgtttggg tatataaagg tttttggggt gtagttggtt ttgagatttt   2460
   ttgtgtagag gttattttag gtggtgttgt ttgttggtgg gtgtggggtt gtttaggttg    2520
   tgatggaagt tgttggtgta gggtggggag ttaagttggg gtgattgggg gggtttgggg    2580
   gagttagtgg ggtgggattt gagtggggtg gagagtggta ggaggaggtg aatttttgtg    2640
   ttttggtgaa ttttattggg ttgaagtttt tgttgttgtt ttttttttgt gtgtggagtt   2700
   gggttttgtg tgtgttgttt ttggaaagtt ggttttagtt atatttttgg gtgttgtttg   2760
   tggttgtttt ttttgtgttt tttatggtat ttgatatgtg gaggtggtgg ttgagggtgg    2820
   ggtgttggtt attattattt ttggtgtggg gtgttggttg ttattatttt tggttgtgta    2880
   tgtatagttg tgtttgttag gttagtgagg tggatgtttg ggttattatt tgtaggttga    2940
   gtttgggtgt gtattgttat tttgttgtgt attggtggtt ttgtggtttt tttgggtgtg    3000
   tttgtggttg tgaagggggt ttttttttta taagggaagt tggaggtgtt tgggtggggg    3060
   tggttttttt tgtttggtgt gttttttttt atatgttgat                         3100
<210> 65
   <211> 3100
   <212> DNA
   <213> Artificial Sequence
<220>
   <223> chemically treated genomic DNA (Homo sapiens)
<400> 65
   attagtatgt aggaaagggt gtgttaggtg ggggaagtta tttttgttta agtgtttttg      60
   gtttttttta taaagggagg gttttttttg tgattgtaag tgtgtttagg aagattatag     120
   agttgttggt gtgtagtgag gtggtgatat gtgtttgggt ttggtttgtg ggtggtggtt     180
   taagtgtttg ttttgttggt ttggtaggtg tgattgtgtg tgtgtagttg agggtggtgg     240
   tggttggtat tttatgttaa gggtggtggt ggttggtatt ttatttttgg ttgttgtttt     300
   tgtgtgttag gtgttgtgag aagtgtggga ggagtggttg taggtagtgt ttagggatat     360
   gattggagtt gattttttag aagtggtgta tgtaaagttt agttttgtat gtaaagggga     420
   ggtgatagta gaaattttaa tttgataaag tttgttggag tgtggagatt tgtttttttt     480
   tgttattttt tgttttgttt gggttttgtt ttgttagttt ttttaggttt ttttagttgt     540
   tttagtttgg ttttttgttt tgtgttaatg gtttttattg tagtttgggt ggttttgtgt     600
   ttattagtgg gtggtgttat ttggagtggt ttttatgtgg gaaattttag ggttagttgt     660
   gttttaggag tttttgtgtg tttaagtatt gttggggttt tggggtgggg gagtggttat     720
   ttttagggat ttttgatttt gttgtaagaa ttggaaaaaa tttagtatgt taaagagttt     780
   ttattgaggt ggtaatttgt ttgtgagaat ttaagataaa atttaaataa ttaattaggg     840
   gtgttgtgag gtaaattgtt gttattatat tggttttttg ggaagtaagt ttaagttgtg     900
   gagagggaag ggaggttgtg tgtttggggt ggggtgtgtt tttaagttga gtgtagtggt     960
   tggggtaggt tgtattgagt gtggttttgg ggatattgtg tggttttgat tggaggtggt    1020
   tgtgatgaag tgtggttatt gtataatgga aatgtgggta tttttgtttt tatgaaagtt    1080
   tgttggtaga gttttgaggt tggttggtgt gtttggatgg gagtttgggt taaagtggtt    1140
   tggtgtgtgg tgtgttttgt tttttgtagg ttttgttttg ttaggttgtg ttgttttttt    1200
   tttatttagt ttttaaaatt tggaggagtg ggatggtgtg ttttgatttt ggagtgggag    1260
   tgggagtgag tgtttttgtg attttagttg tgagagttgt aagggtatgg gaattgatgt    1320
   tatttattga tttttagttt taattttaat gttgtgagga aattttgatt ttgttaggtt    1380
   tttaagggta gtggggtttg gtgagtgagg tatttttttt tgtttttatt ttaatttaag    1440
   tgtttttggt attgatgatg ttaagagatt tgagtgggag ttaaagtttt tagtgagggt    1500
   agtaggtgtt taggttgggt tgtgggtttt tgttgatgtt ttgttttagg taaaggtttt    1560
   agtttttttt tggagttggt tgttttgtgt tattggaaat tgtatttttt tgtaggttag    1620
   tttgtttgtt gaggtgttgt ttggtgattt ttttagatgg attattatag gtagtgggtg    1680
   gtgtggtagg tattttaaag gaaattaagt gttattgttt tgatgtttgt agtgttgttt    1740
   ttagattgta ggaattgtta tgtgttttgt ggggagggga agggtttggt gttgggttat    1800
   agtgaggtgg aaatatgttt tttttttttt ttaagggaga gtgggttggg gatgggaagg    1860
   ggtgtttttg gttatttttt tagagagtta gttttgattt ttttatttaa tggtatttag    1920
   tttttagagg ttggggtagg ggtgtggggt gtttgttttt gtttttgtat ttttgagtgt    1980
   tatgtttttt tttttttgtt tttagtatgg gtattagttt tagtttgaat gatttttggt    2040
   tgttaaattt gttgtttatt tagtagatta tgtttttgtt atttttgttg gtgttggtta    2100
   ttgtgtatgt gggttagtgg ttgttgaggt aatggaggtg gtagttttgg tttgtgtttt    2160
   tgggtttgtt tgtgtggtta ttgattggaa atgtggtggt ggtgggttag gtggtttatt    2220
   ttttgtttgt ttgtttggtg tggtgttatg gtgatgtttt ttagatttgt ttgggtagtt    2280
   gttttatagt ggtgttgaat ggtgagtgtg ttatttatta ggttttggtg tagtagggtt    2340
   tggtttttgt tgattggttg gtttttgttt ttttttgtgt ggtgtttggt ggttgtagta    2400
   tggtttttgg ttattatttg gagtattgga aggtgtagtg gtgtgtagtt tatagtatga    2460
   tgtgtaattt ttttatgtgt tagttgtgta gttgttaagt ttttgagggt tatgtgttga    2520
   gtgaggtgtg tgagttggtg gtgttgttgg tgtgtggtag tgtggatggt gttttttttg    2580
   atttgaggtt gttgattgtt gtggttgtgg ttaatgttat gagtgttgtg tgttttggtt    2640
   gttgttatag ttatgatgat tttgagtttt gtgagttgtt tagttataat gaagagtttg    2700
   ggtgtatggt gggtgtgggt agtttggtgg atgtgatgtt ttggttgtag tattttttta    2760
   atttggtgtg tattgttttt tgtgaatttg agtagtttaa ttgtaatttt agtaatttta    2820
   ttttggataa gtttttgagg tattgtgaaa gtttttggtt tggggttgtt ttttgtgata    2880
   tgatggatgt ttttattttt tttgtggaaa agaaggtggt tggggatttg tatggtggtg    2940
   gtgtgtggtt ggatttggag aatgtattgg ttattattat tgatattttt ggtgttagtt    3000
   aggatatttt gtttattgtg ttgtagtggt tgtttttttt ttttattagg taaagttttt    3060
   gggaggtgtg ggttaggttt tttttttttt gaaaaaggtg                          3100
<210> 66
   <211> 2500
   <212> DNA
   <213> Artificial Sequence
<220>
   <223> chemically treated genomic DNA (Homo sapiens)
<400> 66
   ttgagttgtt tgaatttgtg gaaaatggtg tgtattgggt tggggaagta ttgtagttag      60
   ggtattatgt ttattaggtt gtttgtgttt attgtgtgtt tgaatttttt gttgtggttg     120
   agtagtttat ggaatttggg gttgttgtgg ttgtagtggt agttgaaata tatggtattt     180
   atgatgttgg ttatggttat gatggttagt ggttttgggt tgaggaaggt gttgtttgtg     240
   ttgttgtgta ttagtagtgt tattagtttg tgtgttttgt ttagtatgtg gtttttgagg     300
   atttggtggt tgtgtggttg gtgtgtgaag aagttgtgta ttatgttgtg ggttgtgtgt     360
   tgttgtattt tttagtgttt tgagtagtgg ttgaaagtta tgttgtggtt gttggatatt     420
   atatggaagg aggtgaaggt tggttggttg gtgaaggttg agttttgttg tattagggtt     480
   tggtggatgg tgtgtttgtt atttagtatt attatggggt agttgtttag gtggatttgg     540
   aaaatgttgt tgtagtgttg tgttaggtga gtgaatgaga ggtgagttgt ttggtttatt     600
   gttgttgtgt ttttgattag tggttatgta aatgggtttg ggggtgtgga ttggagttgt     660
   tgtttttgtt gttttagtag ttgttggttt atatgtatag tggttagtat tgataggagt     720
   agtaggagtg tggtttgttg gatggatagt gggtttagtg gttaagggtt gtttgggttg     780
   aggttggtgt ttatgttggg gatagagagg agaaggtgtg atatttaggg gtgtagagat     840
   aggagtgggt gttttatgtt tttattttag tttttgggga ttgagtgttg ttgggtggag     900
   aggttggagt tgattttttg gagaaatggt tgaagatgtt tttttttatt tttaatttat     960
   ttttttttgg agaagagaag gggtgtgttt ttattttgtt gtaatttagt gttaaatttt    1020
   tttttttttt gtaaggtgtg taatggtttt tgtaatttgg ggataatgtt gtgggtattg    1080
   aggtggtggt gtttgatttt ttttaaagta tttattatgt tatttgttat ttgtaataat    1140
   ttatttgaag aggttgttgg gtagtgtttt ggtagataga ttgatttgtg gggaggtgtg    1200
   gtttttagtg gtgtgggata gttggttttg agaaggaatt gggatttttg tttagggtaa    1260
   gatgttaata ggaatttgta gtttggtttg gatatttgtt gtttttattg gaagttttaa    1320
   tttttatttg agttttttgg tgttgttagt gttaggagtg tttggattgg gatggggatg    1380
   gagaagggtg ttttgtttgt tgagttttgt tgtttttggg gatttggtaa agttgaggtt    1440
   tttttatagt gttgagattg agattggggg ttggtgagtg gtgttaattt ttatgttttt    1500
   gtggttttta taattggagt tgtagaagtg tttgttttta tttttatttt agagttaaag    1560
   tgtgttattt tgtttttttg ggttttaagg attgggtaga aggagggtag tgtgatttgg   1620
   tagggtgggg tttgtggggg gtggggtgtg ttgtatatta ggttgttttg atttggattt    1680
   ttgtttaggt gtattggttg gttttggagt tttattagta ggtttttatg ggagtggagg    1740
   tgtttatgtt tttattgtgt ggtaattgtg ttttattata gttattttta attgaggttg    1800
   tatggtgttt ttagaattat gtttggtata atttgttttg gttgttgtgt ttgatttggg    1860
   agtgtgtttt gttttgagtg tatgattttt tttttttttt tgtgatttga gtttgttttt    1920
   tggaaagtta gtgtagtggt agtggtttgt tttatagtgt ttttggttgg ttgtttaaat    1980
   tttattttag gtttttgtaa ataaattgtt attttagtgg aggttttttg gtatgttaaa   2040
   ttttttttag tttttgtggt gagattagga atttttaaaa gtagttgttt ttttgttttg   2100
   gggttttgat agtgtttggg tatataaagg tttttggggt gtagttggtt ttgagatttt    2160
   ttgtgtagag gttattttag gtggtgttgt ttgttggtgg gtgtggggtt gtttaggttg    2220
   tgatggaagt tgttggtgta gggtggggag ttaagttggg gtgattgggg gggtttgggg    2280
   gagttagtgg ggtgggattt gagtggggtg gagagtggta ggaggaggtg aatttttgtg    2340
   ttttggtgaa ttttattggg ttgaagtttt tgttgttgtt ttttttttgt gtgtggagtt   2400
   gggttttgtg tgtgttgttt ttggaaagtt ggttttagtt atatttttgg gtgttgtttg    2460
   tggttgtttt ttttgtgttt tttatggtat ttgatatgtg                          2500
<210> 67
   <211> 2500
   <212> DNA
   <213> Artificial Sequence
<220>
   <223> chemically treated genomic DNA (Homo sapiens)
<400> 67
   tgtgtgttag gtgttgtgag aagtgtggga ggagtggttg taggtagtgt ttagggatat 60
   gattggagtt gattttttag aagtggtgta tgtaaagttt agttttgtat gtaaagggga 120
   ggtgatagta gaaattttaa tttgataaag tttgttggag tgtggagatt tgtttttttt 180
   tgttattttt tgttttgttt gggttttgtt ttgttagttt ttttaggttt ttttagttgt 240
   tttagtttgg ttttttgttt tgtgttaatg gtttttattg tagtttgggt ggttttgtgt 300
   ttattagtgg gtggtgttat ttggagtggt ttttatgtgg gaaattttag ggttagttgt 360
   gttttaggag tttttgtgtg tttaagtatt gttggggttt tggggtgggg gagtggttat 420
   ttttagggat ttttgatttt gttgtaagaa ttggaaaaaa tttagtatgt taaagagttt 480
   ttattgaggt ggtaatttgt ttgtgagaat ttaagataaa atttaaataa ttaattaggg 540
   gtgttgtgag gtaaattgtt gttattatat tggttttttg ggaagtaagt ttaagttgtg 600
   gagagggaag ggaggttgtg tgtttggggt ggggtgtgtt tttaagttga gtgtagtggt 660
   tggggtaggt tgtattgagt gtggttttgg ggatattgtg tggttttgat tggaggtggt 720
   tgtgatgaag tgtggttatt gtataatgga aatgtgggta tttttgtttt tatgaaagtt 780
   tgttggtaga gttttgaggt tggttggtgt gtttggatgg gagtttgggt taaagtggtt 840
   tggtgtgtgg tgtgttttgt tttttgtagg ttttgttttg ttaggttgtg ttgttttttt 900
   tttatttagt ttttaaaatt tggaggagtg ggatggtgtg ttttgatttt ggagtgggag 960
   tgggagtgag tgtttttgtg attttagttg tgagagttgt aagggtatgg gaattgatgt 1020
   tatttattga tttttagttt taattttaat gttgtgagga aattttgatt ttgttaggtt 1080
   tttaagggta gtggggtttg gtgagtgagg tatttttttt tgtttttatt ttaatttaag 1140
   tgtttttggt attgatgatg ttaagagatt tgagtgggag ttaaagtttt tagtgagggt 1200
   agtaggtgtt taggttgggt tgtgggtttt tgttgatgtt ttgttttagg taaaggtttt 1260
   agtttttttt tggagttggt tgttttgtgt tattggaaat tgtatttttt tgtaggttag 1320
   tttgtttgtt gaggtgttgt ttggtgattt ttttagatgg attattatag gtagtgggtg 1380
   gtgtggtagg tattttaaag gaaattaagt gttattgttt tgatgtttgt agtgttgttt 1440
   ttagattgta ggaattgtta tgtgttttgt ggggagggga agggtttggt gttgggttat 1500
   agtgaggtgg aaatatgttt tttttttttt ttaagggaga gtgggttggg gatgggaagg 1560
   ggtgtttttg gttatttttt tagagagtta gttttgattt ttttatttaa tggtatttag 1620
   tttttagagg ttggggtagg ggtgtggggt gtttgttttt gtttttgtat ttttgagtgt 1680
   tatgtttttt tttttttgtt tttagtatgg gtattagttt tagtttgaat gatttttggt 1740
   tgttaaattt gttgtttatt tagtagatta tgtttttgtt atttttgttg gtgttggtta 1800
   ttgtgtatgt gggttagtgg ttgttgaggt aatggaggtg gtagttttgg tttgtgtttt 1860
   tgggtttgtt tgtgtggtta ttgattggaa atgtggtggt ggtgggttag gtggtttatt 1920
   ttttgtttgt ttgtttggtg tggtgttatg gtgatgtttt ttagatttgt ttgggtagtt 1980
   gttttatagt ggtgttgaat ggtgagtgtg ttatttatta ggttttggtg tagtagggtt 2040
   tggtttttgt tgattggttg gtttttgttt ttttttgtgt ggtgtttggt ggttgtagta 2100
   tggtttttgg ttattatttg gagtattgga aggtgtagtg gtgtgtagtt tatagtatga 2160
   tgtgtaattt ttttatgtgt tagttgtgta gttgttaagt ttttgagggt tatgtgttga 2220
   gtgaggtgtg tgagttggtg gtgttgttgg tgtgtggtag tgtggatggt gttttttttg 2280
   atttgaggtt gttgattgtt gtggttgtgg ttaatgttat gagtgttgtg tgttttggtt 2340
   gttgttatag ttatgatgat tttgagtttt gtgagttgtt tagttataat gaagagtttg 2400
   ggtgtatggt gggtgtgggt agtttggtgg atgtgatgtt ttggttgtag tattttttta 2460
   atttggtgtg tattgttttt tgtgaatttg agtagtttaa 2500
<210> 68
   <211> 216
   <212> DNA
   <213> Artificial Sequence
<220>
   <223> chemically treated genomic DNA (Homo sapiens)
<400> 68
   tatgtttttt aaatttagtt gtgtgttatt attgtgtgag tttttggttg tttttttttt 60
   tgtagagagg ataaaggtgt ttattatgtt gtggggggtg gttttgggtt gaaggttttt 120
   gtagtgtttt aagaatttgt ttaggatgaa gttgttgaag ttgtggttga gttgtttgaa 180
   tttgtggaaa atggtgtgta ttgggttggg gaagta 216
<210> 69
   <211> 216
   <212> DNA
   <213> Artificial Sequence
<220>
   <223> chemically treated genomic DNA (Homo sapiens)
<400> 69
   tattttttta atttggtgtg tattgttttt tgtgaatttg agtagtttaa ttgtaatttt      60
   agtaatttta ttttggataa gtttttgagg tattgtgaaa gtttttggtt tggggttgtt     120
   ttttgtgata tgatggatgt ttttattttt tttgtggaaa agaaggtggt tggggatttg     180
   tatggtggtg gtgtgtggtt ggatttggag aatgta                               216
<210> 70
   <211> 9100
   <212> DNA
   <213> Artificial Sequence
<220>
   <223> chemically treated genomic DNA (Homo sapiens)
<400> 70
   atttataatt ttagaagttt ttttaagagg aaaaaaaagg tgttatgtgt ttgttgttta      60
   atagatttaa atattttatt atttttatta ttgttattat tatttttatt gaatattttt     120
   attataagat aggtagatat gtgatatgtg gtgaaggttt tattttattt attattaatt     180
   tttataagta tttttatttt agggtgtttt ggggagggta gaggaggaat atttgggttt     240
   aaaagttgtt tttggattat ttttagatta gtttggtgga aatggtagtg tgagtgttat     300
   gttatttgag attgtgtttg aaaatttttt ttatgttttt aaattaaagt tattttgagg     360
   tttaagaatg ataattatag gtgaatgttt tatttttttt atgagttagg tttttttttt     420
   gtatgatgta gattggtggt tgagatttgt tttgaggttt tgttttttat ttggtttatg     480
   tggttttgtg tagtttatgt agtttttttt gtttttaggt aattagtttg tgggaaattt     540
   attttagaaa agtgtgtgtt atattattat tttgtgattt aataaatttt tttaattgtt     600
   gtttaggaat tagattttag atagattttg ttgtgtttgg tttttttaga aatttttttt     660
   ttagaggaag aagatttggg ttgggaagag tgtgtgatta tggttttggg tttattgaaa     720
   gatttttttt tataaaagtt tagggttgat attaaatgaa gttatggagt tatgtttata     780
   taaatatttt ttttttattt gaattgtggg gtttggattg tgaaggtttt tgttgtaaga     840
   gtgggtattg gtgaatttta gtgtatgttg tggttgagaa tggatgtagg gtgggggatg     900
   gttgagttgt ttgatttttg tagagaattt gtaggggttt ttggagggta ttttttgtgt      960
   ttaagggagt gttttttttt tttagtattt gggggaattg agggtgatgt gttagttttg    1020
   tatttaattt tttttttttt tgtaggtata gagttggttg gtgggggtag aggaggagtt    1080
   gggtttttat tgtgtttgtt taaatttggt taggggttgt gttttttttt tttattttat    1140
   gatgattttt ttttagtttt tgttttttaa tttaattgtt aattatttgg aatgtgtggg    1200
   tggggagtgg tgaggagggt gagtttgggg tttgttgttg ttgttgttgt tgtgtgtgtg 1260
   tgtttaggaa gtggtgtggt tgttattttt ttttgggttt tttttttttt tttttttgtt 1320
   ttgttttttt tttttttttt tttttttttt gttttgttgt ttgtgtttag tgtatttatt 1380
   ttttttttat gttatttgtt agtgtgttat gtaaattatt gttgttgttg gtttttattg 1440
   gttgtggtgt gtttatttaa tggtagtttg ggtttggtgt atggttgttg ggttttgtgt 1500
   gttgttggtt ttgtgtgtgt ttttgttttg agtgtatggt tttgggtagg tagtgggtag 1560
   tttattttgg gtttggtggt tttggttttt tggttttttt tgtgagtttg tgtttttttt 1620
   gttgtttttg ggtttttttt tggttgtatt gtaattgttt tttgtaggtt tttttgtgtt 1680
   tttttttttt tgttattggt gtttggtttt ttttgggtat ttgggattag tatatgttta 1740
   gtgtatgtgg tgtgttgttt tgttagaagt tgtagttttt gagttggagg ttgttgagga 1800
   ttgagtgtag gaggaaggag atagtgtgta gtggtggttg gtgaggagat agtatatttt 1860
   gggttgggtt tagtgtattg tttttggttt taaaagtgga gttgtaattt ggttatgatt 1920
   gtatttgttt gtattgtttt gttgagggtg tttgggttgt ggtggtggtg aagatggtga 1980
   ttttgattgt tggttttttt ggtaagtggt ttgtgtatta ggagaaattt tttatttgtg 2040
   agttgaattg gtgttgagtg tgtgtgtttt tgtttttttt tgttgttgtt gtttttattt 2100
   ttttttattt ttttttttgt taggattttt ttgtttttgt tttattttgt ttgaattttt 2160
   ttttgttttt tttttatttt ggttgtttat gttttaggtt ttttttttgt ggtgttggtg 2220
   aatttgttag ttgttttgat gtatagtatg atgatggaga ttgatttgta tttgtttggt 2280
   ggtgtttagg tttttatgaa ttttttgggt tttgttgggg tgggtggtgg tgggggtgga 2340
   ggtgggggtg gtggtggtgg tgggggtgtt aaggttaatt aggattgggt taaatggttt 2400
   atgaatgttt ttatggtgtg gttttgtggg tagtggtgta agatggttta ggagaatttt 2460
   aagatgtata atttggagat tagtaagtgt ttgggggttg agtggaaggt tatgtttgag 2520
   gttgagaagt ggttgtttat tgatgaggtt aagtggttgt gtgtgttgta tatgaaggag 2580
   tatttggatt ataagtattg gttgtgttgt aagattaaga tgttgtttaa gaaggataag 2640
   tatttgttgg ttggtgggtt tttggtggtt ggtgtgggtg gtggtggtgt ggttgtggtt 2700
   atgggtgtgg gtgtgggtgt gggtgtggtg gttgtgggtt agtgtttgga gagtttaggt 2760
   ggtgtggtgg gtggtggtta tgtgtatgtt aatggttggg ttaatggtgt ttattttggt 2820
   ttggtggtgg tggtggtggt ggttgtggtt atgatgtagg aggtgtagtt ggtttatggg 2880
   tagtatttgg gtgtgggtgg tgtgtatttg tatgtgtatt ttgtgtattt gtatttgtat 2940
   tatttgtatg tgtatttgta taatttgtag tttatgtatt gttatgatat gggtgtgttg 3000
   tagtatagtt ttatttttaa tttgtagggt tatatgagtg tgttgttttt gggttatggt 3060
   ggtttttttt atggtgttgt ggttgttgtt gttgttgttg tgggtggtgt gtattagaat 3120
   ttggttgtgg tggtggtggt ggtggtggtg gttgtgttgt tgggtgtttt gggtgtgttg 3180
   ggttttttgg tgaagttgga gtttagtggt agtttgtttg ttttagtgta tttgtgggtg 3240
   ttgtgttttg gggatttgtg tgagatgatt agtatgtatt tgtttgttgg tgaggggggt 3300
   gatttggtgg tggtagtagt ggttgtggtg tagagttggt tgtatttgtt gttgtagtat 3360
   tattagggtg tgggtgtggg tgtgaatggt atggtgtttt tgatgtatat ttagtgtttt 3420
   tgggatgttg gggattttgt ggtggtgatt tatgagtttg tggtttgtgt ttggtttttg 3480
   ttttgttttg gtgtggtgtg gtttttgtat agatgttttt atatttttgt taaaaggaaa 3540
   atattggaga tgaatgttgg gtgatgtgtg ttttttattt attttttttg gagattttgg 3600
   tgattgttgg gtgttgatat tagatttggg ttttagattg aattttggtg tttttttgag 3660
   attttttgta tagtatttat tatttatgga ggaagtggaa agtgtttttt ttgtttgagg 3720
   ggataaaaaa gttaaaatga ggtgagaggt gaagtttatt tttgtatatt ggttggtgtg 3780
   tttatttttt tttgtgttgt ttttggatgg tgttgattgt tggagtttaa gtgatgtgga 3840
   gtttgttgta tttgttataa atgtagtaag gtaggtttaa gtatttataa gttttttgta 3900
   gttgttattg tttttttggg ttggtttgtt aatttatata aagagattat tattattatt 3960
   ttttttttta gatggtggag ttatattttg ggttttgtaa aattttatgt atttgagtat 4020
   ttttattttt ttttttgggt tttgtattat tttttgtaaa tgtattgtga aaaattttat 4080
   ttttggtgtt gtaatgtggg gaggagaagt tagattatgt atatagtttt ttaaaaagtt 4140
   ttttttttaa aaatgaaaaa agatttttta tttaaaatgt tttgagttaa taaatttaag 4200
   agatagagtt tatttttttt ataaatttgt aatatgttat ttttatgtgt atgttttatg 4260
   agtttaaaat gtaatgagga aatttgatag ggaaattatt tgtatgaatt aaaagtaagg 4320
   gaattttggg gaatgggagg ataggatttt ttaaggaatt ttttttaatg aaagagaagg 4380
   aagttaaaat ttataggtta ttttgtagag ttgagtgtta atatgggttg agaaataaaa 4440
   gtattttttg ttttggttgt tttattgtgg atggttgggg ttgttgtgtg ttattttgtt 4500
   gtaagtgggg tgttttttat ttggttgggg gtttgtgtta tagtttggtt tagaggaggg 4560
   aggaggaagg gaagatttta gtggtgggat tttggattag gttatggatg aaggataaag 4620
   attagggtag gttatgggtt ttttaatttt ttagtaatta agattttgag tagaatttat 4680
   ttaaatgtgt tttaaggaaa tataattgtt gaattaaaat gtattgtagt tgagtttttt 4740
   ttgtttattt tttgtttttt tttttggttt tttttttttg ggaaaatggg taaaataatt 4800
   gtgttggatt tttatatata tagaaatatt gattattatt tttttttgtg tgaattggga 4860
   tgtaagttgt taattgatgt gaatgtaaaa tgttttgttt attatttttg atgagatttt 4920
   gaggttgttt gatgttttaa attttttaat tatattattt tttaggtgtt tattggtata 4980
   ttgtagtttt ttttttgaaa tttaaaaatt tttgtaaaat tttgttttta agtaatttga 5040
   tagtattaaa tattgtattt aaaaattata ttgtagtaaa tatatttaaa aattaattat 5100
   aatgttaaga tgaaattata tttttggaaa aaaaaaatat ttgaagttta gatggaaata 5160
   tgtttatttt agtagtttta ggtttttttt tgttttttta atattttttt ttgttttgga 5220
   gtatggattg tttgtttaaa agtgagttta tgttataagt ttaatgagaa ttgaatttag 5280
   tttgtatttg agaatagttt taagtataat gttgatttga taattgatgt gtaatttggg 5340
   aagttatttt gataattttg tttaaattat ttatttgtta aagtgattat aaaaaagttt 5400
   aagaatgatg tttattgttt tttaataaga taataaattt tttttttttt tttttttttt 5460
   ttatttttat tttttttagt tatttgattt tttttgaagt agttgttttt ggaagagttt 5520
   gtgtgtttat ggatggttga gtattattat gatttagttt gggataaggg tttttttagt 5580
   tttttttggg agatgatttg ggaaatttta taatgtttgt tttgttaatt tattgggatt 5640
   ttgagggttt aatgggattt tgagggtttt ttttgaaata tataaattta aaggattttt 5700
   tttgaggttt tttgattgat gtttattttt agtttggttt ttgtgttttt ttgtgtgtat 5760
   tttggagttt ttgtgtttaa ttgttggtat ttaggttttg gtttaagatt aggatgtggg 5820
   ttttatttta gaggtataga ttatgaaaag ttgagttagt gtgtttggga tgttaggtaa 5880
   gtagttttta tagtttggta ttttattgta ggtgttttgt gtatagttag ttgaaatagt 5940
   taatgttagg tgttttaatt attttatttt tttgttttgt tgattagaat aatatagaaa 6000
   aaagtaaata taaattttta atgattttat ttaaaaatat tatagggtgg gggtaaggaa 6060
   attagttgag atttatttta ggattgagat tttatttttt tttttttgtt tttagtagtg 6120
   ttgttttaat ttaaattagt ggagaaaaga ttatagtagg ttttgagttg attgtgaatt 6180
   tggtgtttgg ttaaggtaat atttattgta tttatggagt gaaatattat atgatgatag 6240
   ttattatatt atatgatgat tttatttatt ttttaaatta tagggtggtg tttttgttgt 6300
   ttagtttaat tatgtttgta atttgtgtag ggaaggtgga taggagtttt tgaggtttag 6360
   tgtaaagttt gtattagttt ttgtgggggt gatagtgtga ttttgtaaat ttgttattgt 6420
   gtttgggttt tgttttttaa attgagggta ggagggttta tttgagggtt tttttgtttt 6480
   gttttgtttt gtttttgttt tgttttgttt tttaatttat ttgttttttt tgttgttgag 6540
   attttgatgt tttttttttt ggagaaattt atttgtaatg tattagatgg attagtttta 6600
   aagaagattt gggtatagtg tgatttttag atgtgtgttg ggagtgtgat tgataggttt 6660
   attaaaatta tgtgtgtggg agaatggtat ttatgttttt gtgttttatt tttttggtaa 6720
   agattgggtg ttgtagattt ttgtttttgg aaaattttag ttaatttaag aaatttattt 6780
   ttggatgtgt gtttgttttt ttttgaaggt ttgttttagg ttagtttgga ttttagtgtt 6840
   tttttgattt tgttgggttt tggttgattt ttgttttttt tttatttatg tttggtttgg 6900
   gtttgggggt agtggtttta gaggtgttgg tggtagaatt taatttttgg tttgagtttt 6960
   tggaaatttt aagtgaaaag tagatttggt tttggtgtgg tttttttttt agtgtgatta 7020
   tttggtgaga ttgaaaggtt aggttagatg gggaaaagtg agtggggtgt gggtttttag 7080
   tatttggtgg ggtggggtgg tgttaggatg gagtgagggt tggtgggggg gtttgtgtgg 7140
   aggtttaggt tgggaggtgg ggatgtaggg attttttatt tagttttttg tttttgtttg 7200
   agttaggttt tgggattatt tttttttgtt tgagggtgag tttttttttt tttttttttt 7260
   ttttgttttt tttgtttttt tttttttttt tttttttttt ttttttggtt tttttttttt 7320
   agttgtgtgt ttgttgggat tatgtttttt ttgtttttgg gagttgagtg gtgtttgtgg 7380
   ttggtttgga tgtgtttttt ttgggtgggg gtggttgggt ttttttgggg ttttgggttt 7440
   tttgggtgta tttttttgtt tgtgtttttt taagttgttt tttttttggt tttgtttgtg 7500
   gttgagtttt gtatattgtg ttgttgtttt tggggttttg gtatatggtg tttttttttt 7560
   gtttttgtta tttattttta ttgttttttt agtttttgtg ttgtttttat tgttttttga 7620
   tttttgtatt gagttgtgtt tttgttgttg gtttttttta gtgtggtttg gttttttttt 7680
   tgtttttgaa gtttttttgt tgaggtttgt gtgtggtttt tttttttgtt tttttttggt 7740
   ttttgggtgt ttagttggag gttggggtgg gatggggtat gagttttggg gtggtttgtg 7800
   ttttttttat tagttttttt attgttttgg ttttgttttt gtagttttgg ggtgggaggg 7860
   tgtaggggtt tttgggtgtt ttgggagttt atttttttta ggtttgtgtt ggtgttagtt 7920
   gttggtgggg ggtgggtgga gattaagatg agtgttgtga gggatggggt tgagggtttt 7980
   gtgtagaagg agagtaggat tagtggagag aaagtgttga tttggggtta gttgtaagtt 8040
   aaattttttt atatttagtt ggtggaatga gtatggtttg ggttggatgg taataagtgg 8100
   gtgtgaaggg agggggaggg gaggttgggt attggttggg tttggtgtga gagaggggag 8160
   ggggagggga tgtggttata gtgttttagg gttttagtgt tattggggat gggtgttgag 8220
   gggagtgtgt attagtgatt tgggtgtgga ttggggtttg tagggtgtga ttggttgttt 8280
   tttaggattt tatttttttt ataaatattt gtttttttta ttttttgggg ttgttttggt 8340
   tgtttttttg gagggaagat tggggagaag gttgtttgtg ttttttgttt tagaggtttt 8400
   gttttagaaa ttgtttttta atttttataa gaaataaaaa aattatagga aggtttggtt 8460
   ttttttttgg atgattgtgt ttttaggttt tttaggaatt tttgttttat ttgagttttt 8520
   ttgtagtttt ggatttattt ttggggggtg tgtgtgatgt agaattgggg tataaaatat 8580
   taatgttgtt tgttatgata ttgttttttt tatttgggta gagttgtggt ttttgggtgt 8640
   tgtgtgtggt gttttttatt atggagggaa agttgtggtt ttgtgggaaa ggagtgaaaa 8700
   ttgagtgtgg gtgaagagga gagggaggtt agggggtaag ggaagaaagt agataaatgt 8760
   atttttttta gtagttaagt tggaaatttg tgttgaaagt ataatagtaa gagtttttat 8820
   atgaaaaata tgtgaagaat ttgagtaagt gatagattta aagatggaaa atgattgttt 8880
   attttgatag taaaaataaa aagaatagaa ttgatatatt tttgtttaaa ttatttttta 8940
   tttgttgata tgaaatatta atatttaaat aaaaataaag gataaagtag tttttaaaaa 9000
   taatataaaa taagtaattt tgagtgaatg atttgtttat agtttatttt gggtgatatg 9060
   ggggtgttgt gatggaaaat gggttttggg tttaaaatta 9100
<210> 71
   <211> 9100
   <212> DNA
   <213> Artificial Sequence
<220>
   <223> chemically treated genomic DNA (Homo sapiens)
<400> 71
   tgattttgaa tttagaattt attttttatt atagtgtttt tatgttattt agggtaagtt      60
   gtggataggt tatttattta gagttgtttg ttttgtgttg tttttaaaaa ttgttttgtt     120
   ttttgttttt gtttgagtgt tggtgtttta tattggtaga tgaaaagtaa tttagatgaa     180
   aatatgttaa ttttgttttt tttattttta ttattaaaat agatgattat tttttgtttt     240
   tggatttatt atttgtttgg gttttttatg tgttttttat gtgaaaattt ttgttgttgt     300
   atttttagta taaattttta gtttggttgt tagggaagat gtatttattt gttttttttt     360
   tttgtttttt gatttttttt ttttttttat ttatatttgg tttttatttt ttttttgtgg     420
   agttgtggtt ttttttttgt gatgaaaaat gttatgtgta atgtttggga gttgtagttt     480
   tgtttgggtg gagaaagtgg tgttgtgata aatagtattg atattttata ttttgatttt     540
   gtgttatata tgttttttag aaatagattt agggttgtga agaagtttgg ataaggtgaa     600
   gatttttgga gagtttgggg atgtagttgt ttgggagagg aattaggttt ttttgtgatt     660
   tttttgtttt ttatggaaat taagaaatag tttttaaaat agagtttttg gggtagagag     720
   tgtaggtaat ttttttttta gttttttttt tagaggagta gttgaagtgg ttttggggaa     780
   tggaaaaggt aggtgtttat gaaggagatg gagttttggg gggtagttag ttatgttttg     840
   taggttttag tttgtgtttg ggttgttggt gtgtgttttt tttggtgttt gtttttgatg     900
   atgttgaggt tttggggtgt tgtggttgtg tttttttttt tttttttttt ttatgttgga     960
   tttggttggt atttggtttt tttttttttt ttttttgtgt ttatttgttg ttgtttggtt    1020
   tgagttgtgt ttgttttgtt ggttgagtgt aaaagggttt gatttgtggt tgattttggg    1080
   ttagtatttt ttttttatta attttgtttt ttttttatat aaaatttttg gttttgtttt    1140
   ttgtagtatt tattttaatt tttatttgtt ttttgttggt ggttgatatt ggtgtggatt    1200
   tgagggagat gggtttttag ggtgtttggg gatttttgtg tttttttgtt ttgaggttgt    1260
   ggggatgggg ttgaggtaat gaggggatta gtagggggag tgtgggttgt tttgagattt    1320
   gtgttttatt ttgttttgat ttttgattgg gtgtttaagg gttggagagg gatggagagg    1380
   ggagttgtgt gtgggttttg gtgggggagt tttaaagata aagaggaggt tgggttgtgt    1440
   tgagagaggt tggtggtgga gatgtagttt ggtgtgggag ttagagggtg gtggagatgg    1500
   tatagggatt ggagaggtgg tgggagtgga tggtgggggt ggggagggaa tgttgtgtgt    1560
   tgggatttta aggatagtgg tgtggtgtgt ggggtttagt tgtgggtaga gttggagaaa    1620
   gggtgatttg gagaggtgta ggtagggggg tgtgtttggg aggtttgagg ttttgaggag    1680
   gtttggttat ttttgtttgg aggggatatg tttggattag ttgtagatgt tgtttggttt    1740
   ttgggggtgg gaggagtgtg gttttggtgg gtgtgtagtt ggagaggaag ggttggggag    1800
   ggaggaggga gagagggagg gaagaatggg ggaggtgggg aggaagaggg aggaggagag    1860
   tttgttttta ggtaagggga ggtgattttg aggtttggtt tgggtgggag tagaggattg    1920
   ggtgaggggt ttttgtgttt ttattttttg gtttgggttt ttatgtgagt ttttttgttg    1980
   gtttttgttt tgttttgatg ttgttttatt ttgttggata ttgggggttt gtattttgtt    2040
   tatttttttt tatttggttt ggttttttag ttttgttaga tagttatgtt ggaaagagag    2100
   ttgtgttaag gttaagtttg ttttttattt gaagttttta aggatttaga ttgggggtta    2160
   aattttgttg ttgatgtttt tggagttgtt gtttttgggt ttaggttgag tgtgggtggg    2220
   gagggggtgg gagttggttg gagtttgatg gggttgaggg ggtgttggag tttgggttgg    2280
   tttgaagtga gtttttgggg gggggtgggt atatgtttgg ggatgggttt tttgaattaa    2340
   ttggaatttt ttggagatag aaatttgtgg tgtttagttt ttgttggggg aatgaaatgt    2400
   gggagtatga gtgttgtttt tttatatgtg tgattttaat agatttatta gttatgtttt    2460
   tgatgtgtat ttggagatta tgttgtgttt gagttttttt taaaattaat ttatttagta    2520
   tgttatgggt gggttttttt ggggaagggg atgttaaaat tttaataata aaaggggtgg    2580
   gtgagttgaa aaataaaata aggtaaaaat agaataaaat aaagtaaaaa aatttttaag    2640
   taaatttttt tatttttagt ttggagggta gggtttgagt gtaatggtag atttatgaag    2700
   ttatattgtt gtttttgtag aaattgatgt aaattttgta ttaggtttta aagattttta    2760
   tttgtttttt ttatatgggt tgtaaatata attggattaa gtgatagaag tattattttg    2820
   tgatttggga agtgaatgaa gttgttatat aatataataa ttattattat atagtatttt    2880
   attttgtgaa tatgatgagt gttattttgg ttaagtattg aatttatagt tggtttaggg    2940
   tttattgtaa tttttttttt attaatttga attggagtga tattgttggg ggtggggaag    3000
   gggggataga attttaattt tgagatgaat tttagttaat tttttgtttt ttattttgtg    3060
   atatttttaa atggagttat taaaatttta tatttgtttt tttttgtgtt gttttaatta    3120
   ataaaataag gaaataaggt ggttggagta tttggtattg gttattttag ttgattgtgt 3180
   atgaagtatt tgtaataaga tgttaaattg taaaagttgt ttgtttggtg ttttgggtgt 3240
   attaatttag ttttttatag tttgtgtttt taaagtgggg tttatatttt aattttgagt 3300
   taagatttag atgttaataa ttggatatag aaattttagg gtatatatag gggagtatag 3360
   gggttagatt gagagtggat gttagttaaa gaattttaga gagagttttt taagtttgta 3420
   tattttagag aaaattttta aggttttatt ggatttttaa ggttttggtg agttaataga 3480
   ataagtatta taaaattttt taaattattt tttggagagg attggggagg tttttatttt 3540
   ggattaagtt gtagtggtgt ttagttattt atgggtgtat agattttttt agaaataatt 3600
   gttttagaaa ggattaaata gttaaaagaa ataaaaataa agaaaaagaa aagagggggg 3660
   gggtttatta ttttgttaga aagtagtgga tattattttt gaattttttt gtaattattt 3720
   taatgaatga gtggtttaag taaaattatt aaaatgattt tttaaattat atgttaattg 3780
   ttagattagt attatattta aagttatttt tgaatgtagg ttgaatttgg tttttattaa 3840
   atttatagta taggtttatt tttggatgga tagtttatat tttaggataa ggaagggtgt 3900
   tgagaaagtg aggggaaatt taaggttgtt gaaataaatg tatttttatt tgggttttaa 3960
   gtgttttttt tttttaaaaa tataatttta ttttaatgtt gtgattaatt tttaaatgta 4020
   tttgttatag tataattttt aaatgtaata tttaatgttg ttagattatt tgaagataaa 4080
   gttttataga aatttttaaa ttttaaaaaa aaaattgtaa tgtattaata aatatttaga 4140
   aaataatata attaaaaaat ttaaagtatt aaataatttt aagattttgt taggaataat 4200
   gaataaggta ttttgtgttt atattggtta gtaatttgta ttttagttta tgtgggggag 4260
   ggtgatggtt gatatttttg tgtgtgtgag aatttagtat aattattttg tttgtttttt 4320
   taagagaaag aagttagggg gaggggtaga ggatggatgg agggggtttg gttgtagtat 4380
   gttttggttt agtgattgtg tttttttgaa atatatttag ataaattttg tttgaaattt 4440
   taattgttgg ggaattggga aatttgtgat ttgttttggt ttttgttttt tatttatggt 4500
   ttggtttagg gttttattat tggggttttt tttttttttt tttttttttg gattaaattg 4560
   tggtgtagat ttttagttag gtggaaggtg ttttgtttgt agtaaggtaa tgtgtagtag 4620
   ttttagttgt ttgtagtgaa atagttggag tagaagatat ttttattttt tggtttgtat 4680
   taatatttag ttttataaaa taatttatag gttttaattt tttttttttt tattgaaaaa 4740
   ggttttttga aaaattttgt ttttttattt tttggggttt ttttattttt agtttatata 4800
   gataattttt ttgttagatt tttttattgt attttgaatt tataaaatat gtatataaaa 4860
   atagtatgtt ataaatttat ggagaaaatg ggttttgttt tttaaatttg ttgatttgaa 4920
   atattttggg tggggggttt ttttttgttt ttagaagaaa ggttttttag aaaattatgt 4980
   atataatttg attttttttt tttgtattgt aatgttgaaa ataaaatttt ttataatgta 5040
   tttataagaa ataatataaa atttaaaaaa aaaaatggaa atgtttagat atataaagtt 5100
   ttataaaatt tagaatataa ttttgttgtt tgaaggaggg ggtggtggtg gtaatttttt 5160
   tgtataaatt aataaattaa tttaaaagag tggtaataat tataaaaaat ttgtaagtgt 5220
   ttggatttgt tttattatat ttataataaa tgtgatgagt tttgtgttat ttgggttttg 5280
   gtggttggtg ttgtttggaa gtaatgtgga ggaaagtgag tgtgttggtt ggtatataaa 5340
   agtgggtttt gttttttgtt ttgttttgat ttttttgttt ttttgagtaa agaaaatgtt 5400
   ttttgttttt tttgtaggtg ataaatattg tataaaaagt tttaagaaaa tattgaagtt 5460
   tagtttaaaa tttaagtttg gtgttagtgt ttggtggttg ttagggtttt tggggaaggt 5520
   gagtggggga tatgtgttat ttggtgtttg tttttagtat tttttttttg ataagaatgt 5580
   gggaatgttt gtataaaagt tatgttgtgt tggggtgggg tgggagttgg gtgtgggttg 5640
   tgagtttgtg ggttgttgtt gtagagtttt tggtgttttg aaggtgttag atgtgtgtta 5700
   ggggtattgt gttgtttatg tttgtgtttg tgttttggta gtgttgtggt agtgagtgta 5760
   gttggttttg tgttgtggtt gttgttgttg ttgttgggtt gttttttttg ttggtgggta 5820
   agtatatgtt gattattttg tgtaggtttt tggggtatgg tgtttgtgag tgtgttgggg 5880
   tgggtgggtt gttgttgggt tttgatttta ttagagagtt tagtgtgttt agggtgtttg 5940
   atgatgtggt tgttgttgtt gttgttgttg ttatggttga gttttggtgt gtgttgtttg 6000
   tagtggtggt ggtggtggtt gtggtgttgt aggggaggtt gttgtagttt gagggtgatg 6060
   tgtttatgta gttttgtgag ttggagatgg ggttgtattg tagtgtgttt atgttgtagt 6120
   ggtgtatggg ttgtgggttg tgtgggtgtg tgtgtgggtg gtgtgggtgt gggtgtgtgg 6180
   ggtgtgtgtg tgggtgtgtg ttgtttgtgt ttgggtgttg tttgtaggtt agttgtgttt 6240
   tttgtattat ggttgtggtt gttgttgttg ttgttattga gttggggtag gtgttgttgg 6300
   tttagttgtt gatgtgtgtg tagttgttgt ttgttgtgtt gtttgggttt tttaggtgtt 6360
   ggtttatggt tgttgtgttt atgtttatgt ttatgtttat ggttatagtt gtgttgttgt 6420
   tatttgtgtt ggttgttagg agtttgttgg ttagtgagta tttgtttttt ttgagtagtg 6480
   ttttggtttt gtggtgtggt tggtatttgt aatttgggtg tttttttatg tgtagtgtgt 6540
   gtagttgttt ggttttgttg atgaatggtt gttttttggt tttggatatg attttttatt 6600
   tggtttttag gtgtttgttg atttttgagt tgtgtatttt ggggtttttt tgggttattt 6660
   tgtgttgttg tttgtgggat tatattatga aggtgtttat gggttgtttg atttggtttt 6720
   ggttggtttt ggtgtttttg ttgttgttgt tgtttttgtt tttgtttttg ttgttgtttg 6780
   ttttggtggg gtttgagagg tttgtggggg tttgggtgtt gttgggtgag tgtaggttgg 6840
   tttttattat tatgttgtat attggggtgg ttggtgggtt tattggtatt gtgaggagag 6900
   ggtttggagt atagatggtt ggggtgggag ggagatggag aggaatttag atggagtgag 6960
   atgggggtgg gggggtttta gtggagagaa gaatggggag gggtggaggt aatgataata 7020
   aaaaaaggta gaaatatatg tatttggtgt tggtttggtt tgtaggtgga aagttttttt 7080
   tgatgtataa attatttgtt aaagaggttg atggttgggg ttgttgtttt tgttgttatt 7140
   gtagtttagg tgtttttggt ggagtggtgt aaataggtgt agttgtggtt aagttgtagt 7200
   tttgtttttg gggttgggag tggtgtgttg ggtttggttt ggggtgtgtt gtttttttgt 7260
   tggttgttgt tgtgtgttgt tttttttttt ttgtgtttgg tttttagtgg tttttaattt 7320
   ggaggttgta atttttagta gggtggtgtg ttgtgtgtgt tgggtatgtg ttggttttag 7380
   gtgtttgggg gaagttaggt gttagtggtg ggggggggga ggtgtagggg ggtttgtagg 7440
   gggtgattat ggtgtagtta gggaggggtt tggggatagt gggaggagtg tgggtttgtg 7500
   gagagggttg gagagttggg gttgttgagt ttgggatggg ttgtttgttg tttgtttggg 7560
   gttgtgtgtt tggagtggag gtgtatgtgg ggttggtggt gtgtggggtt tagtagttat 7620
   atgttgggtt tgggttgtta ttaaatgagt gtgttgtggt taatgggagt tggtggtggt 7680
   ggtgatttgt atggtgtgtt ggtgagtgat gtggggaggg agtagatata ttgggtgtgg 7740
   gtggtggagt ggggaggagg ggaggaagga gggggagtaa agtaggaagg aggggggagg 7800
   aggtttggga gggggtgata gttatattgt tttttgagtg tgtgtgtgtg gtggtggtgg 7860
   tggtggtgaa ttttgagttt gttttttttg ttgttttttg tttgtgtgtt ttgaatgatt 7920
   aatgattggg ttgaaaagtg aagattaaga aaggattgtt gtggggtggg gggaggaaat 7980
   gtagtttttg gttaggttta aatgggtgta gtggagattt agtttttttt ttgtttttgt 8040
   tggttaattt tgtgtttgta gggaagggga aagttgagtg tggggttggt atgttgtttt 8100
   tagttttttt gagtgttgaa aaggaggggt gtttttttgg atgtagggga tattttttga 8160
   gggtttttgt aggttttttg taaggattag gtggtttagt tattttttgt tttgtatttg 8220
   tttttggttg tggtgtgtat tgaagtttgt tagtgtttgt ttttgtagtg aaggttttta 8280
   tagtttaggt tttataattt gggtaaaggg ggagtatttg tgtgggtatg gttttatagt 8340
   tttgtttagt attagtttta aatttttgtg gggaggagtt ttttaatgag tttggggtta 8400
   tagttatgta ttttttttaa tttggatttt ttttttttgg aaaaggaatt tttaggaagg 8460
   ttgaatataa taagatttat ttggaattta gtttttgaat aatagttgaa aaagtttgtt 8520
   gaattgtaaa ataatgatat ggtgtatgtt tttttggagt gaatttttta tgagttggtt 8580
   gtttaagaat ggagaaaatt gtgtgaattg tgtggggtta tataaattga atgaaaggtg 8640
   gggttttagg atgggtttta gttgttggtt tgtgttatgt aaaggaaagg tttggtttgt 8700
   ggaaagagta aggtatttat ttgtggttat tatttttaaa ttttaaagtg gttttgattt 8760
   gggaatgtga aagagatttt taaatgtagt tttaagtagt atagtgttta tattattgtt 8820
   tttattagat tgatttgaaa gtggtttaaa gatgattttt gagtttaagt attttttttt 8880
   tgtttttttt gaggtatttt gagatgagag tgtttgtgga gattggtggt gggtaaagta 8940
   gaatttttat tgtatgttat atgtttgttt gttttgtagt aggaatattt agtaaagatg 9000
   atgataatgg taataggagt aataggatgt ttgagtttgt tgggtagtga atatgtgata 9060
   tttttttttt ttttttaaaa aaatttttga ggttgtgaat 9100
<210> 72
   <211> 8500
   <212> DNA
   <213> Artificial Sequence
<220>
   <223> chemically treated genomic DNA (Homo sapiens)
<400> 72
   gttatttgag attgtgtttg aaaatttttt ttatgttttt aaattaaagt tattttgagg      60
   tttaagaatg ataattatag gtgaatgttt tatttttttt atgagttagg tttttttttt     120
   gtatgatgta gattggtggt tgagatttgt tttgaggttt tgttttttat ttggtttatg      180
   tggttttgtg tagtttatgt agtttttttt gtttttaggt aattagtttg tgggaaattt     240
   attttagaaa agtgtgtgtt atattattat tttgtgattt aataaatttt tttaattgtt     300
   gtttaggaat tagattttag atagattttg ttgtgtttgg tttttttaga aatttttttt     360
   ttagaggaag aagatttggg ttgggaagag tgtgtgatta tggttttggg tttattgaaa     420
   gatttttttt tataaaagtt tagggttgat attaaatgaa gttatggagt tatgtttata     480
   taaatatttt ttttttattt gaattgtggg gtttggattg tgaaggtttt tgttgtaaga      540
   gtgggtattg gtgaatttta gtgtatgttg tggttgagaa tggatgtagg gtgggggatg     600
   gttgagttgt ttgatttttg tagagaattt gtaggggttt ttggagggta ttttttgtgt       660
   ttaagggagt gttttttttt tttagtattt gggggaattg agggtgatgt gttagttttg     720
   tatttaattt tttttttttt tgtaggtata gagttggttg gtgggggtag aggaggagtt     780
   gggtttttat tgtgtttgtt taaatttggt taggggttgt gttttttttt tttattttat     840
   gatgattttt ttttagtttt tgttttttaa tttaattgtt aattatttgg aatgtgtggg      900
   tggggagtgg tgaggagggt gagtttgggg tttgttgttg ttgttgttgt tgtgtgtgtg     960
   tgtttaggaa gtggtgtggt tgttattttt ttttgggttt tttttttttt tttttttgtt    1020
   ttgttttttt tttttttttt tttttttttt gttttgttgt ttgtgtttag tgtatttatt    1080
   ttttttttat gttatttgtt agtgtgttat gtaaattatt gttgttgttg gtttttattg    1140
   gttgtggtgt gtttatttaa tggtagtttg ggtttggtgt atggttgttg ggttttgtgt 1200
   gttgttggtt ttgtgtgtgt ttttgttttg agtgtatggt tttgggtagg tagtgggtag 1260
   tttattttgg gtttggtggt tttggttttt tggttttttt tgtgagtttg tgtttttttt 1320
   gttgtttttg ggtttttttt tggttgtatt gtaattgttt tttgtaggtt tttttgtgtt 1380
   tttttttttt tgttattggt gtttggtttt ttttgggtat ttgggattag tatatgttta 1440
   gtgtatgtgg tgtgttgttt tgttagaagt tgtagttttt gagttggagg ttgttgagga 1500
   ttgagtgtag gaggaaggag atagtgtgta gtggtggttg gtgaggagat agtatatttt 1560
   gggttgggtt tagtgtattg tttttggttt taaaagtgga gttgtaattt ggttatgatt 1620
   gtatttgttt gtattgtttt gttgagggtg tttgggttgt ggtggtggtg aagatggtga 1680
   ttttgattgt tggttttttt ggtaagtggt ttgtgtatta ggagaaattt tttatttgtg 1740
   agttgaattg gtgttgagtg tgtgtgtttt tgtttttttt tgttgttgtt gtttttattt 1800
   ttttttattt ttttttttgt taggattttt ttgtttttgt tttattttgt ttgaattttt 1860
   ttttgttttt tttttatttt ggttgtttat gttttaggtt ttttttttgt ggtgttggtg 1920
   aatttgttag ttgttttgat gtatagtatg atgatggaga ttgatttgta tttgtttggt 1980
   ggtgtttagg tttttatgaa ttttttgggt tttgttgggg tgggtggtgg tgggggtgga 2040
   ggtgggggtg gtggtggtgg tgggggtgtt aaggttaatt aggattgggt taaatggttt 2100
   atgaatgttt ttatggtgtg gttttgtggg tagtggtgta agatggttta ggagaatttt 2160
   aagatgtata atttggagat tagtaagtgt ttgggggttg agtggaaggt tatgtttgag 2220
   gttgagaagt ggttgtttat tgatgaggtt aagtggttgt gtgtgttgta tatgaaggag 2280
   tatttggatt ataagtattg gttgtgttgt aagattaaga tgttgtttaa gaaggataag 2340
   tatttgttgg ttggtgggtt tttggtggtt ggtgtgggtg gtggtggtgt ggttgtggtt 2400
   atgggtgtgg gtgtgggtgt gggtgtggtg gttgtgggtt agtgtttgga gagtttaggt 2460
   ggtgtggtgg gtggtggtta tgtgtatgtt aatggttggg ttaatggtgt ttattttggt 2520
   ttggtggtgg tggtggtggt ggttgtggtt atgatgtagg aggtgtagtt ggtttatggg 2580
   tagtatttgg gtgtgggtgg tgtgtatttg tatgtgtatt ttgtgtattt gtatttgtat 2640
   tatttgtatg tgtatttgta taatttgtag tttatgtatt gttatgatat gggtgtgttg 2700
   tagtatagtt ttatttttaa tttgtagggt tatatgagtg tgttgttttt gggttatggt 2760
   ggtttttttt atggtgttgt ggttgttgtt gttgttgttg tgggtggtgt gtattagaat 2820
   ttggttgtgg tggtggtggt ggtggtggtg gttgtgttgt tgggtgtttt gggtgtgttg 2880
   ggttttttgg tgaagttgga gtttagtggt agtttgtttg ttttagtgta tttgtgggtg 2940
   ttgtgttttg gggatttgtg tgagatgatt agtatgtatt tgtttgttgg tgaggggggt 3000
   gatttggtgg tggtagtagt ggttgtggtg tagagttggt tgtatttgtt gttgtagtat 3060
   tattagggtg tgggtgtggg tgtgaatggt atggtgtttt tgatgtatat ttagtgtttt 3120
   tgggatgttg gggattttgt ggtggtgatt tatgagtttg tggtttgtgt ttggtttttg 3180
   ttttgttttg gtgtggtgtg gtttttgtat agatgttttt atatttttgt taaaaggaaa 3240
   atattggaga tgaatgttgg gtgatgtgtg ttttttattt attttttttg gagattttgg 3300
   tgattgttgg gtgttgatat tagatttggg ttttagattg aattttggtg tttttttgag 3360
   attttttgta tagtatttat tatttatgga ggaagtggaa agtgtttttt ttgtttgagg 3420
   ggataaaaaa gttaaaatga ggtgagaggt gaagtttatt tttgtatatt ggttggtgtg 3480
   tttatttttt tttgtgttgt ttttggatgg tgttgattgt tggagtttaa gtgatgtgga 3540
   gtttgttgta tttgttataa atgtagtaag gtaggtttaa gtatttataa gttttttgta 3600
   gttgttattg tttttttggg ttggtttgtt aatttatata aagagattat tattattatt 3660
   ttttttttta gatggtggag ttatattttg ggttttgtaa aattttatgt atttgagtat 3720
   ttttattttt ttttttgggt tttgtattat tttttgtaaa tgtattgtga aaaattttat 3780
   ttttggtgtt gtaatgtggg gaggagaagt tagattatgt atatagtttt ttaaaaagtt 3840
   ttttttttaa aaatgaaaaa agatttttta tttaaaatgt tttgagttaa taaatttaag 3900
   agatagagtt tatttttttt ataaatttgt aatatgttat ttttatgtgt atgttttatg 3960
   agtttaaaat gtaatgagga aatttgatag ggaaattatt tgtatgaatt aaaagtaagg 4020
   gaattttggg gaatgggagg ataggatttt ttaaggaatt ttttttaatg aaagagaagg 4080
   aagttaaaat ttataggtta ttttgtagag ttgagtgtta atatgggttg agaaataaaa 4140
   gtattttttg ttttggttgt tttattgtgg atggttgggg ttgttgtgtg ttattttgtt 4200
   gtaagtgggg tgttttttat ttggttgggg gtttgtgtta tagtttggtt tagaggaggg 4260
   aggaggaagg gaagatttta gtggtgggat tttggattag gttatggatg aaggataaag 4320
   attagggtag gttatgggtt ttttaatttt ttagtaatta agattttgag tagaatttat 4380
   ttaaatgtgt tttaaggaaa tataattgtt gaattaaaat gtattgtagt tgagtttttt 4440
   ttgtttattt tttgtttttt tttttggttt tttttttttg ggaaaatggg taaaataatt 4500
   gtgttggatt tttatatata tagaaatatt gattattatt tttttttgtg tgaattggga 4560
   tgtaagttgt taattgatgt gaatgtaaaa tgttttgttt attatttttg atgagatttt 4620
   gaggttgttt gatgttttaa attttttaat tatattattt tttaggtgtt tattggtata 4680
   ttgtagtttt ttttttgaaa tttaaaaatt tttgtaaaat tttgttttta agtaatttga 4740
   tagtattaaa tattgtattt aaaaattata ttgtagtaaa tatatttaaa aattaattat 4800
   aatgttaaga tgaaattata tttttggaaa aaaaaaatat ttgaagttta gatggaaata 4860
   tgtttatttt agtagtttta ggtttttttt tgttttttta atattttttt ttgttttgga 4920
   gtatggattg tttgtttaaa agtgagttta tgttataagt ttaatgagaa ttgaatttag 4980
   tttgtatttg agaatagttt taagtataat gttgatttga taattgatgt gtaatttggg 5040
   aagttatttt gataattttg tttaaattat ttatttgtta aagtgattat aaaaaagttt 5100
   aagaatgatg tttattgttt tttaataaga taataaattt tttttttttt tttttttttt 5160
   ttatttttat tttttttagt tatttgattt tttttgaagt agttgttttt ggaagagttt 5220
   gtgtgtttat ggatggttga gtattattat gatttagttt gggataaggg tttttttagt 5280
   tttttttggg agatgatttg ggaaatttta taatgtttgt tttgttaatt tattgggatt 5340
   ttgagggttt aatgggattt tgagggtttt ttttgaaata tataaattta aaggattttt 5400
   tttgaggttt tttgattgat gtttattttt agtttggttt ttgtgttttt ttgtgtgtat 5460
   tttggagttt ttgtgtttaa ttgttggtat ttaggttttg gtttaagatt aggatgtggg 5520
   ttttatttta gaggtataga ttatgaaaag ttgagttagt gtgtttggga tgttaggtaa 5580
   gtagttttta tagtttggta ttttattgta ggtgttttgt gtatagttag ttgaaatagt 5640
   taatgttagg tgttttaatt attttatttt tttgttttgt tgattagaat aatatagaaa 5700
   aaagtaaata taaattttta atgattttat ttaaaaatat tatagggtgg gggtaaggaa 5760
   attagttgag atttatttta ggattgagat tttatttttt tttttttgtt tttagtagtg 5820
   ttgttttaat ttaaattagt ggagaaaaga ttatagtagg ttttgagttg attgtgaatt 5880
   tggtgtttgg ttaaggtaat atttattgta tttatggagt gaaatattat atgatgatag 5940
   ttattatatt atatgatgat tttatttatt ttttaaatta tagggtggtg tttttgttgt 6000
   ttagtttaat tatgtttgta atttgtgtag ggaaggtgga taggagtttt tgaggtttag 6060
   tgtaaagttt gtattagttt ttgtgggggt gatagtgtga ttttgtaaat ttgttattgt 6120
   gtttgggttt tgttttttaa attgagggta ggagggttta tttgagggtt tttttgtttt 6180
   gttttgtttt gtttttgttt tgttttgttt tttaatttat ttgttttttt tgttgttgag 6240
   attttgatgt tttttttttt ggagaaattt atttgtaatg tattagatgg attagtttta 6300
   aagaagattt gggtatagtg tgatttttag atgtgtgttg ggagtgtgat tgataggttt 6360
   attaaaatta tgtgtgtggg agaatggtat ttatgttttt gtgttttatt tttttggtaa 6420
   agattgggtg ttgtagattt ttgtttttgg aaaattttag ttaatttaag aaatttattt 6480
   ttggatgtgt gtttgttttt ttttgaaggt ttgttttagg ttagtttgga ttttagtgtt 6540
   tttttgattt tgttgggttt tggttgattt ttgttttttt tttatttatg tttggtttgg 6600
   gtttgggggt agtggtttta gaggtgttgg tggtagaatt taatttttgg tttgagtttt 6660
   tggaaatttt aagtgaaaag tagatttggt tttggtgtgg tttttttttt agtgtgatta 6720
   tttggtgaga ttgaaaggtt aggttagatg gggaaaagtg agtggggtgt gggtttttag 6780
   tatttggtgg ggtggggtgg tgttaggatg gagtgagggt tggtgggggg gtttgtgtgg 6840
   aggtttaggt tgggaggtgg ggatgtaggg attttttatt tagttttttg tttttgtttg 6900
   agttaggttt tgggattatt tttttttgtt tgagggtgag tttttttttt tttttttttt 6960
   ttttgttttt tttgtttttt tttttttttt tttttttttt ttttttggtt tttttttttt 7020
   agttgtgtgt ttgttgggat tatgtttttt ttgtttttgg gagttgagtg gtgtttgtgg 7080
   ttggtttgga tgtgtttttt ttgggtgggg gtggttgggt ttttttgggg ttttgggttt 7140
   tttgggtgta tttttttgtt tgtgtttttt taagttgttt tttttttggt tttgtttgtg 7200
   gttgagtttt gtatattgtg ttgttgtttt tggggttttg gtatatggtg tttttttttt 7260
   gtttttgtta tttattttta ttgttttttt agtttttgtg ttgtttttat tgttttttga 7320
   tttttgtatt gagttgtgtt tttgttgttg gtttttttta gtgtggtttg gttttttttt 7380
   tgtttttgaa gtttttttgt tgaggtttgt gtgtggtttt tttttttgtt tttttttggt 7440
   ttttgggtgt ttagttggag gttggggtgg gatggggtat gagttttggg gtggtttgtg 7500
   ttttttttat tagttttttt attgttttgg ttttgttttt gtagttttgg ggtgggaggg 7560
   tgtaggggtt tttgggtgtt ttgggagttt atttttttta ggtttgtgtt ggtgttagtt 7620
   gttggtgggg ggtgggtgga gattaagatg agtgttgtga gggatggggt tgagggtttt 7680
   gtgtagaagg agagtaggat tagtggagag aaagtgttga tttggggtta gttgtaagtt 7740
   aaattttttt atatttagtt ggtggaatga gtatggtttg ggttggatgg taataagtgg 7800
   gtgtgaaggg agggggaggg gaggttgggt attggttggg tttggtgtga gagaggggag 7860
   ggggagggga tgtggttata gtgttttagg gttttagtgt tattggggat gggtgttgag 7920
   gggagtgtgt attagtgatt tgggtgtgga ttggggtttg tagggtgtga ttggttgttt 7980
   tttaggattt tatttttttt ataaatattt gtttttttta ttttttgggg ttgttttggt 8040
   tgtttttttg gagggaagat tggggagaag gttgtttgtg ttttttgttt tagaggtttt 8100
   gttttagaaa ttgtttttta atttttataa gaaataaaaa aattatagga aggtttggtt 8160
   ttttttttgg atgattgtgt ttttaggttt tttaggaatt tttgttttat ttgagttttt 8220
   ttgtagtttt ggatttattt ttggggggtg tgtgtgatgt agaattgggg tataaaatat 8280
   taatgttgtt tgttatgata ttgttttttt tatttgggta gagttgtggt ttttgggtgt 8340
   tgtgtgtggt gttttttatt atggagggaa agttgtggtt ttgtgggaaa ggagtgaaaa 8400
   ttgagtgtgg gtgaagagga gagggaggtt agggggtaag ggaagaaagt agataaatgt 8460
   atttttttta gtagttaagt tggaaatttg tgttgaaagt 8500
<210> 73
   <211> 8500
   <212> DNA
   <213> Artificial Sequence
<220>
   <223> chemically treated genomic DNA (Homo sapiens)
<400> 73
   atttttagta taaattttta gtttggttgt tagggaagat gtatttattt gttttttttt      60
   tttgtttttt gatttttttt ttttttttat ttatatttgg tttttatttt ttttttgtgg     120
   agttgtggtt ttttttttgt gatgaaaaat gttatgtgta atgtttggga gttgtagttt     180
   tgtttgggtg gagaaagtgg tgttgtgata aatagtattg atattttata ttttgatttt     240
   gtgttatata tgttttttag aaatagattt agggttgtga agaagtttgg ataaggtgaa     300
   gatttttgga gagtttgggg atgtagttgt ttgggagagg aattaggttt ttttgtgatt     360
   tttttgtttt ttatggaaat taagaaatag tttttaaaat agagtttttg gggtagagag     420
   tgtaggtaat ttttttttta gttttttttt tagaggagta gttgaagtgg ttttggggaa     480
   tggaaaaggt aggtgtttat gaaggagatg gagttttggg gggtagttag ttatgttttg     540
   taggttttag tttgtgtttg ggttgttggt gtgtgttttt tttggtgttt gtttttgatg     600
   atgttgaggt tttggggtgt tgtggttgtg tttttttttt tttttttttt ttatgttgga     660
   tttggttggt atttggtttt tttttttttt ttttttgtgt ttatttgttg ttgtttggtt     720
   tgagttgtgt ttgttttgtt ggttgagtgt aaaagggttt gatttgtggt tgattttggg     780
   ttagtatttt ttttttatta attttgtttt ttttttatat aaaatttttg gttttgtttt     840
   ttgtagtatt tattttaatt tttatttgtt ttttgttggt ggttgatatt ggtgtggatt     900
   tgagggagat gggtttttag ggtgtttggg gatttttgtg tttttttgtt ttgaggttgt     960
   ggggatgggg ttgaggtaat gaggggatta gtagggggag tgtgggttgt tttgagattt    1020
   gtgttttatt ttgttttgat ttttgattgg gtgtttaagg gttggagagg gatggagagg    1080
   ggagttgtgt gtgggttttg gtgggggagt tttaaagata aagaggaggt tgggttgtgt    1140
   tgagagaggt tggtggtgga gatgtagttt ggtgtgggag ttagagggtg gtggagatgg    1200
   tatagggatt ggagaggtgg tgggagtgga tggtgggggt ggggagggaa tgttgtgtgt    1260
   tgggatttta aggatagtgg tgtggtgtgt ggggtttagt tgtgggtaga gttggagaaa    1320
   gggtgatttg gagaggtgta ggtagggggg tgtgtttggg aggtttgagg ttttgaggag    1380
   gtttggttat ttttgtttgg aggggatatg tttggattag ttgtagatgt tgtttggttt    1440
   ttgggggtgg gaggagtgtg gttttggtgg gtgtgtagtt ggagaggaag ggttggggag    1500
   ggaggaggga gagagggagg gaagaatggg ggaggtgggg aggaagaggg aggaggagag    1560
   tttgttttta ggtaagggga ggtgattttg aggtttggtt tgggtgggag tagaggattg    1620
   ggtgaggggt ttttgtgttt ttattttttg gtttgggttt ttatgtgagt ttttttgttg    1680
   gtttttgttt tgttttgatg ttgttttatt ttgttggata ttgggggttt gtattttgtt    1740
   tatttttttt tatttggttt ggttttttag ttttgttaga tagttatgtt ggaaagagag    1800
   ttgtgttaag gttaagtttg ttttttattt gaagttttta aggatttaga ttgggggtta    1860
   aattttgttg ttgatgtttt tggagttgtt gtttttgggt ttaggttgag tgtgggtggg    1920
   gagggggtgg gagttggttg gagtttgatg gggttgaggg ggtgttggag tttgggttgg    1980
   tttgaagtga gtttttgggg gggggtgggt atatgtttgg ggatgggttt tttgaattaa    2040
   ttggaatttt ttggagatag aaatttgtgg tgtttagttt ttgttggggg aatgaaatgt    2100
   gggagtatga gtgttgtttt tttatatgtg tgattttaat agatttatta gttatgtttt    2160
   tgatgtgtat ttggagatta tgttgtgttt gagttttttt taaaattaat ttatttagta    2220
   tgttatgggt gggttttttt ggggaagggg atgttaaaat tttaataata aaaggggtgg   2280
   gtgagttgaa aaataaaata aggtaaaaat agaataaaat aaagtaaaaa aatttttaag    2340
   taaatttttt tatttttagt ttggagggta gggtttgagt gtaatggtag atttatgaag    2400
   ttatattgtt gtttttgtag aaattgatgt aaattttgta ttaggtttta aagattttta    2460
   tttgtttttt ttatatgggt tgtaaatata attggattaa gtgatagaag tattattttg    2520
   tgatttggga agtgaatgaa gttgttatat aatataataa ttattattat atagtatttt    2580
   attttgtgaa tatgatgagt gttattttgg ttaagtattg aatttatagt tggtttaggg    2640
   tttattgtaa tttttttttt attaatttga attggagtga tattgttggg ggtggggaag    2700
   gggggataga attttaattt tgagatgaat tttagttaat ttttttgttt ttattttgtg   2760
   atatttttaa atggagttat taaaaattta tatttgtttt tttttgtgtt gttttaatta    2820
   ataaaataag gaaataaggt ggttggagta tttggtattg gttattttag ttgattgtgt    2880
   atgaagtatt tgtaataaga tgttaaattg taaaagttgt ttgtttggtg ttttgggtgt    2940
   attaatttag ttttttatag tttgtgtttt taaagtgggg tttatatttt aattttgagt    3000
   taagatttag atgttaataa ttggatatag aaattttagg gtatatatag gggagtatag    3060
   gggttagatt gagagtggat gttagttaaa gaattttaga gagagttttt taagtttgta    3120
   tattttagag aaaattttta aggttttatt ggatttttaa ggttttggtg agttaataga    3180
   ataagtatta taaaattttt taaattattt tttggagagg attggggagg tttttatttt    3240
   ggattaagtt gtagtggtgt ttagttattt atgggtgtat agattttttt agaaataatt    3300
   gttttagaaa ggattaaata gttaaaagaa ataaaaataa agaaaaagaa aagagggggg    3360
   gggtttatta ttttgttaga aagtagtgga tattattttt gaattttttt gtaattattt    3420
   taatgaatga gtggtttaag taaaattatt aaaatgattt tttaaattat atgttaattg    3480
   ttagattagt attatattta aagttatttt tgaatgtagg ttgaatttgg tttttattaa    3540
   atttatagta taggtttatt tttggatgga tagtttatat tttaggataa ggaagggtgt    3600
   tgagaaagtg aggggaaatt taaggttgtt gaaataaatg tatttttatt tgggttttaa    3660
   gtgttttttt tttttaaaaa tataatttta ttttaatgtt gtgattaatt tttaaatgta 3720
   tttgttatag tataattttt aaatgtaata tttaatgttg ttagattatt tgaagataaa 3780
   gttttataga aatttttaaa ttttaaaaaa aaaattgtaa tgtattaata aatatttaga 3840
   aaataatata attaaaaaat ttaaagtatt aaataatttt aagattttgt taggaataat 3900
   gaataaggta ttttgtgttt atattggtta gtaatttgta ttttagttta tgtgggggag 3960
   ggtgatggtt gatatttttg tgtgtgtgag aatttagtat aattattttg tttgtttttt 4020
   taagagaaag aagttagggg gaggggtaga ggatggatgg agggggtttg gttgtagtat 4080
   gttttggttt agtgattgtg tttttttgaa atatatttag ataaattttg tttgaaattt 4140
   taattgttgg ggaattggga aatttgtgat ttgttttggt ttttgttttt tatttatggt 4200
   ttggtttagg gttttattat tggggttttt tttttttttt tttttttttg gattaaattg 4260
   tggtgtagat ttttagttag gtggaaggtg ttttgtttgt agtaaggtaa tgtgtagtag 4320
   ttttagttgt ttgtagtgaa atagttggag tagaagatat ttttattttt tggtttgtat 4380
   taatatttag ttttataaaa taatttatag gttttaattt tttttttttt tattgaaaaa 4440
   ggttttttga aaaattttgt ttttttattt tttggggttt ttttattttt agtttatata 4500
   gataattttt ttgttagatt tttttattgt attttgaatt tataaaatat gtatataaaa 4560
   atagtatgtt ataaatttat ggagaaaatg ggttttgttt tttaaatttg ttgatttgaa 4620
   atattttggg tgggggtttt ttttttgttt ttagaagaaa ggttttttag aaaattatgt 4680
   atataatttg attttttttt tttgtattgt aatgttgaaa ataaaatttt ttataatgta 4740
   tttataagaa ataatataaa atttaaaaaa aaaaatggaa atgtttagat atataaagtt 4800
   ttataaaatt tagaatataa ttttgttgtt tgaaggaggg ggtggtggtg gtaatttttt 4860
   tgtataaatt aataaattaa tttaaaagag tggtaataat tataaaaaat ttgtaagtgt 4920
   ttggatttgt tttattatat ttataataaa tgtgatgagt tttgtgttat ttgggttttg 4980
   gtggttggtg ttgtttggaa gtaatgtgga ggaaagtgag tgtgttggtt ggtatataaa 5040
   agtgggtttt gttttttgtt ttgttttgat ttttttgttt ttttgagtaa agaaaatgtt 5100
   ttttgttttt tttgtaggtg ataaatattg tataaaaagt tttaagaaaa tattgaagtt 5160
   tagtttaaaa tttaagtttg gtgttagtgt ttggtggttg ttagggtttt tggggaaggt 5220
   gagtggggga tatgtgttat ttggtgtttg tttttagtat tttttttttg ataagaatgt 5280
   gggaatgttt gtataaaagt tatgttgtgt tggggtgggg tgggagttgg gtgtgggttg 5340
   tgagtttgtg ggttgttgtt gtagagtttt tggtgttttg aaggtgttag atgtgtgtta 5400
   ggggtattgt gttgtttatg tttgtgtttg tgttttggta gtgttgtggt agtgagtgta 5460
   gttggttttg tgttgtggtt gttgttgttg ttgttgggtt gttttttttg ttggtgggta 5520
   agtatatgtt gattattttg tgtaggtttt tggggtatgg tgtttgtgag tgtgttgggg 5580
   tgggtgggtt gttgttgggt tttgatttta ttagagagtt tagtgtgttt agggtgtttg 5640
   atgatgtggt tgttgttgtt gttgttgttg ttatggttga gttttggtgt gtgttgtttg 5700
   tagtggtggt ggtggtggtt gtggtgttgt aggggaggtt gttgtagttt gagggtgatg 5760
   tgtttatgta gttttgtgag ttggagatgg ggttgtattg tagtgtgttt atgttgtagt 5820
   ggtgtatggg ttgtgggttg tgtgggtgtg tgtgtgggtg gtgtgggtgt gggtgtgtgg 5880
   ggtgtgtgtg tgggtgtgtg ttgtttgtgt ttgggtgttg tttgtaggtt agttgtgttt 5940
   tttgtattat ggttgtggtt gttgttgttg ttgttattga gttggggtag gtgttgttgg 6000
   tttagttgtt gatgtgtgtg tagttgttgt ttgttgtgtt gtttgggttt tttaggtgtt 6060
   ggtttatggt tgttgtgttt atgtttatgt ttatgtttat ggttatagtt gtgttgttgt 6120
   tatttgtgtt ggttgttagg agtttgttgg ttagtgagta tttgtttttt ttgagtagtg 6180
   ttttggtttt gtggtgtggt tggtatttgt aatttgggtg tttttttatg tgtagtgtgt 6240
   gtagttgttt ggttttgttg atgaatggtt gttttttggt tttggatatg attttttatt 6300
   tggtttttag gtgtttgttg atttttgagt tgtgtatttt ggggtttttt tgggttattt 6360
   tgtgttgttg tttgtgggat tatattatga aggtgtttat gggttgtttg atttggtttt 6420
   ggttggtttt ggtgtttttg ttgttgttgt tgtttttgtt tttgtttttg ttgttgtttg 6480
   ttttggtggg gtttgagagg tttgtggggg tttgggtgtt gttgggtgag tgtaggttgg 6540
   tttttattat tatgttgtat attggggtgg ttggtgggtt tattggtatt gtgaggagag 6600
   ggtttggagt atagatggtt ggggtgggag ggagatggag aggaatttag atggagtgag 6660
   atgggggtgg gggggtttta gtggagagaa gaatggggag gggtggaggt aatgataata 6720
   aaaaaaggta gaaatatatg tatttggtgt tggtttggtt tgtaggtgga aagttttttt 6780
   tgatgtataa attatttgtt aaagaggttg atggttgggg ttgttgtttt tgttgttatt 6840
   gtagtttagg tgtttttggt ggagtggtgt aaataggtgt agttgtggtt aagttgtagt 6900
   tttgtttttg gggttgggag tggtgtgttg ggtttggttt ggggtgtgtt gtttttttgt 6960
   tggttgttgt tgtgtgttgt tttttttttt ttgtgtttgg tttttagtgg tttttaattt 7020
   ggaggttgta atttttagta gggtggtgtg ttgtgtgtgt tgggtatgtg ttggttttag 7080
   gtgtttgggg gaagttaggt gttagtggtg ggggggggga ggtgtagggg ggtttgtagg 7140
   gggtgattat ggtgtagtta gggaggggtt tggggatagt gggaggagtg tgggtttgtg 7200
   gagagggttg gagagttggg gttgttgagt ttgggatggg ttgtttgttg tttgtttggg 7260
   gttgtgtgtt tggagtggag gtgtatgtgg ggttggtggt gtgtggggtt tagtagttat 7320
   atgttgggtt tgggttgtta ttaaatgagt gtgttgtggt taatgggagt tggtggtggt 7380
   ggtgatttgt atggtgtgtt ggtgagtgat gtggggaggg agtagatata ttgggtgtgg 7440
   gtggtggagt ggggaggagg ggaggaagga gggggagtaa agtaggaagg aggggggagg 7500
   aggtttggga gggggtgata gttatattgt tttttgagtg tgtgtgtgtg gtggtggtgg 7560
   tggtggtgaa ttttgagttt gttttttttg ttgttttttg tttgtgtgtt ttgaatgatt     7620
   aatgattggg ttgaaaagtg aagattaaga aaggattgtt gtggggtggg gggaggaaat     7680
   gtagtttttg gttaggttta aatgggtgta gtggagattt agtttttttt ttgtttttgt     7740
   tggttaattt tgtgtttgta gggaagggga aagttgagtg tggggttggt atgttgtttt     7800
   tagttttttt gagtgttgaa aaggaggggt gtttttttgg atgtagggga tattttttga     7860
   gggtttttgt aggttttttg taaggattag gtggtttagt tattttttgt tttgtatttg     7920
   tttttggttg tggtgtgtat tgaagtttgt tagtgtttgt ttttgtagtg aaggttttta     7980
   tagtttaggt tttataattt gggtaaaggg ggagtatttg tgtgggtatg gttttatagt     8040
   tttgtttagt attagtttta aatttttgtg gggaggagtt ttttaatgag tttggggtta     8100
   tagttatgta ttttttttaa tttggatttt ttttttttgg aaaaggaatt tttaggaagg     8160
   ttgaatataa taagatttat ttggaattta gtttttgaat aatagttgaa aaagtttgtt     8220
   gaattgtaaa ataatgatat ggtgtatgtt tttttggagt gaatttttta tgagttggtt     8280
   gtttaagaat ggagaaaatt gtgtgaattg tgtggggtta tataaattga atgaaaggtg     8340
   gggttttagg atgggtttta gttgttggtt tgtgttatgt aaaggaaagg tttggtttgt     8400
   ggaaagagta aggtatttat ttgtggttat tatttttaaa ttttaaagtg gttttgattt     8460
   gggaatgtga aagagatttt taaatgtagt tttaagtagt                          8500
<210> 74
   <211> 263
   <212> DNA
   <213> Artificial Sequence
<220>
   <223> chemically treated genomic DNA (Homo sapiens)
<400> 74
   tatagtattt attatttatg gaggaagtgg aaagtgtttt ttttgtttga ggggataaaa       60
   aagttaaaat gaggtgagag gtgaagttta tttttgtata ttggttggtg tgtttatttt      120
   tttttgtgtt gtttttggat ggtgttgatt gttggagttt aagtgatgtg gagtttgttg      180
   tatttgttat aaatgtagta aggtaggttt aagtatttat aagttttttg tagttgttat      240
   tgtttttttg ggttggtttg tta                                             263
<210> 75
   <211> 263
   <212> DNA
   <213> Artificial Sequence
<220>
   <223> chemically treated genomic DNA (Homo sapiens)
<400> 75
   taataaatta atttaaaaga gtggtaataa ttataaaaaa tttgtaagtg tttggatttg       60
   ttttattata tttataataa atgtgatgag ttttgtgtta tttgggtttt ggtggttggt      120
   gttgtttgga agtaatgtgg aggaaagtga gtgtgttggt tggtatataa aagtgggttt      180
   tgttttttgt tttgttttga tttttttgtt tttttgagta aagaaaatgt tttttgtttt      240
   ttttgtaggt gataaatatt gta                                              263
<210> 76
   <211> 65
   <212> DNA
   <213> Artificial Sequence
<220>
   <223> PRDM14_Amplifikat
<400> 76
   cgacgttttc gcgtggtttc gcgttgttcg cggttagttt cgtttcgttt cgtaaggagc 60
   gtgtt 65
<210> 77
   <211> 16
   <212> DNA
   <213> Artificial Sequence
<220>
   <223> PRDM14_Primer_forward_
<400> 77
   cgacgttttc gcgtgg 16
<210> 78
   <211> 20
   <212> DNA
   <213> Artificial Sequence
<220>
   <223> PRDM14_Primer_revers
<400> 78
   aacacgctcc ttacgaaacg 20
<210> 79
   <211> 24
   <212> DNA
   <213> Artificial Sequence
<220>
   <223> PRDM14_Probe
<400> 79
   cgcgttgttc gcggttagtt tcgt 24
<210> 80
   <211> 86
   <212> DNA
   <213> Artificial Sequence
<220>
   <223> DMRT2_Amplifikat
<400> 80
   ttttcgatcg tcgaattcgt tggagaagcg ggcgcgatag ggcgatgagt taacgcggag 60
   ggagcgcgga ggtcgcggaa gtcggg 86
<210> 81
   <211> 20
   <212> DNA
   <213> Artificial Sequence
<220>
   <223> DMRT2_Primer_forward
<400> 81
   ttttcgatcg tcgaattcgt 20
<210> 82
   <211> 14
   <212> DNA
   <213> Artificial Sequence
<220>
   <223> DMRT2_Primer_revers
<400> 82
   cccgacttcc gcga 14
<210> 83
   <211> 24
   <212> DNA
   <213> Artificial Sequence
<220>
   <223> DMRT2_Probe
<400> 83
   gcgcgatagg gcgatgagtt aacg 24
<210> 84
   <211> 126
   <212> DNA
   <213> Artificial Sequence
<220>
   <223> AC051635.7_Amplifikat
<400> 84
   cctcgtatcc aacgaacgaa actctaacga actccgcgcg aaaatccgaa ctctaacccg 60
   aacgaataac gctcaaaccc cgacctaaaa aaaataacga cctaaattac gacgaaacgt 120
   cccctc 126
<210> 85
   <211> 20
   <212> DNA
   <213> Artificial Sequence
<220>
   <223> AC051635.7_Primer_forward
<400> 85
   cctcgtatcc aacgaacgaa 20
<210> 86
   <211> 18
   <212> DNA
   <213> Artificial Sequence
<220>
   <223> AC051635.7_Primer_revers
<400> 86
   gaggggacgt ttcgtcgt 18
<210> 87
   <211> 28
   <212> DNA
   <213> Artificial Sequence
<220>
   <223> AC051635.7_Probe
<400> 87
   tccgaactct aacccgaacg aataacgc 28
<210> 88
   <211> 118
   <212> DNA
   <213> Artificial Sequence
<220>
   <223> CYP1B1_Amplifikat
<400> 88
   ttttttaaat ttagtcgcgc gttattatcg tgcgagtttt cggtcgtttt ttttttcgta 60
   gagaggataa aggcgtttat tatgtcgcgg ggggcggttt cgggtcgaag gttttcgt 118
<210> 89
   <211> 22
   <212> DNA
   <213> Artificial Sequence
<220>
   <223> CYP1B1_Primer_forward_
<400> 89
   ttttttaaat ttagtcgcgc gt 22
<210> 90
   <211> 20
   <212> DNA
   <213> Artificial Sequence
<220>
   <223> CYCP1B1_Primer_revers
<400> 90
   acgaaaacct tcgacccgaa 20
<210> 91
   <211> 26
   <212> DNA
   <213> Artificial Sequence
<220>
   <223> CYP1B1_Probe
<400> 91
   tcgtgcgagt tttcggtcgt tttttt 26
<210> 92
   <211> 84
   <212> DNA
   <213> Artificial Sequence
<220>
   <223> SOX1_Amplifikat
<400> 92
   tcggtcggcg cgtttatttt ttttcgcgtt gttttcggac ggcgtcgatc gtcggagttt 60
   aagtgacgcg gagttcgtcg tatt 84
<210> 93
   <211> 15
   <212> DNA
   <213> Artificial Sequence
<220>
   <223> SOX1_Primer_forward_
<400> 93
   tcggtcggcg cgttt 15
<210> 94
   <211> 18
   <212> DNA
   <213> Artificial Sequence
<220>
   <223> SOX1_Primer_revers
<400> 94
   aatacgacga actccgcg 18
<210> 95
   <211> 21
   <212> DNA
   <213> Artificial Sequence
<220>
   <223> SOX1_Probe
<400> 95
   cggcgtcgat cgtcggagtt t 21
<210> 96
   <211> 29262
   <212> DNA
   <213> Homo Sapiens
<400> 96
<210> 97
   <211> 20043
   <212> DNA
   <213> Homo Sapiens
<400> 97
<210> 98
   <211> 7201
   <212> DNA
   <213> Homo Sapiens
<400> 98
   gccagtggcg ctgcgctgaa tgcaactgcg cgcgcccgcc gggtgagcca gcgatctgag      60
   ccaggcgggg gcggcccgac ctttcccgcc cagcctgggt cagaggaagc cgaaagcggc     120
   ctcagcaagg tgaggcgccc gaggctgcag aagaccgagc agaactttgc agagttcggg     180
   atgtgctggg ctaggtcgct gaggacttga ctggcttcct ccctccttcc ctccacctac     240
   cttctccctc cccctccacc atccagtggg ttcgttccgg gcctccagct atcagctccc     300
   gatctcgtgt ttgagggtag cgaccccgat ttctgtttgc atgcacagcc agcagcccta     360
   gcgccagatc ctaaagctgg atatctttta actgggctac cagcccgggc ctcaaagttt     420
   tggtgagtgc tgtgatgccg ctcggggtgt cagggagggc aaagggcgtg aatcatcttt     480
   caccttttct gtgtgacttg gatgccttac cttaaatata taaaccaagg cctaagtaca     540
   gagagcccac gcggctcagc cctgtgcacc tcaggtacta ttaaggctgg acgggcgtct     600
   gtattgtgct ctttgccctt atgggacgct aatgacaaca aaattttggt tttcagttga     660
   gtattttggg gaggggggtg agaggacctc ttcaagccca aggaggggtc cttgtggtgg     720
   tgtaggctga tgagaggcat ttgggaagca tagagtggta cttcagtgag tctgaagggc     780
   cacttgtaat caaaggaaaa ctgttcttcc acggatagtt ttatatgcat catggccata     840
   ctagttgagt atggggaaat ccgacaagtg gcctggggct gcctatttgg acacaatatt     900
   ttgtgtgtgt gtgataagtg agttacccag ggacttgtcc ggaaagcccg tgtaaagcca     960
   gagagggcca cggttaaagg tcaggtactc acagagcact tagaattaat aaaataaaac    1020
   ctttgttggg ttgtggatcc ctgggggccc tggagataca ggaaaggcac gtgtggcctg    1080
   gaagtgaggg acatgtaatg agaacaggat gactcaagcg gccggaggcg ggcagaccag    1140
   gagctttggg ccggaggctc agggatggtc cctgacggcg gccggtgggt ctttggattt    1200
   ctttgtgttt ccccagagtg agggccgcca ggctcaggcc ccagcgaagc cccgagcgcc    1260
   atggccgacc cgcaggctgg ctccgcggcc ggggactggg agatcgatgt cgagagcctg    1320
   gagctggaag aggacgtctg cggggcgccg cggtccacgc cccccgggcc cagcccgccg    1380
   ccggcggacg gggactgcga ggacgacgaa gatgacgacg gggtggacga agacgcggaa    1440
   gaagagggcg acggcgagga ggcaggcgcg tcccccggga tgcccggcca gccggagcag    1500
   cgggggggac cgcagccgag gccgccgctc gcgcctcagg cctcacccgc cggcaccggt    1560
   ccccgagagc gctgcactcc cgcgggcggc ggcgcggagc cgcgcaagct gagccgcacg    1620
   cccaagtgcg cgcgctgccg caaccacggc gtggtgtcct gcctgaaggg ccacaagcgc    1680
   ttctgtcgct ggcgcgactg ccagtgcgcc aactgcctgc tggtggtgga gcggcagcgc 1740
   gtcatggccg cccaggtggc gctccggagg cagcaggcca ccgaggtgcg tacccgcccg 1800
   gcccgggcgt ctcaggccac agtggaggtg ggggagttgg aggggagcgg ggcggccgtc 1860
   cacacccccc gcgcccaggg ccttgcggtg cctggcactc cctaggaagg atggtaagaa 1920
   cgcttttctg cttgcactgg cgagcagggt cgggagggaa aaagcaggca tctctgggga 1980
   acttgggaaa tgtttttgtc ttaaaaagca ggcagagagg ccttgaaagt gtttaggccc 2040
   atttggaaca tcgcgagaaa atcaatgttt ttattttttt gcattttaaa gtgcattttc 2100
   gtggtgttga gggcgggggc ttggggggag agtggttgat ccacagcacg gcgtgcagct 2160
   ccaggctcgt cactgctgta ctggtgatga tggagaaaaa gaaccacgca gcacgcatgc 2220
   gctcggtgct tcagctgtca cctggacgtt tgagatatct agaacactca ttttatggat 2280
   gagacacaaa atgcatgaga aattagatgc ctgcagccca aggtcatcaa tccgaagcta 2340
   ctgtccctgc aaactaccta agagcaaggg gagggtctct gctgcttccc atcggaggac 2400
   tccagctgcg aggagagcca gggctttctt tccgagagac gctcttgaac cttcgatttt 2460
   gtatttgttt ccatgtgcgc ctggacaggg agagttcagc gagcgaggct tcacagggcc 2520
   tcctcccaga ctcaggacca gggaagactt tcaattttac tttttggcgg agaggagctg 2580
   gggagtggat cgccagacac actgaaggca gtaaccccac tggaaagcct ctcgagggcc 2640
   caggagagcg cgctggacgc catgggcacc tggtcctggg cttggtctcc tcccgcgtcc 2700
   cgcatgggtc atcgcctgcc ctgcgttccc acgcgttctc tgtagcatat gctgggtagg 2760
   acgcgggcgt gggagggccc agaatcccac cagctggtgg gataggtgac tgcagaggcg 2820
   agggtgggtg tccgcaaagc gcaaatttgg agtttgcagc cggacccagg agaccctcat 2880
   ctcttcattc ccctatcacc ccaccctgcc tgccctgcca cagggagaaa tccgcccggc 2940
   ctcactgctt cacagcaggc cttgggcacc ttctatccga caggtgggct cccaggaccc 3000
   agcctggctg caaccatctc ttgaggtcgg gtgcccccct gaaagggatt tcactttttc 3060
   acttccctcc gggatgtccc ctcacttctg gccataggtc tgcgtatatc tagagtgatt 3120
   agagtctaga gtgactatgc tgcggaaagc aggggatcca gctcaccagc tgaggttcca 3180
   gctccttgcc ctaggattaa ggggtgctga tattaattcg tttgtaatta tttttcacac 3240
   ggaatgggaa aattcaatac atttagaagg gggagagttt ctagaagatt tacggacatc 3300
   ccgtccttcc cccttctcgc ccccattttc tttcagggca ttattgatga tgtttcttgt 3360
   gtttacagga caagaagggg ctttccggga aacagaataa tttcgagcgc aaagctgtgt 3420
   accagaggca agtcagagcc cccagtttgc tggccaaaag cattttagaa ggtaaagcaa 3480
   caaaattatc cttcagcctt ttaaacagag ttgagtgact ctcattaatc agcacaaagt 3540
   gtgtttatta atctgtgaaa gagctatcta aagggaaagt actttttatt agaaaggttt 3600
   tgtttaggtg ttcgctgagc ccgtacgcta aaactgaacc cgtacggaga agattagcat 3660
   ggcccgggaa ccagaaagtt ttcttctagg aaggaaaccg ccacctggga aaatgaatct 3720
   cacatacctc ctgaggtttc tgtcaccacc agttgaagaa tccctattat ttggttgtct 3780
   gcttagtggt taatcggtta atgtggagtg catgtgaaat ttaccccgta tgagggatgt 3840
   acaaagatat actgccaagt gatcctggag cagatgtctt ggaaggaaat aaataactta 3900
   aacaaagcaa aacaaaacaa cacaaccacg tccacttttc ttagttgtgg gagctgaata 3960
   gatgaagtca agtgaggagt ttaacagctg ctcgcaacca gtaaagccgt ttagtctgga 4020
   agttcaacct cagattccgc tctttttcat tgtacttttt tttttttttt tggattactt 4080
   ggataattcc tattattcca aacttcattt ttaccatttt ttgacagtca aggttataga 4140
   atctgaaaaa ggtttcgaat ccagcaagcg aagtggaggt tgaatgcgaa cagtgcgaaa 4200
   tctctagctt tctgtctcca tgttctcact ttcgtgaaag cctgggggta cattaatttt 4260
   tcttaaaata ttactcattt ttaaaagcat ctctatttgg gtatggaatt tatttattga 4320
   cctcaataat atattgacta tttttaaccg tttctcttta aatttttaaa aatttaaata 4380
   agggacaatt aatgtttgct ttaatcactg ccacttcatg aaattcaggt tgaaagtaaa 4440
   caaaaatttt taagtagaca tttggattct gtttttttct tgtgtagctt ctgaattttg 4500
   ttggttctgg acaggtttgt gtgtgtccta taaaaatttt taagtagaca tttggattct 4560
   gtttttttct tgtgtagctt ctgaattttg ttggttctgg acaggttttg aagtttgtgt 4620
   gtgtcctatc atgtgttgta tacttctgac catagagtgg tttcttatga attaaaaatg 4680
   aatttggtta caaactggat aaacagattt ataataatag ggccacattg ataaactgag 4740
   attatgcaaa gcaacttgtg aagtctgagg actaaatgga atagtaaatt caactaaata 4800
   acttcctggc aaaaacttat catagaaatg taaacattaa ttttcaaatt ttattttatt 4860
   tgcaaatgag tataatgggt ttgtcactat gtaaacacaa atataacaat ggatgcttca 4920
   aacaaatatg acagaggtga gtgcttgggg gtgatgaaat actccaagta ttttatgcct 4980
   ccaggaattc tatttatgtt acactaatct ttgtatttga gtttgctcct attcgtgcga 5040
   tgacttagcc tgtgttttaa tgattgcttg ttcaaccatt ttggtatccg gccaaaatga 5100
   ttagagcttg ggaagaatgt ttcagaacag ttattttcga tagagcaggg aatactcccc 5160
   gccttttttg agttttgttt tattttattt tatttttttt tgtagtggta tcaggggaag 5220
   gggcctggtt aaattgttca ctgtagagtg tgtttgacta gttaacagct cttttcaatt 5280
   ttctatgctt ctctattttc ttaagccttt tttttctttt tctactcgct aatctccttt 5340
   aactttcttt ctttctcttt tttcctagtt ctccttggat tattctacag ctattatgtg 5400
   tacataatga accatcttta gaaaataaaa gtgacatcag ctctaggtat agatatattt 5460
   tttaataatg aaaaaagatg acatggataa taataaaaca ttgattgata caagataaat 5520
   agtcttgtct cttaatgcgt agggggcctg ggaagaatat taaaatttta atcaataaag 5580
   tatgtctgtt gaagcaagca agcaaacaaa tccataacaa ccacaacaag caacaagaac     5640
   aagaacaaca acaagaagaa gttgcagtat ggatatcttt caccctccca ggaaactctt     5700
   tcaaacctaa tctgtacttt tgcatctgta taaaaatatc agtgagatgg aactgtaaat     5760
   aattgttctg ctgatctgta gtgttgctgt taccttctgg gtttccacat ttttattccg     5820
   tagatgttaa tctctttcat gtgcaatctt tgcttcttgt aggctatcgc cccattccag     5880
   cggagactta tgtaggaggg accttccctc tacctccccc agttagtgac aggatgagga     5940
   aaagaagagc ctttgctgat aaagagttgg agaacattat gctggagaga gaatataaag     6000
   aaagggagat gttggaaact tctcaagctg ctgctctgtt tctgcccaac cgcatggtgc     6060
   ctggacctga ctacaattcc tacaaaagtg cctacagccc cagcccagtg gaaccaccaa     6120
   gcaaggactt ctgtaatttt ttgcccacct gccttgattt aaccatgcag tattcagggt     6180
   ctgggaatat ggaactaatt tcttctaatg tcagcgtggc cacaacttat agacagtatc     6240
   ccttgtcctc aagattttta gtttggccca agtgtggccc cattagcgac accctcctct     6300
   accagcaatg cctgctaaat gccaccacct cagttcaagc cctgaagcct ggggccagct     6360
   gggacttgaa gggagcacga gtccaggatg gactcagtgc agagcaggac atgatgccat     6420
   cgaaattgga aggttccctg gtgctgcctc acactcctga gatccagacc acgagaagtg     6480
   accttcaggg tcatcaggct gtcccagaga ggtccgcgtt ctccccaccc cgacggaatt     6540
   tctctcccat tgttgacacg gactccctgg cagctcaagg gcatgtctta acgaagatca     6600
   gcaaagaaaa caccaggcac cctctgccac ttagacataa tccattccac tcattattcc     6660
   agcaaacact tactgacaaa tcgggtcctg agttgaaaac accatttgtc aaagaggcct     6720
   ttgaagagac ccctaagaaa cacagagagt gtttagttaa ggacaaccag aagtacacat     6780
   ttacaataga tagatgtgca aaagaccttt ttgtagccaa acaagttgga acaaaactct     6840
   cggtgaatga accactgtca ttttctgttg agtctattct taagaggcct tcatctgcca     6900
   tcactcgtgt ctctcagtga aaggctgctt aaacagaaag ctggattttc tgcagtctta     6960
   gagcattata gccatttgct acttttttta aaagttaaga tgtttgtgta aagaaatttc     7020
   taatgtaaag atgatgattt tgaaaaattt tatatattcc taatatgcat gtactttttc     7080
   ttatcacata tatttaaact tacagatgga aattgcccaa tgtgcaaatt gttaagtacc     7140
   acactttaca cagcatttca aaaagcaata aaattgacac tgtcttctca aagacccaat     7200
   a                                                                     7201
<210> 99
   <211> 42929
   <212> DNA
   <213> Homo Sapiens
<400> 99
   cattaatagg agctgtagtc tggaagataa agagcctttg gtaccgcaag ccctgtcatc 60
   tgtgagtgtg gcacagtgaa ggcctggaga gtcatgacct aggccctgtg aagtcaagta 120
   tttttaccaa gtaaaaagat tggcaataaa acatttccac atagtacatg acagaatact 180
   tgtacccatc ttattttatt ggacattaat tgcactaatt gaggcacatg tacctttaaa 240
   aaaatgaaag aagaaaatac tcagagaaag gactggtatg aggctgagga agaaaaatgt 300
   gtactgcatt ctttagatga agcagaaatc gatcagcaac ttaaagtcgc aatctattca 360
   atctttcaac attgcaaatt aattctcatt tggcttgatt gtttgattga aatgtaacct 420
   ccgtgttggt tcttagaaat tcaaaaatat agagattaga aatttggttg atgaaaacct 480
   acgggaaaaa aaagcatgtg gcctcggcct gaaatgtaga atggacaaat aaaatgttta 540
   cagtgcaata ttttaattga ataatccata gtaatagttg aaataaagtg atacattaga 600
   ctatatttgc tacaacataa aacttcataa caagattatg aaagtgattt ttattatttt 660
   ggtaatggtg tcccagtata agtaatgaga tacaattttt ttttaatttg gtatatcaaa 720
   cagtaaaggc tacatataaa tgttgtttcc ccagaatgta ctttgtctac aactatgcac 780
   tgtagctatt atgcacacac taaaagcatt catgataaag tacaaaaata agaaaagatt 840
   aaataagaag gtatccttga gttataaatg tctactaaag tatacctttt aatcacacag 900
   ttgtgtcaca gctaaaaaca cacaaattaa catatactgc tatgcaacta tttgatctaa 960
   tatagatcta ctaggaaaat attaaggctg tagtaacact tcacaggtaa aggctaagtt 1020
   ctgggacatg aaagctttta gaaaattcac aaaacaaacc tgagacatct ttttgttttg 1080
   gaaaaaagca atttgcttat agagtcaaag ctttgacata caaatgaagc atttaataaa 1140
   cattatactt tattgcaata acctggagta aaacttctga ctttaatggt tgtcagcaaa 1200
   tacattctct ctccggtaga aacacaatga gggtttctga ttaaagcagt aatctgatta 1260
   ctccagcctc caaattcagt tattttcatt cgttttttgt tttcttttcc tttctttaac 1320
   taggttataa gcatattaaa taagccaggt aaactccaag caccttactt tcttccatat 1380
   aaacacagct ttccttttgc cgcaagcatc tgatgacgac tgggcctaca tacgtaaaaa 1440
   cagattatag cacatctgga cctggttgac ataatgaggc atccagattg gttcatgatt 1500
   atttttgcag ctacacattt ctcaatctaa aaaaataggc tacagcagcc caaattcact 1560
   gtctcagcct tggtggggca taatattttg gaatggcaag tgccaaaaaa tttaaatgta 1620
   ctgggcaagc ctgctttgtg tagttgactc taatgccttt aaaaatactg gcatgatttt 1680
   ttatattcaa gaagtacagc tatgaaattt taaaataaaa ttacatgaag ttttttaata 1740
   ttccaaacca caaaacagat ctgctatgtt gtagactttt ttaatagcct atttgagatt 1800
   taactctgct acgccaaaca tctttcttct aaatgtccat gctttgaatt ttgtgctcca 1860
   aattaattaa ttcagaatga gtgggaaaat gtttaaccat ctttccttct tttcagtgtt 1920
   tctctctttt ctcttaccat ccccccaccc cacacacaca tacacacaac tatcaaaaac 1980
   tccctttttt aaaattttgg tacaaagatt ccaaaaagct taataaaatg taataagcac 2040
   ttagcactta ggacactgta gaactttctt tggaagttta attgcaatga actttaaaac 2100
   ttgaaacttc aattcacttt tctaaatgtg aatgcataca tttcatttga gctttatgct 2160
   ttcccagcta ccaaagtaat tgccatctta aaaaacacaa ctgacctcat ctccactgtc 2220
   ttttccaaac agcttccaag actatcttct gattctgact ttccaaaaag caggcattaa 2280
   tgttaacttt caaaaaatca tctagaagag aaacttggtc agttgtaatg ggagttctgt 2340
   ccccaactct tgtcacctcg taaaaagaac atccaggtaa ttcatggcat tttccattat 2400
   tcattgggca gacacagctt agatttctct gccttccaca ggagaatgtt cagatttcca 2460
   ctaacttcaa ctggaactca ataatcagaa tggctttaaa atttagttaa aatttaagca 2520
   tgccatgaat ttggaatttt aatatattaa ttaggttatt ttcaaaacaa gattctgtat 2580
   tataaataga atttcaataa agatgttata aaaataagaa cacatcttag gatctaattt 2640
   tgagattcgc caaaatttga gtgaagaaaa gaaaccctgc ttcatttcca tgtcaaaaat 2700
   aggtggttga tttaatcaag ctcattttta gcacacttgg ttgcgttagt tgtacttttc 2760
   aattttcaat attatacaag gcatatatta ttcaaccttt tcctgaacaa gataatacta 2820
   atatactgct tacggacagt taagtaatat atttcacact atttggtgac tttttttttt 2880
   aacctatgag acttactgaa atcagtacat tatggtcaca taatttaaag cttggctggc 2940
   tttttttttt ttctttgctg ataattcaaa ttctgtactt tggcaagcaa aagaggtaca 3000
   acatcacctt ggagttttac aatttaataa tgcatacttt aaaattcatg ataactcatg 3060
   gaaccccact atactcacta attcaactat tgatttcttt ttgagcaact gactttatga 3120
   tttatcctta aaagtaagga agtataccag aaggcaaaag gataaatcca cccataaata 3180
   ctagagtcta agttattttc ctgaaaaggt gaaaagggaa tttctggtct ttatcttttc 3240
   ccttatcacc ttcaatcaca cactttacac tgaaattact tgtctctgtc tcttcaacaa 3300
   tatactctga aactttgttt aatatacttt ttgtctattt ctgcatatgg gagaccaatc 3360
   ttggattccc accaaaaatg gcctggttac caaaatactg cttcattcag tagtttggtg 3420
   taagtgtttg ggtgtatgtg tctatatgtg cattttaact ccaaagaatg ctaccttcag 3480
   aaataaccaa gatgcagtat gtatataata attcattggg ccctttaagt ctttgactca 3540
   aaaaaatctc ccagaagctc ctgcatagcc cactactcat gaagaaccgc tgggtatgga 3600
   gcacacctca cctgatggac agttgattta tgctcacctt aaacgctaat tgagaagcag 3660
   cacaaaagag gaactggaaa aaaactgaat tttactcctc atctccgaag atgtgaatat 3720
   ttctaaaatt tcagcttgcc tcttgcttct tattggcaag tttccttggc ttgtaaattt 3780
   tggacagcac tatcaaggag ctccatggac tctctgagag tgacattgac tttaaatgac 3840
   ttgggtttaa tggttagacc ataactgaaa ttcattttcg caggctcatt tgggttggcc 3900
   ctgaaatcgc actggtgagc caggatggag atgaagagaa aaagctgcat cttagaaagt 3960
   tcttcgccaa tgcaccgcct tttgcccact gaaaaaatca tcactctgct ggtcaggtcc 4020
   ttgttgatga ggccatcctt gtccaagaat cgagctggat caaagttctc cgggttaggc 4080
   cacttcactg ggtcatgatt cacagaccac tggttgacaa aaaccacagt gtccttggga 4140
   atgtggtagc ccaagacaga ggtgttggca gtggtggcat gaggaatagt gacaggcaca 4200
   aagctggaga agcgcatggc ttcataaagg aaggccagga catagggcag gttgggctgg 4260
   tcacccatac aaggcagacg gtccctcccc acgacctgat ccaattctgc ctgcactcga 4320
   gtctgcacat caggatacct gtttggtgtt taatgtggag agagaaaagc aagtgagcaa 4380
   aattcttatt tcatctagaa agcacattat aatttatttt aattccactt tttcttaaat 4440
   aggctatcta gctcagtgac taaattaata tttctgggaa aaaaatctaa aacagcttcc 4500
   taatttctca ctaaattaca agatggactt taagaattat atttcctgta ataggccttg 4560
   catgactaat gctgtctttc ccattataag tcaaaaaact cttaagtttt cccagcctca 4620
   aaaagcacaa tagtagataa ggctgacttt tccattgggt accgcaggga cagtgcctag 4680
   ggctcacaac actttttagg ggcccatgaa aatattttta cgtcttttac aatcttcaga 4740
   cctaaaaaaa ctcactttta gttagaagaa aatattttca tacatcattt taatgcattc 4800
   atctttatat cagggtagtc atgaaattta atttttaatt tttttatagt ataaggaatc 4860
   tacaaaggca aaagcaccca gggcccacga aagttacaat gtgaaagtaa attttttttc 4920
   tccctcattt ccctggacaa ttccaacaga catgctgttc tcattttata caaagataat 4980
   tggtgtcctg atggataaag ctacatactg tattttttaa atttgaaagc aaatacccaa 5040
   tgttcataaa attatggtac aaactgtagt acttattcac tgttgatgat acaaactgat 5100
   acaatctttt taaaaaacaa aatcttaata cgaggcaaga gacatgaagg taacctcttc 5160
   ccgcctcatt ttaaacccag taatccatgc ctagaaatgt attttcagaa aataatttaa 5220
   gagatacaaa aatatgtaac cacaacagga cctaaaattc taaaaattag ccaggcatgg 5280
   tggtacacgc ctgtagcccc agctacctgc cagacagcgg cgggagaact gcttgagccc 5340
   aggagtttgg gaccagcctg ggtaaggtag tgagaccctg tctctaaggt aagtaaatac 5400
   ataaaattaa aataaaaatt ttaaaaatgc aatcaaggcc aatgaccaac attaaagttc 5460
   aataaatgat tatatataac tatacattat catagcaaaa tactatatag ccatcaaaaa 5520
   ttaccaaaat aaatattaaa agattagaaa tgtccactac cttgagtcaa agtgttagaa 5580
   gtcaaaaaac aagtaactga ttgtttgcaa ctccgaaaaa aaatgtatac acgtatgtga 5640
   tgaagtggag ttatttttag aatcgcttct tcaacattat attgcaggag tctgccaact     5700
   tttttcatag aaacatgtat ggtggggaga gccagagagt atctatttta gactttgtgg     5760
   accaaaccct ctttgttgca ctactctgct ctgccttgtg ttgtagagtg aacgcagcca     5820
   tcgacaatgt ataaatgaat gggcatgact gcgtcaataa aacgtatata caaaaacaga     5880
   tcgtgtaatt tgccaagccc tgtcatacgt cttttcacta aaaaggaaaa aaaaagtatc     5940
   agcatattga attgaaagtg acctccaaat tgttttattt ctttttgccg cctaattgga     6000
   gtactcggag aactattgtg gttctaatga aaaacaaagg ccgtttcaaa agttaaaatg     6060
   cctatctgcg gatgcacaaa aacctatgga acctattccg ggatttcctt ttccctgatc     6120
   tctggttgac ttttctttat tcgatttctg cctctccctc acccaccatg ggtgtttcta     6180
   tgattttaat actaaaaagc cattttaagt atacagaaac ttttaatttg tgaaggtagc     6240
   tgtacagtat ctaaggaaat atagattgca gtggatttta ataaggaaag agtcattctc     6300
   tgccttccaa acacctttct gtggcgcctg cagattcagc atctccaact gctcacttgt     6360
   gctccaagaa atgacacaaa accaggattt ctggtctctt cggcaatcaa aatttgcctt     6420
   gcgaaacatt tgcccctagg gatagtgttt ccacagtaaa tactacaggc cgcccagtta     6480
   aatatggatt tcagatcaac aagggctaat cttttagtgt gagtattccc gtgtaaaact     6540
   tacttttctt ttgccaaatc tggcaacccg acaccaggga cctgttgaat ccgtgcttag     6600
   tagagaccaa cgtgggcatc agggggtccg ctaactgagc caagcgcagg ggccagaaac     6660
   gtctgcaggc gcctcctgga tatcccatct gcctttctga atgctactct gctttcgtaa     6720
   aagtgactgc ttcttgtctt ctaccccggc atttctcaaa ctcctagcgc ggaggtttta     6780
   taaacctagc gctgcccgct agttgtggcc gccttctgaa agtttctgtg cgggttacct     6840
   gtcggctggc ccggcctcca tgggcttcct tggcctggaa caaagccctg acgggcggcc     6900
   cgcttttagc gccagggctg ggcggcctcg gatgcacgcc gaggactagc ctaggagagg     6960
   ctgctgtgtt taacatattt atgacattac cttttctttc ggaactattc cagttctgat     7020
   tgtaatgcgc cagggagaga gatatcaggg ctcatctcca agcatttaaa aactcttgtt     7080
   tttccggggg gtggaggggt ctttgcaaat gtgttctcac tcgcaagcag gggaggggac     7140
   gatagagaca agataggaga ccggaggggt cggggcaccg ctgggtgggg cgcgtgcgcg     7200
   tgtcgcagcc ctcactgtga gtccctttac cgacgcgatc ttggttttga ggggtgggga     7260
   cctggagcga aaccccaaac ccggggccct gcttgcaaac tcagcatatt ctgtctctac     7320
   tccgcctttt tcagaggaga aaagacctgg cccacgcctc ccagaggctt tacctggtga     7380
   agaggaggag cagccactgc agcgcggtgg acagggtgtc ctggctggcg ccgaagatgt     7440
   cagtgatagt ggccggtacg ttctccaaat ccagccgcgc gccaccaccg tgcgagtccc     7500
   cggccgcctt cttttccgca gagaggataa aggcgtccat catgtcgcgg ggggcggccc     7560
   cgggccgaag gctttcgcag tgcctcaaga acttgtccag gatgaagttg ctgaagttgc     7620
   ggttgagctg ctcgaattcg cggaaaacgg tgcgcaccgg gttggggaag tactgcagcc     7680
   agggcatcac gtccaccagg ctgcccgcgc ccaccgtgcg cccgaactct tcgttgtggc     7740
   tgagcagctc acggaactcg gggtcgtcgt ggctgtagcg gcagccgaaa cacacggcac     7800
   tcatgacgtt ggccacggcc acgacggtca gcggcctcgg gtcgaggaag gcgccgtccg     7860
   cgctgccgcg caccagcagc gccaccagct cgcgcgcctc gctcagcacg tggccctcga     7920
   ggacttggcg gctgcgcggc tggcgcgtga agaagttgcg catcatgctg tgggctgcgc     7980
   gccgctgcac cttccagtgc tccgagtagt ggccgaaagc catgctgcgg ccgccggaca     8040
   ccacacggaa ggaggcgaag gccggccggt cggcgaaggc cgagccctgc tgcaccaggg     8100
   cctggtggat ggcgcgctcg ccattcagca ccactatggg gcagctgccc aggcggatct     8160
   ggaaaacgtc gccgtagcgc cgcgccaggc gagcgaacga gaggtgagcc gcctggccca     8220
   ccgccgccgc gtttccgatc agtggccacg caaacgggcc cgggggcgcg gaccggagct     8280
   gccgcctccg ttgcctcagc agccgctggc ccacatgcac agtggccagc accgacagga     8340
   gtagcaggag cgtggtctgc tggatggaca gcgggtttag cggccaaggg tcgttcgggc     8400
   tgaggctggt gcccatgctg gggacagaga ggagaaggcg tgacactcag gggtgcagag     8460
   acaggagcgg gcgccccacg cccctacccc agcctctggg gactgagtgc cgttgggtgg     8520
   agaggtcgga gctgactctc tggagaaatg gccgaagacg ccccttccca tccccaaccc     8580
   actctccctt ggagaagaga aggggcgtgt ttccacctcg ctgtaaccca gcgccaaacc     8640
   cttcccctcc ccgcaaggcg cgtaacggtt cctgcaatct ggggacaacg ctgcgggcat     8700
   cgaggcggtg gcgcttgatt tcctttaaag tacctaccac gccacccgct acctgtaata     8760
   atccatctga agaggtcgcc gggcagcgcc tcggcagaca gactgacctg cggggaggtg     8820
   cggtttccag tggcgcggga cagccggctc cgagaaggaa ctgggacctt tgcctagggc     8880
   aagacgtcaa caggaacccg cagcccggcc tggacacctg ctgccctcac tggaagcttt     8940
   aactcccact cgagtctctt ggcgtcgtca gtgccaggag cgcttggatt gggatgggga     9000
   cggagaaggg tgcctcgctc gccgagcccc gctgcccttg gggacctggc aaagtcgagg     9060
   tttcctcaca gcgttgagat tgagactggg ggtcggtgag tggcgtcaat tcccatgccc     9120
   ttgcggctct cacaactgga gtcgcagaag cgctcgctcc cactcccact ccagagtcaa     9180
   agcgcgccat cccgctcctc cgggttttaa ggactgggta gaaggagggc agcgcgacct     9240
   ggcagggcgg ggcctgcggg gggcggggcg cgccgcacac caggccgctt tgacccggac     9300
   tcccgtccag gcgcaccggc cggcctcgga gctctaccag caggctttca tgggagcgga     9360
   ggtgcccacg tttccattgt gcggtaaccg cgcttcatca cagccacctc caatcgaggc     9420
   cgcacggtgt ccccagaacc acgctcggta caacctgccc cggccgctgc gctcgacttg     9480
   ggagcgcgcc ccgccccgag cgcacgacct cccttccctc tccgcgactt gagcttgctt     9540
   cccggaaagc cagtgtagtg gcagcggttt gcctcacagc gcccctggtt ggttgtttaa 9600
   attttatctt aggttctcgc aaacaaattg ccacctcagt ggaggctctt tggcatgcta 9660
   aattttttcc agttcttgcg gcgagatcag gaatccctaa aagtagccgc tcccccgccc 9720
   cggggccccg acagtgcttg ggcacacaaa ggctcctggg gcgcagctgg ccctgagatt 9780
   tcccgcgtag aggccactcc aggtggcgcc gcccgctggt gggcgcgggg ccgcccaggc 9840
   tgcgatggaa gccgttggcg cagggcgggg agccaagctg gggcgactgg gggggcctgg 9900
   gggagctagc ggggcgggac ccgagcgggg cggagagtgg caggaggagg cgaatctccg 9960
   cgctccggcg aactttatcg ggttgaagtt tctgctgtcg cctccccttt gcgtgcggag 10020
   ctgggctttg cgtgcgccgc ttctggaaag tcggctccag tcatatccct gggcgctgcc 10080
   tgcggccgct cctcccgcgc ttctcacggc acctgacacg cggaggcggc ggccgagggt 10140
   ggggtgccgg ccaccaccac ccttggcgtg gggtgccggc cgccaccacc ctcggctgcg 10200
   cacgcacagt cgcgcctgcc aggccagcga ggcggacgct tgggccacca cccgcaggcc 10260
   gagcccgggc gcgtatcgcc acctcgctgc gcaccggcgg ctctgtggtc ttcctgggcg 10320
   cgcttgcggt cgcgaagggg gccctccctt tataagggaa gccggaggcg cttgggcggg 10380
   ggtggcttcc cccgcctggc gcgccctttc ctacatgctg atgcatagga ttttcaagac 10440
   agctaagaga acaaggactt cacgaggcgg ggggaagggc gaactcgagg gaagaggtgg 10500
   gatgtatctg gttaaccagg taggaattgt actggcttca tcaaaggata aatataaaag 10560
   tgataggttc accagggaca accctgcact ttgagctcca ctcgcgctgc gaatctcgtg 10620
   ctattattga cctgtttaca gatgaataaa ctagggttca gagaggttgc gtgacttaac 10680
   ccaggtcgcg cagccatcaa gaagtggagc cagggcttga agccaccaag tcccactcca 10740
   gatccttcac ataagtttac acggaagaaa attgcagata agaggcccaa atgccttctt 10800
   tcctgcctcc cttaaaaaaa aaaatgcacg tggggctagg cgcagtggct cacgcctgta 10860
   atcccagcac tttgggaggc ttaagcgggc ggatcacttg aggtcaggag ttcgagacca 10920
   gcctggccaa catggcgaaa ccccatctac taaaaataca aaagttagcc ggacgtggtg 10980
   gtgcacacct gtaatcccag ctactcgggg atcagggggg aaggcagcag aattggttga 11040
   acgtgggagg cagaggttgc agtgagcaga gattgcacca ctgcactaca gcctgggcag 11100
   cagagtttaa gtgagactct gtctcaaaaa aaaaaaaaaa aaaaaaatca gctggaagaa 11160
   ctttttaatt attgtttttg ttttcataat tgagaattct gagaatagca gagaagaaat 11220
   agtatgctaa ccctttgcaa ttaaattgct tatgagggag gccaggggag tgtgcctcat 11280
   agctgaccct ggaatgactg attacattgt aaagaaacac aaaggagtgt ctgaaaagaa 11340
   tctgacttct tgagagattg aaaccagcaa tcaatttgct gacctgataa agaaatgtct 11400
   tttcgtgatg gcttttttta tttttatttt tatttattta ttttttttga gatggagtct 11460
   cactctgtca cctgggctgc agtgcaatgg cgtgatctcg gctcactgca acctccgcct 11520
   gccaggttct cctggctcag cctcccaagt agctgggatt acaggtgcgc accaccacac 11580
   ccggctaatt tttgtatttt tctagtagag atggggtttc accatgctgg ccaggctggt 11640
   ctcaaactcc tgacctcagg tgatccacca gcctgggcct cccaaagtgc tgggattaca 11700
   ggcatgagcc accgtgcccg gcctgtgatg ccttttattc ggtatctttt gaagagtttt 11760
   ctggaaatga tttcacacac catgatgggg aacttagata gtgatggagt tttagttttt 11820
   gagcagttgc attggtttaa taataagcgt tggtttccca gaggtgagtt ggcctacaca 11880
   gcctttgaaa tgagtttcat agcaaccttc ccatgtccag ctttagcaag atccttgcct 11940
   aataattcaa gactctggtg catgcatcaa tctctcatag actttaattt agtagacggg 12000
   aattatctat gagctaatgc caatcctgag ctgttgacat tcctgaaagc ctttatcaca 12060
   atacctctgc cagaatgaga gcagatggaa gttgccttta agtaaattcc tatttttaaa 12120
   aagtataaaa cgaggttgtt cctaagaccg cctgtccaac tcctcaataa tgccatctag 12180
   tttttagatc aactagatgc atgctttcct gccatcgcag atagatcaga tatatcctcc 12240
   aagctgtagt tccttgggct cagctctttt tctaggacat gactcaaaaa tcagaggaac 12300
   taggttggta agtgctgtca gaaagataag cccttatcgg ttatggcaaa ccaaaatgac 12360
   tggctatggt aacacttttt gcagagaatc ttcaagcctg gagttcttag ggctttatgg 12420
   tgcaaacttc attgaatgcc ccatccggta ccctgataag gacttcggat aaatccagag 12480
   cagcctgttg gcaccccgac ccaactcaag gcagagccct gcctctagtg agaatctacg 12540
   caggctcttt aattatcatc acaacttctc aaccaaagca gggacctaag gcccaggaat 12600
   ctcctttcaa tcaacacaga atatgggcat tctgccctcc caaatctcaa acttgaattg 12660
   tttgttgcaa ataactttca aaaagaacag ctaagctaga gggatagttt aaagtagaga 12720
   gaggtttaaa atatcttatc ttgtattgac tcgaaggaac aacttaagct tgtttaagca 12780
   gaacaggaat gcactggccc atgtgcagag aaggataacc gtgatgcttc acaggataca 12840
   aggacccagg ccgctctacc ggctctctgc ctatgctggt ctttgcttgg gctttctctc 12900
   tttgcatggc aaggaagcag cttaaaatta acaaaacaaa gagaaagaga acagctctct 12960
   tccagggtcc tagaaggact ccaggggccc aggctggcat caggttttta ccctgtgggc 13020
   aagggcttgt tctgtcatca gaaaaaaaga gatggcagaa catgctagga agccaaagag 13080
   ctgacccagt ccactccatg tatacatgtc tgcccactgg tttctttggg ttggaggata 13140
   acctaaggaa acccaaagcc attattgtta ttattatttt aaatcagcag atatatgcat 13200
   tgcccttaga gccagtagcc cagaaagctc tgcccttctg ctgtcaggct ggaactcagt 13260
   tttcttcatg gtcttcctct ctagtgtgac ccctaagtca gtttaatggg tagccactgt 13320
   gcttttccag tcatctccaa acacacatac ccacatgcac tgaggagagt ggaggagaat 13380
   aagccatctc tggtaggctc ttgacatttg ggtgggcagg attcagtcat caggcacatg 13440
   ctggtaggca agaggagcag aaataaggag acacaccaag tggctgaggg tgcgatagag 13500
   ggaggaactg taggataatg cacaccaggg gctggaccat gaaaggaatc ttaggaattg 13560
   ataccaaaac cctgtgcctc acctgaaatg ctccacttcc ttcctcctgg caatacacag 13620
   gcagagaacc acagagctaa tagaaaggta ttttatctaa tctcagaagc catcatttgt 13680
   aggatgcacc cttattttta tgtactaaca taaagaaagg aagctaccaa ttataattgc 13740
   atcatcgact ctaagataca tccagatttc ggagatgtaa aatggaaagt tgagggtatg 13800
   atttggagct gacaaagcat tatacatttc atgcgcgtcc gtgtgaagag accaccaaac 13860
   aggctttgtg tgagcaataa agcttttaat cacctgggtg caggtgggct gagtctgaaa 13920
   agaaagtcag ccaagggaga caggggtggg gcggttttat aggatttggg aaggtaaagg 13980
   aaaaaggggg gttgttctct ggcgggcagg agtgggggtc acaaggtact caatggggga 14040
   gcttttgagc caggatgagc caggagaagg aatttcacaa gaaaatgtca tcagttaagg 14100
   caggaacagg ccattttcac ttcttttgtg gtggaatgtc atcagttaag gcaggaaccg 14160
   gcgatctgga tgtgtacgtg caggtcacag gggatatgat ggctttgctt gggctcagag 14220
   ggctgacatt cctgtcttct tatattaata agaaaaataa aactaaatag tggtaaagtt 14280
   ttgggacagc gaaaattttt gggggtggta tggagagata atgggcgatg tttctcaggg 14340
   ctgcttcagc gggattaggg gcggtgtggg aacctagagt gggagagatt aagctgaagg 14400
   aagattttgt ggtaaggggt gatattgtgg ggttgttaga aggaacattt gtcatttaga 14460
   attattggtg atggcctgga tacagttttg tatgaattga aaaactaaat ggaataagag 14520
   aaggagaaaa acaggtataa aaggtctaag aattgggaca actcaggaca tctgattaga 14580
   gagtgcctaa ggagattcag cacagtcctg ccagcaaaga ttatttattt acttcaagag 14640
   ttaagagtgg cagtttgggg atagcaccag gagatatcag ctgtgatggc ttggagaaac 14700
   agcgtaaacc ggcagtgtaa ataagagcag ggcgtgtatg agtagttgag aacggtgaat 14760
   aggagtatga ctagacagaa gataataggg atgataagtt tttttggggc acagtctaag 14820
   ttggtctggt gtctggaatg agactggggc ctaataaaaa ggagcgtcta tacaggagct 14880
   caaatgggct gtaccttgta gcattctgag gacaggtctg acttctgaga aaggaaagtc 14940
   gtaaaagtat tgtccagtcc tttttaagtt ggtagctgag cttggtgagg tgtgttttta 15000
   aaagaccttt agtccgttct acttttcctg aagacggagg accataaggg atataaagat 15060
   ttcactgaat actaagagcc tgaaaaactg cttggctgat ttgactaata aaggctggtc 15120
   tgttatcaga ctgtatagag gtgggaaggc taaactgagg aattatgtct gacagaaggg 15180
   aagaaatgac tgcggtggcc ttctcagacc ctataggaaa ggcctctacc tatccagtga 15240
   aagtgtctat ttagactaag aggtatttta gtttcctgac ttgggacatg ttgagtaaag 15300
   ctaatttgcc agtcctgggt gggggcaaat cctcaagctt gatgtgtagg gaaggaaggg 15360
   gcctgaataa tccctgagga gtagtagaat agcagatgga acactgagaa gttatttcct 15420
   tgaggataga tttccatgat agaaaggaaa tgagaggttc taagaggcgg gctagtggct 15480
   tgtactatag catagcctgc ctttgctggt gtgtggcgat taggcctggt ggaactgcca 15540
   tcaataaatc aagcgtgatc agggtgagga acaggaaaga aggaaatatg ggaaatgggg 15600
   gtgaatgtca ggtggatcag agagatacag tcatgggggt caggtgtggt atcaggaata 15660
   atgtgggagg ccggattgaa gtccgggcca ggaacaatag taattgtggg acttaacaaa 15720
   gactgagtac agctgaagga gccggggagc agaaagtata tgcgtcaggt gtgaggaaga 15780
   aaatagattt tggaagttat gagaaatgta gagagtgagt tgagcatagt ttgtgatttt 15840
   tagggcctct aaaagtatta gggcggcagc agccgctgca tggagacatg agggctaggc 15900
   taaaacagta aggtcaagtt gtttgcatag gaaggctaca gggtgcggtc ctggctcttg 15960
   tgtaagaatt ctgaccgcac taaccatgcc taggaaggaa aggcgttgtt gttttgtaag 16020
   ggattgaggt ttgggagatt aatcggacac gatcagcagg gaaagcacgt gtgtttttat 16080
   gagaattatg ctgagatagg taacagatga ggatgaaatt tgggcttgac tgaagtaatg 16140
   ggggctgtct gtgaagcctt gcggcagtac agcccaggta atttgctgag cctaatgggt 16200
   gtcagggtca gtccaagtga aagcaaagag aggctgggat gacgggttca aaggaatagt 16260
   aaagaaagca tgtttgagat ccaaaacaga ataatggatt gtggagggag gtattgagga 16320
   taggagagta tatgggtttg gcaccatggg gtggataggc aaaacaattt ggttgataag 16380
   gcatagatcc tgaactaact tgtaaggctt gcttggtttt aggacaggta aaatggggga 16440
   atggtaagga gagtttatag gctttaaaag gccatgctgt agcaggcaag tgataacagg 16500
   ctttaaccct ttcaaagcat gctgtgggat gggatattgg cattaatcgg ggtaaaggtg 16560
   gttaggtttt aatgagatgg taagggatgc atgatcggtc accaaggagg gagtagaggt 16620
   atcttatatt tgtgggttaa ggtgggggaa tataagagga ggacgcaaag gaggctttgg 16680
   actgggaaga agggcagcaa tgagatgtag ctgtaatcca ggaatagtca gggaagcaga 16740
   taatttagtt aaagtggctc gccctaataa gggaactggg caggtgtgga taactaaaat 16800
   gagtgcttaa aagagtattg tctaagttgc caccagagtt ggggagtttt aagaggttta 16860
   gaagcctggc tgtcaatacc cacaacagtt atggaggcaa gggaaacagg cccttgaaaa 16920
   gaaggtaatg tagagtgggt agcctccgta ttgattaaga aggggacgga cttactttcc 16980
   actgtgagag ttacgcgaag ctcggcgtct gtgatggtct agggggcttc caaggcagtc 17040
   gggcagcatc agtcttcagc cgctaagcca agaagatctg ggaagcagtc agagagcctt 17100
   gggccagagt tccaggggct ctgggagtgg ctgccaggtg agttggacag tccgatttcc 17160
   agtggggtcc cacacagatg ggacatggct taggaggaat cctgggctgc aggcattcct 17220
   tggcctggtg gccagatttc tagcacttgt agcaagctcc taggggagga ggttctggag 17280
   gaacgcctgg ccgctgcggt tcaggcgttt ggaagttctt gtgtgctgga gatgtggctg 17340
   gggtttgtct cacagtggag gcaaggaatt gcaacttttt tctattattg tacaccttga 17400
   aggcgaggtt aattaaatcc tgttgtgggg tttgagtgcc agattctaat ttttggagtt 17460
   ttatttaatg tcgggagcag attgggtaat aaaatgtata ttgagaataa gacggccttt 17520
   tgacctttta gggtctaggg ctgtaaagcg tctcagggct gctgccaaag gagtcacgaa 17580
   ctgggctggg tttttatgtt tgatgaaaac gctatctgat ttgggataaa gaaaaaggag 17640
   cattaacctt gactgtgcct ttagctccag ccaccttttt aagagtcaat tgctgggcag 17700
   gtgggggagg gctagtcacg aaacgaaact gtaaggcaga ccaggtgtga ggaggggagg 17760
   tgataaaagg attatagggt ggaggagcgg aggctgagga agaattggga cctagctggg 17820
   cctggcgagg agcagcctgg ggaggtgggg agaggtcaga tgggtccgta gaaaaggaag 17880
   attagaaagc ctcagcgacg cttggggttg ggactgaggg gacaggtggg agggaaagaa 17940
   ggaagatttg ggatgagttg cattgggaac agagactaga gagggaccga tgtgtaaaag 18000
   aatgcctgga tgtcaggcac ctcagaccat ttgcccattt tacaacaaga attatttaga 18060
   tcttgtagga tggaaaaatt gaaagtgccg ttttccggct atttggaact actgttgagt 18120
   ttgtattggg gtcaagcggc attgcagaag aaaataagat gcttagattt taatttaggt 18180
   gagagttgaa gaggttttaa gtttttgagg acacaggcta agggagaaga aggaggaatg 18240
   gagggtggaa agttgcccat agtgaaggaa gcaagcttag agaaaagaga gagtagagac 18300
   atggagggaa ggggtttggg ggttcttacc ttccagaaaa gcgggaaagg ggttggggca 18360
   cggagaagag gttggggcac ggaaataagg gatgggggca cagagattag aggttggggt 18420
   gtggaaataa gggattgggg cacagagata taagaggttg gggcgcggaa ataagagatt 18480
   gggcgcagag ataagaggtc ggggcacgga aataagggat tggggtgcag agatacgagg 18540
   ttggggtact tgcccctcct ctagaaaagc aggacttgcc gctcagggtg aaggagaagg 18600
   ggttggggat ttcttgcccc ccagaaagga ggagaagggg tagagacacg gagagaaggg 18660
   gttggggtac ttgccccttc cccagaaaag caggacttgc cgctaagggt gaaggaccaa 18720
   ggcaggcgtc cctgcatggt ctgacacctc tgaaacctgg gtgaataatc attgcagtcc 18780
   ctgcaatgat taaacaccaa gggaaggctg ccttcccagt ccctgaccgg cgccggagtt 18840
   ttgggtccac ggataaaacg tgtctccttt gtctctacca gaaaatgaaa ggaattgaaa 18900
   ttaagagaag ggagagattg aagagtggaa aggagaaagt ggttgaagga cagtgagaga 18960
   ggttggagaa gagagtaaga agaggccgct tacccgattt aaaattggtg agatgttcct 19020
   tgggctggtc ggtctgagga cctgaggtcg taggtggatc tttctcatgg agcaaagaac 19080
   aggaggacag gggattgccc tcccaaggga ggtcccccga tccgactcat ggcaccaaat 19140
   ttcatgcatg tccgtgtgaa gagaccatca aacaggcttt gtgtgagcaa taaagctttt 19200
   aatcacctgg gtgcaggcgg gctgagtcca aaaagagagt cagccaaggg agacaggtgg 19260
   ggccgtttta tatgatttgg gtaggtaaag gaaaaagggg ggttgttctc tggcaggcag 19320
   gagtgggagt cacaaggtac tcagtggggg agcttttgag ccaggatgag ccaggagaag 19380
   gaatttcaca agacaatgtc atcagttaag gcaggaacag gccattttca cttcttttgt 19440
   ggtggaatgt catcagttaa ggcaggaacc ggcgatctgg atgtgtgcgt gcaggtcaca 19500
   ggggatatga tggcttagct tgggctcagg ggcctaacaa tacaggctga caaggattct 19560
   ttctttgtat taagcacttt ctagatttct tggagtggtt agcaccaagg gtattggcta 19620
   ccaatcctca tttctattaa tattatttca tgtagacaaa tgtagacaat tgatgttttc 19680
   tcaatcctat gtaaagttgg tgagaaggtg atttagaaga ttctgctatt tttattgacc 19740
   taaattaatc taagaaaaga tagtcacttg ctaagtagtt gcagcttaaa acaagtttcc 19800
   tactcctgct ccccttccaa tctcgttgcc catgagctct gtctggtatt agtgaaagtg 19860
   acagaaatac atgtagtgaa agctacatgt attctagttc ctcaccaaaa tgcatattta 19920
   gaggtaaagg ggtgtaatgt ctgccactta ccctcaaatg gctcaaggaa aaaatagagg 19980
   ggtttgggga gaaggagagg gagagagagg agaatgagaa agtaagtgtg gtcaatgtta 20040
   attggagaat ctgggtaaat gggagttctt tgtgattcct tgaaactctt ctgtaattat 20100
   atcaaaatta aaagttaaat ataagaacat aaaaaaagat cttcccaaca ttttgatctt 20160
   acattgcctt cttagcttct cttgagggtt tcttgtaagc aaggacagtg gaaagaaagt 20220
   gggattagtc tgctgttcag ggtcctgcat ctgatgtgtc actcctggag ttcaggacta 20280
   ataaaaagtt tgtgctcaat tgcagtcttt ctcctcagca ctatggatta taacctgtac 20340
   tttctgtgcc gtccttgttc tttgaatggc ttgagtgtgt cttgtctctt ttatcttagg 20400
   ctttcttcaa ttccttttga aagcagaaga aatatgatat aaggcagtgt catgccctta 20460
   gttctactgt gcccctgtgt taaggggcat aatctactca gggttctatg tgcttcataa 20520
   atgcagcaaa tggtgctgtg ctttaatggt ttttcccagc atcattccca gttctttcct 20580
   ttggtttgct tccttctgcc cttttccttc cccttgcttc atccttcttt cttctctctc 20640
   attctgtctc tcattttccc tttctctggg tccactccta tcttcctcac cctcattccc 20700
   tcaagacctc cactctcgtc tcaggtgccc ccttttctgt tctgctgtct cccttcctct 20760
   cttcattctc tctcacacag aagagtaaag agtcagggca gaggggaaaa gagtgaggga 20820
   aataagaagg gggagcagag agatacagca actaagaaaa agaaaaagaa aaaaataccc 20880
   agccctgggg ctaaaagata gtcacggtgg tcagtcactt tgtctgggct ctcaactggt 20940
   gtacttacct ctcctgctca acgtttgagc atgtgcctct gctgcttaat atttgtgggg 21000
   tgaagctccc accgacttgg cacagtgatg attccatacg aagggaaggg acgccagggt 21060
   ctgtggttgt ttttgtacgt ttgttctctg agcttctact accacctact ctacttcctg 21120
   aaatagactt ttctctcctc cttttccttc tccttcttct ttcttttttt ttcagagtct 21180
   tgctctgttg cccaggctgg agtgcagtgg caccatctcg actcactgta gcctccacct 21240
   gccatgttca agcgattcac ctgcctcagc ctccccagta gctgggacta caggggtgca 21300
   ccaccatgcc tggctaattt ttgtattttt agtaaaggca gggtttcacc atgttggcca 21360
   ggctggtctc aaactcctga catcaggtga tccacctgcc tcggcctccc aaagtgctgg 21420
   gattacaacc atgagccact gcgcccagcc tgaaatcaac ttttctaaca gtgaaagagg 21480
   gagcctactg tccacaactt tggtcaatag ggaaacactt tacttcccta attctttgac 21540
   caaccataga aaacctttag gcagataaag gatactagtt ttccttttta ttttcatttt 21600
   ctaaaaataa atatttacca ggttaccaag aaaatacata aagcaacaag taaaacaccc 21660
   caccaccaaa agaatgtaca tttgggctca gctaagatca caacatctag ccatggacat 21720
   gttgactggt acagcctcca ggtttttgga tggttagcat ccagattcta gtctttcttt 21780
   ctctctctgt ctgtctgtct gtctgtctgt ctgtctgtct gtctgtctct ctctctctct 21840
   ctctctctct ctctctctct ctctctccct ctctccctct ctccctctct ccctctctcc 21900
   ctctctccct ctctctctct ctccctctct ctctctccat tcacctctta gtttttagca 21960
   ttagatctgc attccctcac aggttttttt ctctttccac aaatggacca aaattccggt 22020
   tgaaaactct gggtgtatcc agggtttgag aaaataattg atagttaaat aacttaagtg 22080
   gttagtcctc ccctgcatga tcccaacgga gaacacaaag gctggatagt tatacgtcag 22140
   cttttttgac cattgactcc caacaggatt tttcccttct actattgaag tgatcatttc 22200
   tcaactgttg tctgttctat tgttggactg ctttggttag aaagctgtag atgctagaag 22260
   gttcttccca atattaagaa aaattctgga gtgaactttc ttgccttctg gtaaatctag 22320
   agctattctt tggagtgaca taaagcaaga ctcattctcc tttgctgtca catgtttgaa 22380
   gatggaattg tgctgcttct gagccatcct tccaccaatt aaaaatgccc cagttcctta 22440
   tctctgcctt ctggacaatg tgctttttca gtatgtccat ctgagtatgg gcctgaaact 22500
   gaacatagta tcccaaatat gacattctgt ctagcccagg ggggtgtggt tacttcccat 22560
   tagacatagt gttactttca atgcaaacaa agtttttgcc ctgttttaat atatttgcat 22620
   cccactatta aaacatctat attctactaa gattgtgata agcaaaaatc ttcatccttt 22680
   aatataggac ttggtagctg gctgagtctt cagttgtact tgtattattg atttttcaaa 22740
   aaaatactac atgtgagatt ttacatttat ttctattaaa tgcatcttgt ggctttagga 22800
   tggtgttctg gcctacagag gccatgttga tactcccagg agcacagact aggacaggac 22860
   aggaagacta tccagggaaa gcatggccca gtctggacta cttctaggca aaaatctttg 22920
   gactgcggtt tatgttatat agttgcatca ttatacatcc agaatattat gcatattaaa 22980
   ttatttttct attttggcca gatatttatt cttaaaacac acacacgtag taacatagat 23040
   tcttgttgca agagagtcaa tcagtatata aaaatatgca gaaaaatgag aataaaatga 23100
   gaaaactcac actcccaccc cttcccagag taaccatggg taccatgtgc cttctattta 23160
   tgtacatgca tacggttgta catatagata gatagataga tctacttcat gattttaaac 23220
   tactgcatgg tatgaccatg acattattat tgtcattact attattattg agacagggtc 23280
   ttgctctgtc acccaggctg gaatgcagtg gtgtgatctt ggctcactgc aacctccacc 23340
   tcccaggctc aagcaatcct cctgcctcag gcttccaagt agctgggacc acaggctcgt 23400
   ggcagcatgc ctggctaatt tttgagtatt ttttagaggt ggggttttgc tatgttgtcc 23460
   aggcttgtct cgaactcctg gactcaagcc atcttcccac ctcagcctcc aaagtgctgg 23520
   gattataggc gtgagccact gcgcctggcc aacattattt tttcaaccat caacggcgac 23580
   ttagatggtc atttgtgttg ttctcaactc tattattaga gactccactg cagtgatttc 23640
   acttttctgt gcaattctga atccatttgc aaatgttccc ccagtatcga ttctcaatag 23700
   aaatgcttga gccataggga tatatgcata tatgctacat cctcactgtc ttctaaaaag 23760
   gtttgccaat tcccatctct atgaaccttg gctttgtacc aagtagaatg tacctaaata 23820
   cttttccttc tataaaggat ggttactttg acttaggttt gccaacatgg aacaaaacaa 23880
   acaaaacaaa aattttactt gtctttcaga cctatttctt ctcttaaaga atacatatgc 23940
   ctgtgttcag attatctcct cttcctgaca aatgagggaa atccccagat gaaagctgat 24000
   aaaacattag acgggggtga ttccggacct ccacatccct atggaacaca tcacagaagc 24060
   caggattgga gagccaactc cacacacctc catatcccct cttagatgaa tggcaattct 24120
   ttacaggccg tgggcgcagg cccaaatgcc ttctgggctt gtctaatcct ccagaggata 24180
   tcaaaggcaa tacagccagc atacccaatc attaggagtt tggcttctgg gaccgggcac 24240
   tcttgggtgt gagcaccagc tctcttattt ataagctgta accttggcca tgttatttaa 24300
   cccttctgag cctgactttc ctcacttgta caagacaggt aatataccaa ccacagaggg 24360
   tggttttgag gattaaatga gatattgatt gtaagaaaac aaatagcata gttgatggca 24420
   gagtgagcac ccaataacag ttagccatta tcattattac tactacttac taagtcctta 24480
   cttctgtcca gtaaaatcca agacatggca aggacttgaa cctcgcagga ataaaaacat 24540
   ggcccacttg gttcagcgtg acagctctca gggtggcgtg ccgttaccca cctgcaaatt 24600
   cctaacagcc caaagtggca ccagtcactt cctccttgcg tttctcaggc agcacattga 24660
   gtggctgcta gcatcactcc agagccagac tgaatctgaa tctggctctc ctacttacac 24720
   cggagtgact tccatgctag aaatcgcttc tctgggtcca gcttacactt aagtgaatgg 24780
   agatgtaaaa tggagacgat accagtaccg attatgcaga gttgtggtga ggactgaatg 24840
   aactaataca tgtaaagctt ttagaagggt gcctggcatg tggtgggtgc tcaggcatca 24900
   ttagctgtta tcattattct gtgggaggcc atttgccgct gccttaagaa ggctgatctc 24960
   acagaaacat ctgctgttgg cagaagagct cagcaccaat agccaatttg tgtttccctc 25020
   caactcccag atggcagctc tgcagcactt tctcctcatc taatttctcc tcacccagac 25080
   gcgctccaca tcaggaccct taggtagtac agagacagtt ctttaggcag gggatgccca 25140
   tgttcccaaa agagcctgat gaggccatag taggcctctt ccaccaagct atgctttcgg 25200
   tcagtccctc tgtgactgtt acggatgctt tgtagccctg tggcaatgag gctccaggac 25260
   aacactcatg caattccctg ctgaggagag ttcagaaaac tccatgctgt gcagtgcaat 25320
   tgggaggaaa ctcaagggag tgctgtcgtg ggacctcccg gtggtgattt gtacaaatga 25380
   cgcaacgcta tccagcttcc cagaaaacac cgcctttttt tcttacatta cagctctgag 25440
   tcaaagtgta ctgtggcctc agacctgggg catacagccc atttgacagc cacaatgagg 25500
   gaaacagcct acttctggag cctgtaccag aaggcagaac gggagactcc cagctctccc 25560
   ttactaaagg ccagggagga aattgtctcg aatgctctcc agtccaaaat ggaaagttag 25620
   attcaatggt ctgtactctt gggctgacta acctccgttt ttccttttcc cattgtgctg 25680
   agtcagccac ggggggtggg ttgtgcattt taagggtttg aatgcagaag gaaggaaggc 25740
   ttcccagttt ctgagccttg agaaggtggc actgtggcct ccaaccagca gccatgcccc 25800
   caggcacctc attctcctac actatttcag ggactttctg gtgcaataag gatgtgactg 25860
   agccctgagc ccccaggtta ccagccacac cccagtggaa tcccgggagc tacggggtaa 25920
   aatggaatcc tcttccacag caagtgcaca gaaggcactc agggctggtg ggaacctcca 25980
   tttctgccct catttactct ggactctgaa cctaatacct gaggcttcct gcagcaaatc 26040
   agagaggtga agagcaactt acacgtatct tttttttttt tttttttttt tttgagacag 26100
   ggtctctcta tgttgcccag gctggtcttg aactcctggg ttcaagggat cctcctgcct 26160
   cagcctcctg agtaacacat atcactcaac aaggaataaa caccacaaca attttgagta 26220
   ccacagtcgg agcactgtgt cctgctaaat gtgaggagca ttctctctga tgttatcatc 26280
   ataaggccag ggcctgtggc ttaagctcct ccgttccctt agagtctccc acaccacaca 26340
   acaagtttgg agacagagaa agtagctaat atttccccct aagtgccagg cactgttttc 26400
   tattttttac attgacagtt tcatttattt ctcccaatca tcctaaaaat attggtatat 26460
   ttagcctgct ttatatacaa agggattaag gctcagagac agtaaactat ttgccaaatg 26520
   tcacagaggt tgcaagtgag aggggtcagg atttgaattc aggtctgcag gcaccaaagc 26580
   ccatctatac cacaccagta cacatttgtt gactcactgc gtatcatcct aagtcttaca 26640
   gaagccccac ccctctaact acagtaactg tagaggtctc tagtactgtt atgcctatta 26700
   gggacctggg ccaccccact gccttctcct gccctgtgag ctgactccct ccaggagccc 26760
   aagaccacaa caagcaaagc catcttgttg ccagagcctg atgcaactca caccctcttc 26820
   ctcccctccc cttgcagaat atccttggta tctgcccagc aatttaatct ccacaattta 26880
   caatcaaatc ttgggatggc ttagaaaatc ttcctttaac acagtggctg gaacatgcag 26940
   agagttggca tgcagcctcc acatgggctt catgactaga tctgctacac tcaaacccat 27000
   tatattgaaa ttaccataaa attcacacta ttccaatcct cagaaatgat ggttttattt 27060
   agaagtaagt gagcaaaagc aatgatttca taggccccag tgccaaaatc ttagctcaat 27120
   gtttctcaaa tgttttgttt tcagaacctc ttcaaagtct taaaaaatat tgtagagtcc 27180
   aaagaactat tgtttagcta agtatctatc aatatttata attctagaaa ttaaagctga 27240
   aaaacttata aaatatttgt tagttcatta aaaataataa acctaataca tgttaacata 27300
   aataacattt tatggaaaat aactagtttc caaaattaca aatatttagt cagagtgggc 27360
   agttttaaaa aatattgtta caaatctctt taatttatga cttaacagaa gatagcaggt 27420
   gactcacatc tgtttctgca gaaagacccc ccagtaaatt tgtaagagaa tgagagttag 27480
   aaatgcaaat aatgttttat tcccacagct gtaaccttgt gggtttcctg gaaagggtct 27540
   caggggccat cccgacgggg tgcctggacc acactttgag aaccattgtc taaacttaag 27600
   agtgtgtttt ttgggccggg cgcggtggct cacgcctgta atcccagcac tttgggaggc 27660
   cgaggcgggt ggatcacgag gtcaggagat cgagaccatc ttggctaaca cggtgaaacc 27720
   ccgtctctac taaaaaatac aaaaaattag cctggtgttg tggcgggcgc ctgtaatccc 27780
   agctactcag gaggctgagg caggagaatg gcgtgaaccc cggaggcgga gcttgcagtg 27840
   agccgagaga gcgccactgc agtccggcct gggcgaaaga gccagactcc gtctcaaaaa 27900
   aaaaaaaaaa agtgtgtttt ttgtttgtgt gtgtgtgtgt gtgtgtgtgt gtgtgtgtgt 27960
   gtgtgaatgt ctcttcatcc aagaaaaatc taaatgggct attgtcttgg cgtaggaagt 28020
   aggtgagggt gcggtggatt tcagaaaaaa taatgtccca gttactgatt ttaagagaat 28080
   agccctgaga caagtgtggg cgcattgaaa caccactgca accctctaaa ctattgcaga 28140
   gttgccacca ccaggtgtct ggctagaaag atgtcatctt cccaagacag ctacctgccc 28200
   agatgatgta ccttagcccc aggaaggagg aggaagaaga ggcagacaga atgatgccca 28260
   aacccctgcg gcccggagac gaggtagtgc ctgagtcagg aagctgggag agcccagctc 28320
   agtggaagag gcatgtggat cctggttagg tgtgaagcca ggagggcttc ttagccacag 28380
   cctgaaatcg aagcccagga tcgagccaca gtctgaaatt tttggatcgc agctctgcca 28440
   attaaaccaa tggtaacaat aataatgtac tgtcatatat gtaagaactg ccatatatgg 28500
   agcattttga gccaggcaat gtgccaagca cttgacagaa atcatttcat taatcccaca 28560
   cagccaaccc tttgagataa ccattgccct catccctttt ttacagacaa ataaagcaag 28620
   gcaggaagag ttggaattat caatcaaggt cacacacact atgttggttg agctgaaatt 28680
   tgaattcagt tccgccgggc tgcaaaaact ctctgctgct tcccagctgc accagctagg 28740
   caaactcctg ccagaggaaa tctcacagtt ggagtgcagt aggcatagat ttctcctact 28800
   taactggggg gaggctgtag tcacagcgaa gggaatatta ttgtgtccca tgggaagaaa 28860
   catgaagacg gagtggacgc ttcattccta ctccaatcag ccatctggag ggttcagggc 28920
   tgaactgtag tgggggcatc acaaaactta gggaggtgag gagcctccct tagggaggtg 28980
   acagcctgag tacatggccc ccaagctttc agatcccttc ctgcacccct ccccacctag 29040
   ctctgcactt ggcattgttg ctactagcca ggaagtctga gatctgcgca aaccccgccc 29100
   ttccccgccg cccccgtaag cactaccgtc ccttcaaagt tcagacgtgg ggtcctttgg 29160
   agccctgctc cctagagggt cgcctggcac atggccacct ccacatcccg ccccttctga 29220
   gctcagcctc ccagaggtgt gaacttctta caaacacaca ctatcaccca attccctgga 29280
   aaaacaaaca ggacaattgt gtgaaaattc cattctaagg catcctccct tctctgtaac 29340
   cagaggctcc aaggagttca gcatagcacg agcttttaat ttgcgtgcag acaagcacaa 29400
   aaggcacaac cggatatacc tgttatttcc caatgacctg agagcccgaa gtttatgtta 29460
   agccttgggt tatggcacag cttgcacgca aggccctgca gctcctgcag gcaattgaga 29520
   ggtggtggtg tacaggacag aggaacaact ctgaagtgac agcacataat ttaattcccc 29580
   ctaagctttc caagcatgca gactgttcct tttttgtcag cgtataacct aagtgatttg 29640
   ttctactcag gcaagaaatg tctaatttaa accacattgc agctaagccc ttagtgaaga 29700
   aggagaactc cttgaggctt agccgtgcgt ggttaggctg gtggtagctg aggcaaagca 29760
   gacctctttc agcccctgta tttcttactc aggtcatgac tggagatacg tgggttgggg 29820
   ggctctaatc accacactca ctcttctgtc tcacacttca tccctcgtga atgggtttag 29880
   ttttagtaag aatgtttaca ggagagtgtt ggctacatgt ccccaacatc ccgggtttga 29940
   gtcccatctg agccaccagg agttgggtga cttcagggaa acctcttaaa ctctaaccat 30000
   tgttttttct tctctgagaa acaggacaaa tcggtagtgg gggtgcacag attggcacat 30060
   tttcgtggca agcgatctgg cttaggaagc aagagatctg tgctccagcc tagctttgtt 30120
   cccaactagc agtggatttg cagcaaggaa cttttctctg ggcctcaatt tcctcatctg 30180
   taaaataagc aggctggatg aaatggtctt tgaagttcct tcagctataa aatactatgt 30240
   atgtcttaaa tcacttggag tttgacaaaa taatagttat aataatttta attattattg 30300
   tgatttcttc ctacaatccc aaagatttca ctagccatgc aaatctttta aaattcttct 30360
   ttaaaagtac ttttcttggc cttactgaca tgaagcaaag aaggattcta ggcccccaga 30420
   tggtaggatt tggggtagac attctgtgtc tgtctcattc cccatggaat tatcaaattt 30480
   cccccctagc cacgccagac tgctaaatgg tctctaagta tagcctacat ttcaaggctg 30540
   tttcctcagc caaaaatgcc ccttctcctt gtctgctgtg aaactccctt ccttttaccc 30600
   tcctctttcc attatgggac agttttctct tctgggctcc cttgagcact tcacactatt 30660
   attccattga cttcatgctg ttatttttgg ttttgtattc aatatatctt tgctcaacta 30720
   gaccctcagg aactctgtca tctgtaccac ccagcctctc cttcccagcc ttccccagca 30780
   cctggtacac attcatggaa gaattaactt ttgaaccctg tggatttcat cacaatagga 30840
   taagaatgct gcatgggttt aggatagacg ttcacaatct gagatgagca gtgctagccc 30900
   cagtccccaa aggaaaccta gttagggtgg tgattagaga cattttgaac aattacttca 30960
   ggaaaggatc aaattcattg aaaagctgct tatcttagtg aaaatgttgt aactgtggaa 31020
   agacataaaa gacaaaatgt tcataactgt atccttggaa gtgcctttct ttaaagaata 31080
   ggaatcatct caccaaagta tttgaagaag aatgtgtgtg gttgtttgca tgtagctgcc 31140
   tatacttaag aatgttacat atttcatttc tcccaggatc actcagccta agagaagaat 31200
   tcgaggttac ttttagggag gaaaggacac ttacactggg aattcaatcg atggtagtta 31260
   agcgtccttt ttctttaata attgggataa gtatgacaca tacaacttta catagcatgc 31320
   tcggggagtg aggagagaag ctagagaaga ctgagatgag tgaaatgcag gcttgattga 31380
   gaaactaagc agaatttcta ctaacagtga ggattgaggg agaagcgttc ctgtaatgga 31440
   ggggaaatga ataaaacctc gactcccagc tcgacaggga cctttcttct cctagccagc 31500
   tggaatgcaa gctttaaaaa aaaaggaagt gtggggaggg ctattttttt taataagacg 31560
   tctcagaaat gatcatgaac acagtaaagc tggttttcaa aaaaggaaag tgagaaaagt 31620
   agtgacaact tgaagctggt agttgaggaa gaaggagaac ttctgaaccc atgaagagag 31680
   tatacccggt ttgggtgggc aggactccaa agcctttagg atcgatggac cagaacagag 31740
   aaatgaaaat gcaacaagaa cagctgtggg gaaaaggcag aaggggggta ggaagcgaag 31800
   agggtgtgta cttttccttt ctgatctgag aaagggaaat tttgtgctgg gaagagttac 31860
   gcgggaggag gaaggaaagg cgccaggaat gcctggaagg tatatgatgt ctgaccttat 31920
   gatctcttgc aacaatttct acaccgattg tgtcccttca cctctgctct aaaaatgtgg 31980
   ttaggtaaat atctgtttta ttaacattgt gagaaattag cctcgtttac agaattccac 32040
   aaaacaactt gcaataatga gaggctgcta ggatgtaggt gatactagga gaagcaatgc 32100
   cttgtctgaa cccccagggc tctggaggca ccgctttggg tttaaaatct ttccttagtg 32160
   accttgaacg taatgaaata ggagagaacg ttgatcttag agtagtaata gcacaaacca 32220
   cagccttaat gcccagcagg cttagttcgc tggaataatt agcctaggaa atgcatgact 32280
   taagatgtcc ccacagtggc ccccacaaag tctgaagggg aaacaaagag aaagccagag 32340
   agcctttcat agaagggaac caactgcgtt tacatgcaaa agatccatct cctgtggcta 32400
   cctaagagca gtgactctct gggggtggaa taatttatac atgtgtaaaa acatttattt 32460
   ttctacatat ttacttcaag catgtgttaa aatatctgca aacaactcca gaccccctcc 32520
   ccatcttatt tttatcctaa aggcagtgtt acatggatat ccaaatgttt agagaggaca 32580
   atcttaaata tacaaaagaa tgataaataa ataaataaat aaataaataa ataaaaaaaa 32640
   gatttcctaa tttgtcacca cttgcatgct ggaaaccgac agcgctaact tgggtaatat 32700
   ccttttaact ctgattgaat tatatgttaa ttccttgcgg tggtcttaga tttcagacgc 32760
   tattagcaaa ttatggagga aaagcaagat catatgattc atgaactatc acagcagcaa 32820
   cagtttctaa tttccaccca tcctctgtgt catgtgatat gtttcagaaa ctcacttaaa 32880
   attgagaaaa taagccattc ctaattgtac ttaagtaagg aaatagctca tttatgaatc 32940
   taagaagatt aaagaatatg gctttagtgt gtttgtttag aatctatgcc aaaatttttt 33000
   tccgaagcat aaatctggtt tttttctttt tccttaaaaa aaactttaat aaagccaaag 33060
   aaagaaagaa aggaaagaaa gaaagaaaga gggctccaca tgcagtttca gcactaaggg 33120
   atcctgacaa gtaattcacc tctgctttga atgtgggatt tttgcactct atgatatata 33180
   cctttggagt ggttttgttt tctttgtggt tttttttttg tttgtttttt gttttttgtt 33240
   ttttttgtgt gagacagtgg atttttttat ccatcattgc attattggag ttttatgtga 33300
   tttaatgtat aaaaagtgag gctattctcc aactagttgt ttgggtttct gtaaagttca 33360
   atttccacag tttacctcaa gaggtctttt aaaaatgaaa tttcaagcat gttgcttgat 33420
   attctttaac atgttctcaa ttaatttgca ataaagtcca gcccccttcc tattgcacac 33480
   tattgtaccc ttcctgcctt tttcatgcac tgctctccac ccaccctggc ttcccggctt 33540
   tgcctccaac gtcagagtga tttctgggct tgcttttttt tttttttttt tgcctggaaa 33600
   cttcttctct gaagaacttt caaatggctg ccttcttcat gtatctcctc tcggagcatc 33660
   attccctgac cttcctttct agggctctga atccacccca ttgctgtcta tccagtcagt 33720
   ctctcttttc tagctggcat ccatcacaag caatctgctt atcttgtttt tggtgagttt 33780
   atatctgttt ctaccactgg attataaata cttccaggat agagatctag tttatatcac 33840
   tcaccattct cttcccttag tgcctggtat gtagtaggtg ttcaataaat gtgtactgaa 33900
   agaatggatg agtgatatga tattgatctg tacctatttt ttctttttag tctatctttg 33960
   ttgagttttg gtattgattt tatgtgtcag tacctgagaa ggagagaagt ccttgagttg 34020
   tttcgtaaat gtggctcaag tgagatgctg aacagtgcca gccatgtgga ctctgccaca 34080
   gtgaaggagg aaggtagaga aaatgggttc cagtgtgtgt gtatgtgggg gtgggagggg 34140
   tgtgttgatt taggggtgta cagaaaatgg tggtgtgctt catgactttt ttttttaaag 34200
   aatgaaacca ggacatcaac tgagcatata agaggggaaa gtggtgcagt agggttaagg 34260
   cataatgaaa agatttagaa ttatcctaaa agagtaggaa agcaaaccac gtagagatgt 34320
   ataatagaat tccagtgttg agtttccatt ttatcatcat gaatttgaag tgagacagct 34380
   ggaatttggg atttccctaa cacttgagct gggaaggtaa attgttaatc acttttatgg 34440
   agccagtgtc ctttgaccaa gcaatcctag tctaggaatt tatcctaaag acaattggac 34500
   aagtttgaga aaatgcatat acaaagtcgt ttaacaaaat attttattat aattgacaac 34560
   aaataaatgg aaagtaccta aatattcagc aatatgggat tggataaatt ctgaaacata 34620
   caatgggata ttttgcagct attaaagact gtctgtctat attcattgat atgatgaata 34680
   ggtatgatga gttattggtg aaaaaaatgc agttttcaaa ctagcacata gtgtaaaacc 34740
   cttttttaaa agtcgtttac actcatgtat tttagcataa agaaagacat ggaaggatgt 34800
   tcaccaaaat gttaaatctt ctcaccctct tctatgtatc tttttgaatt gggtgaaatg 34860
   ttctataatg agtatttgaa aacagaataa acagaaaaaa aaagatttaa ttagatccca 34920
   tttgtcagtt tttgcttttg ttattgttgt ttttggtgtc tttgtcatga aatctttgct 34980
   agttcctatg tacagaatgg tattgcctag gttgtcttcc agggttttta taattttggg 35040
   ttttacattt aagtttttaa ttcatcttga gttgattttt gtatatggtg tatatgtagt 35100
   cttctacata tggctagcga gttatcccag caccatttat tgaataagga gtcctttccc 35160
   cattgctggt ttttgtcagt tttgtcgaag atcagatggt tataggcgtg caggcttatt 35220
   tctgggctct ttatcctgtt tcattggtct gtgtgtctct ttgtaccagt accaggtggc 35280
   tgtggttact gtagccctgt agtatagttt gacgtgatgc ttccagcttt gttctttttg 35340
   catacaattg ccttggctat gtaggctctt ttttgattcc atataaattt taaaatagtt 35400
   tttctttcca gttctgtgaa gaatatcatt gatagtttga taggaatagc actgactctg 35460
   taaattgctt tggacagtat ggccatttta atgatattga ttcttcctat ccatgagcat 35520
   ggaatgtttt tccatttgtt tgtgtcatct ctgattcctt tgagcagtgt tttgtaattc 35580
   tcattgtaga gatctttcac ctccctggtt agctgtattc ctaggtgttt tattcttttg 35640
   tggcaattgt gaatgaaatt gcattcctga tttggctctc agcttggctg ttgctgatgt 35700
   atagaaatgc cagtgatttt tgtacattga ttttatatca tgaaactttg ctgaagttgt 35760
   ttatcagctg ggatctaatg aaacttaaga gcttctgcac agcaaaagaa actaccaaca 35820
   gagtaaacaa caatgatcta cagtatggga gaaaatattt gcaaactata catctgataa 35880
   agatctaata ttcagcatct ataaggaatt taaatttaca agaaaaaagc aaccccatta 35940
   aaaagtgggc aaaggacatg aacagacact tttcaaaaga agacatacat gtggccaaca 36000
   agtgtataaa aaaagctcaa tatcactgat cattagagaa atgaaaatca aaaccacaac 36060
   gagatactat ctcacaccag tcaaaatagc tactattaaa aagtcaaaaa ataacagatg 36120
   ctggtgtggt tgtggagaaa agggaattct tatacactgt tagtgggagt gtaaattagt 36180
   tcaaccattg tggaaagcag tatggcagtt ccttaaagag ctaaaaacaa aactaccatt 36240
   cgacccagca atctcattac tgggtatata cccaaaggaa tataaattct tctactataa 36300
   agacacatgc acacatatgt ttattgcagc actattcata atagcaaaga catggaatca 36360
   acctaaatgc ccatcagtgg cagatcagat aaagaaaatg tgatacatat ataccatgga 36420
   atactatgct gccttaaaaa aaaccagatc atgtgctttg cagaaatatg aatggagctg 36480
   ggggccacta tccttagtaa attcacgcag aaacagaaaa ccaaatacct catgttctca 36540
   cttataagga ggagctaaat gatgagaaca cacggacaca aagaggggag caacagacat 36600
   tgggacctat ttgagggtgg agtgtggcag gaaggcgagg atcagaagaa ataacttttg 36660
   ggtactaggc ttagtacttg ggtgatgaaa taatctgtac agcaaacccc catgacacaa 36720
   gtttacctag gtaacaaact tatacatata ctcctgaacc gaaaataaaa gttaaaagaa 36780
   aaaagaaaga cccatctgta tagtgctctc tctctctttc tcttacttgg tttattctct 36840
   tccttatcca ttgcatgcat taacccttta gaacttccgg tgttctgggg accctgaaag 36900
   atcagctagc aggattattg cggggctcag tgcagtgatg cagaggcctc caccagcaat 36960
   cctgaagttg aaagagaata tccacctccc ttatgcataa gggatagatt tgggggtggc 37020
   attgaaggtc tttctctcat tttgctggca gctgtagcaa tgcattgatt agcaggagag 37080
   gttttctctc taatgagcca gaaactaaca cttgtggagt tcctgctata caccagcact 37140
   gtgcagggtg ctttacattt aaaaaataaa tctgggccgg gcgcagtggc tcaagcctgt 37200
   aatcccagca ctttcggagg ccgaggcggg aggatcacaa ggtcaggaga tcgagaccat 37260
   cctgtttaac atggtgaaac cccatctcta ctaaaaatac aaaaattagc tgggcatagt 37320
   ggcaggtgcc tgtagtccca gctactcggc aggctgaggc aggagaatgg cgtgaacccg 37380
   ggaggcagag cttgcagtga gccgagattg cgccactgca ctctggcctg ggcaacagag 37440
   cgagactcca tctcaaaaaa ataaaaaaat aataaaataa aaataaaaaa taaatctgtt 37500
   taatcttcac aataattcta caacgaaaaa attgttatct ccattttata gctgagaaaa 37560
   aaatgaggca caatgaaaca aaagcacttg catagcacac aatataggaa ggagtggagt 37620
   gagaatctaa agtcaagttt acagcatgct gttttcaagg caaaactact tttgatttaa 37680
   aatcatattc ttttctctcc ccaatctcct ccagctagcg tttcatccgt gtggatttgt 37740
   caggagacaa ggaaaatcaa atttccagca atgcgtatct agtgtttaac aatcacatca 37800
   ttacacatga aaaaaatttg aagcttgtca gatcaccatt gtatgaaata tttcccactt 37860
   gggaccatac agctctgtat ttttctgatc agttacaatt tttaggatct agtctttatc 37920
   ccagagaccc agatctgtgt attctgtatt cttttgtacc ctgtgttatt gcttcaccaa 37980
   gagtatactc caggtgattt ttttgtgtgt ccttaatttt gtcattttgt ggtcattaat 38040
   aacatcagtt aacaataata ttattatact gtccactaca atgtgcgttt gtgagcactg 38100
   gcaacaattt agcagaacag aatgagttag attcccaatt gtggtagatc acgtgtagta   38160
   gcgaacagag cacagaaact ccggatccta attcttgatt tcctggcagt gataccttcc   38220
   cattccatta cataaggtat aagtggcaga agatcaggga gaaccacagg gctgtaagat 38280
   attttataag ttgttaaata aggggacact gctttatttg gggcatgtca ctcaatctct 38340
   tcaacattgt gttaggtagg tatcatgaac taaattgtgg ccacccaaaa ctcatacctc 38400
   gaaccccggg tacctcagaa cataattgta tttggatatg gagtctttaa agaggtaatt 38460
   aagttaaaat gaggttgtta gagagggctg taatctaata tgactggtat ccttataaga 38520
   agattgaaaa cacagatgtg cacagagcaa agaccacgtg aagacacaaa gaaaacagcc 38580
   atctgcaact caagaagaga gtcccagagc acattgtgat ctctgacttc tggcctccag 38640
   aattgtgaga caatgaattt atatcgttta agccaccagt ctgttgtact ttgttatggc 38700
   agcctgaatg ggccaataca gtgaggttcc ctggaagact ttcagatggg gacttgcatg 38760
   caagaagttt aacagggaga gctctcaaga gcaacccccg gagggagaga gggaggaaag 38820
   gaaaattggg cagagagaga agctgacctc tgatgcagct gctacaaggt tctcagctga 38880
   tcctagggtt ctcagctgat cctacagggt gttctggagc tgggatggcc ccgtagagtt 38940
   gcctggaatc aaggcagggc ccagggaggg agataacctt ggcaaaggca gcttccttca 39000
   gcgggggaat tcccagtgag ggactcaatt gtggctgttt gcagtgggca ctggacagct 39060
   ggggagtgag tttctccgcc ctgacgaggg gtctgggtga catctcacag catccatcgc 39120
   agacctgcag ctcctagtct gcagaccagg ataagcacaa tgcctgtggt gcatttcccc 39180
   gcaggcggct gtgaggagct aatgagataa tggatatgaa aatgctctgc aaactgtgaa 39240
   gactatcaat atgctgttat gcatcattct ctgttatttg ttgctatcct gtcctttcac 39300
   cttccccagg ccggaatgat cacattttct ctttccctag ctttattcct gggtcactag 39360
   cacagggaaa aaataagaag aaaaatcaga gtggaattct aaaaaacaaa ggtcctgtgc 39420
   ttttctgcct aaaatatgta caatcctcag ttggcactgt caggaatggg gaccgccctc 39480
   atggagaagt ggcacttcca ctcctagcca gcctctgtgt gagctggact ctaaggtcag 39540
   gagaatgagg aggccgtgaa ggtcttggcc gtctcttttt cccctcatct ctactgagac 39600
   atagatcgag acagggaccc ataaatcacc ttggagttaa atcaggggaa aaaagaaaag 39660
   ccctctcagg caacagcatt tcacagcagc aacaggaagt tgtagggaaa agagaaaagt 39720
   caaaactcaa gtgaaaatct gagtcatggt tcagagaggc cacaaatacc tttcttatgg 39780
   agagtcagaa gagggaacac agagaaaact gagaggtttt ttttttgttt gtttgttttg 39840
   aactttttaa aggagaattt gtttttaatc ctgtgaagct agtaaacatc tctttaaaga 39900
   atgtgtgtgg tcacacaagt tgaagggcta gaaaattacc tcctcacagc ccagatggtt 39960
   ttaagtggga acagatagga aggagcgtga gttaaagctc ccacccacac tacaggaccc 40020
   tgctgttcca gccgctttcc tctgccacta gctcaggaag agcgagggct gcctctcaga 40080
   cacagggagt tcctgcaggc tgacttcagg cactccagaa tgaggtgtcc gtattttcct 40140
   aagtgctgag ggtcacccta cctcctcctc ttttattaag attctctgaa ccactcagag 40200
   aatacacaag ttaaaaataa attacttcgg cagatagtga gggtacagaa gtcctcggta 40260
   aggttttcct tttaatgaaa accagcccca aatacttttc ctttctaaca aagagcagcc 40320
   tgtgaaatca agctacagac atagatgcca gcagaggaat actacctggg actaggcatg 40380
   ttcaaaatgg cggttccatc ttctcttcct tttttttttt tttttttttt tttttttggt 40440
   tagttagttt gtttttgttg ttgttgctgt tgtttgagac ggagtctcgc tctgtcgccc 40500
   aggctggagt gcaatggcat ggtggaggct cattgcaacc tccacctccc aggttcaagc 40560
   gattctcctg cctcagcctc ccaagtagct gggattacag gaatgcacca ccgtgcccag 40620
   ttaattttgt attttagtag agacggggct tcaccatgtt ggtcaggctg gtcttgaact 40680
   cctgacctcg tgatccgcct gcttcggcct cccaaagtgc tgagattaca ggtgtgagcc 40740
   accgcgccca gcccatcttc cttttctcag tgccagccac gtgtacatta aggaacagac   40800
   aacatggcgc gggtcaagtg gaaagctctt ttgcatagta agatgagggt gaggtagcca   40860
   gccttcctcc tgctatgtaa acatcacacc taatcgaact gatctgtggg ccctatgtaa   40920
   atcagacacc gcctcttcaa acctgcctaa aaatttggag aattccgccc cagcaagtct   40980
   ttccctttca gaagccccgc tctctcgcta gagagaaagc tgttctcctt tctctttctt   41040
   ctgcctatta aacctgcgct cctaaaggcc tcctttgtgt ccctgtccta aattttcttg   41100
   gcgccaggta acgaaccctg ggtatttacc ccagacaacg aagccgcttc agaatgacaa   41160
   aacaacagac atggggactt ctggcaaaca ataaaccaca aatgaacaga gagagattag   41220
   aaaataaatc taatcacagc agaggaggag gggagaaaat agcttctctt cccagaaaca   41280
   caggagagac agaaagcagg ctgccctgtt ctgcgcctgc tccattctcc agtaacatac   41340
   cgtcctggtg tagtgagact tttcgcatta tagggcccaa ctagagatcc agtcattttt   41400
   acttacgctg tgagccaaga ttctgagtaa atgggtttat cttgattgca gttaaagtat   41460
   ctggttgggc acatcctaac atgaatatta taccaacaat aaaaatcgtg gaaatgtggg   41520
   gaaatgtcca cgatatgttc ataacttaaa gtaggttagc agtgcattgt caacgtaaat   41580
   aaaacagtat attgagtgtg attccaatat tgaaaaacaa aataaagagc actaaatctg   41640
   ccaagttcaa agaccagagt gccagatgca aaatgtttaa tgtttctttc tgtgatggga   41700
   ttattttttt ctttatattt ttctgtattt cctgattttt ttctacaata atcatgtttc   41760
   tactgtaatc tgagatataa aacattagaa aatttggtca gatgagttgt tccaggattg   41820
   tgattatttc tccacaaaca gatgctcctc aactgataat gggattttgt tcctataagc   41880
   ccatcataag ttgaaaatac cgtatgtcaa aaatgcactt catacacctg acctactgaa   41940
   catcatagct tagcctcgcc taccttaaac atgctcagaa cacttagatt agcctacagt   42000
   tgagcaaaat catcttatgt aatttattgg ctaccatacc gaaagtgaaa atcgaatggt   42060
   tgtgttgttc gttgaagtat ggtttctaca aatacatatt gcctttgtgc catcataaag   42120
   tcaagaaatt gttaagtcaa atcatgagtg agggatcatc tgtacattca tacacacacg   42180
   gtatgaatac atgggggtgt gtgtgtgcat gtgtctatgc atgtaaatgt gtgtttcaaa   42240
   gctctggggt ccttcttcct tcccagctag gagccccttt tcctctgctg agctctcgaa   42300
   tcttgcataa ttgctcacca catttctgaa aagacaatct cttggtacag cacccccagc   42360
   cttctaccaa gggattccag cagatgttca ctcccagctc agcttggata gggccaccaa   42420
   ggagcctgcc tttggtctcc acagtcactt cctgaagtaa gattgaagag ggtgggagca   42480
   ccatggtttg ttgacctcca gctctgcttg tatctccttt ggcagaaggg gtcccaggtg   42540
   gtgcctacat tctagatttt agagaggaca ccacctccag agagctgaac ccaaaaactg   42600
   atgagctcca aagacgtgaa aaaggacaga ccctgccaga cgagcaccct taggttgttg   42660
   gcacggaact gagggaggaa actgggacta cccgcctgat gttgcagctt ttttattctc   42720
   ccctcgtcac tgtgagaggt ctggacttct tctgacataa acagcctcct ttcatcaaag   42780
   gatcgtgcca tcctttctat tttctaagca ttaaaaggca gtttaagcag atcagtgaat   42840
   taggcaaacg tttatattct cccttgccct tttctttttg catctggtag aatttctcct   42900
   gttctattgt tgatctcttt gtaaaagcc                                      42929
<210> 100
   <211> 4108
   <212> DNA
   <213> Homo Sapiens
<400> 100
   ccggccgtct atgctccagg ccctctcctc gcggtgccgg tgaacccgcc agccgccccg      60
   atgtacagca tgatgatgga gaccgacctg cactcgcccg gcggcgccca ggcccccacg     120
   aacctctcgg gccccgccgg ggcgggcggc ggcgggggcg gaggcggggg cggcggcggc     180
   ggcgggggcg ccaaggccaa ccaggaccgg gtcaaacggc ccatgaacgc cttcatggtg     240
   tggtcccgcg ggcagcggcg caagatggcc caggagaacc ccaagatgca caactcggag     300
   atcagcaagc gcctgggggc cgagtggaag gtcatgtccg aggccgagaa gcggccgttc     360
   atcgacgagg ccaagcggct gcgcgcgctg cacatgaagg agcacccgga ttacaagtac     420
   cggccgcgcc gcaagaccaa gacgctgctc aagaaggaca agtactcgct ggccggcggg     480
   ctcctggcgg ccggcgcggg tggcggcggc gcggctgtgg ccatgggcgt gggcgtgggc     540
   gtgggcgcgg cggccgtggg ccagcgcctg gagagcccag gcggcgcggc gggcggcggc     600
   tacgcgcacg tcaacggctg ggccaacggc gcctaccccg gctcggtggc ggcggcggcg     660
   gcggccgcgg ccatgatgca ggaggcgcag ctggcctacg ggcagcaccc gggcgcgggc     720
   ggcgcgcacc cgcacgcgca ccccgcgcac ccgcacccgc accacccgca cgcgcacccg     780
   cacaacccgc agcccatgca ccgctacgac atgggcgcgc tgcagtacag ccccatctcc     840
   aactcgcagg gctacatgag cgcgtcgccc tcgggctacg gcggcctccc ctacggcgcc     900
   gcggccgccg ccgccgccgc tgcgggcggc gcgcaccaga actcggccgt ggcggcggcg     960
   gcggcggcgg cggccgcgtc gtcgggcgcc ctgggcgcgc tgggctctct ggtgaagtcg    1020
   gagcccagcg gcagcccgcc cgccccagcg cactcgcggg cgccgtgccc cggggacctg    1080
   cgcgagatga tcagcatgta cttgcccgcc ggcgaggggg gcgacccggc ggcggcagca    1140
   gcggccgcgg cgcagagccg gctgcactcg ctgccgcagc actaccaggg cgcgggcgcg    1200
   ggcgtgaacg gcacggtgcc cctgacgcac atctagcgcc ttcgggacgc cggggactct 1260
   gcggcggcga cccacgagct cgcggcccgc gcccggctcc cgccccgccc cggcgcggcg 1320
   tggcttttgt acagacgttc ccacattctt gtcaaaagga aaatactgga gacgaacgcc 1380
   gggtgacgcg tgtcccccac tcaccttccc cggagaccct ggcgaccgcc gggcgctgac 1440
   accagacttg ggttttagac tgaacttcgg tgttttcttg agactttttg tacagtattt 1500
   atcacctacg gaggaagcgg aaagcgtttt ctttgctcga ggggacaaaa aagtcaaaac 1560
   gaggcgagag gcgaagccca cttttgtata ccggccggcg cgctcacttt cctccgcgtt 1620
   gcttccggac ggcgccgacc gccggagccc aagtgacgcg gagctcgtcg catttgttat 1680
   aaatgtagta aggcaggtcc aagcacttac aagttttttg tagttgttac cgctcttttg 1740
   ggttggtttg ttaatttata caaagagatt accaccacca ccccctcctt cagacggcgg 1800
   agttatattc tgggttttgt aaaactttat gtatctgagc atttccattt ttttttttgg 1860
   gttttgtatt atttcttgta aatgcattgt gaaaaatttt attttcggcg ttgcaatgcg 1920
   gggaggagaa gtcagattat gtacatagtt ttctaaaaag cctttcttct aaaaacgaaa 1980
   aaagaccccc cacccaaaat gtttcgagtc aacaaattta agagacagag cccattttct 2040
   ccataaattt gtaacatgct atttttatgt gcatgtttta tgagttcaaa atgcaatgag 2100
   gaaatctgac agggaaatta tctgtatgaa ctaaaagtaa gggaaccccg gggaatggga 2160
   ggacaggatt tttcaaggaa cctttttcaa tgaaagagaa ggaagttaaa acctataggt 2220
   tattttgtag agctgagtgt taatacgggc cgagaaataa aagtatcttc tgctccggct 2280
   gtttcactgc ggacggctgg ggctgctgcg cgttaccttg ctgcaagcgg ggcgccttcc 2340
   acctggctgg gggtctgcgc cacagtttgg tccagaggag ggaggaggaa gggaagaccc 2400
   cagtggtggg accctggacc aggccatgga tgaaggacaa agaccagggc aggtcacggg 2460
   tttcccaatt ccccagcaat taagatttcg agcagaattt atctaaatgt gtttcaagga 2520
   aacacaatcg ctgaaccaaa acgtactgca gccgagcccc ctccgtccat cctctgcccc 2580
   tccccctggc ttctttctct tgggaaaacg ggcaaaataa ttgtgctgga ttctcacaca 2640
   cacagaaata tcgaccatca ccctcccccg cgtgaactgg gatgcaagtt gctaaccgat 2700
   gtgaacgcaa aatgccttgt tcattattcc tgacgagatc ttgaggttgt ttgatgcttt 2760
   aaatttttta attatattat tttctaggtg tttattggta cattgcagtt ttttttttga 2820
   aatttaaaaa tttctgtaaa actttgtctt caagtaatct gacagcatta aatattgcat 2880
   ttaaaaatta tactgtagca aatacattta aaaattaatc acaacgttaa gatgaaatta 2940
   tatttttgga aaaaaaaaac acttgaagcc cagatggaaa tacgtttatt tcagcagcct 3000
   taggtttccc ctcgctttct caacaccctt ccttgtcctg gagtatggac tgtccgtcca 3060
   aaagtgagcc tatgctataa gtttaatgag aaccgaattc agcctgcatt cgagaatagc 3120
   tttaagtata atgctgatct gacaattgac gtgtaatttg ggaagtcatt ttgataattt 3180
   tgcttaaacc actcattcgt taaagtgatt acaaaaaagt tcaagaatga tgtccactgc 3240
   tttctaacaa gataataaac cccccccctc ttttcttttt ctttattttt atttctttta 3300
   gctatttgat cctttctgaa gcagttgttt ctggaagagt ctgtgcgccc atggatggct 3360
   gagcaccact acgacttagt ccgggataag ggcctcccca gtcctctccg ggagatgatt 3420
   tgggaaattt tataatgctt gttctgttaa ctcaccggga ccttgagggt ccaatgggac 3480
   cttgagggtt ttctctgaaa tatacaaact taaaggactc tctctgaggt tctttgactg 3540
   acgtccactc tcagtctggc ccctgtgctc ccctgtgtgt accctggagt ttctgtgtcc 3600
   aattgttggc atctaggtct tggctcaaga ttaggatgtg ggccccactt tagaggcaca 3660
   gactatgaaa agctgagtta gtgcgcccgg gacgccaggc aagcagcttt tacagtttgg 3720
   catcttattg caggtgcttc gtgcacagtc agctgaaata gccaatgcca ggtgctccaa 3780
   ccaccttatt tccttgtttt gttgattaga acaacacaga aaaaagcaaa tataaatttt 3840
   taatgactcc atttaaaaat atcacagggt gggggcaagg aaattagctg agattcatct 3900
   caggattgag attctatccc cccttccccg cccccagcag tgtcgctcca attcaaatta 3960
   gtggagaaaa gattacagta ggccctgagc cgactgtgaa ttcggtgctt ggccaaggta 4020
   acactcatcg tattcacgga gtgaaatact atatgatgat agttattata ttatatgacg 4080
   acttcattca cttcccaaat cacagggt 4108
<210> 101
   <211> 29262
   <212> DNA
   <213> Artificial Sequence
<220>
   <223> chemically treated genomic DNA (Homo sapiens)
<400> 101
   ttaagtagaa atgtagttta taatttgtaa ttgttgtatt attttttttc ggtaggttag     60
   tttttttttt atttgatttt gaaaattatt tggtaggaga gagagataga gaaagattaa    120
   ttaaagtgta aaatttattt ttagtttagt tagtaaaaat attattgata gttttatgga    180
   tagttttttt atttttaagt atttttttta taggtaaatt atatgtttgt ttgaagaatg    240
   tttttaatat tttataattt aattagatta gatagtaaat tttgagtgtg ttttatattt    300
   tatagtattt tatattttat ttagtataat tttttgtata tgattatata gaaatatttt 360
   atataattat gttttttaat ttttgtatat ttgtgtaaat tataggtatt gggagttttt 420
   attgttttta gaagtttagt aagagttagt agggtaggtt ttagatagag ttgagtatgg 480
   gaaatgttgt tttttttgta tataatattt ttcgggtaaa taagatgtga gtttgtgaaa 540
   aatgtttgtt tggttttatt gtagtttatt ttatttttaa gagagaatat ttcggtttga 600
   tttttttgtg tagtaaattt ttttattttg ggattagttt tattttatgt ttttgttttg 660
   ttttggtgtt tttttataga ttttatttta atttgagaag aagagaattt taagagatat 720
   tgttatgtaa aatatttttg ttattttata tattgattat atttattgta ttagggaaaa 780
   aatgattttt aaatgtatag tttagtgtta tatttttttt acgtgatttt tttaaaagtg 840
   tggacgtggt taattttgtt tttttttgta ttgaaggaga aatgagtacg attttttatt 900
   gaatttgtgg ttgatagggt agtagtttga ggaaattaga aattttatta ttattttttt 960
   tttttttttt ttttttattt ttatagttaa ttggtgtgtg agttttatgt gttttattat 1020
   tatatttttt tttttttgtt attgttattt tttacgtgta ggtattttat ttttgaatta 1080
   tttttcgatt gattatttgt tattagtttt ttttttttta aatattttta attttttgtt 1140
   taaaaatttt taatggtttt ttaatatttt taggatagta aattttttat tatttatgag 1200
   attttgggta tttagagatg aaaaggtttt gtttttttta ttttttttaa tttttatttt 1260
   tttatattat ttgaattttt tatttaattt taaatatgtg ttttaattaa tttttttaaa 1320
   aagatttaat ttttattgtt atttttgatt ttgatgttaa agttattgta atagagggaa 1380
   gtatgtggtg ttatgtaaag attgagagaa gagttgttat ttattttggg aatattagag 1440
   gggagagttg tttgagtaga tacgtggaga atagtatttt atataaagat atggaaggag 1500
   gagaggggaa ttgtatatgg tttagttggt tgtagtcgag ggtacggagg aaattttttg 1560
   tattatatta gttattaaat tattttttag tttttaatta tgtattatat ttttagtttt 1620
   tgtgtttttg tttttgttgt ggttttgttt ttgtagtaaa taattgtttt ttttttaaag 1680
   tttagtttag atgttatttt ttttaggaag ttttttttaa ttttgtagtt ttttttgtta 1740
   gaattattta tttatttttt tttgtatttt tttagtattt tgtttatatt tgtattaatg 1800
   tatttataat atatgttatg attagttgtt atttatttgt ttttttgtta aatagttgag 1860
   tttttagagg gtaggaagat acgtttagat attttaaaaa tttttaaggt ttattatagt 1920
   gtttggtata cggtaggggt tagttaataa acgaggagta aaaatatata gaattattta 1980
   ttagtaggtt gagattttgt tatttatagt aattatttta ttttggagtt ttagattttt 2040
   atttaaagaa ataaaggtgt gaaataatgt gtttttttag gtgttttttt gttttagtat 2100
   tttaagtttt gatgataggt cgagtgtggt ggtttaagtt aataatttta gttttttggg 2160
   aggttgaagt aggaaaattg tttgagttta ggagtttgag attagtttgg gaaatatgga 2220
   aaaattttgt ttttattaaa aatataaaaa attatttgag tatggaggtg tataattgta 2280
   gttttagtta tataggaggt tgaggtagga gaattatttg ggaagtcgag gttgtagtga 2340
   gttgagattg tattattgta ttttagtttg agtgataaag tgagattttg ttttaaaaaa 2400
   aaaaaaaaga aaaaattttt ggtgataaaa taaattgatt tgattgggtt ttatgtattt 2460
   atttgttttt tttgtgtagg ggaagtgagg gaaagatttt gatttatatt agtaataatt 2520
   aggcgaaagg tattatgtga ggagttagta tttattttat taggttagga tgttttagag 2580
   gtgggtttaa gggtataggg taatagggga attagttttt tataaattgt tttggtttta 2640
   atatttggtt ttgcgttgtg gttcgtaggt ttttaggaag attagtgtgg ttttagatat 2700
   tggagtatat ggtaagtatg gttttttttg tgaagaagaa aatttttttt tgagttttta 2760
   gggatttaag atgtttttga ttagggtggt aaattaagat gaattaggaa ggaggggttg 2820
   tttgaagtcg attaggttgg agtatttttt tgggattata gaagatttta attttagagg 2880
   agggaatagt taagggtaat ttagagtgga atgatgaaaa ttagttttaa tttaatttat 2940
   gtttattttt ttatgatagt atatttaata taatttttta agttaaaatg aaaaataaaa 3000
   aagaagttat taaagtattc gaaataatgg taaatttttt tttaagattg gagtggggaa 3060
   gagtataaaa ggtaaaaatt gaaaaggaaa agattggttg atttgattag atagaaattt 3120
   aaaattttgg tatgttaata aattataaat agattaaatt ttttaaatat aaaatattat 3180
   agttaatatt taaaggtaat tgatataaat taaaaaatat ttgtaatatg ttaaagaatt 3240
   aatattttag tataagaggg tttatataaa ttaataagaa aatgaatata ttaatagaaa 3300
   tatggggaga gaatataaat gggtatttat aaaatatatg aaaggatgtt agatatatga 3360
   aaaatgttta atattattag taatgaaaga aaaggaaata atatgaaata ttaaaatttt 3420
   tttttaagtt ggtaaagatt aaatgaagag atgatatatg ttgttttaaa gatgggagta 3480
   gaaattagtt gaaatttttt ggaaagtaat ttgaaattat atgttaagat ttttaaaaag 3540
   tttatatttt ttaatataat aatattttta ggagtttaat ttaaggaaat aatagtggat 3600
   atatgtaaag atttagttat aagggtgttt attatttaaa atttggataa attttaatgt 3660
   ttaatataat agtatattta tatattgttg tgtagtaata gaacgaggta tattttagtt 3720
   aaaataaaaa ggaagtaaaa aatgttgtgt attatatttt attttgtttt ataaaagtat 3780
   ttaataagta tttaaaaagg ttgaaatttt aaaatgttaa tattggttat ttttaggtgg 3840
   taagattatg ggtgattttt gttttttttt ttttttattt ttgtatattt aaaatttttt 3900
   ataataagga tgtgtatagt ttttgagata taaagattgt taattggtaa atatataaaa 3960
   atttaaaatt ttttttatgt tttttttttt ttttttaaat ttgaggtaga gttttatttt 4020
   gtcgtttagg ttggagtgtc gtggtatgat tttagtttgt tataattttt gttttttggg 4080
   tttaagcgat ttttttgttt tagtttttcg agtagttggg gttataggta tgtattatta 4140
   tgtttggtta ttttttttgt atttttggta gagatggggt tttattatat tggttagggt 4200
   gtttttaaat ttttgatttt aagtgattta tttgttttgg ttttttaaag tattgggatt 4260
   ataggatgag ttattacgtt cgattatatt ttttttatgt tttaaaggtt gatgaagata 4320
   gagtttaatt ggagatggaa aaaattatta agttaatttt tgtaatgaga attatttata 4380
   atttaattat ttttggaaaa tgataatacg aaaggaagta gtaaagaaga gtattgaagt 4440
   gtagtatgtt agttaatata atagtaaggt tgtatattgt aaatttagat agttggaaaa 4500
   gttaaagggt tttttaaata gtaataaaaa gtatttagtt tatattttta gaattataag 4560
   taagaagata aatttatagg gtatatttgg tttatatttg gtttatttgg aatgttttaa 4620
   gtaagtagtg ggttttgggg tttaatttgt ttataaatgt ttttattttg ttatttttat 4680
   ttttaaagaa ttattgaatt tttttttttt tgagataggg ttttgttttg ttatttaggt 4740
   ttgagtgtat tggtgtgatt agagtttatt gtagttttga ttttttgggt ttaagtgatt 4800
   tttttatttt tgttttttta gtagttggga ttatttgtgt atgttattat atttggttaa 4860
   tttgtgtgta tgtgtttgtg tagagatagg gttttagtat gttgtttagg ttgattttaa 4920
   atttttgggt ttaagtaatt tttttgtttc gattttttaa agtgttgaga ttatagattt 4980
   gagttattgt gtttagttaa aatttaaaaa aaaatttttt ttatagttat tttttattga 5040
   aaggagttta atttgtatag tattttggaa tttgttttgt tatttcgatt ttgtatttta 5100
   ttttttagag tatgttataa aaagaaagaa gaaggaagga aggaagggaa ttattgaata 5160
   taaaatgaat ttaataatgg ttatagatga aagatgtaga taattatatt tagggagatg 5220
   agatgtagtt tgtttttaat aataaggatt tttaatagta atttggaaga gtaatttttt 5280
   ataagaattt aaaatgatga tattttagat gaaatttaat attttttata aagttttatt 5340
   tattagtgtt tttttgtaat aattaattgg tttttttatt tagtttttgt aagtgttatg 5400
   aatttgttat tagatttgta aatttaagga tttaatttat gagttttgga tatttatttt 5460
   tatataagtt tttagtgatt taattttatt ttttattgtt agttttaatt tttttgggta 5520
   taattttaat gtttttatta attatttatt gagagtatgt tatatattta taatatgtta 5580
   ggtgttgtag gtggtaatag aagaatattt tgggtagttt ttttttgaat ttatttataa 5640
   tgtgggttgg gggagtggag agtaatacga gtgaaataga ggaggagtta atatagaaaa 5700
   tttatggtat tagaaatgaa tataatagga ttttagaaaa tcgtgtttat tgtaagagtg 5760
   gaaaggtttt ttggatgtgg taaaatttta gttgtgtttt agataagaag gaggatttgg 5820
   atgaggggag gcgttttgga ttagaggaat acggtgtgta gaggtggagg gagaggggtg 5880
   agagtggttt gttaggtaat tggagttgag tcgggggaga agtagaaata agggtggaga    5940
   ggttggggga ggggtatata attatgatag gttttgagag ttagttaatt tagatatatt    6000
   atgatatata gtgtaaatcg ttttagtttt ttgaggtgaa gattttgtgt taaatttaga 6060
   gttttaggaa gatagggtta agagtagtag gtagaaaggg ttggagtgga gaagggaggt 6120
   gaggatgtag gttattttgt tggtgacggt tttttttatt tattgttatt tttagtaggt 6180
   tttaggtttg gggttaggtt ttgaggtttt aagatggtta atgggaagaa ataaaaatga 6240
   ataaaaatta tagtaaaacg atgaatgttg taataaagga aggtataggg tgttggagga 6300
   aaagagagga agaagagata taattgttgg ggaaggtgta tataatgtag tggtaaatat 6360
   aatttttgga tttatagttg taaaaatgat tagattagtg tgagtgaggg agtttggaat 6420
   ggtggaattg ggagttattt ttaataattt aatttgttta attgaatatg atttttattg 6480
   taaaatatgt ttgattagtt attttattgt tattttaaag taattttgat aatggtgggg 6540
   ttttttttaa tttatttttt taaatattta tttttattta attttagttt tttttttttt 6600
   tttagtatat tttttttatt tttttgttgt gttttattcg gttcgttgat taaatttatt 6660
   gtttagtatt aaaataagtt ttattttttg tttttttttt tatagtgata gtttaataaa 6720
   tttttataag ttttggtttt ttatattagt tggtttatta tatagttatt ataagaatgt 6780
   tagtattaaa ttttaaaata gtatatagta gtaataattt atatttaata gtaattatta 6840
   tttatttgtt attatgttaa gtattttatg ttttttattt atttaatttt ttttataatt 6900
   ttaagaggta ggtagtagta ttgttatagt gttgaaaaga gattagggaa tttttttaag 6960
   gttatttagt tagtgagtag agtagttagt atttatattt ggaatttttt ttatgatttg 7020
   ttatattttt ttgtttttgt tgtaatggta aatgtttgtt ttgttttgtt tttttttttt 7080
   tagatttttt tattagggtt atgtataagt attagtggag agtagttttg gaagtttttg 7140
   ttttagtaag cgtagagatt tgtttgtttt tttttttttt tttaattgtt gttttttaaa 7200
   tgtggagatt tgtttttttt tttttatttt tttttttttt tttttttttt tgtttttttt 7260
   tttttttttt tttttttttt tttatttttt tttttttttt ttttttattt gttttttttt 7320
   tttgttattt ttatttgtat tttttttatt tttacgatta tttttgtggt ttttattaat 7380
   ttttggaatt acgataagaa gttaattttt tttttttttt tttttttttt tttggagacg 7440
   gagttttttt gtgttgttta ggttggaggg tagtggcgtg atttcggttt attgtaattt 7500
   ttgtttttcg ggtttaagtg atttttttgt tttagttttt tgagtagttg ggatttatat 7560
   tattatatta gttaattttt tatatttttg gtatagacga ggttttatta tgttggttag 7620
   gttggtttta aatttttgat tttaagtgat ttgtttggtt tagtttttta aagtgttagg 7680
   attataggcg taagttattg tgtttggtta gaagttaaaa atttggattt aagtttagaa 7740
   atgttttttt agagtttagt gttgtttttt tttttttttt ttttttgttt tataattatt 7800
   attataattg tatttatttt gtgtttagta tgtgtatagt attttatata ggttttttat 7860
   ttagtttttt tattttttta agcgatagga attatttgtt ttattttata gtttttaagg 7920
   ttatataatt agtaagtagt agagtttgaa agtgaattta ggtttggtcg atttttgagt 7980
   ttagattttt aatttttata tgttatagtt tttagtttag gtttatagtg ttttaagtga 8040
   attggatttt aagtgtggtg attgtgagta tttgtttatt ttttagtaaa aagttttgta 8100
   aaatttttac gagaattttt ttatgttttt tttcgatgag gttttgtaaa ttttggtttt 8160
   ttttttttta tttgagtgaa tttaaatttg ttttttagtt ttacggtttt ttatatttag 8220
   ttaataagta tataatgtgt atttgttatt tttattcgtg tgaaattgat tgtttttttg 8280
   tggtgtattt aaattacgta ttatttaaat ttgggtatat atatgttatt tttttggtta 8340
   gtggagtatg ttgtagatga aaaagaggaa aatttttttt gttattgttt aattgaagtg 8400
   ttttatatat gtggtaatta tagaggatat atattagtgt atattaaaat gatgaatgag 8460
   aggtttgaat tgaaattggt tagtattgta atattttaat attttgttta acggaaatat 8520
   gtattataga gtagttatta tgaaaaatat aaattattat ggattattta attttttaga 8580
   atgaatatga attggttttt attaatggga tgaattatga ttatagaggt tttataggga 8640
   gttagaaggg tatgttaggg atttaatggg atttggattt attttgttag atttaaaagt 8700
   agggatttgt aaatttaata attagataaa tattagttat tgggaaaacg tatttaatgt 8760
   tttttattgg gggtaaggaa ttgtgtagtt ttttttaaga tatagtttta aaagttagag 8820
   aattttagat ttttttaaag tatttgtttt ttatggtttt aaagatattt taaatgtttt 8880
   tttttttttt aaagttataa gtattataat atttttaatt atgttattaa aagttattgg 8940
   ttattttttt ttttttagtt ttattttaaa gttttgtagg tattttttga atttaaaata 9000
   attgaagtat tatgagtttt agtttttttt ggagtatgta gagaggatat attttgtaga 9060
   tttttttatt ttttgtggtt agagtttttt tttattatgt agttattttt atattttgaa 9120
   tttttattaa atgttttggt taaatttagt ttgatatgtt attaggaatt tggtgttatt 9180
   taaaaatgtt atttaagtgt gttatttagt tgtgttagtg attatattta ggaagtttta 9240
   atgaatgaag atttttaggt tgatattatt gaaattatta taattattta aaaatttttt 9300
   agatgttgat ataggaatta tttggtatag gaattgttaa tttgagtttt gtgatttttg 9360
   gattaataat aagaagtatg ttgatatttt aaaaaagtgt tttggaaaaa attaatttta 9420
   aatatatata attagaaaaa taagtttgta ggttaggtat agtggtttat gtttgtaatt 9480
   ttagtatttt gggaggtcga ggtgggcgga ttatttgagg ttaggagttc gagatttgtt 9540
   tgattaatat ggtgaaattt tgtttttatt aaaaatgtaa aaattagtta ggtgtagtgg 9600
   tgtgtgtttg tagtattagt tatttcggag gttgagatag gagaattgtt tgaatttagg 9660
   tggtagaggt tgtagtgagt tgagattgtg ttattgtatt ttagtttggg tgatagagta 9720
   agattttatt ttaaaaagaa aaaaaaaata agtttgtata ttattttttt attttttttt 9780
   ttttggagag gtaatatgat taaattaaag ttcggaaagt ttgaaaaata ttattgggat 9840
   gtatttgttt agatgtgatt tagattaggg taggtgaatt ttaaaaattt ggatatttat 9900
   tataaagttt taggaaattt taagattttg ggagtagaat taatagtttt taggttataa 9960
   tattttttgt ttttaaattt agaatggaat tttaatatta aatatggatg ggaaatttaa 10020
   aattattaaa attataataa ttttatttaa taaaatagat tgtgtatatt gagtttttaa 10080
   agtttagatt tttatattta tatatatttt aagtattagg aagttattag gaataatttg 10140
   agaatgaaag ttaatttttt tttgtttttt ttattttaga gaatgtaaat ataaagtagt 10200
   ttttttgttt taaaatttat aatcgaatta tatttaaata tttaaatata tttatatatt 10260
   taattatata tttatatata ttttaaaata cgtataatat gtatatatag atatatatat 10320
   atttatatat aaatattaaa aggtttttgg aagaaatttt atttgtgagt taatttaaag 10380
   tttaaataaa attaaaattt atgaatattt aggtagatta tgggaagatt gtgtgtgttt 10440
   tgtataatcg tttaatattt agaattatat ttttaagttt aataatagaa acgtaattaa 10500
   ttattataaa tataatgaaa taaaagtgat tttttattga atattgggaa agatgtgttt 10560
   ttttttaata tttggaattt ttgttttagt cgttttcgta aatttatttg tttaaataaa 10620
   attttttgaa gtttttttaa taaaaaaaag aagagttatt ttattaattt ttataaataa 10680
   ttttttagag aattgttata tatggtaatt tattattata agataaaata ttttattagt 10740
   ttaaaaagtg ttgtatagat ataggataga ttttgtgtag aaatttatga atataaatta 10800
   atgtaatata ttattttaaa gtattatatt atagtgttat gtattgattg atatgtatgt 10860
   attaatttta attatattta tttaatggtt tatatattat ttatatgttg tttgttaagt 10920
   taaaaatatg gtggagaata aaaatatttt tttattattt ttatagtcgt ttaaagttaa 10980
   gagaagtttt atagaattga aatttttatt tatttttttt tatatttttt atattttttt 11040
   ttagtgaatg aggtgagttg gttttttgtg tatgtttatt ttttaaaaaa gtaggaaaaa 11100
   tattattagt tttgtgtagg ttagttttgt aagttttttt ttatattttt ttttaatatt 11160
   tgtaaaatag tttatttaat ataatttgaa attggaagtt tttataatta aatgttgttt 11220
   ttatttattc gtatataatt tttttgaaat acggagggga ggtgtagaaa aggagggtag 11280
   agtttgtttg gtttgtgatg gtagttttcg aaaagtagat ttttgaagtt attttttttt 11340
   taatttttta tcgttattta tttttataag gaaggaaatt gttagaatgg gtaaaagagg 11400
   agtttggata gttaagattt ttggtagaaa tttgattgga gttttattgt tttttgaagg 11460
   gaggaagttt ggtttgaatg gaaaggtttt taagattaga ggtaggtgtt tagttttttc 11520
   gggttattaa aatttgggat attgaaaaag aaatgtgagg aggttttgag ttgtaaatga 11580
   taagtttgga ggggtttttt aataattgat agtttgtttt tttttaaaaa aaataatttg 11640
   ggtcgggcgc ggtggtttac gtttataatt ttagtatttt gggatgtcga ggcgggtaga 11700
   ttatttgacg ttaggagttt aagattagtt tgattaatat ggagaaattt cgtttttatg 11760
   aaaaatataa aattagttgg gcgtggtggt gtatgtttgt aattttagtt attcgagagg 11820
   ttgaggtaga agaattattt gaattcggga ggcggaggtt gcggtgagtt gagattatat 11880
   ttttgtattt tagtttgggt aataagagcg aaattttatt ttaaaaaaaa aaaaaaaatt 11940
   aatttaaagt taggtgtggt gatttatatt tgtaattggg aggttgaggc gggaggattg 12000
   tttgaggtta ggagttcgag attaatttgg gtaatatggt aaaattttgt ttttataaaa 12060
   atagaaaaat tagttgaatg tggtgatgtg tatttgtagt tttagtaatt tggaggttaa 12120
   ggtgggagga ttatttgagt ttaggagatt gaggttgtag tgagttgtga ttgtattatt 12180
   gtattttagt ttgggtgatt ttgttttaaa aaaataaata aataaataaa ataaaaagta 12240
   aaaaaaaatt aaaaattaat ttaaaataga gataaaataa gttaatttat attagtttgt 12300
   aaatagtgat ttgtttgttg atatttttta attgtttttt ttcgattaaa gaaaattata 12360
   ttgcgtataa aggttagatc gttattttta tttcgtttat atgttgatta ggatggtaag 12420
   ttaagaaaat gtttttattt atagttatga attgagttta taaatttggt ttaaataaat 12480
   tagaatttaa ttatttaggt aattagaatt tttaaaaaat ttaatttttt atttttaatg 12540
   tttatataat ttaattaagt tttttatttt tgtttgaaga ggtgtggtag aataaataaa 12600
   ggggttttga ggttttatgg attttttttt tttttatttt attttttttt tgtgttttta 12660
   gagttttttg tataggtttt aggtatggtg ttgttttttt attttttttt tttagttagt 12720
   gggttatgtg ttatttggcg gtagaggtta ggtatggtta gtgtttagta tagcgttgaa 12780
   tatataataa gtatttaata aagggtttgt agttgttttt gaaaaggatt ttaggaggtc 12840
   gtaaatttta aattatagta agcgggggtt agaatttatt aaagattttt ttattattta 12900
   ttttttttag taaaaattaa aatgtaatgg ttttgggtat ttgtagaagt acggggagat 12960
   ttttttgttg aggtaaaaat ttttgtaaag agggaaatag aatttttttt tgaagtgttg 13020
   atttataata gtattatgga tagataggga aatttttaat atgatattat taatggtaat 13080
   ttaagtgtat tttatttgat gattgtgagg agtatgtttg tgtgtgtttg tgtatgtgtg 13140
   tgtgtgtgta ggtaaatata tttgtgaaaa tatatataaa tattttagga tatatgtatt 13200
   attagaggaa ggagtttaat aatatttttg gtatttttaa gttttttaat agtaagtatt 13260
   ttttttaata tttagattag atttttatag ttttattttt atttttattt tggatatgat 13320
   ttttttatag gtttagatgt atttttgaat tttgatattt tggttttttt ttttatataa 13380
   aaagaaaata gagaggaaat atttgtgaag tattttaaat atagatttta atgtaatttt 13440
   tagaaaaata ttgttattta aaataagagt aatatgaagg ttgataataa attattaatg 13500
   ggtaaattaa gaaaaatagt attttgagat gaagatatat gtatattatg tgtagtttta 13560
   gttaattagt taaaggtgta gaagttttaa aaatattggt atataatatt gaatttgaat 13620
   aagagaagaa agaagtatgt atgtattttt gtatttattt gtagggtata atgatattat 13680
   tattttataa tttttttaat tatttaaagt atgatttaaa tgagattttt atttttgaat 13740
   aggtaatttt agaaattata gttttattaa attatagatt agattttttt tgatttatta 13800
   gtatattttg tggattgttt attttatatt atgttttagt tagatagttg aatttaaaaa 13860
   ttaaaatatg atatgtaaaa tgtatttttt ttttatttta taattagtag ttttatattg 13920
   gtatgaaatt attaaataag tagttagtaa atggaaatta tattaaatat aggtaaattt 13980
   attgatgatg ttaataaaaa gtttgttttt ttattagttt tgtagaggtt ttattaataa 14040
   tttgtattgg tatttatttt aaatagatat tttgttgtat tttttgggta aaatttgtta 14100
   attttgagtt tagaggattc gaatgtttta tttataaagg gtttttaaaa tggttaaatg 14160
   ttaggtaaat aaaaagtata gaagttttcg ttgttttttg tgtatttttt attggattgt 14220
   tgtttttcgt atattattaa tttgtgaaaa ttagattgtt aatggtggtt aaaaatgatt 14280
   tttgggagtt tggatatatt tgtttattat tattgttaat ggtttttatt tggttaaaaa 14340
   aaaaggtagt ttaatattag attaaataaa cgtgatttag ttgatagtga gtttttaatt 14400
   tttttaaaaa tttttttagg ttggttatat tttatttttt tagaattaat gaatgggttt 14460
   ggaaatattt ttaaagtgtt tattttttta ttttttatat tttttttatt ttttttgttt 14520
   ttaaatatgg attgttttag aaatttgtat gttatttttg tataggggtt atgttaattt 14580
   tttttgtatt gttttaattt tagtatatgt gttgttaaag tgagtaatgg attttttttt 14640
   tgaaaaaggg gttggtgagt tagatgtggt ggtttatgtg tataatttta gtattttggg 14700
   aggttgaggt aggtggatta tttaaggtta ggagtttaag attagtttgg ttaatatggt 14760
   gaaattttat ttttattaaa aatataaaaa ttagttgggt atggtggtag gtatttgtaa 14820
   ttttagttat ttgggaggtt gaggtaggag aatcgtttga atttaggagg tagagattgt 14880
   agtaaatcga gattacgtta ttgtatttta gtttgggtga tagagtaaaa ttttatttta 14940
   gggaaaaaaa aaaagagagg gggtgggggg aagtatagta attttaatag tgtagagttg 15000
   taggagagaa aattatatat ggaagaagta tattttttgg aagtttagtt ttaaatatat 15060
   ttaagattgt agaaaaaaat tggtttgaag ttggagagta acgggtaatt agggtataaa 15120
   acgttttttg tggatggaag tgattaaaag ttgttgttaa aggtttagtg ttatggttta 15180
   tatttgtaat tttagaattt tgggaggtcg agggagagtg tttgaattta ggagtttgat 15240
   attagattgc gtaatatggc gaaattttgt ttgtataaaa aagataaaaa ttagttggga 15300
   gtggtggtgt atttttttag tttcggttaa tttgagtggg aagattattt gagttttgga 15360
   ggttaaggtt gaggtgagtt attattgtat tattggttat tgtattttag tttgggtgat 15420
   agagtgagat tttgatttaa atataaaaaa gaagaagaaa agaaaaagtt gttgtttagg 15480
   aaggtgttta tattaggatt ttttaagtgt atatttgtag gatattgtga tttgtatggt 15540
   ttttgagaat tttgtttatt taggagggtg gaggtagtat cgtgttatat tagggaaatt 15600
   atattattgt tgagtgtaat tggagattag agagtttgga atgttaggag tcggtttttt 15660
   tggttttttt aaggattttt aatagtagtg gttttatatt ttaggtgaat attttaattt 15720
   atttttggtt ttgtttttat atgttattag aaattggaag tatgagaaag atgggtgaga 15780
   gaagtaatat gttgaagtag atatgagtag taaatagtaa atgaggagga gatggattat 15840
   atgaataata taaataagta aaaaaaaaaa aaaaaaaaaa tagttaatag atttaatagt 15900
   attagttttt aaggtattag aataattatt tttttttaaa tattatggta agtttttgtt 15960
   taagaatttg taatggtttt ttatggtttt tattaaagat tttattttaa agtttttttt 16020
   ataaagtttt taggtttttt tatttttgtt taatttgttg agttattttg tttttttttt 16080
   tgagataagt tttcgttttg ttatttaggt tggagcgtat ttatataatt taggtttatt 16140
   gtagttttaa ttttttgggt ttaggtaatt tttttatttt agttttttag gtagttggga 16200
   ttataggtgt atattattat gtttggttag tttttttttg tatttttttt gtagagacgg 16260
   ggttttatta tgttgtttag gttggttttg aatttttgag tttatgtgat ttatttattt 16320
   cgttttcgta aaatgttgga attatacgtt tgagttattg tatttggttt attttgtgtt 16380
   tattgtttaa taggtattta ttttttatat ttgttgttta taattgtttt atgttatttt 16440
   tttgttttaa ggtgtttttt tttttttttt ttgtgtaatt aaaattagaa tgttatttaa 16500
   gttttttttt gttaaggatt gaatgtttgt gtttttttta tttttaaatt aatatattta 16560
   aattttaatt tttaaggtgg tggtattaga gaagggggtg gggggttttg ggaggtgatt 16620
   aggttaggag ggtagattat ttatgaatgg gattaatgtt tttataaaag aaattttagg 16680
   gagttttttt tttatttttt tattatgtga ggatatagta agaaaatggt tatttatgaa 16740
   ttaggaagag agttttttta ttaattattg aatttgttag tgttttgatt ttggattttt 16800
   tagtatttag aattgagaaa aatagatatt gtttaagtta tttagtcgat ggtattattt 16860
   tttttttata gtagtttggt tagattaaga tatttttttt ttataaggtt tttttaattt 16920
   ttgtaaattt tattgattta tttttttttg ggttgaagtt ttaagtgttt attgttttta 16980
   ttttgttttt ttgtatttga tgttttagtg ttttataata ttagtttggt tttttttagt 17040
   tagaaaatat attttttgag ggtagagaga ttttatttta taggtaattt cgattatttt 17100
   tattttagga aagggtataa atatatttta taaatatttg ttgtattttt taatttattt 17160
   tgttttgtaa gagaaatatt aattaacgtt attttaaatt tttagttgtt atgacgagtt 17220
   ttaaagtatt aatttgtttt tatatgtgaa aagtatattt tgatttattt gtatattttg 17280
   attaaattag gttaattata tatttagttt attggggaaa tttgtgatgt tttaatagaa 17340
   aataagtttt gtttttattt gatattttga aaattttttt taggaataaa taaaattata 17400
   gtattaaatt ttaattatga tttgaaaata ttaattagcg tatatgttta ttgtagtttt 17460
   taatagtaaa aatattgtat atttttagag tttatttaat tataagagtt aaacgaatgg 17520
   ttaggtgatt tgtttcgttg gtggatatga gagaggagaa gttgattaaa tagttattat 17580
   tttttttaag ttgaattttg aatttaataa ataagagtat ttaaatattg tgttttgtat 17640
   atatgtaggg tatattatgt atatggggta taatatgaaa tttttgttat ttgaataaag 17700
   attaaaaaat gtggtattta tttatttttt ttatggaaat atattttttt ttttttttat 17760
   tatttttttt tttttttttt tttttttttt ttttgagata gggttttgtt ttgttgttta 17820
   ggttggagtg tagtggtgtg aatatggttt attgtagttt gtttttttgg gtttatgcga 17880
   tttttttatt ttagttttta gagtagttgt gttattatgt taggttaatt tttaaaaata 17940
   ttttgtaggt ttatttatgt tgtttagttt ggtttcgaat ttttgggttt agggaatttt 18000
   tttgttttat ttttttaaag tattgggatt ataagtatga gttattgtat taggttgttt 18060
   ttagttattt tatttttatt tggtttttta ttttttgttt tggttgtttt ttttttaaat 18120
   ttttttttga gttaataatt ggtatttaga agtatggttt tagttagtag ttttagggat 18180
   taagtggttg tttattattg aaagggttat tatgtttttt tagtattatt gttgatattt 18240
   tatgaaaaat atttggagaa aatgttttgt tttgattttt tttaatagaa ggaaaatgtt 18300
   tgtttataga ggaagtttgt agaaaaaaag aaagaaagtg tgttatttaa ttgtatattt 18360
   ataggtaaat ttagtaaata attttttttt tttttttttt tttttttttt tttttttttg 18420
   agagggagtt ttgttttgtt atttaggttg gagtgtagtg gtatagtttt agtttattgt 18480
   tattttcgtt ttttggattt aagcgatttt tttgttttag tttttcgagt agttgggatt 18540
   ataggtggtt gttattatat ttggttaatt tttgtatttt taatatagat agggttttat 18600
   tttgttggtt aggttggttt tgaatttttg attttaggta atttatttgt tttagttttt 18660
   taaagtgttg ggattatagg tatgagttat tgcgtttggt taatttagta aataaaatta 18720
   ttttagttta agattgtgtt tatatatgag atatatatgt attttaatat ttggatgaag 18780
   aaaggtatta taatttcggt taagtgttta gatgttttta tagtttgtgt agatttatgt 18840
   gttagttgtg tttgtgtttt gttgtagggg agtatatttt agaagagaag ttattttttg 18900
   tatgtaaagt tgtaaataat ttttgtatgt tgtttttaat gtattttttt gtttttttat 18960
   gttgggttga ggttttgttt ttttgttgtt taatagtagg tatagggttt ggttattgtt 19020
   gaataaaagt tttacgtatg tatgtttaat ttttaaataa attttgaatt ttaaaattga 19080
   ttttagtttt ataatttgtt tttaagtgag attttttgga ttttttattt tttttgtttt 19140
   tgatattttt agaggtataa tttggatagg gttaaagtta atttatagtt agtgtaatta 19200
   gtatatttaa gaataattaa aatatagtat aatatatggt atagagaggt ttttaaaaat 19260
   attttgtatt aagttattaa aaaattaatt atataaaaaa gtaaggttaa ataatttttt 19320
   tttttattag tgggatatag atttttaatt ttttggtgtt aaattttata ttgaatgatt 19380
   ttttttgtga tgtttggtat tttagtgagg ttatagaata gaaaagaaaa aaaaattaat 19440
   attattggaa agtatatgtt atagaatgaa ttaattaaat attaatttta ttagtgaaaa 19500
   aaaaaggagt aagtgtaaga ttaattaata ttagtgggat tatagtaaat taagagttat 19560
   ggtaaatttt ataatttatg attaaaattt attgtaaggt gtatatacgt tgtattttga 19620
   attagtaaat taaatatttt tgattagtgc gtttcgagtt tatttagaaa gtttttgagt 19680
   tttagaattg gttttgtgag ttaatgttgt ttaatttttt tttttggata tttttttatt 19740
   gttttttttt atttttttga gttttatatt tttaataatt attagtaaga aggaaacgtt 19800
   tataaataaa aataaataaa tatttgaaat aaggaaatta tttaattttg aatttatgta 19860
   ttgaaagatt ttaaaatata ttgtatataa ttaaatgttg ttagtaaatt agagatagga 19920
   ggttttatta aattttatag gtatattaat tgagaaataa aatagtttaa gggtttattt 19980
   aatgttattt agtgagggaa ttaaaattta gatttattag tttatatttt aaagaatttt 20040
   ttttattttt tgtttttttt taaaaaaatt ataagtatgt ttaaaaatag aatggaataa 20100
   tgaattagag tgttttattt gttatatatt tatgagaagt gtaattgttt tttattattt 20160
   ttagaaattt gtttatagta tattaagtat attagagaat tatattagtg aaaattaaat 20220
   tgattttaaa atttttatat atataaaaag atgtagttat gtttatattg taatggtatt 20280
   agcgtttttt tagtgaaggg gattgaaaag tggggatgat tttattttat ttgatttttt 20340
   tgatttgtaa tagttttgag ttgttttatt agtttatttt attgtattta attttaaatt 20400
   atttaatttt attaaagtta tttaaaatgg aaatgtaaaa ttttattttg ggattgtaat 20460
   tggaaatatt gttatttata tttttgagta ggtaatattt tgatgaaatt attatttttt 20520
   ttatttattt gatttgggtt taattttaaa ggatttatag ataaaagttt ttttggtttt 20580
   tgggtaattt tggttgtttg agaattttat gtttggtttt ttaattatat agaattatat 20640
   ttgttgtatg taaggttaat gattattaat agggaggttt taaaaaaatt atttaaaaat 20700
   gtaatttttt taaaattaat gaaaagtaat agttattgat aaggttgata gattaaagaa 20760
   aggttaaatt taattaaaat gatgaattaa aatattaatt ggaataaagt attgttgtta 20820
   aattgaattt tttaaaggga ttaattaaat ataggtttat atttttgttt agagaaaggt 20880
   tatagagatt ttttggttaa ttatatttat ggttgttacg ggagtgaaaa atttttataa 20940
   aaattaaaaa ttgtatatcg atttattagg tagtttgaaa agatatttcg tttttttttt 21000
   gttatagtta tataagtata gaaataaaat attgatattt tttaataata atggttgaat 21060
   taaatattgg attggttaaa gggatatagg gttatataaa ttttattaga tggttttttg 21120
   tgtagattac gattatagta tttatattgt ttttttaggt aatttgtttt ttttttttga 21180
   ttggaagtaa tgtgagggta gggattcgta ttgattttta tttttgaatt tttagagttt 21240
   aggaaagtat ttattatata gtaggtattt aaagatattt gtggagtaaa taagtaaata 21300
   aataaatgaa tgttattatg tagataacgg ttgttatatg ggggaaaata tttttattaa 21360
   tgagtaaaga atattggata aaatatttgg ttgttattat tttaaagtag ttaattataa 21420
   gcgtttataa tttttttggt ttatttgaat ttttgatatt ttgttaaata aatggatttt 21480
   taggatttga ttataagtaa atatattaat attaaaatat aggttatttg ggttttagtt 21540
   tgggattttt ttaaatgtgt attttaattt gaggattata agttaatttt ttttaaaagt 21600
   aatggtagtt gacggtgtat taagttaagt tttatatagt ttaaattttt taaatattta 21660
   ttggtgttta aaatattttt acgtgttaag taaaaaataa aaaataaata aataaataaa 21720
   aaaaaaattt ataacgatga agtttttgtg atttaagatt ttaagataaa gttaataaag 21780
   tatagttttt atttgaaatt tgtttatttt gataatatat ttttattttt tttatgtttt 21840
   atttgttttt tatttaatat aggataataa gattgtataa ttttttatta tatatttttt 21900
   agaataatgt gagaatgaaa ttgtgatatg gaaaaattaa tgtggaataa gtttttttgg 21960
   gaatttgtat taaattaatt attgtgaggt agggattagt aatattttaa tagtattatt 22020
   acgtgttatt attaatataa ttattgatcg tattgttttg agttttaggt attgtggaat 22080
   ttttatatga atatcgattt tgattgtatt tttatttatt tttttttagt atttatattt 22140
   ttattttaat attaaataat agtagatatt atggttttta tttgtttggt tattattttt 22200
   aaggttttga tggtttgtta gggtttatta ggttaataag tatggggaaa ataaattttt 22260
   tttaaagtta ggaatttgtt tttaaatttt atttaaatta gaggaagatg aaagagagaa 22320
   atttagtttt taataaatat atagagatta ttaatatata tgaaatgaag agaatatagt 22380
   tttgtgaagg atttagtttg tttaaatata tttattaata gattgtttgt gggtatgggt 22440
   atgtatggtt gttgaaatgt taattgaaaa ttttttaaaa aaattatgaa tgttagttgt 22500
   taaatgtagt gtaggaaatt aaagttttta aaagtaaatt ttagattgtg aaatttggat 22560
   ttttttatta aatttataga aaatatttta aatattaaat tattttataa ttttgtatga 22620
   attattgtta ttttttgaaa agttattgta tatgttttat tttttatagt atatttataa 22680
   aaatttttat ttaaatagag tttagtagaa gtagatttat gaagattgat atttatttag 22740
   tagataaacg aaatttaggg ttggggtaaa gtttgatgtt tgtttaaatg tgttattttg 22800
   tattaaatat atgtatataa taatttgaat tttaatatta ttaatagtcg attttaaggt 22860
   aaaaattttt aaatgtagga agttaaaaat aaattaaaat taatgtgtgt aaattataat 22920
   gtattaattt taataagcga ttttaaagtt tgttttaata ttggtgaatg gaaagtaggt 22980
   tttatatttt gtttttgaat gggttatgtt atttaaaaaa tatatagtta ttgttatatg 23040
   aaagtttttt tattttattt aattttaggt tttacggggg tttgttttta aaatggggtt 23100
   atatataaga taaaataaaa taaaataata atttttttaa attaagtttt tgatagtgtt 23160
   attgagatgt gatattgttt ttaaattttt tttttgagtt ttgtttgttt tggtagagag 23220
   gaatgggaat tttttaagtg tttattggtc gtataagata gatttagtaa aggtttttaa 23280
   tttattttag tagttgaaac ggtcggaggt gatttgtatt tattggagta tatatagatt 23340
   ttttatttgt ggtttttagg tatgggagaa attgggttat ttttaaattt atttttagat 23400
   atatagagtt gttaaatatt atttgatttg ggattaagga atgtttttaa aattgttttt 23460
   ttatatttat gaatgtgttg tttttagtgg ttattaattt ttatatatat taaataaata 23520
   aaagtaattg attatttaga aatgtttggg aaatggtaag atattataga attaattgaa 23580
   tagaattata aatttttttt tgtgtgagtg tatgtaaatt tattagaaat tttttggtag 23640
   taatgttttt tattttaggt tttaattttg tgaaatatta tattagggag gaggtagttt 23700
   ttggaaaata ttattgtttt tgtgatttta gagtgtaata ggaaaattta tatttaataa 23760
   ttttattata ttagatattt agggtaaatt tgtaaaagat aattttatga atttttaaag 23820
   gatataaggt tttaatatgt ggaagtgata ataataaaaa tatttaaaat gtgttaataa 23880
   ataggtaatg ttataatgta taaaatgata gtattgtgta ttatatgtag atttgtggtt 23940
   attaattaat gatatgttat aaaattatta ttaataattt tttagtatta ttaattaaaa 24000
   tcgaatattg attaatattt atattatatt tatgaaataa tttggaaata tttgatgata 24060
   tataaagttt tttttttatg atatataaaa tattttattt tattaaagta ggtatttgtt 24120
   ttttgttatt aaaaatattg agaagttttt ttatgagaaa ataaaaataa aggtttggag 24180
   atttttttta ttgtattttg tcgttatata agattttttt aagattttat tttttttatt 24240
   tttgtttttt atattatttt tttttttttt tttatttttt agtttgtttt ttttgtttta 24300
   tttgataggt aaatttgtag atattgtttt aggataataa tttcgtttga tagttaatta 24360
   atcgtgaata gttaaagtta aggtagtttg tattatataa atggaaaatt agattttttt 24420
   tttttaagaa ataaaatttt ttttttaaga tattgtaata gataatttag tattaaaatt 24480
   tttaattacg ttttttttaa attttttgga gatatttagt aattagtaaa aggtggtttt 24540
   atttaatttg tataatgtaa tttttgtaaa tgtattatat attttgtgta gagattatta 24600
   ttatggagat aatataaatg ttggtataga tgttataaaa tatttttaat gtttttttga 24660
   tagatattaa aagtaataag ttgtgatttt ttttaatgga aggtttttaa gagaatattt 24720
   taattattgt tattttatgt ttttttagtt tgttttattg tgtaatttga tatatttttg 24780
   aatttttttt tgaaatatgt ttagtaatta ataattttat tgttattaat gttattgttt 24840
   ttttatttgt gtttttttta gttatattta ttaggaattg ttttttattt ttatttttgt 24900
   attaaatatt tagttttttg attatgaatt ttagtttttt tttttgttat ttgattattt 24960
   atttttttta tttgttttta gtttatttga tttttttttt taaaaatttt ttttagaagt 25020
   attagagttt gtgatgaaaa agttatgttt tttgttttta tttagaatta aaataaaata 25080
   taaaatttgt aagtgattta attgataaga ggtatataaa gatttaattt tttttttttt 25140
   gtagggagga tgttatttat tatatttaag tttgagagtt attaatttta aggagaaata 25200
   attagaaggt tttaggtatt ttttaaaaat gtggtaatta tttagagagg ataaagatat 25260
   ttaagaattt tagtatatat ttatgtaatt gtgtatgttt agagggtaga tattatttaa 25320
   atgtaaagaa gtatgttgag attgtgagaa atggtattgt tatttgggaa attgttatcg 25380
   ttggtaaatt atagaaatgt tgatttttga aatataaatt ttgatattta aaaataaaat 25440
   taaaaaagag tttataaatt aatggttaaa aatagaatgg tggtagtggg taggtgagga 25500
   ggttgggtat aagttttata gaaatggtta aatgtaggta agaaaagtgg tagttgattt 25560
   tttttttttt ttttatttga aaagttgttg ttattttttg tagatataat tatttggtta 25620
   ttttttttag ttttttgatt ttgtgtggaa aagaattaga tatataaatt aatgttcgga 25680
   ttaaattata tgttaatgtt aatagttagt aggaagaagt attaataaaa gtgattttat 25740
   ggagttttag gagatattat tttttgacga ttattttttg aatatttttt tatttaaata 25800
   tatgtgtatg attacgtata tttatattat tattttttaa gaagtaatat agattaatta 25860
   gatatatgtt atgttattta tagaagtata ggtatatgtt tttgtaaata aatatattat 25920
   gttatttgtt aaatttatat tttattagga tagatttatt tcgttttaaa gttttttaga 25980
   gtgttttttg tgtatacgtg agtgaatatt gaatgtttag ttatttaaat ttaattttgg 26040
   ttttttaggg gttttaggta gtatttttta gttggtattt tttcgagaag agggtttata 26100
   ttgaagattt tgttttttgt tttattggat ataatagttg tgtttatttt ggttaaaatt 26160
   gattaaagta gttaaaattg tatttaattt ttgaagttaa tgtatttttt atttttggtt 26220
   atggtggagg ggaggtattg aattatagag aagaaaaggg gtagggtgat ttaggttgtt 26280
   tcggtgttgt ggttttgttt tttttagggg aataaatgtg tttagttgga gggtaaaggg 26340
   gattgttgga ttagaggaag gattaggtta attaaagggt tttttttaat ttttataagt 26400
   agttagttga aatataagag ggaaaggcgg ttttaaagtt tttgaaaggc gttattaaga 26460
   aagggattag aaattatatt tttacggagg attattggta ggaaagaagt ttttttaaat 26520
   atattgaata ttttattttt gtggggaaat ttggtgattt attttttttg cgtatatgta 26580
   atcgatttag tttaaaggtg tatttaaata ataataatta gtttgaagga gtttaaaggg 26640
   gatatttata tttttgtagg agggttgttt ttttagtgat ggatggtgaa ttgggttgtt 26700
   cgggttgttg ttgtcgtcgt tattcgtttt ttggggggtt aggggcggcg gcgggggcgg 26760
   gggtaggttc gagtcggggt tttcgagttt ttcgtttttt tgtagtagtt tggaggagtt 26820
   taggtcgagc gggaacgcgt tcgcgacggt cggcgcgcgg tttcggttgt tttttttttt 26880
   cgacggtttt cggttttgga ggaatcggtc gttttcgatt aaggggtcgt cgagtaggat 26940
   tttcgttttt tcgttttttt tttcgtcgtc gttagggttt cgattttttt tttttttttc 27000
   gttttcgggg ggtttgtggt tttttacgtt tattttttgt tttttttttt cgtcgttttc 27060
   gttttcggag ttgttttcgt tggggtagta gtagttggtt ttttcgggag atagaaggtt 27120
   tcggtggtgc gggtgttggg ttagaggggg cggcgggggc ggcggcggcg gcggcggcgg 27180
   ggtcgggtgg tggcgttcgg aattcggttc gtcgggttgc gggggtagta gtagaagcgg 27240
   cgacggcggt tgcggggggt ggtggtggtg gtggtggtgg tgagggtggt gatggtggtg 27300
   gggatgcgtt tgggtcgagt ggggcgcgtt cgtcgacgtt tcgtgtagtt tggttaggtt 27360
   tttcgcgtcg tttttgtcgt ttattggtcg gaaggcgttg gaatggtggt agttgttatc 27420
   gttcgttttg cgttttttta gaaggtgcgg atggtggggc gtcggaattc gggagttcgc 27480
   ggggttagat tcggcggtcg cggtttttgt ttcggtagtt gttatagagt tcgcgggcgg 27540
   cgtggagttc gcgttgtagt tttcgtcgtt gtcgttcggg ggcgattcga agagcgcgtc 27600
   gcgagagtcg gcgttttcgg taggaggagg tgggtcggag ttcgggtcgt tggttattat 27660
   ttgcgggggt tgtttcggcg ggggcgcggt ttcggcgttt ttagtagtat cgtttggttt 27720
   agttaggttt aggaaggttt ggcgtagtag ggtcgggttt tcgtttagcg cgtagtttag 27780
   cgtcgagggc ggttgaggcg ggggttgtag gtaggtgggt atcgggagtt cgttaggggt 27840
   tcgcggcggt tagaatatgt agaagggcgg atagaaggcg tttttgcggt tcgcgggtta 27900
   gaagaggtcg gataagtcgg ttttgggcgt cgcgttcgcg tcgtttgcgt tcgcgttttt 27960
   tagggtttta gcggcggtat tcgtattttt ttttttatgg tataagttga aggcggcggt 28020
   cgcggtaggg aaggtgtagg ggtgcgggaa gagttcgtcg tagttcggga atttttgtag 28080
   gacgttttcg aacgagtttt tgttgggtat cgggatgatt gggtagttgc gcgggttttt 28140
   ggtttttgtt tcggtattcg ttttcgcgtt cgcgtttacg tttacgtcgg ttatatagtt 28200
   atttttagcg tcgtttttat tagttttcgc gtcgttcgaa gtagtagtta tagagaggtt 28260
   ggcggttgcg gtcgagaggt tggcggcggt tattacggcg gttttttgta aggagtcgtc 28320
   gtcgtcgtcg aagcgcggtc gtttgtgatg ggcgtgcggg gtattagtta gttttgttaa 28380
   gggcggcggt ggcggcggcg gcggcggggg cgtatttagt aggtgggggt ttagtagatt 28440
   ttcgttttcg gttatcgcgg tcgcggcggt tacggcggtc gtttttatag agttgagcgg 28500
   gtggtaggcg gggtgcgcgt tcggttgggg tagtgcgcgt ttgcggttgt cgtcgttgaa 28560
   tatggttttg acgttttttt aggcgaagat tagttcgttt tggggatttt tgtcggtgag 28620
   tttgagatga cggcgttacg agttgaagtt ggttgcgttt ggttgagtgt atttggcgtc 28680
   gggcgtgcgg tgggagtgga aaatgaattt gttgggcgag aagtatatgt tgtagtagtt 28740
   gtatttgatg tatttggcgc gcgagttgtt gtagcgcgcg ggaatgaagt tgtcgcggta 28800
   gttttaggcg tattcgtgtg atacgtcgaa ggcgaaattg tttggtagtt tgggcggttt 28860
   gttttcgttt aggaacgatt tgtatagacg ttcggtttcg cgtttggtga ttatgtcgta 28920
   gcggcgcgat gagatgggta tggtttcggt acgtcgtagg atttttagtt gtatcggcgt 28980
   gtattgtata tacgtgatgt ttagtgttac gcgacggttg tggatttcgt tgtagttgaa 29040
   gtttttgagt agagtgttgg agatttgcgt taggtatagg cgtttttgtt cgtcgattat 29100
   taacgatacg atgggaatgt cgtagaggat tatttggttt atttggttgg gtttgaggtt 29160
   ggcgtgtttt ggtcgcggtt ggtttagcgt gtcgggttgg aaggcgttcg acggcgacgt 29220
   tagtaggatg tcgttgggtt ttggtagcgg attggaagtt at 29262
<210> 102
   <211> 29262
   <212> DNA
   <213> Artificial Sequence
<220>
   <223> chemically treated genomic DNA (Homo sapiens)
<400> 102
   atggttttta gttcgttgtt agggtttaac gatattttgt tggcgtcgtc gtcgagcgtt   60
   ttttagttcg atacgttgag ttagtcgcgg ttagggtacg ttaattttaa atttaattag  120
   gtgggttagg tgatttttta cggtattttt atcgtgtcgt tggtgatcga cgggtaagag  180
   cgtttgtgtt tggcgtagat ttttaatatt ttgtttaaga attttagtta taacgagatt  240
   tataatcgtc gcgtggtatt gggtattacg tgtgtgtagt gtacgtcggt gtaattggag  300
   attttgcggc gtgtcggggt tatgtttatt ttatcgcgtc gttgcggtat gattattaaa  360
   cgcgaggtcg agcgtttgtg taagtcgttt ttgggcgaaa ataggtcgtt taagttgtta  420
   gataatttcg ttttcgacgt gttatacgag tgcgtttggg gttgtcgcgg tagttttatt  480
   ttcgcgcgtt ataatagttc gcgcgttaag tgtattaaat gtagttattg taatatgtat  540
   ttttcgttta ataagtttat tttttatttt tatcgtacgt tcgacgttaa gtatatttag  600
   ttagacgtag ttaattttaa ttcgtggcgt cgttatttta agtttatcga taagagtttt  660
   taggacgagt tggttttcgt ttgggaggac gttaaggtta tgtttaacgg cggtagtcgt  720
   aagcgcgtat tgttttagtc gggcgcgtat ttcgtttgtt attcgtttag ttttgtgaag  780
   gcggtcgtcg tggtcgtcgc ggtcgcggtg gtcggaggcg ggggtttgtt gggtttttat  840
   ttgttgggtg cgttttcgtc gtcgtcgtcg ttatcgtcgt ttttggtaga gttggttggt  900
   gtttcgtacg tttattataa gcggtcgcgt ttcgacgacg acgacgattt tttgtaggag  960
   gtcgtcgtag tggtcgtcgt tagtttttcg gtcgtagtcg ttagtttttt tgtggttgtt 1020
   gtttcgggcg gcgcggggat tggtgggggc ggcgttgggg gtggttgtgt ggtcggcgtg 1080
   ggcgtgggcg cgggcgcggg ggcgggtgtc ggggtagggg ttaaaggttc gcgtagttat 1140
   ttagttattt cggtgtttag taagggttcg ttcgggggcg ttttgtagaa gttttcgggt 1200
   tgcggcgggt ttttttcgta tttttatatt ttttttgtcg cggtcgtcgt ttttagtttg 1260
   tgttataaga aagaggatgc gggtgtcgtc gttgaggttt tggggggcgc gggcgtaggc 1320
   ggcgcgggcg cggcgtttaa ggtcggtttg ttcggttttt tttggttcgc gggtcgtaag 1380
   gacgtttttt attcgttttt ttgtatgttt tggtcgtcgc ggatttttgg cgggttttcg 1440
   gtgtttattt atttgtagtt ttcgttttag tcgttttcgg cgttaggttg cgcgttaggc 1500
   gaaagttcgg ttttgttgcg ttaggttttt ttggatttgg ttgagttagg cggtgttgtt 1560
   gggagcgtcg aggtcgcgtt ttcgtcgggg tagttttcgt aggtagtggt taacggttcg 1620
   ggtttcggtt tatttttttt tgtcgggggc gtcggttttc gcgacgcgtt tttcgagtcg 1680
   ttttcgggcg gtagcggcgg ggattgtagc gcgggtttta cgtcgttcgc ggattttgtg 1740
   gtagttgtcg gggtaggggt cgcggtcgtc gggtttggtt tcgcgggttt tcgggtttcg 1800
   gcgttttatt attcgtattt tttggagggg cgtaaagcgg gcggtggtag ttattattat 1860
   tttagcgttt ttcggttagt gggcggtaag gacgacgcgg agagtttggt taagttgtac 1920
   ggggcgtcgg cgggcgcgtt ttattcggtt tagacgtatt tttattatta ttattatttt 1980
   tattattatt attattatta ttatttttcg tagtcgtcgt cgtcgttttt gttgttgttt 2040
   tcgtagttcg acgagtcggg tttcgagcgt tattattcgg tttcgtcgtc gtcgtcgtcg 2100
   tcgttttcgt cgtttttttt ggtttagtat tcgtattatc gaggtttttt gtttttcggg 2160
   ggaattagtt gttgttattt tagcgaggat agtttcgagg acgaggacga cgaggaagaa 2220
   gagtaggagg tggacgtgga gggttataag tttttcgagg gcgaggaaga ggaggaaggt 2280
   cgagattttg acgacgacga ggaagaggac gaggagacgg aggttttatt cggcgatttt 2340
   ttagtcgggg gcggtcggtt tttttagggt cgagggtcgt cggagaaggg gagtagtcgg 2400
   gatcgcgcgt cggtcgtcgc gggcgcgttt tcgttcggtt tgaatttttt taggttgttg 2460
   taggaagacg ggaaattcgg ggatttcggt tcggatttgt tttcgttttc gtcgtcgttt 2520
   ttggtttttt agaaggcgag tggcggcggt agtagtagtt cgggtagttt agtttattat 2580
   ttattattgg aggagtagtt tttttataaa gatgtaaata ttttttttag gtttttttaa 2640
   gttaattatt attatttaaa tgtattttta ggttgaatcg gttatatatg cgtaggaaag 2700
   atgaattatt agattttttt atagaaatga aatatttagt gtgtttagga gggttttttt 2760
   tttgttagtg gtttttcgta aaaatgtggt ttttggtttt ttttttgata acgtttttta 2820
   aaggttttgg ggtcgttttt tttttttata ttttagttgg ttgtttgtgg aagttggaaa 2880
   agatttttta attgatttgg tttttttttt aatttaataa ttttttttgt tttttaattg 2940
   agtatatttg tttttttaaa aggagtaaga ttataatatc ggagtagttt gaattatttt 3000
   attttttttt ttttttatag tttagtattt tttttttatt atgattaaaa gtaaaaaatg 3060
   tattggtttt aaaagttaaa tgtagtttta attgttttaa ttagttttag ttagaatgga 3120
   tataattgtt gtgtttagtg aaataaaaga taggattttt agtatagatt tttttttcgg 3180
   aaaagtatta attgaaaagt attatttggg atttttaaaa gattaaagtt aaatttaaat 3240
   gattgaatat ttagtgttta tttacgtgtg tatagaaaat attttggagg attttagggc 3300
   gaagtgaatt tgttttaatg aagtgtagat ttgatagata atatggtgtg tttatttata 3360
   gaagtatatg tttatgtttt tgtaaataat atagtatata tttgattaat ttgtattatt 3420
   ttttaaagga taatagtgta gatatacgta gttatatata tgtatttaag taaaaaagta 3480
   tttaaagaat aatcgttaaa aaatgatgtt ttttgaggtt ttatggagtt atttttattg 3540
   atgttttttt ttgttgattg ttgatattag tatatggttt gattcgggta ttgatttgta 3600
   tgtttaattt ttttttatat agagttagaa aattaaggaa aataattaag taattgtatt 3660
   tataaaggat gataatagtt ttttaggtaa aaaaaaaaaa aaaaattagt tgttattttt 3720
   tttgtttgta tttaattatt tttgtgagat ttatgtttag tttttttatt tatttattat 3780
   tattattttg tttttaatta ttgatttgtg ggtttttttt tggttttgtt tttgaatatt 3840
   agagtttata ttttaaagat taatattttt atagtttgtt aacgatgata gttttttaaa 3900
   taatagtgtt attttttata attttagtat atttttttgt atttgaatga tgtttgtttt 3960
   ttaaatatat atagttatat aaatatatgt taaaattttt agatattttt gttttttttg 4020
   aatagttgtt atatttttgg agaatattta gagttttttg attgtttttt tttggagtta 4080
   atagttttta aatttagata taataagtag tatttttttt gtaaaaaaaa aaaaattgga 4140
   tttttgtgtg ttttttgtta gttggattat ttgtagattt tgtgttttgt tttggtttta 4200
   gataagaata aggagtatag tttttttatt atagattttg atgtttttgg aggagatttt 4260
   tggagagaaa gattaggtag gttgggaata aataagagga atgaatagtt agatgatagg 4320
   gagaggggtt gaaatttata attaaaagat tgaatattta gtataagggt aaaaataaaa 4380
   aataattttt agtagatata attaagaaaa atataaatgg ggaagtagtg atattaatag 4440
   taataagatt attaattgtt gggtatattt tagaagaaaa tttagaaata tattaggtta 4500
   tataatgaaa taggttggaa aagtataaaa tgataataat tgaaatgttt ttttagaaat 4560
   tttttattaa aaaggattat agtttattgt ttttaatatt tgttagaaag atattaaagg 4620
   tgttttatga tatttatgtt aatatttata ttatttttat gataatgatt tttatataaa 4680
   atgtataata tatttataaa aattatatta tataaattaa atgaaattat tttttattga 4740
   ttgttaaatg tttttaaggg gtttgggaaa gacgtgatta gaagttttga tattaagttg 4800
   tttattgtag tgttttaagg agagggtttt gttttttggg aaaaaggaat ttaatttttt 4860
   atttatgtaa tgtaaattgt tttggttttg attatttacg gttaattgat tgttaaacga 4920
   ggttgttatt ttgaggtaat gtttgtaaat ttgtttgtta aatgagatag agagagtaag 4980
   ttaggaagtg agggagagag agagagtggt gtagagagta gaaataaaga aaataggatt 5040
   ttaaaggaat tttatataac ggtaaagtat aatgaaaaag gtttttaaat ttttgttttt 5100
   atttttttat aaggaaattt tttaatattt ttaatggtaa aaggtaaata tttattttaa 5160
   tgaaataaaa tgttttatgt attatgggaa gaaagtttta tatattatta gatattttta 5220
   aattatttta tgaatatggt ataagtgttg attaatattc gattttggtt gatgatgttg 5280
   aggagttatt aataataatt ttgtagtata ttattggtta gtggttataa gtttatatat 5340
   aatatataat attattattt tgtgtattat ggtattattt gtttgttagt atattttgaa 5400
   tgtttttatt attattattt ttatatatta aagttttata ttttttgaaa atttataaaa 5460
   ttattttttg taaatttgtt ttaagtgttt aatgtggtaa aattattaaa tgtagatttt    5520
   tttgttatat tttaaagtta taaaggtaat gatatttttt aggaattatt ttttttttag    5580
   tatgatattt tataaaattg aggtttgaaa tggaaagtat tgttattaag gaatttttaa    5640
   tggatttgta tgtatttata taaaaaaggg tttataattt tatttaattg attttgtaat    5700
   attttgttat tttttagata tttttagata gttagttgtt tttgtttgtt tgatatgtgt    5760
   agaaattaat agttattgga aataatatat ttatgaatat gaaaaaataa ttttagaaat    5820
   attttttagt tttagattaa atgatattta gtagttttgt atatttaaaa atagatttaa    5880
   aagtagttta atttttttta tgtttggaaa ttatagatgg gaagtttata tgtattttag    5940
   tgaatgtaaa ttattttcgg tcgttttagt tgttgaagtg gattggagat ttttgttagg    6000
   tttgttttgt acgattaatg agtatttaga gaatttttat ttttttttat taagatagat    6060
   aaagtttaga aagaaagttt aaaagtaatg ttatatttta gtggtattgt taagaattta    6120
   atttggaagg attattattt tgttttgttt tattttatgt atgattttat tttgaaagta    6180
   agttttcgta aagtttaaag ttgaataaaa taagaaaatt tttatgtagt aatggttgtg    6240
   tattttttaa ataatatgat ttatttagag gtaggatgtg aaatttgttt tttatttatt    6300
   agtattaaga taagttttag agtcgtttgt taaaattagt gtattgtaat ttatatatat    6360
   tagttttaat ttgtttttga ttttttatat ttggagattt ttattttgga gtcggttatt    6420
   ggtaatatta aagtttaaat tattatatat atgtatttaa tataaaataa tatatttaga    6480
   tagatattaa attttatttt aattttgaat ttcgtttatt tattagataa atattaattt    6540
   ttataagttt atttttgttg aattttattt gggtaaaaat ttttgtaaat atgttataaa    6600
   agatggaata tgtgtaatag ttttttaagg ggtaataatg atttatataa aattatggga    6660
   taatttaatg tttaaaatat tttttataaa tttaatgaga aaatttaaat tttatagttt    6720
   gaaatttatt tttaggaatt ttaatttttt atattgtatt taatagttaa tatttataat    6780
   ttttttaaga agtttttagt taatatttta ataattatgt atatttatat ttataagtaa    6840
   tttattaata aatatatttg aataaattaa gttttttata gagttatatt ttttttattt    6900
   tatgtatatt aatagttttt gtatatttgt taaaaattga attttttttt tttatttttt    6960
   tttgatttgg atggaatttg aaaataaatt tttggtttta agaggggttt gtttttttta    7020
   tgtttattga tttagtaaat tttaataagt tattagaatt ttaagaatga tagttaagta    7080
   gataaaaatt atggtattta ttgttgtttg atattaagat ggaaatataa atgttaaaag    7140
   gaagtaagta aaagtatagt taggatcggt gtttatataa aaattttata gtgtttggag    7200
   tttagggtag tgcggttaat agttatattg gtaataatac gtagtgatat tattgaaata    7260
   ttgttagttt ttattttata ataattaatt tgatatagat ttttagagaa atttgtttta    7320
   tattgatttt tttatattat agttttattt ttatattatt ttgggaagta tgtagtagga    7380
   ggttatataa ttttattatt ttgtattgaa tgggagatag gtgaaatatg gaagggataa    7440
   aaatatatta ttaaaataag taaattttag gtgaaggtta tattttgtta attttatttt    7500
   ggaattttgg gttataagaa ttttatcgtt atgggttttt tttttgtttg tttgtttgtt    7560
   ttttgttttt tgtttagtac gtggaaatgt tttaaatatt agtaagtgtt taaagggttt    7620
   ggattgtgtg ggatttaatt tggtgtatcg ttagttgtta ttgtttttag aaaagattag    7680
   tttgtggttt ttagattgaa atatatattt gagaaaattt tagattgggg tttaagtgat    7740
   ttatgttttg gtattaatgt atttatttgt ggttagattt tgagaattta tttatttggt    7800
   aaagtgttag aaatttagat gagttaaaga gattatgagc gtttgtgatt agttgttttg    7860
   gagtggtagt agttaaatgt tttatttagt attttttatt tattaatgaa gatatttttt    7920
   tttatgtaat aatcgttatt tatatgatag tatttatttg tttatttatt tatttatttt    7980
   ataaatattt ttgaatgttt attgtataat aagtattttt ttagattttg ggaatttaga    8040
   aatagaaatt aatacgagtt tttgttttta tattgttttt aattaagagg aggagataga    8100
   ttatttaaga aggtagtatg agtattataa tcgtagttta tataaaaaat tatttgataa    8160
   gatttatata attttgtgtt tttttagtta atttaatgtt taatttaatt attattattg    8220
   gaaaatatta atattttatt tttatgttta tatagttata ataaaaaaag aacgaaatgt    8280
   ttttttaaat tgtttgataa gtcggtatgt aatttttgat ttttatagga attttttatt    8340
   ttcgtggtaa ttataaatat agttaattag aaaattttta tgattttttt ttgaataaag    8400
   atatagattt atatttaatt gattttttta aaaaatttag tttagtaata atattttgtt    8460
   ttagttgata ttttagttta ttattttaat taagtttagt ttttttttaa tttgttagtt    8520
   ttattagtaa ttattatttt ttattaattt tgaagagatt atatttttaa atgatttttt    8580
   taaaattttt ttgttaatgg ttattaattt tatatatagt agatataatt ttgtataatt    8640
   gagggattaa atatgaagtt tttaagtagt taaaattatt taaaagttag gagaattttt    8700
   atttatagat tttttaaaat tagatttaag ttagatagat aagggggatg gtagttttat    8760
   taaaatatta tttatttaaa aatataaatg atagtatttt taattgtaat tttagagtaa    8820
   ggttttgtat ttttattttg aataatttta gtgaagttaa gtgatttgaa attgaatgta    8880
   gtggggtaag ttggtaagat agtttaaagt tattgtaggt taaggaaatt aggtaaagtg    8940
   gagttatttt tattttttag ttttttttat tggagaaacg ttggtgttat tgtagtgtga    9000
   gtatggttgt atttttttat atgtatagaa gttttaaaat taatttgatt tttattggta    9060
   tgatttttta gtgtatttaa tatgttatgg atagattttt gagaatagta agaagtaatt    9120
   atatttttta tgaatatata gtagatgaag tattttgatt tattatttta ttttattttt    9180
   gaatatgttt atgatttttt taaaaaaaag taaaaaataa gaaaattttt ttgaagtata    9240
   gattagtaga tttgagtttt aattttttta ttaggtgata ttaggtaaat ttttaaatta    9300
   ttttgttttt tagttaatgt atttataaaa tttgataaaa ttttttgttt ttagtttatt    9360
   agtaatattt agttatgtat aatgtatttt gaagtttttt aatatatgga tttaaaatta 9420
   aatagttttt ttattttaga tatttgttta tttttattta tgaacgtttt ttttttatta 9480
   atgattgtta aaaatataga atttaaggag atagagaagg gtaatgaaaa aatgtttagg 9540
   aaaaaaaatt aaatagtatt aatttataag attagtttta aaatttaaga gttttttaaa 9600
   tgaattcgag acgtattaat taaagatatt taatttattg atttaagatg taacgtgtat 9660
   gtattttgta ataaattttg attatgaatt gtaaagtttg ttataatttt tagtttatta 9720
   taattttatt ggtattagtt aattttgtat ttgttttttt tttttttatt aatggagtta 9780
   atatttagtt agtttatttt gtagtatatg ttttttagtg atattgattt tttttttttt 9840
   tttgttttgt gattttattg aagtgttaaa tattataggg gaagttattt aatgtagaat 9900
   ttaatattaa gaaattagaa gtttatattt tattgataga ggagaaaatt atttaatttt 9960
   gtttttttat ataattgatt ttttaatgat ttaatgtaaa gtgtttttaa aaattttttt 10020
   atgttatatg ttgtgttgta ttttggttat ttttaagtgt attagttata ttaattgtag 10080
   gttaattttg attttattta aattatattt ttaagagtat tagagataga gaaaataaaa 10140
   aatttagaaa attttattta gaagtagatt ataagattaa gattaatttt aaaatttaga 10200
   gtttatttgg aagttaaata tgtatacgtg gagtttttgt ttaatagtgg ttaggttttg 10260
   tgtttgttgt tgagtaatag gaggatagag ttttagttta gtataaaagg gtaagaaggt 10320
   gtattgggag tagtatgtaa agattgtttg tagttttgta tgtaggagat ggtttttttt 10380
   ttagggtgtg tttttttgta gtagaatata gatatagtta gtatatgagt ttgtataaat 10440
   tatggaaata tttggatatt tgatcgaaat tataatattt ttttttattt agatgttaga 10500
   gtgtatgtgt attttatgta tagatatagt tttaaattaa aataatttta tttattaaat 10560
   tggttaagcg tagtggttta tgtttgtaat tttagtattt tgggaggttg aggtaggtgg 10620
   attgtttgag gttaggagtt taagattagt ttggttaata aggtaaaatt ttgtttatat 10680
   taaaaatata aaaattagtt aggtgtggtg gtagttattt gtaattttag ttattcggga 10740
   ggttgaggta ggagaatcgt ttgaatttag gagacgaaga taatagtgag ttgaaattgt 10800
   attattgtat tttagtttgg gtgatagagt aagatttttt tttaaaaaaa aaaaaaaaaa 10860
   aaagaaaaga aaagaaaaaa attatttatt aaatttattt gtgagtgtgt aattaaataa 10920
   tatatttttt tttttttttt ttgtagattt tttttgtgga tagatatttt ttttttatta 10980
   aaaaaaatta aaataaaata ttttttttaa atatttttta tgaagtgtta ataatagtat 11040
   tgggaggata taatagtttt tttagtgata agtaattatt tagtttttgg ggttgttggt 11100
   tggggttatg tttttaagtg ttagttgttg gtttaaggga gaatttaaaa gggagatagt 11160
   taagataagg agtaggaaat taagtaggga tgaaatgatt gggggtagtt tggtatagtg 11220
   gtttatgttt ataattttag tattttggaa gaatgaaata ggaggatttt ttgagtttag 11280
   gagttcgaga ttagattggg taatataggt agatttataa aatattttta aaaattagtt 11340
   tggtatagtg gtatagttat tttggaggtt gaagtgggag gatcgtatga gtttagaagg 11400
   ataggttgta gtaagttatg tttatattat tgtattttag tttgggtaat agagtaagat 11460
   tttgttttaa aaaaaaaaaa aaaagaaaaa gaaaagaaaa tggtggggag gagaaagaat 11520
   gtatttttat agaagggatg ggtgaatgtt atattttttg atttttattt aaatggtaaa 11580
   agttttatat tgtattttat atatatggta tattttatat gtgtatagga tataatattt 11640
   aaatgttttt atttattgaa tttagagttt agtttgagaa gagtgataat tgtttaatta 11700
   gttttttttt ttttatgttt attaacggaa taggttattt gattattcgt ttggttttta 11760
   tagttaaatg agttttaaaa atgtataata tttttgttgt tggaggttat agtagatata 11820
   tacgttaatt aatattttta ggttatggtt aaaatttagt gttgtggttt tgtttgtttt 11880
   tgaaagaaat ttttaaaatg ttagatgaga ataaaattta ttttttattg gagtattata 11940
   aattttttta gtaaattgaa tatataatta gtttaattta gttagaatat ataaatggat 12000
   taaaatgtgt tttttatata taggaataag ttagtgtttt aaaattcgtt atgataatta 12060
   aggatttaga atagcgttag ttggtatttt ttttataaag tagaatgaat taaaaagtat 12120
   aataagtatt tgtggaatgt atttgtattt ttttttaagg taggggtgat cgggattatt 12180
   tgtagggtag gatttttttg tttttagaaa gtatattttt tagttgagga agattagatt 12240
   aatattgtaa aatattagaa tattaggtat aaaggggtag aataaagata ataagtattt 12300
   agaattttag tttaggaagg gataaattag taagatttgt agaagttgaa aaagttttat 12360
   ggaagaaaaa tgttttagtt tggttaggtt gttataaaaa aaaaataata ttatcgattg 12420
   ggtggtttag ataatatttg ttttttttag ttttggatgt tgggaagttt aagattaagg 12480
   tattggtaga tttagtgatt ggtgagggag tttttttttt ggtttataga tggttatttt 12540
   tttgttgtgt ttttatatgg tggaaaagta ggaagggagt tttttggggt tttttttata 12600
   agggtattaa ttttatttat aagtggtttg tttttttggt ttaattattt tttaaagttt 12660
   tttatttttt tttttaatat tattattttg ggggttagga tttaaatatg ttaatttggg 12720
   gataaggggg atataggtat ttaattttta ataagaaaag atttggatgg tattttgatt 12780
   ttggttgtat aagaaagaga ggagagggta ttttaggata gagaaatgat ataaggtagt 12840
   tgtaggtagt agatgtgaaa aataaatgtt tgttaggtaa taaatatagg atgggttagg 12900
   tgtagtgatt taggcgtata attttagtat tttgcgaggg cgagatggat ggattatatg 12960
   agtttagggg tttaagatta gtttgggtaa tatggtgaaa tttcgttttt ataaaaaaaa 13020
   tatagaaaaa aattagttag atatgatggt gtgtatttgt agttttagtt atttgggagg 13080
   ttgaggtgag aggattattt gagtttagga ggttgaggtt gtagtgagtt tggattgtgt 13140
   gagtgcgttt tagtttgggt ggtagagcga gaatttgttt taaaaaggga gataggatgg 13200
   tttagtaggt tggataagaa tgggagaatt tagagatttt gtggggagaa ttttagaatg 13260
   gagtttttga tggagattat agggagttat tgtaggtttt tgagtagagg tttgttatga 13320
   tatttaagag aaagtaatta ttttagtatt ttgaaagtta gtgttattga atttgttggt 13380
   tgtttttttt tttttttttt tttgtttgtt tgtgttgttt atatagttta tttttttttt 13440
   atttgttatt tattgtttat atttgtttta gtatgttatt ttttttattt atttttttta 13500
   tatttttagt ttttaatgat atgtgagggt agaattaaga gtgggttgga atatttattt 13560
   agaatgtgga attattgttg ttggaggttt ttggagagat taggaaagtc gatttttgat 13620
   attttaggtt ttttggtttt tagttgtatt tagtagtgat gtagtttttt tgatatggta 13680
   cgatgttgtt tttatttttt tggatgagta ggatttttag aaattatgta gattataatg 13740
   ttttgtaaat gtgtatttgg aaagttttgg tgtggatatt tttttaagta gtagtttttt 13800
   tttttttttt ttttttttgt atttaagtta gggttttatt ttgttattta ggttggagtg 13860
   tagtggttag tggtgtaatg atgatttatt ttagttttga tttttagggt ttaagtgatt 13920
   tttttattta gattagtcgg aattagaaga gtgtattatt atttttagtt aatttttgtt 13980
   ttttttgtat agatagagtt tcgttatgtt gcgtagtttg gtattaaatt tttgggttta 14040
   agtatttttt ttcggttttt tagagttttg ggattatagg tgtgagttat ggtattgggt 14100
   ttttagtagt agtttttagt tatttttatt tataggagac gttttatgtt ttagttattc 14160
   gttatttttt aattttaaat tagttttttt ttatagtttt agatatgttt gaggttgggt 14220
   ttttaagaaa tatgtttttt ttatgtataa tttttttttt tataatttta tattgttaga 14280
   attgttgtgt ttttttttat tttttttttt tttttttttt tttgagatgg agttttgttt 14340
   tgttatttag attagaatgt agtggcgtga tttcggttta ttgtaatttt tgttttttgg 14400
   gtttaagcga tttttttatt ttagtttttt aagtagttgg gattataggt gtttgttatt 14460
   atgtttagtt agtttttgta tttttagtag agatggggtt ttattatgtt ggttaggttg 14520
   gttttgaatt tttgatttta ggtgatttat ttgttttagt tttttaaagt gttgggatta 14580
   tatatatgag ttattatatt tggtttatta attttttttt taaaagagga atttattgtt 14640
   tattttggta gtatatatat taaaattgga ataatataga gaagattagt atggtttttg 14700
   tgtaaggata atatataaat ttttgaagta gtttatattt aaaaataaaa ggggtgaggg 14760
   aaatataggg gataaaaaag taagtatttt agggatgttt ttaaatttat ttattaattt 14820
   tgaaaaagta aaatgtagtt aatttgaagg aatttttaga aaaattggga gtttattgtt 14880
   aattaaatta cgtttattta atttagtatt agattgtttt tttttttaat tagatggaaa 14940
   ttattagtag taatagtgaa taaatatatt taggttttta gaggttattt ttaattatta 15000
   ttaataattt ggtttttata gattagtaat atacgaaagg taatagttta gtaaaagata 15060
   tataaaaggt agcggaagtt tttgtgtttt ttatttattt aatatttagt tattttaaaa 15120
   attttttgta agtgaggtat tcgaattttt taagtttaaa attggtagat tttatttaga 15180
   agatgtagta aaatatttgt ttgaaataga tgttaatgta aattgttaat aaaattttta 15240
   taaagttgat gaaaagataa gttttttatt aatattatta gtagatttgt ttgtgtttaa 15300
   tatagttttt atttattagt tgtttgttta gtggttttat attaatgtaa agttattaat 15360
   tatgaagtag gaaagaaatg tattttatat attatatttt aatttttaaa tttaattatt 15420
   taattgaaat ataatataag ataaatagtt tataaaatat attaatgagt taaaaggaat 15480
   ttggtttata atttgataag attatggttt ttagaattgt ttatttagga ataaagattt 15540
   tatttaaatt atgttttgag tggttgagaa agttgtagaa tgatagtgtt attatgtttt 15600
   gtagataaat gtagagatat atgtatgttt tttttttttt ttgtttagat ttaatattgt 15660
   atgttagtat ttttgaaatt tttatatttt taattggtta attgagattg tatataatat 15720
   gtatgtattt ttattttaaa atattatttt ttttggttta tttattaata atttgttatt 15780
   aatttttatg ttatttttgt tttagataat agtgtttttt taggaattgt attgaggttt 15840
   atatttaaaa tgttttatag atattttttt tttatttttt ttttatatgg agaagaaagt 15900
   taaggtatta aggtttaaga gtatatttga gtttatagag aggttatgtt taagatgaga 15960
   atgagaatga ggttatagga atttgatttg gatattagag aagatgtttg ttattaagga 16020
   atttagggat gttaaggata ttattaggtt ttttttttta atgatgtata tgttttggaa 16080
   tatttgtgta tatttttata gatgtattta tttatatata tatatatatg tataaatata 16140
   tataaatata ttttttatag ttattaagta aaatgtattt gaattgttat tgataatatt 16200
   atgttgaaaa tttttttgtt tgtttatgat attgttatag gttagtattt tagggagaaa 16260
   ttttgttttt ttttttgtag aaatttttat tttagtaaag aaattttttc gtatttttat 16320
   aagtgtttag ggttattata ttttggtttt tgttgaggga aataaatagt aaggagattt 16380
   ttgatgaatt ttggttttcg tttattgtaa tttagaattt acgatttttt ggggtttttt 16440
   ttagaaataa ttgtagattt tttattgagt gtttattgta tgtttagcgt tgtgttaggt 16500
   attggttata tttaattttt gtcgttaagt aatatatggt ttattgattg aggaagggga 16560
   gtaaggaaat agtattatgt ttgaggtttg tgtagggaat tttggggata tagaggaggg 16620
   atggggtgaa agggaggggg atttatgagg ttttagagtt tttttgtttg ttttattata 16680
   tttttttaga tagaaatgaa aagtttgatt aaattatgtg agtattgagg atagggggtt 16740
   gaatttttta aaaattttga ttgtttgaat gattggattt tggtttattt agattagatt 16800
   tgtagattta atttatgatt gtggataaag atattttttt aatttgttat tttagttagt 16860
   atgtaagcga aataggaata acgatttaat ttttatgcgt aatatagttt tttttggtcg 16920
   aggaagagta gttaagagat attagtaagt aagttattgt ttataaatta atataagtta 16980
   atttattttg tttttgtttt aagttgattt ttaatttttt tttatttttt attttattta 17040
   tttatttgtt tttttgagat agggttattt aggttggagt gtagtagtgt aattatagtt 17100
   tattatagtt ttaatttttt gggtttaagt gattttttta ttttagtttt taagttgttg 17160
   gaattatagg tgtatattat tatatttagt taattttttt atttttgtag ggataaggtt 17220
   ttgttatgtt atttaggttg gtttcgaatt tttggtttta agtaattttt tcgttttagt 17280
   tttttaatta tagatgtgag ttattatatt tagttttaag ttgatttttt tttttttttt 17340
   gagatggagt ttcgtttttg ttgtttaggt tggagtgtaa aggtgtgatt ttagtttatc 17400
   gtaattttcg tttttcgggt ttaagtgatt tttttgtttt agtttttcga gtagttagga 17460
   ttataggtat gtattattac gtttagttaa ttttgtattt tttatagaga cggggttttt 17520
   ttatgttggt taggttggtt ttgaattttt gacgttaagt gatttgttcg tttcggtatt 17580
   ttaaagtgtt gggattatag gcgtgagtta tcgcgttcgg tttaagttgt tttttttaaa 17640
   aaaaaataaa ttattaatta ttagggaatt tttttaggtt tattatttat aatttaaagt 17700
   ttttttatat ttttttttta gtgttttaaa ttttgatggt tcgagaaggt tgggtatttg 17760
   tttttagttt tgagaatttt tttatttaaa ttaagttttt tttttttaag gagtagtggg 17820
   attttaatta gatttttatt agaaattttg attatttaga tttttttttt gtttatttta 17880
   gtaatttttt tttttgtaaa gatggatggc ggtagggggt tggagaggga ataattttaa 17940
   gaatttgttt ttcgggagtt gttattatag gttaggtagg ttttgttttt tttttttgta 18000
   tttttttttc gtgttttagg aggattatat gcgaataaat gaggataata tttggttatg 18060
   aggattttta attttaggtt atattaaata aattgttttg tagatattaa aagaaaatgt 18120
   aaaggaaaat ttatagaatt agtttatata aaattaatag tatttttttt gtttttttaa 18180
   aaggtgaata tatataagaa attaatttat tttatttatt gaaaaaggat gtagaaaata 18240
   tggggaaagg taaatgaaag ttttaatttt gtagaatttt ttttagtttt aaacgattgt 18300
   ggagataata ggaggatatt tttgtttttt attatatttt tagtttgata agtaatatat 18360
   gaataatata tggattattg ggtggatata attgagatta atgtatatat attaattaat 18420
   atatgatatt gtaatataat attttaaaat aatatattat attgatttat atttatggat 18480
   ttttgtataa aatttatttt atgtttatgt aatatttttt aaattggtaa aatattttgt 18540
   tttataataa taaattgtta tatataatag ttttttggag agttatttgt aaagattagt 18600
   aaaatgattt tttttttttt tattagaaga attttagaag gttttatttg aataaataga 18660
   tttacggaga cgattggaat aagaatttta ggtgttaaaa ggaaatatat tttttttagt 18720
   atttagtaag aaattatttt tattttattg tatttatgat aattgattac gtttttatta 18780
   ttaggtttaa gaatgtgatt ttaagtatta agcgattgtg taaaatatat atagtttttt 18840
   tatggtttgt ttaaatattt ataaatttta gttttattta ggttttgaat tgatttatag 18900
   ataaaatttt ttttaagaat tttttggtat ttgtgtatgg atatatgtgt atttgtatgt 18960
   gtatgttgta cgtattttaa aatgtgtgta aatatataat taaatatgta aatatattta 19020
   aatatttaga tatagttcga ttatagattt taaaataaaa gaattatttt gtatttatat 19080
   tttttaaagt agggaaaata agaaaaagtt aatttttatt tttagattgt ttttgatagt 19140
   tttttgatgt ttgagatatg tataaatatg aaaatttaaa ttttaaaggt ttagtatata 19200
   taatttattt tattaaatga aattgttata attttagtag ttttagattt tttatttata 19260
   tttaatgtta aaattttatt ttgaatttag aggtaggaga tattgtagtt taaaaattgt 19320
   tagttttatt tttagagttt tggaattttt taaaatttta tggtaggtgt ttagattttt 19380
   aaaatttatt tgttttggtt tgggttatat ttggataaat atattttagt agtgtttttt 19440
   aagtttttcg gattttgatt tagttatatt atttttttag agaaaggaaa gtgggaaaat 19500
   agtatgtaaa tttatttttt tttttttttt gagatggagt tttgttttgt tatttaggtt 19560
   ggagtgtagt ggtataattt tagtttattg taatttttgt tatttgggtt taagtaattt 19620
   ttttgtttta gttttcggag tagttggtat tataggtata tattattata tttggttaat 19680
   ttttgtattt ttagtagagg tagagtttta ttatattggt taggtaggtt tcgaattttt 19740
   gattttaggt gattcgttta tttcggtttt ttaaagtgtt gggattatag gtatgagtta 19800
   ttgtgtttgg tttgtaaatt tattttttta attatatata tttaaaatta atttttttta 19860
   gggtattttt ttagaatgtt agtatgtttt ttgttattaa tttagaaatt ataaaattta 19920
   agttggtaat ttttgtgtta gatgattttt gtattagtat ttggaagatt tttaaataat 19980
   tatgataatt ttaataatgt tagtttgaag gtttttattt attagggttt tttaaatgta 20040
   attattggta taattgaatg atatatttag gtagtatttt tgaatagtat taggtttttg 20100
   gtaatatgtt aaattaaatt tgattagaat atttagtgag gatttaaaat gtagaggtaa 20160
   ttgtatgatg gaagagaatt ttgattataa ggaatgggag ggtttgtaaa gtgtgttttt 20220
   tttgtatatt ttaaaggagg ttggagttta tggtatttta gttattttga atttagagaa 20280
   tgtttgtagg gttttagaat gagattgaaa gaaggagaat ggttaatagt ttttgatagt 20340
   ataattagga atattgtgat atttatagtt ttgagaaaag gagaaatatt taaaatattt 20400
   ttgaaattat aagaaatagg tgttttgaaa ggatttagga ttttttaatt tttgaaattg 20460
   tattttaaga aagattgtat agttttttgt ttttaatgag aggtattgaa tacgtttttt 20520
   taatagttga tgtttattta attgttgagt ttgtaagttt ttgtttttag atttagtaga 20580
   gtgggtttaa gttttattga atttttaata tgttttttta attttttatg aaatttttgt 20640
   agttataatt tattttatta ataaaagtta atttatattt attttaagga attaagtaat 20700
   ttataataat ttatattttt tatagtaatt gttttgtagt atatgttttc gttaaataaa 20760
   atgttagaat gttatagtat tggttagttt taatttagat tttttattta ttattttgat 20820
   gtgtattaat atgtattttt tgtgattgtt atatgtatga aatattttaa ttaaatagta 20880
   ataaaaaaaa tttttttttt ttttatttgt aatatatttt attgattaag gaagtggtat 20940
   gtgtgtgttt aagtttaaat aatgcgtggt ttagatatat tataaagagg tagttaattt 21000
   tatacggatg aaaatagtaa atgtatatta tgtgtttgtt aattaaatgt gaggggtcgt 21060
   gaggttaggg gatagattta gatttattta gatgaaaagg ggaaaattaa gatttgtaaa 21120
   gttttatcgg aaagaagtat agaggaattt tcgtgagagt tttgtaaagt tttttattag 21180
   gaggtgaata gatgtttata gttattatat ttaaaattta gtttatttag agtattgtgg 21240
   atttaggtta ggagttgtag tatgtgagaa ttaagaattt aggtttaaaa atcgattaga 21300
   tttgagttta tttttaggtt ttgttatttg ttggttgtat gattttggaa attgtaaaat 21360
   ggggtaaata atttttgtcg tttagaagaa tgagaggatt gaatgagaga tttatgtgaa 21420
   gtgttgtgta tatgttgggt ataggataag tataattatg atggtgatta tgaagtagaa 21480
   agaagaaaag gagaggaata gtattgagtt ttagaaaagt atttttaaat ttgggtttaa 21540
   atttttggtt tttggttagg tatagtggtt tacgtttgta attttagtat tttgggaggt 21600
   tgagttaggt agattatttg aggttaggag tttgagatta gtttggttaa tatggtgaaa 21660
   tttcgtttgt attagaaata taaaaaatta gttggtgtgg tggtatgaat tttagttatt 21720
   taggaggttg aagtaggaga attatttgaa ttcgggaggt agaggttgta gtgagtcgag 21780
   attacgttat tgttttttag tttggataat atagggagat ttcgttttta aaaaaaaaaa 21840
   aaaaaaaaaa aaaaaaattg gttttttgtc gtggttttaa gggttaatga ggattatagg 21900
   aatggtcgta agaatggaga gggtataaat aaagataata gagaggaggg gtaggtggag 21960
   gaggaggagg aggaagggta gagggaaaaa ggagaaggag gaggaagagg taggaaagga 22020
   ggaagaagga ggaagatgga ggagaagaaa taagttttta tatttgggga gtagtagttg 22080
   ggaggaggaa gagaaataag taagtttttg cgtttgttgg aatagaggtt tttagagttg 22140
   ttttttattg atatttatgt atagttttga tggggaaatt tggggaggag aaagtaaggt 22200
   aaggtaggta tttgttatta tagtagaggt agagaagtat ggtaggttat agagagggtt 22260
   ttaggtgtgg atgttgattg ttttatttat tggttgagta attttaggaa aattttttga 22320
   ttttttttta atattataat aatattatta tttatttttt ggggttgtgg agaggattaa 22380
   atgagtgaaa ggtatgaagt gtttagtata gtgataaatg aatagtaatt attattaaat 22440
   gtgagttgtt gttattatat attgttttaa agtttgatgt tgatattttt gtagtgattg 22500
   tatagtaaat tagttaatgt aagaaattag aatttgtaaa ggtttattga gttgttatta 22560
   tagagggagg aatagggagt gaaatttgtt ttaatattaa ataataagtt taattaacga 22620
   atcgggtagg atataataga gaggtaggga ggatatatta aggaaaaagg agaagttgga 22680
   attgagtggg agtaggtgtt tggggaaatg gattaggagg ggttttatta ttattaaggt 22740
   tattttgaga tgataatagg atggttggtt aaatatgttt tgtaatgaaa attatgttta 22800
   gttggatagg ttaaattatt aaaggtaatt tttaatttta ttattttaaa tttttttatt 22860
   tatattggtt tgattatttt tgtagttgta aatttagaag ttatgtttgt tattgtatta 22920
   tgtatatttt ttttagtaat tgtatttttt tttttttttt ttttttttag tattttatgt 22980
   ttttttttat tatagtattt atcgttttgt tgtagttttt atttattttt attttttttt 23040
   attagttatt ttaaaatttt agagtttagt tttaaatttg ggatttgtta aaggtggtaa 23100
   tgaatggaga aggtcgttat tagtagggtg gtttgtattt ttattttttt tttttatttt 23160
   agtttttttt gtttgttgtt tttagttttg tttttttaaa attttagatt tggtataaaa 23220
   tttttatttt aagaagttag aacgatttgt attgtgtatt atggtatatt taagttaatt 23280
   gatttttaaa atttgttata attgtgtgtt ttttttttaa tttttttatt tttatttttg 23340
   tttttttttc ggtttagttt tagttatttg gtagattatt tttatttttt tttttttatt 23400
   tttgtatatc gtgttttttt agtttagagc gttttttttt atttaagttt ttttttttat 23460
   ttggagtata gttaaagttt tgttatattt aagaagtttt tttatttttg tagtaggtac 23520
   gattttttga agttttgttg tgtttatttt tagtattatg agttttttgt gttgattttt 23580
   tttttgtttt attcgtgtta ttttttattt ttttaattta tattgtaaat agatttaggg 23640
   aagagttatt tagaatgttt ttttgttgtt atttgtagta tttggtatat tgtgggtatg 23700
   tagtatattt ttaataaatg attgatagga atattggggt tatatttaaa gagattaaaa 23760
   ttgataatgg aggatggaat tggattattg aagatttgta taaaggtgag tatttagggt 23820
   ttatgagttg aatttttaga tttgtagatt tagtgataaa tttatggtat ttataagaat 23880
   tgagtggaaa ggttagttgg ttattatagg aaagtattaa tgaatggggt tttatgaaaa 23940
   atgttagatt ttatttaaaa tgttattatt ttagattttt ataaaaaatt gtttttttaa 24000
   attattgtta agagtttttg ttattaaaaa taaattatat tttatttttt tgaatatggt 24060
   tgtttgtatt ttttatttgt ggttattatt gaatttattt tatgtttagt ggtttttttt 24120
   tttttttttt tttttttttt ttttgtagta tattttggga gatgaagtgt agggtcgggg 24180
   tggtagggta gattttaaaa tattgtatag attaaatttt ttttagtaga agatggttat 24240
   gaaaaaaatt tttttttaag ttttggttgg gtatagtggt ttaggtttgt aattttagta 24300
   ttttgggaag tcgaggtagg aggattgttt gagtttagaa gtttgagatt agtttgggta 24360
   atatattggg attttgtttt tatataaata tatatatata aattagttaa gtgtggtggt 24420
   atgtataggt agttttagtt attaaggagg tagagatggg aggattattt gaatttagga 24480
   ggttaaggtt gtagtgagtt ttggttatat tagtgtattt aagtttgggt gatagagtaa 24540
   gattttgttt taaaaaaaaa aaagtttaat ggttttttaa aaatagagat aataaaatga 24600
   agatatttgt aaataaattg aattttaaag tttattgttt gtttagaata ttttaggtga 24660
   attagatgtg aattagatgt attttatggg tttgtttttt tatttatggt tttaggaatg 24720
   taggttagat attttttatt gttgtttgga aaattttttg gtttttttag ttgtttgagt 24780
   ttgtagtgtg tagttttgtt gttgtgttgg ttgatatgtt gtattttagt gttttttttt 24840
   gttatttttt ttcgtattat tattttttaa aaatagttgg gttataagtg atttttatta 24900
   tagaagttgg tttaatgatt ttttttattt ttaattaaat tttgttttta ttaattttta 24960
   gaatatagaa aaaatatggt cgggcgtggt ggtttatttt gtagttttag tattttggga   25020
   ggttaaggta ggtggattat ttgaggttag gagtttgaga gtattttggt taatatggtg   25080
   aaattttatt tttattaaaa atataaaaaa agtagttagg tatggtggtg tatgtttgta   25140
   attttagtta ttcgggaggt tgaggtagga gaatcgtttg aatttaggag gtagaggttg   25200
   tagtaagttg agattatgtt acggtatttt agtttgggcg atagagtgag attttgtttt   25260
   aaatttaaaa aaaaaaaaaa gaatatagaa aaaattttag atttttgtat gtttgttaat   25320
   taataatttt tatattttaa aagttatata tatttttgtt gtggaaaatt ttaagtatgt   25380
   agaaataaaa aggaaaggaa ataaaaatta tttataattt tgttatttag agataattaa   25440
   tgttaatatt ttggagtttt agttttttta aatgtttgtt aaatgttttt ataaaataaa   25500
   atgaaatgta atatataata ttttttattt tttttttgtt ttagttaaaa tgtgtttcgt   25560
   tttgttatta tatagtagtg tataaatata ttgttatgtt ggatattaag gtttgtttaa   25620
   gttttagata atggatattt ttgtaattaa gtttttgtat gtatttatta ttattttttt   25680
   aggttaaatt tttaaaagtg ttgttgtgtt aaagggtatg aattttttaa aagttttaat   25740
   atataatttt aagttgtttt ttagaaagtt ttaattaatt tttgttttta tttttggaat   25800
   agtatgtatt atttttttat ttaatttttg ttaatttaag aggaaatttt ggtattttat   25860
   attatttttt ttttttttat tgttggtgat gttgaatatt ttttatgtgt ttagtatttt   25920
   tttatatgtt ttgtgaatgt ttatttatat ttttttttta tatttttgtt gatgtgttta   25980
   tttttttatt gatttgtata aattttttta tattaaaatg ttaatttttt gatatgttgt   26040
   aagtattttt tggtttgtat taattatttt taaatgttga ttatggtgtt ttatgtttgg   26100
   ggagtttaat ttgtttgtaa tttattggta tattaaagtt ttaaattttt gtttgattaa   26160
   attaattagt tttttttttt ttagtttttg ttttttatgt ttttttttat tttaatttta   26220
   aaaaaaaatt tgttattatt tcgaatattt taatggtttt ttttttgttt tttattttaa   26280
   tttaaggaat tatattaagt gtattattat agaaaaatga gtatgagtta agttaggatt   26340
   agtttttatt attttatttt gaattgtttt tagttgtttt tttttttaaa attagaattt   26400
   tttgtgattt tagggaagtg ttttagtttg gtcggtttta ggtagttttt tttttttaat   26460
   ttattttagt ttattatttt ggttaaaggt attttggatt tttagaggtt tagaggaaag   26520
   tttttttttt tataaaggga gttatgtttg ttatgtgttt tagtgtttgg aattatattg   26580
   atttttttgg aggtttgcgg gttatagcgt aggattaagt gttggaatta gggtagtttg   26640
   tggagggttg gtttttttgt tgttttgtat ttttagattt atttttagag tattttgatt   26700
   tggtgaggtg gatgttagtt ttttatatgg tatttttcgt ttaattgtta ttggtgtaaa   26760
   ttaaaatttt ttttttattt tttttgtata ggaaagataa gtgggtgtat gaagtttaat   26820
   taaattaatt tattttgtta ttaaaaattt tttttttttt ttttttttga ggtagagttt   26880
   tattttgtta tttagattgg agtgtagtgg tgtaatttta gtttattgta gtttcgattt   26940
   tttaggtgat ttttttattt tagttttttg tgtagttggg attatagttg tgtattttta   27000
   tgtttagata attttttgta tttttagtag agatagagtt tttttatgtt ttttaggttg   27060
   gttttaaatt tttgggttta agtaattttt ttgttttagt tttttaaagg gttgggatta   27120
   ttggtttgag ttattatatt cggtttgtta ttaaagttta aaatgttaga gtaagaaaat   27180
   atttgaaaga gtatattatt ttatattttt gtttttttag ataaaggttt gaggttttag   27240
   agtggggtag ttattataga tggtagaatt ttaatttgtt ggtggataat tttgtgtgtt   27300
   tttatttttc gtttattagt tagtttttgt cgtgtgttag gtattgtggt aggttttaaa   27360
   aatttttaaa gtgtttaaac gtattttttt gttttttaag aatttaattg tttagtaggg   27420
   agatagatgg ataataatta attatagtat gtattatgga tgtattaata taggtatgga   27480
   tagggtgttg gagaagtata aaaaaaaatg ggtaaataat tttggtagga ggggttgtag   27540
   aattggagaa gattttttgg aggaggtagt atttggattg gattttaaag aaaaggtagt   27600
   tgtttgttgt aagggtaaga ttatagtagg ggtaaaggta tagaggttgg aagtataatg   27660
   tatagttgag gattggagag tggtttggtg gttggtgtgg tgtaggggat ttttttcgtg   27720
   ttttcggttg tagttagttg agttatatgt agtttttttt tttttttttt atgtttttat   27780
   gtagaatgtt gttttttacg tgtttgttta agtagttttt ttttttaatg tttttagaat   27840
   gaatgatagt ttttttttta atttttgtat ggtattatat attttttttt attatagtga   27900
   ttttgatatt aaaattagag gtgatagtag ggattgggtt tttttaaaaa aattaattag   27960
   aatatatgtt tggggttgga taagaaattt aaatagtatg aaaaggtaga agttgaaaaa   28020
   aataaaaaga ataaagtttt tttattttta agtatttaga gttttatgag tggtaaagga   28080
   tttattattt tgaaagtgtt ggaaagttat tggagatttt taagtaggga attaaaggta   28140
   tttggagaga aagggattgg tggtaggtgg ttagtcggaa aatgatttag ggatgaggtg   28200
   tttgtacgtg ggaggtaata atggtaaaga ggggagaata tgatggtgga atatatagaa   28260
   tttatatatt agttggttgt gaagatgaaa ggaaaaggag aagaaaagat aatgatgaag   28320
   tttttggttt ttttaagttg ttgttttatt aattataaat ttaataagaa atcgtgttta   28380
   tttttttttt agtgtagaaa aaggtaaagt tagttacgtt tatattttta aaaggattac   28440
   gtggagaaga tgtaatatta aattgtatat ttggaagtta tttttttttt aatgtaataa   28500
   atatggttaa tatgtagaat agtaaaaata ttttgtatga taatattttt taaagttttt   28560
   ttttttttag attaaggtaa ggtttgtaga aaggtattag gataaagtag gaatataaaa   28620
   taaggttgat tttagggtga gagagtttgt tgtatagggg agttaggtcg aagtattttt   28680
   ttttaggagt gagatgagtt gtagtgaagt taggtaggta ttttttatag atttatattt   28740
   tatttattcg gaaggtatta tgtgtaggag gggtagtatt ttttatgttt agttttattt   28800
   gaggtttgtt ttattgattt ttgttggatt tttgaagata gtggaagttt ttaatatttg   28860
   tgatttgtat aagtgtgtaa aggttaaaga atatgattgt gtgggatatt tttgtgtaat   28920
   tatatgtaag agattatatt agatggaata tgagatattg taaaatataa gatatattta   28980
   gaatttattg tttagtttga ttaaattata gagtgttgaa aatatttttt aaataagtat   29040
   ataatttgtt tatgaaaaaa atatttaaaa gtaggggggt tgtttatggg attgttaata    29100
   atgtttttat tagttaaatt aaaagtagat tttatatttt agttaatttt tttttatttt   29160
   ttttttttat tagataattt ttaggattag atgaaaaggg agttagttta tcgagaagaa   29220
   atggtgtaat agttataaat tgtaagttgt atttttattt aa                      29262
<210> 103
   <211> 20043
   <212> DNA
   <213> Artificial Sequence
<220>
   <223> chemically treated genomic DNA (Homo sapiens)
<400> 103
   aggcgtgagt tattatatat agttaattgt agggattttt ataatttata aaaggaaata      60
   gagagaattc gaattttttg tttttatatt gtttttatat ttttgagggt tatttatttt      120
   ttttgttttg ttttgttttg agatagggtt tggttttgtt atttaggttg aaaagtagtg      180
   gggcgatttt agtttatagt aattttcgtt ttttgtgttt aaattatttt gttatttcga      240
   ttttttgagt ggttggaatt ataggtatgt gttattattt ttagttgatt tttgtatttt      300
   tttggtagag ataggatttt attatgttgt ttaggttggt ttcgaatttt tggatttgag      360
   tgatttattt atttttgttt tttaaagtgt tgggattata ggcgtgagtt attgtgtttg      420
   gtagggttag tagtttttat gaaattttat gtgggagtag aatgtttttt gatttgagaa      480
   gattttttta tagatgttgt atgagtagtt attatttttt ttgtgtgaac gtcggatatg      540
   gttgttatgg gtagtatggg atgtaaaaga tttattatag tatttgtatt tgaagggttt      600
   tttttcggag tgttgtttga tgtgtgtgcg gagtatgttg gaggttgtga atttttgtag      660
   agaatagaaa tatagaatat aagaaaaagt taatttttac gagttggata attaattaaa      720
   ttaaggggta aattttattg ttgtttattt ttatggacga attttgggtt ttaaggttat      780
   agagtatttt tggttattat tagtagtttt aattcgaaag gattttattg atgtgttttt      840
   ttggttagtt ttgagaagta ttggatgtta gaatagtggt tttttaatgg ttagattatt      900
   tggaggttta gtttgtaaaa aagtagattg taggttgggc gtagtggttt atatttgtaa      960
   ttttagtatt ttgggaggtt aaggtaggtg gattatttga ggttaggagt tcgagattag     1020
   tttggttaat atggtgaaat tttattttta ttaaaaatat aaaaattagt taggtatgtt     1080
   atcgtgtatt tttaatttta gttttttagg aggttgagat gggagaattt tttgaattta     1140
   ggaggcggag gttgtagtaa gtcgttattg tattattgta ttttagtttg ggtgatagag     1200
   tgagattttg tttttaaaaa aaaaaaaaaa aaaaaaaaaa aagtagattg ttagtttggg     1260
   taatatagta atattttatt tttataaaaa gtaaaaaaat tatttaggta tggtagtatg     1320
   tatttgtggt tttagttatt tggaaagttg aggtgggagg ataatttgag ttagaaggtt     1380
   aaggttgtag tgagttatga tggtattatt gtattttagt ttgggtaata gagtaagatt     1440
   ttttttttaa aaaaaaaaaa aaaaaaaaaa gtagattatt aattttattt aagagagtgt     1500
   gatttaataa atttgatatg agatttagga gtttgtattt aaataagtaa ttttgatgta     1560
   tgtgggtttt aggattatat tttaagataa attttattat agaaagagaa gttaatatcg     1620
   attagaagtt aatatcggtt agtgtttttg tataagttag tgttttattg gttttttttt     1680
   tttttttttt ttttttaatt taaatttttt gagatggttt gtaggtttgt tttatttgtt     1740
   tgtttgtttg tttgttttga gatagaattt tattttatta tttaggttgg agtgtagtgg     1800
   tataattttg gtttattgta atttttattt tttaggttta agcgatttta gtgttttagt     1860
   tttttaagta gttggaatta taggtgtatt atgatgttta gttaattttt ttatttttag     1920
   tagagatggt gtttttttat gtcggttagg ttggttttaa atttttgatt ttaggttgat     1980
   ttatttattt tggtttttta aagtgttggg attataggtg tgagttatta cgtttagttt     2040
   agtggtgttt ttttatgttt attattttta ttttattgat atatattttg gaggtattag     2100
   ttgttttaat ttggtagatt taatgtttta ttttttttga gaggtttaga agtagttgta     2160
   ggatagtttt tatttttttt acggttttat tgtaaaaagc gtaaatgatt tagtgattga     2220
   aaatataatt tatattagtg ttttttaata ggatttgttg taataatggg aatatttatt     2280
   atttacgttg tttattttgg aaattaatgg ttatatggag ttattgagta tttaaaatgt     2340
   ggttaatgta atttagggaa cggaattttt taattttatt tcgtttttat taattgaaat     2400
   agttatatat gattaatcgc gaattgtata ttgggtagta tagagttatt ttggtgtaat     2460
   atgtttaaag cgtggttaat ttattgtttt tattatagta atttttaatt atatgattta     2520
   tttttgtttt tataattttg tattttgtgt aatatagatt agatgtcgtt tttgtaatgt     2580
   ttttatagaa aattatatta gtagtatttt ttatagatta aagtagtaat aatagtagtt     2640
   aatattttag tagtattttt atgttatgtt aggtattgtt ataagagttt tatatatttt     2700
   attgatgtta tttaataatt tggtaaagat tatttttttt agtttattaa tgtggaaatt     2760
   aataggagtt tatggatttg tttagggtgg tatagttagg taagtattag aggtaggatt     2820
   tgaaatttgg ttatgtttgt gtttttaaat attatgaaat agtgattttt tttttttttt 2880
   ttttttggag acggagtttt attttgttat ttaggttgga gtgtagtggt ataattttga 2940
   tttattgtaa tttttgtttt tgggtttaag tgattttttt gttttagttt tttaagtagt 3000
   tgggattata ggaacgtgtt attatgttta gttaattttt gtatttttag tagagatggg 3060
   gttttattat gttggttagg atggtttcga attttttatt tttaggtatt tgtttatttt 3120
   agttttttaa agtgttggga ttacgggttt gagttattac gtttttttat tttttttttt 3180
   tttttttgag atagtgtttt attttgttat ttaggttgga gtgtgtagtg gtgtaatttt 3240
   ggtttattgt aatttttatt ttttggattt aagtaatttt ttagttttag ttttttaagt 3300
   agttgggatt ataggtataa gttatcgatg tatggaaaat ttgattatag gtgtgagtta 3360
   ttatatttag ttagtgtttt taaatattat gtaataggtt attaggtttt tataaatttg 3420
   gaatgtattt tattatatgt attttaaatg ttagtttggt aataggaggt tggttatatt 3480
   tgattatttt tagtaaattt gagtatattt aggggtggaa atgaggattg ggttgatatt 3540
   tgattaatat ttattaagaa tttattaagt taggttttgt tttaagtttt tatttataga 3600
   tatttaagta gagtttagga tatatttttt agtaagaggt tttatttatt ttagtataat 3660
   atatatattg gtatggtttg tttttagagt ggattcgtat gtgtttgttt aggttggaag 3720
   attgagagaa atatttttta tatgtagaat attaaggtga aaaagaaaaa tatagttgaa 3780
   attatatttt tttttaataa gtttatttta attttgcgtt tatttggggt tagagaagaa 3840
   ttttttttta attttttttt tttttttttt tttgagacgg gattttaatt tgttatttag 3900
   attggattgt agtggtataa ttacggttta ttgtagtttg gaatttttag gtttaagcga 3960
   tttttttatt ttagtttttt gagtaattgg gattataggt gcgtgttatt aagttagtta 4020
   atttaaaaaa tttttttttg tagagatgag gttttattat gttgtttagt tttgttttga 4080
   attttaatag atttttttgt tttggttttt taaagtgtta ggattatagt tttgagttac 4140
   ggtattcgat ttgagagaat ttttgtttta gatgtggaat gtgggaattg aataggtttg 4200
   tggtatatgg tagaggtttg aattagtttt gtttttttaa taggtagttg tgtgattata 4260
   gaatattggt taaatttgga attttagttt ttcgattttg tttatgggta aaatatttgt 4320
   tttgtttttt ttatagagtt atgagtaaat tataagtatt gtgtaattaa tttaataatt 4380
   atttacgatt tatagagtat ttattgtgtg ttttatattg ttagaaatta ggagttagaa 4440
   attaaggagg ggtgacgatg aatatgtaag ttaatatggt aatgtatagt agtagattat 4500
   ttgagacgtg ttgaatttgt atgaagaggt taatattttg tttatacgtt gtgaataatt 4560
   aatattgttt tttgattttg gtaatttttt tcgaaattgt tttaggaatt ttatattagg 4620
   tgaagtaaat tattagagag gtgtaagtat cgagagtttg ttagggataa agtagaaggt 4680
   ttttggtttt gatattattt ttaaggaaat tatattaatt aaagagaaaa aataattttt 4740
   ttttgagaaa ggtttttgtg tttttataat ttttttttag gtatgtttat ggggtagagg 4800
   agagagtaaa tttttgtatt tgtttattta tttattttta tttatttttg aggtagagtt 4860
   tcgttttgtt gtttaggttg gagtgtagta gtatgatttc ggtttatcgt aatttttgtt 4920
   ttttgggttt aagtaatttt ggtgttttag tttttcgagt ggttgggatt ataggtgcgt 4980
   attatatgtt tatttttata aaaaatataa aaaattagtt gagtatggtg gtatgtattt 5040
   atagttttag tttagttatt tgggaggttg aggttgggtg attgtttgag cgtaggaggt 5100
   ggaggttgta gtgagttgag attatgttat tgtattttag tttgggtaat agagtgagat 5160
   tgttttaaaa aaaaaaaaaa aaaaaaagat tgttttttta ttgtattgta agagtatttc 5220
   gtttgttgaa gggtaagtta ttattttatt attattagtt tttttattag ttttgttatt 5280
   tattgatttt ttggttgata gttttttagt tttttttttt tttttttttt tttgagacgg 5340
   agtttcgttt tgttatttag gttggagtgc ggtggcgcga tttcggttta ttgtaagttt 5400
   cgttttttag gtttacgtta tttttttgtt ttagtttttc gagtaggtgg gattataggt 5460
   gtttattatt acgtttggtt aattttttgt atttttagta gagatggggt tttattatgt 5520
   tagttacgat ggttttgatt ttttgatttc gtgatttatt tgtttcggtt ttttaaagta 5580
   ttgggattag gcgtgagtta ttattttcgg tttttaattt taaagagagt aagtatttta 5640
   tatatttttt gtaatttggt ttttgtttga taagataata tttttttttt ttttttggga 5700
   tggagtttta tttttgttat ttaggttgta gtgtaatgat atagtttcgg tttattgtaa 5760
   ttttcgtttt tggggttgaa gtaatttttt tgttttaatt tttagagtaa ttggggttat 5820
   aggtgtttgt tattatattc ggttaatgtt tttatatttt tagtagagat ggggtttcgt 5880
   tatgttggtt aggttggttt cgaatttttg gttttaggtg atttgtttat tttagttttt 5940
   taaggtgttg agattatagg cgtgagttat tgcgtttggt tagtaagtta taatttatat 6000
   aattaatttt gtgttgatga atatgtagtt ttaaattttt tattattaga atattggata 6060
   gtttttattt ttgttagttt aataagcgag aaatattttt atcgttttga tatttttaaa 6120
   tttttttaga attatataaa tgtatgataa ttgtaaatgt atttaaattt tataggagta 6180
   tataatatag tgattaaata tgattttttt agaagtttag gaaggaaggg agaaattagg 6240
   ataattttat gatgatttta tttttagttt ttaaaatagg aaaatgggtt taaggtcgta 6300
   gttgtttgag gtagtttcga taaggttaat agttaagtaa gaatatattt gtgtttaagt 6360
   ttcgtttttt ttttaattaa gtttaaggga atattgtatt tttttgtagt aagtttagta 6420
   gagtatttag gtatttggaa tggtttcgta atgttattat tgattttttg ttttataaaa 6480
   ttttcggttt attgtagagg tttttcgttt tttggaagga tcgtgttttt attttttttt 6540
   tatttatatt tgttaatata tttgtataga tttggaataa ggcgtagttt tttaggagta 6600
   tatgttaggt ttttttttgt ttggaatagt tcgatttttt tttgtttaat taattttgtt 6660
   attagtataa attagatatt ttatttgttt ttttatattt ggtttaaggt tatttttgtg 6720
   tattttttat aatttacgtg tttaggtggt cgtggatatt ttttttttta tgtttttttt 6780
   tttgtgggta tttgtatttt aatatatgtg tatcgttgtg tttatttacg tttgtttgtt 6840
   tcgtaatatt attttttttt tttttagaga gggtgttcgt taggtattat agttttgtag 6900
   ttagtattta taaggtaatt tttcgtgttt aggagtttgg gaagggagag agaacgtgat 6960
   ggtgggttat gtgttttttt aatatttatt ttgtgaggtc ggtgtttttt atgaatatga 7020
   agaatgtgga ttcgaagtcg gtttcgtttt ttaaaggatc gtttgtagag agaataggga 7080
   aattttttat cgtttttgtt tatatagggg gtgtatttga ggtgtttatt tttgtaatat 7140
   ttgtaggtaa atattttttt atatttttta tatttgtagt tttttgtaga ttttgttaaa 7200
   tatagtaaat ataatgtttt tttagttatt ttttttttta gtttttttaa gaggatattt 7260
   atttatttat ttatttttag ttaagaattt tttggttatt ttaagaggaa tttttttttt 7320
   ttgagataga attttgttgt attgtttagt ttgtagtgta gtggtacgat tttggtttat 7380
   tgtaattttt gttttttagg tttaagcgat ttttttgttt tagtttttta agtagttggg 7440
   attataggtg tttattatta cgtttggtta atttttgtat ttttagtaga aatagggttt 7500
   tattatgttg gttaggttgg tttagaattt ttgattttag gtgattagtt ttttttggtt 7560
   ttttaaagtg ttaggattat atgcgtgagt tattgtgttt ggttaagaga attttttttt 7620
   tttttttttt tttttttttg agatggagtt ttgttttgtt atttaggttg gagtgtaggg 7680
   gtgtgatttc ggtttattgt aatttttatt tttcgggttt aagtgatttt tttgttttag 7740
   ttttttaagt agttgggatt ataggtatat attattacgt ttagttaatt tttgtatttt 7800
   tagtagaggt ggggttttat tattttggtt aggatagttt tgattttttg attttgtgat 7860
   ttatttgttt cggtttttta aagtgttggg attataggta tgagttatta tgttcggtcg 7920
   ttaagagaat ttttaaaaat ttttttatta tagaaaaatg taaatatatt aaagtgaaaa 7980
   taatagtgta ataaattttt atgtgttaaa tattaagttt tgattaattc gtaattaatt 8040
   ttgtttttat ttatattatc gttcgttata gtagattatt ttaatattaa tttatttttt 8100
   gatttaaaaa tatttaatcg taatttttag aagatgatat atttttaaaa agtataattg 8160
   tgattaggta aagtggttta cgtttataat tttagtattt tgggaagttg aggtaggtgg 8220
   attatttgag gttaggggtt taaaattagt ttggttaata tggtgaaatt tcgtttgtat 8280
   taaaaatata aaattagtta ggcgtggtgg tgtttgtttg taattttagt tatttgggag 8340
   gttgaggtag gagaattatt tgaatttggg aggcgtaggt tgtagtgagt taagattgta 8400
   ttattgtatt ttagtttggg taataatagt aaaattttat tttaaaaaaa aaaaaaaaag 8460
   tataattgtg ttaggtgtag tggtttacgt ttataatttt aatattttgg gaagttgaag 8520
   tggatagatc gtttgagttt aggagtttga gattagtttg ggtaatatag taagatttta 8580
   tttttataaa aaaataataa aaattatttg ggtatgatag tatacgtttt tagttttaga 8640
   ggtcgagatg ggaggattat ttgagtttgg ggaagttagt tgaggttgta atgagtagtg 8700
   attgagttat tgtattttag tttgggtaat agagtaatat tttgtttcga aaaaaaaaaa 8760
   aaaatataat tataatgtta ttgttatatt gttatattta aacgattaat ttttattttt 8820
   ttatagaaat atatatgtat ttatatatgt atatttatat atatatatat atatttgttt 8880
   aattattttt tggttagttt atttaaatta ggttttaaat aaagtttata tgttgtatat 8940
   ggttgttatg ttttttaaat attttaattt ataggttttt tttttttttt gttttttgat 9000
   atgtattgtt gaagagatta ttttattagt tttatttaag ggtagtgtaa ttttgtagtt 9060
   tttgaatata tttttgtttg gtatttgtta taaggttaat atattgaggt attgaggtcg 9120
   atttattttt ttttgtgttt ttaagattag tttgagttat aagtttagag tagttattag 9180
   gatattcgta gtttatagtg ttgttttatt ttatataatt taagggattt ttttagttta 9240
   tattttatta tttaaaattt gtggtagatt ttagagttag aatttttatg tatagagtaa 9300
   ggttgaaagt attttgattt ttttaatgta tagggttatc gtgatgatta atttaatttt 9360
   aatatttatc gagttttatt aatgtgagtg atattattat tgaatggtag ataagattga 9420
   gaggtaaatt tgtttttatt aatggaggag ggaggaggag agaagttaga gagggtgtag 9480
   ttagatttta gtaaagtagg ttttacgttt aggggtgttt atgtgttggg ggaaggcgag 9540
   agaatagggg ttatttataa aagtattttt gtagaggata ttttggattt tagaggataa 9600
   agtggttttg attatgaatg tattatagag attttattgt ttgattttag ggaggaagag 9660
   tagttaagag tgagtttatg taagagtttg tagatagaaa tttttttagt attaggatag 9720
   tagggaagtt gtttattagg ggttttgtta gttgataatt atattttagg tacgtttaat 9780
   aggtggttaa ggaaataaat tatttattaa gtaaataatt aggaaggtaa ttgttagttt 9840
   taagttgatt tatcgttttg ttttgtttat tatttttatt gaggtgttaa tatttttaga 9900
   attttcgtgt tgggatattt ttttggtata gaggtagaat ttttattggt tagaaatatt 9960
   tggttttttt gttttttggg gtgggaaggg aagagagggt tagtaatttt ataaatttaa 10020
   gtattaaagt atattaaaat ttagaataaa gttttaatat tgagttcgtt taagaataat 10080
   tatttaggta gaaatgaaat ttaattttat tttgtttttt atttttttta attttatttt 10140
   gttttttatt tttttttttt ttagtttttt atatttttat attttaaaat atatatttaa 10200
   attttttttt ttttttataa gaggggaatg tttgtatatt taaataattc gtttatatag 10260
   agtttagatt ttttttttat tatttaagat tttaaagagg aaggttttta tttattatta 10320
   atttttatta aaaagttttt ggggaaaaaa atttatttga gaatgtaatg gaattttaat 10380
   tttttttttt tttttattat atttaaatta tttatattaa tagattaata aattaatatt 10440
   tatttataat tatttttaat aaattaagtt gtttataagt ttttttttat tgtttaaaat 10500
   atatgttttt ataataatgt tgtaagtatt aggtattatt tggtttagaa ttggaatata 10560
   agtattttat ttgtatgttg ggtatttttt gtatttaatt attataagtt tttattttgt 10620
   tgtttgtttt gattgttttt gatatcggta tattatttta tgttaataat aaataggttg 10680
   agtatttttt ttttgaaatg tttgggatta gaagtgtttt ggattttagg tttttttcga 10740
   attttggaat attttatata tataatgagg tattttgggg atggaattaa agtttaaata 10800
   taaagtttat ttatgttttg ttttatatat attttatata tatagtttga aggtaatttt 10860
   tttttttttg agacggaatt ttgttcggtt tgtaattttc gttttcggtt ttaagcgatt 10920
   attcggtttt agttttttga gtagttgaga ttataggtat ttgttattat gtttagttaa 10980
   tttgtttgta tttttagtag agacggggtt ttattatgtt tgttatgttg tttttaaatt 11040
   tttgatttta ggtgatttgt ttgtttcggt tttttaaagc gttgggatta taggtatgag 11100
   ttatcgtgtt tggtttattt gaaggtaatt ttatataata ttttaaataa ttttgtgtat 11160
   gaaataaagt ttgtgttaag tattggtgtg tggaaatttt tatttttggt aatatgttag 11220
   tgtttagttt tagattttag agtattttgg atttgggatt ttttgattgg agttgttgag 11280
   tttgtattat tatttattta attattatat tattgaatat ttaggttgtt tgtgattttt 11340
   tatttataag agtggtgtta tagttaaaaa tttttataag tatagttttt tttttttgga 11400
   ttatttttcg ataatagatt tttagaagag tattattgag atattaaagt tataaatatt 11460
   gtaataattt gatatatacg gtgaaaaaat tttttaaaaa tggtcggtta tggtgtttta 11520
   tgtttgttat tttagaattt agggaggtta aggtgggtgg attatttgag tttaggagtt 11580
   taagattagt ttggttaaaa tggcgaaatt ttatttttat taaaaatata aaaattagtt 11640
   gggtgtggtg gtgtatgttt gtaattttag ttattcggga ggttgaggta ggagaaatgt 11700
   ttgaattttg gaggtagagg ttgtagtgag tttagattat attattgtat tttagtttgg 11760
   gtgatagagt gagattttat tttaaaaaat aataatgatt ttttaaaagg ataataatta 11820
   ttttgttagt tattaagtag atattttttt taaatttatt gtttattttt ttttttttta 11880
   tttttttttt tgagatggag ttttattttg ttatttaggt tggagtgtag tggcgttatt 11940
   ttagtttatt gtaatttttg tttttgggtt taagcgattt ttttgtttta gttttttgag 12000
   tagttgggat tataggtatg tattattata tttagttaat tttttgtatt ttagtagaga 12060
   cggagtttta ttatgttggt taggttggtc gtgaattttt gattttaggt gatttaatta 12120
   ttttggtttt ttaaagtgtt ggggttatag gtgtgagtta ttacgtttag tttgttgatt 12180
   tttatttcga aaggagttgg tatattttag gaagtgatta ataggttttg gtttggtgtt 12240
   aagtaggaga taatttagag ttttatagat tatagagaat tttaattatt tttgttttta 12300
   tttttatttt tatttatatt tttatattta tattatttat taaaatattt ttggttttag 12360
   gtattttaga aaaaggatat ttaatttgta tgtagatatt agaggcgatt agaataagta 12420
   atagaatgag ggtttatgat ggtttagtgg cggggggaat ttagtatgga tataagatat 12480
   ttgggggagt aggaagatag tttattaaaa taattttgat agttttttat tattgggatt 12540
   attatttatt attagataaa taatgacggt tgattgttta ttatttatta ttagataaat 12600
   aatgattgtt tattatttat tttttaattt ttggatattt ttttgttttt tgttttattg 12660
   ttttttgagt ttgattgttt tttttttttt ttttttatac gtgttttatt ttattttatt 12720
   tttaagtagt tttttttagt ttagttttaa tgtttttttt ttagttagta cgtattggga 12780
   tttagaagta cgagatatta aagtgtttag ggttattttt tgttttgaag tattttagtt 12840
   tagtaggaaa ataggtatgt agatgtaatt tatttttttt tttttttttt ttgaaacgga 12900
   atttcgtttt gtcgtttagg ttggtgtgta gtggcgtaat tttagtttat tgtaagtttt 12960
   ttttgttggg tttacgttat ttttttgttt tagtttttta agtagttggg attatagggg 13020
   ttcgttatta cgttcggtta atttttttgt atttttagta gagaagaggt tttatcgtgt 13080
   tagttaggat ggttatgatt ttttgatttc gtgattcgtt cgttttattt tttaaagtgt 13140
   tgggattata ggcgtgggtt attacgtttg ggttattttt tttttaaatt tgttttttat 13200
   atatttatta tattataata atttgttttt tttttttttt tttttttttt tttggagacg 13260
   gagttttatt ttgttgttta ggttggagtg tagtggtgcg gttttggtat attgtaattt 13320
   ttgttttttg ggtttaaatg atttttttgt tttagttttt tgagtagttg gaattatagg 13380
   tgtgtgttat tacgtttcgt taattttttg tatttttagt agagacgggt ttttattacg 13440
   ttagttagga tggttttgat tttttgattt ggtgatttgt ttatttcggt attttaaagt 13500
   gttgggatta gaggcgtgag ttattgtgtt cgatttgaga tgaggtttta ttatgttggt 13560
   taggttagtt ttgaagtttt gattttaagt gattcgttta ttttcgtttt taaaagtgtt 13620
   gagattatag gtgtgagtta tagtattcgg tttgtttttt tttttattta taggaattta 13680
   tatttatttt gttttattta ttagattagt agtttttaaa ttttagtgta tatagaaatt 13740
   atttggagag tttatttaat atgtaaattt taaagtttta tttttagagt gggttggtgg 13800
   ttttttcgtt ttttgttatt ggaatgtgga ttagggatta ggattagtag tattttttgt 13860
   aagttgataa aaatattaaa ttttgggttt ttattttgaa tgagaattag tattatgaag 13920
   atttttgggt atttattttt atattaaagt ttgagagtga gagtttagtt tagtattttt 13980
   taaatttgtt tgagatttat agtaggtaat gtattttatg tttgatattt attatattta 14040
   tatatattta tgtgaaatag aaattttata aaataatttt tatttttaag ggatttttat 14100
   taatattttt tattttattt tatatttttt ttttttttta atgtgggttt tgattattaa 14160
   attgattttt tattttatta agatatgtaa ttttgtaatt tattatttga aaaatattgt 14220
   tttagtgatt agattttagt tatattagtt tgggtagaga tgaattagaa attggtttat 14280
   ttatggtaga agttatattt atttagtacg ttttttttta tttttggggt ggaaggtagg 14340
   gaagaagaat aagtttttat aaataggagt atatattttt ataaatagga gtagttttcg 14400
   tagttttggg aaattgagga tgtttttttt tatttgggta attttttttt ggttagagat 14460
   attttcggtt agttttttgt attagttttt aggagggagg agttgtgtat ggagggtttg 14520
   tggaaaagag tttagttttt ttattgggat gtttttattg ggtaaggcga gggtgtgtat 14580
   ttatttttag agggtttaga tggttgtttt ttcggttttg tgatttgaag gtttatagga 14640
   atatttagaa attttttata gtagttttta tattatataa ggagtttttg gttttgatgg 14700
   atttttttgt agtttttata aaatatatgt ttttgatatt gtatagtaat taggttttgt 14760
   tttttgggga agcgggtata gttgatatag gatatttagt ttttcgtatt ttttttttta 14820
   tttataaagt ggtttaaatg attattttta aagatttaaa tgaaataaga tatgaagttt 14880
   gtattgtatt ttttttttgg tttaatttat aaaataaatt ttttaatatt tgtaatttaa 14940
   atattatatt tgagtttatt aaatatattt ttattatttt gatttttatg ttattataag 15000
   tagtttataa atttatttat gttaatatag atttgttagt ttaaggatat ttttttaggt 15060
   aatgttatat attgggtttg tttgtttttt ttagagatag agttttattt tgttgtttag 15120
   attggagtgt agtggtttaa ttataggtta tagtagtttc gaatttttgg gtttaaatga 15180
   tttttttatt ttagtttttt gagtagttgg aattaaaggt atgtattatt acgtttggta 15240
   aattttttta aaaaaatttt ttgtagagat agggttttat ttttttttag gttggtttta 15300
   aatttttggg tttaagtgat ttttttattt tggtttttta aagtgttaga attataggtg 15360
   taagttattg agtttgttta atgtgatata ttttaaaaga tttttatttt tattattttt 15420
   ttgtttttta aatttttata gtgagtatat gttaatttta taagtagagg taagtgaaat 15480
   aaaaaggttt tatttaattt tattatttaa atataattga atagttttat attttatttt 15540
   tagttaggtt ttatgtatat taatggagtt atagattatt ttttttaagg tcgggtttgg 15600
   tggtttatat ttgtaatttt cgtattttgg gaggttaaag taggcggatt atgaggttag 15660
   gagttcgaga ttagtttggt taatatggtg aaattttttt ttattaaaaa tataaaaatt 15720
   agtcgggtat ggtggtatat gtttgtaatt ttagttattc ggaagattga ggtaagagaa 15780
   ttgtttgaat ttaggaggtg gaggttatag tgagttgaga ttacgttatt gtattttaat 15840
   ttgggtaata gataaaaatt tcgttttaag aaaaaaaaaa taaaaaaaaa aaaataaaga 15900
   aagattattt ttttttttgt tgttggtaat ttcgttgttt ttaatatttt gttattataa 15960
   ataattttag gattaatata ttttatatag ttttaatttt agattatttt tttgtattat 16020
   atttttagaa gtagaaattt agggttaagg gattttgaat attttaaggt atttgatata 16080
   ttttgataaa tttttttttg taggtaataa attgtagttt tattagtata tttttaatat 16140
   attattatta gattttgaag agtatttggt tatttttttt ttaatttttg attaatttta 16200
   taggtgaata atattgaatt ttaaaatttg tttttatttt ttttagtata gttttagtat 16260
   tttataatat ttaaagtatt tagttttgtt tataaatata atataatata ttgatttttt 16320
   aaatgtatat tttattgtta attttttaga ttaaaatgat ttaaggtatt tataaaatat 16380
   agaatttatg tagaggtgtt atttttatgg agtaaatatt ggtcgttttt tttttttata 16440
   ggtaatataa attatagata tttgttttgg atgttttttt tttttgatta ggattttttt 16500
   aaaagggtaa aggttaagat tttttcggat tagtttttcg ttttattttt agtagtagat 16560
   ttattagaaa tagatttatt ttttatatta tagaattgtt ttcgtaggtt tttattttat 16620
   tggtattgat tattttattt tgaaagggtt taaatttgat ttttttggcg ataaaattat 16680
   tgtagaatat attaaaatgt gggattttat taaatatcgt ttgtatgagg tatagattta 16740
   ggggaaagtc gaggtaggaa aagagaatga ggagagttga ttttgtatta gtttagggaa 16800
   agaaatttat gtaaggagtt ttgtttattt tttggaagtt gaagggagtt tttatttaga 16860
   gtttgaggaa gagaattaga ttttagtaaa aggtaggtta aaataatttt ttattaaaaa 16920
   tgtattataa agtgttttta tgaggtgttt ttttttttaa ttttttaaag gtaattatta 16980
   ttttagtttg atatttattt gttgttttta tttttttttg tttattttat atggtttgtt 17040
   tatatacgat tttattgtaa ataattttat atataatttt ttatttgaga tttagaatag 17100
   ttttatggaa gtttatgtaa ttaattttat taggtaaatt taaggaattt gaggattaga 17160
   ggtaatgttt gattaaggtt agcgtagtta gttgagttgg gattgttagg tgtgaattgt 17220
   tgaaggttgt tattgtatgt tagtatagaa tttgaattta tttgggagtt cgttgtagga 17280
   attatttttt ttttggtttt tagtttttat taaaattaat tttagttatg tagttaagta 17340
   agtagggttt tagaagtgag tttagaatag ttttggtttt tatggaggta tttttttttt 17400
   ttcgtatttt gtagttttag ttttatttgg aagcgttttt tttcggatat tttcgttttc 17460
   gtttgtattt tcgtttaatt cgatttttaa atggtgtgaa ttttttttat tagagttgtt 17520
   tggggggatt aggaggtttg aaatcgtatg gtgtaggttg gttgggtgtt ttaggttggg 17580
   agtgatttcg tatagaacga agtgtaggtt ttttttcgtg aattggaatc gagtagggga 17640
   tttttttttt tggttggagg gtttgggatt attttttgat tgtttgttgg gtgagtaagg 17700
   taataattgg gaggttttta gttttttagg taatagagaa gtattcgtag ggggcggtgg 17760
   aattaaagtg ttaggtttat aatacgggtt attttcgttg tcgttattaa tgatttggtg 17820
   gtttagattt ttattgttgg tattcgcgta ttcgtggttg ttgtttagga agggcggtat 17880
   ttttttgggg acgtggggaa ttgggtatta cggtggtagt ttgttgtatt agggtagatc 17940
   gtagagaggt tttttttgta ggtttagatt cgggtttagt aagggaggcg ttattcggaa 18000
   ggggaagggg ggtatggcgg gggtagtaga cgttgcggtt tttagttgtc ggaaaggttg 18060
   gaagtttttt tttacggtgg ttaggttgtt tttgtagtgt ttataggacg ggaaaggcgt 18120
   gtagtacgcg gttaggtttt gcgggttgtt tttcggcggg tagtatattt tgttttgagg 18180
   tacggtttta tttggtcggg gtagagttat ttcgggatcg tacgttcggc ggttttgtag 18240
   aaaagcgggc gtcgttgagg atatcgcgcg ggagtttttc ggggttcgat tcgcgttttt 18300
   ttattagttt tttttatttt agttagtttt agatttaagg gaataggata aacgttttta 18360
   gttcgatttc gttagttcgt ttttataatt aagataaaac gaaaaaatag taatagaagt 18420
   aaatataaaa aagttttaaa cggaaatttg tcgggatttt gttttatttt aaatttcggg   18480
   ttagtgttgt tttttttttt tttttttaag tatttttttt ttaagttcgg tcgttttttt   18540
   atattcgttt ttcgttttcg gtttttaatt cggtttttgt aaaatttatt gaagtttttt   18600
   tttttttttt ttttttagtt ttataaaatt cgttggtagg gtttgtggga gtcgcggggt   18660
   tttgttaatt tcggggttgg gtcgggagtc gagcgtaggc ggtcggaggg taatggagta   18720
   agcgatagtt ttaggcgttt ttgggttttc ggttgggagg gaagagtcga ggattttggg   18780
   tcgtatcgcg tagatggaga cgcgttttta gagttttcgg gtaggtttag ggatttcgcg   18840
   atttgttttg gtttagcggt gtgatttcgc gggttttggc ggttttgatt gttcgtaggg   18900
   gaggggcgcg ttatttttgg ttgttttagg atgcgtcgtt tgaatttttt ttttttcgcg   18960
   ggtagcgttc gttatatttt tcgggttttt cggaaatttt aattatttta ttaggattat   19020
   tggggtttgg ggtagttttt cgggagtcgc gtggacgagt tttggttagg tgggagcgaa   19080
   gagtttcggc gattgttagt tttttcgttt tcgtatcgtc gggaaaggat ggcgttttaa   19140
   tagataggta gtaagtttat cgaggttgaa gaatgaagtt tttttagggt cggcgggttt   19200
   tgagattaat gagtttaata agaaaattaa gtagggttat gatttagaaa ttaaagaata   19260
   cggggttagg agaagaggtt agttacgata taggtagcgg ttcgtttttt ttcgattttt   19320
   taaaggcgtt ttttttttag tttttttcga ttttagggat ttgtaatttg ggatttaaag   19380
   ttttcgagag ggtagaacga ttatttaaaa cgaattgcgt tagcgagtgt tgtcgttttt   19440
   tttaacggtt ttggaagatt atcgtttcgg gttttaggcg cgttttgttt cgtgtttatt   19500
   ttcgatatta tttttaagag cgttatcgcg gagcgggcgt tttttggacg ttgcggggtt   19560
   attcgggttc ggtaggttat tcgggttcgg tagatttcgg gcgggttttg agggttgagt   19620
   taggtttaag agggcgggcg gggtcgcggg gtatcgatag gtcgagggta gggaattgat   19680
   tcggtttacg tagattttcg cgtcgttttt ttttcgttta tcgtagttat tggtcgattt   19740
   tcgattcgtt ttttgttttt tggggtcgtt tatttaattt attagttttt taaatttttt   19800
   ttgggatttt agtggcgagt attcgtcgtt ggttttttag ggtaggtagt attgtcgttt   19860
   taggcgtgcg gagtcgggga gaaaaaaatc gaatacgtgt gttatttagg gtttttagat   19920
   aagttttttc gcgcgtatat atattttgaa tcggagttta gaaggtcgga ttcgcgtttc   19980
   ggggtcgagt tttttatttt tcgtttttag ttcggaagat tttagtttgt tcgaaggcgg   20040
   cgt                                                                 20043
<210> 104
   <211> 20043
   <212> DNA
   <213> Artificial Sequence
<220>
   <223> chemically treated genomic DNA (Homo sapiens)
<400> 104
<210> 105
   <211> 7201
   <212> DNA
   <213> Artificial Sequence
<220>
   <223> chemically treated genomic DNA (Homo sapiens)
<400> 105
<210> 106
   <211> 7201
   <212> DNA,
   <213> Artificial Sequence
<220>
   <223> chemically treated genomic DNA (Homo sapiens)
<400> 106
   tattgggttt ttgagaagat agtgttaatt ttattgtttt ttgaaatgtt gtgtaaagtg      60
   tggtatttaa taatttgtat attgggtaat ttttatttgt aagtttaaat atatgtgata     120
   agaaaaagta tatgtatatt aggaatatat aaaatttttt aaaattatta tttttatatt     180
   agaaattttt ttatataaat attttaattt ttaaaaaaag tagtaaatgg ttataatgtt 240
   ttaagattgt agaaaattta gttttttgtt taagtagttt tttattgaga gatacgagtg 300
   atggtagatg aaggtttttt aagaatagat ttaatagaaa atgatagtgg tttatttatc 360
   gagagttttg ttttaatttg tttggttata aaaaggtttt ttgtatattt atttattgta 420
   aatgtgtatt tttggttgtt tttaattaaa tattttttgt gttttttagg ggttttttta 480
   aaggtttttt tgataaatgg tgtttttaat ttaggattcg atttgttagt aagtgtttgt 540
   tggaataatg agtggaatgg attatgttta agtggtagag ggtgtttggt gttttttttg 600
   ttgattttcg ttaagatatg tttttgagtt gttagggagt tcgtgttaat aatgggagag 660
   aaatttcgtc ggggtgggga gaacgcggat ttttttggga tagtttgatg attttgaagg 720
   ttatttttcg tggtttggat tttaggagtg tgaggtagta ttagggaatt ttttaatttc 780
   gatggtatta tgttttgttt tgtattgagt ttattttgga ttcgtgtttt ttttaagttt 840
   tagttggttt taggttttag ggtttgaatt gaggtggtgg tatttagtag gtattgttgg 900
   tagaggaggg tgtcgttaat ggggttatat ttgggttaaa ttaaaaattt tgaggataag 960
   ggatattgtt tataagttgt ggttacgttg atattagaag aaattagttt tatattttta 1020
   gattttgaat attgtatggt taaattaagg taggtgggta aaaaattata gaagtttttg 1080
   tttggtggtt ttattgggtt ggggttgtag gtatttttgt aggaattgta gttaggttta 1140
   ggtattatgc ggttgggtag aaatagagta gtagtttgag aagtttttaa tatttttttt 1200
   tttttatatt ttttttttag tataatgttt tttaattttt tattagtaaa ggtttttttt 1260
   ttttttattt tgttattaat tgggggaggt agagggaagg ttttttttat ataagttttc 1320
   gttggaatgg ggcgatagtt tataagaagt aaagattgta tatgaaagag attaatattt 1380
   acggaataaa aatgtggaaa tttagaaggt aatagtaata ttatagatta gtagaataat 1440
   tatttatagt tttattttat tgatattttt atatagatgt aaaagtatag attaggtttg 1500
   aaagagtttt ttgggagggt gaaagatatt tatattgtaa tttttttttg ttgttgtttt 1560
   tgtttttgtt gtttgttgtg gttgttatgg atttgtttgt ttgtttgttt taatagatat 1620
   attttattga ttaaaatttt aatatttttt ttaggttttt tacgtattaa gagataagat 1680
   tatttatttt gtattaatta atgttttatt attatttatg ttattttttt ttattattaa 1740
   aaaatatatt tatatttaga gttgatgtta tttttatttt ttaaagatgg tttattatgt 1800
   atatataata gttgtagaat aatttaagga gaattaggaa aaaagagaaa gaaagaaagt 1860
   taaaggagat tagcgagtag aaaaagaaaa aaaaggttta agaaaataga gaagtataga 1920
   aaattgaaaa gagttgttaa ttagttaaat atattttata gtgaataatt taattaggtt 1980
   tttttttttg atattattat aaaaaaaaat aaaataaaat aaaataaaat ttaaaaaagg 2040
   cggggagtat tttttgtttt atcgaaaata attgttttga aatatttttt ttaagtttta 2100
   attattttgg tcggatatta aaatggttga ataagtaatt attaaaatat aggttaagtt 2160
   atcgtacgaa taggagtaaa tttaaatata aagattagtg taatataaat agaatttttg 2220
   gaggtataaa atatttggag tattttatta tttttaagta tttatttttg ttatatttgt 2280
   ttgaagtatt tattgttata tttgtgttta tatagtgata aatttattat atttatttgt 2340
   aaataaaata aaatttgaaa attaatgttt atatttttat gataagtttt tgttaggaag 2400
   ttatttagtt gaatttatta ttttatttag tttttagatt ttataagttg ttttgtataa 2460
   ttttagttta ttaatgtggt tttattatta taaatttgtt tatttagttt gtaattaaat 2520
   ttatttttaa tttataagaa attattttat ggttagaagt atataatata tgataggata 2580
   tatataaatt ttaaaatttg tttagaatta ataaaattta gaagttatat aagaaaaaaa 2640
   tagaatttaa atgtttattt aaaaattttt ataggatata tataaatttg tttagaatta 2700
   ataaaattta gaagttatat aagaaaaaaa tagaatttaa atgtttattt aaaaattttt 2760
   gtttattttt aatttgaatt ttatgaagtg gtagtgatta aagtaaatat taattgtttt 2820
   ttatttaaat ttttaaaaat ttaaagagaa acggttaaaa atagttaata tattattgag 2880
   gttaataaat aaattttata tttaaataga gatgttttta aaaatgagta atattttaag 2940
   aaaaattaat gtatttttag gtttttacga aagtgagaat atggagatag aaagttagag 3000
   atttcgtatt gttcgtattt aatttttatt tcgtttgttg gattcgaaat tttttttaga 3060
   ttttataatt ttgattgtta aaaaatggta aaaatgaagt ttggaataat aggaattatt 3120
   taagtaattt aaaaaaaaaa aaaaaagtat aatgaaaaag agcggaattt gaggttgaat 3180
   ttttagatta aacggtttta ttggttgcga gtagttgtta aattttttat ttgattttat 3240
   ttatttagtt tttataatta agaaaagtgg acgtggttgt gttgttttgt tttgttttgt 3300
   ttaagttatt tatttttttt taagatattt gttttaggat tatttggtag tatatttttg 3360
   tatatttttt atacggggta aattttatat gtattttata ttaatcgatt aattattaag 3420
   tagataatta aataataggg attttttaat tggtggtgat agaaatttta ggaggtatgt 3480
   gagatttatt tttttaggtg gcggtttttt ttttagaaga aaattttttg gttttcgggt 3540
   tatgttaatt tttttcgtac gggtttagtt ttagcgtacg ggtttagcga atatttaaat 3600
   aaaatttttt taataaaaag tatttttttt ttagatagtt tttttataga ttaataaata 3660
   tattttgtgt tgattaatga gagttattta attttgttta aaaggttgaa ggataatttt 3720
   gttgttttat tttttaaaat gtttttggtt agtaaattgg gggttttgat ttgtttttgg 3780
   tatatagttt tgcgttcgaa attattttgt ttttcggaaa gttttttttt gttttgtaaa 3840
   tataagaaat attattaata atgttttgaa agaaaatggg ggcgagaagg gggaaggacg 3900
   ggatgttcgt aaatttttta gaaatttttt tttttttaaa tgtattgaat ttttttattt 3960
   cgtgtgaaaa ataattataa acgaattaat attagtattt tttaatttta gggtaaggag 4020
   ttggaatttt agttggtgag ttggattttt tgtttttcgt agtatagtta ttttagattt 4080
   taattatttt agatatacgt agatttatgg ttagaagtga ggggatattt cggagggaag 4140
   tgaaaaagtg aaattttttt taggggggta ttcgatttta agagatggtt gtagttaggt 4200
   tgggttttgg gagtttattt gtcggataga aggtgtttaa ggtttgttgt gaagtagtga 4260
   ggtcgggcgg attttttttt gtggtagggt aggtagggtg gggtgatagg ggaatgaaga 4320
   gatgagggtt ttttgggttc ggttgtaaat tttaaatttg cgttttgcgg atatttattt 4380
   tcgtttttgt agttatttat tttattagtt ggtgggattt tgggtttttt tacgttcgcg 4440
   ttttatttag tatatgttat agagaacgcg tgggaacgta gggtaggcga tgatttatgc 4500
   gggacgcggg aggagattaa gtttaggatt aggtgtttat ggcgtttagc gcgttttttt 4560
   gggttttcga gaggtttttt agtggggtta ttgtttttag tgtgtttggc gatttatttt 4620
   ttagtttttt ttcgttaaaa agtaaaattg aaagtttttt ttggttttga gtttgggagg 4680
   aggttttgtg aagtttcgtt cgttgaattt tttttgttta ggcgtatatg gaaataaata 4740
   taaaatcgaa ggtttaagag cgtttttcgg aaagaaagtt ttggtttttt tcgtagttgg 4800
   agtttttcga tgggaagtag tagagatttt ttttttgttt ttaggtagtt tgtagggata 4860
   gtagtttcgg attgatgatt ttgggttgta ggtatttaat tttttatgta ttttgtgttt 4920
   tatttataaa atgagtgttt tagatatttt aaacgtttag gtgatagttg aagtatcgag 4980
   cgtatgcgtg ttgcgtggtt tttttttttt attattatta gtatagtagt gacgagtttg 5040
   gagttgtacg tcgtgttgtg gattaattat ttttttttta agttttcgtt tttaatatta 5100
   cgaaaatgta ttttaaaatg taaaaaaata aaaatattga ttttttcgcg atgttttaaa 5160
   tgggtttaaa tatttttaag gtttttttgt ttgtttttta agataaaaat attttttaag 5220
   ttttttagag atgtttgttt tttttttttc gattttgttc gttagtgtaa gtagaaaagc 5280
   gtttttatta ttttttttag ggagtgttag gtatcgtaag gttttgggcg cggggggtgt 5340
   ggacggtcgt ttcgtttttt tttaattttt ttatttttat tgtggtttga gacgttcggg 5400
   tcgggcgggt acgtatttcg gtggtttgtt gttttcggag cgttatttgg gcggttatga 5460
   cgcgttgtcg ttttattatt agtaggtagt tggcgtattg gtagtcgcgt tagcgataga 5520
   agcgtttgtg gttttttagg taggatatta cgtcgtggtt gcggtagcgc gcgtatttgg 5580
   gcgtgcggtt tagtttgcgc ggtttcgcgt cgtcgttcgc gggagtgtag cgttttcggg 5640
   gatcggtgtc ggcgggtgag gtttgaggcg cgagcggcgg tttcggttgc ggtttttttc 5700
   gttgtttcgg ttggtcgggt atttcggggg acgcgtttgt tttttcgtcg tcgttttttt 5760
   ttttcgcgtt ttcgtttatt tcgtcgttat tttcgtcgtt ttcgtagttt tcgttcgtcg 5820
   gcggcgggtt gggttcgggg ggcgtggatc gcggcgtttc gtagacgttt ttttttagtt 5880
   ttaggttttc gatatcgatt ttttagtttt cggtcgcgga gttagtttgc gggtcggtta 5940
   tggcgttcgg ggtttcgttg gggtttgagt ttggcggttt ttattttggg gaaatataaa 6000
   gaaatttaaa gatttatcgg tcgtcgttag ggattatttt tgagttttcg gtttaaagtt 6060
   tttggtttgt tcgttttcgg tcgtttgagt tattttgttt ttattatatg ttttttattt 6120
   ttaggttata cgtgtttttt ttgtattttt agggttttta gggatttata atttaataaa 6180
   ggttttattt tattaatttt aagtgttttg tgagtatttg atttttaatc gtggtttttt 6240
   ttggttttat acgggttttt cggataagtt tttgggtaat ttatttatta tatatatata 6300
   aaatattgtg tttaaatagg tagttttagg ttatttgtcg gattttttta tatttaatta 6360
   gtatggttat gatgtatata aaattattcg tggaagaata gttttttttt gattataagt 6420
   ggttttttag atttattgaa gtattatttt atgtttttta aatgtttttt attagtttat 6480
   attattataa ggattttttt ttgggtttga agaggttttt ttattttttt ttttaaaata 6540
   tttaattgaa aattaaaatt ttgttgttat tagcgtttta taagggtaaa gagtataata 6600
   tagacgttcg tttagtttta atagtatttg aggtgtatag ggttgagtcg cgtgggtttt 6660
   ttgtatttag gttttggttt atatatttaa ggtaaggtat ttaagttata tagaaaaggt 6720
   gaaagatgat ttacgttttt tgtttttttt gatatttcga gcggtattat agtatttatt 6780
   aaaattttga ggttcgggtt ggtagtttag ttaaaagata tttagtttta ggatttggcg 6840
   ttagggttgt tggttgtgta tgtaaataga aatcggggtc gttattttta aatacgagat 6900
   cgggagttga tagttggagg ttcggaacga atttattgga tggtggaggg ggagggagaa 6960
   ggtaggtgga gggaaggagg gaggaagtta gttaagtttt tagcgattta gtttagtata 7020
   tttcgaattt tgtaaagttt tgttcggttt tttgtagttt cgggcgtttt attttgttga 7080
   ggtcgttttc ggtttttttt gatttaggtt gggcgggaaa ggtcgggtcg ttttcgtttg 7140
   gtttagatcg ttggtttatt cggcgggcgc gcgtagttgt atttagcgta gcgttattgg 7200
   t 7201
<210> 107
   <211> 42929
   <212> DNA
   <213> Artificial Sequence
<220>
   <223> chemically treated genomic DNA (Homo sapiens)
<400> 107
<210> 108
   <211> 42929
   <212> DNA
   <213> Artificial Sequence
<220>
   <223> chemically treated genomic DNA (Homo sapiens)
<400> 108
<210> 109
   <211> 4108
   <212> DNA
   <213> Artificial Sequence
<220>
   <223> chemically treated genomic DNA (Homo sapiens)
<400> 109
   tcggtcgttt atgttttagg tttttttttc gcggtgtcgg tgaattcgtt agtcgtttcg     60
   atgtatagta tgatgatgga gatcgatttg tattcgttcg gcggcgttta ggtttttacg    120
   aatttttcgg gtttcgtcgg ggcgggcggc ggcgggggcg gaggcggggg cggcggcggc    180
   ggcgggggcg ttaaggttaa ttaggatcgg gttaaacggt ttatgaacgt ttttatggtg    240
   tggtttcgcg ggtagcggcg taagatggtt taggagaatt ttaagatgta taattcggag    300
   attagtaagc gtttgggggt cgagtggaag gttatgttcg aggtcgagaa gcggtcgttt    360
   atcgacgagg ttaagcggtt gcgcgcgttg tatatgaagg agtattcgga ttataagtat    420
   cggtcgcgtc gtaagattaa gacgttgttt aagaaggata agtattcgtt ggtcggcggg    480
   tttttggcgg tcggcgcggg tggcggcggc gcggttgtgg ttatgggcgt gggcgtgggc    540
   gtgggcgcgg cggtcgtggg ttagcgtttg gagagtttag gcggcgcggc gggcggcggt    600
   tacgcgtacg ttaacggttg ggttaacggc gtttatttcg gttcggtggc ggcggcggcg    660
   gcggtcgcgg ttatgatgta ggaggcgtag ttggtttacg ggtagtattc gggcgcgggc    720
   ggcgcgtatt cgtacgcgta tttcgcgtat tcgtattcgt attattcgta cgcgtattcg    780
   tataattcgt agtttatgta tcgttacgat atgggcgcgt tgtagtatag ttttattttt    840
   aattcgtagg gttatatgag cgcgtcgttt tcgggttacg gcggtttttt ttacggcgtc    900
   gcggtcgtcg tcgtcgtcgt tgcgggcggc gcgtattaga attcggtcgt ggcggcggcg    960
   gcggcggcgg cggtcgcgtc gtcgggcgtt ttgggcgcgt tgggtttttt ggtgaagtcg   1020
   gagtttagcg gtagttcgtt cgttttagcg tattcgcggg cgtcgtgttt cggggatttg   1080
   cgcgagatga ttagtatgta tttgttcgtc ggcgaggggg gcgattcggc ggcggtagta   1140
   gcggtcgcgg cgtagagtcg gttgtattcg ttgtcgtagt attattaggg cgcgggcgcg   1200
   ggcgtgaacg gtacggtgtt tttgacgtat atttagcgtt ttcgggacgt cggggatttt   1260
   gcggcggcga tttacgagtt cgcggttcgc gttcggtttt cgtttcgttt cggcgcggcg   1320
   tggtttttgt atagacgttt ttatattttt gttaaaagga aaatattgga gacgaacgtc   1380
   gggtgacgcg tgttttttat ttattttttt cggagatttt ggcgatcgtc gggcgttgat   1440
   attagatttg ggttttagat tgaatttcgg tgtttttttg agattttttg tatagtattt   1500
   attatttacg gaggaagcgg aaagcgtttt ttttgttcga ggggataaaa aagttaaaac   1560
   gaggcgagag gcgaagttta tttttgtata tcggtcggcg cgtttatttt ttttcgcgtt   1620
   gttttcggac ggcgtcgatc gtcggagttt aagtgacgcg gagttcgtcg tatttgttat   1680
   aaatgtagta aggtaggttt aagtatttat aagttttttg tagttgttat cgtttttttg   1740
   ggttggtttg ttaatttata taaagagatt attattatta tttttttttt tagacggcgg   1800
   agttatattt tgggttttgt aaaattttat gtatttgagt atttttattt ttttttttgg   1860
   gttttgtatt attttttgta aatgtattgt gaaaaatttt attttcggcg ttgtaatgcg   1920
   gggaggagaa gttagattat gtatatagtt ttttaaaaag tttttttttt aaaaacgaaa   1980
   aaagattttt tatttaaaat gtttcgagtt aataaattta agagatagag tttatttttt   2040
   ttataaattt gtaatatgtt atttttatgt gtatgtttta tgagtttaaa atgtaatgag   2100
   gaaatttgat agggaaatta tttgtatgaa ttaaaagtaa gggaatttcg gggaatggga   2160
   ggataggatt ttttaaggaa ttttttttaa tgaaagagaa ggaagttaaa atttataggt   2220
   tattttgtag agttgagtgt taatacgggt cgagaaataa aagtattttt tgtttcggtt   2280
   gttttattgc ggacggttgg ggttgttgcg cgttattttg ttgtaagcgg ggcgtttttt   2340
   atttggttgg gggtttgcgt tatagtttgg tttagaggag ggaggaggaa gggaagattt   2400
   tagtggtggg attttggatt aggttatgga tgaaggataa agattagggt aggttacggg   2460
   ttttttaatt ttttagtaat taagatttcg agtagaattt atttaaatgt gttttaagga   2520
   aatataatcg ttgaattaaa acgtattgta gtcgagtttt tttcgtttat tttttgtttt    2580
   tttttttggt tttttttttt tgggaaaacg ggtaaaataa ttgtgttgga tttttatata    2640
   tatagaaata tcgattatta ttttttttcg cgtgaattgg gatgtaagtt gttaatcgat    2700
   gtgaacgtaa aatgttttgt ttattatttt tgacgagatt ttgaggttgt ttgatgtttt    2760
   aaatttttta attatattat tttttaggtg tttattggta tattgtagtt ttttttttga    2820
   aatttaaaaa tttttgtaaa attttgtttt taagtaattt gatagtatta aatattgtat    2880
   ttaaaaatta tattgtagta aatatattta aaaattaatt ataacgttaa gatgaaatta    2940
   tatttttgga aaaaaaaaat atttgaagtt tagatggaaa tacgtttatt ttagtagttt    3000
   taggtttttt ttcgtttttt taatattttt ttttgttttg gagtatggat tgttcgttta    3060
   aaagtgagtt tatgttataa gtttaatgag aatcgaattt agtttgtatt cgagaatagt    3120
   tttaagtata atgttgattt gataattgac gtgtaatttg ggaagttatt ttgataattt    3180
   tgtttaaatt atttattcgt taaagtgatt ataaaaaagt ttaagaatga tgtttattgt    3240
   tttttaataa gataataaat tttttttttt tttttttttt ttttattttt atttttttta    3300
   gttatttgat tttttttgaa gtagttgttt ttggaagagt ttgtgcgttt atggatggtt    3360
   gagtattatt acgatttagt tcgggataag ggttttttta gtttttttcg ggagatgatt    3420
   tgggaaattt tataatgttt gttttgttaa tttatcggga ttttgagggt ttaatgggat    3480
   tttgagggtt ttttttgaaa tatataaatt taaaggattt tttttgaggt tttttgattg    3540
   acgtttattt ttagtttggt ttttgtgttt ttttgtgtgt attttggagt ttttgtgttt    3600
   aattgttggt atttaggttt tggtttaaga ttaggatgtg ggttttattt tagaggtata    3660
   gattatgaaa agttgagtta gtgcgttcgg gacgttaggt aagtagtttt tatagtttgg    3720
   tattttattg taggtgtttc gtgtatagtt agttgaaata gttaatgtta ggtgttttaa    3780
   ttattttatt tttttgtttt gttgattaga ataatataga aaaaagtaaa tataaatttt    3840
   taatgatttt atttaaaaat attatagggt gggggtaagg aaattagttg agatttattt    3900
   taggattgag attttatttt ttttttttcg tttttagtag tgtcgtttta atttaaatta    3960
   gtggagaaaa gattatagta ggttttgagt cgattgtgaa ttcggtgttt ggttaaggta    4020
   atatttatcg tatttacgga gtgaaatatt atatgatgat agttattata ttatatgacg    4080
   attttattta ttttttaaat tatagggt                                      4108
<210> 110
   <211> 4108
   <212> DNA
   <213> Artificial Sequence
<220>
   <223> chemically treated genomic DNA (Homo sapiens)
<400> 110
   attttgtgat ttgggaagtg aatgaagtcg ttatataata taataattat tattatatag      60
   tattttattt cgtgaatacg atgagtgtta ttttggttaa gtatcgaatt tatagtcggt     120
   ttagggttta ttgtaatttt ttttttatta atttgaattg gagcgatatt gttgggggcg     180
   gggaaggggg gatagaattt taattttgag atgaatttta gttaattttt ttgtttttat     240
   tttgtgatat ttttaaatgg agttattaaa aatttatatt tgtttttttt tgtgttgttt     300
   taattaataa aataaggaaa taaggtggtt ggagtatttg gtattggtta ttttagttga     360
   ttgtgtacga agtatttgta ataagatgtt aaattgtaaa agttgtttgt ttggcgtttc     420
   gggcgtatta atttagtttt ttatagtttg tgtttttaaa gtggggttta tattttaatt     480
   ttgagttaag atttagatgt taataattgg atatagaaat tttagggtat atatagggga     540
   gtataggggt tagattgaga gtggacgtta gttaaagaat tttagagaga gttttttaag     600
   tttgtatatt ttagagaaaa tttttaaggt tttattggat ttttaaggtt tcggtgagtt     660
   aatagaataa gtattataaa attttttaaa ttatttttcg gagaggattg gggaggtttt     720
   tatttcggat taagtcgtag tggtgtttag ttatttatgg gcgtatagat ttttttagaa     780
   ataattgttt tagaaaggat taaatagtta aaagaaataa aaataaagaa aaagaaaaga     840
   gggggggggt ttattatttt gttagaaagt agtggatatt atttttgaat ttttttgtaa     900
   ttattttaac gaatgagtgg tttaagtaaa attattaaaa tgatttttta aattatacgt     960
   taattgttag attagtatta tatttaaagt tattttcgaa tgtaggttga attcggtttt    1020
   tattaaattt atagtatagg tttatttttg gacggatagt ttatatttta ggataaggaa    1080
   gggtgttgag aaagcgaggg gaaatttaag gttgttgaaa taaacgtatt tttatttggg    1140
   ttttaagtgt tttttttttt taaaaatata attttatttt aacgttgtga ttaattttta    1200
   aatgtatttg ttatagtata atttttaaat gtaatattta atgttgttag attatttgaa    1260
   gataaagttt tatagaaatt tttaaatttt aaaaaaaaaa ttgtaatgta ttaataaata    1320
   tttagaaaat aatataatta aaaaatttaa agtattaaat aattttaaga tttcgttagg    1380
   aataatgaat aaggtatttt gcgtttatat cggttagtaa tttgtatttt agtttacgcg    1440
   ggggagggtg atggtcgata tttttgtgtg tgtgagaatt tagtataatt attttgttcg    1500
   tttttttaag agaaagaagt taggggaggg ggtagaggat ggacggaggg ggttcggttg    1560
   tagtacgttt tggtttagcg attgtgtttt tttgaaatat atttagataa attttgttcg    1620
   aaattttaat tgttggggaa ttgggaaatt cgtgatttgt tttggttttt gttttttatt    1680
   tatggtttgg tttagggttt tattattggg gttttttttt tttttttttt tttttggatt    1740
   aaattgtggc gtagattttt agttaggtgg aaggcgtttc gtttgtagta aggtaacgcg    1800
   tagtagtttt agtcgttcgt agtgaaatag tcggagtaga agatattttt atttttcggt    1860
   tcgtattaat atttagtttt ataaaataat ttataggttt taattttttt tttttttatt    1920
   gaaaaaggtt ttttgaaaaa ttttgttttt ttatttttcg gggttttttt atttttagtt    1980
   tatatagata atttttttgt tagatttttt tattgtattt tgaatttata aaatatgtat    2040
   ataaaaatag tatgttataa atttatggag aaaatgggtt ttgtttttta aatttgttga    2100
   ttcgaaatat tttgggtggg gggttttttt tcgtttttag aagaaaggtt ttttagaaaa    2160
   ttatgtatat aatttgattt ttttttttcg tattgtaacg tcgaaaataa aattttttat    2220
   aatgtattta taagaaataa tataaaattt aaaaaaaaaa atggaaatgt ttagatatat    2280
   aaagttttat aaaatttaga atataatttc gtcgtttgaa ggagggggtg gtggtggtaa    2340
   tttttttgta taaattaata aattaattta aaagagcggt aataattata aaaaatttgt    2400
   aagtgtttgg atttgtttta ttatatttat aataaatgcg acgagtttcg cgttatttgg    2460
   gtttcggcgg tcggcgtcgt tcggaagtaa cgcggaggaa agtgagcgcg tcggtcggta    2520
   tataaaagtg ggtttcgttt ttcgtttcgt tttgattttt ttgttttttc gagtaaagaa    2580
   aacgtttttc gtttttttcg taggtgataa atattgtata aaaagtttta agaaaatatc    2640
   gaagtttagt ttaaaattta agtttggtgt tagcgttcgg cggtcgttag ggttttcggg    2700
   gaaggtgagt gggggatacg cgttattcgg cgttcgtttt tagtattttt tttttgataa    2760
   gaatgtggga acgtttgtat aaaagttacg tcgcgtcggg gcggggcggg agtcgggcgc    2820
   gggtcgcgag ttcgtgggtc gtcgtcgtag agttttcggc gtttcgaagg cgttagatgt    2880
   gcgttagggg tatcgtgtcg tttacgttcg cgttcgcgtt ttggtagtgt tgcggtagcg    2940
   agtgtagtcg gttttgcgtc gcggtcgttg ttgtcgtcgt cgggtcgttt ttttcgtcgg    3000
   cgggtaagta tatgttgatt atttcgcgta ggttttcggg gtacggcgtt cgcgagtgcg    3060
   ttggggcggg cgggttgtcg ttgggtttcg attttattag agagtttagc gcgtttaggg    3120
   cgttcgacga cgcggtcgtc gtcgtcgtcg tcgtcgttac ggtcgagttt tggtgcgcgt    3180
   cgttcgtagc ggcggcggcg gcggtcgcgg cgtcgtaggg gaggtcgtcg tagttcgagg    3240
   gcgacgcgtt tatgtagttt tgcgagttgg agatggggtt gtattgtagc gcgtttatgt    3300
   cgtagcggtg tatgggttgc gggttgtgcg ggtgcgcgtg cgggtggtgc gggtgcgggt    3360
   gcgcggggtg cgcgtgcggg tgcgcgtcgt tcgcgttcgg gtgttgttcg taggttagtt    3420
   gcgttttttg tattatggtc gcggtcgtcg tcgtcgtcgt tatcgagtcg gggtaggcgt    3480
   cgttggttta gtcgttgacg tgcgcgtagt cgtcgttcgt cgcgtcgttt gggtttttta    3540
   ggcgttggtt tacggtcgtc gcgtttacgt ttacgtttac gtttatggtt atagtcgcgt    3600
   cgtcgttatt cgcgtcggtc gttaggagtt cgtcggttag cgagtatttg ttttttttga    3660
   gtagcgtttt ggttttgcgg cgcggtcggt atttgtaatt cgggtgtttt tttatgtgta    3720
   gcgcgcgtag tcgtttggtt tcgtcgatga acggtcgttt ttcggtttcg gatatgattt    3780
   tttattcggt ttttaggcgt ttgttgattt tcgagttgtg tattttgggg tttttttggg    3840
   ttattttgcg tcgttgttcg cgggattata ttatgaaggc gtttatgggt cgtttgattc    3900
   ggttttggtt ggttttggcg ttttcgtcgt cgtcgtcgtt ttcgttttcg ttttcgtcgt    3960
   cgttcgtttc ggcggggttc gagaggttcg tgggggtttg ggcgtcgtcg ggcgagtgta    4020
   ggtcggtttt tattattatg ttgtatatcg gggcggttgg cgggtttatc ggtatcgcga    4080
   ggagagggtt tggagtatag acggtcgg                                        4108
<210> 111
   <211> 29262
   <212> DNA
   <213> Artificial Sequence
<220>
   <223> chemically treated genomic DNA (Homo sapiens)
<400> 111
<210> 112
   <211> 29262
   <212> DNA
   <213> Artificial Sequence
<220>
   <223> chemically treated genomic DNA (Homo sapiens)
<400> 112
   atggttttta gtttgttgtt agggtttaat gatattttgt tggtgttgtt gttgagtgtt      60
   ttttagtttg atatgttgag ttagttgtgg ttagggtatg ttaattttaa atttaattag     120
   gtgggttagg tgatttttta tggtattttt attgtgttgt tggtgattga tgggtaagag     180
   tgtttgtgtt tggtgtagat ttttaatatt ttgtttaaga attttagtta taatgagatt     240
   tataattgtt gtgtggtatt gggtattatg tgtgtgtagt gtatgttggt gtaattggag     300
   attttgtggt gtgttggggt tatgtttatt ttattgtgtt gttgtggtat gattattaaa     360
   tgtgaggttg agtgtttgtg taagttgttt ttgggtgaaa ataggttgtt taagttgtta     420
   gataattttg tttttgatgt gttatatgag tgtgtttggg gttgttgtgg tagttttatt     480
   tttgtgtgtt ataatagttt gtgtgttaag tgtattaaat gtagttattg taatatgtat     540
   tttttgttta ataagtttat tttttatttt tattgtatgt ttgatgttaa gtatatttag     600
   ttagatgtag ttaattttaa tttgtggtgt tgttatttta agtttattga taagagtttt     660
   taggatgagt tggtttttgt ttgggaggat gttaaggtta tgtttaatgg tggtagttgt     720
   aagtgtgtat tgttttagtt gggtgtgtat tttgtttgtt atttgtttag ttttgtgaag     780
   gtggttgttg tggttgttgt ggttgtggtg gttggaggtg ggggtttgtt gggtttttat      840
   ttgttgggtg tgtttttgtt gttgttgttg ttattgttgt ttttggtaga gttggttggt     900
   gttttgtatg tttattataa gtggttgtgt tttgatgatg atgatgattt tttgtaggag     960
   gttgttgtag tggttgttgt tagttttttg gttgtagttg ttagtttttt tgtggttgtt     1020
   gttttgggtg gtgtggggat tggtgggggt ggtgttgggg gtggttgtgt ggttggtgtg    1080
   ggtgtgggtg tgggtgtggg ggtgggtgtt ggggtagggg ttaaaggttt gtgtagttat    1140
   ttagttattt tggtgtttag taagggtttg tttgggggtg ttttgtagaa gtttttgggt    1200
   tgtggtgggt tttttttgta tttttatatt ttttttgttg tggttgttgt ttttagtttg      1260
   tgttataaga aagaggatgt gggtgttgtt gttgaggttt tggggggtgt gggtgtaggt    1320
   ggtgtgggtg tggtgtttaa ggttggtttg tttggttttt tttggtttgt gggttgtaag    1380
   gatgtttttt atttgttttt ttgtatgttt tggttgttgt ggatttttgg tgggtttttg    1440
   gtgtttattt atttgtagtt tttgttttag ttgtttttgg tgttaggttg tgtgttaggt    1500
   gaaagtttgg ttttgttgtg ttaggttttt ttggatttgg ttgagttagg tggtgttgtt    1560
   gggagtgttg aggttgtgtt tttgttgggg tagtttttgt aggtagtggt taatggtttg    1620
   ggttttggtt tatttttttt tgttgggggt gttggttttt gtgatgtgtt ttttgagttg    1680
   tttttgggtg gtagtggtgg ggattgtagt gtgggtttta tgttgtttgt ggattttgtg    1740
   gtagttgttg gggtaggggt tgtggttgtt gggtttggtt ttgtgggttt ttgggttttg    1800
   gtgttttatt atttgtattt tttggagggg tgtaaagtgg gtggtggtag ttattattat    1860
   tttagtgttt tttggttagt gggtggtaag gatgatgtgg agagtttggt taagttgtat    1920
   ggggtgttgg tgggtgtgtt ttatttggtt tagatgtatt tttattatta ttattatttt    1980
   tattattatt attattatta ttattttttg tagttgttgt tgttgttttt gttgttgttt    2040
   ttgtagtttg atgagttggg ttttgagtgt tattatttgg ttttgttgtt gttgttgttg    2100
   ttgtttttgt tgtttttttt ggtttagtat ttgtattatt gaggtttttt gttttttggg    2160
   ggaattagtt gttgttattt tagtgaggat agttttgagg atgaggatga tgaggaagaa    2220
   gagtaggagg tggatgtgga gggttataag ttttttgagg gtgaggaaga ggaggaaggt    2280
   tgagattttg atgatgatga ggaagaggat gaggagatgg aggttttatt tggtgatttt    2340
   ttagttgggg gtggttggtt tttttagggt tgagggttgt tggagaaggg gagtagttgg    2400
   gattgtgtgt tggttgttgt gggtgtgttt ttgtttggtt tgaatttttt taggttgttg    2460
   taggaagatg ggaaatttgg ggattttggt ttggatttgt ttttgttttt gttgttgttt    2520
   ttggtttttt agaaggtgag tggtggtggt agtagtagtt tgggtagttt agtttattat    2580
   ttattattgg aggagtagtt tttttataaa gatgtaaata ttttttttag gtttttttaa    2640
   gttaattatt attatttaaa tgtattttta ggttgaattg gttatatatg tgtaggaaag    2700
   atgaattatt agattttttt atagaaatga aatatttagt gtgtttagga gggttttttt    2760
   tttgttagtg gttttttgta aaaatgtggt ttttggtttt ttttttgata atgtttttta    2820
   aaggttttgg ggttgttttt tttttttata ttttagttgg ttgtttgtgg aagttggaaa    2880
   agatttttta attgatttgg tttttttttt aatttaataa ttttttttgt tttttaattg    2940
   agtatatttg tttttttaaa aggagtaaga ttataatatt ggagtagttt gaattatttt    3000
   attttttttt ttttttatag tttagtattt tttttttatt atgattaaaa gtaaaaaatg    3060
   tattggtttt aaaagttaaa tgtagtttta attgttttaa ttagttttag ttagaatgga    3120
   tataattgtt gtgtttagtg aaataaaaga taggattttt agtatagatt ttttttttgg    3180
   aaaagtatta attgaaaagt attatttggg atttttaaaa gattaaagtt aaatttaaat    3240
   gattgaatat ttagtgttta tttatgtgtg tatagaaaat attttggagg attttagggt    3300
   gaagtgaatt tgttttaatg aagtgtagat ttgatagata atatggtgtg tttatttata    3360
   gaagtatatg tttatgtttt tgtaaataat atagtatata tttgattaat ttgtattatt    3420
   ttttaaagga taatagtgta gatatatgta gttatatata tgtatttaag taaaaaagta    3480
   tttaaagaat aattgttaaa aaatgatgtt ttttgaggtt ttatggagtt atttttattg    3540
   atgttttttt ttgttgattg ttgatattag tatatggttt gatttgggta ttgatttgta    3600
   tgtttaattt ttttttatat agagttagaa aattaaggaa aataattaag taattgtatt    3660
   tataaaggat gataatagtt ttttaggtaa aaaaaaaaaa aaaaattagt tgttattttt    3720
   tttgtttgta tttaattatt tttgtgagat ttatgtttag tttttttatt tatttattat    3780
   tattattttg tttttaatta ttgatttgtg ggtttttttt tggttttgtt tttgaatatt    3840
   agagtttata ttttaaagat taatattttt atagtttgtt aatgatgata gttttttaaa    3900
   taatagtgtt attttttata attttagtat atttttttgt atttgaatga tgtttgtttt    3960
   ttaaatatat atagttatat aaatatatgt taaaattttt agatattttt gttttttttg    4020
   aatagttgtt atatttttgg agaatattta gagttttttg attgtttttt tttggagtta    4080
   atagttttta aatttagata taataagtag tatttttttt gtaaaaaaaa aaaaattgga    4140
   tttttgtgtg ttttttgtta gttggattat ttgtagattt tgtgttttgt tttggtttta    4200
   gataagaata aggagtatag tttttttatt atagattttg atgtttttgg aggagatttt    4260
   tggagagaaa gattaggtag gttgggaata aataagagga atgaatagtt agatgatagg    4320
   gagaggggtt gaaatttata attaaaagat tgaatattta gtataagggt aaaaataaaa    4380
   aataattttt agtagatata attaagaaaa atataaatgg ggaagtagtg atattaatag    4440
   taataagatt attaattgtt gggtatattt tagaagaaaa tttagaaata tattaggtta    4500
   tataatgaaa taggttggaa aagtataaaa tgataataat tgaaatgttt ttttagaaat    4560
   tttttattaa aaaggattat agtttattgt ttttaatatt tgttagaaag atattaaagg    4620
   tgttttatga tatttatgtt aatatttata ttatttttat gataatgatt tttatataaa    4680
   atgtataata tatttataaa aattatatta tataaattaa atgaaattat tttttattga    4740
   ttgttaaatg tttttaaggg gtttgggaaa gatgtgatta gaagttttga tattaagttg    4800
   tttattgtag tgttttaagg agagggtttt gttttttggg aaaaaggaat ttaatttttt    4860
   atttatgtaa tgtaaattgt tttggttttg attatttatg gttaattgat tgttaaatga    4920
   ggttgttatt ttgaggtaat gtttgtaaat ttgtttgtta aatgagatag agagagtaag    4980
   ttaggaagtg agggagagag agagagtggt gtagagagta gaaataaaga aaataggatt    5040
   ttaaaggaat tttatataat ggtaaagtat aatgaaaaag gtttttaaat ttttgttttt    5100
   atttttttat aaggaaattt tttaatattt ttaatggtaa aaggtaaata tttattttaa    5160
   tgaaataaaa tgttttatgt attatgggaa gaaagtttta tatattatta gatattttta    5220
   aattatttta tgaatatggt ataagtgttg attaatattt gattttggtt gatgatgttg    5280
   aggagttatt aataataatt ttgtagtata ttattggtta gtggttataa gtttatatat    5340
   aatatataat attattattt tgtgtattat ggtattattt gtttgttagt atattttgaa    5400
   tgtttttatt attattattt ttatatatta aagttttata ttttttgaaa atttataaaa    5460
   ttattttttg taaatttgtt ttaagtgttt aatgtggtaa aattattaaa tgtagatttt    5520
   tttgttatat tttaaagtta taaaggtaat gatatttttt aggaattatt ttttttttag    5580
   tatgatattt tataaaattg aggtttgaaa tggaaagtat tgttattaag gaatttttaa    5640
   tggatttgta tgtatttata taaaaaaggg tttataattt tatttaattg attttgtaat    5700
   attttgttat tttttagata tttttagata gttagttgtt tttgtttgtt tgatatgtgt    5760
   agaaattaat agttattgga aataatatat ttatgaatat gaaaaaataa ttttagaaat    5820
   attttttagt tttagattaa atgatattta gtagttttgt atatttaaaa atagatttaa    5880
   aagtagttta atttttttta tgtttggaaa ttatagatgg gaagtttata tgtattttag     5940
   tgaatgtaaa ttatttttgg ttgttttagt tgttgaagtg gattggagat ttttgttagg     6000
   tttgttttgt atgattaatg agtatttaga gaatttttat ttttttttat taagatagat     6060
   aaagtttaga aagaaagttt aaaagtaatg ttatatttta gtggtattgt taagaattta     6120
   atttggaagg attattattt tgttttgttt tattttatgt atgattttat tttgaaagta     6180
   agtttttgta aagtttaaag ttgaataaaa taagaaaatt tttatgtagt aatggttgtg     6240
   tattttttaa ataatatgat ttatttagag gtaggatgtg aaatttgttt tttatttatt     6300
   agtattaaga taagttttag agttgtttgt taaaattagt gtattgtaat ttatatatat     6360
   tagttttaat ttgtttttga ttttttatat ttggagattt ttattttgga gttggttatt     6420
   ggtaatatta aagtttaaat tattatatat atgtatttaa tataaaataa tatatttaga     6480
   tagatattaa attttatttt aattttgaat tttgtttatt tattagataa atattaattt     6540
   ttataagttt atttttgttg aattttattt gggtaaaaat ttttgtaaat atgttataaa     6600
   agatggaata tgtgtaatag ttttttaagg ggtaataatg atttatataa aattatggga     6660
   taatttaatg tttaaaatat tttttataaa tttaatgaga aaatttaaat tttatagttt     6720
   gaaatttatt tttaggaatt ttaatttttt atattgtatt taatagttaa tatttataat     6780
   ttttttaaga agtttttagt taatatttta ataattatgt atatttatat ttataagtaa     6840
   tttattaata aatatatttg aataaattaa gttttttata gagttatatt ttttttattt     6900
   tatgtatatt aatagttttt gtatatttgt taaaaattga attttttttt tttatttttt     6960
   tttgatttgg atggaatttg aaaataaatt tttggtttta agaggggttt gtttttttta     7020
   tgtttattga tttagtaaat tttaataagt tattagaatt ttaagaatga tagttaagta     7080
   gataaaaatt atggtattta ttgttgtttg atattaagat ggaaatataa atgttaaaag     7140
   gaagtaagta aaagtatagt taggattggt gtttatataa aaattttata gtgtttggag     7200
   tttagggtag tgtggttaat agttatattg gtaataatat gtagtgatat tattgaaata     7260
   ttgttagttt ttattttata ataattaatt tgatatagat ttttagagaa atttgtttta     7320
   tattgatttt tttatattat agttttattt ttatattatt ttgggaagta tgtagtagga     7380
   ggttatataa ttttattatt ttgtattgaa tgggagatag gtgaaatatg gaagggataa     7440
   aaatatatta ttaaaataag taaattttag gtgaaggtta tattttgtta attttatttt     7500
   ggaattttgg gttataagaa ttttattgtt atgggttttt tttttgtttg tttgtttgtt     7560
   ttttgttttt tgtttagtat gtggaaatgt tttaaatatt agtaagtgtt taaagggttt     7620
   ggattgtgtg ggatttaatt tggtgtattg ttagttgtta ttgtttttag aaaagattag     7680
   tttgtggttt ttagattgaa atatatattt gagaaaattt tagattgggg tttaagtgat     7740
   ttatgttttg gtattaatgt atttatttgt ggttagattt tgagaattta tttatttggt     7800
   aaagtgttag aaatttagat gagttaaaga gattatgagt gtttgtgatt agttgttttg     7860
   gagtggtagt agttaaatgt tttatttagt attttttatt tattaatgaa gatatttttt     7920
   tttatgtaat aattgttatt tatatgatag tatttatttg tttatttatt tatttatttt     7980
   ataaatattt ttgaatgttt attgtataat aagtattttt ttagattttg ggaatttaga     8040
   aatagaaatt aatatgagtt tttgttttta tattgttttt aattaagagg aggagataga     8100
   ttatttaaga aggtagtatg agtattataa ttgtagttta tataaaaaat tatttgataa     8160
   gatttatata attttgtgtt tttttagtta atttaatgtt taatttaatt attattattg     8220
   gaaaatatta atattttatt tttatgttta tatagttata ataaaaaaag aatgaaatgt     8280
   ttttttaaat tgtttgataa gttggtatgt aatttttgat ttttatagga attttttatt     8340
   tttgtggtaa ttataaatat agttaattag aaaattttta tgattttttt ttgaataaag     8400
   atatagattt atatttaatt gattttttta aaaaatttag tttagtaata atattttgtt     8460
   ttagttgata ttttagttta ttattttaat taagtttagt ttttttttaa tttgttagtt     8520
   ttattagtaa ttattatttt ttattaattt tgaagagatt atatttttaa atgatttttt     8580
   taaaattttt ttgttaatgg ttattaattt tatatatagt agatataatt ttgtataatt     8640
   gagggattaa atatgaagtt tttaagtagt taaaattatt taaaagttag gagaattttt     8700
   atttatagat tttttaaaat tagatttaag ttagatagat aagggggatg gtagttttat     8760
   taaaatatta tttatttaaa aatataaatg atagtatttt taattgtaat tttagagtaa     8820
   ggttttgtat ttttattttg aataatttta gtgaagttaa gtgatttgaa attgaatgta     8880
   gtggggtaag ttggtaagat agtttaaagt tattgtaggt taaggaaatt aggtaaagtg     8940
   gagttatttt tattttttag ttttttttat tggagaaatg ttggtgttat tgtagtgtga     9000
   gtatggttgt atttttttat atgtatagaa gttttaaaat taatttgatt tttattggta     9060
   tgatttttta gtgtatttaa tatgttatgg atagattttt gagaatagta agaagtaatt     9120
   atatttttta tgaatatata gtagatgaag tattttgatt tattatttta ttttattttt     9180
   gaatatgttt atgatttttt taaaaaaaag taaaaaataa gaaaaatttt ttgaagtata     9240
   gattagtaga tttgagtttt aattttttta ttaggtgata ttaggtaaat ttttaaatta     9300
   ttttgttttt tagttaatgt atttataaaa tttgataaaa ttttttgttt ttagtttatt     9360
   agtaatattt agttatgtat aatgtatttt gaagtttttt aatatatgga tttaaaatta     9420
   aatagttttt ttattttaga tatttgttta tttttattta tgaatgtttt ttttttatta     9480
   atgattgtta aaaatataga atttaaggag atagagaagg gtaatgaaaa aatgtttagg     9540
   aaaaaaaatt aaatagtatt aatttataag attagtttta aaatttaaga gttttttaaa     9600
   tgaatttgag atgtattaat taaagatatt taatttattg atttaagatg taatgtgtat     9660
   gtattttgta ataaattttg attatgaatt gtaaagtttg ttataatttt tagtttatta     9720
   taattttatt ggtattagtt aattttgtat ttgttttttt tttttttatt aatggagtta     9780
   atatttagtt agtttatttt gtagtatatg ttttttagtg atattgattt tttttttttt 9840
   tttgttttgt gattttattg aagtgttaaa tattataggg gaagttattt aatgtagaat 9900
   ttaatattaa gaaattagaa gtttatattt tattgataga ggagaaaatt atttaatttt 9960
   gtttttttat ataattgatt ttttaatgat ttaatgtaaa gtgtttttaa aaattttttt 10020
   atgttatatg ttgtgttgta ttttggttat ttttaagtgt attagttata ttaattgtag 10080
   gttaattttg attttattta aattatattt ttaagagtat tagagataga gaaaataaaa 10140
   aatttagaaa attttattta gaagtagatt ataagattaa gattaatttt aaaatttaga 10200
   gtttatttgg aagttaaata tgtatatgtg gagtttttgt ttaatagtgg ttaggttttg 10260
   tgtttgttgt tgagtaatag gaggatagag ttttagttta gtataaaagg gtaagaaggt 10320
   gtattgggag tagtatgtaa agattgtttg tagttttgta tgtaggagat ggtttttttt 10380
   ttagggtgtg tttttttgta gtagaatata gatatagtta gtatatgagt ttgtataaat 10440
   tatggaaata tttggatatt tgattgaaat tataatattt ttttttattt agatgttaga 10500
   gtgtatgtgt attttatgta tagatatagt tttaaattaa aataatttta tttattaaat 10560
   tggttaagtg tagtggttta tgtttgtaat tttagtattt tgggaggttg aggtaggtgg 10620
   attgtttgag gttaggagtt taagattagt ttggttaata aggtaaaatt ttgtttatat 10680
   taaaaatata aaaattagtt aggtgtggtg gtagttattt gtaattttag ttatttggga 10740
   ggttgaggta ggagaattgt ttgaatttag gagatgaaga taatagtgag ttgaaattgt 10800
   attattgtat tttagtttgg gtgatagagt aagatttttt tttaaaaaaa aaaaaaaaaa 10860
   aaagaaaaga aaagaaaaaa attatttatt aaatttattt gtgagtgtgt aattaaataa 10920
   tatatttttt tttttttttt ttgtagattt tttttgtgga tagatatttt ttttttatta 10980
   aaaaaaatta aaataaaata ttttttttaa atatttttta tgaagtgtta ataatagtat 11040
   tgggaggata taatagtttt tttagtgata agtaattatt tagtttttgg ggttgttggt 11100
   tggggttatg tttttaagtg ttagttgttg gtttaaggga gaatttaaaa gggagatagt 11160
   taagataagg agtaggaaat taagtaggga tgaaatgatt gggggtagtt tggtatagtg 11220
   gtttatgttt ataattttag tattttggaa gaatgaaata ggaggatttt ttgagtttag 11280
   gagtttgaga ttagattggg taatataggt agatttataa aatattttta aaaattagtt 11340
   tggtatagtg gtatagttat tttggaggtt gaagtgggag gattgtatga gtttagaagg 11400
   ataggttgta gtaagttatg tttatattat tgtattttag tttgggtaat agagtaagat 11460
   tttgttttaa aaaaaaaaaa aaaagaaaaa gaaaagaaaa tggtggggag gagaaagaat 11520
   gtatttttat agaagggatg ggtgaatgtt atattttttg atttttattt aaatggtaaa 11580
   agttttatat tgtattttat atatatggta tattttatat gtgtatagga tataatattt 11640
   aaatgttttt atttattgaa tttagagttt agtttgagaa gagtgataat tgtttaatta 11700
   gttttttttt ttttatgttt attaatggaa taggttattt gattatttgt ttggttttta 11760
   tagttaaatg agttttaaaa atgtataata tttttgttgt tggaggttat agtagatata 11820
   tatgttaatt aatattttta ggttatggtt aaaatttagt gttgtggttt tgtttgtttt 11880
   tgaaagaaat ttttaaaatg ttagatgaga ataaaattta ttttttattg gagtattata 11940
   aattttttta gtaaattgaa tatataatta gtttaattta gttagaatat ataaatggat 12000
   taaaatgtgt tttttatata taggaataag ttagtgtttt aaaatttgtt atgataatta 12060
   aggatttaga atagtgttag ttggtatttt ttttataaag tagaatgaat taaaaagtat 12120
   aataagtatt tgtggaatgt atttgtattt ttttttaagg taggggtgat tgggattatt 12180
   tgtagggtag gatttttttg tttttagaaa gtatattttt tagttgagga agattagatt 12240
   aatattgtaa aatattagaa tattaggtat aaaggggtag aataaagata ataagtattt 12300
   agaattttag tttaggaagg gataaattag taagatttgt agaagttgaa aaagttttat 12360
   ggaagaaaaa tgttttagtt tggttaggtt gttataaaaa aaaaataata ttattgattg 12420
   ggtggtttag ataatatttg ttttttttag ttttggatgt tgggaagttt aagattaagg 12480
   tattggtaga tttagtgatt ggtgagggag tttttttttt ggtttataga tggttatttt 12540
   tttgttgtgt ttttatatgg tggaaaagta ggaagggagt tttttggggt tttttttata 12600
   agggtattaa ttttatttat aagtggtttg tttttttggt ttaattattt tttaaagttt 12660
   tttatttttt tttttaatat tattattttg ggggttagga tttaaatatg ttaatttggg 12720
   gataaggggg atataggtat ttaattttta ataagaaaag atttggatgg tattttgatt 12780
   ttggttgtat aagaaagaga ggagagggta ttttaggata gagaaatgat ataaggtagt 12840
   tgtaggtagt agatgtgaaa aataaatgtt tgttaggtaa taaatatagg atgggttagg 12900
   tgtagtgatt taggtgtata attttagtat tttgtgaggg tgagatggat ggattatatg 12960
   agtttagggg tttaagatta gtttgggtaa tatggtgaaa ttttgttttt ataaaaaaaa 13020
   tatagaaaaa aattagttag atatgatggt gtgtatttgt agttttagtt atttgggagg 13080
   ttgaggtgag aggattattt gagtttagga ggttgaggtt gtagtgagtt tggattgtgt 13140
   gagtgtgttt tagtttgggt ggtagagtga gaatttgttt taaaaaggga gataggatgg 13200
   tttagtaggt tggataagaa tgggagaatt tagagatttt gtggggagaa ttttagaatg 13260
   gagtttttga tggagattat agggagttat tgtaggtttt tgagtagagg tttgttatga 13320
   tatttaagag aaagtaatta ttttagtatt ttgaaagtta gtgttattga atttgttggt 13380
   tgtttttttt tttttttttt tttgtttgtt tgtgttgttt atatagttta tttttttttt 13440
   atttgttatt tattgtttat atttgtttta gtatgttatt ttttttattt atttttttta 13500
   tatttttagt ttttaatgat atgtgagggt agaattaaga gtgggttgga atatttattt 13560
   agaatgtgga attattgttg ttggaggttt ttggagagat taggaaagtt gatttttgat 13620
   attttaggtt ttttggtttt tagttgtatt tagtagtgat gtagtttttt tgatatggta 13680
   tgatgttgtt tttatttttt tggatgagta ggatttttag aaattatgta gattataatg 13740
   ttttgtaaat gtgtatttgg aaagttttgg tgtggatatt tttttaagta gtagtttttt 13800
   tttttttttt ttttttttgt atttaagtta gggttttatt ttgttattta ggttggagtg 13860
   tagtggttag tggtgtaatg atgatttatt ttagttttga tttttagggt ttaagtgatt 13920
   tttttattta gattagttgg aattagaaga gtgtattatt atttttagtt aatttttgtt 13980
   ttttttgtat agatagagtt ttgttatgtt gtgtagtttg gtattaaatt tttgggttta 14040
   agtatttttt tttggttttt tagagttttg ggattatagg tgtgagttat ggtattgggt 14100
   ttttagtagt agtttttagt tatttttatt tataggagat gttttatgtt ttagttattt 14160
   gttatttttt aattttaaat tagttttttt ttatagtttt agatatgttt gaggttgggt 14220
   ttttaagaaa tatgtttttt ttatgtataa tttttttttt tataatttta tattgttaga 14280
   attgttgtgt ttttttttat tttttttttt tttttttttt tttgagatgg agttttgttt 14340
   tgttatttag attagaatgt agtggtgtga ttttggttta ttgtaatttt tgttttttgg 14400
   gtttaagtga tttttttatt ttagtttttt aagtagttgg gattataggt gtttgttatt 14460
   atgtttagtt agtttttgta tttttagtag agatggggtt ttattatgtt ggttaggttg 14520
   gttttgaatt tttgatttta ggtgatttat ttgttttagt tttttaaagt gttgggatta 14580
   tatatatgag ttattatatt tggtttatta attttttttt taaaagagga atttattgtt 14640
   tattttggta gtatatatat taaaattgga ataatataga gaagattagt atggtttttg 14700
   tgtaaggata atatataaat ttttgaagta gtttatattt aaaaataaaa ggggtgaggg 14760
   aaatataggg gataaaaaag taagtatttt agggatgttt ttaaatttat ttattaattt 14820
   tgaaaaagta aaatgtagtt aatttgaagg aatttttaga aaaattggga gtttattgtt 14880
   aattaaatta tgtttattta atttagtatt agattgtttt tttttttaat tagatggaaa 14940
   ttattagtag taatagtgaa taaatatatt taggttttta gaggttattt ttaattatta 15000
   ttaataattt ggtttttata gattagtaat atatgaaagg taatagttta gtaaaagata 15060
   tataaaaggt agtggaagtt tttgtgtttt ttatttattt aatatttagt tattttaaaa 15120
   attttttgta agtgaggtat ttgaattttt taagtttaaa attggtagat tttatttaga 15180
   agatgtagta aaatatttgt ttgaaataga tgttaatgta aattgttaat aaaattttta 15240
   taaagttgat gaaaagataa gttttttatt aatattatta gtagatttgt ttgtgtttaa 15300
   tatagttttt atttattagt tgtttgttta gtggttttat attaatgtaa agttattaat 15360
   tatgaagtag gaaagaaatg tattttatat attatatttt aatttttaaa tttaattatt 15420
   taattgaaat ataatataag ataaatagtt tataaaatat attaatgagt taaaaggaat 15480
   ttggtttata atttgataag attatggttt ttagaattgt ttatttagga ataaagattt 15540
   tatttaaatt atgttttgag tggttgagaa agttgtagaa tgatagtgtt attatgtttt 15600
   gtagataaat gtagagatat atgtatgttt tttttttttt ttgtttagat ttaatattgt 15660
   atgttagtat ttttgaaatt tttatatttt taattggtta attgagattg tatataatat 15720
   gtatgtattt ttattttaaa atattatttt ttttggttta tttattaata atttgttatt 15780
   aatttttatg ttatttttgt tttagataat agtgtttttt taggaattgt attgaggttt 15840
   atatttaaaa tgttttatag atattttttt tttatttttt ttttatatgg agaagaaagt 15900
   taaggtatta aggtttaaga gtatatttga gtttatagag aggttatgtt taagatgaga 15960
   atgagaatga ggttatagga atttgatttg gatattagag aagatgtttg ttattaagga 16020
   atttagggat gttaaggata ttattaggtt ttttttttta atgatgtata tgttttggaa 16080
   tatttgtgta tatttttata gatgtattta tttatatata tatatatatg tataaatata 16140
   tataaatata ttttttatag ttattaagta aaatgtattt gaattgttat tgataatatt 16200
   atgttgaaaa tttttttgtt tgtttatgat attgttatag gttagtattt tagggagaaa 16260
   ttttgttttt ttttttgtag aaatttttat tttagtaaag aaattttttt gtatttttat 16320
   aagtgtttag ggttattata ttttggtttt tgttgaggga aataaatagt aaggagattt 16380
   ttgatgaatt ttggtttttg tttattgtaa tttagaattt atgatttttt ggggtttttt 16440
   ttagaaataa ttgtagattt tttattgagt gtttattgta tgtttagtgt tgtgttaggt 16500
   attggttata tttaattttt gttgttaagt aatatatggt ttattgattg aggaagggga 16560
   gtaaggaaat agtattatgt ttgaggtttg tgtagggaat tttggggata tagaggaggg 16620
   atggggtgaa agggaggggg atttatgagg ttttagagtt tttttgtttg ttttattata 16680
   tttttttaga tagaaatgaa aagtttgatt aaattatgtg agtattgagg atagggggtt 16740
   gaatttttta aaaattttga ttgtttgaat gattggattt tggtttattt agattagatt 16800
   tgtagattta atttatgatt gtggataaag atattttttt aatttgttat tttagttagt 16860
   atgtaagtga aataggaata atgatttaat ttttatgtgt aatatagttt tttttggttg 16920
   aggaagagta gttaagagat attagtaagt aagttattgt ttataaatta atataagtta 16980
   atttattttg tttttgtttt aagttgattt ttaatttttt tttatttttt attttattta 17040
   tttatttgtt tttttgagat agggttattt aggttggagt gtagtagtgt aattatagtt 17100
   tattatagtt ttaatttttt gggtttaagt gattttttta ttttagtttt taagttgttg 17160
   gaattatagg tgtatattat tatatttagt taattttttt atttttgtag ggataaggtt 17220
   ttgttatgtt atttaggttg gttttgaatt tttggtttta agtaattttt ttgttttagt 17280
   tttttaatta tagatgtgag ttattatatt tagttttaag ttgatttttt tttttttttt 17340
   gagatggagt tttgtttttg ttgtttaggt tggagtgtaa aggtgtgatt ttagtttatt 17400
   gtaatttttg ttttttgggt ttaagtgatt tttttgtttt agttttttga gtagttagga 17460
   ttataggtat gtattattat gtttagttaa ttttgtattt tttatagaga tggggttttt 17520
   ttatgttggt taggttggtt ttgaattttt gatgttaagt gatttgtttg ttttggtatt 17580
   ttaaagtgtt gggattatag gtgtgagtta ttgtgtttgg tttaagttgt tttttttaaa 17640
   aaaaaataaa ttattaatta ttagggaatt tttttaggtt tattatttat aatttaaagt 17700
   ttttttatat ttttttttta gtgttttaaa ttttgatggt ttgagaaggt tgggtatttg 17760
   tttttagttt tgagaatttt tttatttaaa ttaagttttt tttttttaag gagtagtggg 17820
   attttaatta gatttttatt agaaattttg attatttaga tttttttttt gtttatttta 17880
   gtaatttttt tttttgtaaa gatggatggt ggtagggggt tggagaggga ataattttaa 17940
   gaatttgttt tttgggagtt gttattatag gttaggtagg ttttgttttt tttttttgta 18000
   tttttttttt gtgttttagg aggattatat gtgaataaat gaggataata tttggttatg 18060
   aggattttta attttaggtt atattaaata aattgttttg tagatattaa aagaaaatgt 18120
   aaaggaaaat ttatagaatt agtttatata aaattaatag tatttttttt gtttttttaa 18180
   aaggtgaata tatataagaa attaatttat tttatttatt gaaaaaggat gtagaaaata 18240
   tggggaaagg taaatgaaag ttttaatttt gtagaatttt ttttagtttt aaatgattgt 18300
   ggagataata ggaggatatt tttgtttttt attatatttt tagtttgata agtaatatat 18360
   gaataatata tggattattg ggtggatata attgagatta atgtatatat attaattaat 18420
   atatgatatt gtaatataat attttaaaat aatatattat attgatttat atttatggat 18480
   ttttgtataa aatttatttt atgtttatgt aatatttttt aaattggtaa aatattttgt 18540
   tttataataa taaattgtta tatataatag ttttttggag agttatttgt aaagattagt 18600
   aaaatgattt tttttttttt tattagaaga attttagaag gttttatttg aataaataga 18660
   tttatggaga tgattggaat aagaatttta ggtgttaaaa ggaaatatat tttttttagt 18720
   atttagtaag aaattatttt tattttattg tatttatgat aattgattat gtttttatta 18780
   ttaggtttaa gaatgtgatt ttaagtatta agtgattgtg taaaatatat atagtttttt 18840
   tatggtttgt ttaaatattt ataaatttta gttttattta ggttttgaat tgatttatag 18900
   ataaaatttt ttttaagaat tttttggtat ttgtgtatgg atatatgtgt atttgtatgt 18960
   gtatgttgta tgtattttaa aatgtgtgta aatatataat taaatatgta aatatattta 19020
   aatatttaga tatagtttga ttatagattt taaaataaaa gaattatttt gtatttatat 19080
   tttttaaagt agggaaaata agaaaaagtt aatttttatt tttagattgt ttttgatagt 19140
   tttttgatgt ttgagatatg tataaatatg aaaatttaaa ttttaaaggt ttagtatata 19200
   taatttattt tattaaatga aattgttata attttagtag ttttagattt tttatttata 19260
   tttaatgtta aaattttatt ttgaatttag aggtaggaga tattgtagtt taaaaattgt 19320
   tagttttatt tttagagttt tggaattttt taaaatttta tggtaggtgt ttagattttt 19380
   aaaatttatt tgttttggtt tgggttatat ttggataaat atattttagt agtgtttttt 19440
   aagttttttg gattttgatt tagttatatt atttttttag agaaaggaaa gtgggaaaat 19500
   agtatgtaaa tttatttttt tttttttttt gagatggagt tttgttttgt tatttaggtt 19560
   ggagtgtagt ggtataattt tagtttattg taatttttgt tatttgggtt taagtaattt 19620
   ttttgtttta gtttttggag tagttggtat tataggtata tattattata tttggttaat 19680
   ttttgtattt ttagtagagg tagagtttta ttatattggt taggtaggtt ttgaattttt 19740
   gattttaggt gatttgttta ttttggtttt ttaaagtgtt gggattatag gtatgagtta 19800
   ttgtgtttgg tttgtaaatt tattttttta attatatata tttaaaatta atttttttta 19860
   gggtattttt ttagaatgtt agtatgtttt ttgttattaa tttagaaatt ataaaattta 19920
   agttggtaat ttttgtgtta gatgattttt gtattagtat ttggaagatt tttaaataat 19980
   tatgataatt ttaataatgt tagtttgaag gtttttattt attagggttt tttaaatgta 20040
   attattggta taattgaatg atatatttag gtagtatttt tgaatagtat taggtttttg 20100
   gtaatatgtt aaattaaatt tgattagaat atttagtgag gatttaaaat gtagaggtaa 20160
   ttgtatgatg gaagagaatt ttgattataa ggaatgggag ggtttgtaaa gtgtgttttt 20220
   tttgtatatt ttaaaggagg ttggagttta tggtatttta gttattttga atttagagaa 20280
   tgtttgtagg gttttagaat gagattgaaa gaaggagaat ggttaatagt ttttgatagt 20340
   ataattagga atattgtgat atttatagtt ttgagaaaag gagaaatatt taaaatattt 20400
   ttgaaattat aagaaatagg tgttttgaaa ggatttagga ttttttaatt tttgaaattg 20460
   tattttaaga aagattgtat agttttttgt ttttaatgag aggtattgaa tatgtttttt 20520
   taatagttga tgtttattta attgttgagt ttgtaagttt ttgtttttag atttagtaga 20580
   gtgggtttaa gttttattga atttttaata tgttttttta attttttatg aaatttttgt 20640
   agttataatt tattttatta ataaaagtta atttatattt attttaagga attaagtaat 20700
   ttataataat ttatattttt tatagtaatt gttttgtagt atatgttttt gttaaataaa 20760
   atgttagaat gttatagtat tggttagttt taatttagat tttttattta ttattttgat 20820
   gtgtattaat atgtattttt tgtgattgtt atatgtatga aatattttaa ttaaatagta 20880
   ataaaaaaaa tttttttttt ttttatttgt aatatatttt attgattaag gaagtggtat 20940
   gtgtgtgttt aagtttaaat aatgtgtggt ttagatatat tataaagagg tagttaattt 21000
   tatatggatg aaaatagtaa atgtatatta tgtgtttgtt aattaaatgt gaggggttgt 21060
   gaggttaggg gatagattta gatttattta gatgaaaagg ggaaaattaa gatttgtaaa 21120
   gttttattgg aaagaagtat agaggaattt ttgtgagagt tttgtaaagt tttttattag 21180
   gaggtgaata gatgtttata gttattatat ttaaaattta gtttatttag agtattgtgg 21240
   atttaggtta ggagttgtag tatgtgagaa ttaagaattt aggtttaaaa attgattaga 21300
   tttgagttta tttttaggtt ttgttatttg ttggttgtat gattttggaa attgtaaaat 21360
   ggggtaaata atttttgttg tttagaagaa tgagaggatt gaatgagaga tttatgtgaa 21420
   gtgttgtgta tatgttgggt ataggataag tataattatg atggtgatta tgaagtagaa 21480
   agaagaaaag gagaggaata gtattgagtt ttagaaaagt atttttaaat ttgggtttaa 21540
   atttttggtt tttggttagg tatagtggtt tatgtttgta attttagtat tttgggaggt 21600
   tgagttaggt agattatttg aggttaggag tttgagatta gtttggttaa tatggtgaaa 21660
   ttttgtttgt attagaaata taaaaaatta gttggtgtgg tggtatgaat tttagttatt 21720
   taggaggttg aagtaggaga attatttgaa tttgggaggt agaggttgta gtgagttgag 21780
   attatgttat tgttttttag tttggataat atagggagat tttgttttta aaaaaaaaaa 21840
   aaaaaaaaaa aaaaaaattg gttttttgtt gtggttttaa gggttaatga ggattatagg 21900
   aatggttgta agaatggaga gggtataaat aaagataata gagaggaggg gtaggtggag 21960
   gaggaggagg aggaagggta gagggaaaaa ggagaaggag gaggaagagg taggaaagga 22020
   ggaagaagga ggaagatgga ggagaagaaa taagttttta tatttgggga gtagtagttg 22080
   ggaggaggaa gagaaataag taagtttttg tgtttgttgg aatagaggtt tttagagttg 22140
   ttttttattg atatttatgt atagttttga tggggaaatt tggggaggag aaagtaaggt 22200
   aaggtaggta tttgttatta tagtagaggt agagaagtat ggtaggttat agagagggtt 22260
   ttaggtgtgg atgttgattg ttttatttat tggttgagta attttaggaa aattttttga 22320
   ttttttttta atattataat aatattatta tttatttttt ggggttgtgg agaggattaa 22380
   atgagtgaaa ggtatgaagt gtttagtata gtgataaatg aatagtaatt attattaaat 22440
   gtgagttgtt gttattatat attgttttaa agtttgatgt tgatattttt gtagtgattg 22500
   tatagtaaat tagttaatgt aagaaattag aatttgtaaa ggtttattga gttgttatta 22560
   tagagggagg aatagggagt gaaatttgtt ttaatattaa ataataagtt taattaatga 22620
   attgggtagg atataataga gaggtaggga ggatatatta aggaaaaagg agaagttgga 22680
   attgagtggg agtaggtgtt tggggaaatg gattaggagg ggttttatta ttattaaggt 22740
   tattttgaga tgataatagg atggttggtt aaatatgttt tgtaatgaaa attatgttta 22800
   gttggatagg ttaaattatt aaaggtaatt tttaatttta ttattttaaa tttttttatt 22860
   tatattggtt tgattatttt tgtagttgta aatttagaag ttatgtttgt tattgtatta 22920
   tgtatatttt ttttagtaat tgtatttttt tttttttttt ttttttttag tattttatgt 22980
   ttttttttat tatagtattt attgttttgt tgtagttttt atttattttt attttttttt 23040
   attagttatt ttaaaatttt agagtttagt tttaaatttg ggatttgtta aaggtggtaa 23100
   tgaatggaga aggttgttat tagtagggtg gtttgtattt ttattttttt tttttatttt 23160
   agtttttttt gtttgttgtt tttagttttg tttttttaaa attttagatt tggtataaaa 23220
   tttttatttt aagaagttag aatgatttgt attgtgtatt atggtatatt taagttaatt 23280
   gatttttaaa atttgttata attgtgtgtt ttttttttaa tttttttatt tttatttttg 23340
   tttttttttt ggtttagttt tagttatttg gtagattatt tttatttttt tttttttatt 23400
   tttgtatatt gtgttttttt agtttagagt gttttttttt atttaagttt ttttttttat 23460
   ttggagtata gttaaagttt tgttatattt aagaagtttt tttatttttg tagtaggtat 23520
   gattttttga agttttgttg tgtttatttt tagtattatg agttttttgt gttgattttt 23580
   tttttgtttt atttgtgtta ttttttattt ttttaattta tattgtaaat agatttaggg 23640
   aagagttatt tagaatgttt ttttgttgtt atttgtagta tttggtatat tgtgggtatg 23700
   tagtatattt ttaataaatg attgatagga atattggggt tatatttaaa gagattaaaa 23760
   ttgataatgg aggatggaat tggattattg aagatttgta taaaggtgag tatttagggt 23820
   ttatgagttg aatttttaga tttgtagatt tagtgataaa tttatggtat ttataagaat 23880
   tgagtggaaa ggttagttgg ttattatagg aaagtattaa tgaatggggt tttatgaaaa 23940
   atgttagatt ttatttaaaa tgttattatt ttagattttt ataaaaaatt gtttttttaa 24000
   attattgtta agagtttttg ttattaaaaa taaattatat tttatttttt tgaatatggt 24060
   tgtttgtatt ttttatttgt ggttattatt gaatttattt tatgtttagt ggtttttttt 24120
   tttttttttt tttttttttt ttttgtagta tattttggga gatgaagtgt agggttgggg 24180
   tggtagggta gattttaaaa tattgtatag attaaatttt ttttagtaga agatggttat 24240
   gaaaaaaatt tttttttaag ttttggttgg gtatagtggt ttaggtttgt aattttagta 24300
   ttttgggaag ttgaggtagg aggattgttt gagtttagaa gtttgagatt agtttgggta 24360
   atatattggg attttgtttt tatataaata tatatatata aattagttaa gtgtggtggt 24420
   atgtataggt agttttagtt attaaggagg tagagatggg aggattattt gaatttagga 24480
   ggttaaggtt gtagtgagtt ttggttatat tagtgtattt aagtttgggt gatagagtaa 24540
   gattttgttt taaaaaaaaa aaagtttaat ggttttttaa aaatagagat aataaaatga 24600
   agatatttgt aaataaattg aattttaaag tttattgttt gtttagaata ttttaggtga 24660
   attagatgtg aattagatgt attttatggg tttgtttttt tatttatggt tttaggaatg 24720
   taggttagat attttttatt gttgtttgga aaattttttg gtttttttag ttgtttgagt 24780
   ttgtagtgtg tagttttgtt gttgtgttgg ttgatatgtt gtattttagt gttttttttt 24840
   gttatttttt tttgtattat tattttttaa aaatagttgg gttataagtg atttttatta 24900
   tagaagttgg tttaatgatt ttttttattt ttaattaaat tttgttttta ttaattttta 24960
   gaatatagaa aaaatatggt tgggtgtggt ggtttatttt gtagttttag tattttggga 25020
   ggttaaggta ggtggattat ttgaggttag gagtttgaga gtattttggt taatatggtg 25080
   aaattttatt tttattaaaa atataaaaaa agtagttagg tatggtggtg tatgtttgta 25140
   attttagtta tttgggaggt tgaggtagga gaattgtttg aatttaggag gtagaggttg 25200
   tagtaagttg agattatgtt atggtatttt agtttgggtg atagagtgag attttgtttt 25260
   aaatttaaaa aaaaaaaaaa gaatatagaa aaaattttag atttttgtat gtttgttaat 25320
   taataatttt tatattttaa aagttatata tatttttgtt gtggaaaatt ttaagtatgt 25380
   agaaataaaa aggaaaggaa ataaaaatta tttataattt tgttatttag agataattaa 25440
   tgttaatatt ttggagtttt agttttttta aatgtttgtt aaatgttttt ataaaataaa 25500
   atgaaatgta atatataata ttttttattt tttttttgtt ttagttaaaa tgtgttttgt 25560
   tttgttatta tatagtagtg tataaatata ttgttatgtt ggatattaag gtttgtttaa 25620
   gttttagata atggatattt ttgtaattaa gtttttgtat gtatttatta ttattttttt 25680
   aggttaaatt tttaaaagtg ttgttgtgtt aaagggtatg aattttttaa aagttttaat 25740
   atataatttt aagttgtttt ttagaaagtt ttaattaatt tttgttttta tttttggaat 25800
   agtatgtatt atttttttat ttaatttttg ttaatttaag aggaaatttt ggtattttat 25860
   attatttttt ttttttttat tgttggtgat gttgaatatt ttttatgtgt ttagtatttt 25920
   tttatatgtt ttgtgaatgt ttatttatat ttttttttta tatttttgtt gatgtgttta 25980
   tttttttatt gatttgtata aattttttta tattaaaatg ttaatttttt gatatgttgt 26040
   aagtattttt tggtttgtat taattatttt taaatgttga ttatggtgtt ttatgtttgg 26100
   ggagtttaat ttgtttgtaa tttattggta tattaaagtt ttaaattttt gtttgattaa 26160
   attaattagt tttttttttt ttagtttttg ttttttatgt ttttttttat tttaatttta 26220
   aaaaaaaatt tgttattatt ttgaatattt taatggtttt ttttttgttt tttattttaa 26280
   tttaaggaat tatattaagt gtattattat agaaaaatga gtatgagtta agttaggatt 26340
   agtttttatt attttatttt gaattgtttt tagttgtttt tttttttaaa attagaattt 26400
   tttgtgattt tagggaagtg ttttagtttg gttggtttta ggtagttttt tttttttaat 26460
   ttattttagt ttattatttt ggttaaaggt attttggatt tttagaggtt tagaggaaag 26520
   tttttttttt tataaaggga gttatgtttg ttatgtgttt tagtgtttgg aattatattg 26580
   atttttttgg aggtttgtgg gttatagtgt aggattaagt gttggaatta gggtagtttg 26640
   tggagggttg gtttttttgt tgttttgtat ttttagattt atttttagag tattttgatt 26700
   tggtgaggtg gatgttagtt ttttatatgg tattttttgt ttaattgtta ttggtgtaaa 26760
   ttaaaatttt ttttttattt tttttgtata ggaaagataa gtgggtgtat gaagtttaat 26820
   taaattaatt tattttgtta ttaaaaattt tttttttttt ttttttttga ggtagagttt 26880
   tattttgtta tttagattgg agtgtagtgg tgtaatttta gtttattgta gttttgattt 26940
   tttaggtgat ttttttattt tagttttttg tgtagttggg attatagttg tgtattttta 27000
   tgtttagata attttttgta tttttagtag agatagagtt tttttatgtt ttttaggttg 27060
   gttttaaatt tttgggttta agtaattttt ttgttttagt tttttaaagg gttgggatta 27120
   ttggtttgag ttattatatt tggtttgtta ttaaagttta aaatgttaga gtaagaaaat 27180
   atttgaaaga gtatattatt ttatattttt gtttttttag ataaaggttt gaggttttag 27240
   agtggggtag ttattataga tggtagaatt ttaatttgtt ggtggataat tttgtgtgtt 27300
   tttatttttt gtttattagt tagtttttgt tgtgtgttag gtattgtggt aggttttaaa 27360
   aatttttaaa gtgtttaaat gtattttttt gttttttaag aatttaattg tttagtaggg 27420
   agatagatgg ataataatta attatagtat gtattatgga tgtattaata taggtatgga 27480
   tagggtgttg gagaagtata aaaaaaaatg ggtaaataat tttggtagga ggggttgtag 27540
   aattggagaa gattttttgg aggaggtagt atttggattg gattttaaag aaaaggtagt 27600
   tgtttgttgt aagggtaaga ttatagtagg ggtaaaggta tagaggttgg aagtataatg 27660
   tatagttgag gattggagag tggtttggtg gttggtgtgg tgtaggggat tttttttgtg 27720
   tttttggttg tagttagttg agttatatgt agtttttttt tttttttttt atgtttttat 27780
   gtagaatgtt gttttttatg tgtttgttta agtagttttt ttttttaatg tttttagaat 27840
   gaatgatagt ttttttttta atttttgtat ggtattatat attttttttt attatagtga 27900
   ttttgatatt aaaattagag gtgatagtag ggattgggtt tttttaaaaa aattaattag 27960
   aatatatgtt tggggttgga taagaaattt aaatagtatg aaaaggtaga agttgaaaaa 28020
   aataaaaaga ataaagtttt tttattttta agtatttaga gttttatgag tggtaaagga 28080
   tttattattt tgaaagtgtt ggaaagttat tggagatttt taagtaggga attaaaggta 28140
   tttggagaga aagggattgg tggtaggtgg ttagttggaa aatgatttag ggatgaggtg 28200
   tttgtatgtg ggaggtaata atggtaaaga ggggagaata tgatggtgga atatatagaa 28260
   tttatatatt agttggttgt gaagatgaaa ggaaaaggag aagaaaagat aatgatgaag 28320
   tttttggttt ttttaagttg ttgttttatt aattataaat ttaataagaa attgtgttta 28380
   tttttttttt agtgtagaaa aaggtaaagt tagttatgtt tatattttta aaaggattat 28440
   gtggagaaga tgtaatatta aattgtatat ttggaagtta tttttttttt aatgtaataa 28500
   atatggttaa tatgtagaat agtaaaaata ttttgtatga taatattttt taaagttttt 28560
   ttttttttag attaaggtaa ggtttgtaga aaggtattag gataaagtag gaatataaaa 28620
   taaggttgat tttagggtga gagagtttgt tgtatagggg agttaggttg aagtattttt 28680
   ttttaggagt gagatgagtt gtagtgaagt taggtaggta ttttttatag atttatattt 28740
   tatttatttg gaaggtatta tgtgtaggag gggtagtatt ttttatgttt agttttattt 28800
   gaggtttgtt ttattgattt ttgttggatt tttgaagata gtggaagttt ttaatatttg 28860
   tgatttgtat aagtgtgtaa aggttaaaga atatgattgt gtgggatatt tttgtgtaat 28920
   tatatgtaag agattatatt agatggaata tgagatattg taaaatataa gatatattta 28980
   gaatttattg tttagtttga ttaaattata gagtgttgaa aatatttttt aaataagtat 29040
   ataatttgtt tatgaaaaaa atatttaaaa gtaggggggt tgtttatggg attgttaata 29100
   atgtttttat tagttaaatt aaaagtagat tttatatttt agttaatttt tttttatttt 29160
   ttttttttat tagataattt ttaggattag atgaaaaggg agttagttta ttgagaagaa 29220
   atggtgtaat agttataaat tgtaagttgt atttttattt aa 29262
<210> 113
   <211> 20043
   <212> DNA
   <213> Artificial Sequence
<220>
   <223> chemically treated genomic DNA (Homo sapiens)
<400> 113
   aggtgtgagt tattatatat agttaattgt agggattttt ataatttata aaaggaaata      60
   gagagaattt gaattttttg tttttatatt gtttttatat ttttgagggt tatttatttt      120
   ttttgttttg ttttgttttg agatagggtt tggttttgtt atttaggttg aaaagtagtg      180
   gggtgatttt agtttatagt aatttttgtt ttttgtgttt aaattatttt gttattttga      240
   ttttttgagt ggttggaatt ataggtatgt gttattattt ttagttgatt tttgtatttt      300
   tttggtagag ataggatttt attatgttgt ttaggttggt tttgaatttt tggatttgag      360
   tgatttattt atttttgttt tttaaagtgt tgggattata ggtgtgagtt attgtgtttg      420
   gtagggttag tagtttttat gaaattttat gtgggagtag aatgtttttt gatttgagaa      480
   gattttttta tagatgttgt atgagtagtt attatttttt ttgtgtgaat gttggatatg      540
   gttgttatgg gtagtatggg atgtaaaaga tttattatag tatttgtatt tgaagggttt      600
   ttttttggag tgttgtttga tgtgtgtgtg gagtatgttg gaggttgtga atttttgtag      660
   agaatagaaa tatagaatat aagaaaaagt taatttttat gagttggata attaattaaa      720
   ttaaggggta aattttattg ttgtttattt ttatggatga attttgggtt ttaaggttat      780
   agagtatttt tggttattat tagtagtttt aatttgaaag gattttattg atgtgttttt      840
   ttggttagtt ttgagaagta ttggatgtta gaatagtggt tttttaatgg ttagattatt      900
   tggaggttta gtttgtaaaa aagtagattg taggttgggt gtagtggttt atatttgtaa      960
   ttttagtatt ttgggaggtt aaggtaggtg gattatttga ggttaggagt ttgagattag     1020
   tttggttaat atggtgaaat tttattttta ttaaaaatat aaaaattagt taggtatgtt     1080
   attgtgtatt tttaatttta gttttttagg aggttgagat gggagaattt tttgaattta     1140
   ggaggtggag gttgtagtaa gttgttattg tattattgta ttttagtttg ggtgatagag     1200
   tgagattttg tttttaaaaa aaaaaaaaaa aaaaaaaaaa aagtagattg ttagtttggg     1260
   taatatagta atattttatt tttataaaaa gtaaaaaaat tatttaggta tggtagtatg     1320
   tatttgtggt tttagttatt tggaaagttg aggtgggagg ataatttgag ttagaaggtt     1380
   aaggttgtag tgagttatga tggtattatt gtattttagt ttgggtaata gagtaagatt     1440
   ttttttttaa aaaaaaaaaa aaaaaaaaaa gtagattatt aattttattt aagagagtgt     1500
   gatttaataa atttgatatg agatttagga gtttgtattt aaataagtaa ttttgatgta     1560
   tgtgggtttt aggattatat tttaagataa attttattat agaaagagaa gttaatattg     1620
   attagaagtt aatattggtt agtgtttttg tataagttag tgttttattg gttttttttt     1680
   tttttttttt tttttaattt taaatttttt gagatggttt gtaggtttgt tttatttgtt     1740
   tgtttgtttg tttgttttga gatagaattt tattttatta tttaggttgg agtgtagtgg     1800
   tataattttg gtttattgta atttttattt tttaggttta agtgatttta gtgttttagt     1860
   tttttaagta gttggaatta taggtgtatt atgatgttta gttaattttt ttatttttag     1920
   tagagatggt gtttttttat gttggttagg ttggttttaa atttttgatt ttaggttgat     1980
   ttatttattt tggtttttta aagtgttggg attataggtg tgagttatta tgtttagttt     2040
   agtggtgttt ttttatgttt attattttta ttttattgat atatattttg gaggtattag     2100
   ttgttttaat ttggtagatt taatgtttta ttttttttga gaggtttaga agtagttgta     2160
   ggatagtttt tatttttttt atggttttat tgtaaaaagt gtaaatgatt tagtgattga     2220
   aaatataatt tatattagtg ttttttaata ggatttgttg taataatggg aatatttatt     2280
   atttatgttg tttattttgg aaattaatgg ttatatggag ttattgagta tttaaaatgt     2340
   ggttaatgta atttagggaa tggaattttt taattttatt ttgtttttat taattgaaat     2400
   agttatatat gattaattgt gaattgtata ttgggtagta tagagttatt ttggtgtaat     2460
   atgtttaaag tgtggttaat ttattgtttt tattatagta atttttaatt atatgattta     2520
   tttttgtttt tataattttg tattttgtgt aatatagatt agatgttgtt tttgtaatgt     2580
   ttttatagaa aattatatta gtagtatttt ttatagatta aagtagtaat aatagtagtt     2640
   aatattttag tagtattttt atgttatgtt aggtattgtt ataagagttt tatatatttt     2700
   attgatgtta tttaataatt tggtaaagat tatttttttt agtttattaa tgtggaaatt     2760
   aataggagtt tatggatttg tttagggtgg tatagttagg taagtattag aggtaggatt     2820
   tgaaatttgg ttatgtttgt gtttttaaat attatgaaat agtgattttt tttttttttt     2880
   ttttttggag atggagtttt attttgttat ttaggttgga gtgtagtggt ataattttga     2940
   tttattgtaa tttttgtttt tgggtttaag tgattttttt gttttagttt tttaagtagt     3000
   tgggattata ggaatgtgtt attatgttta gttaattttt gtatttttag tagagatggg     3060
   gttttattat gttggttagg atggttttga atttttattt tttaggtatt tgtttatttt     3120
   agttttttaa agtgttggga ttatgggttt gagttattat gtttttttat tttttttttt     3180
   tttttttgag atagtgtttt attttgttat ttaggttgga gtgtgtagtg gtgtaatttt     3240
   ggtttattgt aatttttatt ttttggattt aagtaatttt ttagttttag ttttttaagt 3300
   agttgggatt ataggtataa gttattgatg tatggaaaat ttgattatag gtgtgagtta 3360
   ttatatttag ttagtgtttt taaatattat gtaataggtt attaggtttt tataaatttg 3420
   gaatgtattt tattatatgt attttaaatg ttagtttggt aataggaggt tggttatatt 3480
   tgattatttt tagtaaattt gagtatattt aggggtggaa atgaggattg ggttgatatt 3540
   tgattaatat ttattaagaa tttattaagt taggttttgt tttaagtttt tatttataga 3600
   tatttaagta gagtttagga tatatttttt agtaagaggt tttatttatt ttagtataat 3660
   atatatattg gtatggtttg tttttagagt ggatttgtat gtgtttgttt aggttggaag 3720
   attgagagaa atatttttta tatgtagaat attaaggtga aaaagaaaaa tatagttgaa 3780
   attatatttt tttttaataa gtttatttta attttgtgtt tatttggggt tagagaagaa 3840
   ttttttttta attttttttt tttttttttt tttgagatgg gattttaatt tgttatttag 3900
   attggattgt agtggtataa ttatggttta ttgtagtttg gaatttttag gtttaagtga 3960
   tttttttatt ttagtttttt gagtaattgg gattataggt gtgtgttatt aagttagtta 4020
   atttaaaaaa tttttttttg tagagatgag gttttattat gttgtttagt tttgttttga 4080
   attttaatag atttttttgt tttggttttt taaagtgtta ggattatagt tttgagttat 4140
   ggtatttgat ttgagagaat ttttgtttta gatgtggaat gtgggaattg aataggtttg 4200
   tggtatatgg tagaggtttg aattagtttt gtttttttaa taggtagttg tgtgattata 4260
   gaatattggt taaatttgga attttagttt tttgattttg tttatgggta aaatatttgt 4320
   tttgtttttt ttatagagtt atgagtaaat tataagtatt gtgtaattaa tttaataatt 4380
   atttatgatt tatagagtat ttattgtgtg ttttatattg ttagaaatta ggagttagaa 4440
   attaaggagg ggtgatgatg aatatgtaag ttaatatggt aatgtatagt agtagattat 4500
   ttgagatgtg ttgaatttgt atgaagaggt taatattttg tttatatgtt gtgaataatt 4560
   aatattgttt tttgattttg gtaatttttt ttgaaattgt tttaggaatt ttatattagg 4620
   tgaagtaaat tattagagag gtgtaagtat tgagagtttg ttagggataa agtagaaggt 4680
   ttttggtttt gatattattt ttaaggaaat tatattaatt aaagagaaaa aataattttt 4740
   ttttgagaaa ggtttttgtg tttttataat ttttttttag gtatgtttat ggggtagagg 4800
   agagagtaaa tttttgtatt tgtttattta tttattttta tttatttttg aggtagagtt 4860
   ttgttttgtt gtttaggttg gagtgtagta gtatgatttt ggtttattgt aatttttgtt 4920
   ttttgggttt aagtaatttt ggtgttttag ttttttgagt ggttgggatt ataggtgtgt 4980
   attatatgtt tatttttata aaaaatataa aaaattagtt gagtatggtg gtatgtattt 5040
   atagttttag tttagttatt tgggaggttg aggttgggtg attgtttgag tgtaggaggt 5100
   ggaggttgta gtgagttgag attatgttat tgtattttag tttgggtaat agagtgagat 5160
   tgttttaaaa aaaaaaaaaa aaaaaaagat tgttttttta ttgtattgta agagtatttt 5220
   gtttgttgaa gggtaagtta ttattttatt attattagtt tttttattag ttttgttatt 5280
   tattgatttt ttggttgata gttttttagt tttttttttt tttttttttt tttgagatgg 5340
   agttttgttt tgttatttag gttggagtgt ggtggtgtga ttttggttta ttgtaagttt 5400
   tgttttttag gtttatgtta tttttttgtt ttagtttttt gagtaggtgg gattataggt 5460
   gtttattatt atgtttggtt aattttttgt atttttagta gagatggggt tttattatgt 5520
   tagttatgat ggttttgatt ttttgatttt gtgatttatt tgttttggtt ttttaaagta 5580
   ttgggattag gtgtgagtta ttatttttgg tttttaattt taaagagagt aagtatttta 5640
   tatatttttt gtaatttggt ttttgtttga taagataata tttttttttt ttttttggga 5700
   tggagtttta tttttgttat ttaggttgta gtgtaatgat atagttttgg tttattgtaa 5760
   tttttgtttt tggggttgaa gtaatttttt tgttttaatt tttagagtaa ttggggttat 5820
   aggtgtttgt tattatattt ggttaatgtt tttatatttt tagtagagat ggggttttgt 5880
   tatgttggtt aggttggttt tgaatttttg gttttaggtg atttgtttat tttagttttt 5940
   taaggtgttg agattatagg tgtgagttat tgtgtttggt tagtaagtta taatttatat 6000
   aattaatttt gtgttgatga atatgtagtt ttaaattttt tattattaga atattggata 6060
   gtttttattt ttgttagttt aataagtgag aaatattttt attgttttga tatttttaaa 6120
   tttttttaga attatataaa tgtatgataa ttgtaaatgt atttaaattt tataggagta 6180
   tataatatag tgattaaata tgattttttt agaagtttag gaaggaaggg agaaattagg 6240
   ataattttat gatgatttta tttttagttt ttaaaatagg aaaatgggtt taaggttgta 6300
   gttgtttgag gtagttttga taaggttaat agttaagtaa gaatatattt gtgtttaagt 6360
   tttgtttttt ttttaattaa gtttaaggga atattgtatt tttttgtagt aagtttagta 6420
   gagtatttag gtatttggaa tggttttgta atgttattat tgattttttg ttttataaaa 6480
   tttttggttt attgtagagg ttttttgttt tttggaagga ttgtgttttt attttttttt 6540
   tatttatatt tgttaatata tttgtataga tttggaataa ggtgtagttt tttaggagta 6600
   tatgttaggt ttttttttgt ttggaatagt ttgatttttt tttgtttaat taattttgtt 6660
   attagtataa attagatatt ttatttgttt ttttatattt ggtttaaggt tatttttgtg 6720
   tattttttat aatttatgtg tttaggtggt tgtggatatt ttttttttta tgtttttttt 6780
   tttgtgggta tttgtatttt aatatatgtg tattgttgtg tttatttatg tttgtttgtt 6840
   ttgtaatatt attttttttt tttttagaga gggtgtttgt taggtattat agttttgtag 6900
   ttagtattta taaggtaatt ttttgtgttt aggagtttgg gaagggagag agaatgtgat 6960
   ggtgggttat gtgttttttt aatatttatt ttgtgaggtt ggtgtttttt atgaatatga 7020
   agaatgtgga tttgaagttg gttttgtttt ttaaaggatt gtttgtagag agaataggga 7080
   aattttttat tgtttttgtt tatatagggg gtgtatttga ggtgtttatt tttgtaatat 7140
   ttgtaggtaa atattttttt atatttttta tatttgtagt tttttgtaga ttttgttaaa 7200
   tatagtaaat ataatgtttt tttagttatt ttttttttta gtttttttaa gaggatattt 7260
   atttatttat ttatttttag ttaagaattt tttggttatt ttaagaggaa tttttttttt 7320
   ttgagataga attttgttgt attgtttagt ttgtagtgta gtggtatgat tttggtttat 7380
   tgtaattttt gttttttagg tttaagtgat ttttttgttt tagtttttta agtagttggg 7440
   attataggtg tttattatta tgtttggtta atttttgtat ttttagtaga aatagggttt 7500
   tattatgttg gttaggttgg tttagaattt ttgattttag gtgattagtt ttttttggtt 7560
   ttttaaagtg ttaggattat atgtgtgagt tattgtgttt ggttaagaga attttttttt 7620
   tttttttttt tttttttttg agatggagtt ttgttttgtt atttaggttg gagtgtaggg 7680
   gtgtgatttt ggtttattgt aatttttatt ttttgggttt aagtgatttt tttgttttag 7740
   ttttttaagt agttgggatt ataggtatat attattatgt ttagttaatt tttgtatttt 7800
   tagtagaggt ggggttttat tattttggtt aggatagttt tgattttttg attttgtgat 7860
   ttatttgttt tggtttttta aagtgttggg attataggta tgagttatta tgtttggttg 7920
   ttaagagaat ttttaaaaat ttttttatta tagaaaaatg taaatatatt aaagtgaaaa 7980
   taatagtgta ataaattttt atgtgttaaa tattaagttt tgattaattt gtaattaatt 8040
   ttgtttttat ttatattatt gtttgttata gtagattatt ttaatattaa tttatttttt 8100
   gatttaaaaa tatttaattg taatttttag aagatgatat atttttaaaa agtataattg 8160
   tgattaggta aagtggttta tgtttataat tttagtattt tgggaagttg aggtaggtgg 8220
   attatttgag gttaggggtt taaaattagt ttggttaata tggtgaaatt ttgtttgtat 8280
   taaaaatata aaattagtta ggtgtggtgg tgtttgtttg taattttagt tatttgggag 8340
   gttgaggtag gagaattatt tgaatttggg aggtgtaggt tgtagtgagt taagattgta 8400
   ttattgtatt ttagtttggg taataatagt aaaattttat tttaaaaaaa aaaaaaaaag 8460
   tataattgtg ttaggtgtag tggtttatgt ttataatttt aatattttgg gaagttgaag 8520
   tggatagatt gtttgagttt aggagtttga gattagtttg ggtaatatag taagatttta 8580
   tttttataaa aaaataataa aaattatttg ggtatgatag tatatgtttt tagttttaga 8640
   ggttgagatg ggaggattat ttgagtttgg ggaagttagt tgaggttgta atgagtagtg 8700
   attgagttat tgtattttag tttgggtaat agagtaatat tttgttttga aaaaaaaaaa 8760
   aaaatataat tataatgtta ttgttatatt gttatattta aatgattaat ttttattttt 8820
   ttatagaaat atatatgtat ttatatatgt atatttatat atatatatat atatttgttt 8880
   aattattttt tggttagttt atttaaatta ggttttaaat aaagtttata tgttgtatat 8940
   ggttgttatg ttttttaaat attttaattt ataggttttt tttttttttt gttttttgat 9000
   atgtattgtt gaagagatta ttttattagt tttatttaag ggtagtgtaa ttttgtagtt 9060
   tttgaatata tttttgtttg gtatttgtta taaggttaat atattgaggt attgaggttg 9120
   atttattttt ttttgtgttt ttaagattag tttgagttat aagtttagag tagttattag 9180
   gatatttgta gtttatagtg ttgttttatt ttatataatt taagggattt ttttagttta 9240
   tattttatta tttaaaattt gtggtagatt ttagagttag aatttttatg tatagagtaa 9300
   ggttgaaagt attttgattt ttttaatgta tagggttatt gtgatgatta atttaatttt 9360
   aatatttatt gagttttatt aatgtgagtg atattattat tgaatggtag ataagattga 9420
   gaggtaaatt tgtttttatt aatggaggag ggaggaggag agaagttaga gagggtgtag 9480
   ttagatttta gtaaagtagg ttttatgttt aggggtgttt atgtgttggg ggaaggtgag 9540
   agaatagggg ttatttataa aagtattttt gtagaggata ttttggattt tagaggataa 9600
   agtggttttg attatgaatg tattatagag attttattgt ttgattttag ggaggaagag 9660
   tagttaagag tgagtttatg taagagtttg tagatagaaa tttttttagt attaggatag 9720
   tagggaagtt gtttattagg ggttttgtta gttgataatt atattttagg tatgtttaat 9780
   aggtggttaa ggaaataaat tatttattaa gtaaataatt aggaaggtaa ttgttagttt 9840
   taagttgatt tattgttttg ttttgtttat tatttttatt gaggtgttaa tatttttaga 9900
   atttttgtgt tgggatattt ttttggtata gaggtagaat ttttattggt tagaaatatt 9960
   tggttttttt gttttttggg gtgggaaggg aagagagggt tagtaatttt ataaatttaa 10020
   gtattaaagt atattaaaat ttagaataaa gttttaatat tgagtttgtt taagaataat 10080
   tatttaggta gaaatgaaat ttaattttat tttgtttttt atttttttta attttatttt 10140
   gttttttatt tttttttttt ttagtttttt atatttttat attttaaaat atatatttaa 10200
   attttttttt ttttttataa gaggggaatg tttgtatatt taaataattt gtttatatag 10260
   agtttagatt ttttttttat tatttaagat tttaaagagg aaggttttta tttattatta 10320
   atttttatta aaaagttttt ggggaaaaaa atttatttga gaatgtaatg gaattttaat 10380
   tttttttttt tttttattat atttaaatta tttatattaa tagattaata aattaatatt 10440
   tatttataat tatttttaat aaattaagtt gtttataagt ttttttttat tgtttaaaat 10500
   atatgttttt ataataatgt tgtaagtatt aggtattatt tggtttagaa ttggaatata 10560
   agtattttat ttgtatgttg ggtatttttt gtatttaatt attataagtt tttattttgt 10620
   tgtttgtttt gattgttttt gatattggta tattatttta tgttaataat aaataggttg 10680
   agtatttttt ttttgaaatg tttgggatta gaagtgtttt ggattttagg ttttttttga 10740
   attttggaat attttatata tataatgagg tattttgggg atggaattaa agtttaaata 10800
   taaagtttat ttatgttttg ttttatatat attttatata tatagtttga aggtaatttt 10860
   tttttttttg agatggaatt ttgtttggtt tgtaattttt gtttttggtt ttaagtgatt 10920
   atttggtttt agttttttga gtagttgaga ttataggtat ttgttattat gtttagttaa 10980
   tttgtttgta tttttagtag agatggggtt ttattatgtt tgttatgttg tttttaaatt 11040
   tttgatttta ggtgatttgt ttgttttggt tttttaaagt gttgggatta taggtatgag   11100
   ttattgtgtt tggtttattt gaaggtaatt ttatataata ttttaaataa ttttgtgtat   11160
   gaaataaagt ttgtgttaag tattggtgtg tggaaatttt tatttttggt aatatgttag   11220
   tgtttagttt tagattttag agtattttgg atttgggatt ttttgattgg agttgttgag   11280
   tttgtattat tatttattta attattatat tattgaatat ttaggttgtt tgtgattttt   11340
   tatttataag agtggtgtta tagttaaaaa tttttataag tatagttttt tttttttgga   11400
   ttattttttg ataatagatt tttagaagag tattattgag atattaaagt tataaatatt   11460
   gtaataattt gatatatatg gtgaaaaaat tttttaaaaa tggttggtta tggtgtttta   11520
   tgtttgttat tttagaattt agggaggtta aggtgggtgg attatttgag tttaggagtt   11580
   taagattagt ttggttaaaa tggtgaaatt ttatttttat taaaaatata aaaattagtt   11640
   gggtgtggtg gtgtatgttt gtaattttag ttatttggga ggttgaggta ggagaaatgt   11700
   ttgaattttg gaggtagagg ttgtagtgag tttagattat attattgtat tttagtttgg   11760
   gtgatagagt gagattttat tttaaaaaat aataatgatt ttttaaaagg ataataatta   11820
   ttttgttagt tattaagtag atattttttt taaatttatt gtttattttt ttttttttta   11880
   tttttttttt tgagatggag ttttattttg ttatttaggt tggagtgtag tggtgttatt   11940
   ttagtttatt gtaatttttg tttttgggtt taagtgattt ttttgtttta gttttttgag   12000
   tagttgggat tataggtatg tattattata tttagttaat tttttgtatt ttagtagaga   12060
   tggagtttta ttatgttggt taggttggtt gtgaattttt gattttaggt gatttaatta   12120
   ttttggtttt ttaaagtgtt ggggttatag gtgtgagtta ttatgtttag tttgttgatt   12180
   tttattttga aaggagttgg tatattttag gaagtgatta ataggttttg gtttggtgtt   12240
   aagtaggaga taatttagag ttttatagat tatagagaat tttaattatt tttgttttta   12300
   tttttatttt tatttatatt tttatattta tattatttat taaaatattt ttggttttag   12360
   gtattttaga aaaaggatat ttaatttgta tgtagatatt agaggtgatt agaataagta   12420
   atagaatgag ggtttatgat ggtttagtgg tggggggaat ttagtatgga tataagatat   12480
   ttgggggagt aggaagatag tttattaaaa taattttgat agttttttat tattgggatt   12540
   attatttatt attagataaa taatgatggt tgattgttta ttatttatta ttagataaat   12600
   aatgattgtt tattatttat tttttaattt ttggatattt ttttgttttt tgttttattg   12660
   ttttttgagt ttgattgttt tttttttttt ttttttatat gtgttttatt ttattttatt   12720
   tttaagtagt tttttttagt ttagttttaa tgtttttttt ttagttagta tgtattggga   12780
   tttagaagta tgagatatta aagtgtttag ggttattttt tgttttgaag tattttagtt   12840
   tagtaggaaa ataggtatgt agatgtaatt tatttttttt tttttttttt ttgaaatgga   12900
   attttgtttt gttgtttagg ttggtgtgta gtggtgtaat tttagtttat tgtaagtttt   12960
   ttttgttggg tttatgttat ttttttgttt tagtttttta agtagttggg attatagggg   13020
   tttgttatta tgtttggtta atttttttgt atttttagta gagaagaggt tttattgtgt   13080
   tagttaggat ggttatgatt ttttgatttt gtgatttgtt tgttttattt tttaaagtgt   13140
   tgggattata ggtgtgggtt attatgtttg ggttattttt tttttaaatt tgttttttat   13200
   atatttatta tattataata atttgttttt tttttttttt tttttttttt tttggagatg   13260
   gagttttatt ttgttgttta ggttggagtg tagtggtgtg gttttggtat attgtaattt   13320
   ttgttttttg ggtttaaatg atttttttgt tttagttttt tgagtagttg gaattatagg   13380
   tgtgtgttat tatgttttgt taattttttg tatttttagt agagatgggt ttttattatg   13440
   ttagttagga tggttttgat tttttgattt ggtgatttgt ttattttggt attttaaagt   13500
   gttgggatta gaggtgtgag ttattgtgtt tgatttgaga tgaggtttta ttatgttggt   13560
   taggttagtt ttgaagtttt gattttaagt gatttgttta tttttgtttt taaaagtgtt   13620
   gagattatag gtgtgagtta tagtatttgg tttgtttttt tttttattta taggaattta   13680
   tatttatttt gttttattta ttagattagt agtttttaaa ttttagtgta tatagaaatt   13740
   atttggagag tttatttaat atgtaaattt taaagtttta tttttagagt gggttggtgg   13800
   tttttttgtt ttttgttatt ggaatgtgga ttagggatta ggattagtag tattttttgt   13860
   aagttgataa aaatattaaa ttttgggttt ttattttgaa tgagaattag tattatgaag   13920
   atttttgggt atttattttt atattaaagt ttgagagtga gagtttagtt tagtattttt   13980
   taaatttgtt tgagatttat agtaggtaat gtattttatg tttgatattt attatattta   14040
   tatatattta tgtgaaatag aaattttata aaataatttt tatttttaag ggatttttat   14100
   taatattttt tattttattt tatatttttt ttttttttta atgtgggttt tgattattaa   14160
   attgattttt tattttatta agatatgtaa ttttgtaatt tattatttga aaaatattgt   14220
   tttagtgatt agattttagt tatattagtt tgggtagaga tgaattagaa attggtttat   14280
   ttatggtaga agttatattt atttagtatg ttttttttta tttttggggt ggaaggtagg   14340
   gaagaagaat aagtttttat aaataggagt atatattttt ataaatagga gtagtttttg   14400
   tagttttggg aaattgagga tgtttttttt tatttgggta attttttttt ggttagagat   14460
   atttttggtt agttttttgt attagttttt aggagggagg agttgtgtat ggagggtttg   14520
   tggaaaagag tttagttttt ttattgggat gtttttattg ggtaaggtga gggtgtgtat   14580
   ttatttttag agggtttaga tggttgtttt tttggttttg tgatttgaag gtttatagga   14640
   atatttagaa attttttata gtagttttta tattatataa ggagtttttg gttttgatgg   14700
   atttttttgt agtttttata aaatatatgt ttttgatatt gtatagtaat taggttttgt   14760
   tttttgggga agtgggtata gttgatatag gatatttagt tttttgtatt ttttttttta   14820
   tttataaagt ggtttaaatg attattttta aagatttaaa tgaaataaga tatgaagttt   14880
   gtattgtatt ttttttttgg tttaatttat aaaataaatt ttttaatatt tgtaatttaa   14940
   atattatatt tgagtttatt aaatatattt ttattatttt gatttttatg ttattataag 15000
   tagtttataa atttatttat gttaatatag atttgttagt ttaaggatat ttttttaggt 15060
   aatgttatat attgggtttg tttgtttttt ttagagatag agttttattt tgttgtttag 15120
   attggagtgt agtggtttaa ttataggtta tagtagtttt gaatttttgg gtttaaatga 15180
   tttttttatt ttagtttttt gagtagttgg aattaaaggt atgtattatt atgtttggta 15240
   aattttttta aaaaaatttt ttgtagagat agggttttat ttttttttag gttggtttta 15300
   aatttttggg tttaagtgat ttttttattt tggtttttta aagtgttaga attataggtg 15360
   taagttattg agtttgttta atgtgatata ttttaaaaga tttttatttt tattattttt 15420
   ttgtttttta aatttttata gtgagtatat gttaatttta taagtagagg taagtgaaat 15480
   aaaaaggttt tatttaattt tattatttaa atataattga atagttttat attttatttt 15540
   tagttaggtt ttatgtatat taatggagtt atagattatt ttttttaagg ttgggtttgg 15600
   tggtttatat ttgtaatttt tgtattttgg gaggttaaag taggtggatt atgaggttag 15660
   gagtttgaga ttagtttggt taatatggtg aaattttttt ttattaaaaa tataaaaatt 15720
   agttgggtat ggtggtatat gtttgtaatt ttagttattt ggaagattga ggtaagagaa 15780
   ttgtttgaat ttaggaggtg gaggttatag tgagttgaga ttatgttatt gtattttaat 15840
   ttgggtaata gataaaaatt ttgttttaag aaaaaaaaaa taaaaaaaaa aaaataaaga 15900
   aagattattt ttttttttgt tgttggtaat tttgttgttt ttaatatttt gttattataa 15960
   ataattttag gattaatata ttttatatag ttttaatttt agattatttt tttgtattat 16020
   atttttagaa gtagaaattt agggttaagg gattttgaat attttaaggt atttgatata 16080
   ttttgataaa tttttttttg taggtaataa attgtagttt tattagtata tttttaatat 16140
   attattatta gattttgaag agtatttggt tatttttttt ttaatttttg attaatttta 16200
   taggtgaata atattgaatt ttaaaatttg tttttatttt ttttagtata gttttagtat 16260
   tttataatat ttaaagtatt tagttttgtt tataaatata atataatata ttgatttttt 16320
   aaatgtatat tttattgtta attttttaga ttaaaatgat ttaaggtatt tataaaatat 16380
   agaatttatg tagaggtgtt atttttatgg agtaaatatt ggttgttttt tttttttata 16440
   ggtaatataa attatagata tttgttttgg atgttttttt tttttgatta ggattttttt 16500
   aaaagggtaa aggttaagat ttttttggat tagttttttg ttttattttt agtagtagat 16560
   ttattagaaa tagatttatt ttttatatta tagaattgtt tttgtaggtt tttattttat 16620
   tggtattgat tattttattt tgaaagggtt taaatttgat ttttttggtg ataaaattat 16680
   tgtagaatat attaaaatgt gggattttat taaatattgt ttgtatgagg tatagattta 16740
   ggggaaagtt gaggtaggaa aagagaatga ggagagttga ttttgtatta gtttagggaa 16800
   agaaatttat gtaaggagtt ttgtttattt tttggaagtt gaagggagtt tttatttaga 16860
   gtttgaggaa gagaattaga ttttagtaaa aggtaggtta aaataatttt ttattaaaaa 16920
   tgtattataa agtgttttta tgaggtgttt ttttttttaa ttttttaaag gtaattatta 16980
   ttttagtttg atatttattt gttgttttta tttttttttg tttattttat atggtttgtt 17040
   tatatatgat tttattgtaa ataattttat atataatttt ttatttgaga tttagaatag 17100
   ttttatggaa gtttatgtaa ttaattttat taggtaaatt taaggaattt gaggattaga 17160
   ggtaatgttt gattaaggtt agtgtagtta gttgagttgg gattgttagg tgtgaattgt 17220
   tgaaggttgt tattgtatgt tagtatagaa tttgaattta tttgggagtt tgttgtagga 17280
   attatttttt ttttggtttt tagtttttat taaaattaat tttagttatg tagttaagta 17340
   agtagggttt tagaagtgag tttagaatag ttttggtttt tatggaggta tttttttttt 17400
   tttgtatttt gtagttttag ttttatttgg aagtgttttt ttttggatat ttttgttttt 17460
   gtttgtattt ttgtttaatt tgatttttaa atggtgtgaa ttttttttat tagagttgtt 17520
   tggggggatt aggaggtttg aaattgtatg gtgtaggttg gttgggtgtt ttaggttggg 17580
   agtgattttg tatagaatga agtgtaggtt tttttttgtg aattggaatt gagtagggga 17640
   tttttttttt tggttggagg gtttgggatt attttttgat tgtttgttgg gtgagtaagg 17700
   taataattgg gaggttttta gttttttagg taatagagaa gtatttgtag ggggtggtgg 17760
   aattaaagtg ttaggtttat aatatgggtt atttttgttg ttgttattaa tgatttggtg 17820
   gtttagattt ttattgttgg tatttgtgta tttgtggttg ttgtttagga agggtggtat 17880
   ttttttgggg atgtggggaa ttgggtatta tggtggtagt ttgttgtatt agggtagatt 17940
   gtagagaggt tttttttgta ggtttagatt tgggtttagt aagggaggtg ttatttggaa 18000
   ggggaagggg ggtatggtgg gggtagtaga tgttgtggtt tttagttgtt ggaaaggttg 18060
   gaagtttttt tttatggtgg ttaggttgtt tttgtagtgt ttataggatg ggaaaggtgt 18120
   gtagtatgtg gttaggtttt gtgggttgtt ttttggtggg tagtatattt tgttttgagg 18180
   tatggtttta tttggttggg gtagagttat tttgggattg tatgtttggt ggttttgtag 18240
   aaaagtgggt gttgttgagg atattgtgtg ggagtttttt ggggtttgat ttgtgttttt 18300
   ttattagttt tttttatttt agttagtttt agatttaagg gaataggata aatgttttta 18360
   gtttgatttt gttagtttgt ttttataatt aagataaaat gaaaaaatag taatagaagt 18420
   aaatataaaa aagttttaaa tggaaatttg ttgggatttt gttttatttt aaattttggg 18480
   ttagtgttgt tttttttttt tttttttaag tatttttttt ttaagtttgg ttgttttttt 18540
   atatttgttt tttgtttttg gtttttaatt tggtttttgt aaaatttatt gaagtttttt 18600
   tttttttttt ttttttagtt ttataaaatt tgttggtagg gtttgtggga gttgtggggt 18660
   tttgttaatt ttggggttgg gttgggagtt gagtgtaggt ggttggaggg taatggagta 18720
   agtgatagtt ttaggtgttt ttgggttttt ggttgggagg gaagagttga ggattttggg 18780
   ttgtattgtg tagatggaga tgtgttttta gagtttttgg gtaggtttag ggattttgtg 18840
   atttgttttg gtttagtggt gtgattttgt gggttttggt ggttttgatt gtttgtaggg    18900
   gaggggtgtg ttatttttgg ttgttttagg atgtgttgtt tgaatttttt tttttttgtg   18960
   ggtagtgttt gttatatttt ttgggttttt tggaaatttt aattatttta ttaggattat    19020
   tggggtttgg ggtagttttt tgggagttgt gtggatgagt tttggttagg tgggagtgaa    19080
   gagttttggt gattgttagt ttttttgttt ttgtattgtt gggaaaggat ggtgttttaa    19140
   tagataggta gtaagtttat tgaggttgaa gaatgaagtt tttttagggt tggtgggttt    19200
   tgagattaat gagtttaata agaaaattaa gtagggttat gatttagaaa ttaaagaata    19260
   tggggttagg agaagaggtt agttatgata taggtagtgg tttgtttttt tttgattttt    19320
   taaaggtgtt ttttttttag ttttttttga ttttagggat ttgtaatttg ggatttaaag      19380
   tttttgagag ggtagaatga ttatttaaaa tgaattgtgt tagtgagtgt tgttgttttt    19440
   tttaatggtt ttggaagatt attgttttgg gttttaggtg tgttttgttt tgtgtttatt    19500
   tttgatatta tttttaagag tgttattgtg gagtgggtgt tttttggatg ttgtggggtt    19560
   atttgggttt ggtaggttat ttgggtttgg tagattttgg gtgggttttg agggttgagt    19620
   taggtttaag agggtgggtg gggttgtggg gtattgatag gttgagggta gggaattgat    19680
   ttggtttatg tagatttttg tgttgttttt tttttgttta ttgtagttat tggttgattt    19740
   ttgatttgtt ttttgttttt tggggttgtt tatttaattt attagttttt taaatttttt    19800
   ttgggatttt agtggtgagt atttgttgtt ggttttttag ggtaggtagt attgttgttt    19860
   taggtgtgtg gagttgggga gaaaaaaatt gaatatgtgt gttatttagg gtttttagat    19920
   aagttttttt gtgtgtatat atattttgaa ttggagttta gaaggttgga tttgtgtttt    19980
   ggggttgagt tttttatttt ttgtttttag tttggaagat tttagtttgt ttgaaggtgg    20040
   tgt                                                                  20043
<210> 114
   <211> 20043
   <212> DNA
   <213> Artificial Sequence
<220>
   <223> chemically treated genomic DNA (Homo sapiens)
<400> 114
   atgttgtttt tgggtaggtt gggatttttt gaattgggag tggagagtaa ggggtttgat      60
   tttgggatgt gagtttggtt ttttggattt tggtttaggg tgtgtgtgtg tgtggagggg     120
   tttatttggg ggttttgggt agtatatgtg tttggttttt ttttttttga ttttgtatgt     180
   ttggagtggt aatattgttt gttttagaag gttagtggtg agtgtttgtt attagggttt     240
   tagggagggt ttggaaaatt gatgagttaa gtgagtgatt ttaggggata gagggtgagt     300
   tgagagttgg ttaatggttg tggtgggtgg ggagaagatg atgtggggat ttgtgtgggt     360
   tgggttaatt ttttattttt gatttgttga tgttttgtgg ttttgtttgt ttttttaagt    420
   ttggtttagt ttttagggtt tgtttgaagt ttattgagtt tgagtggttt attgagtttg     480
   agtggttttg tagtgtttag gaggtgtttg ttttgtggtg gtgtttttgg aggtggtgtt     540
   ggaggtaggt atgggatagg atgtgtttgg ggtttggggt ggtggttttt tagggttgtt     600
   ggggagggtg gtagtatttg ttggtgtagt ttgttttgga tggttgtttt gtttttttgg     660
   gggttttgaa ttttaagttg tagatttttg aggttggagg aggttaggag aagggtgttt    720
   ttggaggatt gggaggagat gggttgttgt ttgtgttgtg gttgattttt ttttttgatt     780
   ttgtgttttt taatttttga gttatgattt tgtttggttt ttttattggg tttattgatt     840
   ttaagatttg ttggttttga aggggtttta ttttttagtt ttggtgaatt tgttgtttgt    900
   ttattaaaat gttatttttt tttggtggtg tgggggtggg aggattggta gttgttgagg     960
   ttttttgttt ttatttggtt agggtttgtt tatgtggttt ttgaggggtt attttaggtt   1020
   ttaatagttt tggtagaatg attggagttt ttgaggaatt tggggaatgt ggtggatgtt    1080
   gtttgtgagg gaaaagaggt ttaggtggtg tattttaggg tagttaaaag tggtgtgttt    1140
   tttttttgtg ggtagttagg attgttagga tttgtggggt tatattgttg ggttagagta    1200
   ggttgtgggg tttttggatt tgtttggggg ttttgggagt gtgtttttat ttgtgtggtg    1260
   tgatttaggg tttttggttt ttttttttta gttgagggtt taggagtgtt tggagttgtt   1320
   gtttgtttta ttgttttttg attgtttgtg tttggttttt ggtttagttt tgaggttggt   1380
   agggttttgt ggtttttata gattttatta atgagttttg taggattgag aagaaggaag    1440
   gaaagggaat tttaatgggt tttgtaggaa ttgggttggg ggttgaaagt ggagagtggg    1500
   tgtgggaagg tggttgggtt tagggaaggg gtgtttggag agggaagggg aaggtaatat    1560
   taatttggaa tttagagtag ggtaggattt tggtagattt ttgtttgggg tttttttgtg    1620
   tttgttttta ttgttgtttt tttgttttgt tttaattgtg ggagtgggtt ggtgggattg    1680
   gattgggggt gtttattttg tttttttgga tttggggttg gttggggtgg aggaggttgg    1740
   tggggaggtg tgggttggat tttgggaagt ttttgtgtgg tgtttttagt ggtgtttgtt    1800
   tttttgtaga gttgttgagt gtgtggtttt gggatggttt tattttggtt aagtgaggtt    1860
   gtgttttagg ataaggtgtg ttatttgttg gagagtagtt tgtagaattt ggttgtgtat    1920
   tatatgtttt ttttgtttta tggatattat agaaatagtt tggttattgt ggaggaagat    1980
   ttttaatttt tttggtagtt ggaggttgta gtgtttgttg tttttgttat gttttttttt    2040
   tttttttgga tggtgttttt tttgttgagt ttgggtttgg gtttatagag ggagtttttt   2100
   tatgatttgt tttggtatag taagttgtta ttgtggtatt taatttttta tgtttttagg    2160
   gaagtgttgt tttttttgag tagtagttat gagtatgtgg gtgttagtag tgaagatttg    2220
   ggttattaaa ttattggtgg tgataatgag agtggtttgt gttgtggatt tgatatttta    2280
   attttattgt tttttgtgga tgtttttttg ttatttgagg ggttgaggat tttttagtta   2340
   ttattttgtt tatttagtaa gtagttagag gatggtttta aattttttaa ttaagaaggg   2400
   aagttttttg tttggtttta gtttatggag gaggatttgt attttgtttt gtatggggtt   2460
   atttttagtt tggagtattt agttagtttg tattatgtga ttttaggttt tttggttttt    2520
   ttagatagtt ttggtaaagg gagtttatat tatttgggag ttgaattgga tgggaatgta    2580
   ggtgggaatg ggagtgtttg ggaagaggtg tttttaggtg gagttgaggt tgtagagtgt    2640
   gggaaggggg aagtattttt atgagagtta gggttgtttt aggtttattt ttgaggtttt    2700
   gtttgtttaa ttgtatgatt aaggttggtt ttagtgagag ttgaggatta agagagaaat    2760
   ggtttttgta gtgaattttt aggtaggttt agattttatg ttaatatgta atgatagttt    2820
   ttagtagttt atatttaata gttttagttt agttagttat gttgatttta gttaggtatt    2880
   atttttgatt tttaaatttt ttgaatttgt ttaatggggt taattatata aatttttatg    2940
   aggttgtttt gagttttaaa tgagaaattg tatatggaat tgtttatagt gaaattgtgt    3000
   atgaatagat tatgtaaaat gagtagggaa aaatagaaat aataaataaa tattaaattg    3060
   ggatgatggt tatttttagg gaattaagga gggaaatatt ttataaaagt attttataat    3120
   gtatttttaa tgaaaaattg ttttaatttg ttttttgtta ggatttgatt ttttttttta     3180
   aattttggat aaagattttt tttaattttt agaaggtagg taggattttt tgtatggatt    3240
   tttttttttg aattaatgta gagttaattt tttttatttt ttttttttgt tttggttttt    3300
   ttttaggttt atgttttatg tagatggtgt ttggtgaagt tttatatttt ggtgtgtttt    3360
   gtagtagttt tattgttaaa ggagttaggt ttgggttttt ttaaggtaaa gtggttaatg    3420
   ttagtgaagt gaagatttat ggagataatt ttgtgatgtg ggaggtaaat ttgtttttgg    3480
   tgggtttgtt gttgaaaatg gggtgggaga ttagtttggg gaaattttgg tttttgtttt    3540
   tttgggagag ttttggttag agagagagga tatttagggt agatatttgt ggtttgtgtt    3600
   gtttgtaaaa ggaaggaatg gttagtgttt gttttatgag gatagtattt ttgtatgaat    3660
   tttgtgtttt gtgggtgttt taagttattt tagtttggaa gattagtaat ggaatatata    3720
   tttaaagaat taatatgttg tattatattt atagataaaa ttaaatgttt tagatattat    3780
   agagtgttga aattatgtta aagagaataa aaataagttt taaaatttaa tattatttat    3840
   ttataaaatt agttaaggat taaaaaaaaa ataattagat attttttaag atttagtgat   3900
   gatatattag ggatatgtta gtgggattgt agtttattat ttgtaggaga aaatttgtta    3960
   gaatgtatta gatattttaa aatgtttaaa gttttttgat tttgaatttt tatttttagg   4020
   aatatagtat aggaaaataa tttgaaattg aggttatgtg gaatatgtta attttagggt    4080
   tgtttataat aataaaatgt tggagatagt gaaattgtta gtagtagaga aagaagtaat    4140
   ttttttttgt tttttttttt ttgttttttt ttttttgaga tggagttttt gtttgttatt     4200
   taggttggag tgtagtggtg tgattttagt ttattgtgat ttttattttt tgggtttaag    4260
   taattttttt gttttagttt tttgagtagt taggattata ggtatgtgtt attatgtttg   4320
   gttaattttt gtatttttag tagagagggg ttttattatg ttggttaggt tggttttgaa    4380
   tttttgattt tatgatttgt ttgttttggt tttttaaagt gtggggatta taggtgtgag    4440
   ttattaggtt tggttttgaa agaaataatt tatagtttta ttaatatgta tgaaatttaa    4500
   ttgggagtaa aatataaaat tatttagttg tgtttaagtg gtggaattaa ataaaatttt    4560
   tttgttttat ttatttttgt ttatgaaatt aatatgtgtt tattatagga atttgggaag    4620
   taagaaagtg ataaaagtaa gaatttttta gaatatgtta tattaggtag gtttagtggt   4680
   ttatatttgt aattttagta ttttaggagg ttaaggtggg aggattattt gaatttagga    4740
   gtttgaggtt agtttaggaa agagtgaggt tttgttttta taaaaaattt ttttaaaaaa    4800
   atttgttagg tgtgatggtg tatgttttta gttttagtta tttagggggt taaggtggga    4860
   ggattatttg agtttaggaa tttgaggtta ttgtgatttg tgattgagtt attgtatttt    4920
   agtttgggta atagagtgag attttgtttt tgaaaaaaat aaataaattt aatatgtgat    4980
   attatttaga gaggtgtttt taaattaata aatttatgtt ggtataagta aatttataaa    5040
   ttatttgtaa tgatataaaa attagaatga tagaagtgta tttggtggat ttaagtgtga    5100
   tatttaggtt ataagtatta aaaaatttat tttatagatt agattaaaaa agaagtataa    5160
   tataaatttt atgttttatt ttatttagat ttttgaagat ggttatttga gttattttat    5220
   agatggaaaa ggaggtatgg ggaattggat gttttatgtt aattgtgttt gtttttttaa    5280
   ggagtagaat ttagttgttg tgtagtgtta agggtatata ttttatgaga gttgtaaaga    5340
   gatttattag aattaagagt tttttgtgtg gtatggagat tgttatgaga aatttttgga    5400
   tatttttgtg agtttttagg ttatagagtt ggggaagtag ttatttgggt tttttgaagg    5460
   tgagtgtata tttttgtttt gtttaatgag ggtattttag tagaaggatt gggttttttt    5520
   ttataaattt tttatgtata gttttttttt tttaaaggtt aatgtagagg attaattgag    5580
   agtgttttta attagggggg aattatttag gtgaaggaaa gtatttttaa ttttttaaag   5640
   ttgtggaagt tatttttatt tataaagata tatattttta tttataaaga tttgtttttt    5700
   ttttttattt tttattttaa gagtaaaaga aaatgtatta aatggatgta atttttatta    5760
   taggtgagtt agtttttgat ttatttttgt ttaggttaat gtagttgaag tttaattatt    5820
   agagtagtgt tttttaaatg gtgggttgta gagttgtatg ttttagtggg gtgggaaatt    5880
   aatttagtgg ttaagattta tattaaaaaa aaaaaaaagt atagagtaga atagaaaata 5940
   ttgatgggaa ttttttaaga gtaaggattg ttttgtgaaa tttttatttt atatgggtat 6000
   atgtgggtat ggtgggtgtt aagtataaaa tatattgttt attatgggtt ttaggtaagt 6060
   ttgagaaata ttgagttgga tttttatttt taaattttaa tgtgggaatg aatatttagg 6120
   gatttttatg atgttggttt ttatttaggg taggggttta ggatttggta tttttattaa 6180
   tttgtaggga gtgttgttgg ttttggtttt tggtttatat tttagtagta aggggtgagg 6240
   gaattattag tttattttag gggtgagatt ttggaatttg tatgttaaat aagtttttta 6300
   agtgattttt atatatattg aagtttagga attattaatt tagtaggtgg gataagataa 6360
   atgtaaattt ttgtaaataa gagagaaggt aggttggata ttgtggttta tatttgtaat 6420
   tttagtattt ttggaggtgg agatgggtgg attatttgag gttaggattt taagattagt 6480
   ttggttaata tggtaaaatt ttattttagg ttgggtatag tggtttatgt ttttaatttt 6540
   agtattttgg gatgttgaaa tgggtagatt attaggttag gagattaaga ttattttggt 6600
   taatgtggtg aaaatttgtt tttattaaaa atataaaaaa ttagtggggt gtagtggtat 6660
   atatttgtag ttttagttat ttaggaggtt gaggtaggag aattatttga atttaggaga 6720
   tagaggttgt aatatgttaa gattgtatta ttgtatttta gtttgggtaa tagagtaaga 6780
   ttttgttttt aaaaaaaaaa aaagagagag agagaaggta aattattatg atgtaataaa 6840
   tgtgtaagga gtaggtttaa aaaaaaaatg gtttaggtgt ggtggtttat gtttgtaatt 6900
   ttagtatttt gggaggtgag gtgggtggat tatgaggtta ggagattata gttattttgg 6960
   ttaatatggt gaaatttttt ttttattaaa aatataaaaa aattagttgg gtgtggtggt 7020
   gggtttttgt agttttagtt atttgggagg ttgaggtagg agaatggtgt gaatttagta 7080
   gggggagttt gtagtgagtt gagattgtgt tattgtatat tagtttgggt gatagagtga 7140
   gattttgttt taaaaaaaaa aaaaaaaaaa gtaaattgta tttgtatgtt tgtttttttg 7200
   ttaggttgag atgttttagg gtaggaaata attttaaata ttttagtatt ttgtgttttt 7260
   ggattttagt gtgtattgat tgagaggaag atattagaat taagttagaa aaagttgttt 7320
   agggatgaga tgaaataagg tatgtgtaag agggagagag ggaaggtaat taggtttagg 7380
   gaatagtgaa gtagaaggta aggaaatgtt tagaaattag ggggtaaata ataagtagtt 7440
   attatttatt taatgataaa taatgagtag ttagttgtta ttatttattt aatgataaat 7500
   aatggtttta atgatgaggg gttgttagga ttgttttggt gaattatttt tttatttttt 7560
   taggtatttt atgtttatgt tgggtttttt ttgttattga attattataa gtttttattt 7620
   tgttgtttgt tttgattgtt tttgatattt gtatataggt tgagtatttt ttttttgaaa 7680
   tgtttgggat tagaagtgtt ttggtgggtg gtgtgggtgt gggaatgtag gtgaagatag 7740
   gaatggaggt aaaaatggtt gggatttttt atgatttgtg gagttttaag ttgtttttta 7800
   tttagtatta aattaagatt tgttggttat tttttgaaat gtattaattt tttttgaaat 7860
   aaaagttaat aggttgggtg tggtggttta tatttgtaat tttaatattt tgggaggtta 7920
   aggtggttgg gttatttgag gttaggagtt tatgattagt ttggttaata tggtgaaatt 7980
   ttgtttttat taaaatataa aaaattagtt gggtgtggtg gtgtatgttt gtaattttag 8040
   ttatttagga ggttgagata ggagaattgt ttgaatttag gagtagaggt tgtagtgagt 8100
   tgagatggtg ttattgtatt ttagtttggg tgatagagtg agattttatt ttaaaaagaa 8160
   aaatgaaaga aagaaaagtg aataatggat ttgagaaaag tatttgttta gtaattggta 8220
   agatggttat tatttttttg agaaattatt attatttttt gagatagggt tttattttgt 8280
   tatttagatt ggagtatagt agtgtaattt aggtttattg taatttttgt ttttagggtt 8340
   taggtatttt ttttgtttta gttttttgag tagttgggat tataggtatg tattattata 8400
   tttagttaat ttttgtattt ttagtagaga tggggttttg ttattttggt taggttggtt 8460
   ttgaattttt ggatttaagt gatttattta ttttggtttt tttaaatttt gggataatag 8520
   gtatgagata ttatggttgg ttatttttga gaaatttttt tattgtatgt attaaattat 8580
   tataatgttt gtaattttga tgttttagta atattttttt ggaaatttat tattgagaaa 8640
   tagtttaaaa ggaaaaagtt atgtttatga aaatttttaa ttgtggtatt atttttgtga 8700
   gtgaaaaatt ataaataatt taaatgttta gtagtataat aattaagtaa atgatgatat 8760
   aggtttagta gttttaatta agaaatttta aatttaaaat gttttaaaat ttgaaattga 8820
   gtattgatat attgttaaaa gtgggaattt ttatatatta gtatttgata taaattttgt 8880
   tttatgtata aaattattta aaatattgtg taaaattatt tttaggtggg ttaggtatgg 8940
   tggtttatgt ttgtaatttt agtgttttgg gaggttgagg taggtagatt atttgagatt 9000
   aggagtttga gaatagtatg ataaatatgg tggaattttg tttttattaa aaatataaat 9060
   aaattagttg ggtatggtgg tagatgtttg taattttagt tatttaggag gttggagttg 9120
   gataattgtt tgaaattggg ggtggaggtt gtagattgag taagattttg ttttaaaaaa 9180
   aaaaaaatta tttttaggtt atgtgtataa agtatgtata aaataaaata taaatggatt 9240
   ttatatttag attttggttt tatttttaag atattttatt atgtatatga aatattttaa 9300
   agtttgaaaa aaatttgaaa tttaaaatat ttttggtttt aagtatttta gaaaaaggat 9360
   atttaattta tttgttatta atatggaata gtatgttgat attagaggta attagagtaa 9420
   gtaatagaat gagggtttat gatggttaag tgtaggggat atttagtatg tagataagat 9480
   atttgtgttt tagttttgaa ttaagtgata tttgatgttt atagtattat tatgaggata 9540
   tatattttgg ataatggagg gaaatttgta aataatttaa tttgttggga ataattataa 9600
   atgaatgtta atttgttaat ttgttaatat gaatggtttg gatgtgataa aaaggaaaga 9660
   aaaattaaaa ttttattgta tttttaaatg agtttttttt tttaaaggtt ttttggtggg 9720
   gattggtggt aggtgggagt tttttttttt aaggttttga gtggtgaggg aagggtttag 9780
   attttatgtg ggtgggttat ttgaatatgt aagtattttt tttttgtgag ggagagaggg     9840
   aatttgggtg tgtgttttgg ggtgtggagg tgtgaagagt tagggggagg gaggataggg     9900
   aataagataa aattaggaag gatagggaat aagatgaaat taggttttat ttttatttaa     9960
   gtggttgttt ttaagtgaat ttagtattga aattttattt tgaattttgg tgtgttttag    10020
   tgtttaggtt tgtaaaattg ttgatttttt tttttttttt tattttagga agtaggaagg    10080
   ttaggtattt ttgattaata aaaattttat ttttgtgtta aaaaagtgtt ttagtatggg    10140
   gattttgaga gtattagtat tttaatgaaa gtgatagata gagtagggtg atggattagt    10200
   ttggggttgg tagttgtttt tttggttatt tatttaatga atagtttatt tttttagtta    10260
   tttgttaaat gtgtttggga tgtagttgtt aattaataaa gtttttgatg gatagttttt    10320
   ttgttgtttt gatgttgaga agatttttat ttgtagattt ttgtatagat ttatttttgg    10380
   ttgttttttt tttttggggt taaataatga gatttttgtg gtgtatttat agttaaagtt    10440
   attttatttt ttgggattta gagtgttttt tgtagggatg tttttgtagg tggtttttgt    10500
   ttttttgttt ttttttaata tatagatatt tttggatgtg ggatttgttt tgttagagtt    10560
   tagttgtatt ttttttaatt tttttttttt tttttttttt attgataaaa atagatttat    10620
   tttttagttt tgtttgttat ttagtgatag tattatttat attggtaaga tttggtaagt    10680
   gttagaatta aattagttat tatggtaatt ttgtgtatta gggaagttaa gatgttttta    10740
   attttatttt atatataaag attttagttt tggaatttgt tataagtttt gagtaatgaa    10800
   gtataaatta agaaggtttt ttgaattgtg tagaatagaa tagtattgtg agttgtgggt    10860
   gttttggtga ttgttttgag tttgtaattt aggttgattt taaaggtata aagagaaata    10920
   ggttggtttt agtgttttag tgtattaatt ttgtaataag tgttaggtag gaatgtgttt    10980
   aagagttgta gggttgtatt gtttttaaat ggggttggtg gaatggtttt tttaatagta    11040
   tatattaagg aataaaaggg aaagagagtt tataggttaa gatatttaag agatatagta    11100
   attatatata atatgtagat tttgtttgaa gtttaatttg aatggattga ttagaaagta    11160
   attaaataaa tatatatgtg tgtatataaa tgtgtatata taagtatata tatgttttta    11220
   taaaagaata agagttaatt gtttaggtat gataatgtga taatgatatt atggttatgt    11280
   tttttttttt tttttgagat aaagtgttgt tttgttgttt aggttggagt gtagtggttt    11340
   aattattgtt tattgtagtt ttaattaatt tttttaggtt taggtgattt ttttattttg    11400
   gtttttggga ttaaaggtgt gtgttattat gtttagataa tttttattat ttttttgtag    11460
   agatggggtt ttattatgtt gtttaggttg gttttaaatt tttgggttta agtgatttgt    11520
   ttattttagt tttttagagt gttgggatta taggtgtgag ttattgtatt tggtataatt    11580
   atgttttttt tttttttttt gagatggagt tttgttgttg ttgtttaggt tggagtgtag    11640
   tggtgtaatt ttggtttatt gtaatttgtg ttttttaggt ttaagtgatt tttttgtttt    11700
   agttttttaa gtagttggga ttataggtag gtattattat gtttggttaa ttttgtattt    11760
   ttagtataga tgggatttta ttatgttggt taggttggtt ttgaattttt gattttaggt    11820
   gatttatttg ttttagtttt ttaaagtgtt gggattatag gtgtgagtta ttttgtttgg    11880
   ttataattat gttttttaaa aatatgttat tttttaaaga ttatggttag atgtttttga    11940
   gttaaaggat gaattagtat taaaataatt tattatggtg agtggtgata taaatgaaaa    12000
   taaaattagt tatgagttgg ttaaagtttg gtgtttggta tatgagggtt tattatatta    12060
   ttatttttat tttggtatgt ttatattttt ttgtaataaa aaggttttta aaaatttttt    12120
   tggtggttgg gtatggtggt ttatgtttgt aattttagta ttttgggagg ttgaggtagg    12180
   tggattataa gattaagaga ttaagattgt tttggttaag atggtgaaat tttattttta    12240
   ttaaaaatat aaaaattagt tgggtgtggt ggtgtgtgtt tgtagtttta gttatttggg    12300
   aggttgaggt aggagaatta tttgaatttg ggaggtggag gttgtagtga gttgagatta    12360
   tatttttgta ttttagtttg ggtgatagag taagatttta ttttaaaaaa aaaaaaagaa    12420
   aagaaaagaa aatttttttg attaggtata gtggtttatg tatgtaattt tagtattttg    12480
   ggaggttaag aggggttgat tatttgaggt taggagtttt agattagttt ggttaatatg    12540
   atgaaatttt gtttttatta aaaatataaa aattagttag gtgtggtagt gggtatttgt    12600
   aattttagtt atttgggagg ttgaggtagg agaattgttt gaatttggga ggtagaggtt    12660
   gtagtgagtt aagattgtgt tattgtatta tagattgggt aatatagtaa gattttgttt    12720
   taaaaaaaga aaattttttt tgaagtagtt aaagaatttt tggttaaaaa taaataaata    12780
   aataaatatt tttttgaagg aattagggag ggaagtaatt gaagagatat tgtgtttgtt    12840
   gtgtttgata gagtttgtag aaggttatag atgtgaaaga tgtgggaagg tatttattta    12900
   taaatattat agagataagt attttaagta tattttttgt gtggataagg gtgataggaa    12960
   atttttttgt tttttttgta aatgattttt tgagaagtgg gattggtttt ggatttatat    13020
   tttttatgtt tatgagaagt attggtttta taaggtgagt attaggagga tatatgattt    13080
   attattatgt tttttttttt tttttaagtt tttgaatatg gggagttgtt ttgtaggtgt    13140
   tagttgtagg gttgtgatgt ttgatggata ttttttttga aggaggagag gatagtatta    13200
   tggaataggt aggtgtgaat aggtatagtg atgtatatgt gttaggatgt aggtatttat    13260
   agagggagga atatggagga aggagtgttt atggttattt ggatatgtgg attatgaaag    13320
   atgtatagag gtggttttga gttaagtata gaaggataaa tggggtgttt gatttatatt    13380
   ggtggtaggg ttagttgggt aggaaagagt tgagttgttt tagataggag agagtttggt    13440
   atgtgttttt ggggaattgt gttttgtttt aaatttgtgt aggtgtgttg ataggtatag    13500
   gtaagaagga agtgaaggta tggttttttt aaggagtgga aggtttttat agtgggttga    13560
   ggattttgta agatagaaaa ttagtaatga tattatggag ttattttaaa tatttggatg    13620
   ttttgttagg tttgttgtaa aaaagtataa tatttttttg ggtttaatta aaaggaggat    13680
   gaggtttggg tatagatgta tttttgtttg attgttggtt ttgttggaat tattttaggt 13740
   agttatgatt ttgagtttat ttttttgttt tgaaaattga gaataaaatt attataaaat 13800
   tgttttaatt tttttttttt tttttaagtt tttggaaaag ttatgtttaa ttattatatt 13860
   atatattttt ataaaatttg aatatattta tagttattat gtatttgtgt aattttaaaa 13920
   ggatttaaaa atattaggat ggtgaaggta ttttttgttt attagattgg taaaagtgaa 13980
   aattatttag tattttgata gtgaaaagtt tgagattgta tgtttattaa tataaaattg 14040
   attatataaa ttatggttta ttggttaggt gtagtggttt atgtttgtaa ttttagtatt 14100
   ttgggaggtt gaggtgggta gattatttga ggttaggagt ttgagattag tttggttaat 14160
   atggtgaaat tttattttta ttaaaaatat aaaaatatta gttgggtgtg gtggtaggta 14220
   tttgtagttt tagttatttt agaggttgag gtaggagaat tgttttaatt ttagaggtgg 14280
   aggttgtagt gagttgagat tgtgttattg tattgtagtt tgggtggtag gagtgagatt 14340
   ttattttaaa aaaaaaaaaa aaatattgtt ttattaagta aaaattaaat tatagaagat 14400
   gtatagaatg tttgtttttt ttgaaattgg aggttgggag tggtggttta tgtttaattt 14460
   tagtattttg ggaggttgag ataggtggat tatgaggtta ggagattaag attattgtgg 14520
   ttaatatggt gaaattttat ttttattaaa aatataaaaa attagttagg tgtggtggtg 14580
   ggtatttgta gttttattta tttgggaggt tgaggtagga gaatggtgtg aatttgggag 14640
   gtggagtttg tagtgagttg agattgtgtt attgtatttt agtttgggtg atagagtgag 14700
   attttgtttt aaaaaaaaaa aaaaagaaaa gaaattggag ggttgttaat taggaggtta 14760
   gtagatgata ggattgatga ggaagttggt gatagtgaga tgatggttta ttttttaata 14820
   gatgaggtgt ttttatagta tagtggagga ataatttttt tttttttttt tttttttgag 14880
   atagttttat tttgttgttt aggttagagt ataatggtat gattttagtt tattgtaatt 14940
   tttatttttt atgtttaaat aattatttaa ttttagtttt ttaagtagtt gagttgaggt 15000
   tataagtgta tgttattatg tttagttaat tttttgtatt ttttgtagag atgggtatgt 15060
   ggtgtgtatt tgtggtttta gttatttggg aggttgagat attagaattg tttaaattta 15120
   ggaggtagag gttgtggtgg gttgagatta tgttattgta ttttagtttg ggtaatagag 15180
   tgaggttttg ttttaaaaat aaataaaaat aaataaataa ataaatataa agatttgttt 15240
   ttttttttgt tttataagta tgtttggaaa gaggttgtga aagtatagaa atttttttta 15300
   gagaaaaatt gttttttttt tttagttagt gtaatttttt taaaagtgat gttaaaatta 15360
   aaggtttttt gttttatttt tggtaaattt ttggtgtttg tatttttttg gtagtttatt 15420
   ttatttggtg tagaattttt gagatagttt tgagaggagt tgttagggtt aagaaataat 15480
   attaattatt tatagtgtgt gaataaaata ttaatttttt tatgtaagtt taatatgttt 15540
   tagataattt gttattgtgt attattatgt tggtttatat gtttattgtt attttttttt 15600
   gatttttagt ttttggtttt tagtagtgtg gagtatatag taggtatttt gtaagttgta 15660
   gataattgtt gagttaattg tatagtattt gtagtttatt tatagttttg tgaggaaggt 15720
   agagtaggta ttttgtttat gagtaagatt gggaaattaa gattttagat ttaattaatg 15780
   ttttgtagtt atatagttat ttgttgagag agtaggatta atttaggttt ttgttatata 15840
   ttataaattt gtttagtttt tatattttat atttgaagta agagtttttt taggttgggt 15900
   gttgtggttt aaggttataa ttttagtatt ttgggaggtt aaggtaggag gatttgttgg 15960
   agtttaagat aaggttgggt aatatagtga gattttattt ttataaaaaa aaatttttta 16020
   agttagttgg tttagtggta tgtatttgta gttttagtta tttaggaggt tgaggtggga 16080
   ggattgtttg agtttgggag ttttaggttg tagtgaattg tgattgtgtt attgtagttt 16140
   agtttgggta ataaattgag attttgtttt aaaaaaaaaa aaaagaaaaa aattggaaaa 16200
   gagttttttt ttaattttag gtaaatgtaa ggttgaaatg gatttgttag aggaaggtgt 16260
   gattttaatt atattttttt tttttatttt agtgttttat atgtgggaaa tgtttttttt 16320
   aattttttag tttaaataaa tatatgtgag tttattttgg agatagatta tattagtgtg 16380
   tgtattgtat taaggtaaat ggaatttttt attaagggat gtgttttgag ttttgtttga 16440
   gtgtttatgg ataagaattt gaaataaagt ttggtttaat gagtttttgg taaatgttag 16500
   ttaaatatta gtttagtttt tatttttatt tttaaatatg tttaagtttg ttaaagatgg 16560
   ttaaatataa ttaatttttt attgttaagt tggtatttag agtatatgta ataaaatata 16620
   ttttaaattt atggggattt gatgatttat tatatagtat ttaaaagtat tagttgagtg 16680
   tggtggttta tatttataat taaatttttt atgtattggt ggtttatgtt tgtagtttta 16740
   gttatttggg aggttgaggt tggagaattg tttgagttta ggaagtggag gttgtagtga 16800
   gttaagattg tattattgta tattttagtt tgggtgatag agtgagatat tgttttaaaa 16860
   aaaaaaggag ggggtgggga ggtgtggtgg tttaagtttg taattttagt attttgggag 16920
   gttgaggtgg gtaggtgttt ggagataggg agtttgagat tattttggtt agtatggtga 16980
   aattttattt ttattaaaaa tataaaaatt agttgggtat ggtggtatgt ttttgtaatt 17040
   ttagttattt gggaggttga ggtaggagaa ttatttgaat ttagaggtag aggttgtagt 17100
   gagttaagat tgtgttattg tattttagtt tgagtggtaa agtgaaattt tgtttttaaa 17160
   aaaaaaaaaa aaaaaaaatt attattttat agtatttaag aatataaata tggttaagtt 17220
   ttaaattttg tttttggtgt ttatttagtt gtgttatttt gagtaagttt atgaattttt 17280
   gttagttttt atattgataa attggggaga atagttttta ttaagttgtt ggataatatt 17340
   agtaaagtat ataaaatttt tgtaatagtg tttggtataa tataaaagta ttattggaat 17400
   gttaattgtt attattatta ttttaatttg tgaaggatgt tattgatgta attttttata 17460
   ggagtattgt agaagtggta tttagtttat gttatataga atatagagtt gtaaaaatag 17520
   agatgaatta tgtggttaga ggttgttata atagaaataa taggttgatt atgttttaga 17580
   tatattatat taaaatagtt ttatgttatt taatgtgtag tttgtggtta attatatgtg 17640
   gttattttaa ttagtaaaga tgaagtaaag ttgaaaaatt ttgtttttta ggttgtatta 17700
   gttatatttt aagtgtttaa tggttttatg tggttattga tttttaagat ggatagtgtg 17760
   ggtaatgaat atttttatta ttgtagtaag ttttgttgga aagtattggt atagattatg 17820
   tttttagtta ttgagttatt tatgtttttt atagtaaagt tgtaaaagga gtagaggttg 17880
   ttttatagtt atttttaagt tttttaaaag gaataaaata ttgagtttat tagattagag 17940
   taattaatat ttttaaagtg tgtgttaata gaatgaaaat aatgggtata gaaaaatatt 18000
   attgagttgg gtgtggtggt ttatatttgt aattttagta ttttgagagg ttaaggtggg 18060
   tggattaatt tgaggttagg agtttgaaat tagtttggtt gatatggaga aatattattt 18120
   ttattaaaaa tagaaaaatt agttgggtat tatagtgtat ttgtaatttt agttatttgg 18180
   gaggttgagg tattagaatt gtttgaattt gggaggtgga ggttgtagtg agttaagatt 18240
   gtgttattgt attttaattt gggtgatgga gtgagatttt gttttaaaat aaataaataa 18300
   ataaataaat aaaataaatt tataaattat tttaaaaagt ttaaattaaa aagaagaaga 18360
   agaagaagaa aattagtaag gtattgattt atgtaaagat attggttgat attagttttt 18420
   ggttgatatt agtttttttt tttgtaatga ggtttgtttt gaagtatgat tttaggattt 18480
   atatgtatta gaattgtttg tttaaatgta ggtttttggg ttttatgtta gatttattgg 18540
   gttatatttt tttgggtggg gttggtaatt tgtttttttt tttttttttt tttttaaaga 18600
   gagggttttg ttttgttatt taggttggag tgtagtggtg ttattataat ttattgtagt 18660
   tttgattttt tggtttaagt tattttttta ttttagtttt ttaaatagtt gggattatag 18720
   gtatatgtta ttatgtttag gtaatttttt tattttttgt agaggtgagg tattgttatg 18780
   ttgtttaggt tggtagtttg tttttttttt tttttttttt ttttttttgg agatagagtt 18840
   ttattttgtt atttaggttg gagtatagtg gtgtaataat ggtttattgt aatttttgtt 18900
   ttttgggttt aagaaatttt tttattttag ttttttgaga agttgggatt aaaggtgtat 18960
   gatagtatgt ttgattaatt tttgtatttt tagtagagat ggggttttat tatgttggtt 19020
   aggttggttt tgaatttttg attttaggtg atttatttgt tttggttttt taaagtgttg 19080
   ggattatagg tgtgaattat tgtgtttagt ttgtaatttg tttttttata aattggattt 19140
   ttagataatt tagttattga agaattattg ttttaatatt tagtgttttt taaaattagt 19200
   tagaaggata tattagtgga gtttttttgg attaaggtta ttgatgatga ttaggagtgt 19260
   tttatggttt taaaatttaa ggtttgttta taaggatgag taatagtgaa atttattttt 19320
   tagtttaatt ggttgtttag tttgtggaag ttgatttttt tttgtgtttt gtatttttgt 19380
   tttttgtaga ggtttatagt ttttagtata ttttgtatat atattaggta gtattttggg 19440
   gagaagtttt ttaaatgtaa gtattgtggt aaattttttg tattttatgt tgtttatgat 19500
   agttatgttt ggtgtttata taaggaggat gatggttgtt tatgtagtat ttgtgggaaa 19560
   atttttttag attaagaaat attttatttt tatatgaagt tttatgaaga ttattagttt 19620
   tgttaggtat aatgatttat gtttgtaatt ttagtatttt gggaggtaga ggtgggtgga 19680
   ttatttaagt ttaggagttt gagattagtt tgggtaatat ggtgaaattt tgtttttatt 19740
   aaaaaaatat aaaaattagt tgggggtggt ggtatatgtt tgtggtttta gttatttagg 19800
   aggttgaggt ggtaggatgg tttgagtata ggagatggag gttgttgtga gttgagattg 19860
   ttttattgtt ttttaatttg ggtgatagaa ttagattttg ttttaaaata aaataaaata 19920
   aaaaaaatga gtagttttta agagtgtgga gataatgtaa aaataagaga tttggatttt 19980
   ttttattttt ttttatgggt tatagaagtt tttgtagttg gttgtgtgtg gtggtttatg 20040
   ttt 20043
<210> 115
   <211> 7201
   <212> DNA
   <213> Artificial Sequence
<220>
   <223> chemically treated genomic DNA (Homo sapiens)
<400> 115
   gttagtggtg ttgtgttgaa tgtaattgtg tgtgtttgtt gggtgagtta gtgatttgag      60
   ttaggtgggg gtggtttgat tttttttgtt tagtttgggt tagaggaagt tgaaagtggt    120
   tttagtaagg tgaggtgttt gaggttgtag aagattgagt agaattttgt agagtttggg    180
   atgtgttggg ttaggttgtt gaggatttga ttggtttttt tttttttttt ttttatttat    240
   tttttttttt tttttttatt atttagtggg tttgttttgg gtttttagtt attagttttt    300
   gattttgtgt ttgagggtag tgattttgat ttttgtttgt atgtatagtt agtagtttta    360
   gtgttagatt ttaaagttgg atatttttta attgggttat tagtttgggt tttaaagttt    420
   tggtgagtgt tgtgatgttg tttggggtgt tagggagggt aaagggtgtg aattattttt    480
   tatttttttt gtgtgatttg gatgttttat tttaaatata taaattaagg tttaagtata    540
   gagagtttat gtggtttagt tttgtgtatt ttaggtatta ttaaggttgg atgggtgttt    600
   gtattgtgtt ttttgttttt atgggatgtt aatgataata aaattttggt ttttagttga    660
   gtattttggg gagggggtgg agaggatttt tttaagttta aggaggggtt tttgtggtgg     720
   tgtaggttga tgagaggtat ttgggaagta tagagtggta ttttagtgag tttgaagggt    780
   tatttgtaat taaaggaaaa ttgttttttt atggatagtt ttatatgtat tatggttata    840
   ttagttgagt atggggaaat ttgataagtg gtttggggtt gtttatttgg atataatatt    900
   ttgtgtgtgt gtgataagtg agttatttag ggatttgttt ggaaagtttg tgtaaagtta    960
   gagagggtta tggttaaagg ttaggtattt atagagtatt tagaattaat aaaataaaat   1020
   ttttgttggg ttgtggattt ttggggtttt tggagatata ggaaaggtat gtgtggtttg   1080
   gaagtgaggg atatgtaatg agaataggat gatttaagtg gttggaggtg ggtagattag   1140
   gagttttggg ttggaggttt agggatggtt tttgatggtg gttggtgggt ttttggattt   1200
   ttttgtgttt ttttagagtg agggttgtta ggtttaggtt ttagtgaagt tttgagtgtt   1260
   atggttgatt tgtaggttgg ttttgtggtt ggggattggg agattgatgt tgagagtttg   1320
   gagttggaag aggatgtttg tggggtgttg tggtttatgt tttttgggtt tagtttgttg   1380
   ttggtggatg gggattgtga ggatgatgaa gatgatgatg gggtggatga agatgtggaa   1440
   gaagagggtg atggtgagga ggtaggtgtg tttttttgga tgtttggtta gttggagtag   1500
   tgggggggat tgtagttgag gttgttgttt gtgttttagg ttttatttgt tggtattggt   1560
   ttttgagagt gttgtatttt tgtgggtggt ggtgtggagt tgtgtaagtt gagttgtatg   1620
   tttaagtgtg tgtgttgttg taattatggt gtggtgtttt gtttgaaggg ttataagtgt   1680
   ttttgttgtt ggtgtgattg ttagtgtgtt aattgtttgt tggtggtgga gtggtagtgt   1740
   gttatggttg tttaggtggt gttttggagg tagtaggtta ttgaggtgtg tatttgtttg   1800
   gtttgggtgt tttaggttat agtggaggtg ggggagttgg aggggagtgg ggtggttgtt   1860
   tatatttttt gtgtttaggg ttttgtggtg tttggtattt tttaggaagg atggtaagaa   1920
   tgtttttttg tttgtattgg tgagtagggt tgggagggaa aaagtaggta tttttgggga   1980
   atttgggaaa tgtttttgtt ttaaaaagta ggtagagagg ttttgaaagt gtttaggttt   2040
   atttggaata ttgtgagaaa attaatgttt ttattttttt gtattttaaa gtgtattttt   2100
   gtggtgttga gggtgggggt ttggggggag agtggttgat ttatagtatg gtgtgtagtt   2160
   ttaggtttgt tattgttgta ttggtgatga tggagaaaaa gaattatgta gtatgtatgt   2220
   gtttggtgtt ttagttgtta tttggatgtt tgagatattt agaatattta ttttatggat   2280
   gagatataaa atgtatgaga aattagatgt ttgtagttta aggttattaa tttgaagtta   2340
   ttgtttttgt aaattattta agagtaaggg gagggttttt gttgtttttt attggaggat   2400
   tttagttgtg aggagagtta gggttttttt tttgagagat gtttttgaat ttttgatttt   2460
   gtatttgttt ttatgtgtgt ttggataggg agagtttagt gagtgaggtt ttatagggtt   2520
   tttttttaga tttaggatta gggaagattt ttaattttat tttttggtgg agaggagttg   2580
   gggagtggat tgttagatat attgaaggta gtaattttat tggaaagttt tttgagggtt   2640
   taggagagtg tgttggatgt tatgggtatt tggttttggg tttggttttt ttttgtgttt   2700
   tgtatgggtt attgtttgtt ttgtgttttt atgtgttttt tgtagtatat gttgggtagg   2760
   atgtgggtgt gggagggttt agaattttat tagttggtgg gataggtgat tgtagaggtg   2820
   agggtgggtg tttgtaaagt gtaaatttgg agtttgtagt tggatttagg agatttttat   2880
   tttttatttt ttttattatt ttattttgtt tgttttgtta tagggagaaa tttgtttggt   2940
   tttattgttt tatagtaggt tttgggtatt ttttatttga taggtgggtt tttaggattt   3000
   agtttggttg taattatttt ttgaggttgg gtgttttttt gaaagggatt ttattttttt   3060
   attttttttt gggatgtttt tttatttttg gttataggtt tgtgtatatt tagagtgatt   3120
   agagtttaga gtgattatgt tgtggaaagt aggggattta gtttattagt tgaggtttta   3180
   gttttttgtt ttaggattaa ggggtgttga tattaatttg tttgtaatta ttttttatat  3240
   ggaatgggaa aatttaatat atttagaagg gggagagttt ttagaagatt tatggatatt   3300
   ttgttttttt ttttttttgt ttttattttt ttttagggta ttattgatga tgttttttgt   3360
   gtttatagga taagaagggg ttttttggga aatagaataa ttttgagtgt aaagttgtgt   3420
   attagaggta agttagagtt tttagtttgt tggttaaaag tattttagaa ggtaaagtaa   3480
   taaaattatt ttttagtttt ttaaatagag ttgagtgatt tttattaatt agtataaagt   3540
   gtgtttatta atttgtgaaa gagttattta aagggaaagt attttttatt agaaaggttt  3600
   tgtttaggtg tttgttgagt ttgtatgtta aaattgaatt tgtatggaga agattagtat   3660
   ggtttgggaa ttagaaagtt tttttttagg aaggaaattg ttatttggga aaatgaattt   3720
   tatatatttt ttgaggtttt tgttattatt agttgaagaa tttttattat ttggttgttt   3780
   gtttagtggt taattggtta atgtggagtg tatgtgaaat ttattttgta tgagggatgt   3840
   ataaagatat attgttaagt gattttggag tagatgtttt ggaaggaaat aaataattta   3900
   aataaagtaa aataaaataa tataattatg tttatttttt ttagttgtgg gagttgaata   3960
   gatgaagtta agtgaggagt ttaatagttg tttgtaatta gtaaagttgt ttagtttgga   4020
   agtttaattt tagattttgt ttttttttat tgtatttttt tttttttttt tggattattt   4080
   ggataatttt tattatttta aattttattt ttattatttt ttgatagtta aggttataga   4140
   atttgaaaaa ggttttgaat ttagtaagtg aagtggaggt tgaatgtgaa tagtgtgaaa   4200
   tttttagttt tttgttttta tgtttttatt tttgtgaaag tttgggggta tattaatttt   4260
   ttttaaaata ttatttattt ttaaaagtat ttttatttgg gtatggaatt tatttattga   4320
   ttttaataat atattgatta tttttaattg ttttttttta aatttttaaa aatttaaata   4380
   agggataatt aatgtttgtt ttaattattg ttattttatg aaatttaggt tgaaagtaaa   4440
   taaaaatttt taagtagata tttggatttt gttttttttt tgtgtagttt ttgaattttg   4500
   ttggttttgg ataggtttgt gtgtgtttta taaaaatttt taagtagata tttggatttt   4560
   gttttttttt tgtgtagttt ttgaattttg ttggttttgg ataggttttg aagtttgtgt   4620
   gtgttttatt atgtgttgta tatttttgat tatagagtgg ttttttatga attaaaaatg    4680
   aatttggtta taaattggat aaatagattt ataataatag ggttatattg ataaattgag    4740
   attatgtaaa gtaatttgtg aagtttgagg attaaatgga atagtaaatt taattaaata    4800
   attttttggt aaaaatttat tatagaaatg taaatattaa tttttaaatt ttattttatt    4860
   tgtaaatgag tataatgggt ttgttattat gtaaatataa atataataat ggatgtttta    4920
   aataaatatg atagaggtga gtgtttgggg gtgatgaaat attttaagta ttttatgttt    4980
   ttaggaattt tatttatgtt atattaattt ttgtatttga gtttgttttt atttgtgtga    5040
   tgatttagtt tgtgttttaa tgattgtttg tttaattatt ttggtatttg gttaaaatga    5100
   ttagagtttg ggaagaatgt tttagaatag ttatttttga tagagtaggg aatatttttt    5160
   gttttttttg agttttgttt tattttattt tatttttttt tgtagtggta ttaggggaag    5220
   gggtttggtt aaattgttta ttgtagagtg tgtttgatta gttaatagtt ttttttaatt    5280
   ttttatgttt ttttattttt ttaagttttt tttttttttt tttatttgtt aatttttttt    5340
   aatttttttt tttttttttt ttttttagtt ttttttggat tattttatag ttattatgtg    5400
   tatataatga attattttta gaaaataaaa gtgatattag ttttaggtat agatatattt    5460
   tttaataatg aaaaaagatg atatggataa taataaaata ttgattgata taagataaat    5520
   agttttgttt tttaatgtgt agggggtttg ggaagaatat taaaatttta attaataaag    5580
   tatgtttgtt gaagtaagta agtaaataaa tttataataa ttataataag taataagaat    5640
   aagaataata ataagaagaa gttgtagtat ggatattttt tattttttta ggaaattttt    5700
   ttaaatttaa tttgtatttt tgtatttgta taaaaatatt agtgagatgg aattgtaaat    5760
   aattgttttg ttgatttgta gtgttgttgt tattttttgg gtttttatat ttttattttg    5820
   tagatgttaa ttttttttat gtgtaatttt tgttttttgt aggttattgt tttattttag    5880
   tggagattta tgtaggaggg attttttttt tatttttttt agttagtgat aggatgagga    5940
   aaagaagagt ttttgttgat aaagagttgg agaatattat gttggagaga gaatataaag    6000
   aaagggagat gttggaaatt ttttaagttg ttgttttgtt tttgtttaat tgtatggtgt    6060
   ttggatttga ttataatttt tataaaagtg tttatagttt tagtttagtg gaattattaa    6120
   gtaaggattt ttgtaatttt ttgtttattt gttttgattt aattatgtag tatttagggt    6180
   ttgggaatat ggaattaatt ttttttaatg ttagtgtggt tataatttat agatagtatt    6240
   ttttgttttt aagattttta gtttggttta agtgtggttt tattagtgat attttttttt    6300
   attagtaatg tttgttaaat gttattattt tagtttaagt tttgaagttt ggggttagtt    6360
   gggatttgaa gggagtatga gtttaggatg gatttagtgt agagtaggat atgatgttat    6420
   tgaaattgga aggttttttg gtgttgtttt atatttttga gatttagatt atgagaagtg    6480
   atttttaggg ttattaggtt gttttagaga ggtttgtgtt ttttttattt tgatggaatt    6540
   ttttttttat tgttgatatg gattttttgg tagtttaagg gtatgtttta atgaagatta    6600
   gtaaagaaaa tattaggtat tttttgttat ttagatataa tttattttat ttattatttt    6660
   agtaaatatt tattgataaa ttgggttttg agttgaaaat attatttgtt aaagaggttt    6720
   ttgaagagat ttttaagaaa tatagagagt gtttagttaa ggataattag aagtatatat    6780
   ttataataga tagatgtgta aaagattttt ttgtagttaa ataagttgga ataaaatttt    6840
   tggtgaatga attattgtta ttttttgttg agtttatttt taagaggttt ttatttgtta    6900
   ttatttgtgt tttttagtga aaggttgttt aaatagaaag ttggattttt tgtagtttta    6960
   gagtattata gttatttgtt atttttttta aaagttaaga tgtttgtgta aagaaatttt    7020
   taatgtaaag atgatgattt tgaaaaattt tatatatttt taatatgtat gtattttttt    7080
   ttattatata tatttaaatt tatagatgga aattgtttaa tgtgtaaatt gttaagtatt    7140
   atattttata tagtatttta aaaagtaata aaattgatat tgttttttta aagatttaat    7200
   a                                                                   7201
<210> 116
   <211> 7201
   <212> DNA
   <213> Artificial Sequence
<220>
   <223> chemically treated genomic DNA (Homo sapiens)
<400> 116
   tattgggttt ttgagaagat agtgttaatt ttattgtttt ttgaaatgtt gtgtaaagtg      60
   tggtatttaa taatttgtat attgggtaat ttttatttgt aagtttaaat atatgtgata     120
   agaaaaagta tatgtatatt aggaatatat aaaatttttt aaaattatta tttttatatt     180
   agaaattttt ttatataaat attttaattt ttaaaaaaag tagtaaatgg ttataatgtt     240
   ttaagattgt agaaaattta gttttttgtt taagtagttt tttattgaga gatatgagtg     300
   atggtagatg aaggtttttt aagaatagat ttaatagaaa atgatagtgg tttatttatt     360
   gagagttttg ttttaatttg tttggttata aaaaggtttt ttgtatattt atttattgta     420
   aatgtgtatt tttggttgtt tttaattaaa tattttttgt gttttttagg ggttttttta     480
   aaggtttttt tgataaatgg tgtttttaat ttaggatttg atttgttagt aagtgtttgt     540
   tggaataatg agtggaatgg attatgttta agtggtagag ggtgtttggt gttttttttg     600
   ttgatttttg ttaagatatg tttttgagtt gttagggagt ttgtgttaat aatgggagag      660
   aaattttgtt ggggtgggga gaatgtggat ttttttggga tagtttgatg attttgaagg      720
   ttattttttg tggtttggat tttaggagtg tgaggtagta ttagggaatt ttttaatttt      780
   gatggtatta tgttttgttt tgtattgagt ttattttgga tttgtgtttt ttttaagttt      840
   tagttggttt taggttttag ggtttgaatt gaggtggtgg tatttagtag gtattgttgg      900
   tagaggaggg tgttgttaat ggggttatat ttgggttaaa ttaaaaattt tgaggataag      960
   ggatattgtt tataagttgt ggttatgttg atattagaag aaattagttt tatattttta     1020
   gattttgaat attgtatggt taaattaagg taggtgggta aaaaattata gaagtttttg     1080
   tttggtggtt ttattgggtt ggggttgtag gtatttttgt aggaattgta gttaggttta     1140
   ggtattatgt ggttgggtag aaatagagta gtagtttgag aagtttttaa tatttttttt     1200
   tttttatatt ttttttttag tataatgttt tttaattttt tattagtaaa ggtttttttt     1260
   ttttttattt tgttattaat tgggggaggt agagggaagg ttttttttat ataagttttt     1320
   gttggaatgg ggtgatagtt tataagaagt aaagattgta tatgaaagag attaatattt     1380
   atggaataaa aatgtggaaa tttagaaggt aatagtaata ttatagatta gtagaataat     1440
   tatttatagt tttattttat tgatattttt atatagatgt aaaagtatag attaggtttg     1500
   aaagagtttt ttgggagggt gaaagatatt tatattgtaa tttttttttg ttgttgtttt     1560
   tgtttttgtt gtttgttgtg gttgttatgg atttgtttgt ttgtttgttt taatagatat     1620
   attttattga ttaaaatttt aatatttttt ttaggttttt tatgtattaa gagataagat     1680
   tatttatttt gtattaatta atgttttatt attatttatg ttattttttt ttattattaa     1740
   aaaatatatt tatatttaga gttgatgtta tttttatttt ttaaagatgg tttattatgt     1800
   atatataata gttgtagaat aatttaagga gaattaggaa aaaagagaaa gaaagaaagt     1860
   taaaggagat tagtgagtag aaaaagaaaa aaaaggttta agaaaataga gaagtataga     1920
   aaattgaaaa gagttgttaa ttagttaaat atattttata gtgaataatt taattaggtt     1980
   tttttttttg atattattat aaaaaaaaat aaaataaaat aaaataaaat ttaaaaaagg     2040
   tggggagtat tttttgtttt attgaaaata attgttttga aatatttttt ttaagtttta     2100
   attattttgg ttggatatta aaatggttga ataagtaatt attaaaatat aggttaagtt     2160
   attgtatgaa taggagtaaa tttaaatata aagattagtg taatataaat agaatttttg     2220
   gaggtataaa atatttggag tattttatta tttttaagta tttatttttg ttatatttgt     2280
   ttgaagtatt tattgttata tttgtgttta tatagtgata aatttattat atttatttgt     2340
   aaataaaata aaatttgaaa attaatgttt atatttttat gataagtttt tgttaggaag     2400
   ttatttagtt gaatttatta ttttatttag tttttagatt ttataagttg ttttgtataa     2460
   ttttagttta ttaatgtggt tttattatta taaatttgtt tatttagttt gtaattaaat     2520
   ttatttttaa tttataagaa attattttat ggttagaagt atataatata tgataggata     2580
   tatataaatt ttaaaatttg tttagaatta ataaaattta gaagttatat aagaaaaaaa     2640
   tagaatttaa atgtttattt aaaaattttt ataggatata tataaatttg tttagaatta     2700
   ataaaattta gaagttatat aagaaaaaaa tagaatttaa atgtttattt aaaaattttt     2760
   gtttattttt aatttgaatt ttatgaagtg gtagtgatta aagtaaatat taattgtttt     2820
   ttatttaaat ttttaaaaat ttaaagagaa atggttaaaa atagttaata tattattgag     2880
   gttaataaat aaattttata tttaaataga gatgttttta aaaatgagta atattttaag     2940
   aaaaattaat gtatttttag gtttttatga aagtgagaat atggagatag aaagttagag     3000
   attttgtatt gtttgtattt aatttttatt ttgtttgttg gatttgaaat tttttttaga     3060
   ttttataatt ttgattgtta aaaaatggta aaaatgaagt ttggaataat aggaattatt     3120
   taagtaattt aaaaaaaaaa aaaaaagtat aatgaaaaag agtggaattt gaggttgaat     3180
   ttttagatta aatggtttta ttggttgtga gtagttgtta aattttttat ttgattttat     3240
   ttatttagtt tttataatta agaaaagtgg atgtggttgt gttgttttgt tttgttttgt     3300
   ttaagttatt tatttttttt taagatattt gttttaggat tatttggtag tatatttttg     3360
   tatatttttt atatggggta aattttatat gtattttata ttaattgatt aattattaag     3420
   tagataatta aataataggg attttttaat tggtggtgat agaaatttta ggaggtatgt     3480
   gagatttatt tttttaggtg gtggtttttt ttttagaaga aaattttttg gtttttgggt     3540
   tatgttaatt ttttttgtat gggtttagtt ttagtgtatg ggtttagtga atatttaaat     3600
   aaaatttttt taataaaaag tatttttttt ttagatagtt tttttataga ttaataaata     3660
   tattttgtgt tgattaatga gagttattta attttgttta aaaggttgaa ggataatttt     3720
   gttgttttat tttttaaaat gtttttggtt agtaaattgg gggttttgat ttgtttttgg     3780
   tatatagttt tgtgtttgaa attattttgt tttttggaaa gttttttttt gttttgtaaa     3840
   tataagaaat attattaata atgttttgaa agaaaatggg ggtgagaagg gggaaggatg     3900
   ggatgtttgt aaatttttta gaaatttttt tttttttaaa tgtattgaat ttttttattt     3960
   tgtgtgaaaa ataattataa atgaattaat attagtattt tttaatttta gggtaaggag     4020
   ttggaatttt agttggtgag ttggattttt tgttttttgt agtatagtta ttttagattt     4080
   taattatttt agatatatgt agatttatgg ttagaagtga ggggatattt tggagggaag     4140
   tgaaaaagtg aaattttttt taggggggta tttgatttta agagatggtt gtagttaggt     4200
   tgggttttgg gagtttattt gttggataga aggtgtttaa ggtttgttgt gaagtagtga     4260
   ggttgggtgg attttttttt gtggtagggt aggtagggtg gggtgatagg ggaatgaaga     4320
   gatgagggtt ttttgggttt ggttgtaaat tttaaatttg tgttttgtgg atatttattt     4380
   ttgtttttgt agttatttat tttattagtt ggtgggattt tgggtttttt tatgtttgtg     4440
   ttttatttag tatatgttat agagaatgtg tgggaatgta gggtaggtga tgatttatgt     4500
   gggatgtggg aggagattaa gtttaggatt aggtgtttat ggtgtttagt gtgttttttt    4560
   gggtttttga gaggtttttt agtggggtta ttgtttttag tgtgtttggt gatttatttt    4620
   ttagtttttt tttgttaaaa agtaaaattg aaagtttttt ttggttttga gtttgggagg    4680
   aggttttgtg aagttttgtt tgttgaattt tttttgttta ggtgtatatg gaaataaata    4740
   taaaattgaa ggtttaagag tgttttttgg aaagaaagtt ttggtttttt ttgtagttgg    4800
   agttttttga tgggaagtag tagagatttt ttttttgttt ttaggtagtt tgtagggata    4860
   gtagttttgg attgatgatt ttgggttgta ggtatttaat tttttatgta ttttgtgttt    4920
   tatttataaa atgagtgttt tagatatttt aaatgtttag gtgatagttg aagtattgag    4980
   tgtatgtgtg ttgtgtggtt tttttttttt attattatta gtatagtagt gatgagtttg    5040
   gagttgtatg ttgtgttgtg gattaattat ttttttttta agtttttgtt tttaatatta    5100
   tgaaaatgta ttttaaaatg taaaaaaata aaaatattga tttttttgtg atgttttaaa    5160
   tgggtttaaa tatttttaag gtttttttgt ttgtttttta agataaaaat attttttaag    5220
   ttttttagag atgtttgttt tttttttttt gattttgttt gttagtgtaa gtagaaaagt    5280
   gtttttatta ttttttttag ggagtgttag gtattgtaag gttttgggtg tggggggtgt    5340
   ggatggttgt tttgtttttt tttaattttt ttatttttat tgtggtttga gatgtttggg    5400
   ttgggtgggt atgtattttg gtggtttgtt gtttttggag tgttatttgg gtggttatga    5460
   tgtgttgttg ttttattatt agtaggtagt tggtgtattg gtagttgtgt tagtgataga    5520
   agtgtttgtg gttttttagg taggatatta tgttgtggtt gtggtagtgt gtgtatttgg    5580
   gtgtgtggtt tagtttgtgt ggttttgtgt tgttgtttgt gggagtgtag tgtttttggg    5640
   gattggtgtt ggtgggtgag gtttgaggtg tgagtggtgg ttttggttgt ggtttttttt    5700
   gttgttttgg ttggttgggt attttggggg atgtgtttgt ttttttgttg ttgttttttt    5760
   tttttgtgtt tttgtttatt ttgttgttat ttttgttgtt tttgtagttt ttgtttgttg    5820
   gtggtgggtt gggtttgggg ggtgtggatt gtggtgtttt gtagatgttt ttttttagtt    5880
   ttaggttttt gatattgatt ttttagtttt tggttgtgga gttagtttgt gggttggtta    5940
   tggtgtttgg ggttttgttg gggtttgagt ttggtggttt ttattttggg gaaatataaa    6000
   gaaatttaaa gatttattgg ttgttgttag ggattatttt tgagtttttg gtttaaagtt    6060
   tttggtttgt ttgtttttgg ttgtttgagt tattttgttt ttattatatg ttttttattt    6120
   ttaggttata tgtgtttttt ttgtattttt agggttttta gggatttata atttaataaa    6180
   ggttttattt tattaatttt aagtgttttg tgagtatttg atttttaatt gtggtttttt    6240
   ttggttttat atgggttttt tggataagtt tttgggtaat ttatttatta tatatatata    6300
   aaatattgtg tttaaatagg tagttttagg ttatttgttg gattttttta tatttaatta    6360
   gtatggttat gatgtatata aaattatttg tggaagaata gttttttttt gattataagt    6420
   ggttttttag atttattgaa gtattatttt atgtttttta aatgtttttt attagtttat    6480
   attattataa ggattttttt ttgggtttga agaggttttt ttattttttt ttttaaaata    6540
   tttaattgaa aattaaaatt ttgttgttat tagtgtttta taagggtaaa gagtataata    6600
   tagatgtttg tttagtttta atagtatttg aggtgtatag ggttgagttg tgtgggtttt    6660
   ttgtatttag gttttggttt atatatttaa ggtaaggtat ttaagttata tagaaaaggt    6720
   gaaagatgat ttatgttttt tgtttttttt gatattttga gtggtattat agtatttatt    6780
   aaaattttga ggtttgggtt ggtagtttag ttaaaagata tttagtttta ggatttggtg    6840
   ttagggttgt tggttgtgta tgtaaataga aattggggtt gttattttta aatatgagat    6900
   tgggagttga tagttggagg tttggaatga atttattgga tggtggaggg ggagggagaa    6960
   ggtaggtgga gggaaggagg gaggaagtta gttaagtttt tagtgattta gtttagtata    7020
   ttttgaattt tgtaaagttt tgtttggttt tttgtagttt tgggtgtttt attttgttga    7080
   ggttgttttt ggtttttttt gatttaggtt gggtgggaaa ggttgggttg tttttgtttg    7140
   gtttagattg ttggtttatt tggtgggtgt gtgtagttgt atttagtgta gtgttattgg    7200
   t                                                                    7201
<210> 117
   <211> 42929
   <212> DNA
   <213> Artificial Sequence
<220>
   <223> chemically treated genomic DNA (Homo sapiens)
<400> 117
   tattaatagg agttgtagtt tggaagataa agagtttttg gtattgtaag ttttgttatt      60
   tgtgagtgtg gtatagtgaa ggtttggaga gttatgattt aggttttgtg aagttaagta     120
   tttttattaa gtaaaaagat tggtaataaa atatttttat atagtatatg atagaatatt     180
   tgtatttatt ttattttatt ggatattaat tgtattaatt gaggtatatg tatttttaaa     240
   aaaatgaaag aagaaaatat ttagagaaag gattggtatg aggttgagga agaaaaatgt     300
   gtattgtatt ttttagatga agtagaaatt gattagtaat ttaaagttgt aatttattta     360
   attttttaat attgtaaatt aatttttatt tggtttgatt gtttgattga aatgtaattt     420
   ttgtgttggt ttttagaaat ttaaaaatat agagattaga aatttggttg atgaaaattt     480
   atgggaaaaa aaagtatgtg gttttggttt gaaatgtaga atggataaat aaaatgttta 540
   tagtgtaata ttttaattga ataatttata gtaatagttg aaataaagtg atatattaga 600
   ttatatttgt tataatataa aattttataa taagattatg aaagtgattt ttattatttt 660
   ggtaatggtg ttttagtata agtaatgaga tataattttt ttttaatttg gtatattaaa 720
   tagtaaaggt tatatataaa tgttgttttt ttagaatgta ttttgtttat aattatgtat 780
   tgtagttatt atgtatatat taaaagtatt tatgataaag tataaaaata agaaaagatt 840
   aaataagaag gtatttttga gttataaatg tttattaaag tatatttttt aattatatag 900
   ttgtgttata gttaaaaata tataaattaa tatatattgt tatgtaatta tttgatttaa 960
   tatagattta ttaggaaaat attaaggttg tagtaatatt ttataggtaa aggttaagtt 1020
   ttgggatatg aaagttttta gaaaatttat aaaataaatt tgagatattt ttttgttttg 1080
   gaaaaaagta atttgtttat agagttaaag ttttgatata taaatgaagt atttaataaa 1140
   tattatattt tattgtaata atttggagta aaatttttga ttttaatggt tgttagtaaa 1200
   tatatttttt ttttggtaga aatataatga gggtttttga ttaaagtagt aatttgatta 1260
   ttttagtttt taaatttagt tatttttatt tgttttttgt tttttttttt tttttttaat 1320
   taggttataa gtatattaaa taagttaggt aaattttaag tattttattt ttttttatat 1380
   aaatatagtt ttttttttgt tgtaagtatt tgatgatgat tgggtttata tatgtaaaaa 1440
   tagattatag tatatttgga tttggttgat ataatgaggt atttagattg gtttatgatt 1500
   atttttgtag ttatatattt tttaatttaa aaaaataggt tatagtagtt taaatttatt 1560
   gttttagttt tggtggggta taatattttg gaatggtaag tgttaaaaaa tttaaatgta 1620
   ttgggtaagt ttgttttgtg tagttgattt taatgttttt aaaaatattg gtatgatttt 1680
   ttatatttaa gaagtatagt tatgaaattt taaaataaaa ttatatgaag ttttttaata 1740
   ttttaaatta taaaatagat ttgttatgtt gtagattttt ttaatagttt atttgagatt 1800
   taattttgtt atgttaaata tttttttttt aaatgtttat gttttgaatt ttgtgtttta 1860
   aattaattaa tttagaatga gtgggaaaat gtttaattat tttttttttt ttttagtgtt 1920
   tttttttttt tttttattat ttttttattt tatatatata tatatataat tattaaaaat 1980
   tttttttttt aaaattttgg tataaagatt ttaaaaagtt taataaaatg taataagtat 2040
   ttagtattta ggatattgta gaattttttt tggaagttta attgtaatga attttaaaat 2100
   ttgaaatttt aatttatttt tttaaatgtg aatgtatata ttttatttga gttttatgtt 2160
   tttttagtta ttaaagtaat tgttatttta aaaaatataa ttgattttat ttttattgtt 2220
   ttttttaaat agtttttaag attatttttt gattttgatt ttttaaaaag taggtattaa 2280
   tgttaatttt taaaaaatta tttagaagag aaatttggtt agttgtaatg ggagttttgt 2340
   ttttaatttt tgttattttg taaaaagaat atttaggtaa tttatggtat tttttattat 2400
   ttattgggta gatatagttt agattttttt gttttttata ggagaatgtt tagattttta 2460
   ttaattttaa ttggaattta ataattagaa tggttttaaa atttagttaa aatttaagta 2520
   tgttatgaat ttggaatttt aatatattaa ttaggttatt tttaaaataa gattttgtat 2580
   tataaataga attttaataa agatgttata aaaataagaa tatattttag gatttaattt 2640
   tgagatttgt taaaatttga gtgaagaaaa gaaattttgt tttattttta tgttaaaaat 2700
   aggtggttga tttaattaag tttattttta gtatatttgg ttgtgttagt tgtatttttt 2760
   aatttttaat attatataag gtatatatta tttaattttt ttttgaataa gataatatta 2820
   atatattgtt tatggatagt taagtaatat attttatatt atttggtgat tttttttttt 2880
   aatttatgag atttattgaa attagtatat tatggttata taatttaaag tttggttggt 2940
   tttttttttt ttttttgttg ataatttaaa ttttgtattt tggtaagtaa aagaggtata 3000
   atattatttt ggagttttat aatttaataa tgtatatttt aaaatttatg ataatttatg 3060
   gaattttatt atatttatta atttaattat tgattttttt ttgagtaatt gattttatga 3120
   tttattttta aaagtaagga agtatattag aaggtaaaag gataaattta tttataaata 3180
   ttagagttta agttattttt ttgaaaaggt gaaaagggaa tttttggttt ttattttttt 3240
   ttttattatt tttaattata tattttatat tgaaattatt tgtttttgtt tttttaataa 3300
   tatattttga aattttgttt aatatatttt ttgtttattt ttgtatatgg gagattaatt 3360
   ttggattttt attaaaaatg gtttggttat taaaatattg ttttatttag tagtttggtg 3420
   taagtgtttg ggtgtatgtg tttatatgtg tattttaatt ttaaagaatg ttatttttag 3480
   aaataattaa gatgtagtat gtatataata atttattggg ttttttaagt ttttgattta 3540
   aaaaaatttt ttagaagttt ttgtatagtt tattatttat gaagaattgt tgggtatgga 3600
   gtatatttta tttgatggat agttgattta tgtttatttt aaatgttaat tgagaagtag 3660
   tataaaagag gaattggaaa aaaattgaat tttatttttt atttttgaag atgtgaatat 3720
   ttttaaaatt ttagtttgtt ttttgttttt tattggtaag tttttttggt ttgtaaattt 3780
   tggatagtat tattaaggag ttttatggat tttttgagag tgatattgat tttaaatgat 3840
   ttgggtttaa tggttagatt ataattgaaa tttatttttg taggtttatt tgggttggtt 3900
   ttgaaattgt attggtgagt taggatggag atgaagagaa aaagttgtat tttagaaagt 3960
   tttttgttaa tgtattgttt tttgtttatt gaaaaaatta ttattttgtt ggttaggttt 4020
   ttgttgatga ggttattttt gtttaagaat tgagttggat taaagttttt tgggttaggt 4080
   tattttattg ggttatgatt tatagattat tggttgataa aaattatagt gtttttggga 4140
   atgtggtagt ttaagataga ggtgttggta gtggtggtat gaggaatagt gataggtata 4200
   aagttggaga agtgtatggt tttataaagg aaggttagga tatagggtag gttgggttgg 4260
   ttatttatat aaggtagatg gttttttttt atgatttgat ttaattttgt ttgtatttga 4320
   gtttgtatat taggatattt gtttggtgtt taatgtggag agagaaaagt aagtgagtaa 4380
   aatttttatt ttatttagaa agtatattat aatttatttt aattttattt ttttttaaat 4440
   aggttattta gtttagtgat taaattaata tttttgggaa aaaaatttaa aatagttttt 4500
   taatttttta ttaaattata agatggattt taagaattat attttttgta ataggttttg 4560
   tatgattaat gttgtttttt ttattataag ttaaaaaatt tttaagtttt tttagtttta 4620
   aaaagtataa tagtagataa ggttgatttt tttattgggt attgtaggga tagtgtttag 4680
   ggtttataat attttttagg ggtttatgaa aatattttta tgttttttat aatttttaga 4740
   tttaaaaaaa tttattttta gttagaagaa aatattttta tatattattt taatgtattt 4800
   atttttatat tagggtagtt atgaaattta atttttaatt tttttatagt ataaggaatt 4860
   tataaaggta aaagtattta gggtttatga aagttataat gtgaaagtaa attttttttt 4920
   ttttttattt ttttggataa ttttaataga tatgttgttt ttattttata taaagataat 4980
   tggtgttttg atggataaag ttatatattg tattttttaa atttgaaagt aaatatttaa 5040
   tgtttataaa attatggtat aaattgtagt atttatttat tgttgatgat ataaattgat 5100
   ataatttttt taaaaaataa aattttaata tgaggtaaga gatatgaagg taattttttt 5160
   ttgttttatt ttaaatttag taatttatgt ttagaaatgt atttttagaa aataatttaa 5220
   gagatataaa aatatgtaat tataatagga tttaaaattt taaaaattag ttaggtatgg 5280
   tggtatatgt ttgtagtttt agttatttgt tagatagtgg tgggagaatt gtttgagttt 5340
   aggagtttgg gattagtttg ggtaaggtag tgagattttg tttttaaggt aagtaaatat 5400
   ataaaattaa aataaaaatt ttaaaaatgt aattaaggtt aatgattaat attaaagttt 5460
   aataaatgat tatatataat tatatattat tatagtaaaa tattatatag ttattaaaaa 5520
   ttattaaaat aaatattaaa agattagaaa tgtttattat tttgagttaa agtgttagaa 5580
   gttaaaaaat aagtaattga ttgtttgtaa ttttgaaaaa aaatgtatat atgtatgtga 5640
   tgaagtggag ttatttttag aattgttttt ttaatattat attgtaggag tttgttaatt 5700
   ttttttatag aaatatgtat ggtggggaga gttagagagt atttatttta gattttgtgg 5760
   attaaatttt ttttgttgta ttattttgtt ttgttttgtg ttgtagagtg aatgtagtta 5820
   ttgataatgt ataaatgaat gggtatgatt gtgttaataa aatgtatata taaaaataga 5880
   ttgtgtaatt tgttaagttt tgttatatgt ttttttatta aaaaggaaaa aaaaagtatt 5940
   agtatattga attgaaagtg atttttaaat tgttttattt ttttttgttg tttaattgga 6000
   gtatttggag aattattgtg gttttaatga aaaataaagg ttgttttaaa agttaaaatg 6060
   tttatttgtg gatgtataaa aatttatgga atttattttg ggattttttt ttttttgatt 6120
   tttggttgat ttttttttat ttgatttttg tttttttttt atttattatg ggtgttttta 6180
   tgattttaat attaaaaagt tattttaagt atatagaaat ttttaatttg tgaaggtagt 6240
   tgtatagtat ttaaggaaat atagattgta gtggatttta ataaggaaag agttattttt 6300
   tgttttttaa atattttttt gtggtgtttg tagatttagt atttttaatt gtttatttgt 6360
   gttttaagaa atgatataaa attaggattt ttggtttttt tggtaattaa aatttgtttt 6420
   gtgaaatatt tgtttttagg gatagtgttt ttatagtaaa tattataggt tgtttagtta 6480
   aatatggatt ttagattaat aagggttaat tttttagtgt gagtattttt gtgtaaaatt 6540
   tatttttttt ttgttaaatt tggtaatttg atattaggga tttgttgaat ttgtgtttag 6600
   tagagattaa tgtgggtatt agggggtttg ttaattgagt taagtgtagg ggttagaaat 6660
   gtttgtaggt gttttttgga tattttattt gtttttttga atgttatttt gtttttgtaa 6720
   aagtgattgt tttttgtttt ttattttggt attttttaaa tttttagtgt ggaggtttta 6780
   taaatttagt gttgtttgtt agttgtggtt gttttttgaa agtttttgtg tgggttattt 6840
   gttggttggt ttggttttta tgggtttttt tggtttggaa taaagttttg atgggtggtt 6900
   tgtttttagt gttagggttg ggtggttttg gatgtatgtt gaggattagt ttaggagagg 6960
   ttgttgtgtt taatatattt atgatattat tttttttttt ggaattattt tagttttgat 7020
   tgtaatgtgt tagggagaga gatattaggg tttattttta agtatttaaa aatttttgtt 7080
   tttttggggg gtggaggggt ttttgtaaat gtgtttttat ttgtaagtag gggaggggat 7140
   gatagagata agataggaga ttggaggggt tggggtattg ttgggtgggg tgtgtgtgtg 7200
   tgttgtagtt tttattgtga gtttttttat tgatgtgatt ttggttttga ggggtgggga 7260
   tttggagtga aattttaaat ttggggtttt gtttgtaaat ttagtatatt ttgtttttat 7320
   tttgtttttt ttagaggaga aaagatttgg tttatgtttt ttagaggttt tatttggtga 7380
   agaggaggag tagttattgt agtgtggtgg atagggtgtt ttggttggtg ttgaagatgt 7440
   tagtgatagt ggttggtatg ttttttaaat ttagttgtgt gttattattg tgtgagtttt 7500
   tggttgtttt tttttttgta gagaggataa aggtgtttat tatgttgtgg ggggtggttt 7560
   tgggttgaag gtttttgtag tgttttaaga atttgtttag gatgaagttg ttgaagttgt 7620
   ggttgagttg tttgaatttg tggaaaatgg tgtgtattgg gttggggaag tattgtagtt 7680
   agggtattat gtttattagg ttgtttgtgt ttattgtgtg tttgaatttt ttgttgtggt 7740
   tgagtagttt atggaatttg gggttgttgt ggttgtagtg gtagttgaaa tatatggtat 7800
   ttatgatgtt ggttatggtt atgatggtta gtggttttgg gttgaggaag gtgttgtttg 7860
   tgttgttgtg tattagtagt gttattagtt tgtgtgtttt gtttagtatg tggtttttga 7920
   ggatttggtg gttgtgtggt tggtgtgtga agaagttgtg tattatgttg tgggttgtgt 7980
   gttgttgtat tttttagtgt tttgagtagt ggttgaaagt tatgttgtgg ttgttggata 8040
   ttatatggaa ggaggtgaag gttggttggt tggtgaaggt tgagttttgt tgtattaggg 8100
   tttggtggat ggtgtgtttg ttatttagta ttattatggg gtagttgttt aggtggattt 8160
   ggaaaatgtt gttgtagtgt tgtgttaggt gagtgaatga gaggtgagtt gtttggttta 8220
   ttgttgttgt gtttttgatt agtggttatg taaatgggtt tgggggtgtg gattggagtt 8280
   gttgtttttg ttgttttagt agttgttggt ttatatgtat agtggttagt attgatagga 8340
   gtagtaggag tgtggtttgt tggatggata gtgggtttag tggttaaggg ttgtttgggt 8400
   tgaggttggt gtttatgttg gggatagaga ggagaaggtg tgatatttag gggtgtagag 8460
   ataggagtgg gtgttttatg tttttatttt agtttttggg gattgagtgt tgttgggtgg 8520
   agaggttgga gttgattttt tggagaaatg gttgaagatg ttttttttta tttttaattt 8580
   attttttttt ggagaagaga aggggtgtgt ttttattttg ttgtaattta gtgttaaatt 8640
   tttttttttt ttgtaaggtg tgtaatggtt tttgtaattt ggggataatg ttgtgggtat 8700
   tgaggtggtg gtgtttgatt tttttaaaag tatttattat gttatttgtt atttgtaata 8760
   atttatttga agaggttgtt gggtagtgtt ttggtagata gattgatttg tggggaggtg 8820
   tggtttttag tggtgtggga tagttggttt tgagaaggaa ttgggatttt tgtttagggt 8880
   aagatgttaa taggaatttg tagtttggtt tggatatttg ttgtttttat tggaagtttt 8940
   aatttttatt tgagtttttt ggtgttgtta gtgttaggag tgtttggatt gggatgggga 9000
   tggagaaggg tgttttgttt gttgagtttt gttgtttttg gggatttggt aaagttgagg 9060
   tttttttata gtgttgagat tgagattggg ggttggtgag tggtgttaat ttttatgttt 9120
   ttgtggtttt tataattgga gttgtagaag tgtttgtttt tatttttatt ttagagttaa 9180
   agtgtgttat tttgtttttt tgggttttaa ggattgggta gaaggagggt agtgtgattt 9240
   ggtagggtgg ggtttgtggg gggtggggtg tgttgtatat taggttgttt tgatttggat 9300
   ttttgtttag gtgtattggt tggttttgga gttttattag taggttttta tgggagtgga 9360
   ggtgtttatg tttttattgt gtggtaattg tgttttatta tagttatttt taattgaggt 9420
   tgtatggtgt ttttagaatt atgtttggta taatttgttt tggttgttgt gtttgatttg 9480
   ggagtgtgtt ttgttttgag tgtatgattt tttttttttt tttgtgattt gagtttgttt 9540
   tttggaaagt tagtgtagtg gtagtggttt gttttatagt gtttttggtt ggttgtttaa 9600
   attttatttt aggtttttgt aaataaattg ttattttagt ggaggttttt tggtatgtta 9660
   aatttttttt agtttttgtg gtgagattag gaatttttaa aagtagttgt ttttttgttt 9720
   tggggttttg atagtgtttg ggtatataaa ggtttttggg gtgtagttgg ttttgagatt 9780
   ttttgtgtag aggttatttt aggtggtgtt gtttgttggt gggtgtgggg ttgtttaggt 9840
   tgtgatggaa gttgttggtg tagggtgggg agttaagttg gggtgattgg gggggtttgg 9900
   gggagttagt ggggtgggat ttgagtgggg tggagagtgg taggaggagg tgaatttttg 9960
   tgttttggtg aattttattg ggttgaagtt tttgttgttg tttttttttt gtgtgtggag 10020
   ttgggttttg tgtgtgttgt ttttggaaag ttggttttag ttatattttt gggtgttgtt 10080
   tgtggttgtt ttttttgtgt tttttatggt atttgatatg tggaggtggt ggttgagggt 10140
   ggggtgttgg ttattattat ttttggtgtg gggtgttggt tgttattatt tttggttgtg 10200
   tatgtatagt tgtgtttgtt aggttagtga ggtggatgtt tgggttatta tttgtaggtt 10260
   gagtttgggt gtgtattgtt attttgttgt gtattggtgg ttttgtggtt tttttgggtg 10320
   tgtttgtggt tgtgaagggg gttttttttt tataagggaa gttggaggtg tttgggtggg 10380
   ggtggttttt tttgtttggt gtgttttttt ttatatgttg atgtatagga tttttaagat 10440
   agttaagaga ataaggattt tatgaggtgg ggggaagggt gaatttgagg gaagaggtgg 10500
   gatgtatttg gttaattagg taggaattgt attggtttta ttaaaggata aatataaaag 10560
   tgataggttt attagggata attttgtatt ttgagtttta tttgtgttgt gaattttgtg 10620
   ttattattga tttgtttata gatgaataaa ttagggttta gagaggttgt gtgatttaat 10680
   ttaggttgtg tagttattaa gaagtggagt tagggtttga agttattaag ttttatttta 10740
   gattttttat ataagtttat atggaagaaa attgtagata agaggtttaa atgttttttt 10800
   ttttgttttt tttaaaaaaa aaaatgtatg tggggttagg tgtagtggtt tatgtttgta 10860
   attttagtat tttgggaggt ttaagtgggt ggattatttg aggttaggag tttgagatta 10920
   gtttggttaa tatggtgaaa ttttatttat taaaaatata aaagttagtt ggatgtggtg 10980
   gtgtatattt gtaattttag ttatttgggg attagggggg aaggtagtag aattggttga 11040
   atgtgggagg tagaggttgt agtgagtaga gattgtatta ttgtattata gtttgggtag 11100
   tagagtttaa gtgagatttt gttttaaaaa aaaaaaaaaa aaaaaaatta gttggaagaa 11160
   ttttttaatt attgtttttg tttttataat tgagaatttt gagaatagta gagaagaaat 11220
   agtatgttaa ttttttgtaa ttaaattgtt tatgagggag gttaggggag tgtgttttat 11280
   agttgatttt ggaatgattg attatattgt aaagaaatat aaaggagtgt ttgaaaagaa 11340
   tttgattttt tgagagattg aaattagtaa ttaatttgtt gatttgataa agaaatgttt 11400
   ttttgtgatg gtttttttta tttttatttt tatttattta ttttttttga gatggagttt 11460
   tattttgtta tttgggttgt agtgtaatgg tgtgattttg gtttattgta atttttgttt 11520
   gttaggtttt tttggtttag ttttttaagt agttgggatt ataggtgtgt attattatat 11580
   ttggttaatt tttgtatttt tttagtagag atggggtttt attatgttgg ttaggttggt 11640
   tttaaatttt tgattttagg tgatttatta gtttgggttt tttaaagtgt tgggattata 11700
   ggtatgagtt attgtgtttg gtttgtgatg ttttttattt ggtatttttt gaagagtttt 11760
   ttggaaatga ttttatatat tatgatgggg aatttagata gtgatggagt tttagttttt 11820
   gagtagttgt attggtttaa taataagtgt tggtttttta gaggtgagtt ggtttatata 11880
   gtttttgaaa tgagttttat agtaattttt ttatgtttag ttttagtaag atttttgttt 11940
   aataatttaa gattttggtg tatgtattaa ttttttatag attttaattt agtagatggg 12000
   aattatttat gagttaatgt taattttgag ttgttgatat ttttgaaagt ttttattata 12060
   atatttttgt tagaatgaga gtagatggaa gttgttttta agtaaatttt tatttttaaa 12120
   aagtataaaa tgaggttgtt tttaagattg tttgtttaat tttttaataa tgttatttag 12180
   tttttagatt aattagatgt atgttttttt gttattgtag atagattaga tatatttttt 12240
   aagttgtagt tttttgggtt tagttttttt tttaggatat gatttaaaaa ttagaggaat 12300
   taggttggta agtgttgtta gaaagataag tttttattgg ttatggtaaa ttaaaatgat 12360
   tggttatggt aatatttttt gtagagaatt tttaagtttg gagtttttag ggttttatgg 12420
   tgtaaatttt attgaatgtt ttatttggta ttttgataag gattttggat aaatttagag 12480
   tagtttgttg gtattttgat ttaatttaag gtagagtttt gtttttagtg agaatttatg 12540
   taggtttttt aattattatt ataatttttt aattaaagta gggatttaag gtttaggaat 12600
   ttttttttaa ttaatataga atatgggtat tttgtttttt taaattttaa atttgaattg 12660
   tttgttgtaa ataattttta aaaagaatag ttaagttaga gggatagttt aaagtagaga 12720
   gaggtttaaa atattttatt ttgtattgat ttgaaggaat aatttaagtt tgtttaagta 12780
   gaataggaat gtattggttt atgtgtagag aaggataatt gtgatgtttt ataggatata 12840
   aggatttagg ttgttttatt ggttttttgt ttatgttggt ttttgtttgg gttttttttt 12900
   tttgtatggt aaggaagtag tttaaaatta ataaaataaa gagaaagaga atagtttttt 12960
   tttagggttt tagaaggatt ttagggggtt aggttggtat taggttttta ttttgtgggt 13020
   aagggtttgt tttgttatta gaaaaaaaga gatggtagaa tatgttagga agttaaagag 13080
   ttgatttagt ttattttatg tatatatgtt tgtttattgg tttttttggg ttggaggata 13140
   atttaaggaa atttaaagtt attattgtta ttattatttt aaattagtag atatatgtat 13200
   tgtttttaga gttagtagtt tagaaagttt tgtttttttg ttgttaggtt ggaatttagt 13260
   tttttttatg gttttttttt ttagtgtgat ttttaagtta gtttaatggg tagttattgt 13320
   gtttttttag ttatttttaa atatatatat ttatatgtat tgaggagagt ggaggagaat 13380
   aagttatttt tggtaggttt ttgatatttg ggtgggtagg atttagttat taggtatatg 13440
   ttggtaggta agaggagtag aaataaggag atatattaag tggttgaggg tgtgatagag 13500
   ggaggaattg taggataatg tatattaggg gttggattat gaaaggaatt ttaggaattg 13560
   atattaaaat tttgtgtttt atttgaaatg ttttattttt ttttttttgg taatatatag 13620
   gtagagaatt atagagttaa tagaaaggta ttttatttaa ttttagaagt tattatttgt 13680
   aggatgtatt tttattttta tgtattaata taaagaaagg aagttattaa ttataattgt 13740
   attattgatt ttaagatata tttagatttt ggagatgtaa aatggaaagt tgagggtatg 13800
   atttggagtt gataaagtat tatatatttt atgtgtgttt gtgtgaagag attattaaat 13860
   aggttttgtg tgagtaataa agtttttaat tatttgggtg taggtgggtt gagtttgaaa 13920
   agaaagttag ttaagggaga taggggtggg gtggttttat aggatttggg aaggtaaagg 13980
   aaaaaggggg gttgtttttt ggtgggtagg agtgggggtt ataaggtatt taatggggga 14040
   gtttttgagt taggatgagt taggagaagg aattttataa gaaaatgtta ttagttaagg 14100
   taggaatagg ttatttttat tttttttgtg gtggaatgtt attagttaag gtaggaattg 14160
   gtgatttgga tgtgtatgtg taggttatag gggatatgat ggttttgttt gggtttagag 14220
   ggttgatatt tttgtttttt tatattaata agaaaaataa aattaaatag tggtaaagtt 14280
   ttgggatagt gaaaattttt gggggtggta tggagagata atgggtgatg ttttttaggg 14340
   ttgttttagt gggattaggg gtggtgtggg aatttagagt gggagagatt aagttgaagg 14400
   aagattttgt ggtaaggggt gatattgtgg ggttgttaga aggaatattt gttatttaga 14460
   attattggtg atggtttgga tatagttttg tatgaattga aaaattaaat ggaataagag 14520
   aaggagaaaa ataggtataa aaggtttaag aattgggata atttaggata tttgattaga 14580
   gagtgtttaa ggagatttag tatagttttg ttagtaaaga ttatttattt attttaagag 14640
   ttaagagtgg tagtttgggg atagtattag gagatattag ttgtgatggt ttggagaaat 14700
   agtgtaaatt ggtagtgtaa ataagagtag ggtgtgtatg agtagttgag aatggtgaat 14760
   aggagtatga ttagatagaa gataataggg atgataagtt tttttggggt atagtttaag 14820
   ttggtttggt gtttggaatg agattggggt ttaataaaaa ggagtgttta tataggagtt 14880
   taaatgggtt gtattttgta gtattttgag gataggtttg atttttgaga aaggaaagtt 14940
   gtaaaagtat tgtttagttt tttttaagtt ggtagttgag tttggtgagg tgtgttttta 15000
   aaagattttt agtttgtttt attttttttg aagatggagg attataaggg atataaagat 15060
   tttattgaat attaagagtt tgaaaaattg tttggttgat ttgattaata aaggttggtt 15120
   tgttattaga ttgtatagag gtgggaaggt taaattgagg aattatgttt gatagaaggg 15180
   aagaaatgat tgtggtggtt tttttagatt ttataggaaa ggtttttatt tatttagtga 15240
   aagtgtttat ttagattaag aggtatttta gttttttgat ttgggatatg ttgagtaaag 15300
   ttaatttgtt agttttgggt gggggtaaat ttttaagttt gatgtgtagg gaaggaaggg 15360
   gtttgaataa tttttgagga gtagtagaat agtagatgga atattgagaa gttatttttt 15420
   tgaggataga tttttatgat agaaaggaaa tgagaggttt taagaggtgg gttagtggtt 15480
   tgtattatag tatagtttgt ttttgttggt gtgtggtgat taggtttggt ggaattgtta 15540
   ttaataaatt aagtgtgatt agggtgagga ataggaaaga aggaaatatg gggaaatggg 15600
   gtgaatgtta ggtggattag agagatatag ttatgggggt taggtgtggt attaggaata 15660
   atgtgggagg ttggattgaa gtttgggtta ggaataatag taattgtggg atttaataaa 15720
   gattgagtat agttgaagga gttggggagt agaaagtata tgtgttaggt gtgaggaaga 15780
   aaatagattt tggaagttat gagaaatgta gagagtgagt tgagtatagt ttgtgatttt 15840
   tagggttttt aaaagtatta gggtggtagt agttgttgta tggagatatg agggttaggt 15900
   taaaatagta aggttaagtt gtttgtatag gaaggttata gggtgtggtt ttggtttttg 15960
   tgtaagaatt ttgattgtat taattatgtt taggaaggaa aggtgttgtt gttttgtaag 16020
   ggattgaggt ttgggagatt aattggatat gattagtagg gaaagtatgt gtgtttttat 16080
   gagaattatg ttgagatagg taatagatga ggatgaaatt tgggtttgat tgaagtaatg 16140
   ggggttgttt gtgaagtttt gtggtagtat agtttaggta atttgttgag tttaatgggt 16200
   gttagggtta gtttaagtga aagtaaagag aggttgggat gatgggttta aaggaatagt 16260
   aaagaaagta tgtttgagat ttaaaataga ataatggatt gtggagggag gtattgagga 16320
   taggagagta tatgggtttg gtattatggg gtggataggt aaaataattt ggttgataag 16380
   gtatagattt tgaattaatt tgtaaggttt gtttggtttt aggataggta aaatggggga 16440
   atggtaagga gagtttatag gttttaaaag gttatgttgt agtaggtaag tgataatagg 16500
   ttttaatttt tttaaagtat gttgtgggat gggatattgg tattaattgg ggtaaaggtg 16560
   gttaggtttt aatgagatgg taagggatgt atgattggtt attaaggagg gagtagaggt 16620
   attttatatt tgtgggttaa ggtgggggaa tataagagga ggatgtaaag gaggttttgg 16680
   attgggaaga agggtagtaa tgagatgtag ttgtaattta ggaatagtta gggaagtaga 16740
   taatttagtt aaagtggttt gttttaataa gggaattggg taggtgtgga taattaaaat 16800
   gagtgtttaa aagagtattg tttaagttgt tattagagtt ggggagtttt aagaggttta 16860
   gaagtttggt tgttaatatt tataatagtt atggaggtaa gggaaatagg tttttgaaaa 16920
   gaaggtaatg tagagtgggt agtttttgta ttgattaaga aggggatgga tttatttttt 16980
   attgtgagag ttatgtgaag tttggtgttt gtgatggttt agggggtttt taaggtagtt 17040
   gggtagtatt agtttttagt tgttaagtta agaagatttg ggaagtagtt agagagtttt 17100
   gggttagagt tttaggggtt ttgggagtgg ttgttaggtg agttggatag tttgattttt 17160
   agtggggttt tatatagatg ggatatggtt taggaggaat tttgggttgt aggtattttt 17220
   tggtttggtg gttagatttt tagtatttgt agtaagtttt taggggagga ggttttggag 17280
   gaatgtttgg ttgttgtggt ttaggtgttt ggaagttttt gtgtgttgga gatgtggttg 17340
   gggtttgttt tatagtggag gtaaggaatt gtaatttttt tttattattg tatattttga 17400
   aggtgaggtt aattaaattt tgttgtgggg tttgagtgtt agattttaat ttttggagtt 17460
   ttatttaatg ttgggagtag attgggtaat aaaatgtata ttgagaataa gatggttttt 17520
   tgatttttta gggtttaggg ttgtaaagtg ttttagggtt gttgttaaag gagttatgaa 17580
   ttgggttggg tttttatgtt tgatgaaaat gttatttgat ttgggataaa gaaaaaggag 17640
   tattaatttt gattgtgttt ttagttttag ttattttttt aagagttaat tgttgggtag 17700
   gtgggggagg gttagttatg aaatgaaatt gtaaggtaga ttaggtgtga ggaggggagg 17760
   tgataaaagg attatagggt ggaggagtgg aggttgagga agaattggga tttagttggg 17820
   tttggtgagg agtagtttgg ggaggtgggg agaggttaga tgggtttgta gaaaaggaag 17880
   attagaaagt tttagtgatg tttggggttg ggattgaggg gataggtggg agggaaagaa 17940
   ggaagatttg ggatgagttg tattgggaat agagattaga gagggattga tgtgtaaaag 18000
   aatgtttgga tgttaggtat tttagattat ttgtttattt tataataaga attatttaga 18060
   ttttgtagga tggaaaaatt gaaagtgttg ttttttggtt atttggaatt attgttgagt 18120
   ttgtattggg gttaagtggt attgtagaag aaaataagat gtttagattt taatttaggt 18180
   gagagttgaa gaggttttaa gtttttgagg atataggtta agggagaaga aggaggaatg 18240
   gagggtggaa agttgtttat agtgaaggaa gtaagtttag agaaaagaga gagtagagat 18300
   atggagggaa ggggtttggg ggtttttatt ttttagaaaa gtgggaaagg ggttggggta 18360
   tggagaagag gttggggtat ggaaataagg gatgggggta tagagattag aggttggggt 18420
   gtggaaataa gggattgggg tatagagata taagaggttg gggtgtggaa ataagagatt 18480
   gggtgtagag ataagaggtt ggggtatgga aataagggat tggggtgtag agatatgagg 18540
   ttggggtatt tgtttttttt ttagaaaagt aggatttgtt gtttagggtg aaggagaagg 18600
   ggttggggat tttttgtttt ttagaaagga ggagaagggg tagagatatg gagagaaggg 18660
   gttggggtat ttgttttttt tttagaaaag taggatttgt tgttaagggt gaaggattaa 18720
   ggtaggtgtt tttgtatggt ttgatatttt tgaaatttgg gtgaataatt attgtagttt 18780
   ttgtaatgat taaatattaa gggaaggttg tttttttagt ttttgattgg tgttggagtt 18840
   ttgggtttat ggataaaatg tgtttttttt gtttttatta gaaaatgaaa ggaattgaaa 18900
   ttaagagaag ggagagattg aagagtggaa aggagaaagt ggttgaagga tagtgagaga 18960
   ggttggagaa gagagtaaga agaggttgtt tatttgattt aaaattggtg agatgttttt 19020
   tgggttggtt ggtttgagga tttgaggttg taggtggatt ttttttatgg agtaaagaat 19080
   aggaggatag ggattgtttt ttttaaggga ggttttttga tttgatttat ggtattaaat 19140
   tttatgtatg tttgtgtgaa gagattatta aataggtttt gtgtgagtaa taaagttttt 19200
   aattatttgg gtgtaggtgg gttgagttta aaaagagagt tagttaaggg agataggtgg 19260
   ggttgtttta tatgatttgg gtaggtaaag gaaaaagggg ggttgttttt tggtaggtag 19320
   gagtgggagt tataaggtat ttagtggggg agtttttgag ttaggatgag ttaggagaag 19380
   gaattttata agataatgtt attagttaag gtaggaatag gttattttta ttttttttgt 19440
   ggtggaatgt tattagttaa ggtaggaatt ggtgatttgg atgtgtgtgt gtaggttata 19500
   ggggatatga tggtttagtt tgggtttagg ggtttaataa tataggttga taaggatttt 19560
   ttttttgtat taagtatttt ttagattttt tggagtggtt agtattaagg gtattggtta 19620
   ttaattttta tttttattaa tattatttta tgtagataaa tgtagataat tgatgttttt 19680
   ttaattttat gtaaagttgg tgagaaggtg atttagaaga ttttgttatt tttattgatt 19740
   taaattaatt taagaaaaga tagttatttg ttaagtagtt gtagtttaaa ataagttttt 19800
   tatttttgtt ttttttttaa ttttgttgtt tatgagtttt gtttggtatt agtgaaagtg 19860
   atagaaatat atgtagtgaa agttatatgt attttagttt tttattaaaa tgtatattta 19920
   gaggtaaagg ggtgtaatgt ttgttattta tttttaaatg gtttaaggaa aaaatagagg 19980
   ggtttgggga gaaggagagg gagagagagg agaatgagaa agtaagtgtg gttaatgtta 20040
   attggagaat ttgggtaaat gggagttttt tgtgattttt tgaaattttt ttgtaattat 20100
   attaaaatta aaagttaaat ataagaatat aaaaaaagat ttttttaata ttttgatttt 20160
   atattgtttt tttagttttt tttgagggtt ttttgtaagt aaggatagtg gaaagaaagt 20220
   gggattagtt tgttgtttag ggttttgtat ttgatgtgtt atttttggag tttaggatta 20280
   ataaaaagtt tgtgtttaat tgtagttttt ttttttagta ttatggatta taatttgtat 20340
   tttttgtgtt gtttttgttt tttgaatggt ttgagtgtgt tttgtttttt ttattttagg 20400
   ttttttttaa ttttttttga aagtagaaga aatatgatat aaggtagtgt tatgttttta 20460
   gttttattgt gtttttgtgt taaggggtat aatttattta gggttttatg tgttttataa 20520
   atgtagtaaa tggtgttgtg ttttaatggt tttttttagt attattttta gttttttttt 20580
   ttggtttgtt tttttttgtt tttttttttt tttttgtttt attttttttt tttttttttt 20640
   attttgtttt ttattttttt tttttttggg tttattttta ttttttttat ttttattttt 20700
   ttaagatttt tatttttgtt ttaggtgttt ttttttttgt tttgttgttt tttttttttt 20760
   ttttattttt ttttatatag aagagtaaag agttagggta gaggggaaaa gagtgaggga 20820
   aataagaagg gggagtagag agatatagta attaagaaaa agaaaaagaa aaaaatattt 20880
   agttttgggg ttaaaagata gttatggtgg ttagttattt tgtttgggtt tttaattggt 20940
   gtatttattt tttttgttta atgtttgagt atgtgttttt gttgtttaat atttgtgggg 21000
   tgaagttttt attgatttgg tatagtgatg attttatatg aagggaaggg atgttagggt 21060
   ttgtggttgt ttttgtatgt ttgttttttg agtttttatt attatttatt ttattttttg 21120
   aaatagattt tttttttttt tttttttttt tttttttttt tttttttttt tttagagttt 21180
   tgttttgttg tttaggttgg agtgtagtgg tattattttg atttattgta gtttttattt 21240
   gttatgttta agtgatttat ttgttttagt ttttttagta gttgggatta taggggtgta 21300
   ttattatgtt tggttaattt ttgtattttt agtaaaggta gggttttatt atgttggtta 21360
   ggttggtttt aaatttttga tattaggtga tttatttgtt ttggtttttt aaagtgttgg 21420
   gattataatt atgagttatt gtgtttagtt tgaaattaat ttttttaata gtgaaagagg 21480
   gagtttattg tttataattt tggttaatag ggaaatattt tattttttta attttttgat 21540
   taattataga aaatttttag gtagataaag gatattagtt ttttttttta tttttatttt 21600
   ttaaaaataa atatttatta ggttattaag aaaatatata aagtaataag taaaatattt 21660
   tattattaaa agaatgtata tttgggttta gttaagatta taatatttag ttatggatat 21720
   gttgattggt atagttttta ggtttttgga tggttagtat ttagatttta gttttttttt 21780
   ttttttttgt ttgtttgttt gtttgtttgt ttgtttgttt gtttgttttt tttttttttt 21840
   tttttttttt tttttttttt tttttttttt tttttttttt tttttttttt tttttttttt 21900
   tttttttttt tttttttttt tttttttttt ttttttttat ttatttttta gtttttagta 21960
   ttagatttgt atttttttat aggttttttt ttttttttat aaatggatta aaattttggt 22020
   tgaaaatttt gggtgtattt agggtttgag aaaataattg atagttaaat aatttaagtg 22080
   gttagttttt ttttgtatga ttttaatgga gaatataaag gttggatagt tatatgttag 22140
   tttttttgat tattgatttt taataggatt tttttttttt attattgaag tgattatttt 22200
   ttaattgttg tttgttttat tgttggattg ttttggttag aaagttgtag atgttagaag 22260
   gtttttttta atattaagaa aaattttgga gtgaattttt ttgttttttg gtaaatttag 22320
   agttattttt tggagtgata taaagtaaga tttatttttt tttgttgtta tatgtttgaa 22380
   gatggaattg tgttgttttt gagttatttt tttattaatt aaaaatgttt tagtttttta 22440
   tttttgtttt ttggataatg tgttttttta gtatgtttat ttgagtatgg gtttgaaatt 22500
   gaatatagta ttttaaatat gatattttgt ttagtttagg ggggtgtggt tattttttat 22560
   tagatatagt gttattttta atgtaaataa agtttttgtt ttgttttaat atatttgtat 22620
   tttattatta aaatatttat attttattaa gattgtgata agtaaaaatt tttatttttt 22680
   aatataggat ttggtagttg gttgagtttt tagttgtatt tgtattattg attttttaaa 22740
   aaaatattat atgtgagatt ttatatttat ttttattaaa tgtattttgt ggttttagga 22800
   tggtgttttg gtttatagag gttatgttga tatttttagg agtatagatt aggataggat 22860
   aggaagatta tttagggaaa gtatggttta gtttggatta tttttaggta aaaatttttg 22920
   gattgtggtt tatgttatat agttgtatta ttatatattt agaatattat gtatattaaa 22980
   ttattttttt attttggtta gatatttatt tttaaaatat atatatgtag taatatagat 23040
   ttttgttgta agagagttaa ttagtatata aaaatatgta gaaaaatgag aataaaatga 23100
   gaaaatttat atttttattt ttttttagag taattatggg tattatgtgt tttttattta 23160
   tgtatatgta tatggttgta tatatagata gatagataga tttattttat gattttaaat 23220
   tattgtatgg tatgattatg atattattat tgttattatt attattattg agatagggtt 23280
   ttgttttgtt atttaggttg gaatgtagtg gtgtgatttt ggtttattgt aatttttatt 23340
   ttttaggttt aagtaatttt tttgttttag gtttttaagt agttgggatt ataggtttgt 23400
   ggtagtatgt ttggttaatt tttgagtatt ttttagaggt ggggttttgt tatgttgttt 23460
   aggtttgttt tgaatttttg gatttaagtt atttttttat tttagttttt aaagtgttgg 23520
   gattataggt gtgagttatt gtgtttggtt aatattattt ttttaattat taatggtgat 23580
   ttagatggtt atttgtgttg tttttaattt tattattaga gattttattg tagtgatttt 23640
   atttttttgt gtaattttga atttatttgt aaatgttttt ttagtattga tttttaatag 23700
   aaatgtttga gttataggga tatatgtata tatgttatat ttttattgtt ttttaaaaag 23760
   gtttgttaat ttttattttt atgaattttg gttttgtatt aagtagaatg tatttaaata 23820
   tttttttttt tataaaggat ggttattttg atttaggttt gttaatatgg aataaaataa 23880
   ataaaataaa aattttattt gttttttaga tttatttttt tttttaaaga atatatatgt 23940
   ttgtgtttag attatttttt ttttttgata aatgagggaa atttttagat gaaagttgat 24000
   aaaatattag atgggggtga ttttggattt ttatattttt atggaatata ttatagaagt 24060
   taggattgga gagttaattt tatatatttt tatatttttt tttagatgaa tggtaatttt 24120
   ttataggttg tgggtgtagg tttaaatgtt ttttgggttt gtttaatttt ttagaggata 24180
   ttaaaggtaa tatagttagt atatttaatt attaggagtt tggtttttgg gattgggtat 24240
   ttttgggtgt gagtattagt ttttttattt ataagttgta attttggtta tgttatttaa 24300
   tttttttgag tttgattttt tttatttgta taagataggt aatatattaa ttatagaggg 24360
   tggttttgag gattaaatga gatattgatt gtaagaaaat aaatagtata gttgatggta 24420
   gagtgagtat ttaataatag ttagttatta ttattattat tattatttat taagttttta 24480
   tttttgttta gtaaaattta agatatggta aggatttgaa ttttgtagga ataaaaatat 24540
   ggtttatttg gtttagtgtg atagttttta gggtggtgtg ttgttattta tttgtaaatt 24600
   tttaatagtt taaagtggta ttagttattt tttttttgtg ttttttaggt agtatattga 24660
   gtggttgtta gtattatttt agagttagat tgaatttgaa tttggttttt ttatttatat 24720
   tggagtgatt tttatgttag aaattgtttt tttgggttta gtttatattt aagtgaatgg 24780
   agatgtaaaa tggagatgat attagtattg attatgtaga gttgtggtga ggattgaatg 24840
   aattaatata tgtaaagttt ttagaagggt gtttggtatg tggtgggtgt ttaggtatta 24900
   ttagttgtta ttattatttt gtgggaggtt atttgttgtt gttttaagaa ggttgatttt 24960
   atagaaatat ttgttgttgg tagaagagtt tagtattaat agttaatttg tgtttttttt 25020
   taatttttag atggtagttt tgtagtattt tttttttatt taattttttt ttatttagat 25080
   gtgttttata ttaggatttt taggtagtat agagatagtt ttttaggtag ggggatgtta 25140
   tgtttttaaa agagtttgat gaggttatag taggtttttt ttattaagtt atgtttttgg 25200
   ttagtttttt tgtgattgtt atggatgttt tgtagttttg tggtaatgag gttttaggat 25260
   aatatttatg taattttttg ttgaggagag tttagaaaat tttatgttgt gtagtgtaat 25320
   tgggaggaaa tttaagggag tgttgttgtg ggattttttg gtggtgattt gtataaatga 25380
   tgtaatgtta tttagttttt tagaaaatat tgtttttttt ttttatatta tagttttgag 25440
   ttaaagtgta ttgtggtttt agatttgggg tatatagttt atttgatagt tataatgagg 25500
   gaaatagttt atttttggag tttgtattag aaggtagaat gggagatttt tagttttttt 25560
   ttattaaagg ttagggagga aattgttttg aatgtttttt agtttaaaat ggaaagttag 25620
   atttaatggt ttgtattttt gggttgatta atttttgttt tttttttttt tattgtgttg 25680
   agttagttat ggggggtggg ttgtgtattt taagggtttg aatgtagaag gaaggaaggt 25740
   tttttagttt ttgagttttg agaaggtggt attgtggttt ttaattagta gttatgtttt 25800
   taggtatttt atttttttat attattttag ggattttttg gtgtaataag gatgtgattg 25860
   agttttgagt ttttaggtta ttagttatat tttagtggaa ttttgggagt tatggggtaa 25920
   aatggaattt ttttttatag taagtgtata gaaggtattt agggttggtg ggaattttta 25980
   tttttgtttt tatttatttt ggattttgaa tttaatattt gaggtttttt gtagtaaatt 26040
   agagaggtga agagtaattt atatgtattt tttttttttt tttttttttt tttgagatag 26100
   ggttttttta tgttgtttag gttggttttg aatttttggg tttaagggat ttttttgttt 26160
   tagttttttg agtaatatat attatttaat aaggaataaa tattataata attttgagta 26220
   ttatagttgg agtattgtgt tttgttaaat gtgaggagta ttttttttga tgttattatt 26280
   ataaggttag ggtttgtggt ttaagttttt ttgttttttt agagtttttt atattatata 26340
   ataagtttgg agatagagaa agtagttaat attttttttt aagtgttagg tattgttttt 26400
   tattttttat attgatagtt ttatttattt tttttaatta ttttaaaaat attggtatat 26460
   ttagtttgtt ttatatataa agggattaag gtttagagat agtaaattat ttgttaaatg 26520
   ttatagaggt tgtaagtgag aggggttagg atttgaattt aggtttgtag gtattaaagt 26580
   ttatttatat tatattagta tatatttgtt gatttattgt gtattatttt aagttttata 26640
   gaagttttat ttttttaatt atagtaattg tagaggtttt tagtattgtt atgtttatta 26700
   gggatttggg ttattttatt gttttttttt gttttgtgag ttgatttttt ttaggagttt 26760
   aagattataa taagtaaagt tattttgttg ttagagtttg atgtaattta tatttttttt 26820
   tttttttttt tttgtagaat atttttggta tttgtttagt aatttaattt ttataattta 26880
   taattaaatt ttgggatggt ttagaaaatt tttttttaat atagtggttg gaatatgtag 26940
   agagttggta tgtagttttt atatgggttt tatgattaga tttgttatat ttaaatttat 27000
   tatattgaaa ttattataaa atttatatta ttttaatttt tagaaatgat ggttttattt 27060
   agaagtaagt gagtaaaagt aatgatttta taggttttag tgttaaaatt ttagtttaat 27120
   gttttttaaa tgttttgttt ttagaatttt tttaaagttt taaaaaatat tgtagagttt 27180
   aaagaattat tgtttagtta agtatttatt aatatttata attttagaaa ttaaagttga 27240
   aaaatttata aaatatttgt tagtttatta aaaataataa atttaatata tgttaatata 27300
   aataatattt tatggaaaat aattagtttt taaaattata aatatttagt tagagtgggt 27360
   agttttaaaa aatattgtta taaatttttt taatttatga tttaatagaa gatagtaggt 27420
   gatttatatt tgtttttgta gaaagatttt ttagtaaatt tgtaagagaa tgagagttag 27480
   aaatgtaaat aatgttttat ttttatagtt gtaattttgt gggttttttg gaaagggttt 27540
   taggggttat tttgatgggg tgtttggatt atattttgag aattattgtt taaatttaag 27600
   agtgtgtttt ttgggttggg tgtggtggtt tatgtttgta attttagtat tttgggaggt 27660
   tgaggtgggt ggattatgag gttaggagat tgagattatt ttggttaata tggtgaaatt 27720
   ttgtttttat taaaaaatat aaaaaattag tttggtgttg tggtgggtgt ttgtaatttt 27780
   agttatttag gaggttgagg taggagaatg gtgtgaattt tggaggtgga gtttgtagtg 27840
   agttgagaga gtgttattgt agtttggttt gggtgaaaga gttagatttt gttttaaaaa 27900
   aaaaaaaaaa agtgtgtttt ttgtttgtgt gtgtgtgtgt gtgtgtgtgt gtgtgtgtgt 27960
   gtgtgaatgt ttttttattt aagaaaaatt taaatgggtt attgttttgg tgtaggaagt 28020
   aggtgagggt gtggtggatt ttagaaaaaa taatgtttta gttattgatt ttaagagaat 28080
   agttttgaga taagtgtggg tgtattgaaa tattattgta attttttaaa ttattgtaga 28140
   gttgttatta ttaggtgttt ggttagaaag atgttatttt tttaagatag ttatttgttt 28200
   agatgatgta ttttagtttt aggaaggagg aggaagaaga ggtagataga atgatgttta 28260
   aatttttgtg gtttggagat gaggtagtgt ttgagttagg aagttgggag agtttagttt 28320
   agtggaagag gtatgtggat tttggttagg tgtgaagtta ggagggtttt ttagttatag 28380
   tttgaaattg aagtttagga ttgagttata gtttgaaatt tttggattgt agttttgtta 28440
   attaaattaa tggtaataat aataatgtat tgttatatat gtaagaattg ttatatatgg 28500
   agtattttga gttaggtaat gtgttaagta tttgatagaa attattttat taattttata 28560
   tagttaattt tttgagataa ttattgtttt tatttttttt ttatagataa ataaagtaag 28620
   gtaggaagag ttggaattat taattaaggt tatatatatt atgttggttg agttgaaatt 28680
   tgaatttagt tttgttgggt tgtaaaaatt ttttgttgtt ttttagttgt attagttagg 28740
   taaatttttg ttagaggaaa ttttatagtt ggagtgtagt aggtatagat tttttttatt 28800
   taattggggg gaggttgtag ttatagtgaa gggaatatta ttgtgtttta tgggaagaaa 28860
   tatgaagatg gagtggatgt tttattttta ttttaattag ttatttggag ggtttagggt 28920
   tgaattgtag tgggggtatt ataaaattta gggaggtgag gagttttttt tagggaggtg 28980
   atagtttgag tatatggttt ttaagttttt agattttttt ttgtattttt ttttatttag 29040
   ttttgtattt ggtattgttg ttattagtta ggaagtttga gatttgtgta aattttgttt 29100
   ttttttgttg tttttgtaag tattattgtt tttttaaagt ttagatgtgg ggttttttgg 29160
   agttttgttt tttagagggt tgtttggtat atggttattt ttatattttg ttttttttga 29220
   gtttagtttt ttagaggtgt gaatttttta taaatatata ttattattta attttttgga 29280
   aaaataaata ggataattgt gtgaaaattt tattttaagg tatttttttt tttttgtaat 29340
   tagaggtttt aaggagttta gtatagtatg agtttttaat ttgtgtgtag ataagtataa 29400
   aaggtataat tggatatatt tgttattttt taatgatttg agagtttgaa gtttatgtta 29460
   agttttgggt tatggtatag tttgtatgta aggttttgta gtttttgtag gtaattgaga 29520
   ggtggtggtg tataggatag aggaataatt ttgaagtgat agtatataat ttaatttttt 29580
   ttaagttttt taagtatgta gattgttttt tttttgttag tgtataattt aagtgatttg 29640
   ttttatttag gtaagaaatg tttaatttaa attatattgt agttaagttt ttagtgaaga 29700
   aggagaattt tttgaggttt agttgtgtgt ggttaggttg gtggtagttg aggtaaagta 29760
   gatttttttt agtttttgta ttttttattt aggttatgat tggagatatg tgggttgggg 29820
   ggttttaatt attatattta tttttttgtt ttatatttta ttttttgtga atgggtttag 29880
   ttttagtaag aatgtttata ggagagtgtt ggttatatgt ttttaatatt ttgggtttga 29940
   gttttatttg agttattagg agttgggtga ttttagggaa attttttaaa ttttaattat 30000
   tgtttttttt tttttgagaa ataggataaa ttggtagtgg gggtgtatag attggtatat 30060
   ttttgtggta agtgatttgg tttaggaagt aagagatttg tgttttagtt tagttttgtt 30120
   tttaattagt agtggatttg tagtaaggaa tttttttttg ggttttaatt tttttatttg 30180
   taaaataagt aggttggatg aaatggtttt tgaagttttt ttagttataa aatattatgt 30240
   atgttttaaa ttatttggag tttgataaaa taatagttat aataatttta attattattg 30300
   tgattttttt ttataatttt aaagatttta ttagttatgt aaatttttta aaattttttt 30360
   ttaaaagtat tttttttggt tttattgata tgaagtaaag aaggatttta ggtttttaga 30420
   tggtaggatt tggggtagat attttgtgtt tgttttattt tttatggaat tattaaattt 30480
   tttttttagt tatgttagat tgttaaatgg tttttaagta tagtttatat tttaaggttg 30540
   tttttttagt taaaaatgtt tttttttttt gtttgttgtg aaattttttt ttttttattt 30600
   tttttttttt attatgggat agtttttttt tttgggtttt tttgagtatt ttatattatt 30660
   attttattga ttttatgttg ttatttttgg ttttgtattt aatatatttt tgtttaatta 30720
   gatttttagg aattttgtta tttgtattat ttagtttttt ttttttagtt ttttttagta 30780
   tttggtatat atttatggaa gaattaattt ttgaattttg tggattttat tataatagga 30840
   taagaatgtt gtatgggttt aggatagatg tttataattt gagatgagta gtgttagttt 30900
   tagtttttaa aggaaattta gttagggtgg tgattagaga tattttgaat aattatttta 30960
   ggaaaggatt aaatttattg aaaagttgtt tattttagtg aaaatgttgt aattgtggaa 31020
   agatataaaa gataaaatgt ttataattgt atttttggaa gtgttttttt ttaaagaata 31080
   ggaattattt tattaaagta tttgaagaag aatgtgtgtg gttgtttgta tgtagttgtt 31140
   tatatttaag aatgttatat attttatttt ttttaggatt atttagttta agagaagaat 31200
   ttgaggttat ttttagggag gaaaggatat ttatattggg aatttaattg atggtagtta 31260
   agtgtttttt ttttttaata attgggataa gtatgatata tataatttta tatagtatgt 31320
   ttggggagtg aggagagaag ttagagaaga ttgagatgag tgaaatgtag gtttgattga 31380
   gaaattaagt agaattttta ttaatagtga ggattgaggg agaagtgttt ttgtaatgga 31440
   ggggaaatga ataaaatttt gatttttagt ttgataggga tttttttttt tttagttagt 31500
   tggaatgtaa gttttaaaaa aaaaggaagt gtggggaggg ttattttttt taataagatg 31560
   ttttagaaat gattatgaat atagtaaagt tggtttttaa aaaaggaaag tgagaaaagt 31620
   agtgataatt tgaagttggt agttgaggaa gaaggagaat ttttgaattt atgaagagag 31680
   tatatttggt ttgggtgggt aggattttaa agtttttagg attgatggat tagaatagag   31740
   aaatgaaaat gtaataagaa tagttgtggg gaaaaggtag aaggggggta ggaagtgaag   31800
   agggtgtgta tttttttttt ttgatttgag aaagggaaat tttgtgttgg gaagagttat   31860
   gtgggaggag gaaggaaagg tgttaggaat gtttggaagg tatatgatgt ttgattttat   31920
   gattttttgt aataattttt atattgattg tgttttttta tttttgtttt aaaaatgtgg   31980
   ttaggtaaat atttgtttta ttaatattgt gagaaattag ttttgtttat agaattttat   32040
   aaaataattt gtaataatga gaggttgtta ggatgtaggt gatattagga gaagtaatgt   32100
   tttgtttgaa tttttagggt tttggaggta ttgttttggg tttaaaattt ttttttagtg   32160
   attttgaatg taatgaaata ggagagaatg ttgattttag agtagtaata gtataaatta   32220
   tagttttaat gtttagtagg tttagtttgt tggaataatt agtttaggaa atgtatgatt   32280
   taagatgttt ttatagtggt ttttataaag tttgaagggg aaataaagag aaagttagag   32340
   agttttttat agaagggaat taattgtgtt tatatgtaaa agatttattt tttgtggtta   32400
   tttaagagta gtgatttttt gggggtggaa taatttatat atgtgtaaaa atatttattt   32460
   ttttatatat ttattttaag tatgtgttaa aatatttgta aataatttta gatttttttt   32520
   ttattttatt tttattttaa aggtagtgtt atatggatat ttaaatgttt agagaggata   32580
   attttaaata tataaaagaa tgataaataa ataaataaat aaataaataa ataaaaaaaa   32640
   gattttttaa tttgttatta tttgtatgtt ggaaattgat agtgttaatt tgggtaatat   32700
   ttttttaatt ttgattgaat tatatgttaa ttttttgtgg tggttttaga ttttagatgt   32760
   tattagtaaa ttatggagga aaagtaagat tatatgattt atgaattatt atagtagtaa   32820
   tagtttttaa tttttattta ttttttgtgt tatgtgatat gttttagaaa tttatttaaa   32880
   attgagaaaa taagttattt ttaattgtat ttaagtaagg aaatagttta tttatgaatt   32940
   taagaagatt aaagaatatg gttttagtgt gtttgtttag aatttatgtt aaaatttttt   33000
   tttgaagtat aaatttggtt tttttttttt tttttaaaaa aaattttaat aaagttaaag   33060
   aaagaaagaa aggaaagaaa gaaagaaaga gggttttata tgtagtttta gtattaaggg   33120
   attttgataa gtaatttatt tttgttttga atgtgggatt tttgtatttt atgatatata   33180
   tttttggagt ggttttgttt tttttgtggt tttttttttg tttgtttttt gttttttgtt   33240
   ttttttgtgt gagatagtgg atttttttat ttattattgt attattggag ttttatgtga   33300
   tttaatgtat aaaaagtgag gttatttttt aattagttgt ttgggttttt gtaaagttta   33360
   atttttatag tttattttaa gaggtttttt aaaaatgaaa ttttaagtat gttgtttgat   33420
   attttttaat atgtttttaa ttaatttgta ataaagttta gttttttttt tattgtatat   33480
   tattgtattt tttttgtttt ttttatgtat tgttttttat ttattttggt tttttggttt   33540
   tgtttttaat gttagagtga tttttgggtt tgtttttttt tttttttttt tgtttggaaa   33600
   tttttttttt gaagaatttt taaatggttg ttttttttat gtattttttt ttggagtatt   33660
   attttttgat tttttttttt agggttttga atttatttta ttgttgttta tttagttagt   33720
   tttttttttt tagttggtat ttattataag taatttgttt attttgtttt tggtgagttt   33780
   atatttgttt ttattattgg attataaata tttttaggat agagatttag tttatattat   33840
   ttattatttt ttttttttag tgtttggtat gtagtaggtg tttaataaat gtgtattgaa   33900
   agaatggatg agtgatatga tattgatttg tatttatttt ttttttttag tttatttttg   33960
   ttgagttttg gtattgattt tatgtgttag tatttgagaa ggagagaagt ttttgagttg   34020
   ttttgtaaat gtggtttaag tgagatgttg aatagtgtta gttatgtgga ttttgttata   34080
   gtgaaggagg aaggtagaga aaatgggttt tagtgtgtgt gtatgtgggg gtgggagggg   34140
   tgtgttgatt taggggtgta tagaaaatgg tggtgtgttt tatgattttt ttttttaaag   34200
   aatgaaatta ggatattaat tgagtatata agaggggaaa gtggtgtagt agggttaagg   34260
   tataatgaaa agatttagaa ttattttaaa agagtaggaa agtaaattat gtagagatgt   34320
   ataatagaat tttagtgttg agtttttatt ttattattat gaatttgaag tgagatagtt   34380
   ggaatttggg atttttttaa tatttgagtt gggaaggtaa attgttaatt atttttatgg   34440
   agttagtgtt ttttgattaa gtaattttag tttaggaatt tattttaaag ataattggat   34500
   aagtttgaga aaatgtatat ataaagttgt ttaataaaat attttattat aattgataat   34560
   aaataaatgg aaagtattta aatatttagt aatatgggat tggataaatt ttgaaatata   34620
   taatgggata ttttgtagtt attaaagatt gtttgtttat atttattgat atgatgaata   34680
   ggtatgatga gttattggtg aaaaaaatgt agtttttaaa ttagtatata gtgtaaaatt   34740
   tttttttaaa agttgtttat atttatgtat tttagtataa agaaagatat ggaaggatgt   34800
   ttattaaaat gttaaatttt tttatttttt tttatgtatt tttttgaatt gggtgaaatg   34860
   ttttataatg agtatttgaa aatagaataa atagaaaaaa aaagatttaa ttagatttta   34920
   tttgttagtt tttgtttttg ttattgttgt ttttggtgtt tttgttatga aatttttgtt   34980
   agtttttatg tatagaatgg tattgtttag gttgtttttt agggttttta taattttggg   35040
   ttttatattt aagtttttaa tttattttga gttgattttt gtatatggtg tatatgtagt   35100
   tttttatata tggttagtga gttattttag tattatttat tgaataagga gttttttttt   35160
   tattgttggt ttttgttagt tttgttgaag attagatggt tataggtgtg taggtttatt   35220
   tttgggtttt ttattttgtt ttattggttt gtgtgttttt ttgtattagt attaggtggt   35280
   tgtggttatt gtagttttgt agtatagttt gatgtgatgt ttttagtttt gttttttttg   35340
   tatataattg ttttggttat gtaggttttt ttttgatttt atataaattt taaaatagtt   35400
   ttttttttta gttttgtgaa gaatattatt gatagtttga taggaatagt attgattttg   35460
   taaattgttt tggatagtat ggttatttta atgatattga ttttttttat ttatgagtat   35520
   ggaatgtttt tttatttgtt tgtgttattt ttgatttttt tgagtagtgt tttgtaattt   35580
   ttattgtaga gattttttat ttttttggtt agttgtattt ttaggtgttt tatttttttg   35640
   tggtaattgt gaatgaaatt gtatttttga tttggttttt agtttggttg ttgttgatgt   35700
   atagaaatgt tagtgatttt tgtatattga ttttatatta tgaaattttg ttgaagttgt   35760
   ttattagttg ggatttaatg aaatttaaga gtttttgtat agtaaaagaa attattaata   35820
   gagtaaataa taatgattta tagtatggga gaaaatattt gtaaattata tatttgataa   35880
   agatttaata tttagtattt ataaggaatt taaatttata agaaaaaagt aattttatta   35940
   aaaagtgggt aaaggatatg aatagatatt ttttaaaaga agatatatat gtggttaata   36000
   agtgtataaa aaaagtttaa tattattgat tattagagaa atgaaaatta aaattataat   36060
   gagatattat tttatattag ttaaaatagt tattattaaa aagttaaaaa ataatagatg   36120
   ttggtgtggt tgtggagaaa agggaatttt tatatattgt tagtgggagt gtaaattagt   36180
   ttaattattg tggaaagtag tatggtagtt ttttaaagag ttaaaaataa aattattatt   36240
   tgatttagta attttattat tgggtatata tttaaaggaa tataaatttt tttattataa   36300
   agatatatgt atatatatgt ttattgtagt attatttata atagtaaaga tatggaatta   36360
   atttaaatgt ttattagtgg tagattagat aaagaaaatg tgatatatat atattatgga   36420
   atattatgtt gttttaaaaa aaattagatt atgtgttttg tagaaatatg aatggagttg   36480
   ggggttatta tttttagtaa atttatgtag aaatagaaaa ttaaatattt tatgttttta   36540
   tttataagga ggagttaaat gatgagaata tatggatata aagaggggag taatagatat   36600
   tgggatttat ttgagggtgg agtgtggtag gaaggtgagg attagaagaa ataatttttg   36660
   ggtattaggt ttagtatttg ggtgatgaaa taatttgtat agtaaatttt tatgatataa   36720
   gtttatttag gtaataaatt tatatatata tttttgaatt gaaaataaaa gttaaaagaa   36780
   aaaagaaaga tttatttgta tagtgttttt tttttttttt ttttatttgg tttatttttt   36840
   tttttattta ttgtatgtat taatttttta gaatttttgg tgttttgggg attttgaaag   36900
   attagttagt aggattattg tggggtttag tgtagtgatg tagaggtttt tattagtaat   36960
   tttgaagttg aaagagaata tttatttttt ttatgtataa gggatagatt tgggggtggt   37020
   attgaaggtt ttttttttat tttgttggta gttgtagtaa tgtattgatt agtaggagag   37080
   gttttttttt taatgagtta gaaattaata tttgtggagt ttttgttata tattagtatt   37140
   gtgtagggtg ttttatattt aaaaaataaa tttgggttgg gtgtagtggt ttaagtttgt   37200
   aattttagta tttttggagg ttgaggtggg aggattataa ggttaggaga ttgagattat   37260
   tttgtttaat atggtgaaat tttattttta ttaaaaatat aaaaattagt tgggtatagt   37320
   ggtaggtgtt tgtagtttta gttatttggt aggttgaggt aggagaatgg tgtgaatttg   37380
   ggaggtagag tttgtagtga gttgagattg tgttattgta ttttggtttg ggtaatagag   37440
   tgagatttta ttttaaaaaa ataaaaaaat aataaaataa aaataaaaaa taaatttgtt   37500
   taatttttat aataatttta taatgaaaaa attgttattt ttattttata gttgagaaaa   37560
   aaatgaggta taatgaaata aaagtatttg tatagtatat aatataggaa ggagtggagt   37620
   gagaatttaa agttaagttt atagtatgtt gtttttaagg taaaattatt tttgatttaa   37680
   aattatattt tttttttttt ttaatttttt ttagttagtg ttttatttgt gtggatttgt   37740
   taggagataa ggaaaattaa atttttagta atgtgtattt agtgtttaat aattatatta   37800
   ttatatatga aaaaaatttg aagtttgtta gattattatt gtatgaaata ttttttattt   37860
   gggattatat agttttgtat ttttttgatt agttataatt tttaggattt agtttttatt   37920
   ttagagattt agatttgtgt attttgtatt tttttgtatt ttgtgttatt gttttattaa   37980
   gagtatattt taggtgattt ttttgtgtgt ttttaatttt gttattttgt ggttattaat   38040
   aatattagtt aataataata ttattatatt gtttattata atgtgtgttt gtgagtattg   38100
   gtaataattt agtagaatag aatgagttag atttttaatt gtggtagatt atgtgtagta   38160
   gtgaatagag tatagaaatt ttggatttta atttttgatt ttttggtagt gatatttttt   38220
   tattttatta tataaggtat aagtggtaga agattaggga gaattatagg gttgtaagat   38280
   attttataag ttgttaaata aggggatatt gttttatttg gggtatgtta tttaattttt   38340
   ttaatattgt gttaggtagg tattatgaat taaattgtgg ttatttaaaa tttatatttt   38400
   gaattttggg tattttagaa tataattgta tttggatatg gagtttttaa agaggtaatt   38460
   aagttaaaat gaggttgtta gagagggttg taatttaata tgattggtat ttttataaga   38520
   agattgaaaa tatagatgtg tatagagtaa agattatgtg aagatataaa gaaaatagtt   38580
   atttgtaatt taagaagaga gttttagagt atattgtgat ttttgatttt tggtttttag   38640
   aattgtgaga taatgaattt atattgttta agttattagt ttgttgtatt ttgttatggt   38700
   agtttgaatg ggttaatata gtgaggtttt ttggaagatt tttagatggg gatttgtatg   38760
   taagaagttt aatagggaga gtttttaaga gtaatttttg gagggagaga gggaggaaag   38820
   gaaaattggg tagagagaga agttgatttt tgatgtagtt gttataaggt ttttagttga   38880
   ttttagggtt tttagttgat tttatagggt gttttggagt tgggatggtt ttgtagagtt   38940
   gtttggaatt aaggtagggt ttagggaggg agataatttt ggtaaaggta gtttttttta   39000
   gtgggggaat ttttagtgag ggatttaatt gtggttgttt gtagtgggta ttggatagtt   39060
   ggggagtgag tttttttgtt ttgatgaggg gtttgggtga tattttatag tatttattgt   39120
   agatttgtag tttttagttt gtagattagg ataagtataa tgtttgtggt gtattttttt   39180
   gtaggtggtt gtgaggagtt aatgagataa tggatatgaa aatgttttgt aaattgtgaa   39240
   gattattaat atgttgttat gtattatttt ttgttatttg ttgttatttt gtttttttat   39300
   tttttttagg ttggaatgat tatatttttt ttttttttag ttttattttt gggttattag   39360
   tatagggaaa aaataagaag aaaaattaga gtggaatttt aaaaaataaa ggttttgtgt   39420
   ttttttgttt aaaatatgta taatttttag ttggtattgt taggaatggg gattgttttt   39480
   atggagaagt ggtattttta tttttagtta gtttttgtgt gagttggatt ttaaggttag 39540
   gagaatgagg aggttgtgaa ggttttggtt gttttttttt ttttttattt ttattgagat 39600
   atagattgag atagggattt ataaattatt ttggagttaa attaggggaa aaaagaaaag 39660
   tttttttagg taatagtatt ttatagtagt aataggaagt tgtagggaaa agagaaaagt 39720
   taaaatttaa gtgaaaattt gagttatggt ttagagaggt tataaatatt ttttttatgg 39780
   agagttagaa gagggaatat agagaaaatt gagaggtttt ttttttgttt gtttgttttg 39840
   aattttttaa aggagaattt gtttttaatt ttgtgaagtt agtaaatatt tttttaaaga 39900
   atgtgtgtgg ttatataagt tgaagggtta gaaaattatt tttttatagt ttagatggtt 39960
   ttaagtggga atagatagga aggagtgtga gttaaagttt ttatttatat tataggattt 40020
   tgttgtttta gttgtttttt tttgttatta gtttaggaag agtgagggtt gttttttaga 40080
   tatagggagt ttttgtaggt tgattttagg tattttagaa tgaggtgttt gtattttttt 40140
   aagtgttgag ggttatttta tttttttttt ttttattaag attttttgaa ttatttagag 40200
   aatatataag ttaaaaataa attattttgg tagatagtga gggtatagaa gtttttggta 40260
   aggttttttt tttaatgaaa attagtttta aatatttttt ttttttaata aagagtagtt 40320
   tgtgaaatta agttatagat atagatgtta gtagaggaat attatttggg attaggtatg 40380
   tttaaaatgg tggttttatt tttttttttt tttttttttt tttttttttt tttttttggt 40440
   tagttagttt gtttttgttg ttgttgttgt tgtttgagat ggagttttgt tttgttgttt 40500
   aggttggagt gtaatggtat ggtggaggtt tattgtaatt tttatttttt aggtttaagt 40560
   gatttttttg ttttagtttt ttaagtagtt gggattatag gaatgtatta ttgtgtttag 40620
   ttaattttgt attttagtag agatggggtt ttattatgtt ggttaggttg gttttgaatt 40680
   tttgattttg tgatttgttt gttttggttt tttaaagtgt tgagattata ggtgtgagtt 40740
   attgtgttta gtttattttt ttttttttag tgttagttat gtgtatatta aggaatagat 40800
   aatatggtgt gggttaagtg gaaagttttt ttgtatagta agatgagggt gaggtagtta 40860
   gttttttttt tgttatgtaa atattatatt taattgaatt gatttgtggg ttttatgtaa 40920
   attagatatt gtttttttaa atttgtttaa aaatttggag aattttgttt tagtaagttt 40980
   ttttttttta gaagttttgt ttttttgtta gagagaaagt tgtttttttt tttttttttt 41040
   ttgtttatta aatttgtgtt tttaaaggtt ttttttgtgt ttttgtttta aatttttttg 41100
   gtgttaggta atgaattttg ggtatttatt ttagataatg aagttgtttt agaatgataa 41160
   aataatagat atggggattt ttggtaaata ataaattata aatgaataga gagagattag 41220
   aaaataaatt taattatagt agaggaggag gggagaaaat agtttttttt tttagaaata 41280
   taggagagat agaaagtagg ttgttttgtt ttgtgtttgt tttatttttt agtaatatat 41340
   tgttttggtg tagtgagatt ttttgtatta tagggtttaa ttagagattt agttattttt 41400
   atttatgttg tgagttaaga ttttgagtaa atgggtttat tttgattgta gttaaagtat 41460
   ttggttgggt atattttaat atgaatatta tattaataat aaaaattgtg gaaatgtggg 41520
   gaaatgttta tgatatgttt ataatttaaa gtaggttagt agtgtattgt taatgtaaat 41580
   aaaatagtat attgagtgtg attttaatat tgaaaaataa aataaagagt attaaatttg 41640
   ttaagtttaa agattagagt gttagatgta aaatgtttaa tgtttttttt tgtgatggga 41700
   ttattttttt ttttatattt ttttgtattt tttgattttt ttttataata attatgtttt 41760
   tattgtaatt tgagatataa aatattagaa aatttggtta gatgagttgt tttaggattg 41820
   tgattatttt tttataaata gatgtttttt aattgataat gggattttgt ttttataagt 41880
   ttattataag ttgaaaatat tgtatgttaa aaatgtattt tatatatttg atttattgaa 41940
   tattatagtt tagttttgtt tattttaaat atgtttagaa tatttagatt agtttatagt 42000
   tgagtaaaat tattttatgt aatttattgg ttattatatt gaaagtgaaa attgaatggt 42060
   tgtgttgttt gttgaagtat ggtttttata aatatatatt gtttttgtgt tattataaag 42120
   ttaagaaatt gttaagttaa attatgagtg agggattatt tgtatattta tatatatatg 42180
   gtatgaatat atgggggtgt gtgtgtgtat gtgtttatgt atgtaaatgt gtgttttaaa 42240
   gttttggggt tttttttttt ttttagttag gagttttttt ttttttgttg agtttttgaa 42300
   ttttgtataa ttgtttatta tatttttgaa aagataattt tttggtatag tatttttagt 42360
   tttttattaa gggattttag tagatgttta tttttagttt agtttggata gggttattaa 42420
   ggagtttgtt tttggttttt atagttattt tttgaagtaa gattgaagag ggtgggagta 42480
   ttatggtttg ttgattttta gttttgtttg tatttttttt ggtagaaggg gttttaggtg 42540
   gtgtttatat tttagatttt agagaggata ttatttttag agagttgaat ttaaaaattg 42600
   atgagtttta aagatgtgaa aaaggataga ttttgttaga tgagtatttt taggttgttg 42660
   gtatggaatt gagggaggaa attgggatta tttgtttgat gttgtagttt ttttattttt 42720
   tttttgttat tgtgagaggt ttggattttt tttgatataa atagtttttt tttattaaag 42780
   gattgtgtta ttttttttat tttttaagta ttaaaaggta gtttaagtag attagtgaat 42840
   taggtaaatg tttatatttt tttttgtttt tttttttttg tatttggtag aatttttttt 42900
   gttttattgt tgattttttt gtaaaagtt 42929
<210> 118
   <211> 42929
   <212> DNA
   <213> Artificial Sequence
<220>
   <223> chemically treated genomic DNA (Homo sapiens)
<400> 118
<210> 119
   <211> 4108
   <212> DNA
   <213> Artificial Sequence
<220>
   <223> chemically treated genomic DNA (Homo sapiens)
<400> 119
   ttggttgttt atgttttagg tttttttttt gtggtgttgg tgaatttgtt agttgttttg 60
   atgtatagta tgatgatgga gattgatttg tatttgtttg gtggtgttta ggtttttatg 120
   aattttttgg gttttgttgg ggtgggtggt ggtgggggtg gaggtggggg tggtggtggt 180
   ggtgggggtg ttaaggttaa ttaggattgg gttaaatggt ttatgaatgt ttttatggtg 240
   tggttttgtg ggtagtggtg taagatggtt taggagaatt ttaagatgta taatttggag 300
   attagtaagt gtttgggggt tgagtggaag gttatgtttg aggttgagaa gtggttgttt 360
   attgatgagg ttaagtggtt gtgtgtgttg tatatgaagg agtatttgga ttataagtat 420
   tggttgtgtt gtaagattaa gatgttgttt aagaaggata agtatttgtt ggttggtggg 480
   tttttggtgg ttggtgtggg tggtggtggt gtggttgtgg ttatgggtgt gggtgtgggt 540
   gtgggtgtgg tggttgtggg ttagtgtttg gagagtttag gtggtgtggt gggtggtggt 600
   tatgtgtatg ttaatggttg ggttaatggt gtttattttg gtttggtggt ggtggtggtg 660
   gtggttgtgg ttatgatgta ggaggtgtag ttggtttatg ggtagtattt gggtgtgggt 720
   ggtgtgtatt tgtatgtgta ttttgtgtat ttgtatttgt attatttgta tgtgtatttg 780
   tataatttgt agtttatgta ttgttatgat atgggtgtgt tgtagtatag ttttattttt 840
   aatttgtagg gttatatgag tgtgttgttt ttgggttatg gtggtttttt ttatggtgtt 900
   gtggttgttg ttgttgttgt tgtgggtggt gtgtattaga atttggttgt ggtggtggtg 960
   gtggtggtgg tggttgtgtt gttgggtgtt ttgggtgtgt tgggtttttt ggtgaagttg 1020
   gagtttagtg gtagtttgtt tgttttagtg tatttgtggg tgttgtgttt tggggatttg 1080
   tgtgagatga ttagtatgta tttgtttgtt ggtgaggggg gtgatttggt ggtggtagta 1140
   gtggttgtgg tgtagagttg gttgtatttg ttgttgtagt attattaggg tgtgggtgtg 1200
   ggtgtgaatg gtatggtgtt tttgatgtat atttagtgtt tttgggatgt tggggatttt 1260
   gtggtggtga tttatgagtt tgtggtttgt gtttggtttt tgttttgttt tggtgtggtg 1320
   tggtttttgt atagatgttt ttatattttt gttaaaagga aaatattgga gatgaatgtt 1380
   gggtgatgtg tgttttttat ttattttttt tggagatttt ggtgattgtt gggtgttgat 1440
   attagatttg ggttttagat tgaattttgg tgtttttttg agattttttg tatagtattt 1500
   attatttatg gaggaagtgg aaagtgtttt ttttgtttga ggggataaaa aagttaaaat 1560
   gaggtgagag gtgaagttta tttttgtata ttggttggtg tgtttatttt tttttgtgtt 1620
   gtttttggat ggtgttgatt gttggagttt aagtgatgtg gagtttgttg tatttgttat 1680
   aaatgtagta aggtaggttt aagtatttat aagttttttg tagttgttat tgtttttttg 1740
   ggttggtttg ttaatttata taaagagatt attattatta tttttttttt tagatggtgg 1800
   agttatattt tgggttttgt aaaattttat gtatttgagt atttttattt ttttttttgg 1860
   gttttgtatt attttttgta aatgtattgt gaaaaatttt atttttggtg ttgtaatgtg 1920
   gggaggagaa gttagattat gtatatagtt ttttaaaaag tttttttttt aaaaatgaaa 1980
   aaagattttt tatttaaaat gttttgagtt aataaattta agagatagag tttatttttt 2040
   ttataaattt gtaatatgtt atttttatgt gtatgtttta tgagtttaaa atgtaatgag 2100
   gaaatttgat agggaaatta tttgtatgaa ttaaaagtaa gggaattttg gggaatggga 2160
   ggataggatt ttttaaggaa ttttttttaa tgaaagagaa ggaagttaaa atttataggt 2220
   tattttgtag agttgagtgt taatatgggt tgagaaataa aagtattttt tgttttggtt 2280
   gttttattgt ggatggttgg ggttgttgtg tgttattttg ttgtaagtgg ggtgtttttt 2340
   atttggttgg gggtttgtgt tatagtttgg tttagaggag ggaggaggaa gggaagattt 2400
   tagtggtggg attttggatt aggttatgga tgaaggataa agattagggt aggttatggg 2460
   ttttttaatt ttttagtaat taagattttg agtagaattt atttaaatgt gttttaagga 2520
   aatataattg ttgaattaaa atgtattgta gttgagtttt ttttgtttat tttttgtttt 2580
   tttttttggt tttttttttt tgggaaaatg ggtaaaataa ttgtgttgga tttttatata 2640
   tatagaaata ttgattatta tttttttttg tgtgaattgg gatgtaagtt gttaattgat 2700
   gtgaatgtaa aatgttttgt ttattatttt tgatgagatt ttgaggttgt ttgatgtttt 2760
   aaatttttta attatattat tttttaggtg tttattggta tattgtagtt ttttttttga 2820
   aatttaaaaa tttttgtaaa attttgtttt taagtaattt gatagtatta aatattgtat 2880
   ttaaaaatta tattgtagta aatatattta aaaattaatt ataatgttaa gatgaaatta 2940
   tatttttgga aaaaaaaaat atttgaagtt tagatggaaa tatgtttatt ttagtagttt    3000
   taggtttttt tttgtttttt taatattttt ttttgttttg gagtatggat tgtttgttta    3060
   aaagtgagtt tatgttataa gtttaatgag aattgaattt agtttgtatt tgagaatagt    3120
   tttaagtata atgttgattt gataattgat gtgtaatttg ggaagttatt ttgataattt    3180
   tgtttaaatt atttatttgt taaagtgatt ataaaaaagt ttaagaatga tgtttattgt    3240
   tttttaataa gataataaat tttttttttt tttttttttt ttttattttt atttttttta   3300
   gttatttgat tttttttgaa gtagttgttt ttggaagagt ttgtgtgttt atggatggtt    3360
   gagtattatt atgatttagt ttgggataag ggttttttta gttttttttg ggagatgatt   3420
   tgggaaattt tataatgttt gttttgttaa tttattggga ttttgagggt ttaatgggat    3480
   tttgagggtt ttttttgaaa tatataaatt taaaggattt tttttgaggt tttttgattg    3540
   atgtttattt ttagtttggt ttttgtgttt ttttgtgtgt attttggagt ttttgtgttt    3600
   aattgttggt atttaggttt tggtttaaga ttaggatgtg ggttttattt tagaggtata    3660
   gattatgaaa agttgagtta gtgtgtttgg gatgttaggt aagtagtttt tatagtttgg    3720
   tattttattg taggtgtttt gtgtatagtt agttgaaata gttaatgtta ggtgttttaa    3780
   ttattttatt tttttgtttt gttgattaga ataatataga aaaaagtaaa tataaatttt    3840
   taatgatttt atttaaaaat attatagggt gggggtaagg aaattagttg agatttattt    3900
   taggattgag attttatttt tttttttttg tttttagtag tgttgtttta atttaaatta    3960
   gtggagaaaa gattatagta ggttttgagt tgattgtgaa tttggtgttt ggttaaggta    4020
   atatttattg tatttatgga gtgaaatatt atatgatgat agttattata ttatatgatg    4080
   attttattta ttttttaaat tatagggt                                        4108
<210> 120
   <211> 4108
   <212> DNA
   <213> Artificial Sequence
<220>
   <223> chemically treated genomic DNA (Homo sapiens)
<400> 120
   attttgtgat ttgggaagtg aatgaagttg ttatataata taataattat tattatatag      60
   tattttattt tgtgaatatg atgagtgtta ttttggttaa gtattgaatt tatagttggt     120
   ttagggttta ttgtaatttt ttttttatta atttgaattg gagtgatatt gttgggggtg     180
   gggaaggggg gatagaattt taattttgag atgaatttta gttaattttt ttgtttttat     240
   tttgtgatat ttttaaatgg agttattaaa aatttatatt tgtttttttt tgtgttgttt     300
   taattaataa aataaggaaa taaggtggtt ggagtatttg gtattggtta ttttagttga     360
   ttgtgtatga agtatttgta ataagatgtt aaattgtaaa agttgtttgt ttggtgtttt     420
   gggtgtatta atttagtttt ttatagtttg tgtttttaaa gtggggttta tattttaatt     480
   ttgagttaag atttagatgt taataattgg atatagaaat tttagggtat atatagggga     540
   gtataggggt tagattgaga gtggatgtta gttaaagaat tttagagaga gttttttaag     600
   tttgtatatt ttagagaaaa tttttaaggt tttattggat ttttaaggtt ttggtgagtt     660
   aatagaataa gtattataaa attttttaaa ttattttttg gagaggattg gggaggtttt     720
   tattttggat taagttgtag tggtgtttag ttatttatgg gtgtatagat ttttttagaa    780
   ataattgttt tagaaaggat taaatagtta aaagaaataa aaataaagaa aaagaaaaga     840
   gggggggggt ttattatttt gttagaaagt agtggatatt atttttgaat ttttttgtaa    900
   ttattttaat gaatgagtgg tttaagtaaa attattaaaa tgatttttta aattatatgt    960
   taattgttag attagtatta tatttaaagt tatttttgaa tgtaggttga atttggtttt   1020
   tattaaattt atagtatagg tttatttttg gatggatagt ttatatttta ggataaggaa   1080
   gggtgttgag aaagtgaggg gaaatttaag gttgttgaaa taaatgtatt tttatttggg   1140
   ttttaagtgt tttttttttt taaaaatata attttatttt aatgttgtga ttaattttta   1200
   aatgtatttg ttatagtata atttttaaat gtaatattta atgttgttag attatttgaa   1260
   gataaagttt tatagaaatt tttaaatttt aaaaaaaaaa ttgtaatgta ttaataaata   1320
   tttagaaaat aatataatta aaaaatttaa agtattaaat aattttaaga ttttgttagg   1380
   aataatgaat aaggtatttt gtgtttatat tggttagtaa tttgtatttt agtttatgtg   1440
   ggggagggtg atggttgata tttttgtgtg tgtgagaatt tagtataatt attttgtttg   1500
   tttttttaag agaaagaagt tagggggagg ggtagaggat ggatggaggg ggtttggttg  1560
   tagtatgttt tggtttagtg attgtgtttt tttgaaatat atttagataa attttgtttg   1620
   aaattttaat tgttggggaa ttgggaaatt tgtgatttgt tttggttttt gttttttatt  1680
   tatggtttgg tttagggttt tattattggg gttttttttt tttttttttt tttttggatt   1740
   aaattgtggt gtagattttt agttaggtgg aaggtgtttt gtttgtagta aggtaatgtg   1800
   tagtagtttt agttgtttgt agtgaaatag ttggagtaga agatattttt attttttggt  1860
   ttgtattaat atttagtttt ataaaataat ttataggttt taattttttt tttttttatt  1920
   gaaaaaggtt ttttgaaaaa ttttgttttt ttattttttg gggttttttt atttttagtt   1980
   tatatagata atttttttgt tagatttttt tattgtattt tgaatttata aaatatgtat  2040
   ataaaaatag tatgttataa atttatggag aaaatgggtt ttgtttttta aatttgttga     2100
   tttgaaatat tttgggtggg gggttttttt ttgtttttag aagaaaggtt ttttagaaaa     2160
   ttatgtatat aatttgattt tttttttttg tattgtaatg ttgaaaataa aattttttat     2220
   aatgtattta taagaaataa tataaaattt aaaaaaaaaa atggaaatgt ttagatatat     2280
   aaagttttat aaaatttaga atataatttt gttgtttgaa ggagggggtg gtggtggtaa     2340
   tttttttgta taaattaata aattaattta aaagagtggt aataattata aaaaatttgt     2400
   aagtgtttgg atttgtttta ttatatttat aataaatgtg atgagttttg tgttatttgg     2460
   gttttggtgg ttggtgttgt ttggaagtaa tgtggaggaa agtgagtgtg ttggttggta     2520
   tataaaagtg ggttttgttt tttgttttgt tttgattttt ttgttttttt gagtaaagaa     2580
   aatgtttttt gttttttttg taggtgataa atattgtata aaaagtttta agaaaatatt     2640
   gaagtttagt ttaaaattta agtttggtgt tagtgtttgg tggttgttag ggtttttggg     2700
   gaaggtgagt gggggatatg tgttatttgg tgtttgtttt tagtattttt tttttgataa     2760
   gaatgtggga atgtttgtat aaaagttatg ttgtgttggg gtggggtggg agttgggtgt     2820
   gggttgtgag tttgtgggtt gttgttgtag agtttttggt gttttgaagg tgttagatgt     2880
   gtgttagggg tattgtgttg tttatgtttg tgtttgtgtt ttggtagtgt tgtggtagtg     2940
   agtgtagttg gttttgtgtt gtggttgttg ttgttgttgt tgggttgttt tttttgttgg     3000
   tgggtaagta tatgttgatt attttgtgta ggtttttggg gtatggtgtt tgtgagtgtg     3060
   ttggggtggg tgggttgttg ttgggttttg attttattag agagtttagt gtgtttaggg     3120
   tgtttgatga tgtggttgtt gttgttgttg ttgttgttat ggttgagttt tggtgtgtgt     3180
   tgtttgtagt ggtggtggtg gtggttgtgg tgttgtaggg gaggttgttg tagtttgagg     3240
   gtgatgtgtt tatgtagttt tgtgagttgg agatggggtt gtattgtagt gtgtttatgt     3300
   tgtagtggtg tatgggttgt gggttgtgtg ggtgtgtgtg tgggtggtgt gggtgtgggt     3360
   gtgtggggtg tgtgtgtggg tgtgtgttgt ttgtgtttgg gtgttgtttg taggttagtt     3420
   gtgttttttg tattatggtt gtggttgttg ttgttgttgt tattgagttg gggtaggtgt     3480
   tgttggttta gttgttgatg tgtgtgtagt tgttgtttgt tgtgttgttt gggtttttta     3540
   ggtgttggtt tatggttgtt gtgtttatgt ttatgtttat gtttatggtt atagttgtgt     3600
   tgttgttatt tgtgttggtt gttaggagtt tgttggttag tgagtatttg ttttttttga     3660
   gtagtgtttt ggttttgtgg tgtggttggt atttgtaatt tgggtgtttt tttatgtgta     3720
   gtgtgtgtag ttgtttggtt ttgttgatga atggttgttt tttggttttg gatatgattt     3780
   tttatttggt ttttaggtgt ttgttgattt ttgagttgtg tattttgggg tttttttggg     3840
   ttattttgtg ttgttgtttg tgggattata ttatgaaggt gtttatgggt tgtttgattt     3900
   ggttttggtt ggttttggtg tttttgttgt tgttgttgtt tttgtttttg tttttgttgt     3960
   tgtttgtttt ggtggggttt gagaggtttg tgggggtttg ggtgttgttg ggtgagtgta     4020
   ggttggtttt tattattatg ttgtatattg gggtggttgg tgggtttatt ggtattgtga     4080
   ggagagggtt tggagtatag atggttgg

## Claims

1. A method for detecting bladder carcinoma comprising determining the methylation status of CYP1B1 genomic DNA according to SEQ NO: 12 in a biological sample isolated from a subject, wherein hypermethylation is indicative of the presence of bladder carcinoma.

2. A method according to claim 1, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of SEQ NO: 12 respectively, wherein said contiguous nucleotides comprise at least one CpG dinucleotide sequence, and whereby detecting bladder carcinoma is, at least in part, afforded.

3. A method according to claim 1, comprising:
a. extracting or otherwise isolating genomic DNA from a biological sample obtained from the subject;
b. treating the genomic DNA of a), or a fragment thereof, with one or more reagents to convert cytosine bases that are unmethylated in the 5-position thereof to uracil or to another base that is detectably dissimilar to cytosine in terms of hybridization properties;
c. contacting the treated genomic DNA, or the treated fragment thereof, with an amplification enzyme and at least one primer comprising a contiguous sequence of at least 9 nucleotides that is complementary to, or hybridizes under moderately stringent or stringent conditions to a sequence selected from the group consisting of SEQ NOS: 38, 39, 68, and 69 and complements thereof, wherein the treated genomic DNA or the fragment thereof is either amplified to produce at least one amplificate, or is not amplified; and
d. determining, based on a presence or absence of, or on a property of said amplificate, the methylation state or level of at least one CpG dinucleotide of a genomic sequence of SEQ NO: 12 or an average, or a value reflecting an average methylation state or level of a plurality of CpG dinucleotides of at least one of said genomic sequences, whereby at least one of detecting and diagnosing bladder carcinoma is, at least in part, afforded.

4. The method of claim 3, wherein treating the genomic DNA or the fragment thereof in b) comprises use of a reagent selected from the group comprising of bisulfite, hydrogen sulfite, disulfite, and combinations thereof.

5. The method of any of claim 1 to 4, wherein the biological sample obtained from the subject is selected from the group comprising bladder tissue samples, histological slides, tissue embedded in paraffin, body fluids like blood plasma or serum, cell lines, urine specimen and combinations thereof.

6. A method according to claim 1, comprising:
a. extracting or otherwise isolating genomic DNA from a biological sample obtained from the subject;
b. digesting the genomic DNA of a), or a fragment thereof, with one or more methylation sensitive restriction enzymes; contacting the DNA restriction enzyme digest of b) with an amplification enzyme and at least two primers suitable for the amplification of a genomic sequence comprising at least one CpG dinucleotide of a genomic sequence of SEQ NO: 12; and
c. determining, based on a presence or absence of an amplificate the methylation state or level of at least one CpG dinucleotide of a genomic sequence of SEQ NO: 12, whereby detecting said disorder is at least in part, afforded.

7. Use of a nucleic acid for performing the method of claims 1 to 6 in the detection of bladder carcinoma, wherein the nucleic acid comprises at least 16 contiguous nucleotides of a sequence selected from the group consisting of SEQ NOS: 38, 39, 68, and 69, and sequences complementary thereto.

8. The use according to claim 7, wherein the nucleic acid comprises at least 50 contiguous nucleotides of a sequence selected from the group consisting of SEQ NOS: 38, 39, 68, and 69, and sequences complementary thereto.

9. The use according to any of claims 7 to 8, wherein the contiguous base sequence comprises at least one CpG, TpG or CpA dinucleotide sequence.

10. Use of a kit for performing the method according to claims 1 to 6, wherein the kit comprises (a) a bisulfite reagent; (b) a container suitable for containing the said bisulfite reagent and the biological sample of the patient; (c) at least one set of oligonucleotides containing two oligonucleotides whose sequences in each case are identical, are complementary, or hybridize under stringent or highly stringent conditions to a 9 or more preferably 18 base long segment of a sequence selected from SEQ NOS: 38, 39, 68, and 69.

11. Use of a kit for performing the method according to claims 1 to 6, wherein the kit comprises (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of a genomic sequence of SEQ NO: 12; and optionally (d) instructions for use and interpretation of the kit results.

## Patentansprüche

1. Verfahren zur Detektion von Blasenkarzinom, umfassend das Bestimmen des Methylierungsstatus von genomischer CYP1B1-DNA gemäß SEQ ID NO: 12 in einer von einem Individuum isolierten biologischen Probe, wobei die Hypermethylierung auf das Vorhandensein von Blasenkarzinom hinweist.

2. Verfahren gemäß Anspruch 1, umfassend das Inkontaktbringen genomischer DNA, welche aus einer von einem Individuum erhaltenen biologischen Probe isoliert wurde, mit mindestens einem Reagenz oder einer Serie von Reagenzien, die zwischen methylierten und niclat-methylierten CpG Dinukleotiden innerhalb mindestens einer Targetregion der genomischen DNA unterscheiden, wobei die Targetregion eine Sequenz von mindestens 16 benachbarten Nukleotiden der SEQ ID NO: 12 umfasst oder unter stringenten Bedingungen damit hybridisiert, wobei besagte benachbarte Nukleotide mindestens eine CpG Dinukleotid Sequenz umfassen, und wobei das Detektieren eines Blasenkarzinoms zumindest teilweise gewährleistet ist.

3. Verfahren gemäß Anspruch 1, umfassend:
a. Extrahieren oder in anderer Weise Isolieren genomischer DNA aus einer biologischen vom Individuum erhaltenen Probe;
b. Behandeln der genomischen DNA aus Schritt a), oder eines Fragments davon, mit einem oder mehreren Reagenzien, um in der 5'-Position unmethylierte Cytosinbasen in Uracil oder in eine andere Base, die Cytosin hinsichtlich der Hybridisierungseigenschaften nachweisbar unähnlich ist, umzuwandeln;
c. Inkontaktbringen der behandelten genomischen DNA oder des behandelten Fragments davon mit einem Amplifikationsenzym und mindestens einem Primer, der eine benachbarte Sequenz von mindestens 9 Nukleotiden umfasst, die komplementär zu ist oder unter moderat stringenten oder stringenten Bedingungen an eine Sequenz ausgewählt aus der Gruppe bestehend aus SEQ ID NOs: 38, 39, 68 und 69 und Komplementären dieser hybridisiert, wobei die behandelte genomischen DNA oder das Fragment davon entweder amplifiziert wird, um mindestens ein Amplifikat zu bilden, oder nicht amplifiziert wird; und
d. Bestimmen des Methylierungsstatus oder -levels von mindestens einem CpG Dinukleotid einer genomischen Sequenz von SEQ ID NO: 12 oder eines Mittelwertes oder eines Wertes der einen mittleren Methylierungsstatus oder -levels einer Vielzahl von CpG Dinukleotiden von mindestens einer der genannten genomischen Sequenzen reflektiert, basierend auf der Anwesenheit oder Abwesenheit von, oder einer Eigenschaft des besagten Amplifikats, wobei mindestens eines von Detektieren und Diagnose eines Blasenkarzinoms zumindest teilweise gewährleistet ist.

4. Verfahren gemäß Anspruch 3, wobei Behandeln der genomischen DNA oder des Fragments davon in Schritt b) die Verwendung eines Reagenz ausgewählt aus der Gruppe bestehend aus Bisulfit, Hydrogensulfit, Disulfit und Kombinationen dieser, umfasst.

5. Verfahren gemäß einem der Ansprüche 1 bis 4, wobei die von dem Individuum erhaltene biologische Probe ausgewählt ist aus der Gruppe bestehend aus Blasengewebeproben, histologischen Präparaten, in Paraffin eingebettetem Gewebe, Körperflüssigkeiten wie Blutplasma oder Serum, Zelllinien, Urinproben und Kombinationen davon.

6. Verfahren gemäß Anspruch 1, umfassend:
a. Extrahieren oder in anderer Weise Isolieren genomischen DNA aus einer biologischen vom Individuum erhaltenen Probe;
b. Verdauen der genomischen DNA aus Schritt a) oder eines Fragments davon mit einem oder mehreren methylierungssensitiven Restriktionsenzymen; Inkontaktbringen des DNA-Restriktionsenzymverdaus von b) mit einem Amplifikationsenzym und mindestens zwei Primern, die für die Amplifikation einer genomischen Sequenz geeignet sind, die mindestens ein CpG Dinukleotid einer genomischen Sequenz von SEQ ID NO: 12 umfasst; und
c. Bestimmen des Methylierungsstatus oder -levels von mindestens einem CpG Dinukleotid einer genomischen Sequenz von SEQ ID NO: 12, basierend auf der Anwesenheit oder Abwesenheit von, oder einer Eigenschaft des besagten Amplifikats, wobei das Detektieren der Störung zumindest teilweise gewährleistet ist.

7. Verwendung einer Nukleinsäure zur Durchführung des Verfahrens gemäß den Ansprüchen 1 bis 6 beim Detektieren von Blasenkarzinom, wobei die Nukleinsäure mindestens 16 benachbarte Nukleotide einer Sequenz ausgewählt aus der Gruppe bestehend aus SEQ ID NOs: 38, 39, 68, und 69 und dazu komplementären Sequenzen umfasst.

8. Verwendung gemäß Anspruch 7, wobei die Nukleinsäure mindestens 50 benachbarte Nukleotide einer Sequenz ausgewählt aus der Gruppe bestehend aus SEQ ID NOs: 38, 39, 68 und 69 und dazu komplementären Sequenzen umfasst.

9. Verwendung gemäß einem der Ansprüche 7 bis 8, wobei die benachbarte Basensequenz mindestens eine CpG, TpG oder CpA Dinukleotidsequenz umfasst.

10. Verwendung eines Kits zur Durchführung des Verfahrens gemäß den Ansprüchen 1 bis 6, wobei das Kit umfasst: (a) ein Bisulfit-Reagenz; (b) einen Behälter, der geeignet ist, das besagte Bisulfit-Reagenz und die biologische Probe des Patienten zu enthalten; (c) mindestens ein Satz Oligonukleotide, der zwei Oligonukleotide enthält, deren Sequenzen jeweils identisch sind zu, komplementär sind zu, oder unter stringenten oder hoclzstringenten Bedingungen an ein 9 oder mehr bevorzugt 18 Basen langes Segment einer Sequenz ausgewählt aus SEQ ID NOs: 38, 39, 68 und 69 hybridisieren.

11. Verwendung eines Kits zur Durchführung des Verfahrens gemäß den Ansprüchen 1 bis 6, wobei das Kit umfasst: (a) ein methylierungssensitives Restriktionsenzymreagens; (b) einen Behälter, der geeignet ist, das besagte Reagenz und die biologische Probe des Patienten zu enthalten; (c) mindestens ein Satz Oligonukleotide, eine oder eine Vielzahl an Nukleinsäuren oder Peptidnukleinsäuren, die identisch sind zu, komplementär sind zu, oder unter stringenten oder hochstringenten Bedingungen an ein mindestens 9 Basen langes Segment einer genomischen Sequenz von SEQ ID NO: 12 hybridisieren; und gegebenenfalls (d) Anweisungen zur Verwendung und Interpretation der Kit-Ergebnisse.

## Revendications

1. Procédé pour la détection du carcinome de la vessie comprenant la détermination de l'état de méthylation de l'ADN génomique CYP1B1 selon SEQ ID NO: 12 dans un échantillon biologique isolé à partir d'un sujet, où une hyperméthylation est indicatrice de la présence de carcinome de la vessie.

2. Procédé selon la revendication 1, comprenant la mise en contact d'ADN génomique isolé à partir d'un échantillon biologique obtenu à partir dudit sujet avec au moins un réactif, ou une série de réactifs faisant la distinction entre dinucléotides CpG méthylés et non-méthylés dans au moins une région cible de l'ADN génomique, tandis que la région cible comprend, ou hybride dans des conditions stringentes à une séquence d'au moins 16 nucléotides contigus de SEQ ID NO: 12 respectivement, tandis que lesdits nucléotides contigus comprennent au moins une séquence dinucléotidique CpG, et ce par quoi est procurée, au moins pour partie, la détection du carcinome de la vessie.

3. Procédé selon la revendication 1, comprenant:
a. l'extraction ou isolement d'une autre manière d'ADN génomique à partir d'un échantillon biologique obtenu à partir d'un sujet;
b. le traitement de l'ADN génomique de a), ou d'un fragment de celui-ci, avec un ou plusieurs réactifs pour convertir les bases cytosine qui sont non-méthylées dans la position 5 en uracile ou en une autre base qui à la détection n'apparaît pas semblable à la cytosine en termes de propriétés d'hybridation;
c. la mise en contact de l'ADN génomique traité, ou du fragment traité de celui-ci, avec une enzyme d'amplification et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NOS: 38, 39, 68, et 69 et leurs compléments, tandis que l'ADN génomique traité ou le fragment de celui-ci soit est amplifié pour produire au moins un produit d'amplification soit n'est pas amplifié; et
d. la détermination, sur la base d'une présence ou d'une absence de, ou d'une propriété dudit produit d'amplification, de l'état ou du niveau de méthylation d'au moins un dinucléotide CpG d'une séquence génomique selon SEQ ID NO: 12 ou d'une moyenne, ou d'une valeur reflétant un état ou un niveau de méthylation moyen d'une pluralité de dinucléotides CpG d'au moins l'une desdites séquences génomiques, ce par quoi est procuré, au moins pour partie, au moins l'un parmi la détection et le diagnostic du carcinome de la vessie.

4. Procédé selon la revendication 3, dans lequel l'ADN génomique ou le fragment de celui-ci dans b) comprend l'utilisation d'un réactif choisi dans le groupe consistant en bisulfite, hydrogèno sulfite, disulfite, et leurs combinaisons.

5. Procédé selon l'une quelconque des revendications 1 à 4, où l'échantillon biologique obtenu à partir du sujet est choisi dans le groupe comprenant des échantillons de tissu de la vessie, des coupes histologiques, du tissu enrobé dans la paraffine, des fluides corporels tels que plasma ou sérum sanguin, des lignées cellulaires, un échantillon d'urine et leurs combinaison.

6. Procédé selon la revendication 1, comprenant:
a. l'extraction ou l'isolement d'une autre manière d'ADN génomique à partir d'un échantillon biologique obtenu à partir du sujet;
b. la digestion de l'ADN génomique de a), ou d'un fragment de celui-ci, avec une ou plusieurs enzymes de restriction sensibles à la méthylation; la mise en contact du produit de digestion de l'enzyme de restriction d'ADN de b) avec une enzyme d'amplification et au moins deux amorces convenant pour l'amplification d'une séquence génomique comprenant au moins un dinucléotide CpG d'une séquence génomique selon SEQ ID NO: 12; et
c. la détermination, sur la base d'une présence ou d'une absence d'un produit d'amplification de l'état ou du niveau de méthylation d'au moins un dinucléotide CpG d'une séquence génomique selon SEQ ID NO: 12, ce par quoi est procurée, au moins pour partie, la détection dudit trouble.

7. Utilisation d'un acide nucléique pour la mise en oeuvre du procédé selon les revendications 1 à 6 dans la détection du carcinome de la vessie, où l'acide nucléique comprend au moins 16 nucléotides contigus d'une séquence choisie dans le groupe consistant en SEQ ID NOS: 38, 39, 68, et 69, et des séquences qui leur sont complémentaires de celles-ci.

8. Utilisation selon la revendication 7, où l'acide nucléique comprend au moins 50 nucléotides contigus d'une séquence choisie dans le groupe consistant en SEQ ID NOS: 38, 39, 68, et 69 et des séquences qui leur sont complémentaires.

9. Utilisation selon l'une quelconque des revendications 7 à 8, où la séquence de bases contiguës comprend au moins une séquence dinucléotidique CpG, TpG ou CpA.

10. Utilisation d'un kit pour la mise en oeuvre du procédé selon les revendications 1 à 6, où le kit comprend (a) un réactif au bisulfite,; (b) un récipient approprié pour contenir ledit réactif au bisulfite et l'écllantillon biologique du patient; (c) au moins un ensemble d'oligonucléotides contenant deux oligonucléotides dont les séquences sont dans chaque cas identiques, sont complémentaires, ou hybrident dans des conditions stringentes ou fortement stringentes à un segment de 9 ou plus préférablement 18 bases de long d'une séquence choisie parmi SEQ ID NOS: 38, 39, 68, et 69.

11. Utilisation d'un kit pour la mise en oeuvre du procédé selon les revendications 1 à 6, où le kit comprend (a) un réactif enzyme de restriction sensible à la méthylation; (b) un récipient approprié pour contenir ledit réactif et l'échantillon biologique du patient; (c) au moins un ensemble d'oligonucléotides un ou une pluralité d'acides nucléiques ou d'acides nucléiques peptidiques qui sont identiques, sont complémentaires, ou hybrident dans des conditions stringentes ou fortement stringentes à un segment d'au moins 9 bases de long d'une séquence génomique selon SEQ ID NO: 12, et en option (d) des instructions pour l'utilisation et l'interprétation des résultats du kit.
